(19) Europäisches Patentamt / European Patent Office / Office européen des brevets

(11) EP 4 644 383 A1

(12) **EUROPEAN PATENT APPLICATION**
published in accordance with Art. 153(4) EPC

(43) Date of publication:
**05.11.2025 Bulletin 2025/45**

(21) Application number: **23910904.4**

(22) Date of filing: **29.12.2023**

(51) International Patent Classification (IPC):
***C07D 401/14*** (2006.01) ***A61K 31/4545*** (2006.01)
***A61K 31/4439*** (2006.01) ***A61K 31/496*** (2006.01)
***A61K 31/5377*** (2006.01) ***A61P 35/00*** (2006.01)

(52) Cooperative Patent Classification (CPC):
**A61K 31/4439; A61K 31/4545; A61K 31/496; A61K 31/5377; A61P 35/00; C07D 401/14**

(86) International application number:
**PCT/CN2023/143092**

(87) International publication number:
**WO 2024/140990 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.12.2022 CN 202211728240**
**30.03.2023 CN 202310333126**

(71) Applicants:
- **Jiangsu Yahong Meditech Co., Ltd.**
  **Taizhou, Jiangsu 225316 (CN)**
- **Asieris Pharmaceuticals (Shanghai) Co., Ltd.**
  **Pudong, Shanghai 201203 (CN)**

(72) Inventors:
- **SONG, Haifeng**
  **Taizhou, Jiangsu 225316 (CN)**
- **WANG, Tielin**
  **Taizhou, Jiangsu 225316 (CN)**
- **GUAN, Jingmin**
  **Taizhou, Jiangsu 225316 (CN)**
- **XIAO, Lu**
  **Taizhou, Jiangsu 225316 (CN)**
- **PENG, Cheng**
  **Taizhou, Jiangsu 225316 (CN)**

(74) Representative: **Regimbeau**
**20, rue de Chazelles**
**75847 Paris Cedex 17 (FR)**

(54) **PYRAZOLE FUSED RING COMPOUND, AND PREPARATION METHOD THEREFOR AND USE THEREOF**

(57) The present invention relates to a pyrazole fused ring compound, and a preparation method therefor and the use thereof. Specifically, the present invention relates to a compound as represented by general formula (I), a preparation method therefor, a pharmaceutical composition comprising the compound, and the use thereof as an FGFR inhibitor in the treatment of tumors. Each group in the general formula (I) is as defined in the description.

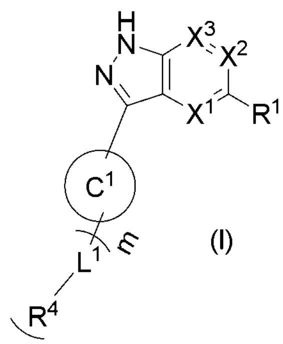

(I)

EP 4 644 383 A1

## Description

### FIELD OF THE INVENTION

The present invention belongs to the field of medicinal chemistry. In particular, the present invention relates to an inhibitor of FGFRs (fibroblast growth factor receptors) and their mutants, a preparation method therefor, and a use of the compound in the treatment of a disease such as cancer.

### BACKGROUND OF THE INVENTION

Fibroblast growth factor receptors (FGFRs) are a subfamily of tyrosine kinase receptors, and include four subtypes: FGFR1, FGFR2, FGFR3, and FGFR4. Each FGFR consists of three distinct domains, namely an extracellular ligand-binding domain, a single-channel transmembrane domain, and an intracellular TK domain. After binding to a fibroblast growth factor (FGF) ligand, FGFR is activated and phosphorylates several signaling proteins downstream, including PI3K-AKT, RAS-MAPK, and STAT. Therefore, FGFR plays a crucial role in many intracellular processes, including development, differentiation, survival, migration, and angiogenesis.

However, FGFR is susceptible to various somatic cell aberrations and prone to mutation such as common overexpression, point mutation and gene translocation, thereby resulting in carcinogenesis. Overexpression of FGFR caused by gene amplification, EC region (extracellular domain)/TK region (kinase domain) mutation of FGFR, FGFR fusion and so on can lead to signal dysregulation of FGFR, thereby promoting proliferation, survival, invasion, metastasis, and development of drug resistance of tumor cells, as well as occurrence of angiogenesis in tumor microenvironment (TME) and immune evasion. FGFR aberration accounts for approximately 7.1% of all patients with solid tumor. The cancers most frequently affected by FGFR aberration are urothelial cell carcinoma (32%), liver cancer (30%), cholangiocarcinoma (25%), breast cancer (18%), gastric cancer (7%), etc.

FGFR inhibitors achieve the goal of inhibiting tumor growth by blocking FGFR-mediated signaling pathways, making them a new choice for targeted cancer therapy. At present, three drugs targeting FGFR have been approved around the world: Balversa (Erdafitinib) from Johnson&Johnson, Pemigatinib from Incyte, and Infigratinib from Bridge-Bio/Helsinn. However, in clinical trials, the three approved FGFR inhibitors above still cause some treatment-related adverse events, such as hyperphosphatemia, dry mouth, fatigue, skin change, nail change, and eye disease. In addition, in clinical trials, it has been found that mutations affecting amino acids in FGFR can lead to resistance or reduce sensitivity to FGFR inhibitors. Therefore, there is a highly unmet clinical demand for the development of drugs targeting FGFR.

### SUMMARY OF THE INVENTION

The inventors of the present application have conducted extensive and in-depth research and developed a novel FGFR inhibitor. The compounds of the present invention have good activity against FGFR, especially against FGFR3, and are expected to develop into a new generation of FGFR inhibitors.

The present invention provides a compound of formula (I),

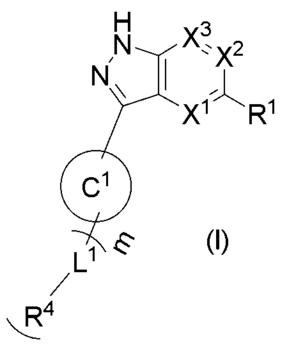

(I)

or a pharmaceutically acceptable salt, hydrate, solvate, isotope substitute or stereoisomer thereof, wherein,

$X^1$ is N atom or $CR^5$;
$X^2$ is N atom or $CR^2$;
$X^3$ is N atom or $CR^3$;
$R^1$ is selected from the group consisting of

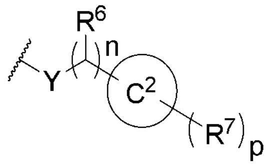

,

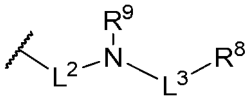 and 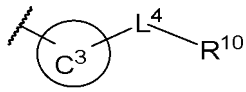;

Y is selected from the group consisting of O atom, S atom, -S(O)-, $-S(O)_2-$ and $NR^{11}$;

$R^2$ and $R^5$ are each independently selected from the group consisting of H atom, - OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of $C_{1-6}$ alkoxy, -OH, -COOH, $-NR^{11}R^{12}$, -CN, halogen, $C_{1-6}$ haloalkyl, $C_{1-6}$ haloalkoxy and $C_{1-6}$ hydroxyalkyl;

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$;

provided that $R^3$ is not H atom when X is $CR^5$, $R^1$ is

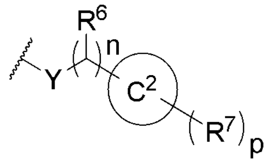

and $C^2$ is a 6 membered nitrogen-containing heteroaryl ring;

ring $C^1$ is selected from the group consisting of $C_{6-10}$ aryl, 5 to 10 membered heteroaryl, $C_{3-8}$ cycloalkyl, 3 to 8 membered heterocyclyl, 5 to 10 membered heteroaryl fused with 3 to 8 membered heterocyclyl, 5 to 10 membered heteroaryl fused with $C_{6-10}$ aryl, 3 to 8 membered heterocyclyl fused with 5 to 10 membered heteroaryl, and $C_{6-10}$ aryl fused with 5 to 10 membered heteroaryl;

each $L^1$ is independently selected from the group consisting of chemical bond, $-C_{1-6}$ alkylene-, -O-, $-C_{1-6}$ alkylene-O-, $-O-C_{1-6}$ alkylene-, -C(O)-, -O-C(O)-, -C(O)-O-, -S-, - S(O)-, $-S(O)_2-$, $-S(O)(=NR^{11})-$, $-C_{1-6}$ alkylene-C(O)-, $-C(O)-C_{1-6}$ alkylene-, $-C_{1-6}$ alkylene-$S(O)_2$-, $-S(O)_2-C_{1-6}$ alkylene-, $-C_{1-6}$ alkylene-$S(O)(=NR^{11})$-, $-S(O)(=NR^{11})-C_{1-6}$ alkylene-, 3 to 8 membered heterocyclylene, $C_{3-8}$ cycloalkylene, $C_{6-10}$ arylene, 5 to 10 membered heteroarylene, $-C_{1-6}$ alkylene-$O-C_{1-6}$ alkylene-O-, $-O-C_{1-6}$ alkylene-$O-C_{1-6}$ alkylene-, $-NR^{11}-$, $-NR^{11}-C_{1-6}$ alkylene-, $-C_{1-6}$ alkylene-$NR^{11}$-, $-C_{1-6}$ alkylene-$NR^{11}-C_{1-6}$ alkylene-O-, $-O-C_{1-6}$ alkylene-$NR^{11}-C_{1-6}$ alkylene-, -NH-C(O)-, $-C(O)-NR^{11}-$, $-C(O)-NR^{11}-C_{1-6}$ alkylene-, $-C_{1-6}$ alkylene-$C(O)-NR^{11}$-, $-C_{1-6}$ alkylene-$NR^{11}$-C(O)- and $-NR^{11}-C(O)-C_{1-6}$ alkylene-, wherein the $C_{1-6}$ alkylene is each independently and optionally substituted by one or more substituents selected from the group consisting of $C_{1-6}$ alkyl, halogen, -OH, -COOH, $-NR^{11}R^{12}$, -CN, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;

each $R^4$ is independently selected from the group consisting of H atom, -OH, - COOH, -CN, halogen, $C_{1-6}$ alkyl, $C_{1-6}$ deuterated alkyl, $C_{2-6}$ alkenyl, $C_{2-6}$ alkynyl, $C_{1-6}$ alkoxy, $C_{1-6}$ haloalkyl, $C_{1-6}$ haloalkoxy, $-C(O)-NR^{11}R^{12}$, $-NR^{11}R^{12}$, $C_{1-6}$ hydroxyalkyl, $C_{6-10}$ aryl, 5 to 10 membered heteroaryl, $C_{3-8}$ cycloalkyl and 3 to 10 membered heterocyclyl, wherein the $C_{6-10}$ aryl, 5 to 10 membered heteroaryl, $C_{3-8}$ cycloalkyl and 3 to 10 membered heterocyclyl are each independently and optionally substituted by one or more substituents selected from the group consisting of -CN, -OH, $C_{1-6}$ alkyl, $C_{1-6}$ hydroxyalkyl, $-C(O)-R^{14}$, $-C(O)-NR^{11}R^{12}$, oxo, $C_{1-6}$ alkoxy, $-S(O)_2-C_{1-6}$ alkyl, $-NR^{11}R^{12}$, imino, halogen, $-C_{1-6}$ alkylene-$NR^{11}R^{12}$, $-NR^{11}-C(O)-R^{14}$, $-NR^{11}-S(O)_2-R^{14}$, - $S(O)(=NR^{11})-R^{14}$, $-S(O)_2-R^{14}$, $-C(=NR^{11})-NR^{11}R^{12}$, $-S(O)_2-NR^{11}R^{12}$ and $-C_{1-6}$ alkylene-$C(O)-NR^{11}R^{12}$;

ring $C^2$ is selected from the group consisting of $C_{6-10}$ aryl, 5 to 10 membered heteroaryl, $C_{3-8}$ cycloalkyl, 3 to 8 membered heterocyclyl, $C_{3-8}$ cycloalkyl fused with 5 to 10 membered heteroaryl, 5 to 10 membered heteroaryl fused with $C_{6-10}$ aryl, $C_{6-10}$ aryl fused with 3 to 8 membered heterocyclyl, 5 to 10 membered heteroaryl fused with $C_{3-8}$ cycloalkyl, $C_{6-10}$ aryl fused with 5 to 10 membered hereroaryl, and 3 to 8 membered heterocyclyl fused with $C_{6-10}$ aryl;

ring $C^3$ is selected from the group consisting of $C_{6-10}$ aryl, 5 to 10 membered heteroaryl, $C_{3-8}$ cycloalkyl, 3 to 8

membered heterocyclyl, $C_{3-8}$ cycloalkyl fused with 5 to 10 membered heteroaryl, 5 to 10 membered heteroaryl fused with $C_{6-10}$ aryl, 5 to 10 membered heteroaryl fused with $C_{3-8}$ cycloalkyl, and $C_{6-10}$ aryl fused with 5 to 10 membered heteroaryl;

$R^6$ is selected from the group consisting of H atom, -OH, -COOH, $-NR^{11}R^{12}$, -CN, halogen, $C_{1-6}$ alkyl, $C_{1-6}$ deuterated alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $-C_{1-6}$ alkylene-$NR^{11}R^{12}$, $C_{6-10}$ aryl, 5 to 10 membered heteroaryl, $C_{3-8}$ cycloalkyl and 3 to 8 membered heterocyclyl;

$R^7$ is selected from the group consisting of H atom, -OH, -COOH, $-C(O)-R^{14}$, $-C(O)-NR^{11}R^{12}$, $-NR^{11}R^{12}$, -CN, halogen, oxo, $C_{1-6}$ alkyl, $C_{1-6}$ deuterated alkyl, $C_{2-6}$ alkenyl, $C_{2-6}$ alkynyl, $C_{1-6}$ alkoxy, $C_{1-6}$ deuterated alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;

$L^2$ is selected from the group consisting of -C(O)-, $-S(O)_2-$ and -S(O)-;

$L^3$ and $L^4$ are each independently selected from the group consisting of chemical bond, $-C_{1-6}$ alkylene-, -O-, $-C_{1-6}$ alkylene-O-, $-O-C_{1-6}$ alkylene-, -C(O)-, -O-C(O)-, -C(O)-O-, -S-, -S(O)-, $-S(O)_2-$, $-C_{1-6}$ alkylene-C(O)-, $-C(O)-C_{1-6}$ alkylene-, $-C_{1-6}$ alkylene-$S(O)_2$-, $-S(O)_2-C_{1-6}$ alkylene-, $-C_{1-6}$ alkylene-$O-C_{1-6}$ alkylene-O-, $-O-C_{1-6}$ alkylene-$O-C_{1-6}$ alkylene-, $-NR^{11}-$, $-NR^{11}-C_{1-6}$ alkylene-, $-C_{1-6}$ alkylene-$NR^{11}$-, $-C_{1-6}$ alkylene-$NR^{11}-C_{1-6}$ alkylene-O-, $-O-C_{1-6}$ alkylene-$NR^{11}-C_{1-6}$ alkylene-, $-NR^{11}-C(O)-$, $-C(O)-NR^{11}-$, $-C(O)-NR^{11}-C_{1-6}$ alkylene-, $-C_{1-6}$ alkylene-$C(O)-NR^{11}$-, $-C_{1-6}$ alkylene-$NR^{11}-C(O)$- and $-NR^{11}-C(O)-C_{1-6}$ alkylene-, wherein the $C_{1-6}$ alkylene is each independently and optionally substituted by one or more substituents selected from the group consisting of $C_{1-6}$ alkyl, halogen, -OH, -COOH, $-NR^{11}R^{12}$, -CN, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;

$R^8$ and $R^{10}$ are each independently selected from the group consisting of H atom, - OH, -COOH, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of -CN, -OH, $C_{1-6}$ alkyl, $-C(O)-C_{1-6}$ alkyl, $-C(O)-C_{1-6}$ hydroxyalkyl, - $C(O)-NR^{11}R^{12}$, oxo, $C_{1-6}$ alkoxy, $-S(O)_2-C_{1-6}$ alkyl, $-NR^{11}R^{12}$ and halogen;

$R^9$ is selected from the group consisting of H atom, $C_{1-6}$ alkyl, $C_{1-6}$ alkoxy, $C_{1-6}$ haloalkyl, $C_{1-6}$ haloalkoxy and $C_{1-6}$ hydroxyalkyl;

each $R^{11}$ is independently selected from the group consisting of H atom, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $C_{3-8}$ cycloalkyl, wherein the $C_{3-8}$ cycloalkyl is optionally substituted by one or more substituents selected from the group consisting of $C_{1-6}$ alkyl, $C_{1-6}$ alkoxy, -OH, -COOH, - $NH_2$, -CN, halogen, $C_{1-6}$ haloalkyl, $C_{1-6}$ haloalkoxy and $C_{1-6}$ hydroxyalkyl;

each $R^{12}$ is independently selected from the group consisting of H atom, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $C_{3-8}$ cycloalkyl, wherein the $C_{3-8}$ cycloalkyl is optionally substituted by one or more substituents selected from the group consisting of $C_{1-6}$ alkyl, $C_{1-6}$ alkoxy, -OH, -COOH, - $NH_2$, -CN, halogen, $C_{1-6}$ haloalkyl, $C_{1-6}$ haloalkoxy and $C_{1-6}$ hydroxyalkyl;

or, $R^{11}$ and $R^{12}$ together with the N atom to which they are attached form 3 to 8 membered heterocyclyl, wherein the 3 to 8 membered heterocyclyl is optionally substituted by one or more substituents selected from the group consisting of $C_{1-6}$ alkyl, $C_{1-6}$ alkoxy, -OH, -COOH, $-NH_2$, -CN, halogen, $C_{1-6}$ haloalkyl, $C_{1-6}$ haloalkoxy and $C_{1-6}$ hydroxyalkyl;

$R^{13}$ is selected from the group consisting of 3 to 8 membered heterocyclyl, $C_{3-8}$ cycloalkyl, $C_{6-10}$ aryl and 5 to 10 membered heteroaryl, wherein the 3 to 8 membered heterocyclyl, $C_{3-8}$ cycloalkyl, $C_{6-10}$ aryl and 5 to 10 membered heteroaryl are each independently and optionally substituted by one or more substituents selected from the group consisting of $C_{1-6}$ alkoxy, -OH, -COOH, $-NR^{11}R^{12}$, -CN, halogen, $C_{1-6}$ haloalkyl, $C_{1-6}$ haloalkoxy and $C_{1-6}$ hydroxyalkyl;

each $R^{14}$ is independently selected from the group consisting of H atom, $C_{1-6}$ alkyl, $C_{1-6}$ alkoxy, $C_{1-6}$ haloalkoxy and $C_{3-8}$ cycloalkyl, wherein the $C_{1-6}$ alkyl and $C_{3-8}$ cycloalkyl are each independently and optionally substituted by one or more substituents selected from the group consisting of -CN, -OH, -COOH, $-NR^{11}R^{12}$, halogen and $C_{1-6}$ alkoxy;

m is 0, 1 or 2;

n is 0 or 1; and

p is 0, 1 or 2.

In some embodiments, the compound of formula (I) is a compound of formula (II),

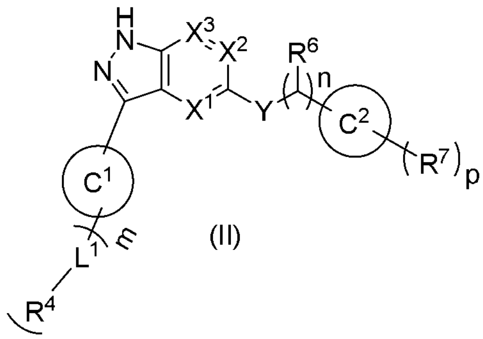

wherein, $X^1$, $X^2$, $X^3$, Y, $C^1$, $C^2$, $R^4$, $R^6$, $R^7$, $L^1$, m, n and p are as defined in formula (I).

In some embodiments, the compound of formula (I) is a compound of formula (II-1),

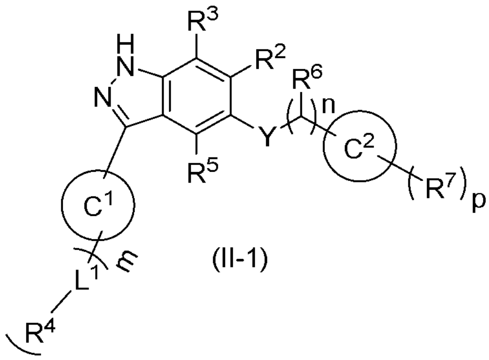

wherein,

Y is selected from the group consisting of O atom, S atom, -S(O)-, $-S(O)_2-$, NH and N-$C_{1-6}$ alkyl;
$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$;
$C^1$, $C^2$, $R^2$-$R^7$, $R^{11}$-$R^{13}$, $L^1$, m, n and p are as defined in formula (I);
provided that $R^3$ is not H atom when $C^2$ is 6 membered nitrogen-containing heteroaryl ring, preferably pyridyl, pyridazinyl or pyrimidinyl;
particularly,
$R^3$ is selected from the group consisting of H atom, $-N(C_{1-6}\ alkyl)_2$, $-NH_2$, $-NH(C_{1-6}$ alkyl), -CN, halogen, $C_{1-6}$ alkyl, $C_{1-6}$ alkoxy, $C_{1-6}$ haloalkyl, $C_{3-8}$ cycloalkyl, -O-$C_{3-8}$ cycloalkyl, -O-3 to 8 membered heterocyclyl and -O-$C_{6-10}$ aryl;
provided that $R^3$ is not H atom when $C^2$ is 6 membered nitrogen-containing heteroaryl ring, preferably pyridyl, pyridazinyl or pyrimidinyl;
more particularly,
$R^3$ is selected from the group consisting of H atom, F atom, $-CF_3$, methyl, Cl atom, methoxy, cyclopropyl, isopropyl, -CN, isopropoxy, furanyl-O-, ethoxy, cyclopropoxy, phenyl-O- and $-N(CH_3)_2$;
provided that $R^3$ is not H atom when $C^2$ is 6 membered nitrogen-containing heteroaryl ring, preferably pyridyl, pyridazinyl or pyrimidinyl.

In some embodiments, the compound of formula (I) is a compound of formula (II-2),

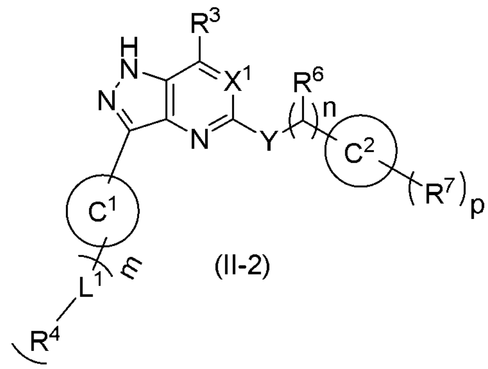

(II-2)

wherein,

Y is selected from the group consisting of O atom, S atom, -S(O)-, $-S(O)_2-$, NH and N-$C_{1-6}$ alkyl;
$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$;
$X^1$, $C^1$, $C^2$, $R^3$, $R^4$, $R^6$, $R^7$, $R^{11}$-$R^{13}$, $L^1$, m, n and p are as defined in formula (I);
particularly,
$R^3$ is selected from the group consisting of H atom, $C_{1-6}$ alkyl, $C_{1-6}$ alkoxy and $C_{3-8}$ cycloalkyl;
more particularly,
$R^3$ is selected from the group consisting of H atom, methyl, methoxy and cyclopropyl.

In some embodiments, the compound of formula (I) is a compound of formula (III),

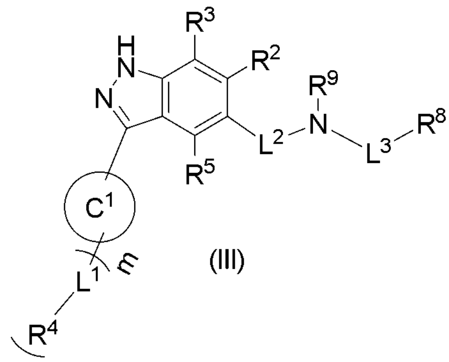

(III)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom or F atom;
$C^1$, $R^2$, $R^4$, $R^5$, $R^8$, $R^9$, $R^{11}$-$R^{13}$, $L^1$, $L^2$, $L^3$ and m are as defined in formula (I).

In some embodiments, the compound of formula (I) is a compound of formula (III-1),

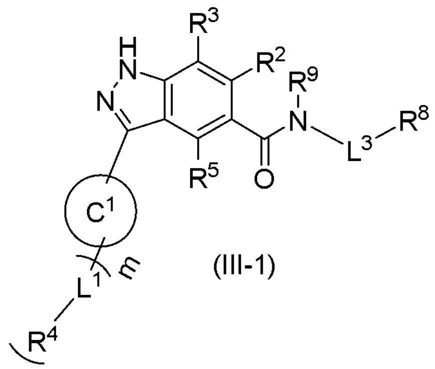

(III-1)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom or F atom;

$C^1$, $R^2$, $R^4$, $R^5$, $R^8$, $R^9$, $R^{11}$-$R^{13}$, $L^1$, $L^3$ and m are as defined in formula (I).

In some embodiments, the compound of formula (I) is a compound of formula (III-2),

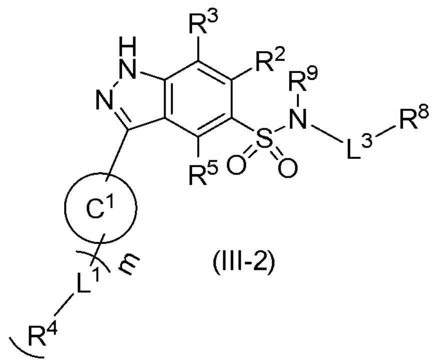

(III-2)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom or F atom;

$C^1$, $R^2$, $R^4$, $R^5$, $R^8$, $R^9$, $R^{11}$-$R^{13}$, $L^1$, $L^3$ and m are as defined in formula (I).

In some embodiments, the compound of formula (I) is a compound of formula (IV),

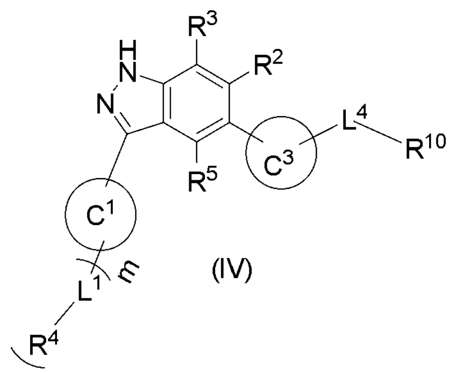

(IV)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom;

$C^1$, $C^3$, $R^2$, $R^4$, $R^5$, $R^{10}$, $R^{11}$-$R^{13}$, $L^1$, $L^4$ and m are as defined in formula (I),

particularly,

$C^3$ is selected from the group consisting of $C_{6-10}$ aryl, 5 to 10 membered heteroaryl, $C_{3-8}$ cycloalkyl, and 3 to 8 membered heterocyclyl; preferably triazolyl, tetrazolyl, pyridyl, pyrimidinyl, pyrazolyl, imidazolyl, thiazolyl, thienyl, furyl, pyranyl, pyrrolyl, pyrazinyl, pyridazinyl, piperidyl, piperazinyl, pyrrolidinyl, morpholinyl, tetrahydropyranyl, phenyl, naphthyl, cyclohexyl, cyclopentyl, cyclobutyl or cyclopropyl; more preferably 1,2,3-triazolyl; even more preferably

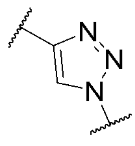;

and/or

$L^4$ is selected from the group consisting of chemical bond, $-C_{1-6}$ alkylene-, $-C_{1-6}$ alkylene-NH-, $-C_{1-6}$ alkylene-N($C_{1-6}$ alkyl)-, -NH-$C_{1-6}$ alkylene- and -N($C_{1-6}$ alkyl)-$C_{1-6}$ alkylene-; preferably chemical bond, $-CH_2-$, $-CH_2-CH_2-$, $-CH_2-CH_2-NH-$, $-CH_2-CH_2-N(CH_3)-$, $-NH-CH_2-CH_2-$ and $-CH_2-CH_2-N(CH_3)-$; and/or

$R^{10}$ is selected from the group consisting of H atom, -OH, -COOH, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy and $C_{1-6}$ hydroxyalkyl; preferably H atom and methyl.

In some embodiments, provided is the compound of formula (I), (II), (II-1), (II-2), (III), (III-1), (III-2) or (IV), wherein

ring $C^1$ is selected from the group consisting of 5 to 10 membered heteroaryl, $C_{3-8}$ cycloalkyl, 3 to 8 membered heterocyclyl, 5 to 10 membered heteroaryl fused with 3 to 8 membered heterocyclyl, 5 to 10 membered heteroaryl fused with $C_{6-10}$ aryl, 3 to 8 membered heterocyclyl fused with 5 to 10 membered heteroaryl, and $C_{6-10}$ aryl fused with 5 to 10 membered heteroaryl;

preferably, ring $C^1$ is selected from the group consisting of triazolyl, tetrazolyl, pyridyl, pyrimidinyl, pyrazolyl, imidazolyl, thiazolyl, thienyl, furyl, pyranyl, pyrrolyl, pyrazinyl, pyridazinyl, pyrazolo[1,5-a]pyridyl, tetrahydropyrazolo[1,5-a]pyridyl, tetrahydropyrrolo[1,2-b]pyrazolyl, tetrahydropyrrolo[3,4-d]imidazolyl, benzo[d]imidazolyl, pyrazolo[5,1-b][1,3]oxazinanyl, cyclopropyl and pyrrolidinyl;

more preferably, ring $C^1$ is selected from the group consisting of

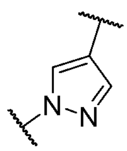,

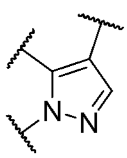, 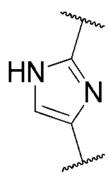, 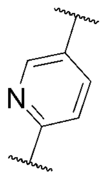, 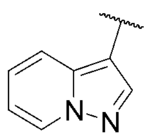, 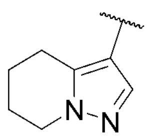, 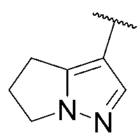, 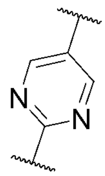,

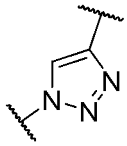, 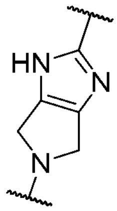, 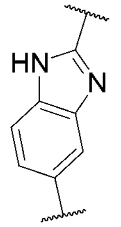, 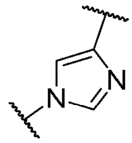, 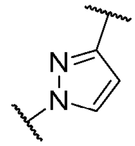, 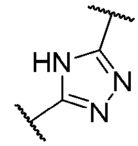, 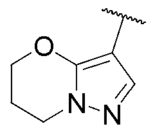,

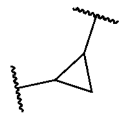 and 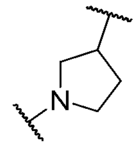.

In some embodiments, provided is the compound of formula (I), (II), (II-1), (II-2), (III), (III-1), (III-2) or (IV), wherein

each $L^1$ is independently selected from the group consisting of chemical bond, -$C_{1-6}$ alkylene-, -O-, -$C_{1-6}$ alkylene-O-, -O-$C_{1-6}$ alkylene-, -S-, -S(O)-, -$S(O)_2$-, -S(O)(=$NR^{11}$)-, -$C_{1-6}$ alkylene-C(O)-, -C(O)-$C_{1-6}$ alkylene-, -$C_{1-6}$ alkylene-$S(O)_2$-, -$S(O)_2$-$C_{1-6}$ alkylene-, 3 to 8 membered heterocyclylene, -$C_{1-6}$ alkylene-S(O)(=$NR^{11}$)-, -S(O)(=$NR^{11}$)-$C_{1-6}$ alkylene-, $C_{3-8}$ cycloalkylene, $C_{6-10}$ arylene, 5 to 10 membered heteroarylene, -$C_{1-6}$ alkylene-O-$C_{1-6}$ alkylene-O-, -O-$C_{1-6}$ alkylene-O-$C_{1-6}$ alkylene-, -$NR^{11}$-, -$C_{1-6}$ alkylene-$NR^{11}$-, -$NR^{11}$-$C_{1-6}$ alkylene-, -$C_{1-6}$ alkylene-$NR^{11}$-$C_{1-6}$ alkylene-O-, -O-$C_{1-6}$ alkylene-$NR^{11}$-$C_{1-6}$ alkylene-, -$NR^{11}$-C(O)-, -C(O)-$NR^{11}$-, -$C_{1-6}$ alkylene-C(O)-$NR^{11}$- and -$NR^{11}$-C(O)-$C_{1-6}$ alkylene-, wherein the $C_{1-6}$ alkylene is each independently and optionally substituted by one or more substituents selected from the group consisting of $C_{1-6}$ alkyl, halogen, -OH, -COOH, -$NH_2$, -CN, $C_{1-6}$ alkoxy, $C_{1-6}$ haloalkyl, $C_{1-6}$ haloalkoxy and $C_{1-6}$ hydroxyalkyl; each $R^{11}$ is independently H atom or $C_{1-6}$ alkyl;

preferably, each $L^1$ is independently selected from the group consisting of chemical bond, -$C_{1-6}$ alkylene-, -$C_{1-6}$ alkylene-O-, -O-$C_{1-6}$ alkylene-, -$S(O)_2$-, -S(O)(=$NR^{11}$)-, -$C_{1-6}$ alkylene-C(O)-, -C(O)-$C_{1-6}$ alkylene-, -$C_{1-6}$ alkylene-$S(O)_2$-, -$S(O)_2$-$C_{1-6}$ alkylene-, -$C_{1-6}$ alkylene-S(O)(=$NR^{11}$)-, -S(O)(=$NR^{11}$)-$C_{1-6}$ alkylene-, 3 to 8 membered heterocyclylene, $C_{3-8}$ cycloalkylene, $C_{6-10}$ arylene, 5 to 10 membered heteroarylene, -$C_{1-6}$ alkylene-O-$C_{1-6}$ alkylene-O-, -O-$C_{1-6}$ alkylene-O-$C_{1-6}$ alkylene-, -$C_{1-6}$ alkylene-$NR^{11}$-, -$NR^{11}$-$C_{1-6}$ alkylene-, -$C_{1-6}$ alkylene-$NR^{11}$-$C_{1-6}$ alkylene-O-, -O-$C_{1-6}$ alkylene-$NR^{11}$-$C_{1-6}$ alkylene-, - $C_{1-6}$ alkylene-C(O)-$NR^{11}$- and -$NR^{11}$-C(O)-$C_{1-6}$ alkylene-, wherein the $C_{1-6}$ alkylene is each independently and optionally substituted by one or more substituents selected from the group consisting of $C_{1-6}$ alkyl, halogen and -OH; each $R^{11}$ is independently H atom or $C_{1-6}$ alkyl;

more preferably, each $L^1$ is independently selected from the group consisting of chemical bond, -$CH_2$-$CH_2$-, -$CH_2$-, -$CH_2$-$CH_2$-$CH_2$-, -$S(O)_2$-, -S(O)(=NH)-, -$S(O)_2$-$CH_2$-$CH_2$-, -$NCH_3$-$CH_2$-$CH_2$-, -NH-C(O)-$CH_2$-, -$NCH_3$-$CH_2$-$CH_2$-$CH_2$-, -C(O)-$CH_2$-, -O-$CH_2$-$CH_2$-$NCH_3$-$CH_2$-$CH_2$-, -O-$CH_2$-$CH_2$-O-$CH_2$-$CH_2$-, -$C(CH_3)_2$-$CH_2$-, -$C(CH_3)$(OH)-$CH_2$-, -O-$CH_2$-$CH_2$-, -CH(F)-$CH_2$-, -$CH(CH_3)$-$CH_2$-, azetidinyl, -$CH_2$-$CH_2$-$S(O)_2$-, -$CH_2$-$CH_2$-$NCH_3$-, -$CH_2$-C(O)-NH-, -$CH_2$-$CH_2$-$CH_2$-$NCH_3$-, -$CH_2$-C(O)-, -$CH_2$-$CH_2$-$NCH_3$-$CH_2$-$CH_2$-O-, -$CH_2$-$CH_2$-O-$CH_2$-$CH_2$-O-, -$CH_2$-$C(CH_3)_2$-, -$CH_2$-$C(CH_3)$(OH)-, -$CH_2$-$CH_2$-O-, -$CH_2$-CH(F)-, -$CH_2$-$CH(CH_3)$-, -$CH_2$-$CH_2$-$CH_2$-S(O)(=NH)- and -S(O)(=NH) - $CH_2$-$CH_2$-$CH_2$-.

In some embodiments, provided is the compound of formula (I), (II), (II-1), (II-2), (III), (III-1), (III-2) or (IV), wherein

each $R^4$ is independently selected from the group consisting of H atom, -OH, - COOH, -CN, halogen, $C_{1-6}$ alkyl, $C_{1-6}$ alkoxy, $C_{1-6}$ haloalkyl, $C_{1-6}$ haloalkoxy, $C_{1-6}$ hydroxyalkyl, -C(O)-$NR^{11}R^{12}$, -$NR^{11}R^{12}$, $C_{6-10}$ aryl, 5 to 10 membered heteroaryl, $C_{3-8}$ cycloalkyl, 3 to 8 membered monocyclic heterocyclyl, 7 to 10 membered spiro heterocyclyl and 7 to 10 membered bridged heterocyclyl, wherein the $C_{6-10}$ aryl, 5 to 10 membered heteroaryl, $C_{3-8}$ cycloalkyl and 3 to 8 membered monocyclic heterocyclyl are each independently and optionally substituted by one or more substituents selected from the group consisting of -CN, -OH, $C_{1-6}$ alkyl, $C_{1-6}$ hydroxyalkyl, -C(O)-$R^{14}$, -C(O)-$NR^{11}R^{12}$, oxo, $C_{1-6}$ alkoxy, -$S(O)_2$-$C_{1-6}$ alkyl, -$NR^{11}R^{12}$, imino, halogen, -$C_{1-6}$ alkylene-$NR^{11}R^{12}$, -$NR^{11}$-C(O)-$R^{14}$, -$NR^{11}$-$S(O)_2$-$R^{14}$, -S(O)(=$NR^{11}$)-$R^{14}$, -$S(O)_2$-$R^{14}$, -C(=$NR^{11}$)-$NR^{11}R^{12}$, -$S(O)_2$-$NR^{11}R^{12}$ and -$C_{1-6}$ alkylene-C(O)-$NR^{11}R^{12}$; each $R^{11}$ is independently H atom, $C_{1-6}$ alkyl or $C_{3-8}$ cycloalkyl; each $R^{12}$ is independently H atom, $C_{1-6}$ alkyl or $C_{3-8}$ cycloalkyl; each $R^{14}$ is independently selected from the group consisting of H atom, $C_{1-6}$ alkyl and $C_{3-8}$ cycloalkyl, wherein the $C_{1-6}$ alkyl and $C_{3-8}$ cycloalkyl are each independently and optionally substituted by one or more substituents selected from the group consisting of -CN, -OH, -$NR^{11}R^{12}$ and $C_{1-6}$ alkoxy;

preferably, each $R^4$ is independently selected from the group consisting of H atom, - OH, -COOH, -CN, halogen, $C_{1-6}$ alkyl, $C_{1-6}$ hydroxyalkyl, -C(O)-$NR^{11}R^{12}$, -$NR^{11}R^{12}$, phenyl, pyridyl, imidazolyl, pyrazolyl, cyclopropyl, oxetanyl,

azetidinyl, azolidinyl, morpholinyl, piperidyl, tetrahydropyranyl, piperazinyl, 1,1-dioxo-thiomorpholinyl, 1-oxo-1-imino-thiomorpholinyl, thiomorpholinyl, pyrrolidinyl, diazaspiro[4.5]decanyl, diazabicyclo[2.2.1]heptanyl and diazabicyclo[3.2.1]octyl, wherein the phenyl, pyridyl, imidazolyl, pyrazolyl, cyclopropyl, oxetanyl, azetidinyl, azolidinyl, morpholinyl, piperidyl, tetrahydropyranyl, piperazinyl, 1,1-dioxo-thiomorpholinyl, 1-oxo-1-imino-thiomorpholinyl, thiomorpholinyl, pyrrolidinyl, diazaspiro[4.5]decanyl, diazabicyclo[2.2.1]heptanyl and diazabicyclo[3.2.1]octyl are each independently and optionally substituted by one or more substituents selected from the group consisting of - CN, -OH, $C_{1-6}$ alkyl, $C_{1-6}$ hydroxyalkyl, $-C(O)-R^{14}$, $-C(O)-NR^{11}R^{12}$, oxo, $C_{1-6}$ alkoxy, - $S(O)_2$-$C_{1-6}$ alkyl, $-NR^{11}R^{12}$, imino, $-C_{1-6}$ alkylene-$NR^{11}R^{12}$, $-NR^{11}-C(O)-R^{14}$, $-NR^{11}-S(O)_2-R^{14}$, $-S(O)(=NR^{11})-R^{14}$, $-S(O)_2-R^{14}$, $-C(=NR^{11})-NR^{11}R^{12}$, $-S(O)_2-NR^{11}R^{12}$ and $-C_{1-6}$ alkylene-$C(O)-NR^{11}R^{12}$; $R^{11}$ is H atom, $C_{1-6}$ alkyl or $C_{3-6}$ cycloalkyl; $R^{12}$ is H atom, $C_{1-6}$ alkyl or $C_{3-6}$ cycloalkyl; each $R^{14}$ is independently selected from the group consisting of H atom, $C_{1-6}$ alkyl and $C_{3-6}$ cycloalkyl, wherein the $C_{1-6}$ alkyl and $C_{3-6}$ cycloalkyl are each independently and optionally substituted by one or more substituents selected from the group consisting of -CN, -OH, $-NR^{11}R^{12}$ and $C_{1-6}$ alkoxy;

more preferably, each $R^4$ is independently selected from the group consisting of H atom, methyl,

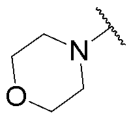,

propyl,

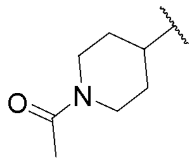,

cyclopropyl,

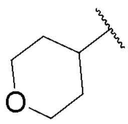, 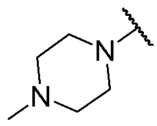

, 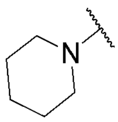, 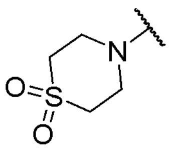, 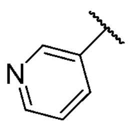, 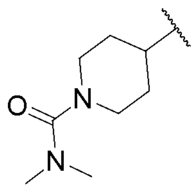, 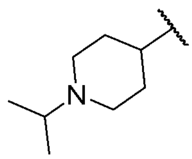,

-OH, F atom, CN,

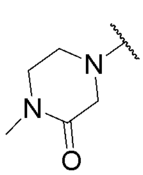, 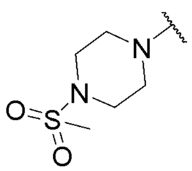, 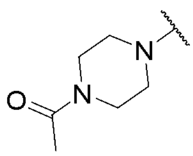, 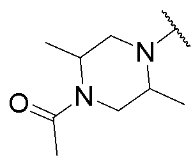, 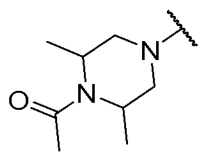,

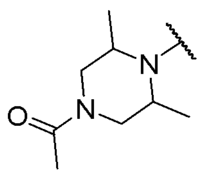, 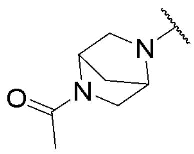, 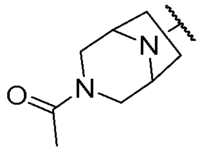, 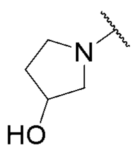, 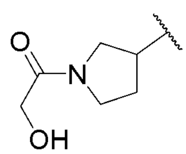,

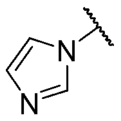, 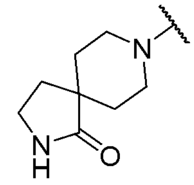, 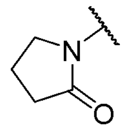,

phenyl,

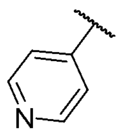, 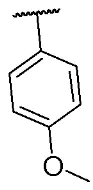, 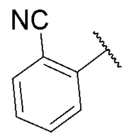,

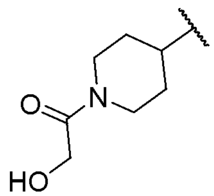, 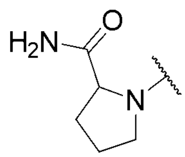, 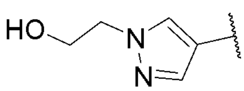, 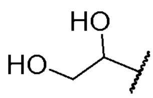, 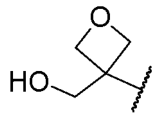,

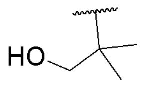, 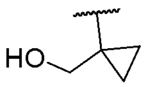, 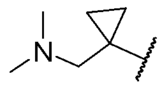,

$-COOH$, $-C(O)-NH_2$, $-N(CH_3)_2$,

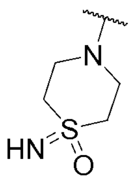,

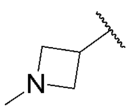, 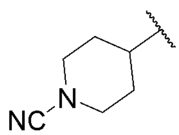, 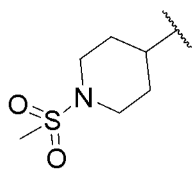, 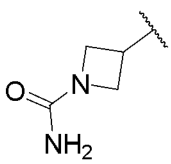, 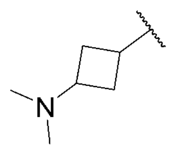,

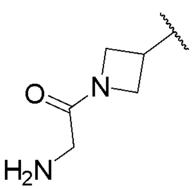, 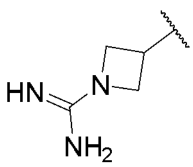, 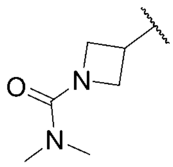, 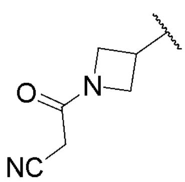, 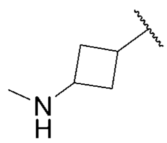,

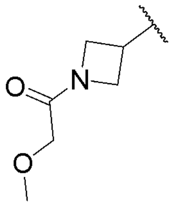, 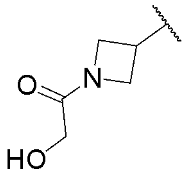, 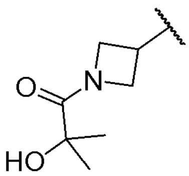, 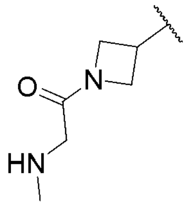, 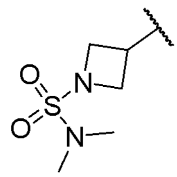,

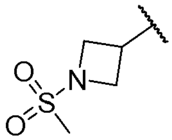, 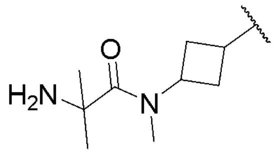, 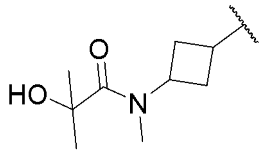, 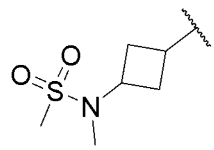,

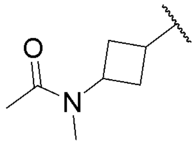, 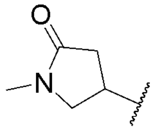, 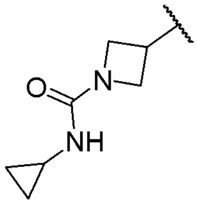, 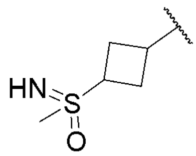, 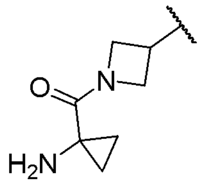,

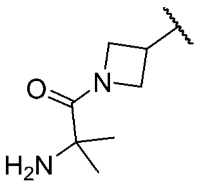, 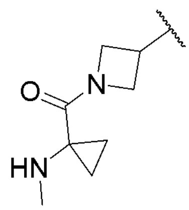, 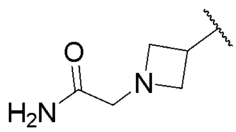, 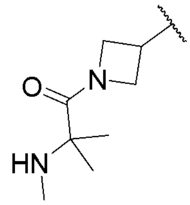, 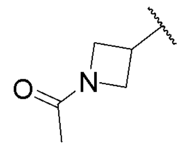,

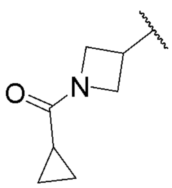, 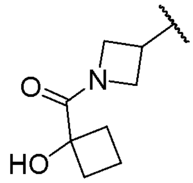, 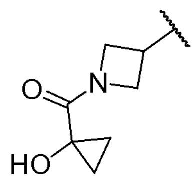, 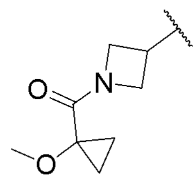, 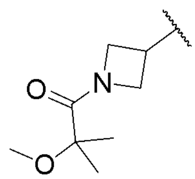,

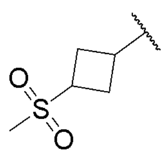, 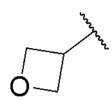 and 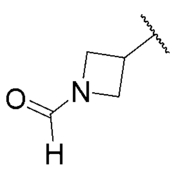.

In some embodiments, provided is the compound of formula (I), (II), (II-1), (II-2), (III), (III-1), (III-2) or (IV), wherein

$R^2$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, $C_{1-6}$ alkoxy-$C_{6-10}$ aryl-, 5 to 10 membered heteroaryl, $C_{3-8}$ cycloalkyl and 3 to 8 membered heterocyclyl;
preferably, $R^2$ is selected from the group consisting of H atom, halogen, $C_{1-6}$ alkoxy, $C_{6-10}$ aryl and $C_{1-6}$ alkoxy-$C_{6-10}$ aryl-;
more preferably, $R^2$ is selected from the group consisting of H atom,

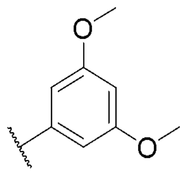

, methoxy and F atom.

In some embodiments, provided is the compound of formula (I), (II), (II-1), (III), (III-1), (III-2) or (IV), wherein

$R^5$ is selected from the group consisting of H atom, -OH, -COOH, $-NH_2$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;
preferably, $R^5$ is selected from the group consisting of H atom, -OH, -COOH, $-NH_2$, -CN, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl;
more preferably, $R^5$ is H atom.

In some embodiments, provided is the compound of formula (I), (II), (II-1) or (II-2), wherein

ring $C^2$ is selected from the group consisting of 5 to 10 membered heteroaryl, 3 to 8 membered heterocyclyl, $C_{3-8}$ cycloalkyl fused with 5 to 10 membered heteroaryl, 5 to 10 membered heteroaryl fused with $C_{6-10}$ aryl, $C_{6-10}$ aryl fused with 3 to 8 membered heterocyclyl, 5 to 10 membered heteroaryl fused with $C_{3-8}$ cycloalkyl, $C_{6-10}$ aryl fused with 5 to 10 membered hereroaryl, and 3 to 8 membered heterocyclyl fused with $C_{6-10}$ aryl;
preferably, ring $C^2$ is selected from the group consisting of triazolyl, tetrazolyl, pyridyl, pyrimidinyl, pyrazolyl, imidazolyl, thiazolyl, thienyl, furyl, pyranyl, pyrrolyl, pyrazinyl, pyridazinyl, piperidyl, piperazinyl, pyrrolidinyl, morpholinyl, tetrahydropyranyl, tetrahydroisoquinolyl, tetrahydroquinolyl, imidazo[1,2-a]pyridyl, quinolyl, isoquinolyl, naphthyridinyl, quinoxalinyl, quinazolinyl, triazolopyridyl, benzodioxanyl, benzoimidazolyl, cinnolinyl, benzooxazolyl, benzothiazolyl, indolinyl, benzofuryl, thienopyridyl, pyrazolopyridyl and pyridinopyrazinyl;
more preferably, ring $C^2$ is selected from the group consisting of

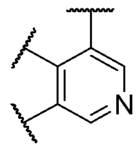,

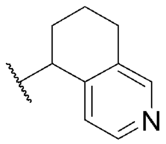, 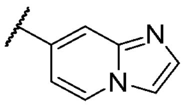, 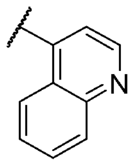, 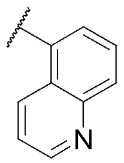, 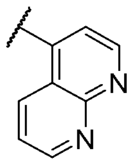, 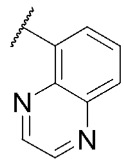,

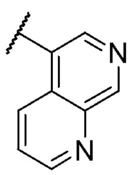, 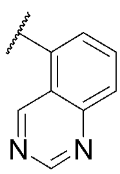, 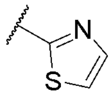, 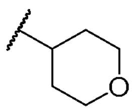, 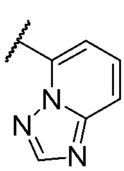, 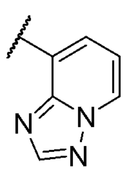, 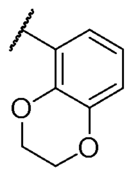,

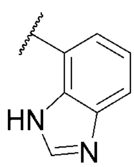, 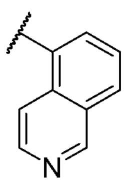, 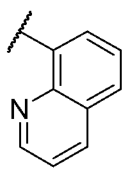, 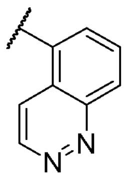, 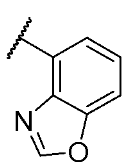, 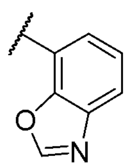, 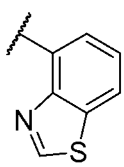,

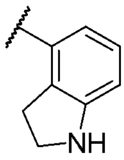 , 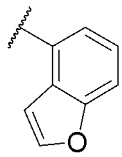 , 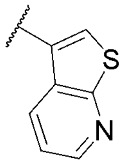 , 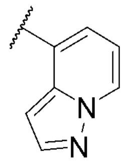 and 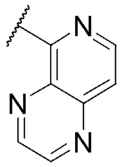 ;

and/or

$R^6$ is selected from the group consisting of H atom, -OH, -COOH, $-NR^{11}R^{12}$, -CN, halogen, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $-C_{1-6}$ alkylene-$NR^{11}R^{12}$; each $R^{11}$ is independently H atom or $C_{1-6}$ alkyl; each $R^{12}$ is independently H atom or $C_{1-6}$ alkyl;

preferably, $R^6$ is selected from the group consisting of $C_{1-6}$ alkyl, $C_{1-6}$ deuterated alkyl, $C_{1-6}$ hydroxyalkyl, $-C_{1-6}$ alkylene-NH($C_{1-6}$ alkyl), -CN and $C_{1-6}$ haloalkyl;

more preferably, $R^6$ is selected from the group consisting of methyl, ethyl, hydroxymethyl,

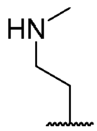 ,

-CN, trifluoromethyl and trideuteriomethyl; and/or

$R^7$ is selected from the group consisting of H atom, -OH, -COOH, $-NH_2$, -NH($C_{1-6}$ alkyl), -N($C_{1-6}$ alkyl)$_2$, -CN, halogen, oxo, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $C_{3-8}$ cycloalkyl;

preferably, $R^7$ is selected from the group consisting of H atom, $-NH_2$, -N($C_{1-6}$ alkyl)$_2$, -CN, halogen, oxo, $C_{1-6}$ alkyl, $C_{1-6}$ deuterated alkyl, $C_{1-6}$ alkoxy, $C_{1-6}$ hydroxyalkyl, $C_{3-6}$ cycloalkyl and $C_{1-6}$ haloalkyl;

more preferably, $R^7$ is selected from the group consisting of H atom, $-NH_2$, $-N(CH_3)_2$, -CN, Cl atom, F atom, Br atom, oxo, methyl, ethyl, isopropyl, methoxy, cyclopropyl, difluoromethyl, monofluoromethyl, trifluoromethyl, hydroxymethyl and trideuteriomethyl.

In some embodiments, provided is the compound of formula (I), (III), (III-1) or (III-2), wherein

$L^3$ is selected from the group consisting of chemical bond, $-C_{1-6}$ alkylene-, -O-, $-C_{1-6}$ alkylene-O-, $-O-C_{1-6}$ alkylene-, -S-, -S(O)-, $-S(O)_2-$, $-C_{1-6}$ alkylene-C(O)-, $-C(O)-C_{1-6}$ alkylene-, $-C_{1-6}$ alkylene-$S(O)_2$-, $-S(O)_2-C_{1-6}$ alkylene-, $-C_{1-6}$ alkylene-$O-C_{1-6}$ alkylene-O-, $-O-C_{1-6}$ alkylene-$O-C_{1-6}$ alkylene-, $-NR^{11}-$, $-NR^{11}-C_{1-6}$ alkylene-, $-C_{1-6}$ alkylene-$NR^{11}$-, $-C_{1-6}$ alkylene-$NR^{11}-C_{1-6}$ alkylene-O-, $-O-C_{1-6}$ alkylene-$NR^{11}-C_{1-6}$ alkylene-, $-NR^{11}$-C(O)-, $-C(O)-NR^{11}-$, $-C_{1-6}$ alkylene-C(O)-$NR^{11}$- and $-NR^{11}-C(O)-C_{1-6}$ alkylene-, wherein the $C_{1-6}$ alkylene is each independently and optionally substituted by one or more substituents selected from the group consisting of $C_{1-6}$ alkyl, halogen, -OH, -COOH, $-NH_2$, -CN, $C_{1-6}$ alkoxy, $C_{1-6}$ haloalkyl, $C_{1-6}$ haloalkoxy, and $C_{1-6}$ hydroxyalkyl; each $R^{11}$ is independently H atom or $C_{1-6}$ alkyl;

preferably, $L^3$ is selected from the group consisting of chemical bond, $-C_{1-6}$ alkylene-, $-C_{1-6}$ alkylene-NH-, $-C_{1-6}$ alkylene-N($C_{1-6}$ alkyl)-, $-NH-C_{1-6}$ alkylene- and -N($C_{1-6}$ alkyl)-$C_{1-6}$ alkylene-; wherein the $C_{1-6}$ alkylene is each independently and optionally substituted by one or more $C_{1-6}$ alkyl or -OH groups;

preferably, $L^3$ is selected from the group consisting of chemical bond, $-CH_2-CH_2-NCH_3-$, $-CH_2-CH_2-NH-$, $-CH_2-C(CH_3)(OH)-$, $-CH_2-C(CH_3)_2-$, $-NCH_3-CH_2-CH_2-$, $-NH-CH_2-CH_2-$, $-C(CH_3)(OH)-CH_2-$ and $-C(CH_3)_2-CH_2-$; and/or

$R^3$ is selected from the group consisting of H atom, -OH, -COOH, -CN, halogen, $C_{1-6}$ alkyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of -CN, -OH, $C_{1-6}$ alkyl, $-C(O)-C_{1-6}$ alkyl, $-C(O)-C_{1-6}$ hydroxyalkyl, $C_{1-6}$ alkoxy, $-NH_2$ and halogen;

preferably, $R^8$ is selected from the group consisting of H atom, -OH, $C_{6-10}$ aryl, 5 to 10 membered heteroaryl and 3 to 8 membered heterocyclyl, wherein the $C_{6-10}$ aryl and 5 to 10 membered heteroaryl are each independently and optionally substituted by one or more $C_{1-6}$ alkyl or halogen groups;

more preferably, $R^8$ is selected from the group consisting of H, methyl, -OH,

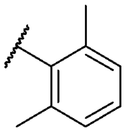, 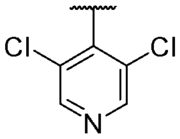, 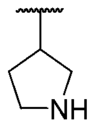 and 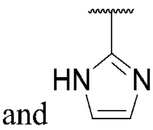;

and/or

$R^9$ is selected from the group consisting of H atom, $C_{1-6}$ alkyl, $C_{1-6}$ alkoxy, $C_{1-6}$ haloalkyl and $C_{1-6}$ hydroxyalkyl; preferably, $R^9$ is H atom or $C_{1-6}$ alkyl;

more preferably, $R^9$ is H atom or methyl.

The representative compound of formula (I), (II), (II-1), (II-2), (III), (III-1), (III-2) or (IV) of the present invention includes, but is not limited to the following compounds:

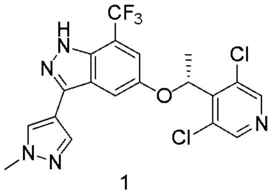
1
,
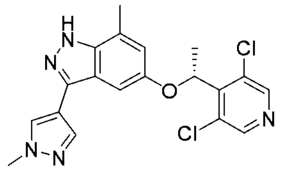
2
,
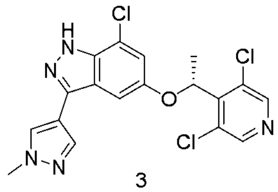
3
,
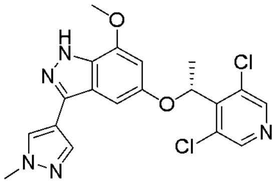
4
,
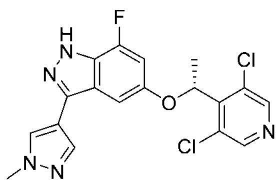
5
,
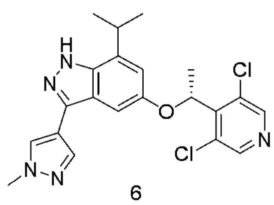
6
,
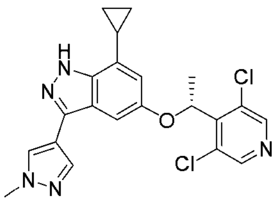
7
,
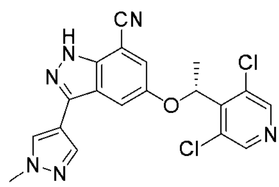
8
,
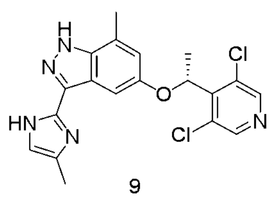
9
,
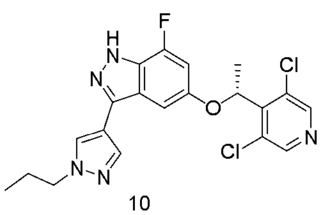
10
,
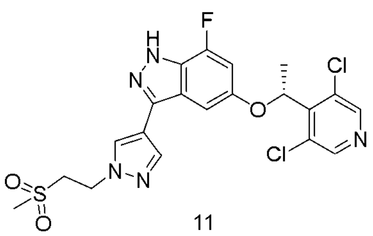
11
,
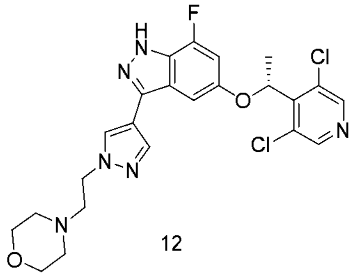
12
,

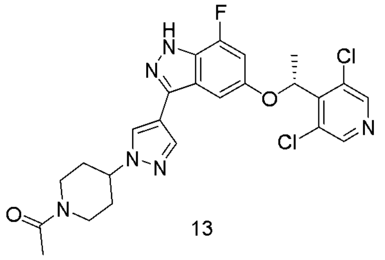

13,

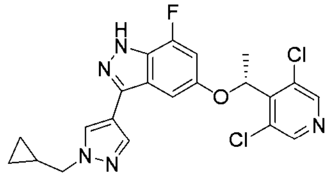

14,

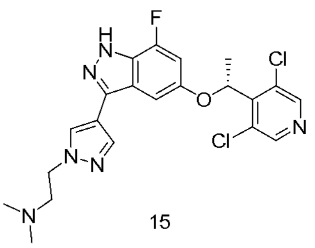

15,

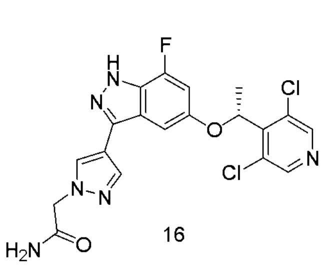

16,

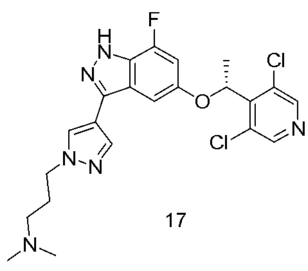

17,

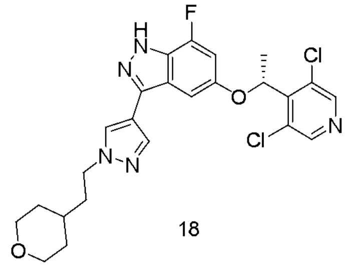

18,

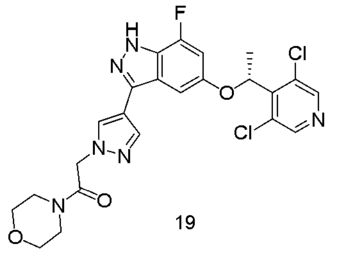

19,

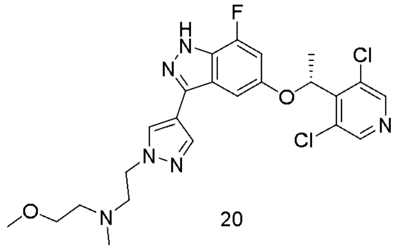

20,

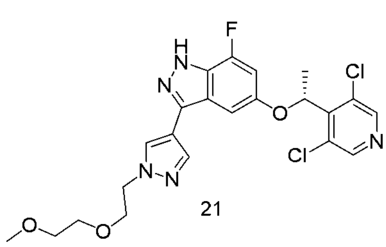

21,

–

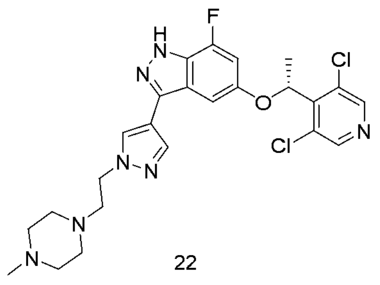

22,

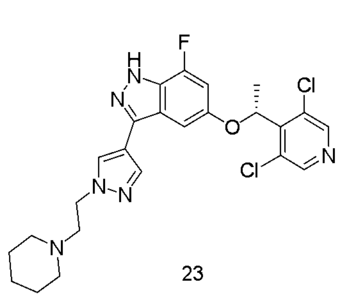

23,

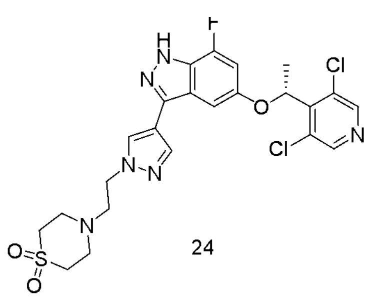

24,

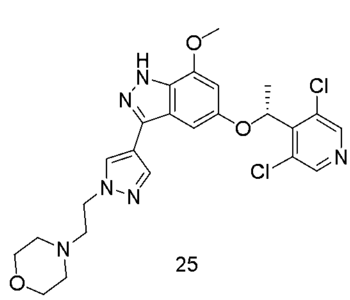

25,

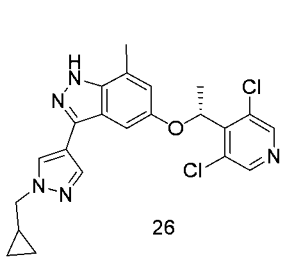
26 ,
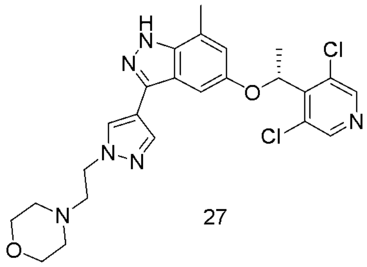
27 ,
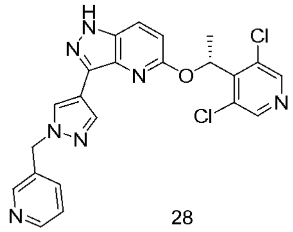
28 ,
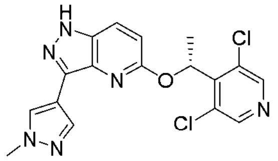
29 ,
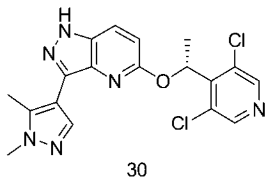
30 ,
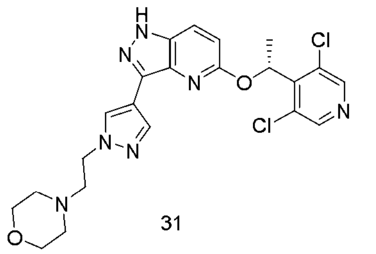
31 ,
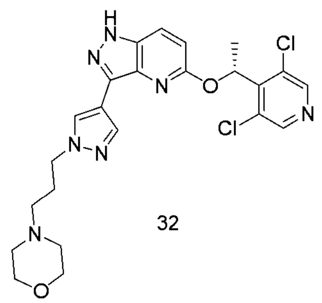
32 ,
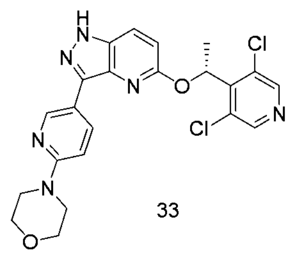
33 ,
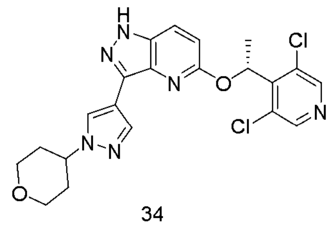
34 ,
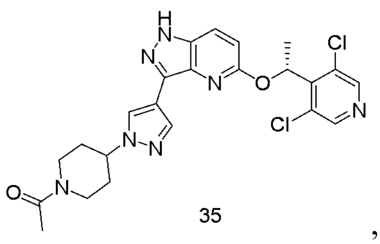
35 ,
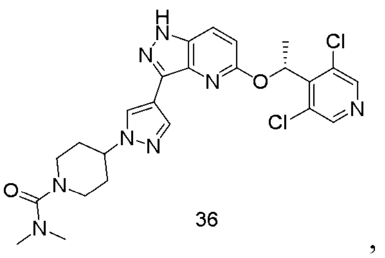
36 ,
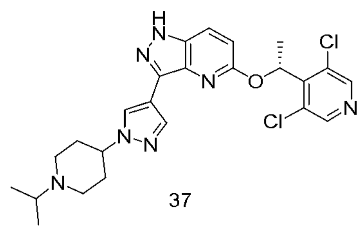
37
, 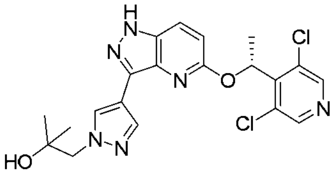
38 ,
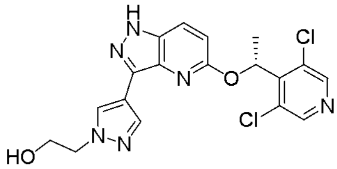
39 ,
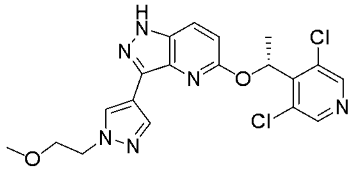
40 ,
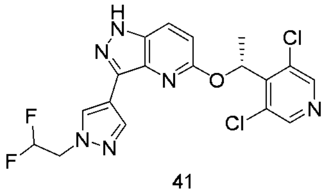
41 ,
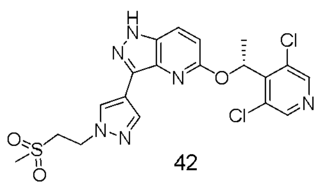
42 ,
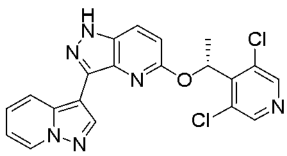
43 ,

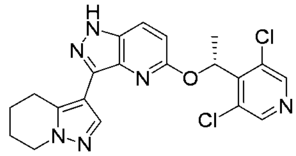
44
,
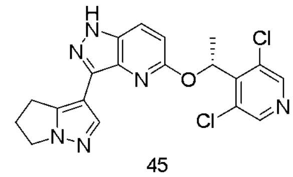
45
,
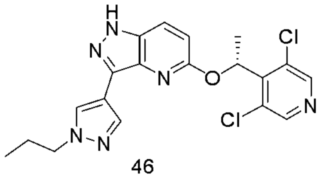
46
,

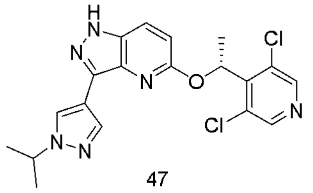
47
,
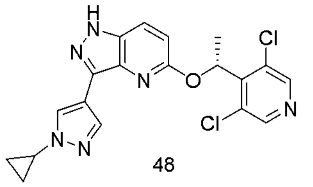
48
,
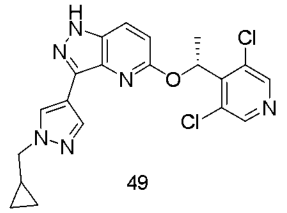
49
,

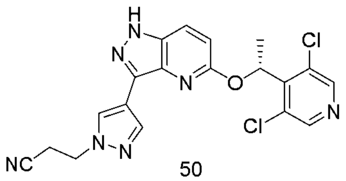
50
,
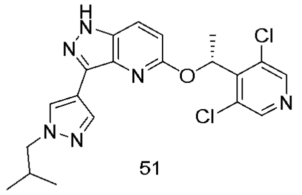
51
,
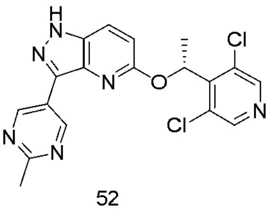
52
,

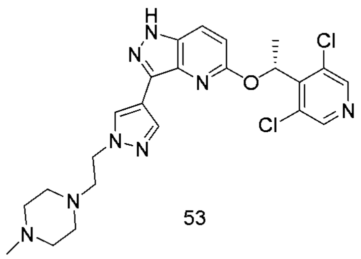
53
,
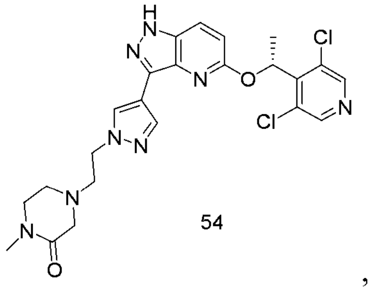
54
,
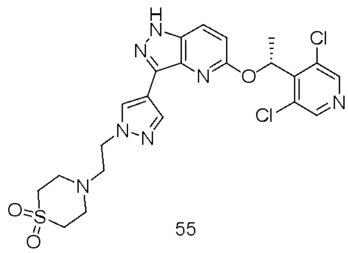
55
,

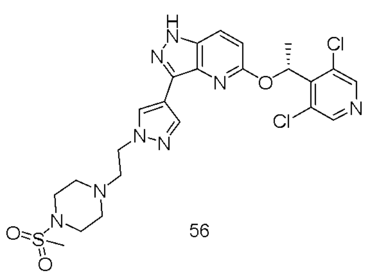
56
,
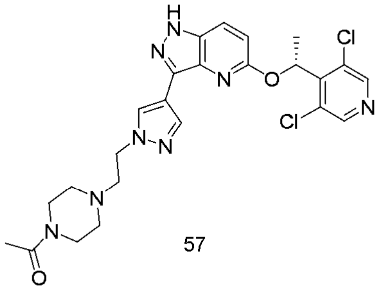
57
,
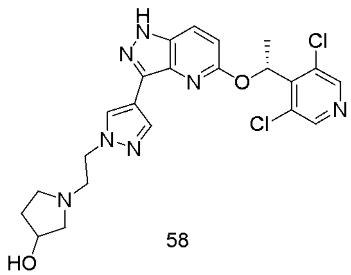
58

,
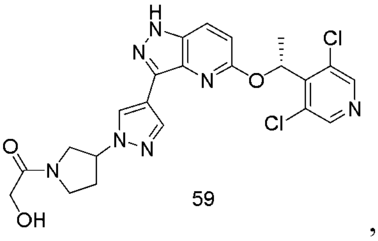
59
,
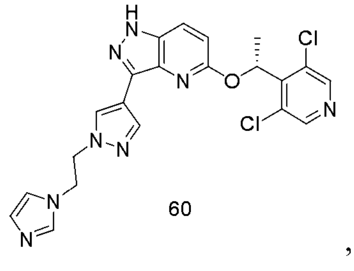
60
,
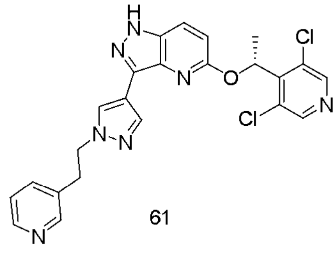
61
,

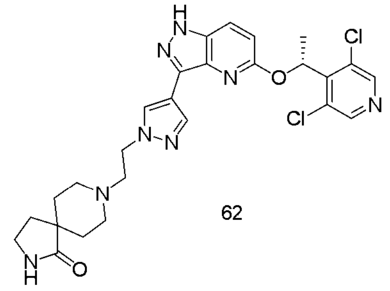 62, 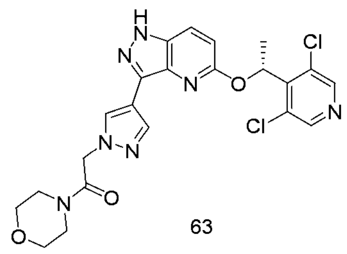 63, 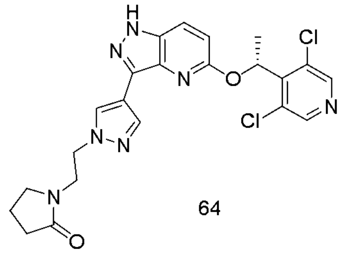 64,

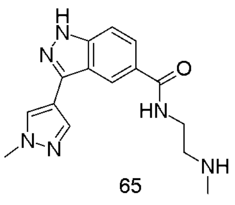 65, 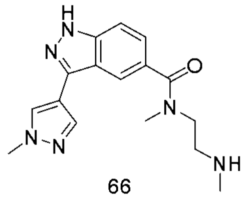 66, 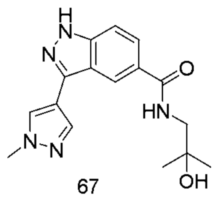 67, 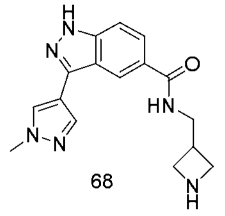 68,

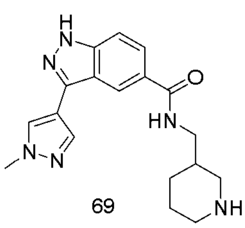 69, 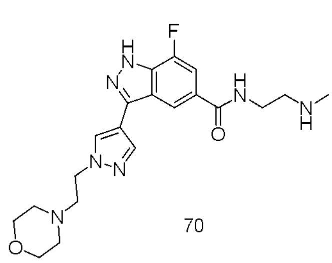 70, 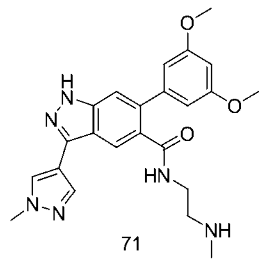 71,

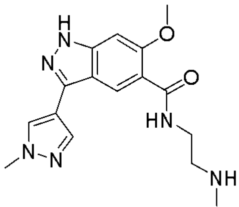 72, 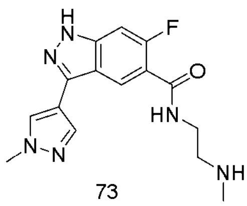 73, 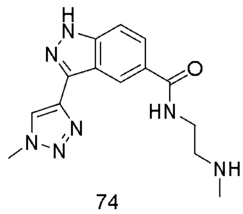 74, 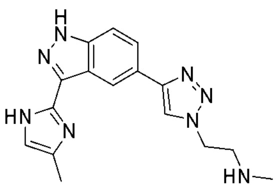 75,

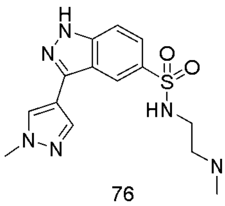 76, 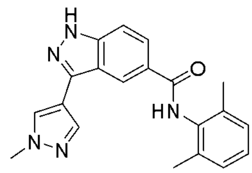 77, 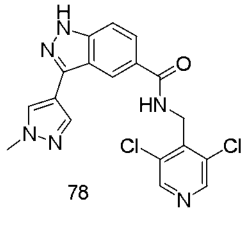 78, 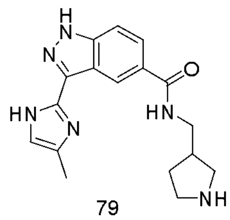 79,

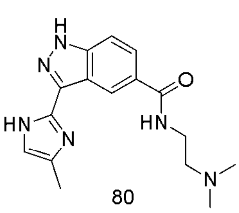 80, 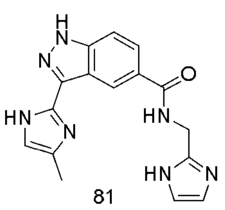 81, 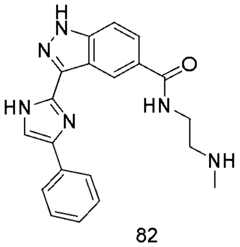 82, 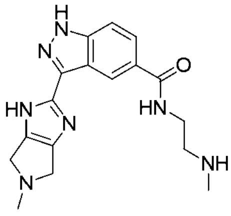 83,

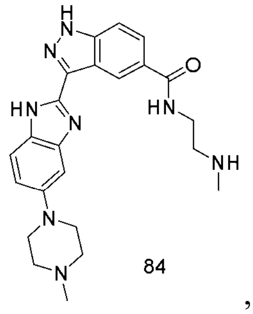
84 ,

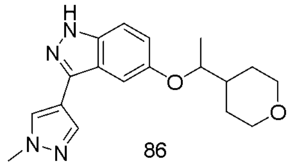
86 ,

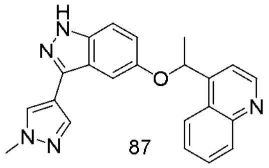
87 ,

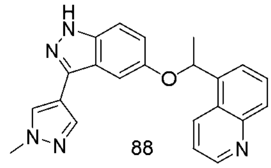
88 ,

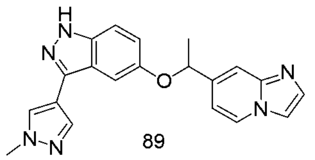
89 ,

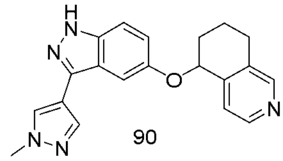
90 ,

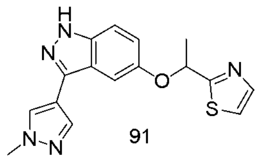
91 ,

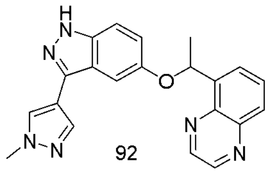
92 ,

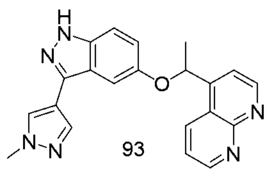
93 ,

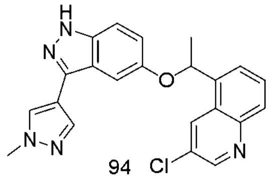
94 ,

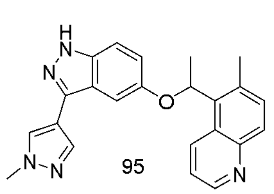
95 ,

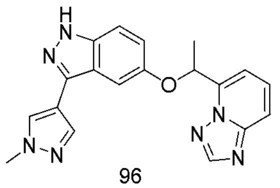
96 ,

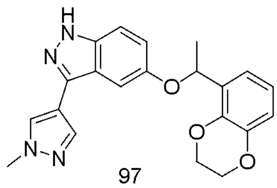
97 ,

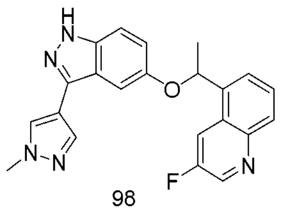
98 ,

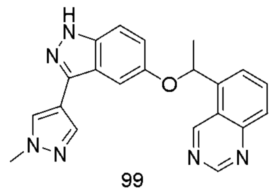
99 ,

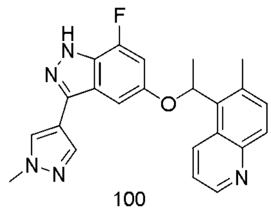
100 ,

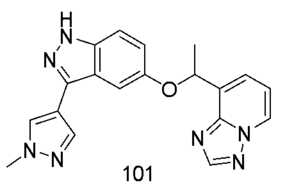
101 ,

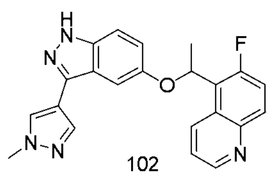
102 ,

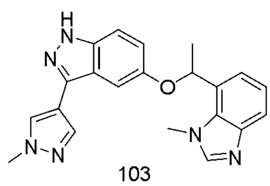
103 ,

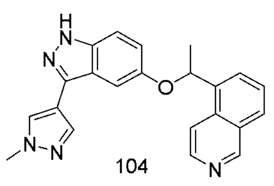
104 ,

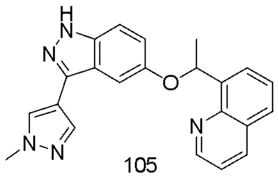
105 ,

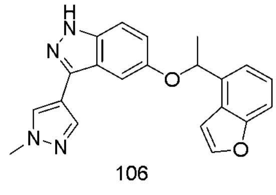
106 ,

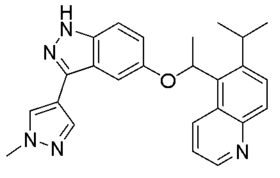
107
,
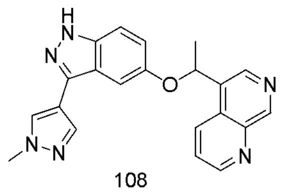
108
,
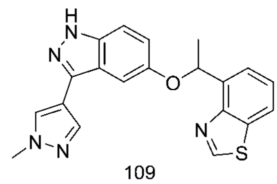
109
,

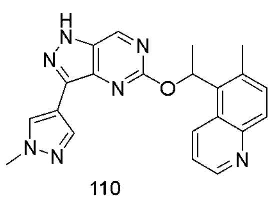
110
,
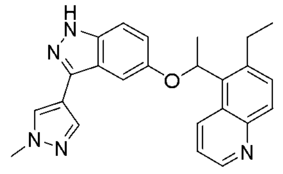
111
,
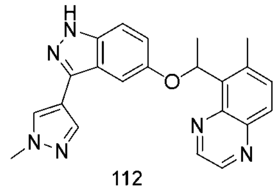
112
,

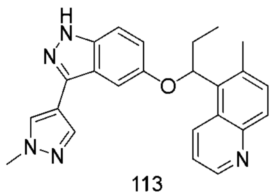
113
,
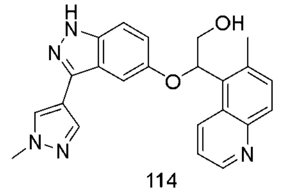
114
,
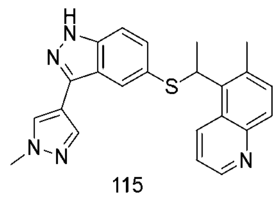
115
,

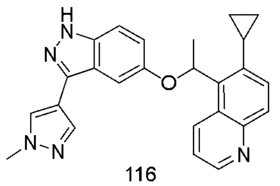
116
,
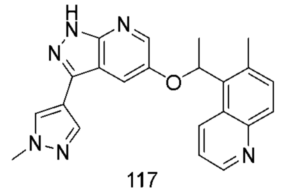
117
,
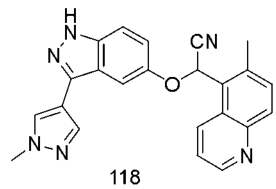
118
,

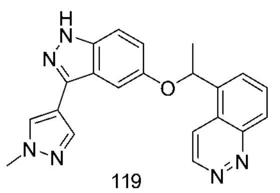
119
,
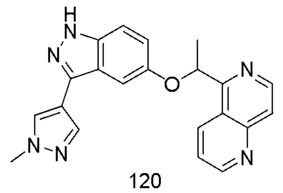
120
,
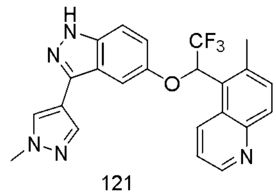
121
,

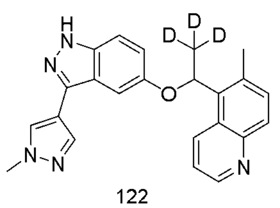
122
,
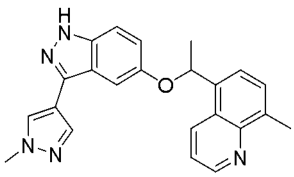
123
,
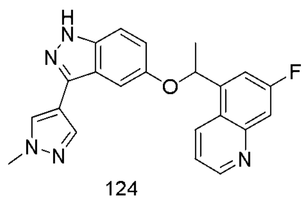
124
,

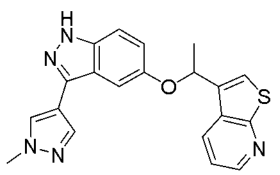
125
,
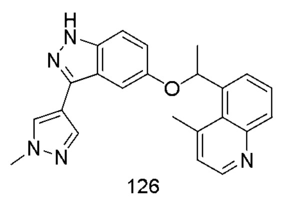
126
,
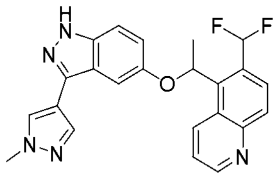
127
,

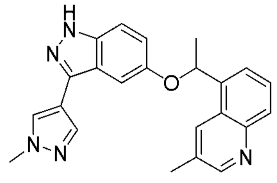
128
,

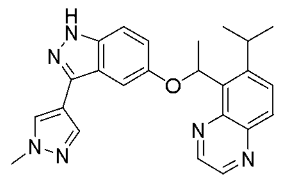
129
,

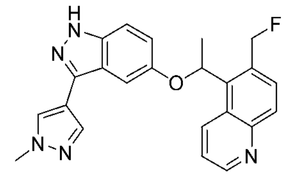
130
,

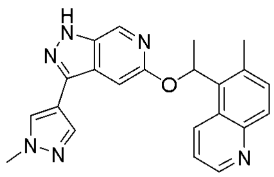
131
,

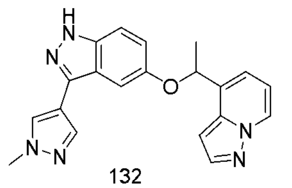
132
,

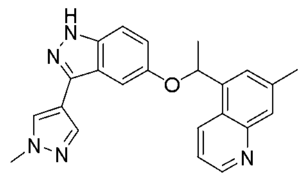
133
,

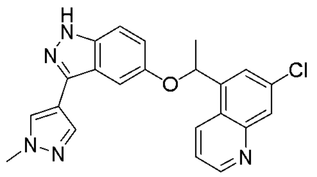
134
,

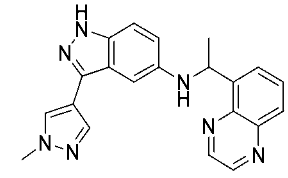
135
,

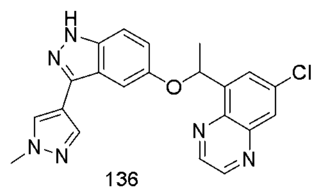
136
,

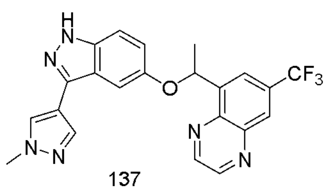
137
,

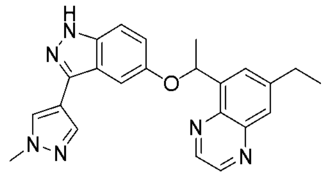
138
,

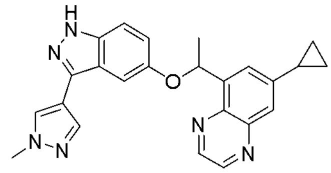
139
,

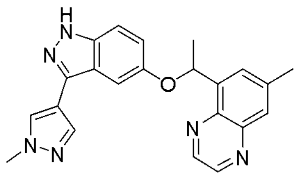
140
,

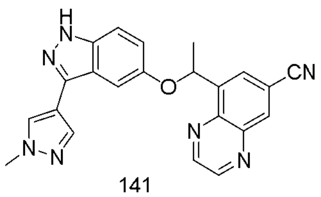
141
,

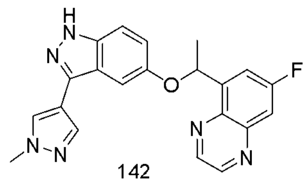
142
,

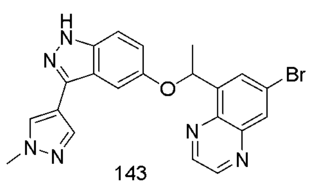
143
,

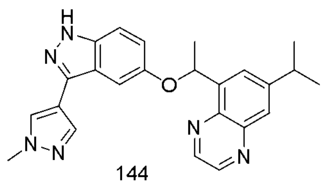
144
,

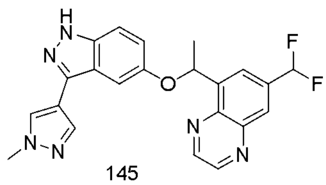
145
,

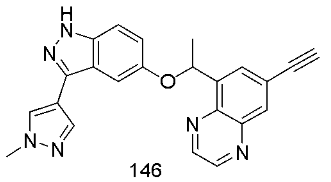
146
,

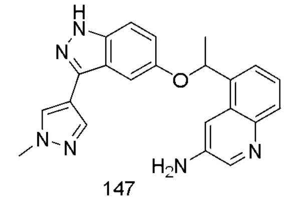
147
,

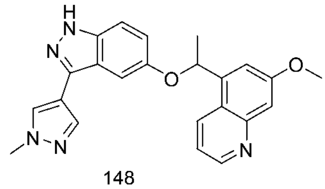
148
,

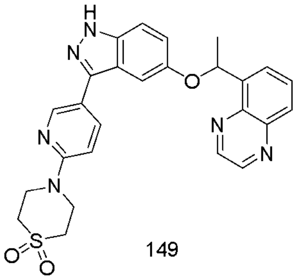
149,
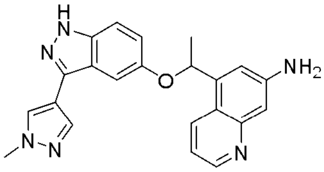
150,
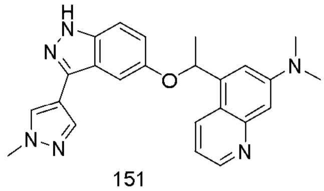
151,

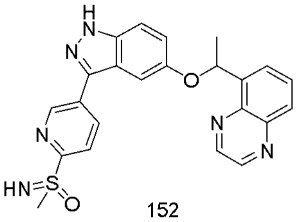
152,
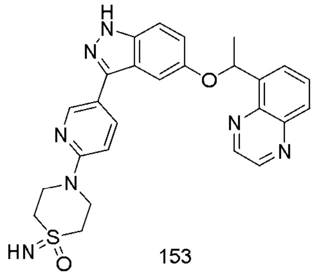
153,
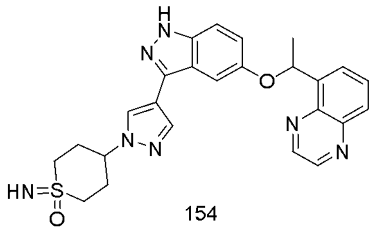
154,

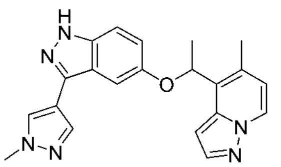
155,
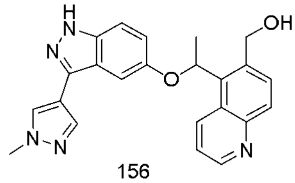
156,
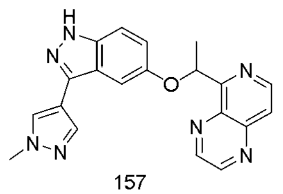
157,

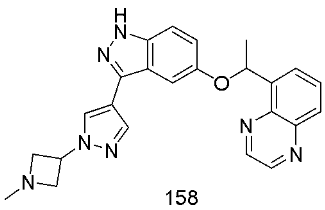
158,
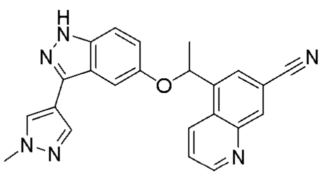
159,
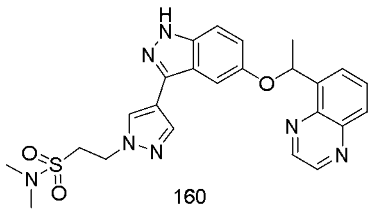
160,

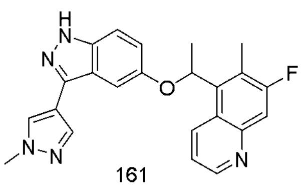
161,
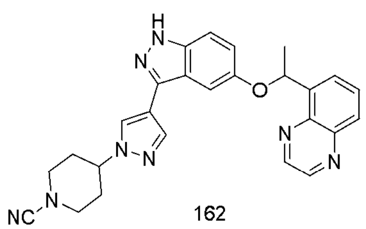
162,
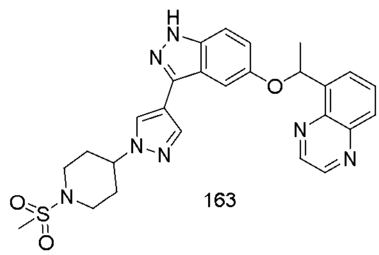
163,

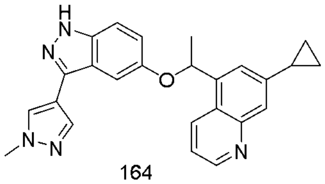
164,
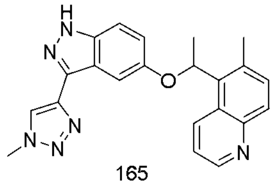
165,
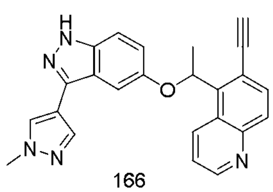
166,

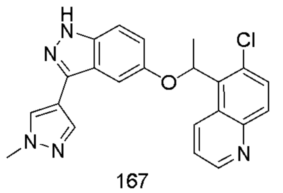
167
,
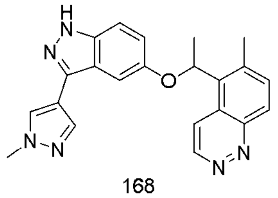
168
,
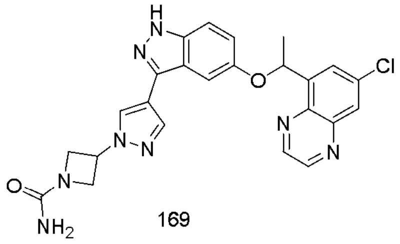
169
,

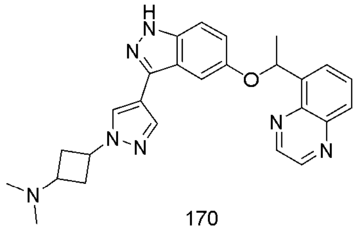
170
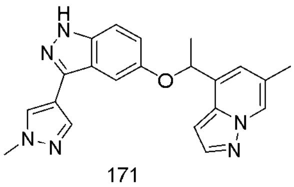
171
,
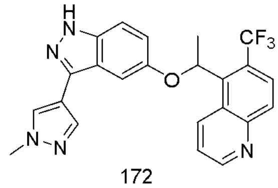
172
,

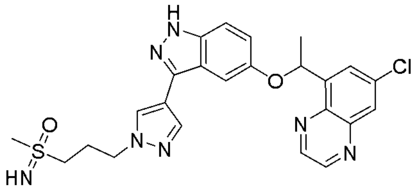
173
,
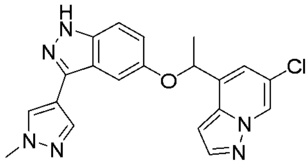
174
,

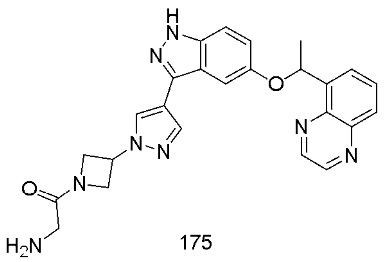
175
,
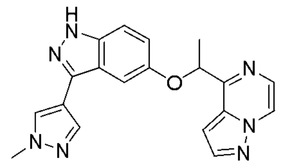
176
,
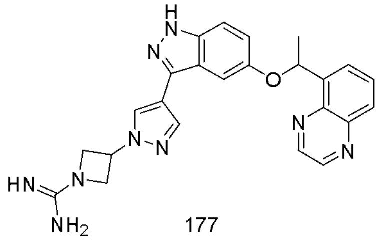
177
,

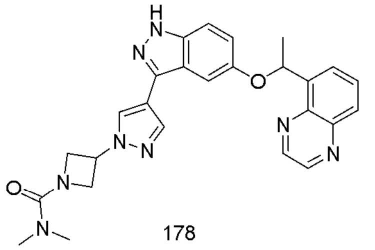
178
,
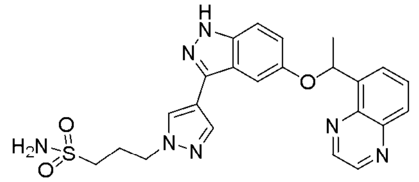
179
,

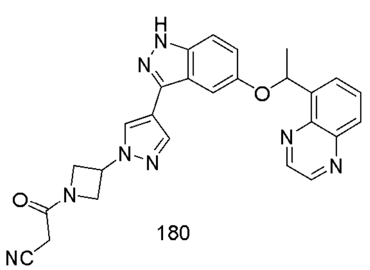
180
,
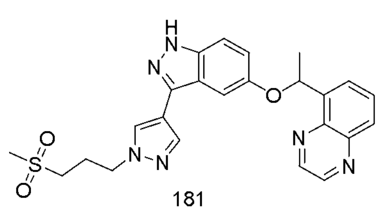
181
,

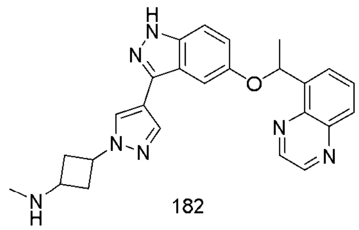
182 ,
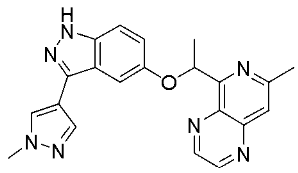
183 ,
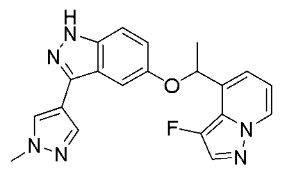
184 ,

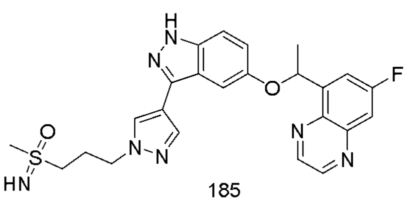
185 ,
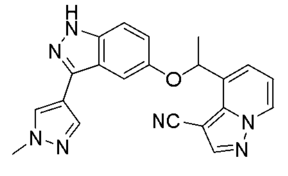
186 ,
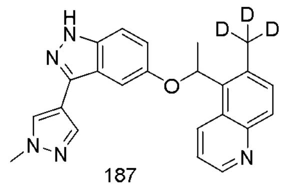
187 ,

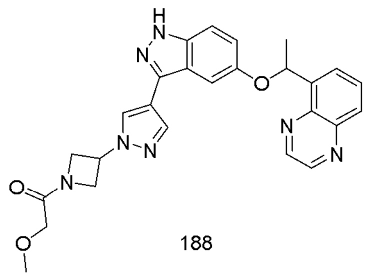
188 ,
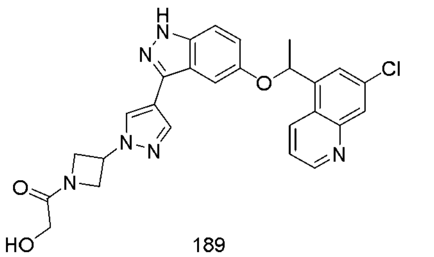
189 ,

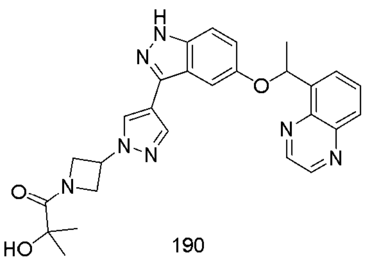
190 ,
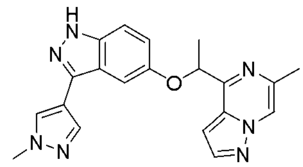
191 ,
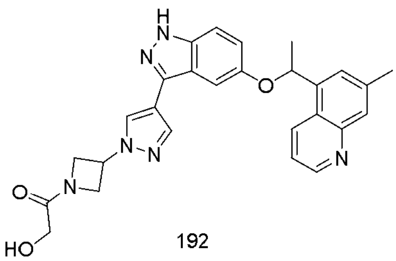
192 ,

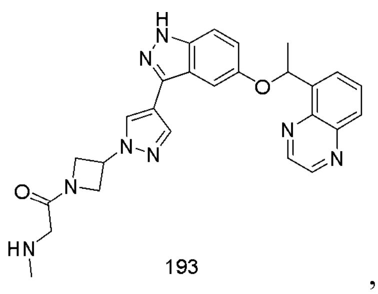
193 ,
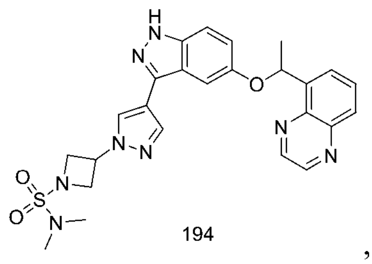
194 ,
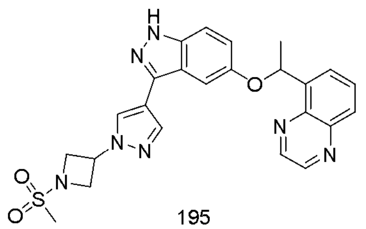
195

,
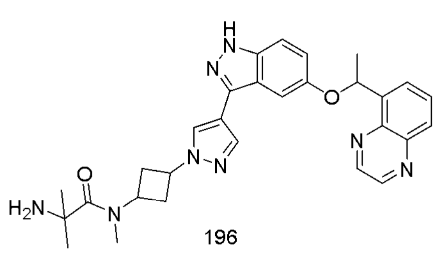
196 ,
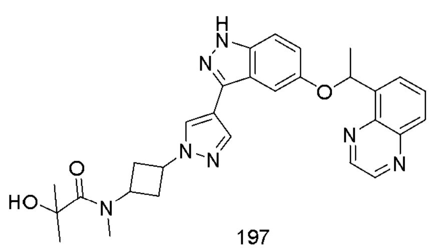
197 ,

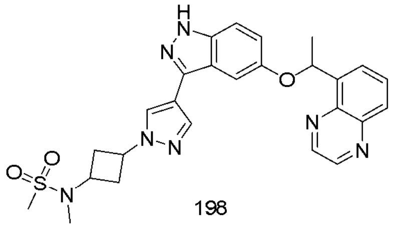
198 ,

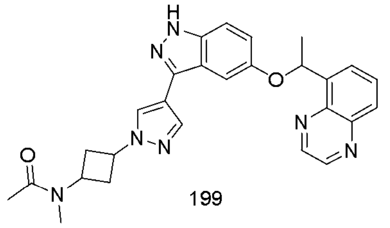
199 ,

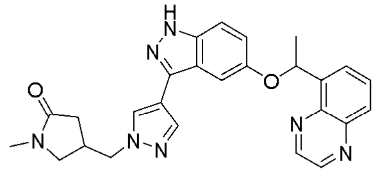
200 ,

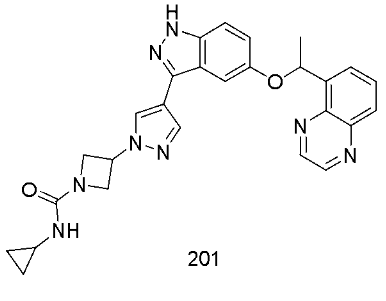
201 ,

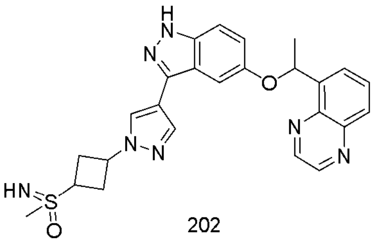
202 ,

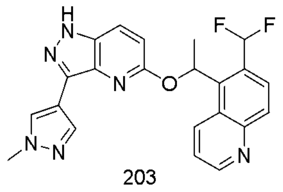
203 ,

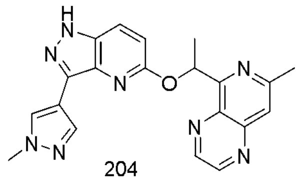
204 ,

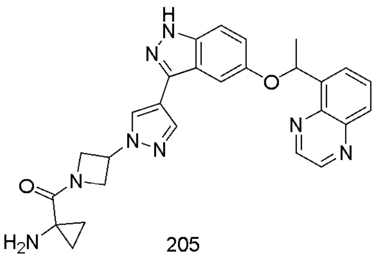
205 ,

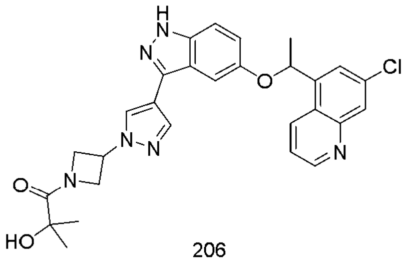
206 ,

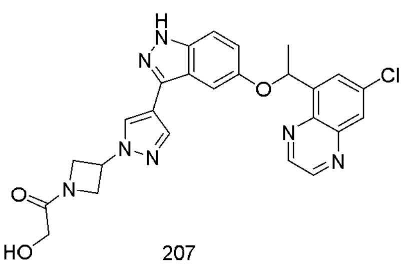
207 ,

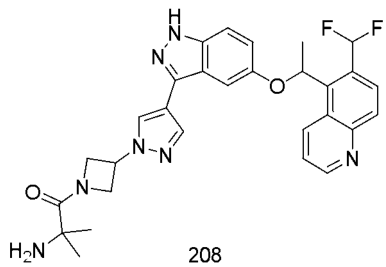
208 ,

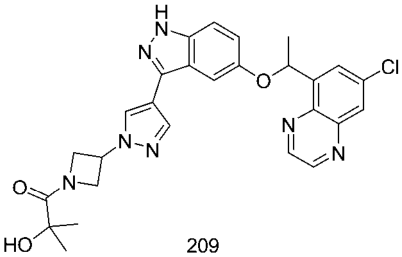
209 ,

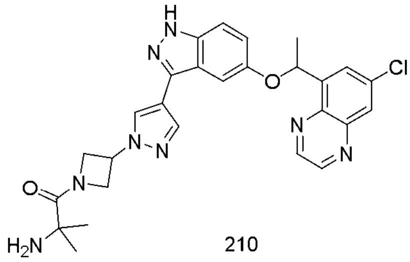
210 ,

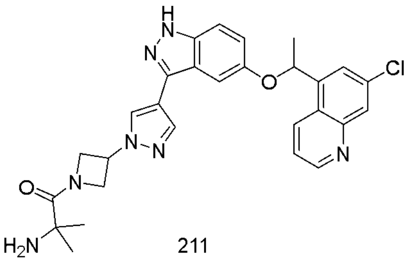
211 ,

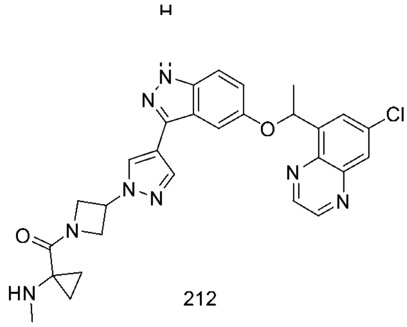
212 ,

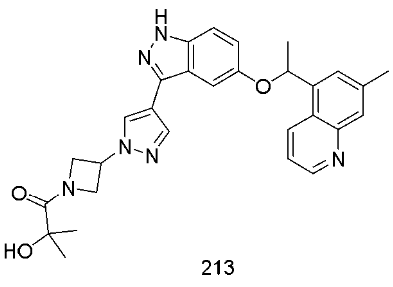
213 ,

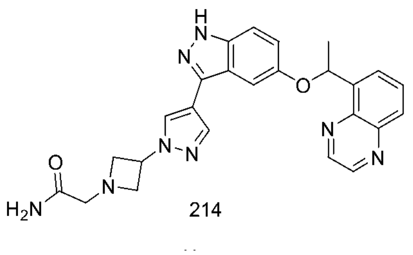
214 ,

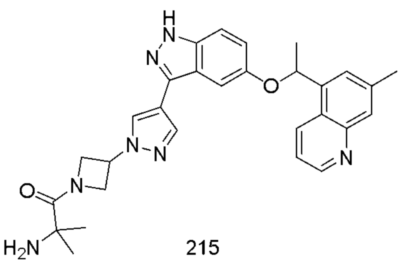
215 ,

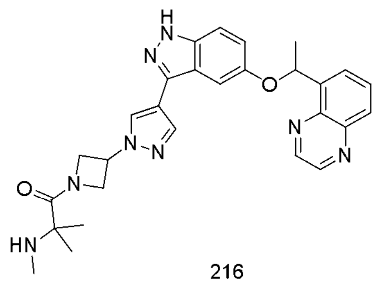
216 ,

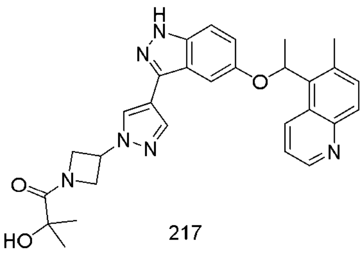
217 ,

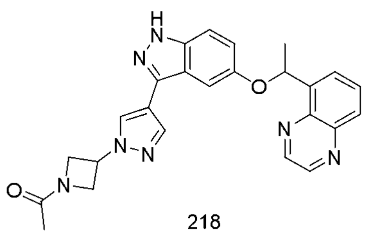
218 ,

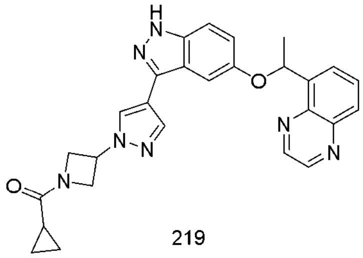
219
,
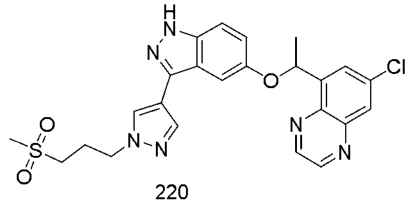
220
,
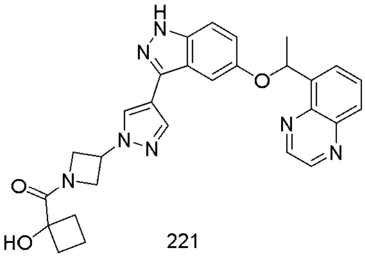
221
,
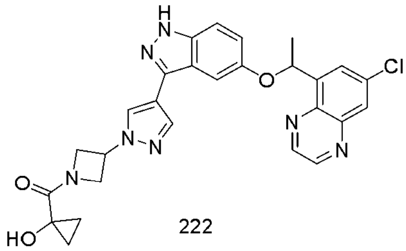
222
,
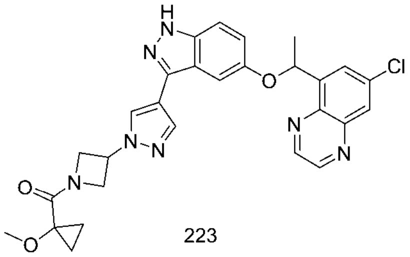
223
,
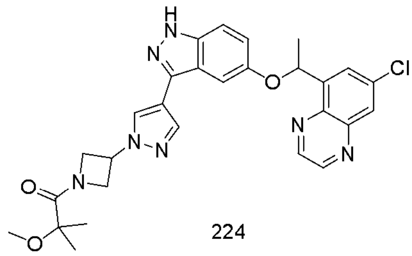
224
,
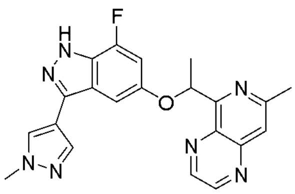
225
,
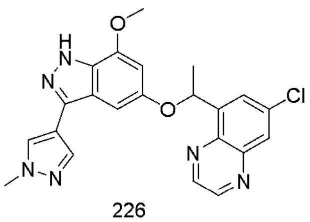
226
,
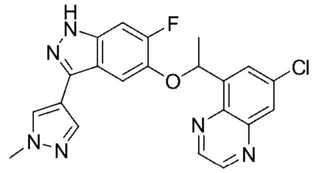
227
,
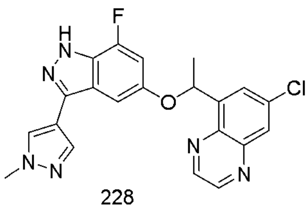
228
,
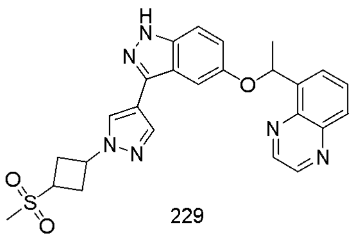
229
,
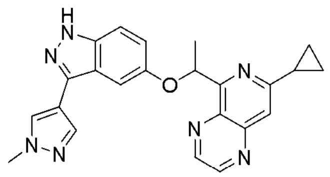
230
,
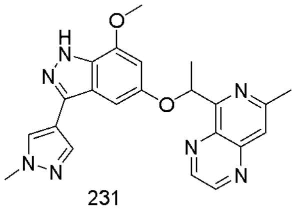
231
,
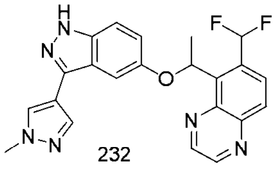
232
,
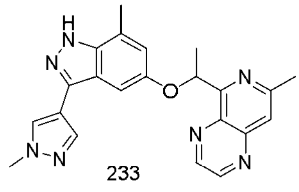
233
,

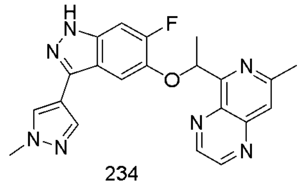
234,
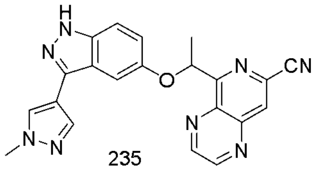
235,
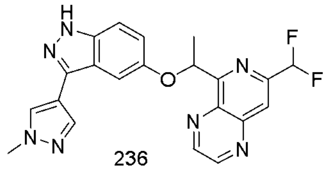
236,

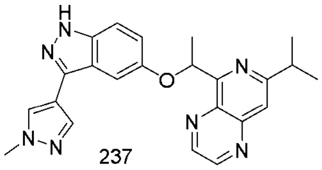
237,
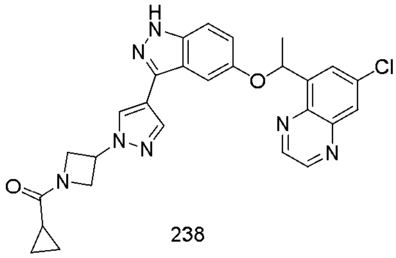
238,
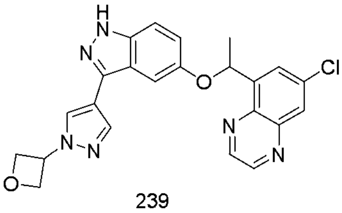
239,

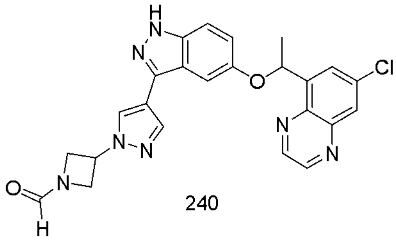
240
and
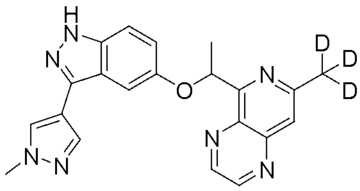
241.

The present invention also provides a method for preparing a compound of formula (II), comprising:

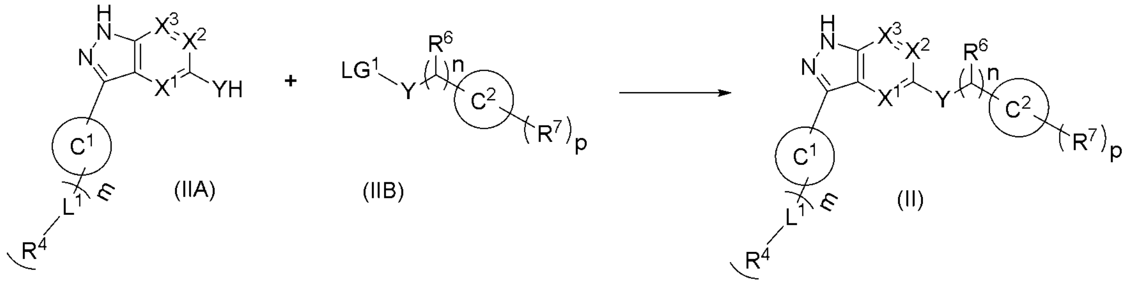
(IIA) (IIB) (II)

reacting a compound of formula (IIA) with a compound of formula (IIB) to obtain the compound of formula (II); wherein, $LG^1$ is a leaving group, preferably halogen, methylsulfonyloxy or p-tolylsulfonyloxy; and $X^1$, $X^2$, $X^3$, Y, $C^1$, $C^2$, $R^4$, $R^6$, $R^7$, $L^1$, m, n and p are as defined in formula (II);

or

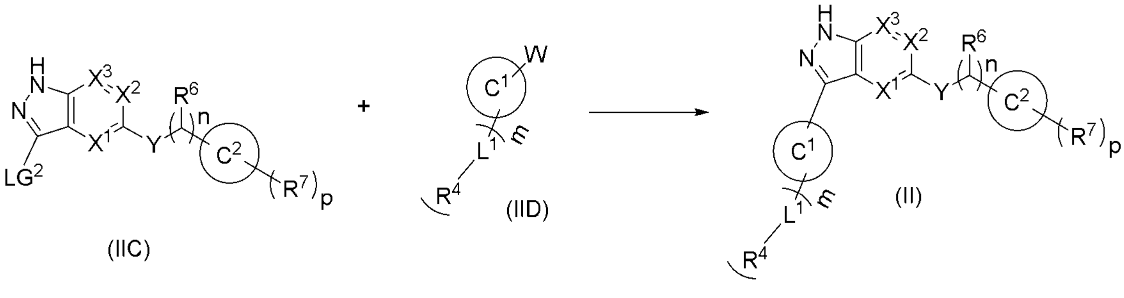
(IIC) (IID) (II)

reacting a compound of formula (IIC) with a compound of formula (IID) to obtain the compound of formula (II); wherein, $LG^2$ is a leaving group, preferably halogen, methylsulfonyloxy or p-tolylsulfonyloxy;

W is

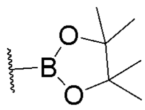 or 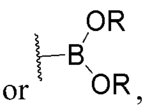,

R is hydrogen atom or $C_{1-6}$ alkyl; and
$X^1$, $X^2$, $X^3$, Y, $C^1$, $C^2$, $R^4$, $R^6$, $R^7$, $L^1$, m, n and p are as defined in formula (II);
or

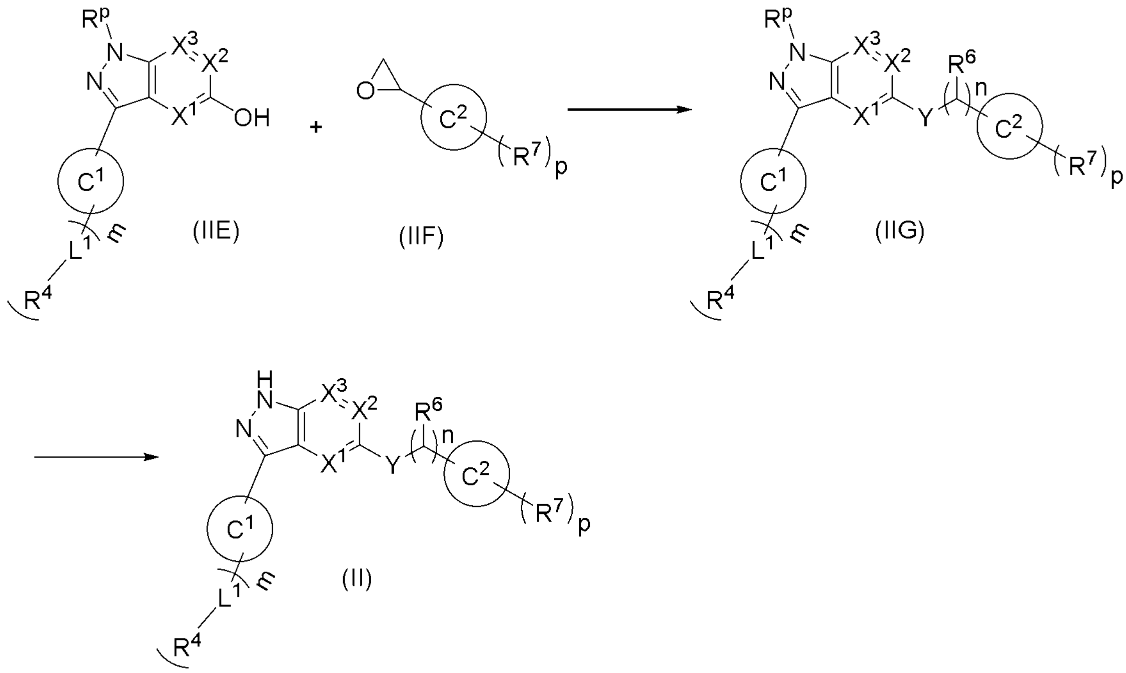

(IIE) (IIF) (IIG) (II)

reacting a compound of formula (IIE) with a compound of formula (IIF) to obtain a compound of formula (IIG), and removing the protecting group $R^p$ from the compound of formula (IIG) to obtain the compound of formula (II);
wherein, $R^p$ is tetrahydropyranyl, (trimethylsilyl)ethoxymethyl, p-tosyl, t-butyloxycarbonyl, benzyl or p-methoxybenzyl;
Y is O atom, $R^6$ is $-CH_2OH$, n is 1, and
$X^1$, $X^2$, $X^3$, $C^1$, $C^2$, $R^4$, $R^7$, $L^1$, m and p are as defined in formula (II);
or

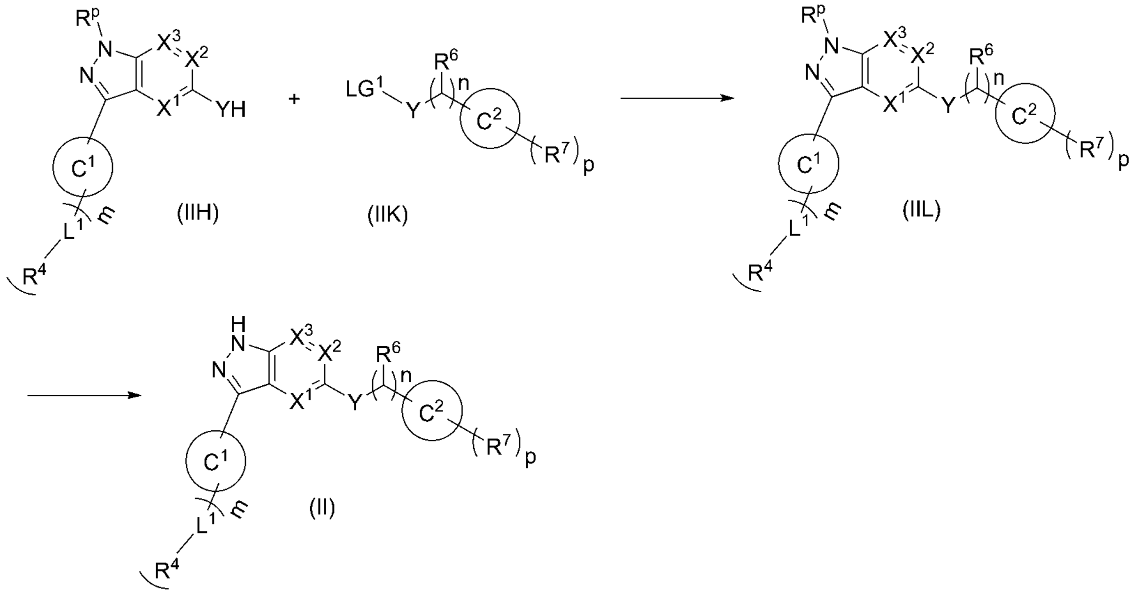

(IIH) (IIK) (IIL) (II)

reacting a compound of formula (IIH) with a compound of formula (IIK) to obtain a compound of formula (IIL), and removing the protecting group $R^p$ from the compound of formula (IIL) to obtain the compound of formula (II);
wherein, $R^p$ is tetrahydropyranyl, (trimethylsilyl)ethoxymethyl, p-tosyl, t-butyloxycarbonyl, benzyl or p-methoxybenzyl;
$LG^1$ is a leaving group, preferably halogen, methylsulfonyloxy or p-tolylsulfonyloxy; and
$X^1$, $X^2$, $X^3$, Y, $C^1$, $C^2$, $R^4$, $R^6$, $R^7$, $L^1$, m, n and p are as defined in formula (II);
or

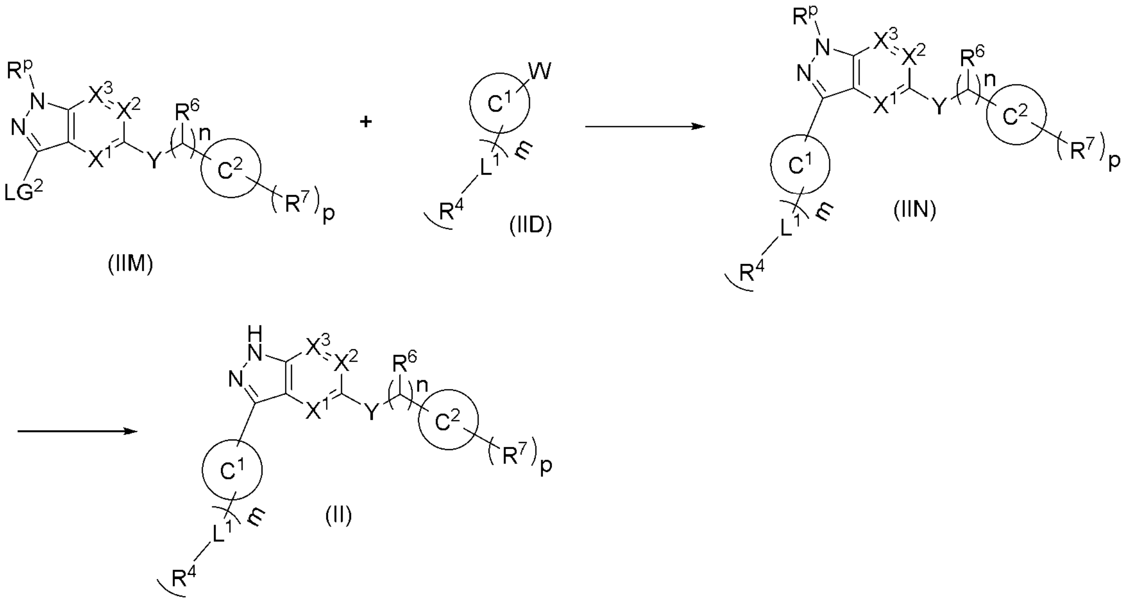

(IIM) (IID) (IIN) (II)

reacting a compound of formula (IIM) with a compound of formula (IID) to obtain a compound of formula (IIN), and removing the protecting group $R^p$ from the compound of formula (IIN) to obtain the compound of formula (II);
wherein, $R^p$ is tetrahydropyranyl, (trimethylsilyl)ethoxymethyl, p-tosyl, t-butyloxycarbonyl, benzyl or p-methoxybenzyl;
$LG^2$ is a leaving group, preferably halogen, methylsulfonyloxy or p-tolylsulfonyloxy;
W is

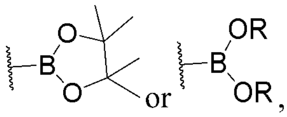

R is hydrogen atom or $C_{1-6}$ alkyl; and
$X^1$, $X^2$, $X^3$, Y, $C^1$, $C^2$, $R^4$, $R^6$, $R^7$, $L^1$, m, n and p are as defined in formula (II);
or

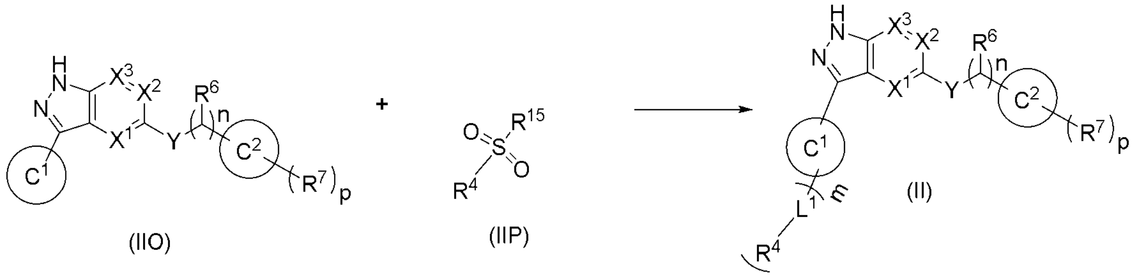

(IIO) (IIP) (II)

reacting a compound of formula (IIO) with a compound of formula (IIP) to obtain the compound of formula (II);
wherein, m is 1, $L^1$ is $-S(O)_2-C_{1-6}$ alkyl-, $R^{15}$ is $C_{1-6}$ alkenyl or $-C_{1-6}$ alkylene-halogen; and
$X^1$, $X^2$, $X^3$, Y, $C^1$, $C^2$, $R^4$, $R^6$, $R^7$, n and p are as defined in formula (II).

The present invention also provides a method for preparing a compound of formula (III-1), comprising:

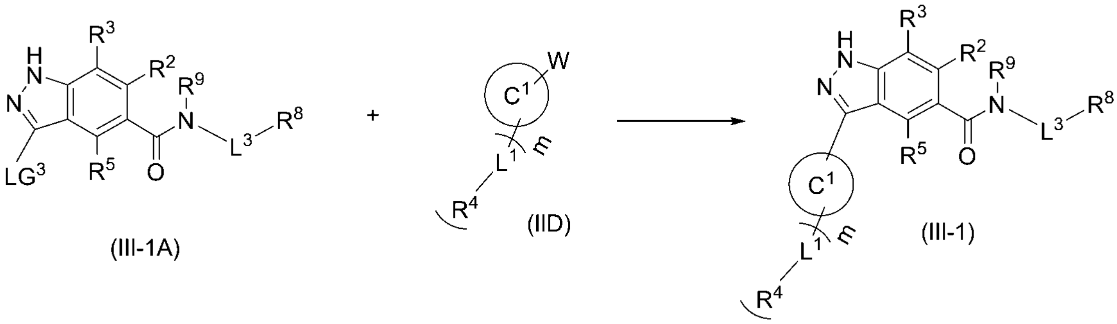

(III-1A) (IID) (III-1)

reacting a compound of formula (III-1A) with a compound of formula (IID) to obtain the compound of formula (III-1);
wherein, $LG^3$ is a leaving group, preferably halogen, methylsulfonyloxy or p-tolylsulfonyloxy;
W is

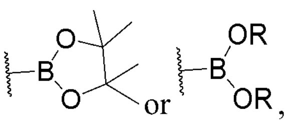

R is hydrogen atom or $C_{1-6}$ alkyl; and
$C^1$, $R^2$, $R^4$, $R^5$, $R^8$, $R^9$, $L^1$, $L^3$ and m are as defined in formula (III-1).

The present invention also provides a method for preparing a compound of formula (III-2), comprising:

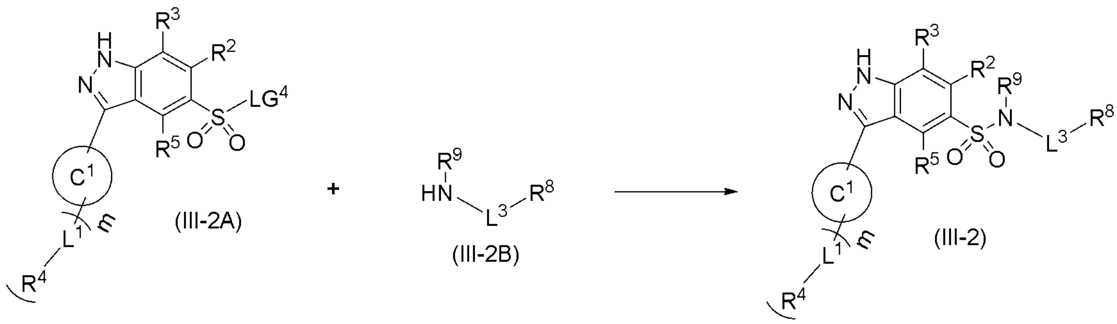

reacting a compound of formula (III-2A) with a compound of formula (III-2B) to obtain the compound of formula (III-2);
wherein, $LG^4$ is halogen, preferably Cl atom;
$C^1$, $R^2$, $R^4$, $R^5$, $R^8$, $R^9$, $L^1$, $L^3$ and m are as defined in formula (III-2).

The present invention also provides a pharmaceutical composition comprising the compound of formula (I), (II), (II-1), (II-2), (III), (III-1), (111-2) or (IV), and one or more pharmaceutically acceptable excipients.

The present invention relates to a use of the compound of formula (I), (II), (II-1), (II-2), (III), (III-1), (III-2) or (IV) or the pharmaceutical composition comprising the same in the preparation of a FGFR inhibitor.

The present invention also relates to a use of the compound of formula (I), (II), (II-1), (II-2), (III), (III-1), (III-2) or (IV) or the pharmaceutical composition comprising the same in the preparation of a medicament for treating and/or preventing a tumor.

The present invention also relates to the compound of formula (I), (II), (II-1), (II-2), (III), (III-1), (III-2) or (IV) or the pharmaceutical composition comprising the same for use as a medicament.

The present invention also relates to the compound of formula (I), (II), (II-1), (II-2), (III), (III-1), (III-2) or (IV) or the pharmaceutical composition comprising the same, for use as a FGFR inhibitor.

The present invention also relates to a use of the compound of formula (I), (II), (II-1), (II-2), (III), (III-1), (III-2) or (IV) or the pharmaceutical composition comprising the same, for use in treating and/or preventing a tumor.

The present invention also relates to a method for inhibiting FGFR, comprising administering a therapeutically effective amount of the compound of formula (I), (II), (II-1), (II-2), (III), (III-1), (III-2) or (IV) or the pharmaceutical composition comprising the same to a patient in need thereof.

The present invention also relates to a method for treating and/or preventing a tumor, comprising administering a therapeutically effective amount of the compound of formula (I), (II), (II-1), (II-2), (III), (III-1), (III-2) or (IV) or the pharmaceutical composition comprising the same to a patient in need thereof.

In some embodiments, the tumor is a cancer; the cancer is preferably selected from the group consisting of cholangiocarcinoma, liver cancer, breast cancer, prostatic cancer, lung cancer, thyroid cancer, gastric cancer, ovarian cancer, colorectal cancer, endometrial cancer, urothelial cell carcinoma, testicular cancer, cervical cancer, leukemia, skin cancer, squamous-cell carcinoma, basal cell carcinoma, bladder cancer, esophagus cancer, head and neck cancer, kidney cancer, pancreatic cancer, bone cancer, lymphoma, melanoma, sarcoma, peripheral neuroepithelioma, glioma, ependymoma, neuroblastoma, gangliocytoma, medulloblastoma, pinealocytoma, meningeoma, neurofibroma, schwannoma and Wilm's tumor; and more preferably, is selected from the group consisting of cholangiocarcinoma, liver cancer, breast cancer, prostatic cancer, lung cancer, thyroid cancer, gastric cancer, ovarian cancer, colorectal cancer, endometrial cancer and urothelial cell carcinoma.

The pharmaceutical composition of the present invention can be various conventional dosage forms, for example, tablet, aqueous suspension, oily suspension, dispersible powder, dispersible granule, emulsion, hard capsule, soft capsule, sterile aqueous solution for injection, sterile oil-in-water microemulsion for injection, or suppository. Each of the above dosage forms can be prepared by conventional preparation methods.

It is well known to those skilled in the art that the administration dose of a drug depends on a variety of factors including, but not limited to the following factors: activity of a specific compound used, age of the patient, weight of the patient, general health of the patient, behavior of the patient, diet of the patient, administration time, administration route, excretion rate, drug combination and the like. In addition, the optimal treatment, such as treatment mode, daily dose of the compound or the type of pharmaceutically acceptable salt thereof can be verified according to the traditional therapeutic regimens.

Definition of terms

Regarding the terms not defined herein, they have the meanings commonly understood by those skilled in the art.

Regarding the terms defined herein, they have the meanings defined in the description.

The term "substitution" or "substituent" refers to one or more hydrogen atoms being replaced by a group indicated. When the substitution position is not indicated, the substitution may occur at any position, provided that the formation of a stable or chemically feasible chemical is allowed.

The term "optional" or "optionally" means that the event or circumstance described subsequently may, but not necessarily, occur, and such a description includes the situation in which the event or circumstance occurs or the situation in which the event or circumstance does not occur.

When any variable (such as R) appears more than once in the structure of a compound, its definition in each case is independent. For example, if a group is substituted by 0 to 2 R, the group may optionally be substituted by up to two R, and R in each case has independent options.

The term "alkyl" refers to a saturated linear or branched monovalent hydrocarbon group having 1 to 20 (for example, 1, 2, 3, 4, 5, 6, 7, 8, 9, 10, 11, 12, 13, 14, 15, 16, 17, 18, 19 and 20) carbon atoms, preferably a $C_{1-10}$ alkyl, and more preferably a $C_{1-6}$ alkyl. Examples of the alkyl include, but are not limited to, methyl, ethyl, n-propyl, isopropyl, n-butyl, isobutyl, tert-butyl, sec-butyl, n-pentyl, 2,2-dimethylpropyl, 2-methylbutyl, n-hexyl, 2,2-dimethylbutyl, 2-methylpentyl, 3-methylpentyl, 2,3-dimethylbutyl, n-heptyl, 2-methylhexyl, 3-methylhexyl, 2,3-dimethylpentyl, 2,4-dimethylpentyl, 2,2-dimethylpentyl, 3,3-dimethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 3-ethylhexyl, 4-ethylhexyl, 2-methyl-3-ethylpentyl, n-nonyl, 2-methyl-3-ethylhexyl, n-decyl and 3,3-diethylhexyl.

The term "alkylene" refers to a divalent functional group formed by removing 1 hydrogen atom from the alkyl as defined above.

The term "alkenyl" refers to a linear or branched monovalent hydrocarbon group having 2 to 6 (for example 2, 3, 4, 5 and 6) carbon atoms and at least one carbon-carbon double bond, in which the carbon-carbon double bond may be located anywhere within the alkenyl. The alkenyl is preferably a $C_{2-5}$ alkenyl. Examples of the alkenyl include, but are not limited to, $-CH=CH_2$, $-CH=CH-CH_3$, $-CH_2-CH=CH_2$, $-CH=CH-CH_2-CH_3$, $-CH_2-CH=CH-CH_3$, $-CH=CH-CH=CH_2$, $-CH=C(CH_3)-CH_3$ and $-CH_2-C(CH_3)=CH_2$.

The term "alkynyl" refers to a linear or branched monovalent hydrocarbon group having 2 to 6 (for example 2, 3, 4, 5 and 6) carbon atoms and at least one carbon-carbon triple bond, in which the carbon-carbon triple bond may be located anywhere within the alkynyl. The alkynyl is preferably a $C_{2-5}$ alkynyl. Examples of the alkynyl include, but are not limited to, $-C\equiv CH$, $-C\equiv C-CH_3$, $-CH_2-C\equiv CH$, $-C\equiv C-CH_2-CH_3$, $-CH_2-CH_2-C\equiv CH$, $-CH(CH_3)C\equiv CH$ and $-CH_2-C\equiv C-CH_3$.

The term "cycloalkyl" includes two categories, one is conventional cycloalkyl, and the other is heterostructured cycloalkyl.

The conventional cycloalkyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) carbon atoms, preferably a $C_{3-12}$ conventional cycloalkyl, more preferably a $C_{3-10}$ conventional cycloalkyl, further preferably a $C_{3-8}$ conventional cycloalkyl, and most preferably a $C_{3-6}$ conventional cycloalkyl. The conventional cycloalkyl optionally comprises one or more double or triple bonds.

The conventional cycloalkyl may be a monocyclic cycloalkyl, and examples of the monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl and cyclooctyl. The conventional cycloalkyl may also be a polycyclic cycloalkyl (for example, bicycloalkyl and tricycloalkyl), and the polycyclic cycloalkyl includes spiro cycloalkyl, fused cycloalkyl and bridged cycloalkyl.

The term "spiro cycloalkyl" refers to a 5 to 20 membered (for example, 5, 6, 7, 8, 9, 10, 11, 12, 13, 14, 15, 16, 17, 18, 19 and 20 membered) spiro cycloalkyl, preferably a 6 to 14 membered spiro cycloalkyl, and more preferably a 7 to 10 membered spiro cycloalkyl. The spiro cycloalkyl may be a mono-spiro cycloalkyl, a di-spiro cycloalkyl, or a poly-spiro cycloalkyl, preferably a mono-spiro cycloalkyl, and more preferably a 4-membered/4-membered, 4-membered/5-membered, 4-membered/6-membered, 5-membered/5-membered or 5-membered/6-membered mono-spiro cycloalkyl. Examples of the spiro cycloalkyl include, but are not limited to:

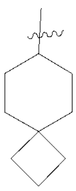 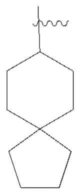 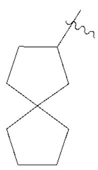 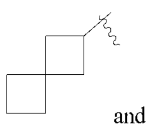 and 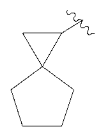 .

The term "fused cycloalkyl" refers to a 5 to 20 membered (for example, 5, 6, 7, 8, 9, 10, 11, 12, 13, 14, 15, 16, 17, 18, 19 and 20 membered) fused cycloalkyl, preferably a 6 to 14 membered fused cycloalkyl, and more preferably a 7 to 10

membered fused cycloalkyl. The fused cycloalkyl may be a bicyclic, tricyclic, tetracyclic, pentacyclic or more fused cycloalkyl, preferably a bicyclic or tricyclic fused cycloalkyl, and more preferably a 5-membered/5-membered, or 5-membered/6-membered fused cycloalkyl. Examples of the fused cycloalkyl include, but are not limited to:

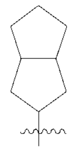 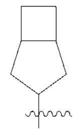 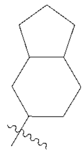 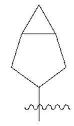 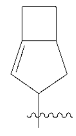 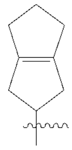 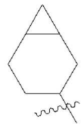 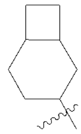 and 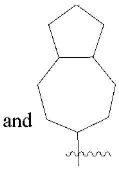 .

The term "bridged cycloalkyl" refers to a 5 to 20 membered (for example, 5, 6, 7, 8, 9, 10, 11, 12, 13, 14, 15, 16, 17, 18, 19 and 20 membered) bridged cycloalkyl, preferably a 6 to 14 membered bridged cycloalkyl, and more preferably a 7 to 10 membered bridged cycloalkyl. The bridged cycloalkyl may be a bicyclic, tricyclic, tetracyclic, pentacyclic or more bridged cycloalkyl, preferably a bicyclic, tricyclic or tetracyclic bridged cycloalkyl, and more preferably a bicyclic or tricyclic bridged cycloalkyl. Examples of the bridged cycloalkyl include, but are not limited to:

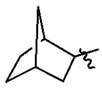 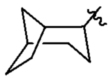 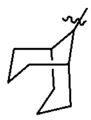 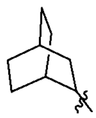 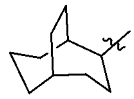

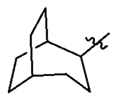 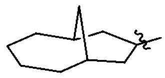 and 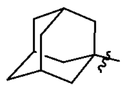 .

The term "heterostructured cycloalkyl" includes a monocyclic cycloalkyl, spiro cycloalkyl, fused cycloalkyl and bridged cycloalkyl fused with any one selected from the group consisting of conventional aryl, conventional heteroaryl and conventional heterocyclyl, in which the attachment point is located on the corresponding conventional cycloalkyl (referring to a monocyclic cycloalkyl, spiro cycloalkyl, fused cycloalkyl or bridged cycloalkyl). Examples of the heterostructured cycloalkyl include, but are not limited to:

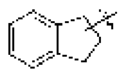 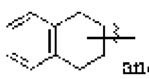 and 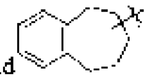 .

The term "cycloalkylene" refers to a divalent functional group formed by removing 1 hydrogen atom from the cycloalkyl as defined above.

The term "heterocyclyl" includes two categories, one is conventional heterocyclyl, and the other is heterostructured heterocyclyl.

The conventional heterocyclyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) ring atoms, in which one or more ring atoms are replaced by one or more elements selected from the group consisting of N, O, S, S(O) and $S(O)_2$, and the replacement does not result in formation of -O-O-, -O-S- or -S-S-. The conventional heterocyclyl is preferably a 3 to 12 membered conventional heterocyclyl in which 1 to 4 (for example 1, 2, 3 and 4) atoms are heteroatoms; more preferably a 3 to 10 membered or 3 to 8 membered conventional heterocyclyl in which 1 to 3 (for example 1, 2 and 3) atoms are heteroatoms; and most preferably a 5 to 7 membered or 4 to 6 membered conventional heterocyclyl in which 1 to 2 or 1 to 3 atoms are heteroatoms.

The conventional heterocyclyl may be a monocyclic heterocyclyl, and examples of the monocyclic heterocyclyl include, but are not limited to oxetanyl, 3-pyrrolinyl, pyrrolidinyl, imidazolidinyl, tetrahydrofuranyl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl and tetrahydropyranyl, and preferably 1,2,5-oxadiazolyl, tetrahydropyranyl or morpholinyl. The conventional heterocyclyl may also be a polycyclic heterocyclyl and the polycyclic heterocyclyl includes spiro heterocyclyl, fused heterocyclyl and bridged heterocyclyl.

The term "spiro heterocyclyl" refers to a 5 to 20 membered (for example, 5, 6, 7, 8, 9, 10, 11, 12, 13, 14, 15, 16, 17, 18, 19 and 20 membered) spiro heterocyclyl, preferably a 6 to 14 membered spiro heterocyclyl, and more preferably a 7 to 10 membered spiro heterocyclyl. The spiro heterocyclyl may be a mono-spiro heterocyclyl, a di-spiro heterocyclyl, or a

poly-spiro heterocyclyl, preferably a mono-spiro heterocyclyl or a di-spiro heterocyclyl, and more preferably a 3-membered/6-membered, 4-membered/4-membered, 4-membered/5-membered, 4-membered/6-membered, 5-membered/5-membered or 5-membered/6-membered mono-spiro heterocyclyl. Examples of the spiro heterocyclyl include, but are not limited to:

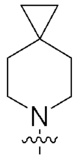 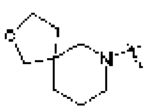 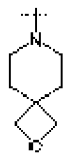 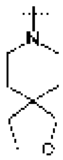 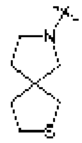 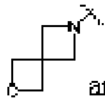 and 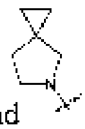.

The term "fused heterocyclyl" refers to a 5 to 20 membered (for example, 5, 6, 7, 8, 9, 10, 11, 12, 13, 14, 15, 16, 17, 18, 19 and 20 membered) fused heterocyclyl, preferably a 6 to 14 membered fused heterocyclyl, and more preferably a 7 to 10 membered fused heterocyclyl. The fused heterocyclyl may be a bicyclic, tricyclic, tetracyclic, pentacyclic or more fused heterocyclyl, preferably a bicyclic or tricyclic fused heterocyclyl, and more preferably a 5-membered/5-membered or 5-membered/6-membered bicyclic fused heterocyclyl. Examples of the fused heterocyclyl include, but are not limited to:

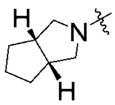 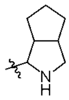 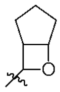 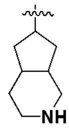 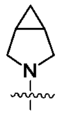 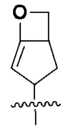 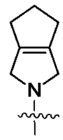 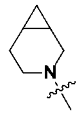

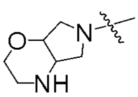 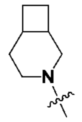 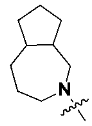 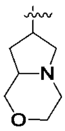 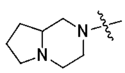 and 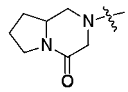.

The term "bridged heterocyclyl" refers to a 5 to 14 membered (for example, 5, 6, 7, 8, 9, 10, 11, 12, 13 and 14 membered) bridged heterocyclyl, preferably a 6 to 14 membered bridged heterocyclyl, and more preferably a 7 to 10 membered bridged heterocyclyl. The bridged heterocyclyl may be a bicyclic, tricyclic, tetracyclic, pentacyclic or more bridged heterocyclyl, preferably a bicyclic, tricyclic or tetracyclic bridged heterocyclyl, and more preferably a bicyclic or tricyclic bridged heterocyclyl. Examples of the bridged heterocyclyl include, but are not limited to:

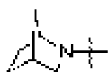 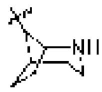 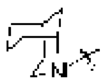 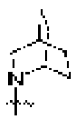

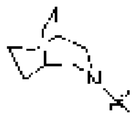 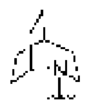 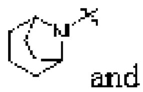 and 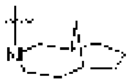.

The term "heterostructured heterocyclyl" includes a monocyclic heterocyclyl, spiro heterocyclyl, fused heterocyclyl and bridged heterocyclyl fused with any one selected from the group consisting of conventional aryl, conventional heteroaryl and conventional cycloalkyl, in which the attachment point is located on the corresponding conventional heterocyclyl (referring to a monocyclic heterocyclyl, spiro heterocyclyl, fused heterocyclyl or bridged heterocyclyl). Examples of the heterostructured heterocyclyl include, but are not limited to:

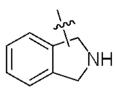 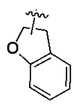 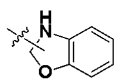 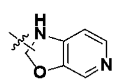 and 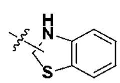.

The term "heterocycloalkylene" refers to a divalent functional group formed by removing 1 hydrogen atom from the heterocycloalkyl as defined above.

The term "aryl" includes two categories, one is conventional aryl, and the other is heterostructured aryl.

The conventional aryl refers to a 6 to 14 membered (for example, 6, 7, 8, 9, 10, 11, 12, 13 and 14 membered) aromatic hydrocarbon group, preferably a $C_{6-10}$ conventional aryl, and more preferably phenyl, naphthyl, phenanthryl or anthracenyl.

The term "heterostructured aryl" includes a conventional aryl fused with any one selected from the group consisting of conventional heteroaryl, conventional heterocyclyl and conventional cycloalkyl, in which the attachment point is located on the conventional aryl. Examples of the heterostructured aryl include, but are not limited to:

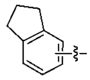 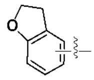 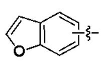 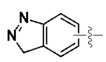 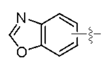 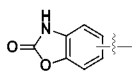 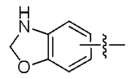 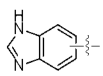

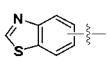 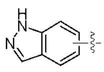 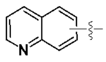 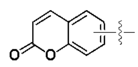 and 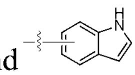 .

The term "arylene" refers to a divalent functional group formed by removing 1 hydrogen atom from the aryl as defined above.

The term "heteroaryl" includes two categories, one is conventional heteroaryl, and the other is heterostructured heteroaryl.

The conventional heteroaryl refers to a 5 to 14 membered (for example, 5, 6, 7, 8, 9, 10, 11, 12, 13 and 14 membered) aromatic hydrocarbon group in which 1 to 4 (for example, 1, 2, 3 and 4) carbon atoms are replaced by heteroatoms selected from the group consisting of oxygen, sulfur and nitrogen. Preferably, the number of ring atoms is 5 to 10, including 1 to 3 (for example, 1, 2 and 3) heteroatoms. More preferably, the number of ring atoms is 5 or 6, including 1 to 2 heteroatoms. Examples of the conventional heteroaryl include, but are not limited to imidazolyl, furyl, thienyl, thiazolyl, pyrazolyl, oxazolyl, pyrrolyl, triazolyl, tetrazolyl, pyridyl, pyrimidinyl, thiadiazolyl, pyridazinyl, pyrazinyl, quinolyl, isoquinolyl, naphthyridinyl and imidazopyridyl, preferably imidazolyl, thiazolyl, pyrazolyl, pyrimidinyl or thiazolyl, and more preferably pyrazolyl or thiazolyl.

The term "heterostructured heteroaryl" includes a conventional heteroaryl fused with any one selected from the group consisting of conventional aryl, conventional cycloalkyl and conventional heterocyclyl, in which the attachment point is located on the conventional heteroaryl. Examples of the heterostructured heteroaryl include, but are not limited to:

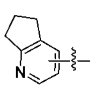 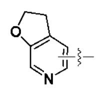 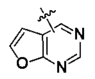 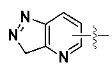 and 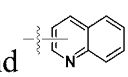 .

The term "heteroarylene" refers to a divalent functional group formed by removing 1 hydrogen atom from the heteroaryl as defined above.

The term "alkoxy" includes -O-alkyl and -O-cycloalkyl, in which the "alkyl" and "cycloalkyl" are as defined above. Examples of the alkoxy include, but are not limited to: methoxy, ethoxy, propoxy, butoxy, cyclopropyloxy, cyclobutyloxy, cyclopentyloxy and cyclohexyloxy.

The term "haloalkyl" refers to an alkyl substituted by one or more halogens, in which the alkyl is as defined above.

The term "haloalkoxy" refers to an alkoxy substituted by one or more halogens, in which the alkoxy is as defined above.

The term "hydroxy" refers to an -OH group.

The term "halogen" refers to a -F, -Cl, -Br or -I group.

The term "amino" refers to a $-NH_2$ group.

The term "cyano" refers to a -CN group.

The term "nitro" refers to a $-NO_2$ group.

The term "oxo" refers to an =O group.

The term "carboxy" refers to a -C(= O)OH group.

The term "thiol" refers to a -SH group.

The term "alkoxycarbonyl" refers to a -C(=O)O-alkyl or -C(=O)O-cycloalkyl group, in which the alkyl and cycloalkyl are as defined above.

The term "acyl" refers to a -C(= O)R group, in which R is selected from the group consisting of alkyl, cycloalkyl, heterocyclyl, aryl and heteroaryl.

The symbol "" refers to an attachment point.

The term "stereoisomer" refers to isomers with the same structure but different arrangements of atoms in space.

It includes cis and trans (or Z and E) isomers, (-)- and (+)-isomers, (*R*)- and (*S*)-enantiomers, diastereomers, (*D*)- and (*L*)-isomers, tautomers, atropisomers, conformers and mixtures thereof (e.g., mixtures of racemates and diastereomers). Substituents in the compounds of the present invention may have additional asymmetric atoms. All of these stereoisomers and mixtures thereof are included within the scope of the present invention. Optically active (-)- and (+)-isomers, (*R*)- and (*S*)-enantiomers and (*D*)- and (*L*)-isomers may be prepared by chiral synthesis, chiral reagents or other conventional techniques. An isomer of a compound of the present invention can be prepared by asymmetric synthesis or chiral auxiliary, or, when the molecule contains a basic functional group (such as amino) or an acidic functional group (such as carboxyl), it can be formed into a diastereoisomer salt with an appropriate optically active acid or base, and then the diastereoisomers can be resolved by conventional methods known in the art to obtain pure isomers. In addition, the resolution of enantiomers and diastereomers is usually achieved by chromatography.

In the chemical structures of the compounds of the present invention, the bond "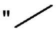" represents an unspecified configuration, that is, if chiral isomers exist in the chemical structures, the bond "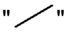" may be "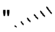"or "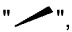", or contains both "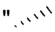" and "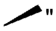" configurations. For all of carbon-carbon double bonds, even if only one configuration is named, both Z and E configurations are included.

The compounds and intermediates of the present invention may also exist in different tautomeric forms, and all such forms are included within the scope of the present invention. The term "tautomer" or "tautomeric form" refers to structural isomers of different energies that can be interconverted via a low energy barrier. For example, proton tautomers (also known as prototropic tautomers) include interconversions via proton migration, such as keto-enol and imine-enamine, lactam-lactimide isomerizations.

The compounds of the present invention includes all suitable isotope substitutes of the compounds. The term "isotope substitute" refers to a compound in which at least one atom is replaced by an atom having the same atomic number but different atomic mass. Examples of isotopes that can be incorporated into the compounds of the present invention include stable and radioactive isotopes of hydrogen, carbon, nitrogen, oxygen, phosphorus, sulphur, fluorine, chlorine, bromine, iodine, etc, such as $^{2}H$ (deuterium, D), $^{3}H$ (tritium , T), $^{11}C$, $^{13}C$, $^{14}C$, $^{15}N$, $^{17}O$, $^{18}O$, $^{32}p$, $^{33}p$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$, $^{131}I$, etc., preferably deuterium.

As used herein, the singular forms of "a," "an," and "the" include plural references, and vice versa, unless the context clearly dictates otherwise.

When the term "about" is applied to a parameter such as pH, concentration, temperature, etc., it indicates that the parameter may vary by $\pm$10%, and is sometimes more preferably within $\pm$5%. As will be appreciated by those skilled in the art, a number is generally given only for illustrative purpose, instead of being limiting.

**DETAILED DESCRIPTION OF THE INVENTION**

The present invention is further described below in conjunction with the examples, but these examples are not intended to limit the scope of the present disclosure.

The compounds of the present invention are prepared by using convenient starting materials and general preparation procedures. The present invention provides typical or preferential reaction conditions, such as reaction temperature, time, solvent, pressure and molar ratio of reactants. However, unless specially specified, other reaction conditions can also be adopted. Optimal conditions may vary with the use of specific reactants or solvents, but under normal circumstances, the steps and conditions optimized for reaction can be determined.

In addition, some protecting groups may be used in the present invention to protect certain functional groups from unnecessary reactions. The protecting groups suitable for various functional groups and their protection or deprotection conditions are already well known to those skilled in the art.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific methods used may be referred to the examples described in the present invention. Of course, other similar isolation and purification methods can also be used. They can be characterized using conventional methods (including physical constants and spectral data).

The purity analysis method is as follows: a Kinetex EVO C18 (50$\times$4.6 mm, 5 $\mu$m, 100Å) chromatographic column is used, acetonitrile-water is used as the mobile phase for gradient elution, the flow rate is 1.5 mL/min, and the detection wavelength is 220 nm.

MS is determined on a LC (Agilent 1260 Infinity II)/MS (G6125B single quadrupole) mass spectrograph (manufacturer: Agilent) (Photodiode Array Detector).

The structures of the compounds are identified by hydrogen nuclear magnetic resonance, and the equipment model is WNMR-I-400MHz.

Preparative liquid chromatography is determined using an Agilent 1260 Infinity II high performance liquid

chromatograph (manufacturer: Agilent). The chromatographic column is Daisogel C18 10 μm 100A (30 mm×250 mm), and the mobile phase is acetonitrile/water.

GF254 silica gel plates from Qingdao Haiyang Chemical are used for thin-layer chromatography (TLC). For the silica gel plates used, the specification is 0.20 mm to 0.25 mm for the thin-layer chromatography for monitoring of reactions, and 0.5 mm for the thin-layer chromatography for separation and purification.

Silica gels of 100 to 200 mesh, 200 to 300 mesh and 300 to 400 mesh from Qingdao Haiyang Chemical are used as a carrier for silica gel column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Shanghai Innochem Science & Technology, Energy Chemical, Shanghai Bide Pharmatech and the like.

Unless specifically stated in the examples, all reactions are carried out under nitrogen atmosphere.

Nitrogen atmosphere means that a reaction flask is connected to a nitrogen balloon with a volume of about 1 L.

The reaction solvent, organic solvent or inert solvent is each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP).

Unless specifically stated in the examples, a solution refers to an aqueous solution.

The chemical reactions described in the present invention are generally carried out under normal pressure. The reaction time and conditions are, for example, between -78°C and 200°C at one atmosphere, and completed within about 1 to 24 hours. If the reaction is carried out overnight, the reaction time is generally 16 hours. Unless specifically stated in the examples, the reaction temperature is room temperature, which is 20°C to 30°C.

Unless otherwise defined, all professional and scientific terms used herein have the same meaning as those familiar to those skilled in the art. In addition, any methods and materials similar or equivalent to the content described herein can be applied to the methods of the present invention.

Unless specifically stated, the mixing ratios of different solvents are volume ratios.

Unless specifically stated, aqueous KF solution in the examples is a saturated solution.

Synthesis of key Intermediate A1

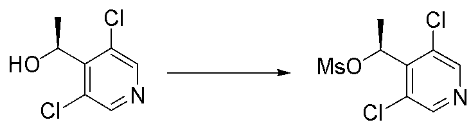

A1-1 A1

Compound A1-1 (10 g, 52.1 mmol) was dissolved in 100 mL of dichloromethane, TEA (8.0 mL, 57.3 mmol) was added, and cooled to 0°C, and then MsCl (6.23 g, 53.7 mmol) was slowly added dropwise. After the dropwise addition was completed, the reaction was carried out at room temperature for 5 h. LCMS monitoring showed no remaining raw materials, and the reaction solution was washed once with aqueous ammonium chloride solution and then once with water. The organic phase was dried over anhydrous sodium sulfate, filtered and subjected to rotary drying to obtain a white solid A1(13.5 g, 95% yield, 98% purity).

Synthesis of key Intermediate A2

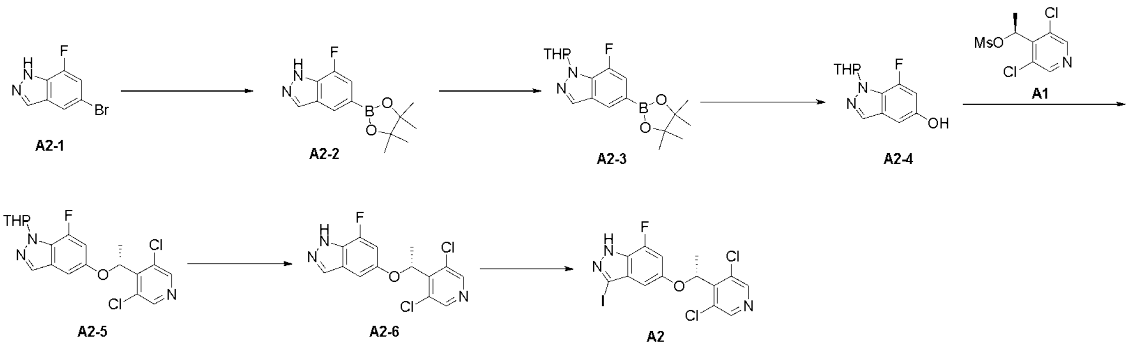


A2-1 A2-2 A2-3 A2-4 A2-5 A2-6 A2

Compound A2-1(5.0 g, 23.25 mmol, 1.0 eq) was dissolved in dioxane (80 mL), and KOAc (4.6 g, 46.51 mmol, 2.0 eq), bis(pinacolato)diboron (12 g, 46.51 mmol, 2.0 eq) and Pd(dppf)$Cl_2$ (1.9 g, 2.3 mmol, 0.1 eq) were added, and reacted at 85°C for 12 hours under $N_2$ protection. LCMS showed the generation of a product. The reaction mixture was concentrated, then mixed with silica gel, and purified by silica gel column chromatography (eluent: $CH_3OH/CH_2Cl_2$=0~5%) to obtain 6.5 g of a crude Compound A2-2 as a white oil, which was directly used for the next reaction step.

Compound A2-2 (6.5 g, 24.80 mmol, 1.0 eq) was dissolved in THF (70 mL), and DHP (4.2 g, 49.60 mmol, 2.0 eq) and TsOH (2.1 g, 12.40 mmol, 0.5eq) were added, and reacted at 60°C for 4 hours. LCMS showed the generation of a product. The reaction mixture was concentrated, then mixed with silica gel, and purified by silica gel column chromatography (eluent: $CH_3OH/CH_2Cl_2$=0~3%) to obtain 6.4 g of a crude Compound A2-3 as a white oil, which was directly used for the next reaction step.

Compound A2-3 (6.4 g, 18.49 mmol, 1.0 eq) was dissolved in THF/$H_2O$ (80 mL), and NaOH (1.1 g, 27.74 mmol, 1.0 eq) and $H_2O_2$ (6 mL) were added thereto in an ice bath, and reacted under stirring at room temperature for 3 hours. LCMS showed the generation of a product. The reaction solution was concentrated, then dissolved in DCM, mixed with silica gel, subjected to rotary drying, and then passed through a normal-phase column to obtain 4.9 g of a crude Compound A2-4 as a white oil, which was directly used for the next reaction step.

Compound A2-4 (4.9 g, 20.74 mmol, 1.0 eq) and Compound A1(8.6 g, 32 mmol, 1.5 eq) were dissolved in ACN (50 mL), and $C_{S2}CO_3$ (20 g, 62.22 mmol, 3.0 eq) was added, and reacted under stirring at 80°C for 4 hours. LCMS showed the generation of a product. 50 mL of ice water was added to the reaction mixture, and then a total of 200 mL of EA was added in three batches to extract the reaction mixture three times. The organic phases were combined, dried over anhydrous sodium sulfate, subjected to rotary drying, mixed with silica gel, and purified by silica gel column chromatography (eluent: $CH_3OH/CH_2Cl_2$=0~4%) to obtain 4.3 g of a crude Compound A2-5 as a white solid, which was directly used for the next reaction step.

Compound A2-5 (4.3 g, 10.48 mmol, 1.0 eq) was dissolved in MeOH (20 mL), HCl/EA (30 mL) was added thereto in an ice bath, and stirred at room temperature for 2 hours. LCMS showed the generation of a product. The reaction solution was neutralized with saturated $NaHCO_3$ solution, and then extracted with DCM. The organic phase was subjected to rotary drying to obtain 3.8 g of a crude Compound A2-6 as a white solid, which was directly used for the next reaction step.

Compound A2-6 (3.8 g, 11.65 mmol, 1.0 eq) was dissolved in DMF (50 mL), NIS (5.2 g, 23.30 mmol, 2.0 eq) was added thereto in an ice bath, and stirred at room temperature for 12 hours. LCMS showed the generation of a product. 50 mL of water was added to the reaction solution, 150 mL of EA was added in three batches to extract the reaction solution three times, and then a total of 100 mL of saturated NaCl solution was added in two batches to wash the organic phase twice. The organic phase was dried and concentrated, then mixed with silica gel, and purified by silica gel column chromatography (eluent: $CH_3OH/CH_2Cl_2$=0~3%) to obtain 4.5 g of Compound A2 as a white solid (the total yield of the six steps: 42.7%).

Synthesis of key Intermediate A3

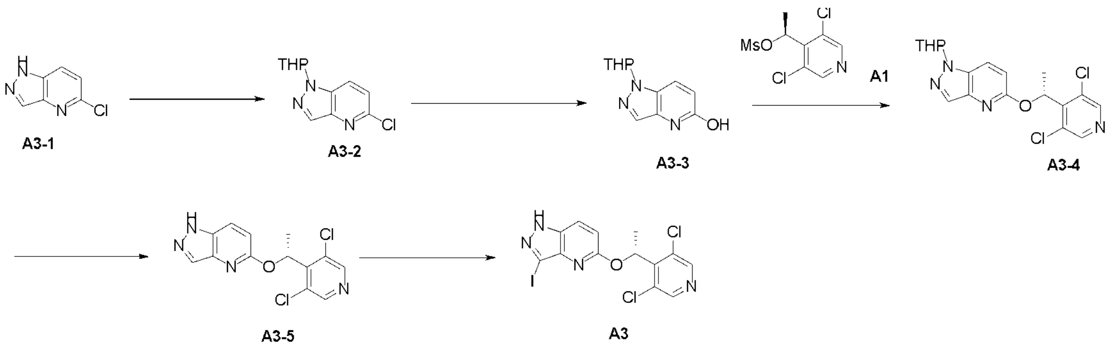


Compound A3-1 (15.0 g, 97.9 mmol, 1.0 eq), DHP (16.5 g, 196 mmol, 2.0 eq) and $TsOH.H_2O$ (1.9 g, 9.8 mmol, 0.1 eq) were dissolved in THF (10 mL), and stirred at 60°C for 8 hours. LCMS showed the generation of a product. 100 mL of water was added to the reaction mixture, and then EA (100 mL×3 ) was added to extract and wash the reaction mixture. The organic phases were combined, dried over anhydrous sodium sulfate, and purified by a silica gel column to obtain 21.0 g of Compound A3-2 as a white solid, with a yield of 90.1%.

Compound A3-2 (11 g, 46.4 mmol, 1.0 eq) was added to a 500 mL single-neck flask, then KOH (5.2 g, 927 mmol, 2.0 eq), $H_2O$ (10 mL), DMF (100 mL) and $Pd_2(dba)_3$ (1.0 g, 4.64 mmol, 0.1 eq) were added, purged with $N_2$, and then stirred at 80°C for 14 h. LCMS showed the generation of a product. The reaction solution was cooled, subjected to rotary drying, mixed with silica gel, and purified by a silica gel column (PE/EA=1:1) to obtain 4.6 g of Compound A3-3 as a white solid, with a yield: 45.2%.

Compound A3-3 (4.6 g, 21 mmol, 1.0 eq), Compound A2 (8.6 g, 32 mmol, 1.5 eq) and $C_{S2}CO_3$ (20.3 g, 63 mmol, 3.0 eq) were dissolved in ACN (50 mL), and stirred at 80°C for 8 hours. LCMS showed the generation of a product. Water (30 mL) was added to the reaction mixture, and then EA (30 mL×3) was added to extract and wash the reaction mixture. The organic phases were combined, dried over anhydrous sodium sulfate, and purified by a silica gel column to obtain 4.3 g of Compound A3-4 as a white solid, with a yield of 52.4%.

Compound A3-4 (4.3 g, 11 mmol, 1.0 eq) was added to a 25 mL single-neck flask, 4M HCl/EA (50 mL) was added, and stirred at room temperature for 3 h. LCMS showed the generation of a product. The reaction solution was subjected to rotary drying and passed through a reversed-phase column to obtain 4.2 g of a crude Compound A3-5 as a white solid, which was directly used for the next reaction step.

Compound A3-5 (4.2 g, 13.6 mmol, 1.0 eq) and NIS (3.6 g, 20.5 mmol, 1.5 eq) were dissolved in DMF (40 mL), and stirred at 25°C for 8 hours. LCMS showed the generation of a product. Water (50 mL) was added to the reaction mixture, and then EA (50 mL×3) was added. The organic phases were combined, dried over anhydrous sodium sulfate, subjected to rotary drying, mixed with silica gel, and purified by silica gel column chromatography (eluent: $CH_3OH/CH_2Cl_2$=0~5%) to obtain 3.0 g of A3 as a white solid, with a yield of 50.8%.

Synthesis of key Intermediate A4

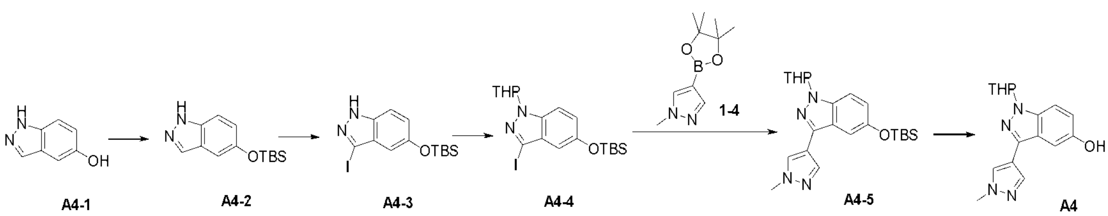


Compound A4-1 (20 g, 149 mmol, 1.0 eq) and imidazole (20.3 g, 298 mmol, 2 eq.) were dissolved in MeCN (400 mL), and TBSCl (44.9 g, 298.2 mmol, 2 eq.) was added at 0°C. The reaction solution was stirred at 20°C for 12 hours. LCMS showed the complete reaction of Compound A4-1. 200 mL of saturated $NaHCO_3$ solution was added, and the reaction solution was extracted with EA, and washed with saturated brine. The organic phase was concentrated to obtain a liquid as a yellow oil, and the crude was purified by a 220 g silica gel column (0-5% EA) to obtain a product A4-2 (35 gg, yield: 94%). LCMS (ESI) m/z 249.1, $(M+H)^+$.

NIS (29.25 g, 169.08 mol, 1.2 eq.) was added to a solution of Compound A4-2 (35 g, 140.9 mmol, 1.0 eq.) in DMF (300 mL) at 0°C, and stirred at room temperature for 2 hours. LCMS showed the complete reaction of Compound A-2 and the generation of a product. Ice water was added to the reaction solution, and the reaction solution was triturated with

petroleum ether, filtered and dried to obtain Compound A4-3 as as yellow solid (50 g, yield 94.8 %). LCMS (ESI) m/z 374.1, $(M+H)^+$.

Compound A4-3 (50 g, 133.58 mmol, 1.0 eq.) and TsOH (2.3 g, 13.4 mmol, 2.0 eq.) were dissolved in DCM (300 mL), and DHP (22.47 g, 267.2 mmol, 2 eq.) was added dropwise at 0°C. The reaction was carried out at 25°C for 4 h. LCMS showed the complete reaction of Compound A4-3 and the generation of a product A4-4. Aqueous $NaHCO_3$ solution was added to the reaction mixture, and the reaction mixture was extracted with DCM. The organic phase was separated and dried by rotary drying to obtain a liquid Compound A4-4 as a yellow oil (30 mg, yield 45.5%, purity 70%), which was directly used for the next reaction step. LCMS (ESI) m/z 459.1, $(M+H)^+$.

Compound A4-4 (40 g, 61.08 mmol, 1.0 eq.), Compound 1-4 (25.4 g, 122 mmol, 2 eq.) and $K_2CO_3$ (25.3 g, 183 mmol, 3 eq.) were dissolved in dioxane (400 mL), and $Pd(dppf)Cl_2$ (4.5 g, 6.11 mmol, 0.1 eq.) was added, purged with $N_2$ three times, and reacted at 90°C for 6 hours. LCMS showed the complete reaction of Compound A4-4 and the generation of a product. The reaction mixture was filtered, followed by the addition of a silica gel powder, subjected to rotary drying, and purified by a 330 g silica gel column to obtain a liquid Compound A4-5 as a brown oil (22 g, yield 61%, purity 70%): LCMS (ESI) m/z 468.23, $(M+H)^+$.

Compound A4-5 (15 g, 25.4 mmol, 1.0 eq.) was dissolved in MeOH (200 mL), and $K_2CO_3$ (7 g, 50.9 mmol, 2 eq.) was added, and reacted at 60°C for 2 hours. LCMS showed the complete reaction of Compound A-6 and the generation of a product. 1M HCl was added to the reaction solution, and the reaction solution was extracted with EtOAc, separated and dried by rotary drying to obtain a crude, which was purified by a 220 g silica gel column to obtain Compound A4 as an off-white solid (7 g, yield 92 %); LCMS (ESI) m/z 299.2 $(M+H)^+$.

Synthesis of key Intermediates A5 and A6

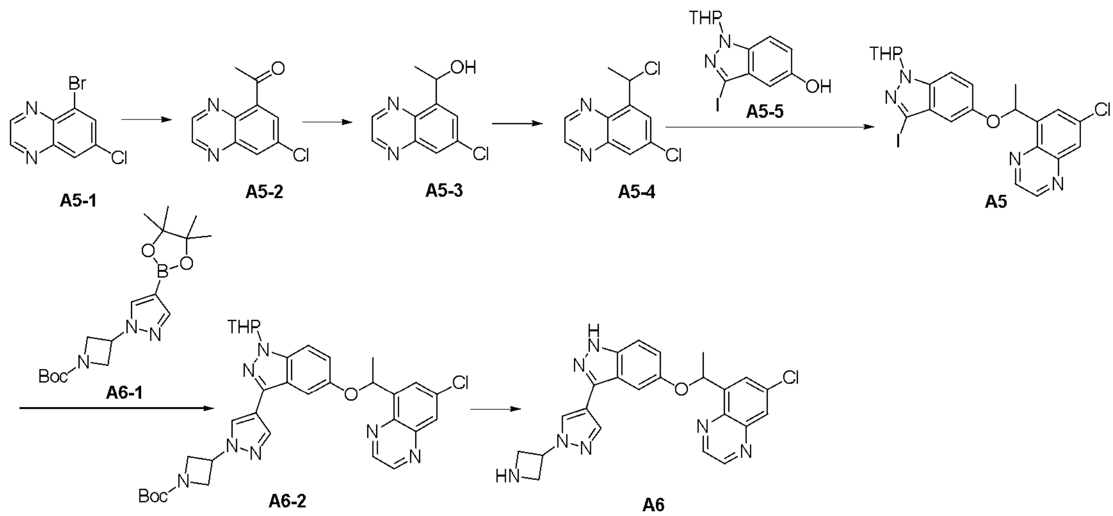


At room temperature, Compound A5-1 (1.2 g, 4.928 mmol) was dissolved in dioxane (12 mL), and tri-n-butyl(1-ethoxyvinyl)tin (2.1 g, 5.91 mmol) was added. Then $Pd(PPh_3)_4$ (569 mg, 0.49 mmol) was added under nitrogen protection. The reaction solution was stirred at 110°C for 48 h. LCMS showed no remaining raw materials. The reaction solution was cooled to room temperature, then quenched with 100 mL of aqueous KF solution, and stirred for 1 hour to generate a solid which was filtered through diatomite. The filtrate was extracted with ethyl acetate (100 mL × 3), and the organic phases were combined and then subjected to rotary drying. The crude was redissolved in ethyl acetate (30 mL), and 10 mL of hydrochloric acid in ethyl acetate (4 M) was added, and stirred at room temperature for 2 hours. LCMS showed a product A5-2. The reaction solution was adjusted to neutral pH with aqueous $Na_2CO_3$ solution, and extracted with ethyl acetate. Then the organic phases were combined and subjected to rotary drying. The crude was purified by silica gel column chromatography (eluent: petroleum ether:ethyl acetate=5:1) to obtain a product A5-2 as a brown solid (310 mg, purity 95%). LCMS (ESI) m/z 207.02, (M+H)+.

Compound A5-2 (310 mg, 1.5 mmol) was dissolved in MeOH (5 mL) at room temperature, and $NaBH_4$ (23 mg, 0.6 mmol) was added at 0°C. The reaction solution was stirred at room temperature for 5 min. LCMS showed the complete reaction of raw materials. The reaction solution was quenched with saturated aqueous $NH_4Cl$ solution, and extracted with DCM (20 mL × 3). The organic phases were combined and then subjected to rotary drying. The crude was purified by silica gel column chromatography (eluent: petroleum ether:ethyl acetate=4:1) to obtain a product A5-3 as a brown oil (259 mg, purity 90% ). LCMS (ESI) m/z 208.04, (M+H)+.

Compound A5-3 (250 mg, 1.2 mmol) was dissolved in DCM (3 mL) at room temperature, and $SOCl_2$ (1 mL) was added at 0°C. The reaction solution was stirred at room temperature for 2 hours. LCMS showed the complete reaction of raw materials. The reaction solution was directly subjected to rotary drying to obtain a crude product A5-4 (263 mg).

Compound A5-4 (263 mg, 1.16 mmol) and Compound A5-5 (400 mg, 1.16 mmol) were dissolved in DMF (4 mL) at room temperature, and $C_{S2}CO_3$ (1.13 g, 3.48 mmol) was added. The reaction solution was stirred at 50°C overnight. LCMS showed the complete reaction of raw materials. The reaction solution was diluted with water and then extracted with ethyl acetate (30 mL x 3). The organic phases were combined and then subjected to rotary drying. The crude was purified by silica gel column chromatography (eluent: petroleum ether:ethyl acetate=3:1) to obtain a product A5 as a yellow oil (533 mg, purity 78%). LCMS (ESI) m/z 535.03, (M+H)+.

Compound A5 (533 mg, 0.777 mmol) and Compound A6-1were dissolved in dioxane /$H_2O$ (6 mL, v/v=5/1) at room temperature, and $K_2CO_3$ (320 mg, 2.33 mmol) was added. Pd(dppf)$Cl_2$.DCM (126 mg, 0.155 mmol) was added under nitrogen protection. The reaction solution was stirred at 80°C for 12 h. LCMS showed the complete reaction of raw materials. The reaction solution was diluted with water and extracted with ethyl acetate (30 mL × 3). The organic phases were combined and subjected to rotary drying. The crude was purified by silica gel column chromatography (eluent: petroleum ether:ethyl acetate=1:1) to obtain a product A6-2 as a brown oil (420 mg, purity 80%). LCMS (ESI) m/z 630.25, (M+H)+.

Compound A6-2 (420 mg, 0.474 mmol) was dissolved in MeOH (5 mL) at room temperature, and HCl (4 mL, 4 M in dioxane) was added thereto in an ice bath. The reaction solution was stirred at room temperature for 3 h. LCMS showed the complete reaction of raw materials. The reaction solution was directly subjected to rotary drying to obtain a product A6 as a brown oil (244 mg, purity 70%, hydrochloride salt). LCMS (ESI) m/z 446.14, (M+H)+.

Synthesis of key Intermediate A7

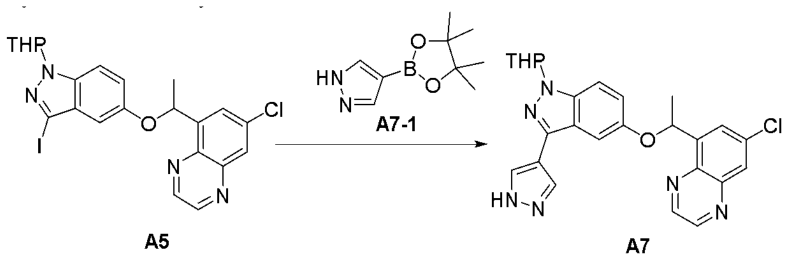


A5 A7

Compound A5 (35 mg, 0.06 mmol, 1.0 eq), and Compound A7-1 (19 mg, 0.09 mmol, 1.5 eq) were dissolved in dioxane (2 mL) and $H_2O$ (2 mL), and then $C_{S2}CO_3$ (64 mg, 0.19 mmol, 3 eq) and Pd(dppf)$Cl_2$ (9.5 mg, 0.013 mmol, 0.2 eq) were added, purged with N2 three times, and stirred at 90°C for 6 hours. LCMS showed the complete reaction of Compound A5. The reaction solution was filtered and subjected to rotary drying, and the crude was purified by a TLC plate (PE:EA=1:9) to obtain Compound A7 (28 mgg, yield: 90%). LCMS (ESI) m/z 475.2, (M+H)+.

Synthesis of key Intermediates A8 and A9

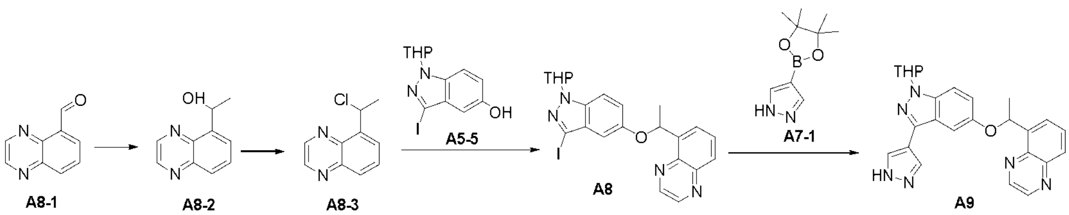


Methyl magnesium bromide (4.9 g, 41.10 mmol, 1.3 eq) was added to a solution of Compound A8-1 (5.0 g, 31.61 mmol, 1.0 eq) in tetrahydrofuran (5 mL) at 0°C, purged with $N_2$ three times, and stirred at room temperature for 1 hour. LCMS showed the generation of a product. The reaction solution was extracted with dichloromethane, washed with saturated brine, dried over anhydrous sodium sulfate, concentrated by rotary drying, and purified by silica gel column chromatography (eluent: petroleum ether:ethyl acetate = 4:1) to obtain a yellowish solid A8-2 (4.7 g, yield 85%): LCMS (ESI) m/z 175.09, (M+H)+.

Thionyl chloride (6.4 mg, 53.96 mmol, 2.0 eq.) was added to a solution of Compound A8-2 (4.7 g, 26.98 mmol, 1.0 eq.) in dichloromethane (40 mL) at 0°C, and stirred at room temperature for 1 hour. LCMS showed the generation of a product. The reaction mixture was extracted with dichloromethane, washed with saturated brine, dried over anhydrous sodium sulfate, and concentrated by rotary drying to obtain a brownish oil A8-3 (5.0 g, yield 96%): LCMS (ESI) m/z 193.05, (M+H)+.

Compound A8-3 (5.0 g, 26.44 mmol, 1.3 eq.), Compound A5-5 (7.0 g, 20.34 mmol, 1.0 eq.) and cesium carbonate (39.77 g, 122.04 mmol, 6 eq.) were dissolved in N,N-dimethylformamide (50 mL), and stirred at 45°C for 6 hours. LCMS showed the generation of a product. The reaction mixture was extracted with dichloromethane, washed with saturated brine, dried over anhydrous sodium sulfate, concentrated by rotary drying, and purified by silica gel column chromatography (eluent: petroleum ether:ethyl acetate= 1:1) to obtain a yellowish solid A8 (7.4 g, yield 73%): LCMS (ESI) m/z 501.34, (M+H)+.

Compound A8 (2 g, 3.997 mmol), Compound A7-1 (7.75 g, 39.97 mmol) and $Na_2CO_3$ (1.69 g, 15.989 mmol) were dissolved in THF/$H_2O$ (20 mL, v/v=3/1) at room temperature. $Pd(PPh_3)_4$ (462 mg, 0.399 mmol) was added under nitrogen protection and purged with nitrogen three times. The reaction solution was stirred at 85°C for 72 h. LCMS showed the complete reaction. The reaction solution was diluted with water and then extracted with ethyl acetate (200 mL × 3). The organic phases were combined and then subjected to rotary drying. The crude was purified by silica gel column chromatography (eluent: dichloromethane:methanol=20:1) to obtain a product A9 as a yellow solid (1.59 g, purity 90%, yield 81.26%). LCMS (ESI) m/z 440.8, (M+H)+.

Example 1

(R)-5-(1-(3,5-dichloropyridin-4-yl)ethoxy)-3-(1-methyl-1H-pyrazol-4-yl)-7-(trifluoromethyl)-1H-indazole

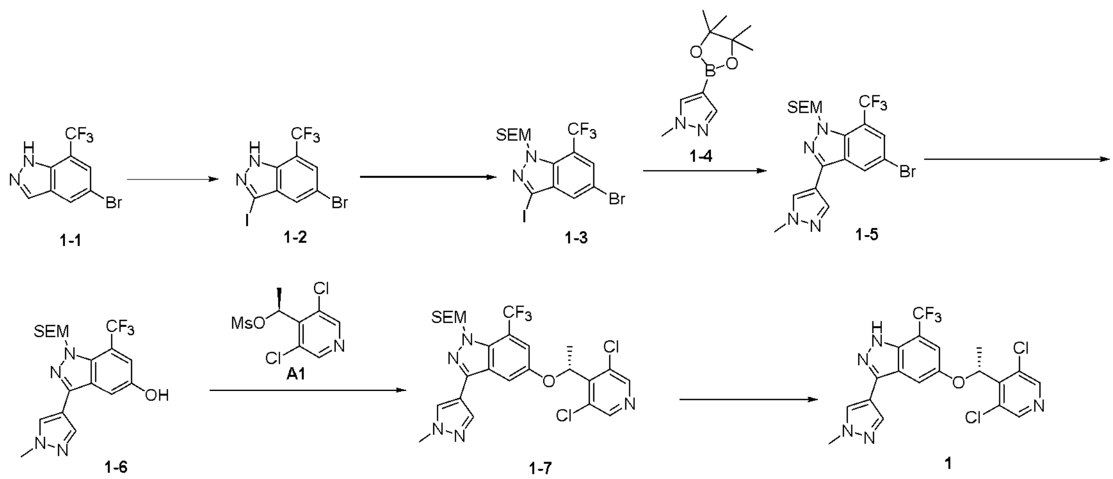
1-1
1-2
1-3
1-4
1-5
1-6
A1
1-7
1

Compound 1-1 (250 mg, 0.94 mmol, 1.0 eq), KOH (307 mg, 5.5 mmol, 5.8 eq) and iodine (479 mg, 1.89 mmol, 2.0 eq) were added successively to dioxane (12.0 mL) at room temperature. The reaction mixture was stirred at 75°C for 16 hours. The reaction solution was cooled to room temperature, followed by the addition of aqueous sodium thiosulfate solution (50.0 mL), and extracted with ethyl acetate (20.0 mL × 2). The organic phases were combined, washed with saturated brine (20.0 mL), dried over anhydrous sodium sulfate, filtered, and concentrated under reduced pressure to obtain Compound 1-2 (425 mg, yield 100%).

At 0°C, a solution of Compound 1-2 (318 mg, 0.813 mmol, 1.0 eq) in THF (3.0 mL) was slowly added to a suspension of sodium hydride (59 mg, 1.46 mmol, 1.8 eq) in THF (5.0 mL). The reaction mixture was warmed up to room temperature, stirred for 0.5 hour, and then cooled to 0°C, and SEMCl (0.19 mL, 1.06 mmol, 1.3 eq) was slowly added thereto. The reaction mixture was warmed up to room temperature, and stirred for 2 hours. The reaction solution was cooled to 0°C, slowly poured into aqueous $NH_4Cl$ solution (30.0 mL), and extracted with EA (20.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/petroleum ether=0-10%) to obtain Compound 1-3 (404 mg, yield 95.3%).

At room temperature, Compound 1-3 (404 mg, 0.775 mmol, 1.0 eq), Compound 1-4 (170 mg, 0.817 mmol, 1.05 eq), $Pd(dppf)Cl_2$ (64 mg, 0.078 mmol, 0.1 eq) and potassium carbonate (320 mg, 2.32 mmol, 3.0 eq) were added successively to dioxane /$H_2O$ (8 mL/ 2 mL). The reaction mixture was stirred at 80°C for 10 hours. The reaction solution was

cooled to room temperature, and concentrated under reduced pressure. The resulting residue was purified by silica gel column chromatography (eluent: ethyl acetate/petroleum ether=0-22%) to obtain Compound 1-5 (245 mg, yield 66.6%).

At room temperature, Compound 1-5 (245 mg, 0.515 mmol, 1.0 eq), $Pd_2(dba)_3$ (141.6 mg, 0.155 mmol, 0.3 eq), t-BuXphos (131 mg, 0.31 mmol, 0.6 eq) and KOH (116 mg, 2.06 mmol, 4.0 eq) were added successively to dioxane /$H_2O$ (5 mL/ 1 mL). The reaction mixture was stirred at 90°C for 16 hours. The reaction solution was cooled to room temperature, and concentrated under reduced pressure. The resulting residue was purified by silica gel column chromatography (eluent: (dichloromethane:methanol=4:1)/dichloromethane=0-20%) to obtain Compound 1-6 (188 mg, yield 86.8%).

At room temperature, Compound 1-6 (188 mg, 0.45 mmol, 1.0 eq), Compound A1 (130 mg, 0.48 mmol, 1.1 eq) and cesium carbonate (450 mg, 1.38 mmol, 3.0 eq) were added successively to acetonitrile (5.0 mL). The reaction mixture was stirred at 80°C for 6 hours. The reaction mixture was concentrated under reduced pressure, and the resulting residue was purified by silica gel column chromatography (eluent: (dichloromethane:methanol=4:1)/dichloromethane=0-13%) to obtain Compound 1-7 (200 mg, yield 74.9%).

At room temperature, TFA (2.0 mL) was slowly added to a solution of Compound 1-7 (200 mg, 0.34 mmol, 1.0 eq) in DCM (6.0 mL). The reaction mixture was stirred at room temperature for 3 hours. The reaction mixture was concentrated under reduced pressure, the resulting residue was dissolved in THF (5.0 mL), and ammonia water was added to adjust pH to >10. The reaction solution was stirred at room temperature for 0.5 hour. Water (10.0 mL) was added, and the reaction solution was extracted with DCM (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-15%) to obtain Compound 1 (75 mg, yield 48.4%, purity 100%). LCMS (ESI) m/z 456.1/458.1/460.1 (M+H)+; $^1$H NMR (400 MHz, DMSO) δ 13.43 (s, 1H), 8.63 (s, 2H), 8.24 (s, 1H), 7.90 (s, 1H), 7.54 (s, 1H), 7.43 (s, 1H), 6.27 (q, J = 6.6 Hz, 1H), 3.98 (s, 3H), 1.81 (d, J = 6.6 Hz, 3H).

The following compounds were synthesized according to the the route of Example 1.

| Example | Name | Structure | LC-MS (M+H)$^+$ | $^1$H NMR |
|---|---|---|---|---|
| 3 | (R)-7-chloro-5-(1-(3,5-dichloropyridin-4-yl) ethoxy)-3-(1-methyl-1H-pyrazol-4-yl)-1H-indazole | 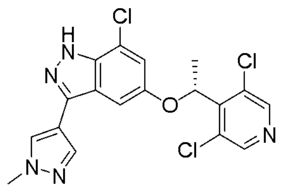 | 454 | $^1$H NMR (400 MHz, DMSO) δ 13.39 (s, 1H), 8.63 (s, 2H), 8.19 (s, 1H), 7.87 (s, 1H), 7.21 (d, J = 7.9 Hz, 2H), 6.20 (q, J = 6.6 Hz, 1H), 3.97 (s, 3H), 1.80 (d, J = 6.6 Hz, 3H). |
| 4 | (R)-5-(1-(3,5-dichloropyridin-4-yl)ethoxy)-7-methoxy-3-(1-methyl-1H-pyrazol-4-yl)-1H-indazole | 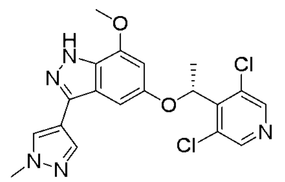 | 450 | $^1$H NMR (400 MHz, DMSO) δ 13.09 (s, 1H), 8.63 (s, 2H), 8.07 (s, 1H), 7.79 (s, 1H), 6.64 (s, 1H), 6.61 (s, 1H), 6.18 - 6.08 (m, 1H), 3.96 (s, 3H), 3.95 (s, 3H), 1.79 (d, J = 6.6 Hz, 3H). |
| 5 | (R)-5-(1-(3,5-dichloropyridin-4-yl)ethoxy)-7-fluoro-3-(1-methyl-1H-pyrazol-4-yl)-1H-indazole | 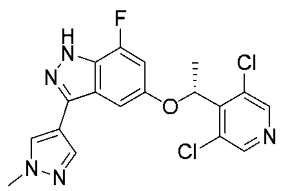 | 406 | $^1$H NMR (500 MHz, DMSO) δ 8.61 (s, 2H), 8.13 (s, 1H), 7.82 (d, J = 0.5 Hz, 1H), 7.02 - 6.91 (m, 2H), 6.16 (q, J = 6.6 Hz, 1H), 3.94 (s, 3H), 1.76 (d, J = 6.6 Hz, 3H). |

(continued)

| Example | Name | Structure | LC-MS (M+H)+ | 1H NMR |
|---|---|---|---|---|
| 6 | (R)-5-(1-(3,5-dichloropyridin-4-yl)ethoxy)-7-isopropyl-3-(1-methyl-1H-pyrazol-4-yl)-1H-indazole | 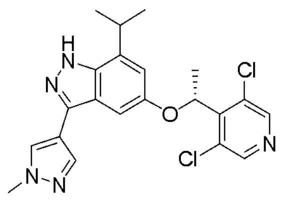 | 430 | $^1$H NMR (400 MHz, DMSO-d6) δ 12.96 (s, 1H), 8.61 (s, 2H), 8.11 (s, 1H), 7.80 (s, 1H), 6.93 (dd, J = 24.0, 4.0 Hz, 2H), 6.12 (q, J = 6.0 Hz, 1H), 4.52 (s, 1H), 3.95 (s, 3H), 1.78 (d, J = 8.0 Hz, 3H), 1.30 - 1.26 (m, 6H). |
| 25 | (R)-4-(2-(4-(5-(1-(3,5-dichloropyridin-4-yl) ethoxy)-7-methoxy-1H-indazol-3-yl)-1H-pyrazol-1-yl)ethyl)morphol ine | 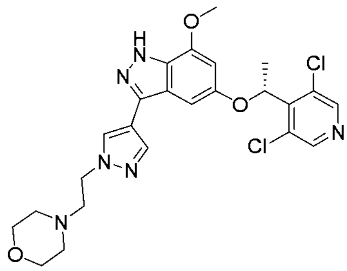 | 518 | $^1$H NMR (400 MHz, DMSO-d6) δ 13.30 (s, 1H), 8.62 (s, 2H), 8.25 (s, 1H), 8.18 (s, 1H), 7.98 (s, 1H), 6.36 (s, 1H), 5.86 (q, J=6.0 Hz, 1H), 4.33 (t, J = 6.0 Hz, 2H), 3.82 (s, 3H), 3.61 - 3.57 (m, 4H), 2.80 (t, J = 6.0 Hz, 2H), 2.47 (s, 4H), 1.78 (d, J = 8.0 Hz, 3H). |

Example 2

(R)-5-(1-(3,5-dichloropyridin-4-yl)ethoxy)-7-methyl-3-(1-methyl-1H-pyrazol-4-yl)-1H-indazole

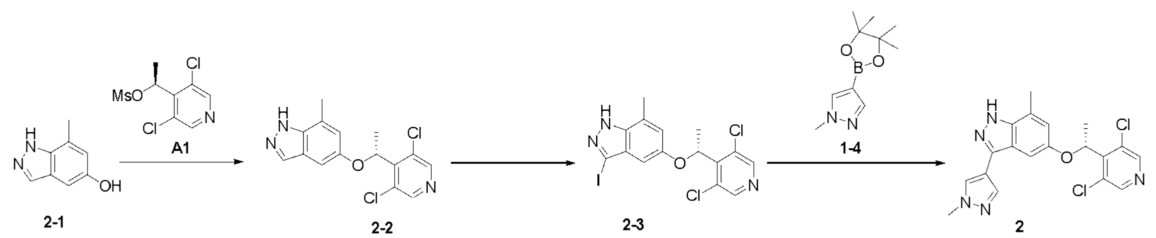
2-1 A1 2-2 2-3 1-4 2

Compound 2-1 (350mg, 2.36 mmol, 1.0 eq), Compound A1 (941mg, 3.5 mmol, 1.5 eq) and $C_{S2}CO_3$ (2.3g, 7.0 mmol, 3.0 eq) were dissolved in ACN (8 mL), and stirred at 80°C for 8 hours. LCMS showed the generation of a product. 30 mL of water was added to the reaction mixture, and then 30 mL of EA was added to wash and extract the reaction mixture three times. The organic phases were combined, dried over anhydrous sodium sulfate, and purified by a silica gel column to obtain 270 mg of Compound 2-2 as a white solid, with a yield of 35.5%.

Compound 2-2 (270mg, 0.84 mmol, 1.0 eq), $I_2$ (324mg, 1.26 mmol, 1.5 eq) and KOH (190 mg, 1.69 mmol, 2.0 eq) were dissolved in DMF (10 mL), and stirred at 25°C for 8 hours. LCMS showed the generation of a product. 50 mL of water was added to the reaction mixture, and then 50 mL of EA was added to extract the reaction mixture three times. The organic phases were combined, dried over anhydrous sodium sulfate, subjected to rotary drying, mixed with silica gel, and purified by a silica gel column (eluent: petroleum ether/ethyl acetate=3/1) to obtain 120 mg of Compound 2-3 as a white solid, with a yield of 31.9%.

Compound 2-3 (120 mg, 0.27 mmol, 1.0 eq), Compound 1-4 (86 mg, 0.41 mmol, 1.5 eq), $C_{S2}CO_3$ (270 mg, 0.81 mmol, 3.0 eq) and Pd(dppf)$Cl_2$ (40 mg, 0.027 mmol, 0.1 eq) were dissolved in dioxane (5 mL), and then 1 mL of water was added, purged with nitrogen, and stirred at 80°C for 8 hours. LCMS showed the generation of a product. 30 mL of water was added to the reaction mixture, and then 30 mL of EA was added to wash and extract the reaction mixture three times. The organic phases were combined, dried over anhydrous sodium sulfate, and purified by a silica gel column followed by a reversed-phase column to obtain 18.6 mg of Compound 2 as a white solid (yield: 17.4%, purity 95.5%). LCMS (ESI) m/z 402.08, (M+H)+; $^1$H NMR (400 MHz, DMSO) δ 8.59 (s, 2H), 8.08 (s, 1H), 7.80 (s, 1H), 6.91 (s, 1H), 6.88 (s, 1H), 6.10 (q, J = 6.6 Hz, 1H), 3.94 (s, 3H), 2.44 (s, 3H), 1.75 (d, J = 6.6 Hz, 3H).

The following compounds were synthesized according to the the route of Example 2.

| Example | Name | Structure | LC-MS (M+H)+ | $^1$H NMR |
|---|---|---|---|---|
| 26 | (R)-3-(1-(cyclopropylmethyl)-1H-pyrazol-4-yl)-5-(1-(3,5-dichloropyridin-4-yl)ethoxy)-7-methyl-1H-indazole | 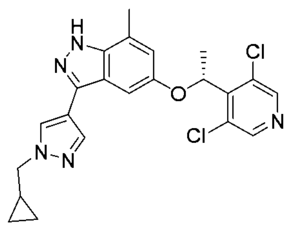 | 443 | $^1$H NMR (400 MHz, DMSO-d6) δ 12.93 (s, 1H), 8.60 (s, 2H), 8.16 (s, 1H), 7.83 (s, 1H), 6.93 (d, J = 16.0 Hz, 2H), 6.14 (q, J = 6.0 Hz, 1H), 4.07 (d, J = 4.0 Hz, 2H), 2.47 (s, 3H), 1.77 (d, J = 8.0 Hz, 3H), 1.37 - 1.31 (m, 1H), 0.65 - 0.56 (m, 2H), 0.45 (q, J = 4.0 Hz, 2H). |
| 27 | (R)-4-(2-(4-(5-(1-(3,5-dichloropyridin-4-yl) ethoxy)-7-methyl-1H-indazol-3-yl)-1H-pyrazol-1-yl) ethyl)morphol ine | 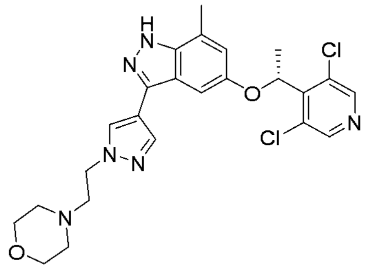 | 501 | $^1$H NMR (400 MHz, DMSO-d6) δ 12.94 (s, 1H), 8.61 (s, 2H), 8.16 (s, 1H), 7.83 (s, 1H), 6.93 (d, J = 16.0 Hz, 2H), 6.13 (q, J = 6.6 Hz, 1H), 4.34 (t, J = 6.0 Hz, 2H), 3.63 - 3.53 (m, 4H), 2.81 (t, J = 8.0 Hz, 2H), 2.48 (d, J = 4.0 Hz, 7H), 1.78 (d, J = 4.0 Hz, 3H). |

Example 7

(R)-7-cyclopropyl-5-(1-(3,5-dichloropyridin-4-yl)ethoxy)-3-(1-methyl-1H-pyrazol-4-yl)-1H-indazole

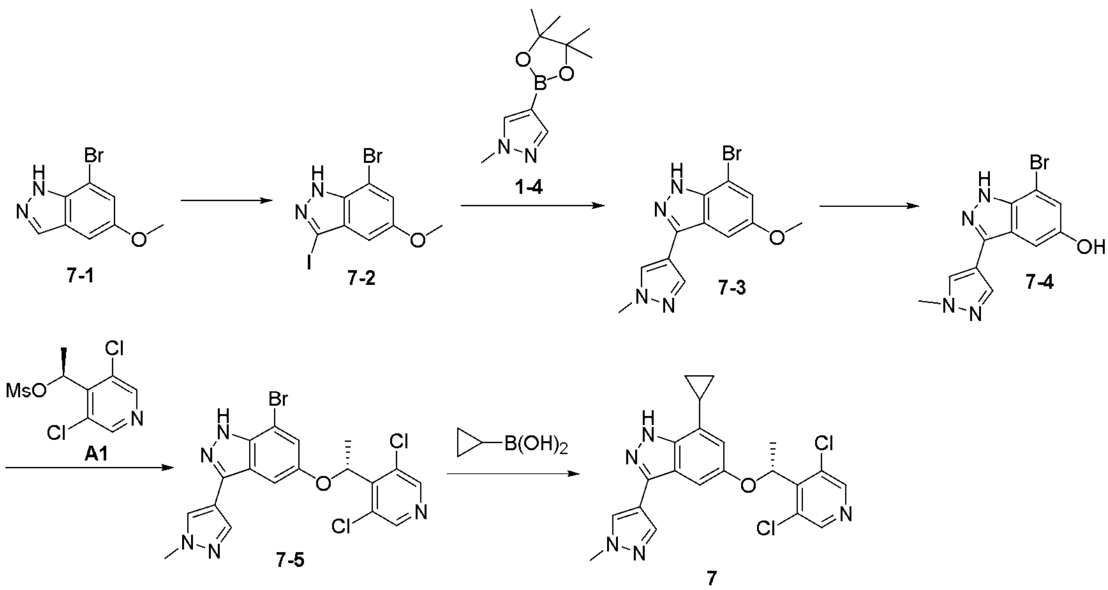


At room temperature, NIS (1.486 g, 6.6 mmol) was added to a solution of Compound 7-1 (1.0 g, 4.4 mmol) in N,N-dimethylformamide (40.0 mL). The resulting mixture was stirred at 50°C for 4 hours. LCMS showed the generation of a product. The reaction mixture was diluted with ethyl acetate (100.0 mL), washed successively with saturated aqueous

sodium bicarbonate solution (40.0 mL) and water (40.0 mL × 2), dried over anhydrous sodium sulfate, and filtered. The filtrate was concentrated to dryness under reduced pressure. The resulting residue was purified by silica gel column chromatography (eluent: petroleum ether/ethyl acetate=4/1, v/v) to obtain Compound 7-2 as a yellow solid (1.362 g, yield: 88%, purity: 95.03%). LCMS (ESI) m/z 352.9 (M+H)+.

At room temperature, Compound 7-2 (1.059 g, 3.0 mmol), Compound 1-4 (0.811 g, 3.9 mmol), Pd(dppf)$Cl_2 \cdot CH_2Cl_2$ (0.246 g, 0.30 mmol) and $K_2CO_3$ (0.829 g, 6.0 mmol) were added to a mixture of 1,4-dioxane (15.0 mL) and water (7.5 mL). The resulting mixture was stirred at 100°C for 15 hours. LCMS showed the generation of a product. The reaction mixture was cooled to room temperature, and diluted with dichloromethane (20.0 mL) and water (10.0 mL). The organic phase was separated, and the aqueous phase was extracted with dichloromethane (10.0 mL × 4). The organic phases were combined, dried over anhydrous sodium sulfate, and filtered. The filtrate was concentrated to dryness under reduced pressure. The resulting residue was purified by silica gel column chromatography (eluent: dichloromethane/-methanol=60/1, v/v) to obtain Compound 7-3 as a light brown solid (0.719 g, yield: 78%, purity: 100%). LCMS (ESI) m/z 307.1 (M+H)+.

At room temperature, Compound 7-3 (0.412 g, 1.34 mmol) was added to dichloromethane (10.0 mL), and then a solution of boron tribromide in dichloromethane (2.0 M) (3.4 mL, 6.7 mmol) was slowly added dropwise. The resulting mixture was stirred at room temperature for 18 hours. LCMS showed the generation of a product. The pH of the reaction mixture was adjusted to about 7 to 8 with saturated aqueous sodium bicarbonate solution. The organic phase was separated, and the aqueous phase was extracted with a mixture of dichloromethane and methanol (dichloromethane/-methanol=5/1, v/v) (10.0 mL x 5). The organic phases were combined, dried over anhydrous sodium sulfate, and filtered. The filtrate was concentrated to dryness under reduced pressure. The resulting residue was purified by silica gel column chromatography (gradient elution, eluent: dichloromethane/methanol=50/1-10/1, v/v) to obtain a dark brown solid (0.48 g). The solid was added to ethanol (1.5 mL), stirred at 80°C for 30 minutes, and cooled to room temperature. The resulting mixture was filtered under reduced pressure, and the filter cake was washed with ethanol (1.0 mL × 2), and dried under vacuum to obtain Compound 7-4 as a brown solid (0.362 g, yield: 92%, purity: 96.3%). LCMS (ESI) m/z 293.0 (M+H)+.

At room temperature, Compound 7-4 (0.362 g, 1.23 mmol), Compound A1 (0.332 g, 1.23 mmol) and cesium carbonate (0.240 g, 0.74 mmol) were added to N,N-dimethylformamide (3.1 mL). The resulting mixture was stirred at 65°C for 18 hours. LCMS showed the generation of a product. The reaction mixture was cooled to room temperature, diluted with ethyl acetate (15.0 mL), then washed with water (10.0 mL × 3), dried over anhydrous sodium sulfate, and filtered. The filtrate was concentrated to dryness under reduced pressure. The resulting residue was purified by silica gel column chromatography (gradient elution, eluent: dichloromethane/methanol=50/1-30/1, v/v) to obtain Compound 7-5 as a light brown solid (0.303g, yield: 53%, purity: 64.89%). LCMS (ESI) m/z 466.0 (M+H)+.

At room temperature, Compound 7-5 (0.0467 g, 0.10 mmol), cyclopropylboronic acid (0.0146 g, 0.17 mmol), palladium acetate (0.0033 g, 0.015 mmol), tricyclohexylphosphine (0.0084 g, 0.03 mmol) and potassium carbonate (0.0276 g, 0.20 mmol) were added to a mixture of 1,4-dioxane (0.5 mL) and water (0.25 mL). The resulting mixture was stirred at 100°C for 20 hours. LCMS showed the generation of a product. The reaction mixture was cooled to room temperature, and diluted with dichloromethane (10.0 mL) and water (5.0 mL). The organic phase was separated, and the aqueous phase was extracted with dichloromethane (5.0 mL × 3). The organic phases were combined, dried over anhydrous sodium sulfate, and filtered. The filtrate was concentrated to dryness under reduced pressure. The resulting residue was purified by thin-layer chromatography (developping agent: dichloromethane/methanol=30/1, v/v) to obtain Compound 7 as a yellow solid (0.0165 g, yield: 38%, purity: 92.68%). LCMS (ESI) m/z 428.0, (M+H)+; $^1$H NMR (400 MHz, DMSO) δ 13.06 (s, 1H), 8.61 (s, 2H), 8.12 (s, 1H), 7.82 (s, 1H), 6.94 (s, 1H), 6.57 (d, J = 2.0 Hz, 1H), 6.11 (q, J = 6.6 Hz, 1H), 3.96 (s, 3H), 2.30 - 2.20 (m, 1H), 1.77 (d, J = 6.6 Hz, 3H), 1.07 (d, J = 2.2 Hz, 1H), 1.05 (d, J = 2.4 Hz, 1H), 0.85 - 0.78 (m, 1H), 0.74 - 0.67 (m, 1H).

The following compound was synthesized according to the the route of Example 7.

| Example | Name | Structure | LC-MS $(M+H)^+$ | $^1$H NMR |
|---|---|---|---|---|
| 8 | (R)-5-(1-(3,5-dichloropyridin-4-yl) ethoxy)-3-(1-methyl-1H-pyrazol-4-yl)-1H-indazole-7-formonitrile | 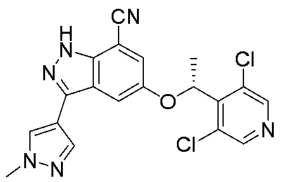 | 413 | $^1$H NMR (400 MHz, DMSO) δ 13.82 (s, 1H), 8.64 (s, 2H), 8.27 (s, 1H), 7.93 (s, 1H), 7.68 (s, 1H), 7.66 (s, 1H), 6.28 (q, J = 6.2 Hz, 1H), 3.99 (s, 3H), 1.81 (d, J = 6.6 Hz, 3H). |

Example 9

(R)-5-(1-(3,5-dichloropyridin-4-yl)ethoxy)-7-methyl-3-(4-methyl- 1H-imidazol-2-yl)-1H-indazole

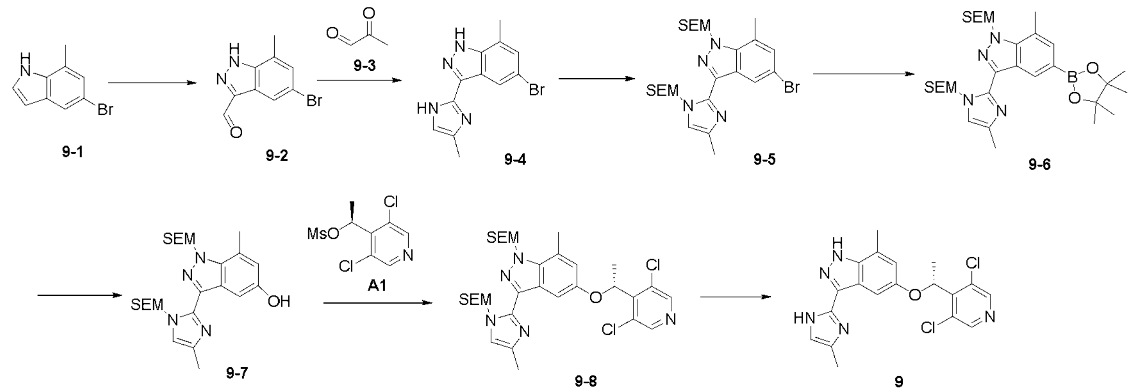


$NaNO_2$ (2.628 g, 38 mmol, 8.0 eq) was dissolved in water (30 mL) to obtain Solution 1, hydrogen chloride solution (38 mmol) was added to Solution 1, reacted under stirring at 0°C for 10 min, and then a solution of Compound 9-1 (1 g, 4.76 mmol, 1.0 eq) in DMF (50 mL) was slowly added to Solution 1, and reacted at room temperature for 3 hours. LCMS showed the generation of a product. The reaction mixture was cooled, extracted with EA and water three times, and the extract was concentrated under vacuum to obtain a crude Compound 9-2 which was directly used for the next reaction step. LCMS (ESI) m/z 238.97, (M+H)+.

7N ammonia water-methanol solution (7 mL) was added to a solution of the crude Compound 9-2 in $CH_3OH$ (40 mL) at 0°C, reacted under stirring for 10 min, and then Compound 9-3 (5 mL, 5.0 eq) was slowly added, and reacted at room temperature for 3 hours. LCMS showed the generation of a product. The reaction mixture was cooled and concentrated under vacuum, and then the residue was purified by silica gel column chromatography (eluent: $CH_3OH$:DCM=1:10) and concentrated to obtain Compound 9-4 (416 mg, yield 30.5%): LCMS (ESI) m/z 291.02 (M+H)+.

NaH (120 mg, 3.0 mmol, 2.0 eq) was added to Compound 9-4 (416 mg, 1.43 mmol, 1.0 eq) in THF (15 mL) at 0°C, purged with nitrogen for replacing air, and reacted at room temperature for 40 min. Then SEMCl (0.66 mL, 1.3 eq) was added, and reacted at room temperature for 3 h. LCMS showed the generation of a product. The reaction solution was directly concentrated under vacuum, and the mixture was cooled, extracted with EA and water three times, and washed with saturated brine. The residue was purified by silica gel column chromatography (eluent: PE:EA=10:1) and concentrated to obtain Compound 9-5 (450 mg, yield 52.6%): LCMS (ESI) m/z 551.18, (M+H)+.

Potassium acetate (321 mg, 3.28 mmol, 4.0 eq) was added to a solution of Compound 9-5 (450 mg, 0.82 mmol, 1.0 eq), $Pd(dppf)Cl_2$ (134 mg, 0.162 mmol, 0.2eq) and bis(pinacolato)diboron (622 mg, 2.45 mmol, 3.0 eq) in dioxane (9 mL), and reacted at 90°C for three hours. LCMS showed the generation of a product. The reaction solution was directly concentrated under vacuum, and the residue was filtered through diatomite. The filtrate was concentrated under vacuum to obtain a crude Compound 9-6 (LCMS (ESI) m/z 599.35, (M+H)+) with boronic acid generated as a by-product.

Sodium perborate tetrahydrate (730 mg, 4.8 mmol, 6.0 eq) was added to a solution of the crude Compound 9-6 in THF/$H_2O$ (8 mL/2 mL), and reacted at room temperature for 2 hours. LCMS showed the generation of a product. The reaction solution was directly concentrated under vacuum, and the residue was extracted with DCM and water three times, and washed with saturated brine. The residue was purified by silica gel column chromatography (eluent: DCM:$H_2O$=10:1) and concentrated to obtain a liquid crude Compound 9-7 which was directly used for the next reaction step. LCMS (ESI) m/z 489.26 (M+H)+.

Cesium carbonate (750 mg, 2.0 mmol, 2.5 eq) was added to a solution of the crude Compound 9-7 and Compound A1 (270 mg, 0.96 mmol, 1.2 eq) in DMF (8 mL), and reacted at room temperature for 12 h. LCMS showed the generation of a product. The reaction solution was extracted with EA and water three times, and washed with saturated brine. The residue was purified by silica gel column chromatography (eluent: DCM:$CH_3OH$=15:1) and concentrated to obtain Compound 9-8 (36 mg, yield 6.7% for 3 steps). LCMS (ESI) m/z 662.24, (M+H)+.

2 mL of trifluoroacetic acid was added to a solution of Compound 9-8 (36 mg) in DCM (4 mL), and reacted under stirring at room temperature for 3 h. LCMS showed the generation of a product. The trifluoroacetate salt of Compound 9 (5.3 mg, yield 24.3%, purity 94.45%) was obtained by preparative high performance liquid chromatography. LCMS (ESI) m/z 402.3 (M+H)+; 8.60 (s, 2H), 7.60 (s, 1H),6.90 (s, 1H), 6.87 (s, 1H), 6.10 (q, J = 6.6 Hz, 1H), 2.44 (s, 3H), 2.42 (s, 3H), 1.75 (d, J = 6.6 Hz, 3H).

Example 10

(R)-4-(2-(4-(5-(1-(3,5-dichloropyridin-4-yl)ethoxy)-7-fluoro-1H-indazol-3-yl)-1H-pyrazol-1-yl)ethyl)morpholine

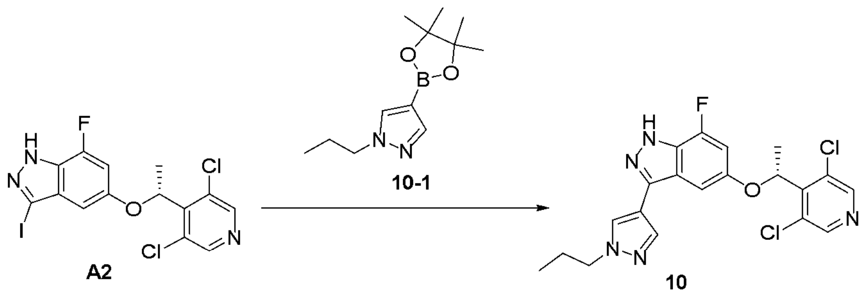


Compound A2 (200 mg, 0.44 mmol, 1.0 eq) and Compound 10-1 (209 mg, 0.88 mmol, 2.0 eq) were dissolved in dioxane /$H_2O$(5mL), and $Cs_2CO_3$ (432 mg, 1.33 mmol, 3.0 eq) and Pd(dppf)$Cl_2$ (33 mg, 0.04 mmol, 0.1eq) were added, and stirred at 85°C for 12 hours under $N_2$ protection. LCMS showed the generation of a product. 5 mL of water was added to the reaction mixture, and then a total of 15 mL of EA was added in three batches to extract the reaction mixture three times. The organic phases were combined, dried over anhydrous sodium sulfate, concentrated, mixed with silica gel, and purified by silica gel column chromatography (eluent: DCM:$CH_3OH$=20:1) to obtain 24.6 mg of Compound 10 as a white solid (yield 12.8%, purity 97.5%). LCMS (ESI) m/z 435.3 (M+H)+; $^1H$ NMR (400 MHz, DMSO) δ 13.41 (s, 1H), 8.59 (s, 2H), 8.16 (s, 1H), 7.83 (s, 1H), 6.97 (s, 2H), 6.16 (d, J = 6.4 Hz, 1H), 4.15 (t, J = 6.4 Hz, 2H), 1.92 - 1.80 (m, 2H), 1.76 (d, J = 6.0 Hz, 3H), 0.87 (t, J = 7.2 Hz, 3H).

The following compounds were synthesized according to the the route of Example 10.

| Example | Name | Structure | LC-MS $(M+H)^+$ | $^1H$ NMR |
|---|---|---|---|---|
| 11 | (R)-5-(1-(3,5-dichloropyridin -4-yl)ethoxy)-7-fluoro-3-(1-(2-(methylsulfonyl ) ethyl)-1H-pyrazol-4-yl)-1H-indazole | 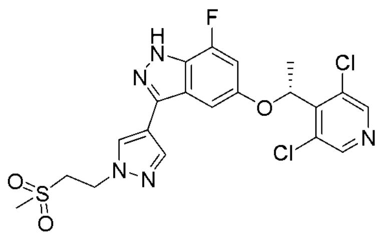 | 498 | $^1H$ NMR (400 MHz, DMSO) δ 13.47 (s, 0H), 8.59 (s, 2H), 8.31 (s, 1H), 7.92 (s, 1H), 7.10 - 6.93 (m, 1H), 6.16 (d, J = 6.76Hz, 1H), 4.66 (t, J = 6.4 Hz, 1H), 3.80 (d, J = 6.4 Hz, 1H), 2.92 (s, 2H), 1.76 (d, J = 5.8 Hz, 2H). |
| 12 | (R)-4-(2-(4-(5-(1-(3,5-dichloropyridin -4-yl) ethoxy)-7-fluoro-1H-indazol-3-yl)-1H-pyrazol-1-yl) ethyl)morph oline | 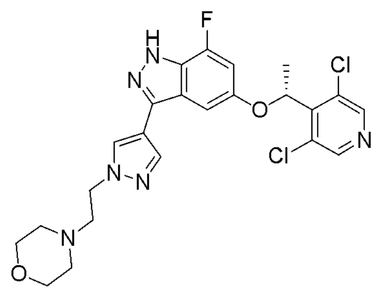 | 505 | $^1H$ NMR (400 MHz, DMSO) δ 13.43 (s, 1H), 8.62 (s, 2H), 8.22 (s, 1H), 7.86 (s, 1H), 7.08 - 6.89 (m, 2H), 6.29 - 6.12 (m, 1H), 4.45 - 4.22 (m, 2H), 3.69 - 3.55 (m, 4H), 2.89 - 2.78 (m, 2H), 2.50 2.44 (m, 4H), 1.79 (d, J = 6.6 Hz, 3H). |

(continued)

| Example | Name | Structure | LC-MS (M+H)+ | 1H NMR |
|---|---|---|---|---|
| 13 | (R)-1-(4-(5-(1-(3,5-dichloropyridin -4-yl)ethoxy)-7-fluoro-1H-indazol-3-yl)-1H-pyrazol-1-yl)piperidin-1-yl) ethan-1-one | 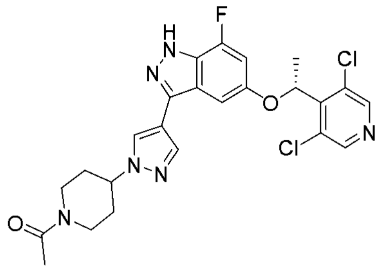 | 517 | $^1$H NMR (400 MHz, DMSO) δ 13.42 (s, 1H), 8.59 (s, 2H), 8.21 (d, J = 6.2Hz, 1H), 7.85 (s, 1H), 6.98 (d, J = 11.6 Hz, 2H), 6.17 (s, 1H), 4.52 (d, J = 11.6 Hz, 2H), 3.97 (d, J = 13.8 Hz, 1H), 3.25 (t, J = 12.6 Hz, 1H), 2.76 (t, J = 12.8 Hz, 1H), 2.17 - 2.04 (m, 5H), 1.98 (s, 2H), 1.76 (d, J = 6.0 Hz, 3H). |
| 14 | (R)-3-(1-(cyclopropylmethyl)-1H-pyrazol-4-yl)-5-(1-(3,5-dichloropyridin -4-yl)ethoxy)-7-fluoro-1H-indazole | 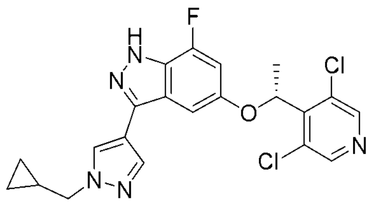 | 446 | $^1$H NMR (400 MHz, DMSO) δ 13.42 (s, 1H), 8.59 (s, 2H), 8.20 (s, 1H), 7.84 (s, 1H), 6.99 (d, J= 10.4 Hz, 2H), 6.17 (d, J = 7.0 Hz, 1H), 4.06 (d, J = 7.2 Hz, 2H), 1.76 (d, J = 5.8 Hz, 3H), 0.85 (s, 1H), 0.57 (d, J = 6.2 Hz, 2H), 0.42 (s, 2H). |
| 15 | (R)-2-(4-(5-(1-(3,5-dichloropyridin -4-yl)ethoxy)-7-fluoro-1H-indazol-3-yl)-1H-pyrazol-1-yl)-N,N-dimethylethan-1-amine | 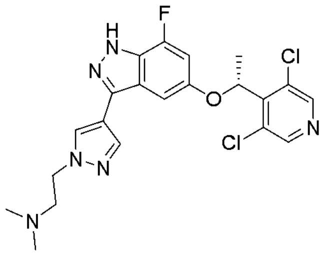 | 463 | $^1$H NMR (400 MHz, DMSO) δ 13.41 (s, 1H), 8.62 (s, 2H), 8.22 (s, 1H), 7.88 (s, 1H), 7.01 (s, 2H), 6.24 - 6.15 (m, 1H), 4.37 (t, J = 6.2 Hz, 2H), 2.91 (s, 2H), 2.35 (s, 6H), 1.79 (d, J = 6.6 Hz, 3H). |
| 16 | (R)-2-(4-(5-(1-(3,5-dichloropyridin -4-yl)ethoxy)-7-fluoro-1H-indazol-3-yl)-1H-pyrazol-1-yl)acetamide | 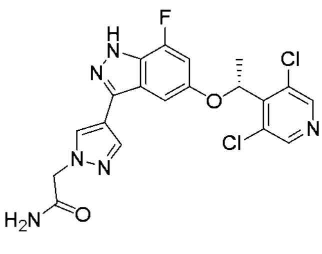 | 449 | $^1$H NMR (400 MHz, DMSO) δ 13.47 (s, 2H), 8.17 (d, J = 4.1 Hz, 2H), 7.87 (d, J = 4.2 Hz, 2H), 7.71 (d, J = 2.1 Hz, 1H), 7.58 (d, J = 16.5 Hz, 1H), 7.34 (d, J = 9.7 Hz, 2H), 7.05 - 6.98 (m, 4H), 6.21 - 6.14 (m, 1H), 4.77 (d, J = 5.5 Hz, 2H). |

(continued)

| Example | Name | Structure | LC-MS (M+H)+ | 1H NMR |
|---|---|---|---|---|
| 17 | (R)-3-(4-(5-(1-(3,5-dichloropyridin -4-yl)ethoxy)-7-fluoro-1H-indazol-3-yl)-1H-pyrazol-1-yl)-N,N-dimethylpropan -1-amine | 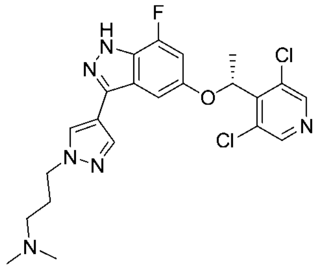 | 477 | 1H NMR (400 MHz, DMSO) δ 13.47 (s, 1H), 8.63 (s, 2H), 8.23 (s, 1H), 7.89 (s, 1H), 7.00 (d, J = 9.8 Hz, 2H), 6.18 (d, J = 6.7 Hz, 1H), 4.29 (t, J = 6.8 Hz, 2H), 2.76 (s, 2H), 2.17 (dd, J = 14.3, 7.5 Hz, 2H), 1.79 (d, J = 6.6 Hz, 3H), 1.33 - 1.21 (m, 6H). |
| 18 | (R)-5-(1-(3,5-dichloropyridin -4-yl)ethoxy)-7-fluoro-3-(1-(2-(tetrahydro-2H-pyran-4-yl)ethyl)-1H-pyrazol-4-yl)-1H-indazole | 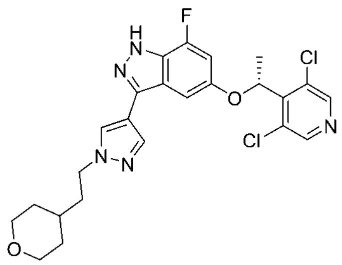 | 505 | 1H NMR (400 MHz, DMSO) δ 13.43 (s, 1H), 8.61 (s, 3H), 8.22 (s, 1H), 7.86 (s, 1H), 7.01 (d, J = 10.9 Hz, 2H), 6.19 (d, J = 6.6 Hz, 1H), 4.26 (t, J = 7.2 Hz, 2H), 3.85 (d, J = 9.2 Hz, 2H), 3.23 (d, J = 5.2 Hz, 3H), 1.86 - 1.76 (m, 6H), 1.65 (d, J = 12.3 Hz, 2H), 1.29 - 1.22 (m, 2H). |
| 19 | (R)-2-(4-(5-(1-(3,5-dichloropyridin -4-yl)ethoxy)-7-fluoro-1H-indazol-3-yl)-1H-pyrazol-1-yl)-1-morpholinoetha n-1-one | 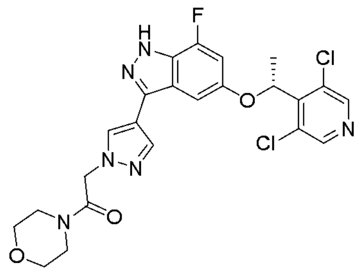 | 519 | 1H NMR (400 MHz, CDC13) δ 8.46 (s, 2H), 8.02 (s, 1H), 7.95 (s, 1H), 6.89 (s, 1H), 6.80 (s, 1H), 6.03 (d, J = 6.7 Hz, 1H), 5.08 (s, 2 H), 3.69 (s, 8H), 1.82 (d, J = 6.6 Hz, 3H). |
| 20 | (R)-2-(4-(5-(1-(3,5-dichloropyridin -4-yl)ethoxy)-7-fluoro-1H-indazol-3-yl)-1H-pyrazol-1-yl)-N-(2-methoxyethyl)-N-methylethan-1-amine | 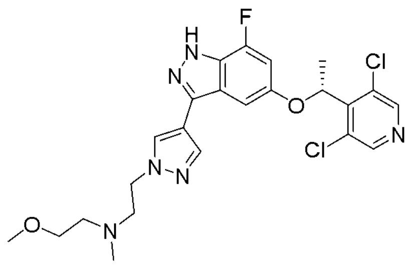 | 507 | 1H NMR (400 MHz, CDC13) δ 8.43 (s, 2H), 7.92 (d, J = 16.6 Hz, 2H), 6.88 - 6.81 (m, 2H), 6.03 (d, J = 6.7 Hz, 1H), 4.31 (d, J = 6.7 Hz, 2H), 3.45 (s, 2H), 3.30 (s, 3H), 2.97 (s, 2H), 2.66 (s, 2H), 2.37 (s, 3H), 1.80 (d, J = 6.7 Hz, 3H). |

(continued)

| Exa mple | Name | Structure | LC-MS $(M+H)^+$ | $^1H$ NMR |
|---|---|---|---|---|
| 21 | (R)-5-(1-(3,5-dichloropyridin -4-yl)ethoxy)-7-fluoro-3-(1-(2-(2-methoxyethoxy ethyl)ethyl)-1H-pyrazol-4-yl)-1H-indazole | 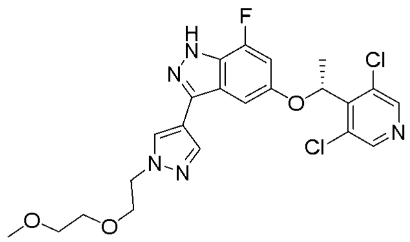 | 494 | $^1H$ NMR (400 MHz, CDC13) δ 8.42 (s, 2H), 7.93 (d, J = 17.8 Hz, 2H), 6.84 (d, J = 12.7 Hz, 2H), 6.03 (d, J = 6.7 Hz, 1H), 4.38 (s, 2H), 3.90 (s, 2H), 3.59 (s, 2H), 3.49 (d, J = 5.1 Hz, 2H), 3.32 (d, J = 4.2 Hz, 3H), 1.79 (d, J = 6.6 Hz, 3H). |
| 22 | (R)-5-(1-(3,5-dichloropyridin -4-yl)ethoxy)-7-fluoro-3-(1-(2-(4- methylpiperazi n-1-yl)ethyl)-1H-pyrazol-4-yl)-1H-indazole | 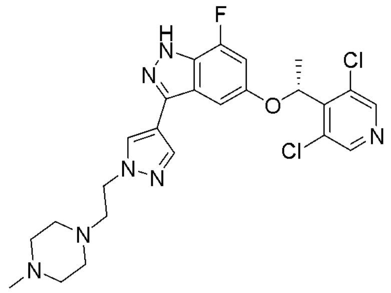 | 518 | $^1H$ NMR (400 MHz, CDCl3) δ 8.43 (s, 1H), 7.938-7.873 (d, J = 25.8 Hz, 2H), 6.879-6.799 (d, J = 31.9 Hz, 2H), 6.04 (d, J = 6.7 Hz, 1H), 4.40 - 4.18 (m, 2H), 2.95 - 2.78 (m, 2H), 2.62-2.49 (d, J = 49.6 Hz, 8H), 2.28 (s, 3H), 1.82 (d, J = 6.7 Hz, 3H). |
| 23 | (R)-5-(1-(3,5-dichloropyridin -4-yl)ethoxy)-7-fluoro-3-(1-(2-(piperidin-1- yl) ethyl)-1H-pyrazol-4-yl)-1H-indazole | 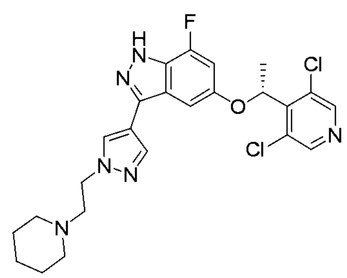 | 503 | $^1H$ NMR (400 MHz, DMSO) δ 13.45 (s, 1H), 8.62 (s, 2H), 8.23 (s, 1H), 7.88 (s, 1H), 7.01 (s, 2H), 6.19 (q, J = 6.6 Hz, 1H), 4.50-4.23 (m, 2H), 2.90 - 2.54 (m, 4H), 2.49-2.26 (m, 2H), 1.79 (d, J = 6.6 Hz, 3H), 1.68 - 1.34 (m, 6H). |
| 24 | (R)-4-(2-(4-(5-(1-(3,5-dichloropyridin -4-yl) ethoxy)-7-fluoro-1H-indazol-3-yl)-1H-pyrazol-1-yl) ethyl)thiomo rpholine 1,1-dioxide | 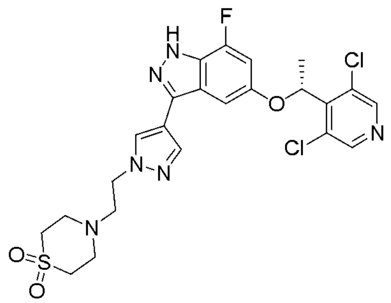 | 553 | $^1H$ NMR (400 MHz, DMSO) δ 13.44 (s, 1H), 8.63 (s, 2H), 8.25 (s, 1H), 7.87 (s, 1H), 7.01 (d, J = 12.1 Hz, 2H), 6.19 (d, J = 6.7 Hz, 1H), 4.34 (t, J = 6.2 Hz, 2H), 3.05 (dd, J = 16.3, 9.9 Hz, 10H), 1.79 (d, J = 6.6 Hz, 3H). |

Example 28

(R)-5-(1-(3,5-dichloropyridin-4-yl)ethoxy)-3-(1-(pyridin-3-ylmethyl)-1H-pyrazol-4-yl)-1H-pyrazolo[4,3-b]pyridine

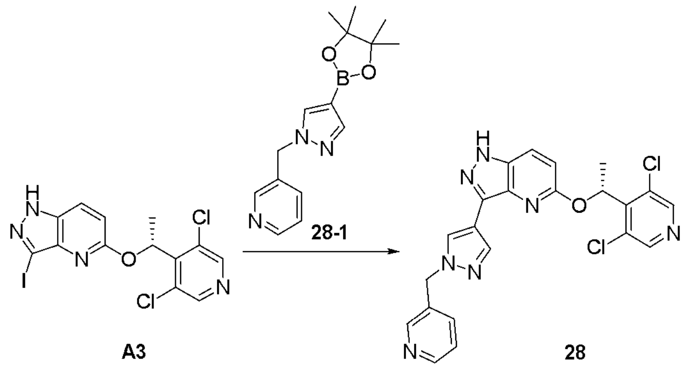


A3

28

Compound A3 (120 mg, 0.28 mmol, 1.0 eq), Compound 28-1 (118 mg, 0.41 mmol, 1.5 eq), $C_{S2}CO_3$ (270 mg, 0.83 mmol, 3.0 eq) and $Pd(dppf)Cl_2$ (22.5 mg, 0.027 mmol, 0.1 eq) were dissolved in dioxane (5 mL), then 1 mL of water was added, purged with nitrogen, and stirred at 80°C for 8 hours. LCMS showed the generation of a product. 30 mL of water was added to the reaction mixture, and then 30 mL of EA was added to wash and extract the reaction mixture three times. The organic phases were combined, dried over anhydrous sodium sulfate, and purified by a silica gel column followed by a reversed-phase column to obtain 52.2 mg of Compound 28 as a yellow powder (yield: 40.5%, purity 97.2%). LCMS (ESI) m/z 467.32(M+H)+); $^1$H NMR (400 MHz, ) δ 8.91 (d, J = 6.4 Hz), 8.50 (d, J = 1.3 Hz), 8.37 (d, J = 8.2 Hz), 8.30 (d, J = 0.92 Hz), 8.12 - 8.05 (m), 8.01 - 7.93 (m), 6.96 (t, J = 3.8 Hz), 6.43 (d, J = 1.2 Hz), 5.71 (s), 1.69 (d, J = 6.8 Hz).

The following compounds were synthesized according to the the route of Example 28.

| Example | Name | Structure | LC-MS $(M+H)^+$ | $^1H$ NMR |
|---|---|---|---|---|
| 29 | (R)-5-(1-(3,5-dichloropyridin-4-yl)ethoxy)-3-(1-methyl-1H-pyrazol-4-yl)-1H-pyrazolo[4,3-b] pyridine | 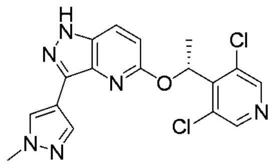 | 389 | $^1H$ NMR (400 MHz, DMSO) δ 8.59 (s, 2H), 8.03 (s, 1H), 7.96 (d, J = 9.0 Hz, 1H), 7.85 (s, 1H), 6.99 (d, J = 9.0 Hz, 1H), 6.45 (q, J = 6.8 Hz, 1H), 3.95 (s, 2H), 1.73 (d, J = 6.8 Hz, 2H). |
| 30 | (R)-5-(1-(3,5-dichloropyridin-4-yl)ethoxy)-3-(1,5-dimethyl-1H-pyrazol-4-yl)-1H-pyrazolo[4,3-b]pyridine | 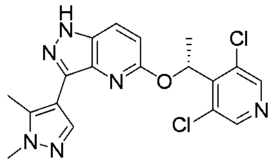 | 404 | $^1H$ NMR (400 MHz, DMSO) δ 8.54 (s, 2H), 7.97 (d, J = 9.0 Hz, 1H), 7.81 (s, 1H), 6.99 (d, J = 9.0 Hz, 1H), 6.45 (d, J = 6.8 Hz, 1H), 3.79 (s, 3H), 2.54 (s, 3H), 1.72 (d, J =6.8 Hz, 3H). |
| 31 | (R)-4-(2-(4-(5-(1-(3,5-dichloropyridin-4-yl)ethoxy)-1H-pyrazolo[4,3-b]pyridin-3-yl)-1H-pyrazol-1-yl)ethyl)morpholin e | 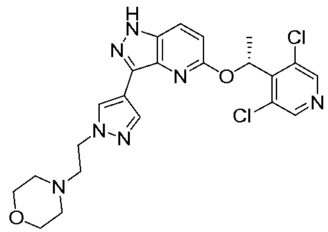 | 489 | $^1H$ NMR (400 MHz, DMSO) δ 11.50 (s, 1H), 8.62 (s, 2H), 8.21 (s, 1H), 7.98 (d, J = 9.2 Hz, 2H), 7.01 (d, J = 9.0 Hz, 1H), 6.48 (q, J = 6.8 Hz, 1H), 4.75 (t, J = 6.6 Hz, 2H), 3.98 (d, J = 11.4 Hz, 2H), 3.84 (d, J = 7.2 Hz, 3H), 3.69 (s, 1H), 3.44 (d, J = 11.2 Hz, 3H), 3.17 (s, 2H), 1.74 (d, J = 6.8 Hz, 3H). |
| 32 | (R)-4-(3-(4-(5-(1-(3,5-dichloropyridin-4-yl)ethoxy)-1H-pyrazolo[4,3-b]pyridin-3-yl)-1H-pyrazol-1-yl)propyl)morpholi ne | 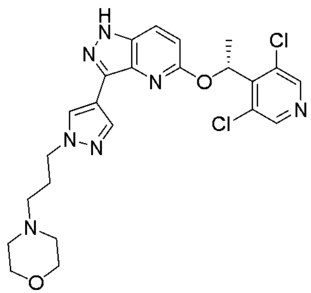 | 502 | $^1H$ NMR (400 MHz, DMSO) δ 8.61 (s, 2H), 8.11 (s, 1H), 7.97 (d, J = 18.6 Hz, 2H), 7.00 (s, 1H), 6.48 (s, 1H), 4.34 - 4.30 (m, 2H), 3.99 (d, J = 10.6 Hz, 4H), 3.12 (dd, J = 31.4, 8.8 Hz, 6H), 2.34 (s, 2H), 1.74 (d, J = 3.4 Hz, 3H). |

(continued)

| Exa mple | Name | Structure | LC-MS $(M+H)^+$ | $^1H$ NMR |
|---|---|---|---|---|
| 33 | (R)-4-(5-(5-(1-(3,5-dichloropyridin-4-yl)ethoxy)-1H-pyrazolo [4,3-b]pyridin-3-yl)pyridin-2-yl) morpholine | 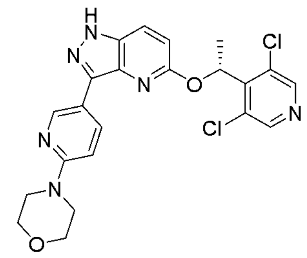 | 472 | $^1H$ NMR (400 MHz, DMSO) δ 13.18 (s, 1H), 8.76 (s, 1H), 8.62 (s, 2H), 8.14 (d, J = 9.0 Hz, 1H), 8.01 (d, J = 8.8 Hz, 1H), 7.02 (d, J = 9.2 Hz, 1H), 6.90 (d, J = 9.2 Hz, 1H), 6.38 (d, J = 6.8 Hz, 1H), 3.77 (s, 4H), 3.56 (s, 4H), 1.74 (d, J = 6.8 Hz, 3H). |
| 34 | (R)-5-(1-(3,5-dichloropyridin-4-yl)ethoxy)-3-(1-(tetrahydro-2H-pyran-4-yl)-1H-pyrazol-4-yl) 1H-pyrazolo[4,3-b]pyridine | 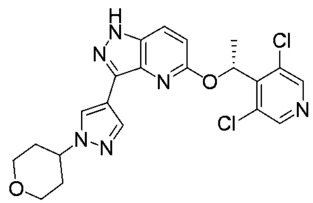 | 459 | $^1H$ NMR (400 MHz, DMSO) δ 8.58 (s, 2H), 8.11 (d, J = 5.2 Hz, 1H), 7.98 (d, J = 9.0 Hz, 1H), 7.89 (s, 1H), 6.96 (d, J = 9.0 Hz, 1H), 6.48 (dd, J = 6.8, 2.2 Hz, 1H), 4.03 (d, 2H), 4.44-4.50 (m, 1H), 3.52-3.57(m, 2H), 2.01-2.09(m, 4H), 1.73 (d, J = 6.8 Hz, 3H). |
| 35 | (R)-1-(4-(5-(1-(3,5-dichloropyridin-4-yl)ethoxy)-1H-pyrazolo [4,3-b]pyridin-3-yl)-1H-pyrazol-1-yl)piperidin-1-yl)ethan-1-one | 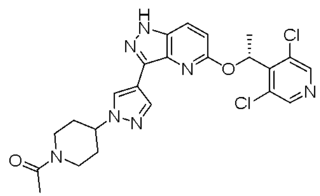 | 501 | $^1H$ NMR (400 MHz, DMSO) δ 8.56 (s, 2H), 8.09 (d, J = 5.2 Hz, 1H), 7.97 (d, J = 9.0 Hz, 1H), 7.91 (s, 1H), 6.99 (d, J = 9.0 Hz, 1H), 6.49 (dd, J = 6.8, 2.2 Hz, 1H), 4.53 (d, J = 11.6 Hz, 2H), 4.01 (s, 1H), 3.28 (dd, J = 21.8, 11.8 Hz, 1H), 2.79 (t, J = 13.4 Hz, 1H), 2.13 - 1.95 (m, 6H), 1.84 - 1.77 (m, 1H), 1.73 (d, J = 6.8 Hz, 3H). |
| 36 | (R)-4-(4-(5-(1-(3,5-dichloropyridin-4-yl)ethoxy)-1H-pyrazolo [4,3-b]pyridin-3-yl)-1H-pyrazol-1-yl)-N,N-dimethylpiperidine -1-carboxamide | 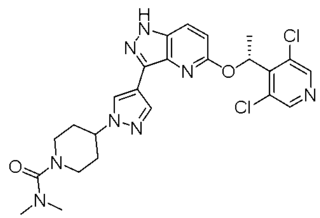 | 530 | $^1H$ NMR (400 MHz, DMSO) δ 8.58 (s, 2H), 8.07 (s, 1H), 7.96 (d, J = 9.0 Hz, 1H), 7.91 (s, 1H), 6.99 (d, J = 9.0 Hz, 1H), 6.49 (q, J = 6.8 Hz, 1H), 4.49 - 4.37 (m, 1H), 3.72 (d, J = 13.0 Hz, 2H), 2.96 (dt, J = 19.6, 6.6 Hz, 2H), 2.80 (s, 6H), 2.08 - 1.94 (m, 4H), 1.73 (d, J = 6.8 Hz, 3H). |

(continued)

| Example | Name | Structure | LC-MS (M+H)+ | 1H NMR |
|---|---|---|---|---|
| 37 | (R)-5-(1-(3,5-dichloropyridin-4-yl)ethoxy)-3-(1-(1-isopropylpiperidin-4-yl)-1H-pyrazol-4-yl) 1H-pyrazolo[4,3-b]pyridine | 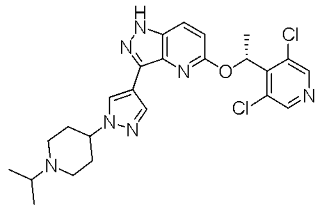 | 501 | $^1$H NMR (400 MHz, DMSO) δ 13.03 (s, 1H), 8.57 (s, 2H), 8.04 (s, 1H), 7.96 (d, J = 9.0 Hz, 1H), 7.90 (s, 1H), 6.99 (d, J = 9.0 Hz, 1H), 6.49 (q, J = 6.8 Hz, 1H), 4.25 - 4.13 (m, 1H), 2.96 (d, J = 9.4 Hz, 2H), 2.82 (dd, J = 13.0, 6.6 Hz, 1H), 2.36 (d, J = 7.8 Hz, 2H), 2.00 (dd, J = 15.6, 7.2 Hz, 4H), 1.73 (d, J = 6.8 Hz, 3H), 1.04 (d, J = 6.6 Hz, 6H). |
| 38 | (R)-1-(4-(5-(1-(3,5-dichloropyridin-4-yl)ethoxy)-1H-pyrazolo [4,3-b]pyridin-3-yl)-1H-pyrazol-1-yl)-2-methylpropan-2-ol | 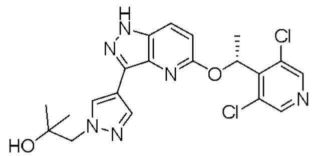 | 448 | $^1$H NMR (400 MHz, DMSO) δ 8.56 (s, 2H), 8.10 (s, 1H), 7.97 (d, J = 9.0 Hz, 1H), 7.90 (s, 1H), 6.99 (d, J = 9.0 Hz, 1H), 6.51 (q, J = 6.8 Hz, 1H), 4.10 (s, 2H), 1.73 (d, J = 6.8 Hz, 3H), 1.11 (s, 6H). |
| 39 | (R)-2-(4-(5-(1-(3,5-dichloropyridin-4-yl)ethoxy)-1H-pyrazolo [4,3-b]pyridin-3-yl)-1H-pyrazol-1-yl)ethan-1-ol | 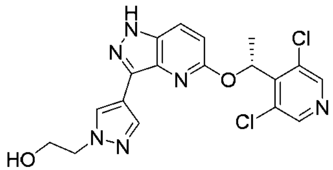 | 419 | $^1$H NMR (400 MHz, DMSO) δ 8.57 (s, 2H), 8.09 (s, 1H), 7.96 (d, J = 9.0 Hz, 1H), 7.89 (s, 1H), 6.99 (d, J = 9.0 Hz, 1H), 6.48 (q, J = 6.8 Hz, 1H), 4.24 (t, J = 5.6 Hz, 2H), 3.80 (d, J = 5.6 Hz, 2H), 1.73 (d, J = 6.8 Hz, 3H). |
| 40 | (R)-5-(1-(3,5-dichloropyridin-4-yl)ethoxy)-3-(1-(2-methoxyethyl)-1H-pyrazol-4-yl)-1H-pyrazolo[4,3-b]pyridine | 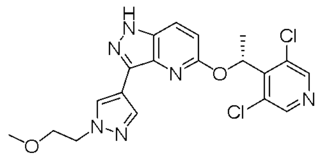 | 434 | $^1$H NMR (400 MHz, DMSO) δ 8.57 (s, 2H), 8.07 (s, 1H), 7.97 (d, J = 9.0 Hz, 1H), 7.90 (s, 1H), 6.99 (d, J = 9.0 Hz, 1H), 6.48 (q, J = 6.8 Hz, 1H), 4.35 (t, J = 5.2 Hz, 2H), 3.74 (t, J = 5.2 Hz, 3H), 3.25 (s, 3H), 1.73 (d, J = 6.8 Hz, 3H). |
| 41 | (R)-5-(1-(3,5-dichloropyridin-4-yl)ethoxy)-3-(1-(2,2-difluoroethyl)-1H-pyrazol-4-yl)-1H-pyrazolo[4,3-b]pyridine | 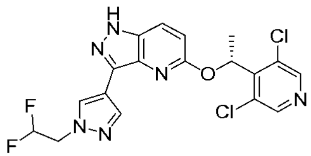 | 439 | $^1$H NMR (400 MHz, DMSO) δ 13.09 (s, 1H), 8.56 (s, 2H), 8.19 (s, 1H), 7.98 (d, J = 8.4 Hz, 2H), 7.01 (d, J = 9.0 Hz, 1H), 6.57 (t, J = 3.6 Hz, 1H), 6.51 (q, J = 6.8 Hz, 1H), 6.44 (t, J = 3.6 Hz, 1H), 6.30 (t, J = 3.6 Hz, 1H), 4.72 (td, J = 15.3, 3.0 Hz, 2H), 1.73 (d, J = 7.0 Hz, 3H). |

(continued)

| Example | Name | Structure | LC-MS $(M+H)^+$ | $^1H$ NMR |
|---|---|---|---|---|
| 42 | (R)-5-(1-(3,5-dichloropyridin-4-yl)ethoxy)-3-(1-(2-(methylsulfonyl)et hyl)-1H-pyrazol-4-yl)-1H-pyrazolo[4,3-b]pyridine | 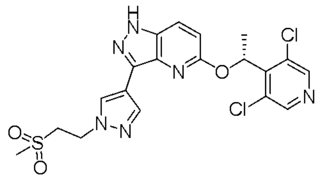 | 481 | $^1H$ NMR (400 MHz, DMSO) δ 8.57 (s, 2H), 8.20 (s, 1H), 7.97 (d, J = 8.6 Hz, 2H), 7.00 (d, J = 9.0 Hz, 1H), 6.49 (q, J = 6.8 Hz, 1H), 4.65 (t, J = 6.8 Hz, 2H), 3.79 (s, 2H), 2.90 (s, 3H), 1.73 (d, J = 6.8 Hz, 3H). |
| 43 | (R)-5-(1-(3,5-dichloropyridin-4-yl)ethoxy)-3-(pyrazolo[1,5-a]pyridin-3-yl)-1H-pyrazolo[4,3-b]pyridine | 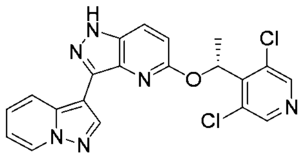 | 425 | $^1H$ NMR (400 MHz, DMSO) δ 13.14 (s, 1H), 8.77 (d, J = 7.0 Hz, 1H), 8.56 (s, 3H), 8.28 (d, J = 8.8 Hz, 1H), 8.02 (d, J = 9.0 Hz, 1H), 7.40 - 7.33 (m, 1H), 7.01 (ddd, J = 10.7, 8.0, 5.0 Hz, 2H), 6.52 (q, J = 6.8 Hz, 1H), 1.75 (d, J = 6.8 Hz, 3H). |
| 44 | (R)-5-(1-(3,5-dichloropyridin-4-yl)ethoxy)-3-(4,5,6,7-tetrahydropyrazolo[1,5-a]pyridin-3-yl)-1H-pyrazolo[4,3-b]pyridine | 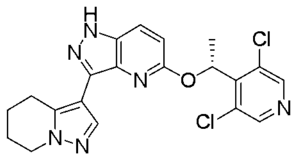 | 429 | $^1H$ NMR (400 MHz, DMSO) δ 8.54 (s, 2H), 7.95 (d, J = 9.0 Hz, 1H), 7.90 (s, 1H), 6.98 (d, J = 9.0 Hz, 1H), 6.46 (q, J = 6.8 Hz, 1H), 4.11 (t, J = 5.8 Hz, 2H), 3.01 (t, J = 6.2 Hz, 2H), 1.98 (d, J = 3.8 Hz, 2H), 1.88 - 1.79 (m, 2H), 1.72 (d, J = 6.8 Hz, 3H). |
| 45 | (R)-5-(1-(3,5-dichloropyridin-4-yl)ethoxy)-3-(5,6-dihydro-4H-pyrrolo[1,2-b]pyrazol-3-yl)-1H-pyrazolo[4,3-b]pyridine | 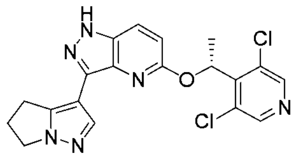 | 415 | $^1H$ NMR (400 MHz, DMSO) δ 12.94 (s, 1H), 8.54 (s, 2H), 8.00 - 7.89 (m, 2H), 6.97 (d, J = 9.0 Hz, 1H), 6.49 (q, J = 6.8 Hz, 1H), 4.11 (t, J = 7.2 Hz, 2H), 3.08 - 2.97 (m, 2H), 2.60 (p, J = 7.4 Hz, 2H), 1.72 (d, J = 6.8 Hz, 3H). |
| 46 | (R)-5-(1-(3,5-dichloropyridin-4-yl)ethoxy)-3-(1-propyl-1H-pyrazol-4-yl)-1H-pyrazolo[4,3-b]pyridine | 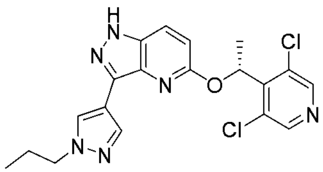 | 417 | $^1H$ NMR (400 MHz, DMSO) δ 13.01 (s, 1H), 8.58 (s, 2H), 8.04 (s, 1H), 7.96 (d, J = 9.0 Hz, 1H), 7.89 (s, 1H), 7.00 (d, J = 9.0 Hz, 1H), 6.47 (q, J = 6.8 Hz, 1H), 4.15 (t, J = 6.8 Hz, 2H), 1.86 (h, J = 7.2 Hz, 2H), 1.73 (d, J = 6.8 Hz, 3H), 0.88 (d, J = 7.4 Hz, 3H). |
| 47 | (R)-5-(1-(3,5-dichloropyridin-4-yl)ethoxy)-3-(1-isopropyl-1H-pyrazol-4-yl)-1H-pyrazolo[4,3-b]pyridine | 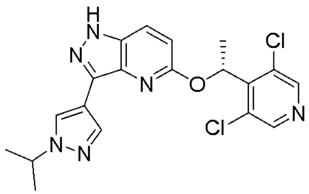 | 417 | $^1H$ NMR (400 MHz, DMSO) δ 13.01 (s, 1H), 8.57 (s, 2H), 8.06 (s, 1H), 7.96 (d, J = 9.0 Hz, 1H), 7.89 (s, 1H), 6.99 (d, J = 9.0 Hz, 1H), 6.50 (q, J = 6.8 Hz, 1H), 4.58 (dd, J = 13.2, 6.8 Hz, 1H), 1.73 (d, J = 6.8 Hz, 3H), 1.50 - 1.43 (m, 6H). |

(continued)

| Example | Name | Structure | LC-MS $(M+H)^+$ | $^1H$ NMR |
|---|---|---|---|---|
| 48 | (R)-3-(1-cyclopropyl-1H-pyrazol-4-yl)-5-(1-(3,5-dichloropyridin-4-yl)ethoxy)-1H-pyrazino [4,3-b]pyridine | 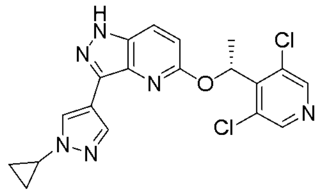 | 415 | $^1H$ NMR (400 MHz, DMSO) δ 13.02 (s, 1H), 8.59 (s, 2H), 8.09 (s, 1H), 7.96 (d, J = 9.0 Hz, 1H), 7.84 (s, 1H), 7.00 (d, J = 9.0 Hz, 1H), 6.47 (q, J = 6.8 Hz, 1H), 3.79 (dd, J = 6.4, 3.8 Hz, 1H), 1.73 (d, J = 6.8 Hz, 3H), 1.15 - 1.06 (m, 4H). |
| 49 | (R)-3-(1-(cyclopropylmethy 1)-1H-pyrazol-4-yl)-5-(1-(3,5-dichloropyridin-4-yl)ethoxy)-1H-pyrazolo[4,3-b]pyridine | 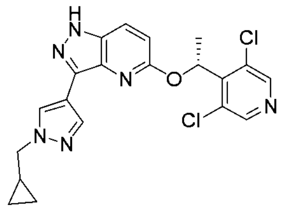 | 429 | $^1H$ NMR (400 MHz, DMSO) δ 13.01 (s, 1H), 8.57 (s, 2H), 8.09 (s, 1H), 7.97 (d, J = 8.8 Hz, 1H), 7.90 (s, 1H), 6.99 (d, J = 9.0 Hz, 1H), 6.49 (q, J = 6.8 Hz, 1H), 4.06 (d, J = 7.0 Hz, 2H), 1.73 (d, J = 6.8 Hz, 3H), 1.37 - 1.27 (m, 1H), 0.60 - 0.53 (m, 2H), 0.41 (dd, J = 5.2, 3.4 Hz, 2H). |
| 50 | (R)-3-(4-(5-(1-(3,5-dichloropyridin-4-yl)ethoxy)-1H-pyrazolo [4,3-b]pyridin-3-yl)-1H-pyrazol-1-yl)propionitrile | 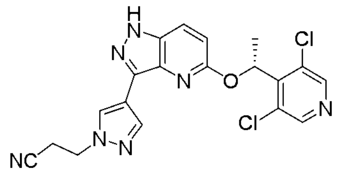 | 428 | $^1H$ NMR (400 MHz, DMSO) δ 13.07 (s, 1H), 8.58 (s, 2H), 8.20 (s, 1H), 7.98 (t, J = 4.2 Hz, 2H), 7.01 (d, J = 9.0 Hz, 1H), 6.51 (d, J = 6.8 Hz, 1H), 4.49 (t, J = 6.4 Hz, 2H), 3.15 (t, J = 6.4 Hz, 2H), 1.74 (d, J = 6.8 Hz, 3H). |
| 51 | (R)-5-(1-(3,5-dichloropyridin-4-yl)ethoxy)-3-(1-isobutyl-1H-pyrazol-4-yl)-1H-pyrazolo[4,3-b]pyridine | 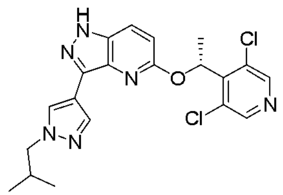 | 431 | $^1H$ NMR (400 MHz, DMSO) δ 13.01 (s, 1H), 8.57 (s, 2H), 8.03 (s, 1H), 7.96 (d, J = 8.8 Hz, 1H), 7.90 (s, 1H), 7.00 (d, J = 8.8 Hz, 1H), 6.47 (d, J = 6.8 Hz, 1H), 3.99 (d, J = 7.2 Hz, 2H), 2.17 (dd, J = 13.6, 6.6 Hz, 1H), 1.73 (d, J = 6.8 Hz, 3H), 0.91 - 0.86 (m, 6H). |
| 52 | (R)-5-(1-(3,5-dichloropyridin-4-yl)ethoxy)-3-(2-methylpyrimidin-5-yl)-1H-pyrazolo[4,3-b] pyridine | 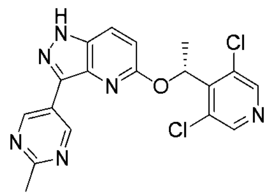 | 401 | $^1H$ NMR (400 MHz, DMSO) δ 13.61 (s, 1H), 9.22 (s, 2H), 8.55 (s, 2H), 8.08 (d, J = 9.0 Hz, 1H), 7.07 (d, J = 9.0 Hz, 1H), 6.46 (q, J = 6.8 Hz, 1H), 2.70 (s, 3H), 1.74 (d, J = 6.8 Hz, 3H). |

(continued)

| Example | Name | Structure | LC-MS $(M+H)^+$ | $^1H$ NMR |
|---|---|---|---|---|
| 53 | (R)-5-(1-(3,5-dichloropyridin-4-yl)ethoxy)-3-(1-(2-(4-methylpiperazin-1-yl)ethyl)-1H-pyrazol-4-yl)-1H-pyrazolo[4,3-b] pyridine | 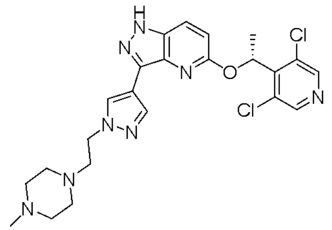 | 501 | $^1H$ NMR (400 MHz, DMSO) δ 12.12 - 11.45 (m, 2H), 8.62 (s, 2H), 8.21 (s, 1H), 7.98 (d, J = 5.6Hz, 2H), 7.01 (d, J = 8.8 Hz, 1H), 6.53 - 6.45 (m, 1H), 4.77 - 4.68 (m, 3H), 3.70 (dd, J = 6.6, 5.6 Hz, 6H), 3.56 - 3.34 (m, 4H), 2.83 (s, 3H), 1.74 (d, J = 6.6 Hz, 3H). |
| 54 | (R)-4-(2-(4-(5-(1-(3,5-dichloropyridin-4-yl)ethoxy)-1H-pyrazolo[4,3-b]pyridin-3-yl)-1H-pyrazol-1-yl)ethyl)-1-methylpiperazin-2-one | 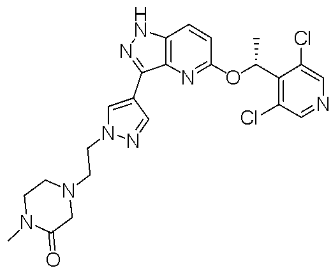 | 515 | $^1H$ NMR (400 MHz, DMSO) δ 8.61 (s, 2H), 8.23 (s, 1H), 7.99 (d, J = 12.6 Hz, 2H), 7.01 (d, J = 9.0 Hz, 1H), 6.49 (q, J = 6.4 Hz, 1H), 4.75 (t, J = 6.4 Hz, 1H), 3.82 (s, 1H), 3.74 (s, 1H), 3.61 (d, J = 6.4 Hz, 2H), 2.89 (s, 2H), 1.74 (d, J = 6.6 Hz, 1H). |
| 55 | (R)-4-(2-(4-(5-(1-(3,5-dichloropyridin-4-yl)ethoxy)-1H-pyrazolo[4,3-b]pyridin-3-yl)-1H-pyrazol-1-yl)ethyl)thiomorph oline 1,1-dioxide | 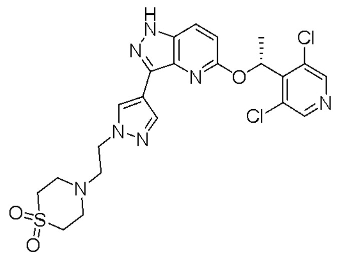 | 536 | $^1H$ NMR (400 MHz, DMSO) δ 8.62 (s, 2H), 8.21 (s, 1H), 7.98 (d, J = 6.6 Hz, 2H), 7.00 (d, J = 9.2 Hz, 1H), 6.48 (d, J = 7.0 Hz, 1H), 4.77 - 4.72 (m, 2H), 3.75 (dd, J = 15.0, 3.0 Hz, 9H), 1.73 (d, J = 6.8 Hz, 3H). |
| 56 | (R)-5-(1-(3,5-dichloropyridin-4-yl)ethoxy)-3-(1-(2-(4-(methylsulfonyl)pi perazin-1-yl) ethyl)-1H-pyrazol-4-yl)-1H-pyrazolo[4,3-b]pyridine | 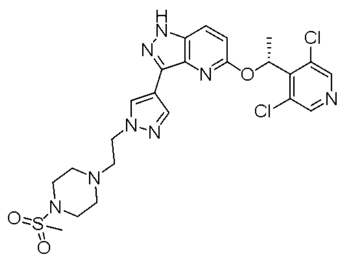 | 565 | $^1H$ NMR (400 MHz, DMSO) δ 11.93 (dd, J = 35.6, 12.8 Hz, 1H), 8.62 (s, 3H), 8.21 (s, 1H), 7.98 (d, J = 6.8 Hz, 3H), 7.00 (d, J = 8.8 Hz, 1H), 6.53 - 6.44 (m, 1H), 4.80 - 4.76 (m, 2H), 3.74 - 3.26 (m, 11H), 3.02 (s, 3H), 1.73 (d, J = 6.6 Hz, 3H). |

(continued)

| Example | Name | Structure | LC-MS $(M+H)^+$ | $^1H$ NMR |
|---|---|---|---|---|
| 57 | (R)-1-(4-(2-(4-(5-(1-(3,5-dichloropyridin-4-yl)ethoxy)-1H-pyrazolo[4,3-b]pyridin-3-yl)-1H-pyrazol-1-yl)ethyl)piperazin-1-yl)ethan-1-one | 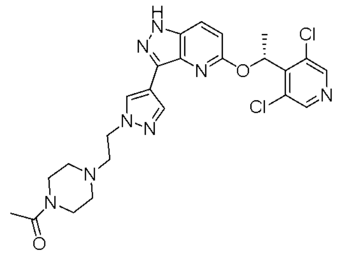 | 529 | $^1H$ NMR (400 MHz, DMSO) δ 11.94 - 11.81 (m, 1H), 8.62 (s, 2H), 8.21 (s, 1H), 7.98 (d, J = 7.2 Hz, 2H), 7.00 (d, J = 9.0 Hz, 1H), 6.50 - 6.44 (m, 1H), 4.79 - 4.73 (m, 3H), 4.42 (d, J = 8.6 Hz, 1H), 4.02 (dd, J = 9.8, 5.6 Hz, 1H), 3.71 - 3.64 (m, 3H), 3.53 - 3.44 (m, 2H), 3.19 - 3.05 (m, 3H), 2.04 (s, 3H), 1.73 (d, J = 6.8 Hz, 3H). |
| 58 | 1-(2-(4-(5-((R)-1-(3,5-dichloropyridin-4-yl)ethoxy)-1H-pyrazolo[4,3-b]pyridin-3-yl)-1H-pyrazol-1-yl)ethyl)pyrrolidin -3-ol | 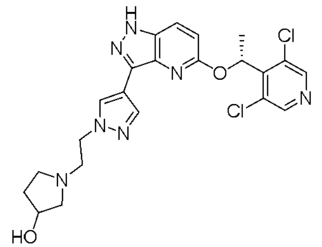 | 488 | $^1H$ NMR (400 MHz, DMSO) δ 11.39 (s, 1H), 10.79 (s, 1H), 8.60 (d, J = 4.6 Hz, 2H), 8.21 (d, J = 5.4 Hz, 1H), 7.98 (d, J = 9.2 Hz, 2H), 7.00 (d, J = 9.0 Hz, 1H), 6.49 (d, J = 7.6 Hz, 1H), 4.67 (d, J = 7.0 Hz, 1H), 3.73 (dd, J = 17.8, 6.4 Hz, 1H), 3.64 - 3.47 (m, 1H), 3.41 - 3.30 (m, 0H), 3.19 (dd, J = 16.8, 10.0 Hz, 0H), 3.14 - 3.04 (m, 1H), 2.89 (t, J = 12.2 Hz, 0H), 2.25 (dd, J = 14.4, 9.6 Hz, 0H), 2.07 - 1.80 (m, 1H), 1.73 (d, J = 6.6 Hz, 2H). |
| 59 | 1-(3-(4-(5-((R)-1-(3,5-dichloropyridin-4-yl)ethoxy)-1H-pyrazolo[4,3-b]pyridin-3-yl)-1H-pyrazol-1-yl)pyrrolidin-1-yl)-2-hydroxyethan-1-one | 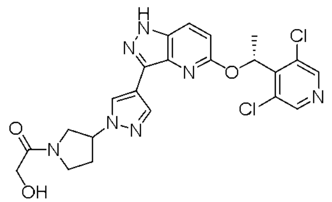 | 502 | $^1H$ NMR (400 MHz, DMSO) δ 13.06 (s, 1H), 8.59 (s, 2H), 8.17 - 8.11 (m, 1H), 7.98 - 7.91 (m, 2H), 7.00 (d, J = 8.8 Hz, 1H), 6.49 (d, J = 6.2 Hz, 1H), 5.18 - 5.04 (m, 1H), 4.67 (dd, J = 3.4, 1.0 Hz, 1H), 4.09 - 4.02 (m, 2H), 3.97 - 3.86 (m, 1H), 3.78 - 3.70 (m, 1H), 3.66 - 3.53 (m, 2H), 2.46 - 2.24 (m, 2H), 1.73 (d, J = 6.8 Hz, 3H). |
| 60 | (R)-3-(1-(2-(1H-imidazol-1-yl)ethyl)-1H-pyrazol-4-yl)-5-(1-(3,5-dichloropyridin-4-yl)ethoxy)-1H-pyrazolo[4,3-b]pyridine | 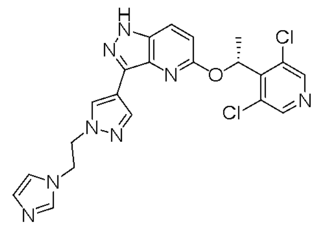 | 469 | $^1H$ NMR (400 MHz, DMSO) δ 14.61 (s, 1H), 8.97 (s, 1H), 8.58 (s, 2H), 8.02 - 7.89 (m, 3H), 7.61 (d, J = 17.9 Hz, 2H), 6.99 (d, J = 8.3 Hz, 1H), 6.44 (d, J = 6.7 Hz, 1H), 4.72 (d, J = 9.8 Hz, 4H), 1.72 (d, J = 6.5 Hz, 3H). |

(continued)

| Example | Name | Structure | LC-MS $(M+H)^+$ | $^1H$ NMR |
|---|---|---|---|---|
| 61 | (R)-5-(1-(3,5-dichloropyridin-4-yl)ethoxy)-3-(1-(2-(pyridin-3-yl) ethyl)-1H-pyrazol-4-yl)-1H-pyrazolo[4,3-b]pyridine | 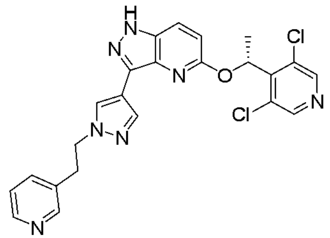 | 480 | $^1H$ NMR (400 MHz, DMSO) δ 13.01 (s, 1H), 8.56 (s, 2H), 8.38 (d, J = 14.0 Hz, 2H), 7.98 - 7.89 (m, 2H), 7.58 (d, J = 8.0 Hz, 1H), 7.29 (s, 1H), 6.98 (d, J = 9.0 Hz, 1H), 6.44 (d, J = 6.4 Hz, 1H), 4.46 (s, 2H), 3.21 (s, 2H), 1.72 (d, J = 6.6 Hz, 3H). |
| 62 | (R)-8-(2-(4-(5-(1-(3,5-dichloropyridin-4-yl)ethoxy)-1H-pyrazolo[4,3-b]pyridin-3-yl)-1H-pyrazol-1-yl)ethyl)-2,8-diazaspiro [4.5]dec an-1-one | 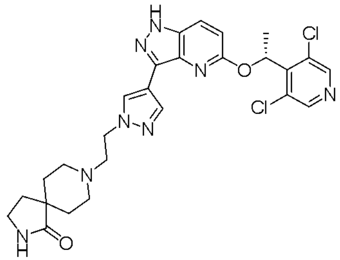 | 555 | $^1H$ NMR (400 MHz, DMSO) δ 13.04 (s, 1H), 8.58 (s, 2H), 8.09 (s, 1H), 7.96 (d, J = 9.0Hz, 1H), 7.88 (s, 1H), 7.53 (s, 1H), 6.99 (d, J = 9.4 Hz, 1H), 6.49 (s, 1H), 4.30 (s, 2H), 3.14 (s, 2H), 2.77 (s, 4H), 2.10 (s, 2H), 1.95 (d, J = 32.0 Hz, 4H), 1.73 (d, J = 6.8 Hz, 3H), 1.69 - 1.59 (m, 2H). |
| 63 | (R)-2-(4-(5-(1-(3,5-dichloropyridin-4-yl)ethoxy)-1H-pyrazolo [4,3-b]pyridin-3-yl)-1H-pyrazol-1-yl)-1-morpholinoethan-1-one | 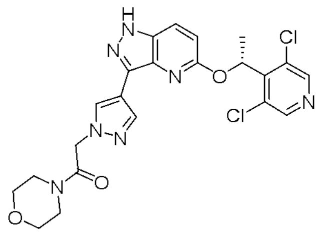 | 502 | $^1H$ NMR (400 MHz, DMSO) δ 8.53 (s, 2H), 8.10 (s, 1H), 7.97 (d, J = 9.4 Hz, 1H), 7.87 (s, 1H), 6.99 (d, J = 8.2 Hz, 1H), 6.46 (d, J = 6.8 Hz, 1H), 5.24 (s, 2H), 3.69 - 3.57 (m, 5H), 3.51 (s, 3H), 1.98 (s, 1H), 1.72 (d, J = 6.2 Hz, 3H) |
| 64 | (R)-1-(2-(4-(5-(1-(3,5-dichloropyridin-4-yl)ethoxy)-1H-pyrazolo[4,3-b]pyridin-3-yl)-1H-pyrazol-1-yl)ethyl)pyrrolidin -2-one | 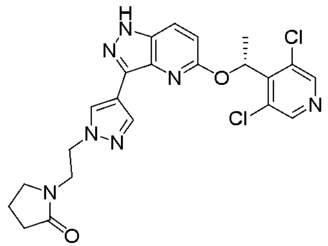 | 486 | $^1H$ NMR (400 MHz, DMSO) δ 8.59 (s, 2H), 8.07 (s, 1H), 7.97 (d, J = 8.8Hz, 1H), 7.91 (s, 1H), 6.99 (d, J = 9.2 Hz, 1H), 6.46 (d, J = 6.8 Hz, 1H), 4.32 (s, 2H), 3.61 (s, 2H), 3.08 (s, 2H), 2.16 (d, J = 7.4 Hz, 2H), 1.82 (d, J = 6.8 Hz, 2H), 1.72 (d, J = 6.6Hz, 3H). |

Example 65

3-(1-methyl-1H-pyrazol-4-yl)-N-(2-(methylamino)ethyl)-1H-indazole-5-carboxami de

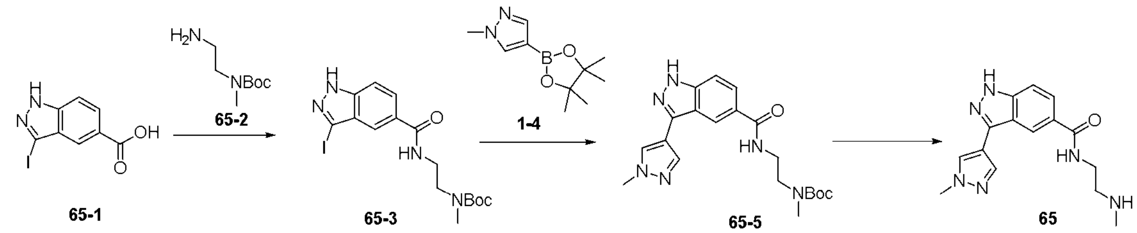

65-1 65-3 65-5 65

DIPEA (0.5 mL, 3.12 mmol, 3.0 eq) was added to a solution of Compound 65-1 (300 mg, 1.04 mmol, 1.0 eq), Compound 65-2 (0.3 mL, 1.56 mmol, 1.5eq) and HATU (613 mg, 1.6 mmol, 1.5 eq) in DMF (10 mL), and reacted at room temperature for 12 hours. LCMS showed the generation of a product. The reaction mixture was extracted with EA and water, and concentrated under reduced pressure. The residue was purified by silica gel column chromatography (eluent: DCM:methanol=15:1) to obtain Compound 65-3 (600 mg, purity 70%, yield 90%): LCMS (ESI) m/z 445, (M+H)+.

Pd(dppf)$Cl_2$ (231 mg, 0.19 mmol, 0.2 eq) was added to a solution of Compound 65-3 (600 mg, 0.95 mmol, 1.0 eq), Compound 65-4 (350 mg, 1.68 mmol, 1.1 eq) and sodium carbonate (497 mg, 4.79 mmol, 3.0 eq) in dioxane/water (12 mL/3 mL), and reacted at 100°C for 12 h. LCMS showed the generation of a product. The reaction mixture was directly concentrated under reduced pressure, and the residue was purified by silica gel column chromatography (eluent: DCM:methanol=10:1) to obtain Compound 65-5 (337 mg, yield 89%): LCMS (ESI) m/z 399.21, (M+H)+.

2 mL of EA-HCl was added to a solution of Compound 65-5 (150 mg, 0.38 mmol, 1.0 eq) in ethyl acetate (2 mL), and reacted under stirring at room temperature for 2 h. LCMS showed the generation of a product. The reaction mixture was directly concentrated under reduced pressure, and subjected to preparative high performance liquid chromatography to obtain Compound 65 (11.7 mg, purity 100%, yield 10.3%): LCMS (ESI) m/z 299.21, (M+H)+. $^1$H NMR (400 MHz, DMSO) δ 9.01 (t, J = 5.4 Hz, 1H), 8.86 (s, 2H), 8.67 (s, 1H), 8.63 (s, 1H), 8.10 (s, 1H), 7.96 (dd, J = 8.8, 1.4 Hz, 1H), 7.59 (d, J = 8.7 Hz, 1H), 3.98 (s, 3H), 3.64 (s, 3H), 3.20 - 3.12 (m, 2H), 2.63 (t, J = 5.4 Hz, 3H).

The following compounds were synthesized according to the the route of Example 65.

| Exa mple | Name | Structure | LC-MS $(M+H)^+$ | $^1$H NMR |
|---|---|---|---|---|
| 66 | N-methyl-3-(1-methyl-1H-pyrazol-4-yl)-N-(2-(methylamino)ethyl) -1H-indazole-5-carboxamide | 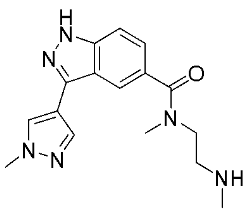 | 312.17 | $^1$H NMR (400 MHz, DMSO) δ 13.16 (s, 1H), 8.44 (s, 1 H), 8.08 (s, 1H), 8.01 (s, 1H), 7.57 (d, J = 7.3 Hz, 1H), 7.42 (d, J = 7.3 Hz,1H), 3.96 (s, 3H), 3.68-3.47 (m, 2H), 3.26 (s, 3H), 3.02 (s, 3H), 2.92-2.65 (m, 2H). |
| 67 | N-(2-hydroxy-2-methylpropyl)-3-(1-methyl-1H-pyrazol-4-yl)-1H-indazole-5-carboxamide | 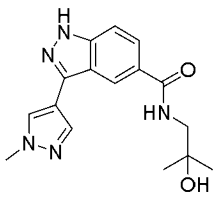 | 314 | $^1$H NMR (400 MHz, DMSO) δ 13.16 (s, 1H), 8.55 (s, 1H), 8.46 (t, J = 6.0 Hz, 1H), 8.40 (s, 1H), 8.14 (s, 1H), 7.93 (d, J = 8.8 Hz, 1H), 7.58 (d, J = 8.8 Hz, 1H), 4.65 (s, 1H), 3.99 (s, 3H), 3.34 (d, J = 6.3 Hz, 2H), 1.16 (s, 6H). |
| 68 | N-(azetidin-3-ylmethyl)-3-(1-methyl-1H-pyrazol-4-yl)-1H-indazole-5-carboxamide | 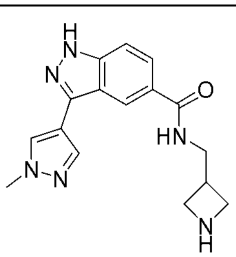 | 311 | $^1$H NMR (500 MHz, DMSO) δ 8.75 (t, J = 5.6 Hz, 1H), 8.52 (s, 1H), 8.45 (s, 1H), 8.40 (s, 1H), 8.10 (s, 1H), 7.90 (dd, J = 8.8, 1.3 Hz, 1H), 7.56 (d, J = 8.8 Hz, 1H), 3.96 (s, 3H), 3.58-3.63 (m, 2H) ,3.30-3.35(m, 2H), 3.1 (d, 2H), 2.81-2.85 (m, 1H) |

(continued)

| Example | Name | Structure | LC-MS (M+H)+ | 1H NMR |
|---|---|---|---|---|
| 69 | 3-(1-methyl-1H-pyrazol-4-yl)-N-(piperidin-3-ylmethyl)-1H-indazole-5-carboxamide | 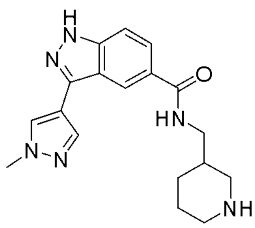 | 339 | 1H NMR (500 MHz, DMSO) δ 8.75 (t, J = 5.6 Hz, 1H), 8.52 (s, 1H), 8.45 (s, 1H), 8.40 (s, 1H), 8.10 (s, 1H), 7.90 (dd, J = 8.8, 1.3 Hz, 1H), 7.56 (d, J = 8.8 Hz, 1H), 3.96 (s, 3H), 3.31 - 3.03 (m, 5H), 2.67 (tt, J = 28.6, 14.3 Hz, 1H), 1.98 (s, 1H), 1.82 - 1.68 (m, 2H), 1.55 (td, J = 12.3, 3.6 Hz, 1H), 1.25 - 1.17 (m, 1H) |
| 77 | N-(2,6-dimethylphenyl)-3-(1-methyl-1H-pyrazol-4-yl)-1H-indazole-5-carboxamide | 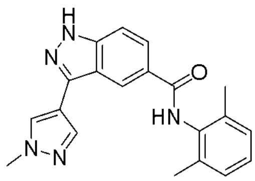 | 346 | 1H NMR (400 MHz, DMSO) δ 13.22 (s, 1H), 9.86 (s, 1H), 8.74 (s, 1H), 8.42 (s, 1H), 8.16 (s, 1H), 8.03 (s, 1H), 7.64 (d, J = 8.8 Hz, 1H), 7.18 (s, 3H), 3.98 (s, 3H), 2.24 (s, 6H). |
| 78 | N-((3,5-dichloropyridin-4-yl) methyl)-3-(1-methyl-1H-pyrazol-4-yl)-1H-indazole-5-carboxamide | 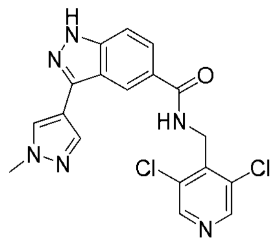 | 401 | 1H NMR (400 MHz, DMSO) δ 13.19 (s, 1H), 8.98 (t, J = 4.6 Hz, 1H), 8.67 (s, 2H), 8.50 (s, 1H), 8.35 (s, 1H), 8.10 (s, 1H), 7.90 (d, J = 8.8, 1H), 7.57 (d, J = 8.8 Hz, 1H), 4.76 (d, J = 4.6 Hz, 2H), 3.96 (s, 3H). |

Example 70

7-fluoro-N-(2-(methylamino)ethyl)-3-(1-(2-morpholinoethyl)-1H-pyrazol-4-yl)-1H-indazole-5-carboxamide

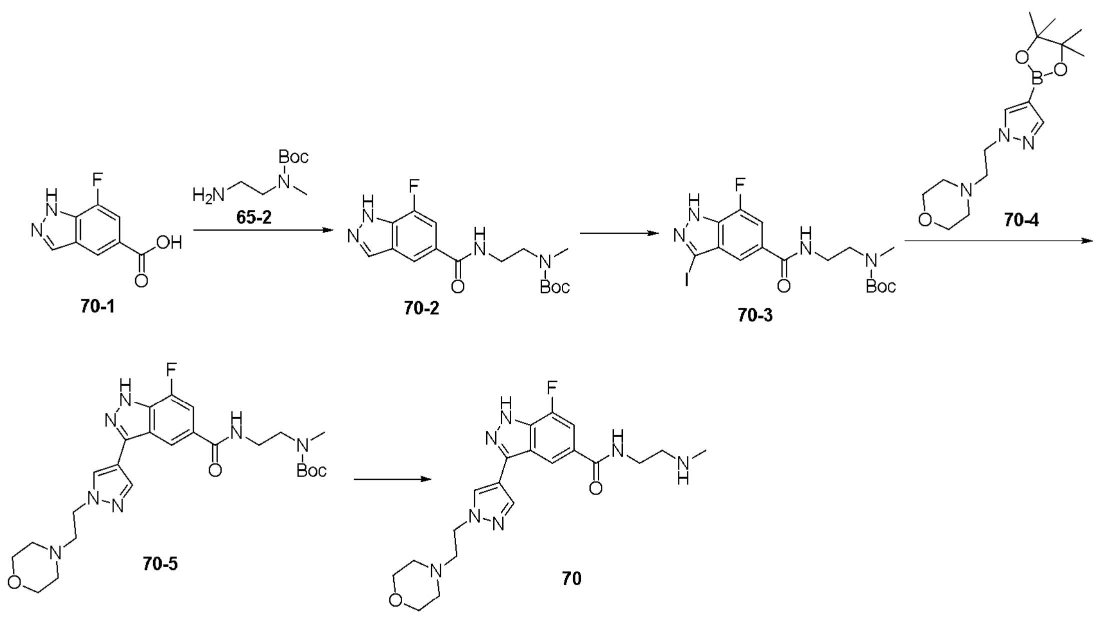


At room temperature, Compound 70-1 (0.108 g, 0.60 mmol), Compound 65-2 (0.125 g, 0.72 mmol), HATU (0.342

g, 0.90 mmol) and N,N-diisopropylethylamine (0.233 g, 1.8 mmol) were added to N,N-dimethylformamide (1.2 mL). The reaction mixture was stirred at room temperature for 14 hours. LCMS showed the generation of a product. The reaction mixture was diluted with ethyl acetate (10.0 mL), washed with saturated aqueous sodium bicarbonate solution (5.0 mL × 2), dried over anhydrous sodium sulfate, and filtered. The filtrate was concentrated to dryness under reduced pressure. The resulting residue was purified by silica gel column chromatography (eluent: dichloromethane/methanol=40/1, v/v) to obtain Compound 70-2 as an off-white solid (0.197 g, yield: 97%, purity: 100%) LCMS (ESI) m/z 359.2, (M+Na)+.

At room temperature, NIS (0.158 g, 0.70 mmol) was added to a solution of Compound 70-2 (0.197 g, 0.59 mmol) in N,N-dimethylformamide (1.2 mL). The resulting mixture was stirred at room temperature for 17 hours. LCMS showed the generation of a product. The reaction mixture was diluted with ethyl acetate (10.0 mL), washed successively with saturated aqueous sodium bicarbonate solution (10.0 mL) and water (10.0 mL), dried over anhydrous sodium sulfate, and filtered. The filtrate was concentrated to dryness under reduced pressure. The resulting residue was purified by silica gel column chromatography (eluent: dichloromethane/methanol=60/1, v/v) to obtain Compound 70-3 as a yellow solid (0.247 g, yield: 91%, purity: 100%). LCMS (ESI) m/z 485.0, (M+Na)+.

At room temperature, Compound 70-3 (0.0647 g, 0.14 mmol), Compound 70-4 (0.0731 g, 0.24 mmol), Pd(dppf) $Cl_2 \cdot CH_2Cl_2$ (0.0172 g, 0.021 mmol) and $Na_2CO_3$ (0.0297 g, 0.28 mmol) were added to a mixture of 1,4-dioxane (0.7 mL) and water (0.35 mL). The resulting mixture was stirred at 100°C for 19 hours. LCMS showed the generation of a product. The reaction mixture was cooled to room temperature, and diluted with dichloromethane (10.0 mL) and water (5.0 mL). The organic phase was separated, and the aqueous phase was extracted with dichloromethane (5.0 mL × 3). The organic phases were combined, dried over anhydrous sodium sulfate, and filtered. The filtrate was concentrated to dryness under reduced pressure. The resulting residue was purified by silica gel column chromatography (gradient elution, eluent: dichloromethane/methanol=30/1-20/1, v/v) to obtain Compound 70-5 as a light brown solid (0.0355 g, yield: 49%, purity: 92.48%). LCMS (ESI) m/z 516.3, (M+H)+.

At room temperature, Compound 70-5 (0.0355 g, 0.069 mmol) was added to a solution of hydrogen chloride in ethyl acetate (2.0 M) (0.35 mL). The resulting mixture was stirred at room temperature for 4 hours. LCMS showed the generation of a product. The reaction mixture was diluted with ethyl acetate (0.3 mL), and filtered under reduced pressure. The filter cake was washed with ethyl acetate (0.1 mL × 3), and dried under vacuum to obtain Compound 70 as a light brown solid (0.0176 g, yield: 52%, purity: 92.96%). LCMS (ESI) m/z 416.2, (M+H)+; $^1$H NMR (400 MHz, DMSO) δ 9.27 (t, J = 5.2 Hz, 1H), 9.08 (br s, 2H), 8.83 (s, 1H), 8.64 (s, 1H), 8.15 (s, 1H), 7.76 (d, J = 12.2 Hz, 1H), 4.00 (s, 3H), 3.68 (dd, J = 11.0, 5.4 Hz, 2H), 3.22 - 3.14 (m, 2H), 2.64 (t, J = 5.2 Hz, 3H).

Example 71

6-(3,5-dimethoxyphenyl)-3-(1-methyl-1H-pyrazol-4-yl)-N-(2-(methylamino)ethyl) -1H-indazole-5-carboxamide

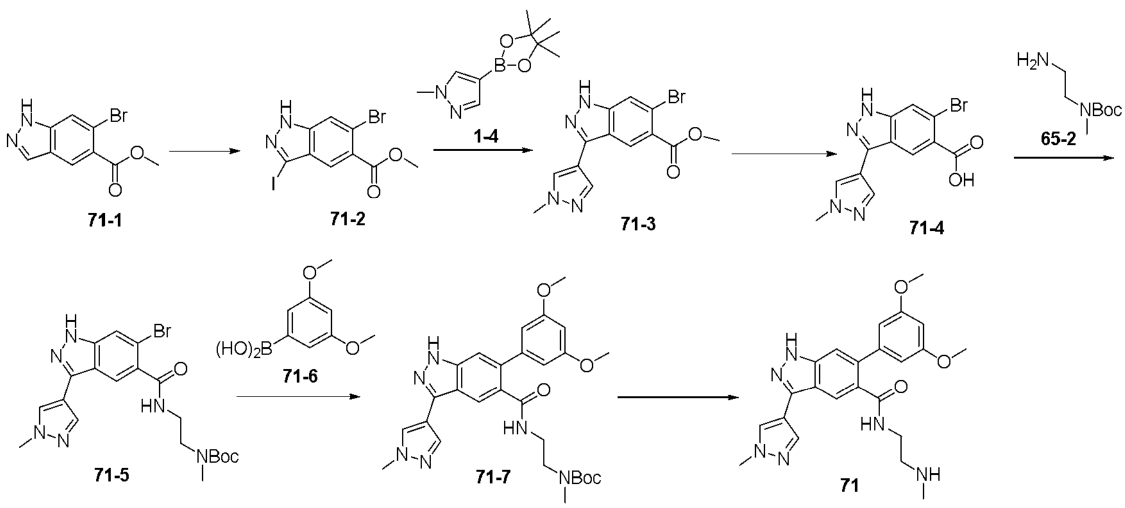


NIS (437 mg, 1.94 mmol, 1.5 eq) was added to a solution of Compound 71-1 (330 mg, 1.29 mmol, 1.0 eq) in DMF (13 mL), and reacted at 50°C for 4 hours. LCMS showed the generation of a product. The reaction mixture was cooled, extracted with EA and water three times, washed with saturated brine, and concentrated under vacuum. The residue was purified by silica gel column chromatography (eluent: PE:EA=4:1) and concentrated to obtain a crude Compound 71-2 as a light yellow oil liquid (purity 90%, LCMS (ESI) m/z 380.87, (M+H)+) which was directly used for the next reaction step.

$Pd(dppf)Cl_2$ (187 mg, 0.258 mmol, 0.2eq) and potassium carbonate (416 mg, 3.2 mmol, 2.5 eq) were added to a solution of the crude Compound 71-2 and Compound 1-4 (295 mg, 1.42 mmol, 1.1 eq) in dioxane/water (12 mL/3mL), purged with $N_2$ for replacing air, and reacted at 80°C for 12 h. LCMS showed the generation of a product. The reaction mixture was cooled, and concentrated under vacuum. Then, the residue was purified by silica gel column chromatography (eluent: DCM:$CH_3OH$=15:1) and concentrated to obtain Compound 71-3 (600 mg, purity 70%, yield 65% for 2 steps): LCMS (ESI) m/z 337.02 (M+H)+.

LiOH (120 mg, 4.98 mmol, 4.0 eq) was added to a solution of Compound 71-3 (420 mg, 1.24 mmol, 1.0 eq) in THF/$H_2O$ (10 mL/10 mL), and reacted at 50°C for 12 h. LCMS showed the generation of a product. The reaction solution was directly concentrated under vacuum, the residue was extracted with DCM and water, and the extract was concentrated to obtain a crude Compound 71-4 which was directly used for the next reaction step. LCMS (ESI) m/z320.19, (M+H)+.

HATU (425 mg, 1.12 mmol, 1.5 eq) and HOAT (152 mg, 1.12 mmol, 3.0 eq) were added to a solution of the crude Compound 71-4 and Compound 65-2 (0.2 mL, 1.12 mmol, 1.5 eq) and DIPEA (0.40 mL, 2.24 mmol, 3.0 eq) in DMF (8 mL), and reacted at room temperature for 3 hours. LCMS showed the generation of a product. The reaction solution was extracted with EA and $H_2O$ three times, and washed with saturated brine to ensure the reaction solution was free of DMF. The extract was purified by silica gel column chromatography (eluent: DCM:$H_2O$=10:1) to obtain Compound 71-5 (100 mg, yield 30% for 2 steps, LCMS (ESI) m/z 477.12, (M+H)+).

$Pd(dppf)Cl_2$ (30 mg, 0.04 mmol, 0.2eq) and potassium carbonate (83 mg, 0.6 mmol, 3.0 eq) were added to a solution of Compound 71-5 (100 mg, 0.2 mmol, 1.0 eq) and Compound 71-6 (42 mg, 0.22 mmol, 1.1eq) in dioxane/water (2 mL/ 0.5 mL), purged with $N_2$ for replacing air, and reacted at 90°C for 12 h. LCMS showed the generation of a product. The reaction solution was directly concentrated under vacuum, and the residue was purified by silica gel column chromatography (eluent: DCM:$H_2O$=10:1) and concentrated to obtain Compound 71-7 (10 mg, yield 9.4%): LCMS (ESI) m/z 535.26, (M+H)+.

2 mL of HCl-dioxane solution was added to a solution of Compound 71-7 (10 mg) in DCM (2 mL), and reacted under stirring at room temperature for 3 h. LCMS showed the generation of a product. The hydrochloride salt of Compound 71 (1 mg, purity 94.87 %, yield 12.3%) was obtained by preparative high performance liquid chromatography. LCMS (ESI) m/z 435, (M+H)+; $^1H$ NMR (400 MHz, DMSO) $\delta$ 9.07 (s, 2H), 8.70 (s, 1H), 8.61 (s, 1H), 8.45 (s, 1H), 8.03 (s, 1H), 7.39 (s, 1H), 7.01 (s, 2H), 6.50 (s, 1H), 3.96 (s, 3H), 3.90 (s, 6H), 3.61 (q, J = 5.6 Hz, 2H), 3.17 - 3.09 (m, 2H), 2.61 (t, J = 5.4 Hz, 3H)).

Example 72

6-methoxy-3-(1-methyl-1H-pyrazol-4-yl)-N-(2-(methylamino)ethyl)-1H-indazole-5-carboxamide

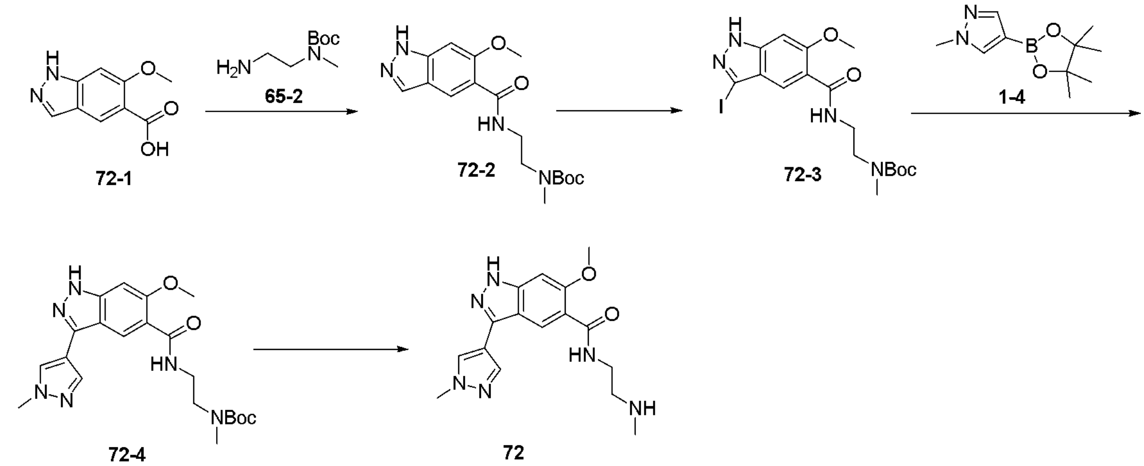

At room temperature, HATU (795 mg, 2.09 mmol, 1.5 eq) was slowly added to a solution of Compound 72-1 (268 mg, 1.39 mmol, 1.0 eq), Compound 65-2 (364 mg, 2.09 mmol, 1.5 eq) and DIPEA (0.75 mL, 4.17 mmol, 3.0 eq) in DMF (10 mL). The reaction solution was stirred at room temperature for 4 hours. Saturated aqueous sodium bicarbonate solution (40.0 mL) was added, and the reaction solution was extracted with ethyl acetate (20.0 mL $\times$ 2). The organic phases were combined, washed with saturated brine (20.0 mL), dried over anhydrous sodium sulfate, filtered, and concentrated under reduced pressure. The resulting residue was purified by silica gel column chromatography (eluent: (dichloromethane:methanol=4:1)/dichloromethane=0-16%) to obtain Compound 72-2 (464 mg, yield 95.5%).

At room temperature, Compound 72-2 (464 mg, 1.33 mmol, 1.0 eq) and NIS (330 mg, 1.46 mmol, 1.1 eq) were added successively to DMF (7.0 mL). The reaction mixture was stirred at room temperature for 16 hours. The reaction solution was cooled to room temperature, water (30.0 mL) was added, and then the reaction solution was extracted with ethyl acetate (15.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-15%) to obtain Compound 72-3 (490 mg, yield 77.6%).

At room temperature, Compound 72-3 (247 mg, 0.52 mmol, 1.0 eq), Compound 1-4 (119 mg, 0.57 mmol, 1.1 eq), Pd(dppf)$Cl_2$ (75.5 mg, 0.1 mmol, 0.2 eq) and sodium carbonate (165 mg, 1.56 mmol, 3.0 eq) were added successively to dioxane/$H_2O$ (5 mL/1.3 mL). The reaction mixture was stirred at 90°C for 16 hours. The reaction solution was cooled to room temperature, and concentrated under reduced pressure. The resulting residue was purified by silica gel column chromatography (eluent: (dichloromethane:methanol=4:1)/dichloromethane=0-25%) to obtain Compound 72-4 (192 mg, yield 86.1%).

At room temperature, hydrochloric acid-dioxane solution (4M, 5 mL, 20 mmol, 44 eq) was slowly added to a solution of Compound 72-4 (192 mg, 0.45 mmol, 1.0 eq) in DCM (2.0 mL). The reaction solution was stirred at room temperature for 5 hours. The reaction solution was concentrated under reduced pressure, and EA (10 mL) was added to the resulting residue, stirred for 15 min, and filtered. The filter cake was dried under reduced pressure to obtain Compound 72 (130 mg, yield 79.8%, purity 99.2%). LCMS (ESI) m/z 329.2 (M+H)+; $^1$H NMR (400 MHz, DMSO) δ 8.98 (s, 2H), 8.54 (t, J = 5.7 Hz, 1H), 8.41 (s, 1H), 8.40 (s, 1H), 7.95 (s, 1H), 7.04 (s, 1H), 3.96 (s, 6H), 3.64 (t, J = 5.8 Hz, 2H), 3.10 (t, J = 5.8 Hz, 2H), 2.61 (t, J = 5.4 Hz, 3H).

The following compound was synthesized according to the the route of Example 72.

| Example | Name | Structure | LC-MS $(M+H)^+$ | $^1$H NMR |
|---|---|---|---|---|
| 73 | 6-fluoro-3-(1-methyl-1H-pyrazol-4-yl)-N-(2-(methylamino)ethyl )-1H-indazole-5-carboxamide | 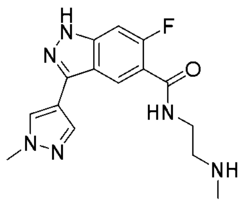 | 317 | $^1$H NMR (400 MHz, DMSO) δ 9.07 (s, 2H), 8.70 (s, 1H), 8.61 (s, 1H), 8.45 (d, J = 6.8 Hz, 1H), 8.03 (s, 1H), 7.39 (d, J = 11.2 Hz, 1H), 3.96 (s, 3H), 3.61 (q, J = 5.6 Hz, 2H), 3.17 - 3.09 (m, 2H), 2.61 (t, J = 5.4 Hz, 3H). |

Example 74

3-(1-methyl-1H-1,2,3-triazol-4-yl)-N-(2-(methylamino)ethyl)-1H-indazole-5-carboxamide

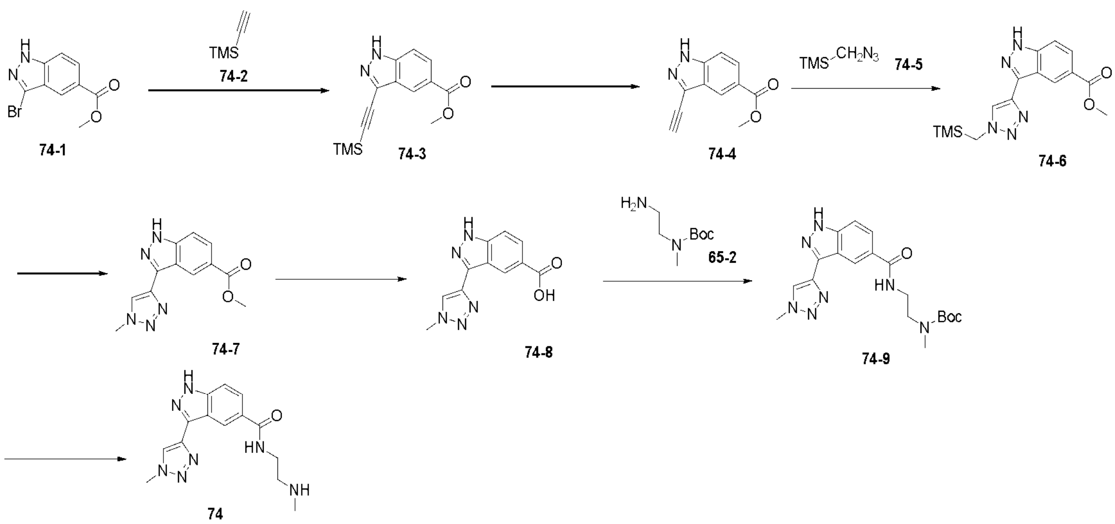

74-1 74-3 74-4 74-6 74-7 74-8 74-9 74

Compound 74-2 (1.15 g, 11.76 mmol, 1.5 eq), DIEA (2.0 g, 15.69 mmol, 2.0 eq), Pd(dppf)$Cl_2$ (400 mg, 0.2w) and

CuI (200 mg, 0.1 w) were added to a solution of Compound 74-1 (2 g, 7.84 mmol, 1.0 eq) in DMF (20 mL), purged with $N_2$ three times, warmed up to 100°C and reacted for 15 h. LCMS showed the generation of a product. The reaction solution was concentrated, and subjected to column chromatography (PE:EA=50%-80%) to obtain Compound 74-3 as a yellow solid (1.5 g, yield 70.4 %, purity 95%). LCMS (ESI) m/z 273.4 (M+H)+.

TBAF (3.17 g, 12.1 mmol, 1.5 eq) was added to a solution of Compound 74-3 (2.2 g, 8.08 mmol, 1.0 eq) in THF (20 mL), and reacted at 25°C for 2 h. LCMS showed the generation of a product. Water (50 mL) and EA (20 mL) were added to separate and wash the reaction solution, and concentration was carried out to obtain Compound 74-4 as a white solid (1.2 g, yield 74.0%, purity 95%). LCMS (ESI) m/z 201.2 (M+H)+.

Compound 74-5 (397.4 mg, 3.07 mmol, 1.5 eq), DIEA (615.82 mg, 4.10 mmol, 2.0 eq) and CuI (41 mg, 0.1 w) were added to a solution of Compound 74-4 (410 mg, 2.05 mmol, 1.0 eq) in DMF (5 mL), and reacted at 25°C for 2 h. LCMS showed the generation of a product. The reaction solution was concentrated, and subjected to column chromatography (PE:EA=50%-80%) to obtain Compound 74-6 (600 mg, yield90 %, purity 95%). LCMS (ESI) m/z 330.4 (M+H)+.

TBAF (714.3 mg, 2.73 mmol, 1.5 eq) was added to a solution of Compound 74-6 (600 mg, 1.82 mmol, 1.0 eq) in THF (6 mL), and reacted at 25°C for 2 h. LCMS showed the generation of a product. Water (50 mL) and EA (20 mL) were added to separate and wash the reaction solution, and concentration was carried out to obtain Compound 74-7 (374 mg, yield 80%, purity 95%). LCMS (ESI) m/z 258.3 (M+H)+.

LiOH (28.0 mg, 1.17 mmol, 3 eq) was added to a solution of Compound 74-7 (100 mg, 0.39 mmol, 1.0 eq) in MeOH/$H_2O$ (5 mL/1 mL), and reacted at 50°C for 15 h. LCMS showed the generation of a product. The reaction solution was subjected to rotary drying, and water (10 mL) and EA (10 mL) were added to separate and wash the reaction solution. The pH of the aqueous phase was adjusted to 3 to 4 with diluted hydrochloric acid (20 mL, 1 N), the aqueous phase was subjected to suction filtration, and the filter cake was dried to obtain Compound 74-8 (70 mg, yield74%, purity 95%). LCMS (ESI) m/z 244.2, (M+H)+.

HATU (142.3 mg, 0.37 mmol, 1.3eq) and DIEA(74.3 mg, 0.58 mmol, 2.0 eq) were added to a solution of Compound 74-8 (70 mg, 0.29 mmol, 1.0 eq) in DMF (2 mL), stirred for 20 min, and Compound 65-2 (65.25 mg, 0.37 mmol, 1.3 eq) was added, and reacted at 25°C for 15 h. LCMS showed the generation of a product. Water (50 mL) and EA (20 mL) were added to separate and wash the reaction solution, and concentration and column chromatography (PE:EA=50%-80%) were carried out to obtain Compound 74-9 (40 mg, yield 35%, purity 95%). LCMS (ESI) m/z 400.5, (M+H)+.

HCl/EA (5 mL) was added to a solution of Compound 74-9 (40 mg, 0.1 mmol, 1.0 eq) in MeOH (6 mL), and reacted at 25°C for 2 h. LCMS showed the generation of a product. The reaction solution was concentrated and subjected to C18 reversed-phase column chromatography ($H_2O$:ACN=30%-50%) to obtain Compound 74 (4 mg, yield 13%, purity 95%). LCMS (ESI) m/z 300.4, (M+H)+; $^1H$ NMR (400 MHz, DMSO) δ 13.49 (s, 1H), 8.87 (d, J = 12.2 Hz, 2H), 8.70 (d, J = 11.6 Hz, 3H), 7.96 (d, J = 8.2 Hz, 1H), 7.63 (d, J = 8.8Hz, 1H), 4.17 (s, 3H), 3.60 (s, 2H), 3.13 (s, 2H), 2.60 (s, 3H).

Example 75

N-methyl-2-(4-(3-(4-methyl-1H-imidazol-2-yl)-1H-indazol-5-yl)-1H-1,2,3-triazol-1-yl)ethan-1-amine

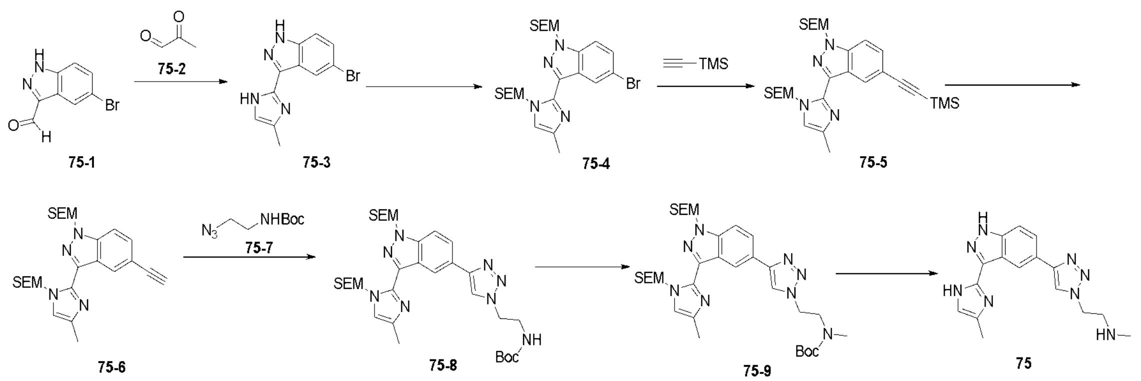


At 0°C, $NH_3/CH_3OH$ (7M, 3.2 mL, 22.2 mmol, 10 eq) was slowly added to a solution of Compound 75-1 (500 mg, 2.22 mmol, 1.0 eq) in $CH_3OH$ (6.0 mL). The reaction solution was stirred at 0°C for 10 minutes, and Compound 75-2 (40.81%, 2.0 mL, 11.1 mmol, 5 eq) was slowly added. The reaction mixture was stirred at 0°C for 10 minutes, then warmed up to room temperature, and stirred for 3 hours. The reaction mixture was concentrated under reduced pressure, and the resulting residue was purified by silica gel column chromatography (eluent: (dichloromethane:methanol=4:1)/dichloromethane=0-20%) to obtain Compound 75-3 (415 mg, yield 67.5%).

At 0°C, a solution of Compound 75-3 (415 mg, 1.498 mmol, 1.0 eq) in THF (8.0 mL) was slowly added to a suspension of sodium hydride (210 mg, 5.24 mmol, 3.5 eq) in THF (5.0 mL). The reaction mixture was warmed up to room temperature, stirred for 0.5 hour, and then cooled to 0°C, and SEMCl (0.69 mL, 3.89 mmol, 2.6 eq) was slowly added thereto. The reaction mixture was warmed up to room temperature, and stirred for 3 hours. The reaction solution was cooled to 0°C, slowly poured into aqueous $NH_4Cl$ solution (20.0 mL), and extracted with EA (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/petroleum ether=0-10%) to obtain Compound 75-4 (316 mg, yield 39.3%).

At room temperature, Compound 75-4 (312 mg, 0.58 mmol, 1.0 eq), trimethylethynylsilane (0.41 mL, 2.9 mmol, 5.0 eq), $Pd(PPh_3)_2Cl_2$ (122 mg, 0.17 mmol, 0.3 eq), CuI (34 mg, 0.17 mmol, 0.3 eq) and TEA (0.41 mL, 2.9 mmol, 5.0 eq) were added successively to DMF (4.5 mL). The reaction mixture was stirred at 80°C for 24 hours. The reaction solution was cooled to room temperature, water (20.0 mL) was added, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/petroleum ether=0-8%) to obtain Compound 75-5 (310 mg, yield 96.3%).

At room temperature, potassium carbonate (820 mg, 5.9 mmol, 10.5 eq) was added to a solution of Compound 75-5 (310 mg, 0.56 mmol, 1.0 eq) in $CH_3OH$ (8.0 mL). The reaction solution was stirred at room temperature for 2 hours. The reaction solution was concentrated under reduced pressure, and water (10.0 mL) and DCM (10.0 mL) were added to separate the reaction solution. The organic phase was dried over anhydrous sodium sulfate, filtered, and concentrated under reduced pressure to obtain Compound 75-6 (270 mg, yield 100%).

At room temperature, Compound 75-6 (245 mg, 0.51 mmol, 1.0 eq), Compound 75-7 (112 mg, 0.6 mmol, 1.2 eq) and $Cu(CH_3CN)_4.PF_6$ (20 mg, 0.05 mmol, 0.1 eq) were added successively to chloroform (8.0 mL). The reaction mixture was stirred at 40°C for 7 hours. The reaction mixture was concentrated under reduced pressure, and the resulting residue was purified by silica gel column chromatography (eluent: (dichloromethane:methanol=4:1)/dichloromethane=0-10%) to obtain Compound 75-8 (239 mg, yield 70.4%).

At 0°C, a solution of Compound 75-8 (120 mg, 0.18 mmol, 1.0 eq) in THF (2.0 mL) was slowly added to a suspension of sodium hydride (14.5 mg, 0.36 mmol, 2.0 eq) in THF (2.0 mL). The reaction mixture was warmed up to room temperature, stirred for 0.5 hour, and then cooled to 0°C, and $CH_3I$ (51 mg, 0.36 mmol, 2.0 eq) was slowly added. The reaction mixture was warmed up to room temperature, and stirred for 16 hours. The reaction solution was cooled to 0°C, slowly poured into aqueous $NH_4Cl$ solution (10.0 mL), and extracted with EA (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane: methanol=4:1 )/dichloromethane=0-18%) to obtain Compound 75-9 (65 mg, yield 53.1%).

At room temperature, TFA (2.0 mL) was slowly added to a solution of Compound 75-9 (65 mg, 0.095 mmol, 1.0 eq) in DCM (4.0 mL). The reaction mixture was stirred at room temperature for 5 hours. The reaction mixture was concentrated under reduced pressure, the resulting residue was dissolved in THF (3.0 mL), and ammonia water was added to adjust pH to >10. The reaction solution was stirred at room temperature for 0.5 hour. Water (10.0 mL) was added, and the reaction solution was extracted with DCM/i-PrOH=85/15 (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-93.3%) to obtain 75 (15 mg, yield 49%, purity 98.2%). LCMS (ESI) m/z 323.2 (M+H)+; $^1H$ NMR (400 MHz, DMSO) δ 13.27 (s, 1H), 12.40 (s, 1H), 8.82 (s, 1H), 8.62 (s, 1H), 7.91 (d, J = 8.5 Hz, 1H), 7.64 (d, J = 8.5 Hz, 1H), 6.84 (s, 1H), 4.58 (t, J = 6.0 Hz, 2H), 3.15 (t, J = 6.0 Hz, 2H), 2.41 (s, 3H), 2.28 (s, 3H).

Example 76

3-(1-methyl-1H-pyrazol-4-yl)-N-(2-(methylamino)ethyl)-1H-indazole-5-sulfonamide

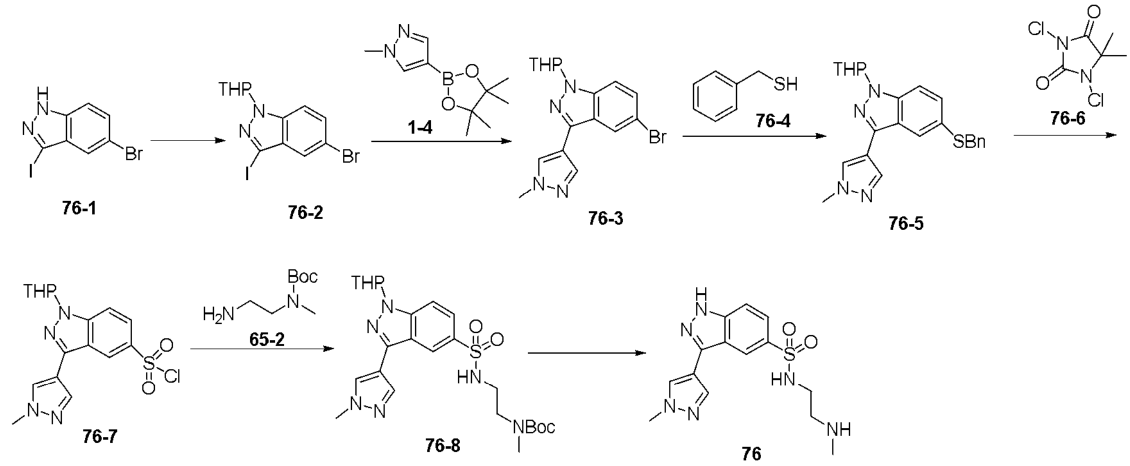


P-toluenesulfonic acid TsOH (122 mg, 0.64 mmol, 0.1 eq) and DHP (1.68 mL, 3.0 eq) were added to to a solution of Compound 76-1 (2 g, 6.19 mmol, 1.0 eq) in DCM (60 mL), and stirred at room temperature for 12 hours. LCMS showed the generation of a product. The reaction mixture was cooled, extracted with EA and water, washed with saturated brine, and concentrated under vacuum. The residue was purified by silica gel column chromatography (eluent: PE:EA=10:1) and concentrated to obtain 76-2 as a white solid (2.36 g, yield 93.9%): LCMS (ESI) m/z 406.92, (M+H)+.

Sodium carbonate (1.8 g, 17.01 mmol, 3.0 eq) and Pd(dppf)$Cl_2$ (800 mg, 1.1 mmol, 0.2 eq) were added to a solution of Compound 76-2 (2.31 g, 5.67 mmol, 1.0 eq) and Compound 1-4 (1.3 mg, 6.25 mmol, 1.1 eq) in 1, 4 dioxane/water (56 mL/14 mL), purged with $N_2$ three times, and reacted at 100°C overnight. The reaction solution was directly concentrated under vacuum, and the residue was purified by silica gel column chromatography (eluent: PE:EA=2:1) and concentrated to obtain Compound 76-3 (855 mg, yield74.1%): LCMS (ESI) m/z 361.06, (M+H)+.

$Pd_2(dba)_3$ (105 mg, 0.115 mmol, 0.05eq), DIPEA (0.7 mL, 3.0 eq) and Xant-tphos (133 mg, 0.23 mmol, 0.1 eq) were added to a solution of Compound 76-3 (855 mg, 2.375 mmol, 1.0 eq) and Compound 76-4 (0.3 mL, 1.1 eq) in dioxane (23 mL), and reacted at 100°C for 18 h. LCMS showed the generation of a product. The reaction solution was directly concentrated under vacuum, and the residue was purified by silica gel column chromatography (eluent: PE:EA=2:1) and concentrated to obtain Compound 76-5 (1.122 g, purity 74%): LCMS (ESI) m/z 405.17, (M+H)+.

Compound 76-6 (617 mg, 3.13 mmol, 3.0 eq) was added to a solution of Compound 76-5 (422 mg, 1.04 mmol, 1.0 eq) and 1 mL of acetic acid in THF/$H_2O$ (12 mL/3 mL) at 0°C, kept at this temperature and reacted for two hours. LCMS showed the generation of a product. The reaction solution was directly concentrated under vacuum, and the residue was extracted with EA and water, and washed with saturated brine. The residue was purified by silica gel column chromatography (eluent: PE:EA=4:1) and concentrated to obtain Compound 76-7 (326 mg, yield 82%): LCMS (ESI) m/z 381.06, (M+H)+.

DIPEA (0.26 mL, 1.5 mmol, 2.0 eq) was added to a solution of Compound 76-7 (284 mg, 0.75 mmol, 1.0 eq) and Compound 65-2 (156 mg, 0.89 mmol, 1.2 eq) in DCM (8 mL), and reacted at room temperature for 1 hour. LCMS showed the generation of a product. The reaction solution was directly concentrated under vacuum, and the residue was extracted with DCM and water, and washed with saturated brine. The residue was purified by silica gel column chromatography (eluent: DCM:$H_2O$=15:1) and concentrated to obtain Compound 76-8 (344 mg, yield 88.65%): LCMS (ESI) m/z 519.23, (M+H)+.

3 mL of HCl-dioxane solution was added to a solution of Compound 76-8 (344 mg, 0.66 mmol, 1.0 eq) in dioxane (3 mL), and stirred at room temperature for 2 h. LCMS showed the generation of a product. Sodium bicarbonate solution was added to adjust the pH of the reaction solution to neutral, and the residue was extracted with DCM and water, and washed with saturated brine. The residue was purified by silica gel column chromatography (eluent: DCM:$CH_3OH$=15:1), and concentrated to obtain Compound 76 (220.8 mg, yield 88%): LCMS (ESI) m/z 335.12, (M+H)+; $^1H$ NMR (400 MHz, DMSO) δ 8.91 (s, 2H), 8.54 (s, 1H), 8.50 (s, 1H), 8.14 (t, J = 5.6 Hz, 1H), 8.03 (s, 1H), 7.80 (dt, J = 19.6, 5.1 Hz, 2H), 3.99 (s, 3H), 3.07 - 2.94 (m, 4H), 2.56 - 2.53 (m, 3H). $^1H$ NMR (400 MHz, DMSO) δ 8.91 (s, 2H), 8.54 (s, 1H), 8.50 (s, 1H), 8.14 (t, J = 5.6 Hz, 1H), 8.03 (s, 1H), 7.80 (dt, J = 19.6, 5.1 Hz, 2H), 3.99 (s, 3H), 3.07 - 2.94 (m, 4H), 2.56 - 2.53 (m, 3H).

Example 79

3-(4-methyl-1H-imidazol-2-yl)-N-(pyrrolidin-3-ylmethyl)-1H-indazole-5-carboxa mide

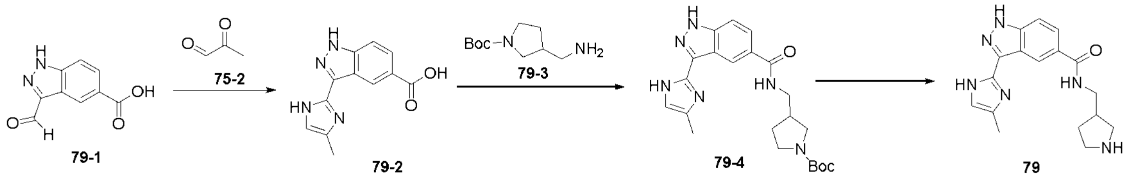


At 0°C, $NH_3/CH_3OH$ (7M, 3.0 mL, 21 mmol, 11 eq) was slowly added to a solution of Compound 79-1 (355 mg, 1.87 mmol, 1.0 eq) in $CH_3OH$ (5.0 mL). The reaction solution was stirred at 0°C for 10 minutes, and Compound 75-2 (40.81%, 1.6 mL, 9.35 mmol, 5 eq) was slowly added. The reaction mixture was stirred at 0°C for 10 minutes, then warmed up to room temperature, and stirred for 3 hours. After concentration under reduced pressure, the resulting residue was purified by silica gel column chromatography (eluent: (ethyl acetate:methanol=4:1)/ethyl acetate=0-49%) to obtain Compound 79-2 (445 mg, yield 98.45%).

At room temperature, HATU (240 mg, 0.63 mmol, 1.5 eq) was slowly added to a solution of Compound 79-2 (102 mg, 0.42 mmol, 1.0 eq), Compound 79-3 (127 mg, 0.63 mmol, 1.5 eq) and DIPEA (0.22 mL, 1.26 mmol, 3.0 eq) in DMF (2.0 mL). The reaction solution was stirred at room temperature for 2 hours. Saturated aqueous sodium bicarbonate solution (10.0 mL) was added, and the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-29%) to obtain Compound 79-4 (95 mg, yield 53.16%).

At room temperature, hydrochloric acid-dioxane solution (4M, 5 mL, 20 mmol, 89 eq) was slowly added to a solution of Compound 79-4 (95 mg, 0.224 mmol, 1.0 eq) in $DCM/CH_3OH$ (2.0 mL/2.0 mL). The reaction solution was stirred at room temperature for 3 hours. The reaction solution was concentrated under reduced pressure, water (10 mL) and EA (10 mL) were added to separate the reaction solution, and the aqueous phase was freeze-dried to obtain Compound 79 (80 mg, yield 90%, purity 96.43%): LCMS (ESI) m/z 325.2 (M+H)+; $^1H$ NMR (400 MHz, DMSO) δ 15.04 (s, 1H), 14.59 (s, 1H), 9.41 (s, 1H), 9.21 (s, 2H), 9.04 (t, J = 5.7 Hz, 1H), 8.06 (d, J = 8.8 Hz, 1H), 7.81 (d, J = 8.8 Hz, 1H), 7.57 (s, 1H), 3.64-3.57 (m, 2H), 3.37-3.3 (m, 1H), 3.29-3.21 (m, 1H), 3.22-3.09 (m, 1H), 3.06-2.98 (m, 1H), 2.71-2.59 (m, 1H), 2.45 (s, 3H), 2.14 - 2.03 (m, 1H), 1.84 - 1.71 (m, 1H).

The following compounds were synthesized according to the the route of Example 79.

| Example | Name | Structure | LC-MS $(M+H)^+$ | $^1H$ NMR |
|---|---|---|---|---|
| 80 | N-(2-(dimethylamino)eth yl)-3-(4-methyl-1H-imidazol-2-yl)-1H-indazole-5-carboxamide | 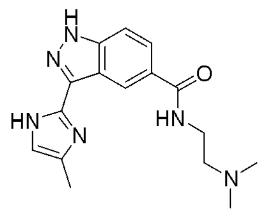 | 313 | $^1H$ NMR (400 MHz, DMSO) δ 8.88 (s, 1H), 8.66 (s, 1H), 8.43 (s, 1H), 8.25 (d, J = 8.3 Hz, 1H), 7.92 (dd, J = 8.8, 1.5 Hz, 1H), 7.60 (d, J = 8.8 Hz, 1H), 7.27 (dd, J = 8.2, 4.2 Hz, 1H), 6.88 (s, 1H), 2.88 (dd, J = 16.0, 9.7 Hz, 6H), 2.27 (s, 4H), 2.19 (d, J = 3.5 Hz, 7H). |
| 81 | N-((1H-imidazolin-2-yl)methyl)-3-(4-methyl-1H-imidazol-2-yl)-1H-indazole-5-carboxamide | 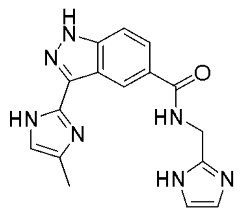 | 322 | $^1H$ NMR (400 MHz, DMSO) δ 14.67 (s, 3H), 9.69 (t, J = 5.3 Hz, 1H), 9.44 (s, 1H), 8.08 (d, J = 8.8Hz, 1H), 7.85 (d, J = 8.8 Hz, 1H), 7.64 (s, 2H), 7.59 (s, 1H), 4.85 (d, J = 5.3 Hz, 2H), 2.44 (s, 3H). |

Example 82

N-(2-(methylamino)ethyl)-3-(4-phenyl-1H-imidazol-2-yl)-1H-indazole-5-carboxa mide

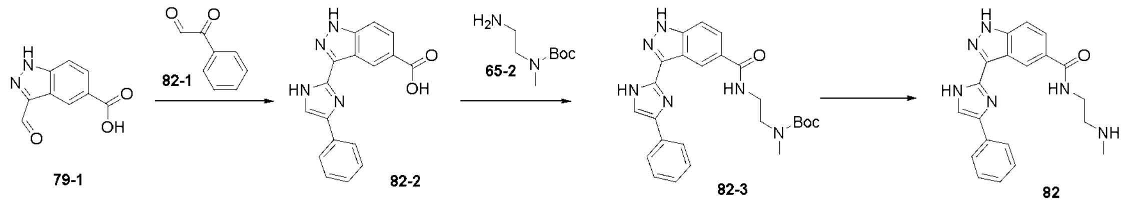


Compound 79-1 (120 mg, 0.63 mmol, 1.00 eq) was dissolved in methanol (3 mL), cooled to 0°C, and $NH_3/CH_3OH$ (7N, 1.01 mL, 10.00 eq) was slowly added, and stirred for 10 min. Then Compound 82-1 (480 mg, 3.15 mmol, 5 eq) was added, and reacted at 0°C for 10 min. Then the reaction solution was stirred at room temperature for 3 hours, concentrated under vacuum to obtain a crude, and the crude was subjected to column chromatography (eluent: 80%ethyl acetate/petroleum ether) to obtain Compound 82-2 as a yellow solid (110 mg, yield 57.3%, purity 90% ). LCMS (ESI) m/z 305, (M+H)+.

Compound 82-2 (50 mg, 0.16 mmol, 1.00 eq), Compound 65-2 (57 mg, 0.33 mmol, 2.0 eq), HATU (75 mg, 0.20 mmol, 1.2 eq) and DIPEA (64 mg, 0.49 mmol, 3.00 eq) were dissolved in DMF (2mL), and reacted at room temperature for 2 hours. Then, the reaction mixture was cooled, quenched with water (50.0 mL), and extracted with ethyl acetate (80.0 mL) three time. The organic phases were combined, washed with saturated brine (80.0 mL) twice, dried over anhydrous sodium sulfate, and filtered to obtain a filtrate. The filtrate was concentrated under vacuum to obtain a crude, and the crude was purified by column chromatography (eluent: 100% ethyl acetate/petroleum ether) to obtain Compound 82-3 as a yellow solid (50 mg, yield 65.7%, purity 100%). LCMS (ESI) m/z 461, (M+H)+.

Compound 82-3 (50 mg, 0.11 mmol, 1.00 eq) was dissolved in dichloromethane (2.0 mL), and hydrochloric acid/dioxane (2.0 mL) was added, and then stirred at 25°C for 1 hour. Then, the reaction mixture was filtered to obtain a target Compound 82 as a white solid (33.5 mg, yield 85%, purity 100%). LCMS (ESI) m/z 360.42, (M+H)+; $^1H$ NMR(400 MHz, DMSO) δ 14.36 (s, 1H), 9.21 (s, 1H), 9.03 (s, 1H), 8.92 (s, 2H), 8.22 (s, 1H), 8.11 - 8.03 (m, 3H), 7.80 (d,J = 9.0 Hz, 1H), 7.58 - 7.50 (m, 2H), 7.44 (d,J = 7.5 Hz, 1H), 3.76 - 3.66 (m, 2H), 3.22 - 3.12 (m, 2H), 2.69 - 2.60 (m, 3H).

Example 83

3-(5-methyl-1,4,5,6-tetrahydropyrrolo[3,4-d]imidazol-2-yl)-N-(2-(methylamino)et hyl)-1H-indazole-5-carboxamide

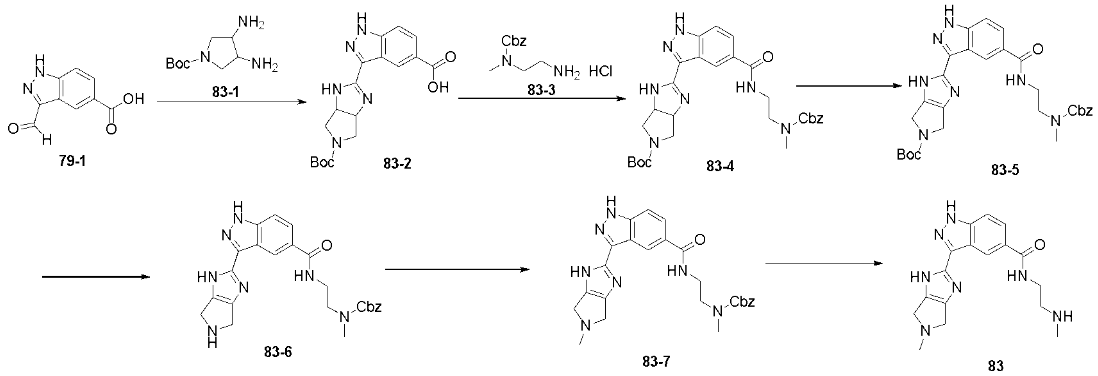


At room temperature, Compound 79-1 (100 mg, 0.526 mmol, 1.0 eq), Compound 83-1 (106 mg, 0.526 mmol, 1.0 eq), iodine (200 mg, 0.79 mmol, 1.5 eq) and potassium carbonate (218 mg, 1.58 mmol, 3.0 eq) were added successively to tert-butanol (20 mL). The reaction mixture was stirred at 70°C for 3 hours. The reaction solution was cooled to room temperature, concentrated under reduced pressure, and methanol (20 mL) was added, stirred for 15 min, and filtered. The filtrate was concentrated under reduced pressure to obtain Compound 83-2 (270 mg), which was directly used for the next reaction step without purification.

At room temperature, HATU (410 mg, 1.08 mmol, 1.5 eq) was slowly added to a solution of Compound 83-2 (270 mg, 0.727 mmol, 1.0 eq), Compound 83-3 (265 mg, 1.08 mmol, 1.5 eq) and DIPEA (0.65 mL, 3.67 mmol, 5.0 eq) in DMF (6.0 mL). The reaction solution was stirred at room temperature for 2 hours. Saturated aqueous sodium bicarbonate solution (20.0 mL) was added, and the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-32%) to obtain Compound 83-4 (303 mg, total yield 100% for two

steps).

At room temperature, Compound 83-4 (150 mg, 0.267 mmol, 1.0 eq) and IBX (150 mg, 0.534 mmol, 2.0 eq) were added successively to DMSO (10 mL). The reaction mixture was stirred at 50°C for 10 hours. The reaction solution was cooled to room temperature, water (30.0 mL) was added, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-28%) to obtain Compound 83-5 (80 mg, yield 53.5%).

At room temperature, zinc bromide (131 mg, 0.58 mmol, 4.0 eq) was added to a solution of Compound 83-5 (80 mg, 0.143 mmol, 1.0 eq) in THF (4.0 mL). The reaction solution was stirred at 50°C for 16 hours. The reaction solution was cooled to room temperature, concentrated under reduced pressure, and water (10.0 mL) and EA (10.0 mL) were added to separate the reaction solution, and the aqueous phase was freeze-dried to obtain Compound 83-6 (150 mg), which was directly used for the next reaction step without purification.

At room temperature, Compound 83-6 (100 mg, 0.22 mmol, 1.0 eq), aqueous formaldehyde solution (37%, 52 mg, 0.66 mmol, 3.0 eq) and two drops of acetic acid were added successively to methanol (3.0 mL). The reaction mixture was stirred at room temperature for 15 minutes. Sodium triacetoxyborohydride (192 mg, 0.9 mmol, 4.0 eq) was added, and the reaction mixture was stirred at room temperature for 5 hours. The reaction solution was was concentrated under reduced pressure, and the residue was purified by preparative high performance liquid chromatography to obtain Compound 83-7 (20 mg, total yield 44.4% for two steps).

At room temperature, Compound 83-7 (20 mg, 0.04 mmol, 1.0 eq) and palladium/carbon (palladium loading: 10 wt.%) (50 mg) were added successively to THF (2.0 mL) and DMF (2.0 mL). The reaction mixture was stirred at room temperature under a hydrogen atmosphere for 5 hours, filtered through diatomite, and the filtrate was concentrated under reduced pressure to obtain Compound 83 (2.5 mg, yield 17.5%, purity 100%).

LCMS (ESI) m/z 340.2 (M+H)+; $^1$H NMR (400 MHz, DMSO) δ 8.67 (s, 1H), 7.96 (dd, J = 8.8, 1.4 Hz, 1H), 7.59 (d, J = 8.7 Hz, 1H), 3.52-3.68 (m, 6H), 3.20 - 3.12 (m, 2H), 2.63 (t, J = 5.4 Hz, 3H), 2.20(s, 3H).

Example 84

N-(2-(methylamino)ethyl)-3-(5-(4-methylpiperazin-1-yl)-1H-benzo[d]imidazol-2-yl)-1H-indazole-5-carboxamide

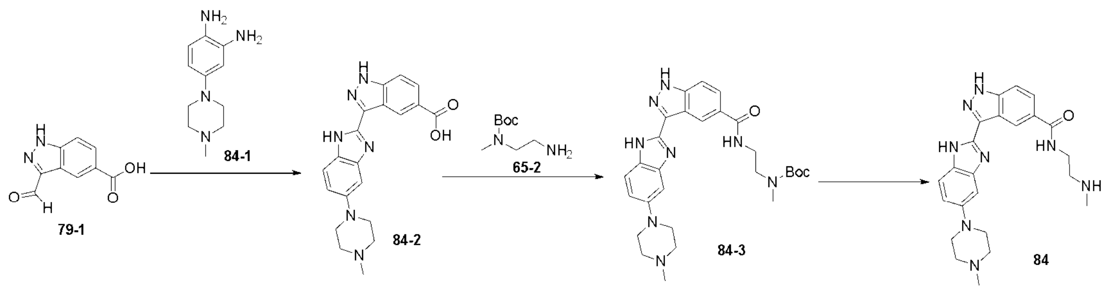

At room temperature, sodium hydrogen sulfite (147 mg, 1.41 mmol, 2.1 eq) was added to a solution of Compound 79-1 (145 mg, 0.76 mmol, 1.1 eq) and Compound 84-1 (140 mg, 0.68 mmol, 1.0 eq) in DMF (3.0 mL). The reaction solution was stirred at 160°C for 2 hours. The reaction solution was cooled to room temperature, and water (10 mL) and EA (10 mL) were added, stirred for 15 min, filtered, and the filter cake was dried under reduced pressure to obtain Compound 84-2 (120 mg, purity 73%, yield 34.3%).

At room temperature, HATU (180 mg, 0.48 mmol, 1.5 eq) was slowly added to a solution of Compound 84-2 (120 mg, purity 73%, 0.32 mmol, 1.0 eq), Compound 65-2 (83 mg, 0.48 mmol, 1.5 eq) and DIPEA (0.17 mL, 0.96 mmol, 3.0 eq) in DMF (3.0 mL). The reaction solution was stirred at room temperature for 2 hours. Saturated aqueous sodium bicarbonate solution (10.0 mL) was added, and the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-65%) to obtain Compound 84-3 (100 mg, yield 80.65%).

At room temperature, hydrochloric acid-dioxane solution (4M, 5 mL, 20 mmol, 105 eq) was slowly added to a solution of Compound 84-3 (100 mg, 0.19 mmol, 1.0 eq) in DCM (2.0 mL). The reaction solution was stirred at room temperature for 6 hours. The reaction solution was concentrated under reduced pressure, and water (10 mL) and EA (10 mL) were added to separate the reaction solution, and the aqueous phase was freeze-dried to obtain Compound 84 (95

mg, yield 87.9%, purity 97.9%). LCMS (ESI) m/z 433.2 (M+H)+; $^1$H NMR (400 MHz, DMSO) δ 14.78 (s, 1H), 11.07 (s, 1H), 9.54 (s, 1H), 9.10 - 9.03 (m, 1H), 8.93 (s, 2H), 8.11 (dt, J = 5.2, 2.6 Hz, 1H), 7.88 (d, J = 9.1 Hz, 1H), 7.80 (d, J = 9.1 Hz, 1H), 7.44 - 7.39 (m, 1H), 7.31 (s, 1H), 3.72 - 3.69 (m, 4H), 3.29-3.19 (m, 6H), 2.87 (s, 3H), 2.68-2.62 (m, 5H).

Comparative Example 85

(5-(5-((R)-1-(3,5-dichloropyridin-4-yl)ethoxy)-1H-indazol-3-yl)pyridin-2-yl)(imin o)(methyl)-sulfanone

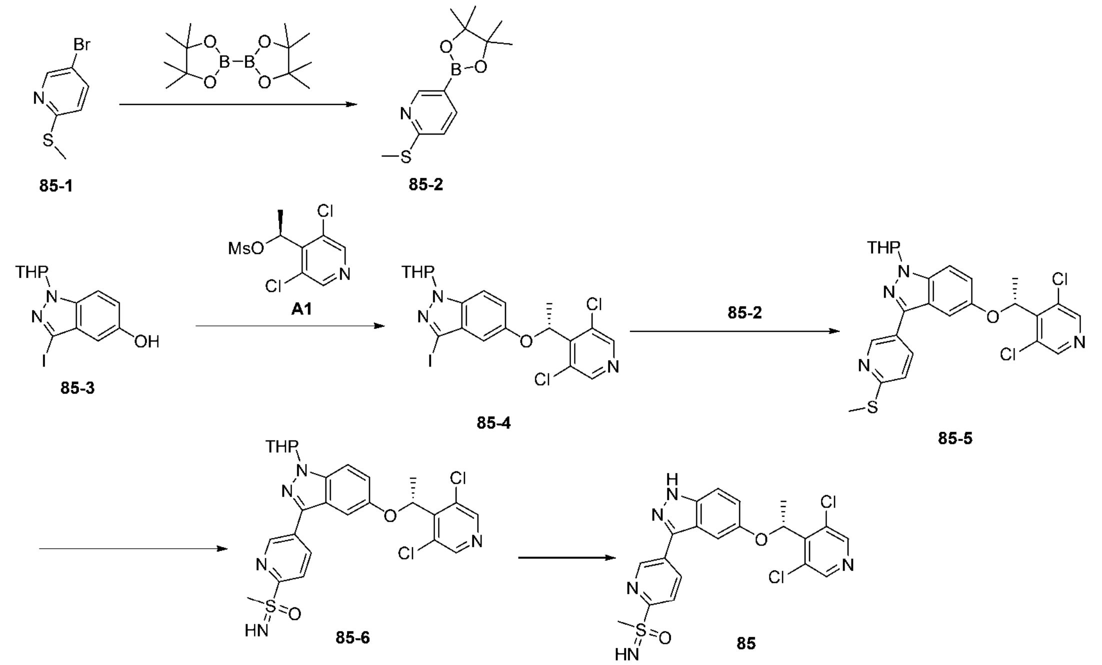


Compound 85-1 (2.0 g, 9.8 mmol, 1.0 eq) was added to a 100 mL single-neck flask, dioxane /KOAc (20mL) was added, and then bis(pinacolato)diboron and Pd (dppf)$Cl_2$ (800mg, 0.98 mmol, 0.1eq) were added successively, purged with $N_2$, and then stirred at 80°C for 12 h. LCMS showed the generation of a product. 30 mL of water was added to the reaction mixture, and then 30 mL of EA was added to extract the reaction mixture three times. The organic phases were combined, dried over anhydrous sodium sulfate, subjected to rotary drying, mixed with silica gel, and purified by silica gel column chromatography to obtain 2.0 g of Compound 85-2 as a yellow solid, with a yield of 81.3%.

Compound 85-3 (2.0 g, 5.8 mmol, 1.0 eq) and Compound A1 (2.35 g, 8.7 mmol, 1.5 eq) were dissolved in ACN (20 mL), and $Cs_2CO_3$ (5.7g, 17.4 mmol, 3.0 eq) was added, and stirred at 80 °C for 6 hours. LCMS showed the generation of a product. 30 mL of water was added to the reaction mixture, and then 30 mL of EA was added to extract the reaction mixture three times. The organic phases were combined, dried over anhydrous sodium sulfate, subjected to rotary drying, mixed with silica gel, and purified by silica gel column chromatography to obtain 1.3g of Compound 85-4 as a white solid, with a yield of 43.3%.

Compound 85-4 (500 mg, 0.97 mmol, 1.0 eq) and Compound 85-2 (364 mg, 1.44 mmol, 1.5eq) were dissolved in dioxane/$H_2O$, $Cs_2CO_3$ (948mg, 2.91 mmol, 3.0 eq) and Pd(dppf)$Cl_2$ (79.2 mg, 0.097 mmol, 0.1 eq) were added successively, dissolved in dioxane (10 mL), and then 2 mL of water was added, purged with nitrogen, and stirred at 80°C for 12 h. LCMS showed the generation of a product. 20 mL of water was added to the reaction mixture, and then 20 mL of EA was added to extract the reaction mixture three times. The organic phases were combined, dried, concentrated, then mixed with silica gel, subjected to rotary drying, and then passed through a normal-phase column to obtain 370 mg of Compound 85-5 as a white solid, with a yield of 74.4%.

Compound 85-5 (370 mg, 0.72 mmol, 1.0 eq) was dissolved in methanol, and $CH_3COONH_4$ (83.2 mg, 1.08 mmol, 1.5 eq.) and iodobenzene diacetate (463.7 mg, 1.44 mmol, 2.0 eq.) were added successively, and stirred at room temperature for 3 hours. LCMS showed the generation of a product. 5 mL of water was added to the reaction mixture, and then 10 mL of EA was added to extract the reaction mixture three times. The organic phases were combined, dried, concentrated, then mixed with silica gel, subjected to rotary drying, and then passed through a normal-phase column to obtain 320 mg of Compound 85-6 as a white solid, with a yield of 81.6%.

Compound 85-6 (320 mg, 0.58 mmol, 1.0 eq) was dissolved in HCl/EA (4 mol, 10 mL) solvent, and stirred at room

temperature for 1 h. LCMS showed the generation of a product. The reaction solution was subjected to rotary drying, dried under vacuum with an oil pump and then passed through a reversed-phase column to obtain 83.4 mg of compound 85 as a white solid (yield: 30.9 %, purity 98.8% ). LCMS (ESI) m/z 463.35, (M+H)+); $^1$H NMR (400 MHz, DMSO) δ 9.28 (s, 1H), 8.60 (s, 2H), 8.55 (dd, J = 8.3, 1.8 Hz, 1H), 8.32 (d, J = 8.4 Hz, 1H), 7.60 (d, J = 9.2 Hz, 1H), 7.40 (d, J = 1.6 Hz, 1H), 7.17 (dd, J = 9.0, 1.8 Hz, 1H), 6.19 (d, J = 6.8 Hz, 1H), 3.59 (s, 3H), 1.79 (d, J = 6.6 Hz, 3H).

Example 86

3-(1-methyl-1H-pyrazol-4-yl)-5-(1-(tetrahydro-2H-pyran-4-yl)ethoxy)-1H-indazol e

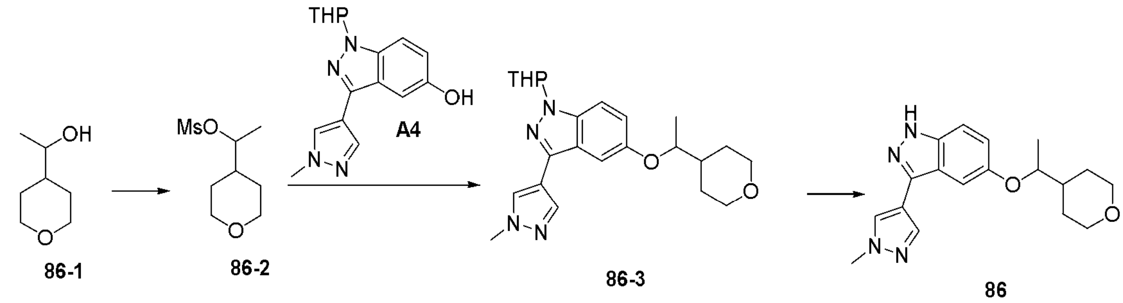

MsCl (176 mg, 1.54 mmol, 1.0 eq.) was added to a mixed system of Compound 86-1 (200 mg, 1.54 mmol, 1.0 eq.), TEA (311 mg, 3.07 mmol, 2.0 eq.) and DCM (5 mL) at 0°C, and reacted at 0°C for 0.5 h. TLC showed the complete reaction and the generation of a product spot. 30 mL of water was added to the reaction solution, and then the reaction solution was extracted with DCM three times (20 mL×3). The organic phases were combined, washed with saturated brine, dried over anhydrous sodium sulfate, filtered, and concentrated to obtain a crude Compound 86-2 as a colourless oil (350 mg). $^1$H NMR (400 MHz, $CDCl_3$) δ 4.56 (t, *J* = 6.4 Hz, 1H), 3.97 (dd, *J* = 12.2, 4.2 Hz, 2H), 3.32 (m, 2H), 2.97 (s, 3H), 1.81 - 1.72 (m, 1H), 1.68 (m, 1H), 1.57 - 1.49 (m, 1H), 1.43 (m, 2H), 1.36 (d, *J* = 6.4 Hz, 3H).

Cesium carbonate (98 mg, 0.30 mmol, 2.0 eq.) was added to a mixed system of Compound 86-2 (45 mg, 0.15 mmol, 1.0 eq.), CompoundA4 (46 mg, 0.23 mmol, 1.5 eq.) and acetonitrile (3 mL), and reacted at 70°C overnight. TLC showed the complete reaction, and LCMS showed the generation of a product. The reaction solution was directly concentrated, 50 mL of water was added, and then the reaction solution was extracted with ethyl acetate twice (30 mL×2). The organic phases were combined, and concentrated to obtain a crude Compound 86-3 as a colourless oil (100 mg). LCMS (ESI) m/z 411.20, $(M+1)^+$.

Compound 86-3 (50 mg, 0.12 mmol, 1.0 eq.) was added to HCl/dioxane (4M, 2 mL), warmed up to 45°C, and reacted for 1 hour. TLC showed the complete reaction. 30 mL of saturated sodium bicarbonate solution was added, and then the reaction solution was extracted with EA twice (20 mL×2). The organic phases were combined, concentrated, and purified by preparative high performance liquid chromatography to obtain Compound 86 as a white solid (22 mg, 55%). LCMS (ESI) m/z 327.10, $(M+1)^+$, purity 99%; $^1$H NMR (400 MHz, $CDCl_3$) δ 8.01 (s, 1H), 7.86 (s, 1H), 7.30 (d, *J* = 9.0 Hz, 1H), 7.20 (d, *J* = 2.2 Hz, 1H), 7.03 (dd, *J* = 9.0, 2.2 Hz, 1H), 4.13 (t, *J* = 6.2 Hz, 1H), 4.07 - 4.00 (m, 3H), 3.98 (s, 3H), 3.68 (s, 2H), 3.40 (t, *J* = 11.5 Hz, 2H), 1.94 - 1.76 (m, 3H), 1.50 (m, 3H), 1.28 (d, *J* = 6.1 Hz, 3H).

The following compounds were synthesized according to the the route of Example 86.

| Exa mple | Name | Structure | LC-MS $(M+H)^+$ | $^1$H NMR |
|---|---|---|---|---|
| 88 | 5-(1-((3-(1-methyl-1H-pyra-zol-4-yl)-1H-inda-zol-5-yl)oxy)ethyl) quin oline | 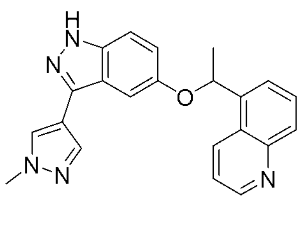 | 370.1 | $^1$H NMR (400 MHz, methanol-d4) δ 8.99 (d, J = 8.5 Hz, 1H), 8.94 - 8.88 (m, 1H), 7.97 (d, J = 8.4 Hz, 1H), 7.83 (d, J = 7.0 Hz, 1H), 7.77 - 7.71 (m, 2H), 7.68 - 7.62 (m, 2H), 7.37 (d, J = 9.1 Hz, 1H), 7.17 (dd, J = 9.0, 2.2 Hz, 1H), 7.08 - 7.04 (m, 1H), 6.23 (q, J = 6.3 Hz, 1H), 3.89 (s, 3H), 1.84(d,J=6.4 Hz, 3H). |

(continued)

| Example | Name | Structure | LC-MS (M+H)+ | 1H NMR |
|---|---|---|---|---|
| 90 | 5-((3-(1-methyl-1H-pyrazol-4-yl)-1H-indazol-5-yl)oxy)-5,6,7,8-tetrahydroisoquin oline | 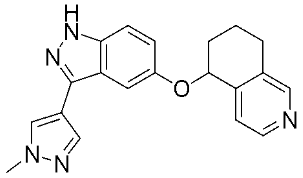 | 346.2 | $^1H$ NMR (400 MHz, $CD_3OD$) δ 8.36 (s, 1H), 8.30 (d, J = 5.1 Hz, 1H), 8.18 (s, 1H), 7.99 (s, 1H), 7.51 - 7.41 (m, 3H), 7.18 (d, J = 11.1 Hz, 1H), 5.52 (t, J = 5.5 Hz, 1H), 3.97 (s, 3H), 2.97 - 2.75 (m, 2H), 2.20 - 1.98 (m, 3H), 1.90 (s, 1H). |
| 91 | 2-(1-((3-(1-methyl-1H-pyrazol-4-yl)-1H-indazol-5-yl)oxy)ethyl) thia zole | 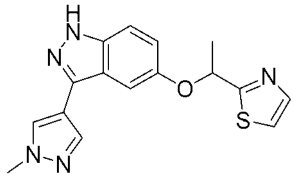 | 326.05 | $^1H$ NMR (400 MHz, $CD_3OD$) δ 8.11 (s, 1H), 7.93 (s, 1H), 7.78 (d, J = 3.3 Hz, 1H), 7.56 (d, J = 3.3 Hz, 1H), 7.43 (d, J = 9.0 Hz, 1H), 7.35 (d, J = 2.2 Hz, 1H), 7.16 (dd, J = 9.0, 2.3 Hz, 1H), 5.86 (q, J = 6.5 Hz, 1H), 3.99 (s, 3H), 1.78 (d, J = 6.4 Hz, 3H). |
| 96 | 5-(1-((3-(1-methyl-1H-pyrazol-4-yl)-1H-indazol-5-yl)oxy) ethyl)-[1,2,4]triazolo[1, 5-a]pyridine | 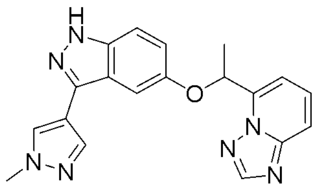 | 360.2 | $^1H$ NMR (400 MHz, DMSO) δ 12.85 (s, 1H), 8.70 (s, 1H), 8.08 (s, 1H), 7.87 - 7.82 (m, 1H), 7.78 (s, 1H), 7.71 (dd, J = 8.8, 7.3 Hz, 1H), 7.44 (d, J = 9.0 Hz, 1H), 7.38 (d, J = 7.1 Hz, 1H), 7.33 (s, 1H), 7.15 (dd, J = 9.0, 2.2 Hz, 1H), 6.33 (q, J = 6.3 Hz, 1H), 3.93 (s, 3H), 1.82 (d, J = 6.4 Hz, 3H). |
| 100 | 5-(1-((7-fluoro-3-(1-methyl-1H-pyrazol-4-yl)-1H-indazol-5-yl)oxy) ethyl)-6-methyl-quinoline | 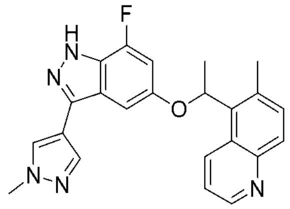 | 371.15 | $^1H$ NMR (400 MHz, DMSO) δ 13.25 (d, J = 17.6 Hz, 1H), 9.22 - 9.09 (m, 1H), 8.81 (t, J = 12.4 Hz, 1H), 8.81 (t, J = 12.4 Hz, 1H), 7.90 (d, J = 10.0 Hz, 1H), 7.82 (t, J = 7.6 Hz, 1H), 7.63 (s, 1H), 7.56 (t, J = 8.4 Hz, 2H), 7.03 - 6.83 (m, 1H), 6.39 - 6.28 (m, 1H), 3.87 (d, J = 19.2 Hz, 3H), 2.65 (d, J = 28.8 Hz, 3H), 1.82 (t, J = 14.4 Hz, 3H). |
| 101 | 8-(1-((3-(1-methyl-1H-pyrazol-4-yl)-1H-indazol-5-yl)oxy) ethyl)-[1,2,4]triazolo[1, 5-a]pyridine | 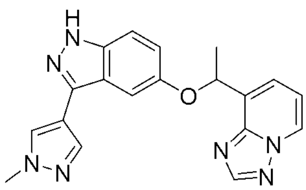 | 360.2 | $^1H$ NMR (400 MHz, DMSO) δ 12.85 (s, 1H), 8.70 (s, 1H), 8.08 (s, 1H), 7.84 (d, J = 8.6 Hz, 1H), 7.78 (s, 1H), 7.74 - 7.68 (m, 1H), 7.45 (d, J = 9.0 Hz, 1H), 7.38 (d, J = 7.1 Hz, 1H), 7.32 (s, 1H), 7.15 (dd, J = 9.0, 2.1 Hz, 1H), 6.37 - 6.29 (m, 1H), 3.93 (s, 3H), 1.82 (d, J = 6.3 Hz, 3H) |

Example 87

4-(1-((3-(1-methyl-1H-pyrazol-4-yl)-1H-indazol-5-yl)oxy)ethyl)quinoline

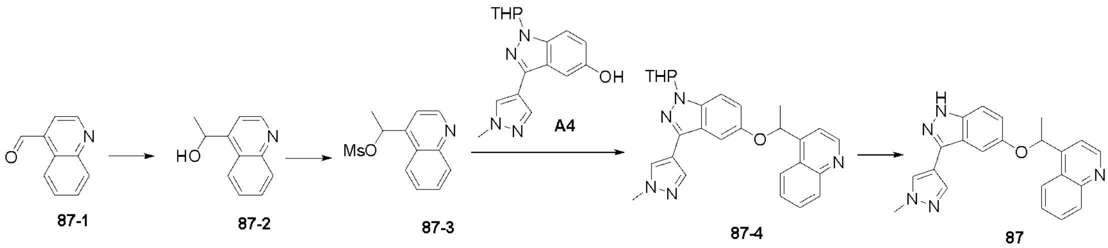

A4

87-1 87-2 87-3 87-4 87

Compound 87-1 (50 mg, 0.32 mmol, 1.0 eq.) was added to a 25 mL dried three-necked flask at room temperature, and dry THF (3.0 mL) was added, purged with nitrogen multiple times, and then cooled in an ice salt bath. $CH_3MgBr$ (0.23 mL, 0.67 mmol, 2.1 eq.) was added dropwise at 0°C, kept at low temperature and reacted for 2 hours. TLC showed the complete reaction and the generation of a product spot. 10 mL of water was added to the reaction solution, and then the reaction solution was extracted with EA three times (5.0 mL×3). The organic phases were combined, washed with saturated brine, dried over anhydrous sodium sulfate, filtered, and concentrated to obtain a crude product Compound 87-2 (60 mg) which was directly used for the next reaction step: LCMS: (ESI) m/z 174.05, $(M+H)^+$.

Compound 87-2 (60 mg, 0.35 mmol, 1.0 eq) was completly dissolved in DCM (3.0 mL) at room temperature, TEA (141 mg, 1.4 mmol, 4.0 eq) was added at room temperature, purged with nitrogen, cooled in an ice water bath, and MsCl (81 mg, 0.7 mmol, 2.0 eq) was added dropwise, and reacted at room temperature for 2 hours. TLC monitoring showed the generation of a new spot and the disappearance of raw material spot. LCMS monitoring showed the generation of a product. 5.0 mL of water was added to the reaction solution, and then the reaction solution was extracted with DCM twice (5.0 mL×2). The organic phases were combined, and washed and extracted with saturated aqueous $NaHCO_3$ solution twice (10.0 mL×2). The organic phase was dried over anhydrous sodium sulfate, and subjected to rotary drying to obtain Compound 87-3 (80 mg, 91%): LCMS (ESI) m/z 252.05, $(M+1)^+$.

Compound 87-3 (90 mg, 0.36 mmol, 1.5 eq) was dissolved in MeCN (5.0 mL) at room temperature, and Compound A4 (70 mg, 0.23 mmol, 1.0 eq) and $Cs_2CO_3$ (150 mg, 0.46 mmol, 2.0 eq) were added under stirring, purged with nitrogen, and reacted at 80°C overnight. TLC monitoring showed the disappearance of raw materials and the generation of a new spot, and LCMS monitoring showed the generation of a product. 10.0 mL of water was added to the reaction solution, and then the reaction solution was extracted with EA three times (5.0 mL×3). The organic phases were combined, dried over anhydrous sodium sulfate, subjected to rotary drying and prep-TLC (DCM/MeOH=20/1) to obtain a crude product Compound 87-4 (108 mg), which was directly used for the next step. LCMS (ESI) m/z 452.90, $(M+1)^+$ .

Compound 87-4 (100 mg, 0.22 mmol, 1.0 eq) was completely dissoved in MeOH (3.0 mL), 4.0 N HCl-dioxane solution was added, and reacted at room temperature for 3 hours. LCMS monitoring showed the disappearance of Compound 87-4 which was completely converted into a product. The reaction solution was concentrated to dryness, and purified by preparative high performance liquid chromatography to obtain a final product Compound 87 as a white solid (45.4 mg, 55.7%). LCMS (ESI) m/z 370.05, $(M+H)^+$; $^1$H NMR (400 MHz, $CD_3OD$) δ 8.81 - 8.76 (m, 1H), 8.51 (d, J = 8.9 Hz, 1H), 8.12 (d, J = 8.4 Hz, 1H), 7.88 (t, J = 8.2 Hz, 1H), 7.80 (t, J = 7.7 Hz, 1H), 7.71 (d, J = 4.6 Hz, 1H), 7.62 (d, J = 4.9 Hz, 2H), 7.40 (d, J = 9.0 Hz, 1H), 7.22 - 7.17 (m, 1H), 7.03 - 6.99 (m, 1H), 6.33 (q, J = 6.5 Hz, 1H), 3.85 (s, 3H), 1.83 (d, J = 6.4 Hz, 3H).

The following compounds were synthesized according to the the route of Example 87.

| Example | Name | Structure | LC-MS $(M+H)^+$ | $^1$H NMR |
|---|---|---|---|---|
| 89 | 5-(1-(imidazo[1, 2-a]pyridine-7-yl) ethoxy)-3-(1-methyl-1H-pyrazol-4-yl)-1H-indazole | 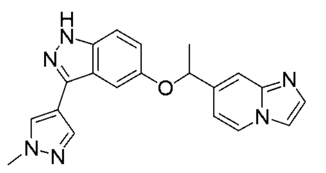 | 359.45 | $^1$H NMR (400 MHz, $CD_3OD$) δ 8.54 (s, 1H), 7.97 (s, 1H), 7.91 (s, 1H), 7.81 (s, 1H), 7.76 (s, 1H), 7.70 (s, 1H), 7.33 (s, 2H), 7.21 (s, 1H), 7.11 (s, 1H), 5.66 (s, 1H), 3.88 (s, 3H), 3.22 (s, 3H). |
| 92 | 5-(1-((3-(1-methyl-1H-pyrazol-4-yl)-1H-indazol-5-yl)oxy)ethyl) quin oxaline | 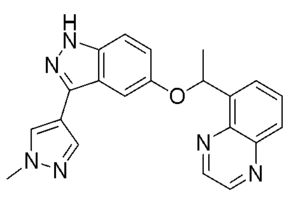 | 371.15 | $^1$H NMR (400 MHz, DMSO) δ 12.76 (s, 1H), 9.13 (dd, J = 16.8, 1.7 Hz, 2H), 8.07 - 7.98 (m, 2H), 7.93 (s, 1H), 7.90 - 7.84 (m, 1H), 7.55 (s, 1H), 7.39 (d, J = 9.0 Hz, 1H), 7.19 (s, 1H), 7.11 (dd, J = 9.0, 2.2 Hz, 1H), 6.75 (q, J = 6.3 Hz, 1H), 3.90 (s, 3H), 1.76 (d, J = 6.3 Hz, 3H). |

(continued)

| Example | Name | Structure | LC-MS $(M+H)^+$ | $^1H$ NMR |
|---|---|---|---|---|
| 93 | 4-(1-((3-(1-methyl-1H-pyrazol-4-yl)-1H-indazol-5-yl)oxy) ethyl)-1,8-naphthyridine | 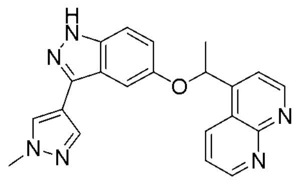 | 371.15 | $^1H$ NMR (400 MHz, DMSO) δ 9.30 (d, J = 9.1 Hz, 2H), 9.21 (d, J = 4.7 Hz, 1H), 8.14 (s, 1H), 8.03 (d, J = 4.7 Hz, 1H), 7.98 (dd, J = 8.3, 4.6 Hz, 1H), 7.81 (d, J = 4.1 Hz, 1H), 7.42 (dd, J = 14.7, 5.4 Hz, 2H), 7.18 (dt, J = 10.5, 5.3 Hz, 1H), 6.62 (q, J = 6.3 Hz, 1H), 3.93 (d, J = 12.5 Hz, 5H), 1.78 (d, J = 6.4 Hz, 3H). |
| 94 | 3-chloro-5-(1-((3-(1-methyl-1H-pyrazol-4-yl)-1H-indazol-5-yl)oxy)ethyl) quin oline | 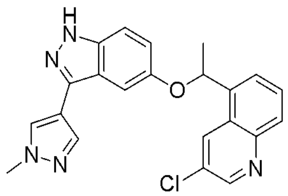 | 404.87 | $^1H$ NMR (400 MHz, DMSO) δ 12.78 (s, 1H), 9.12 (d, J = 1.8 Hz, 1H), 8.97 (d, J = 2.2 Hz, 1H), 8.10 (s, 1H), 7.98 (d, J = 8.4 Hz, 1H), 7.90 (d, J = 6.8 Hz, 1H), 7.78 (dd, J = 13.0, 5.4 Hz, 2H), 7.39 (d, J = 8.8 Hz, 1H), 7.33 (s, 1H), 7.12 (dd, J = 9.0, 2.1 Hz, 1H), 6.48 (q, J = 6.4 Hz, 1H), 3.91 (s, 3H), 1.75 (d, J = 6.4 Hz, 3H). |
| 97 | 5-(1-(2,3-dihydrobenzo[b][ 1,4]dioxan-5-yl) ethoxy)-3-(1-methyl-1H-pyrazol-4-yl)-1H-indazole | 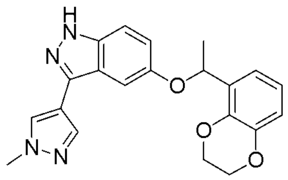 | 377.1 | - |
| 103 | 5-(1-(1-methyl-1H-benzo [d]imidazol -7-yl) ethoxy)-3-(1-methyl-1-H-pyrazol-4-yl)-1H-indazole | 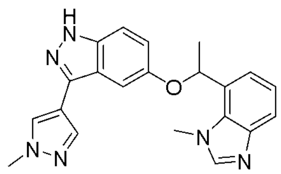 | 373.3 | $^1H$ NMR(400 MHz, DMSO) δ 12.72 (d,J = 51.0 Hz, 1H), 8.26 (s, 1H), 8.09 (s, 1H), 7.80 (s, 1H), 7.60 (d,J = 7.9 Hz, 1H), 7.49 (d,J = 7.4 Hz, 1H), 7.41 (d,J = 8.9 Hz, 1H), 7.34 (s, 1H), 7.22 (t,J = 7.8 Hz, 1H), 7.12 (dt,J = 10.4, 5.2 Hz, 1H), 6.40 - 6.22 (m, 1H), 4.18 (s, 3H), 3.94 (s, 3H), 1.80 (d,J = 6.3 Hz, 3H). |
| 104 | 5-(1-((3-(1-methyl-1H-pyrazol-4-yl)-1H-indazol-5-yl)oxy)ethyl) isoq uinoline | 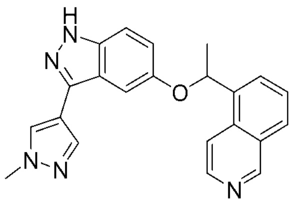 | 370.2 | $^1H$ NMR (400 MHz, DMSO) δ 9.53 (d, J = 25.2 Hz, 1H), 8.72 (s, 1H), 8.60 (t, J = 12.0 Hz, 1H), 8.18 (dd, J = 22.4, 7.7 Hz, 2H), 8.04 (s, 1H), 7.81 (t, J = 7.6 Hz, 1H), 7.74 (d, J = 22. Hz, 1H), 7.40 (d, J = 9.0 Hz, 1H), 7.28 (d, J = 14.0 Hz, 1H), 7.13 (t, J = 10.8 Hz, 1H), 6.45 (t, J = 11.2 Hz, 1H), 3.91 (s, 3H), 1.75 (t, J = 20.4 Hz, 3H). |
| 105 | 8-(1-((3-(1-methyl-1H-pyrazol-4-yl)-1H-indazol-5-yl)oxy)ethyl) quin oline | 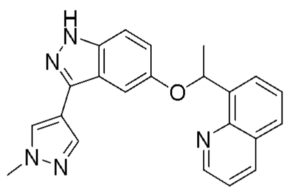 | 370.2 | $^1H$ NMR (400 MHz, DMSO) δ 12.77 (s, 1H), 9.21 - 9.14 (m, 1H), 8.47 (d, J = 8.3 Hz, 1H), 7.92 (t, J = 8.1 Hz, 2H), 7.76 (s, 1H), 7.71 (dd, J = 8.3, 4.2 Hz, 1H), 7.61 (t, J = 7.7 Hz, 1H), 7.49 (s, 1H), 7.39 (d, J = 8.9 Hz, 1H), 7.18 - 7.07 (m, 2H), 6.84 (q, J = 6.3 Hz, 1H), 3.88 (s, 3H), 1.75 (d, J = 6.3 Hz, 3H). |

Example 95

6-methyl-5-(1-((3-(1-methyl-1H-pyrazol-4-yl)-1H-indazol-5-yl)oxy)ethyl)quinolin e

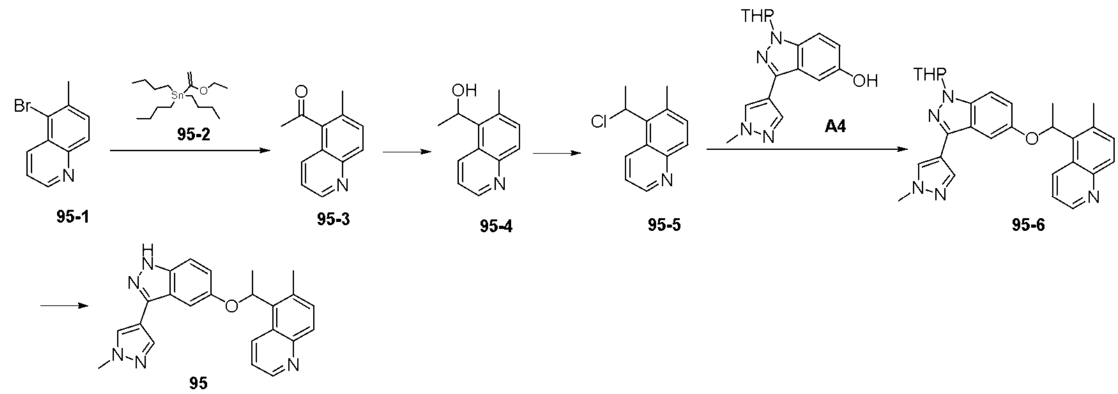


95-1 95-3 95-4 95-5 95-6 95

Tetrakis(triphenylphosphine)palladium (312.2 mg, 0.27 mmol, 0.2 eq) was added to a solution of Compound 95-1 (300 mg, 1.35 mmol, 1.0 eq) and Compound 95-2 (731.8 mg, 2.03 mmol, 1.5eq) in dioxane (10 mL), purged with $N_2$ three times, and stirred at 110 °C for 12 hours. LCMS showed the complete reaction of Compound 95-1. The reaction mixture was cooled, and 20 mL of saturated KF solution was added, stirred at room temperature for 30 min, filtered, then extracted with EA, and washed with saturated brine. The organic phase was concentrated to obtain a liquid as a yellow oil, and hydrochloric acid-dioxane solution (14 mL, 4M) was added, and stirred at room temperature for 30 min. LCMS showed the complete reaction. The pH was adjusted to 7 with saturated $NaHCO_3$ (20 mL), then the reaction mixture was extracted with ethyl acetate, separated and dried by rotary drying to obtain a liquid as a yellow oil, and the crude was purified by a 4 g silica gel column (0-50% EA) to obtain a product Compound 95-3 (230 mg, yield: 92%). LCMS (ESI) m/z 186.2, $(M+H)^+$ .

$NaBH_4$ (94 mg, 2.48 mol, 2.0 eq.) was added to a solution of Compound 95-3 (230 mg, 1.24 mmol, 1.0 eq.) in MeOH (15 mL), and stirred at room temperature for 12 hours. LCMS showed the generation of a product. The reaction solution was quenched with saturated $NH_4Cl$ solution, extracted with EA, separated and dried by rotary drying to obtain a yellow solid, which was subjected to column chromatography (0-50%EA) to obtain Compound 95-4 as an off-white solid (200 mg, yield 86%). LCMS (ESI) m/z 188.24, $(M+H)^+$.

Compound 95-4 (60 mg, 0.32 mmol, 1.0 eq.) and triethylamine (64 mg, 0.64 mmol, 2.0 eq.) were dissolved in DCM (3 mL), and MsCl (54 mg, 0.48 mmol, 1.5 eq.) was added dropwise at 0°C. Saturated aqueous $NaHCO_3$ solution (15 mL) was added to the reaction mixture, and then the reaction mixture was extracted with DCM (15 mL×3). The organic phase was separated, dried by rotary drying, and purified by TLC (PE:EA=1:1) to obtain Compound 95-5 as a yellow solid (30 mg, yield 45.5%): LCMS (ESI) m/z 206.07, $(M+H)^+$.

Compound 95-5 (30 mg, 0.146 mmol, 1.0 eq.) and Compound A4 (64 mg, 0.218 mmol, 1.5 eq.) were dissolved in DMF (2 mL), and $Cs_2CO_3$ (95 mg, 0.28 mmol, 2 eq.) was added, and reacted at 80°C for 3 hours. The reaction mixture was extracted with water and EtOAc. The organic phase was separated, dried by rotary drying, and purified by TLC (DCM:MeOH=10:1) to obtain Compound 95-6 as a yellow solid (25 mg, yield 36.6%): LCMS (ESI) m/z 468.23, $(M+H)^+$.

Compound 95-6 (30 mg, 0.146 mmol, 1.0 eq.) was dissolved in DCM (2 mL), and TFA (1 mL) was added, and reacted at 25°C for 3 hours. LCMS showed the complete reaction of Compound 95-6 and the generation of a product. The reaction solution was subjected to rotary drying to obtain a crude, which was purified by TLC (DCM:MeOH=10:1) to obtain Compound 95 as an off-white solid (15.8 mg, yield 73.2 %) LCMS (ESI) m/z 384.17, $(M+H)^+$; $^1H$ NMR (400 MHz, DMSO) $\delta$ 12.75 (s, 1H), 9.24 (d, J = 8.4 Hz, 1H), 8.86 (d, J = 3.2 Hz, 1H), 7.92 (s, 1H), 7.85 (d, J = 8.4 Hz, 1H), 7.66 (s, 1H), 7.60 (dd, J = 8.8, 4.0 Hz, 2H), 7.35 (d, J = 8.8 Hz, 1H), 7.11 - 7.04 (m, 2H), 6.35 (d, J = 6.4 Hz, 1H), 3.93 (s, 3H), 2.72 (s, 3H), 1.87 (d, J = 6.7 Hz, 3H).

The following compounds were synthesized according to the the route of Example 95.

| Exam ple | Name | Structure | LC-MS (M+H)+ | 1H NMR |
|---|---|---|---|---|
| 98 | 3-fluoro-5-(1-((3-(1-methyl-1H-pyrazol-4-yl)-1H-indazol-5-yl)oxy) ethyl)qu inoline | 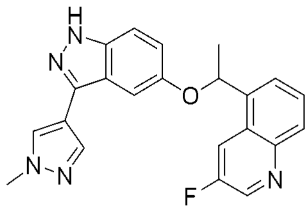 | 388.42 | $^1$H NMR (400 MHz, DMSO) δ 9.06 (d, J = 2.8 Hz, 1H), 8.88 (dd, J = 10.8, 2.4 Hz, 1H), 8.13 (s, 1H), 8.01 (d, J = 8.4 Hz, 1H), 7.91 (d, J = 7.2 Hz, 1H), 7.81 - 7.71 (m, 2H), 7.40 (d, J = 9.0 Hz, 1H), 7.33 (d, J = 1.8 Hz, 1H), 7.14 (dd, J = 9.0, 2.2 Hz, 1H), 6.43 (q, J = 6.6 Hz, 1H), 3.92 (s, 3H), 1.75 (d, J = 6.4 Hz, 3H). |
| 99 | 5-(1-((3-(1-methyl-1H-pyrazol-4-yl)-1H-indazol-5-yl) oxy)ethyl)qu inazoline | 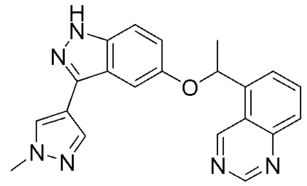 | 371.15 | $^1$H NMR (400 MHz, DMSO) δ 12.75 (d, J = 28.4 Hz, 1H), 10.21 (d, J = 17.2 Hz, 1H), 9.35 (d, J = 16.4 Hz, 1H), 8.12 (d, J = 11.2 Hz, 1H), 8.06 - 7.93 (m, 3H), 7.82 (s, 1H), 7.52 - 7.28 (m, 2H), 7.18 - 7.13 (m, 1H), 6.60 (q, J = 6.6 Hz, 1H), 3.93 (s, 3H), 1.81 (t, J = 9.2 Hz, 3H). |
| 102 | 6-fluoro-5-(1-((3-(1-methyl-1H-pyrazol-4-yl)-1H-indazol-5-yl)oxy) ethyl)qu inoline | 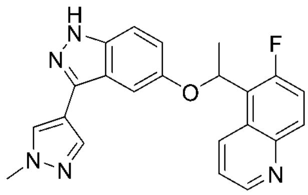 | 388.42 | $^1$H NMR (400 MHz, DMSO) δ 9.76 (t, J = 15.0 Hz, 1H), 9.25 (d, J = 4.708 Hz, 1H), 8.41 (dd, J = 9.4, 4.6 Hz, 1H), 8.18 (s, 1H), 8.13 (dd, J = 8.8, 5.0 Hz, 1H), 8.06 (t, J = 9.8 Hz, 1H), 7.85 (s, 1H), 7.40 (dd, J = 17.8, 5.4 Hz, 2H), 7.10 (dd, J = 9.0, 2.2 Hz, 1H), 6.52 (q, J = 6.6 Hz, 1H), 3.96 (s, 3H), 1.92 (d, J = 6.4 Hz, 3H). |
| 106 | 5-(1-(benzofuran-4-yl) ethoxy)-3-(1-methyl-1H-pyrazol-4-yl)-1H-indazole | 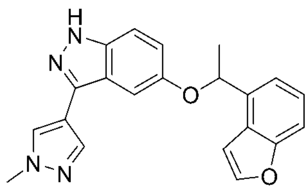 | 359.2 | $^1$H NMR(400 MHz, DMSO) δ 12.73 (s, 1H), 8.10 (s, 1H), 8.06 (d,J = 2.1 Hz, 1H), 7.79 (s, 1H), 7.49 (d,J = 8.2 Hz, 1H), 7.42 (d,J = 7.2 Hz, 1H), 7.36 (d,J = 5.8 Hz, 2H), 7.31 (d,J = 8.1 Hz, 1H), 7.27 (s, 1H), 7.10 (d,J = 7.2 Hz, 1H), 5.98 (d,J = 6.2 Hz, 1H), 3.94 (s, 3H), 1.71 (d,J = 6.3 Hz, 3H). |

(continued)

| Example | Name | Structure | LC-MS (M+H)+ | 1H NMR |
|---|---|---|---|---|
| 107 | 6-isopropyl-5-(1-((3-(1-methyl-1H-pyrazol-4-yl)-1H-indazol-5-yl)oxy) ethyl)qu inoline | 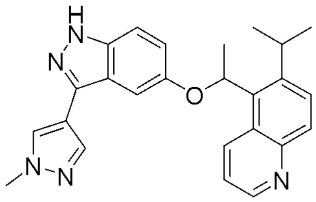 | 412.1 | $^1H$ NMR(400 MHz, DMSO) δ 12.77 (s, 1H), 9.29 (d,J = 9.0 Hz, 1H), 8.90 (s, 1H), 8.48 (s, 1H), 7.90 (t,J = 6.9 Hz, 1H), 7.75 (d,J = 8.9 Hz, 1H), 7.60 (dd,J = 32.4, 6.3 Hz, 2H), 7.33 (d,J = 9.1 Hz, 1H), 7.11 (s, 1H), 7.02 (dd,J = 9.0, 1.9 Hz, 1H), 6.56 (d,J = 6.8 Hz, 1H), 3.90 (s, 3H), 1.89 (d,J = 6.6 Hz, 3H), 1.28 (d,J = 6.7 Hz, 3H), 1.17 (d,J = 5.9 Hz, 3H). |
| 111 | 6-ethyl-5-(1-((3-(1-methyl-1H-pyrazol-4-yl)-1H-indazol-5-yl)oxy) ethyl)qu inoline | 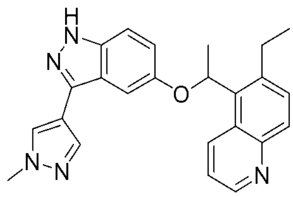 | 398.1 | $^1H$ NMR(400 MHz, DMSO) δ 12.75 (s, 1H), 9.28 (d,J = 8.8 Hz, 1H), 8.87 (d,J = 2.8 Hz, 1H), 7.95 (s, 1H), 7.89 (d,J = 8.7 Hz, 1H), 7.68 (s, 1H), 7.66 - 7.57 (m, 2H), 7.34 (d,J = 9.0 Hz, 1H), 7.17 (s, 1H), 7.04 (dd,J = 9.0, 2.2 Hz, 1H), 6.37 (d,J = 6.5 Hz, 1H), 3.93 (s, 4H), 3.18 (dq,J = 14.8, 7.3 Hz, 1H), 3.10 - 2.95 (m, 1H), 1.91 (d,J = 6.6 Hz, 3H), 1.25 (t,J = 7.5 Hz, 3H). |
| 120 | 5-(1-((3-(1-methyl-1H-pyrazol-4-yl)-1H-indazol-5-yl) oxy)ethyl)-1,6-naphthyridine | 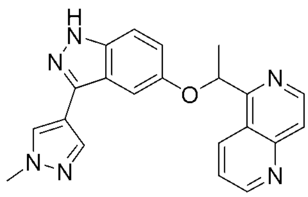 | 371.2 | $^1H$ NMR (400 MHz, $CD_3OD$) δ 9.27 (s, 1H), 9.07 (s, 1H), 8.72 (s, 1H), 7.91 (s, 1H), 7.87 (s, 1H), 7.75 (s, 1H), 7.70 (s, 1H), 7.34 (s, 1H), 7.17 (s, 2H), 6.19 (d, J = 6.7 Hz, 1H), 3.98 (s, 3H), 1.94 (d, J = 6.6 Hz, 3H) |
| 123 | 8-methyl-5-(1-((3-(1-methyl-1H-pyrazol-4-yl)-1H-indazol-5-yl)oxy) ethyl)qu inoline | 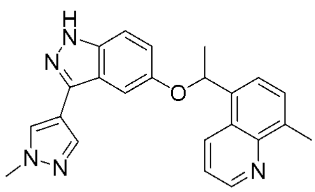 | 384.1 | $^1H$ NMR (400 MHz, DMSO-$d_6$) δ 12.72 (s, 1H), 8.96 (s, 2H), 8.01 (s, 1H), 7.69 (d, *J* = 16.8 Hz, 3H), 7.58 (s, 1H), 7.34 (d, *J* = 9.0 Hz, 1H), 7.25 (s, 1H), 7.08 (d, *J* = 9.1 Hz, 1H), 6.36 (d, *J* = 6.5 Hz, 1H), 3.87 (s, 3H), 2.65 (s, 3H), 1.73 (d, *J* = 6.3 Hz, 3H). |
| 124 | 7-fluoro-5-(1-((3-(1-methyl-1H-pyrazol-4-yl)-1H-indazol-5-yl)oxy) ethyl)qu inoline | 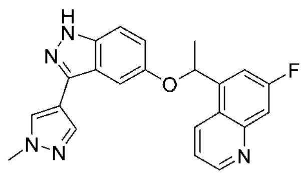 | 388.2 | $^1H$ NMR(400 MHz, DMSO) δ 12.79 (s, 1H), 9.06 - 8.93 (m, 2H), 8.07 (s, 1H), 7.77 (s, 1H), 7.74 (dd,J = 9.8, 2.6 Hz, 1H), 7.70 (dd,J = 10.1, 2.7 Hz, 1H), 7.66 (dd,J = 5.5, 3.7 Hz, 1H), 7.41 (d,J = 9.0 Hz, 1H), 7.33 (s, 1H), 7.15 (dd,J = 9.0, 2.2 Hz, 1H), 6.48 (q,J = 6.3 Hz, 1H), 3.91 (s, 3H), 1.77 (d,J = 6.3 Hz, 3H) |

(continued)

| Example | Name | Structure | LC-MS (M+H)+ | 1H NMR |
|---|---|---|---|---|
| 125 | 3-(1-((3-(1-methyl-1H-pyrazol-4-yl)-1H-indazol-5-yl)oxy)ethyl)thi eno[2,3-b] pyridine | 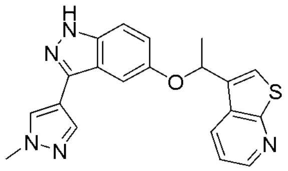 | 376.2 | 1H NMR(400 MHz, DMSO) δ 12.77 (s, 1H), 8.59 (dd,J = 4.6, 1.5 Hz, 1H), 8.53 (dd,J = 8.2, 1.6 Hz, 1H), 8.21 (s, 1H), 7.98 (s, 1H), 7.89 (s, 1H), 7.50 (dd,J = 8.1, 4.6 Hz, 1H), 7.44 (d,J = 1.8 Hz, 1H), 7.39 (d, J = 9.0 Hz, 1H), 7.11 (dd,J = 9.0, 2.2 Hz, 1H), 6.09 (q,J = 6.3 Hz, 1H), 3.94 (s, 3H), 1.76 (d,J = 6.4 Hz, 3H). |
| 126 | 4-methyl-5-(1-((3-(1-methyl-1H-pyrazol-4-yl)-1H-indazol-5-yl)oxy) ethyl)qu inoline | 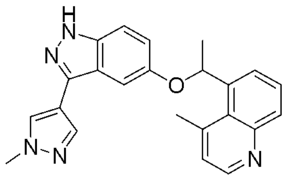 | 384.1 | 1H NMR (400 MHz, DMSO-d6) δ 9.21 (d, J = 5.5 Hz, 1H), 8.39 (dd, J = 8.4, 1.3 Hz, 1H), 8.29 (dd, J = 7.6, 1.3 Hz, 1H), 8.14 - 8.06 (m, 3H), 7.71 (s, 1H), 7.47 - 7.39 (m, 2H), 7.26 (d, J = 2.2 Hz, 1H), 7.13 (dd, J = 9.0, 2.3 Hz, 1H), 6.66 (q, J = 6.1 Hz, 1H), 3.90 (s, 3H), 3.32 (s, 3H), 1.81 (d, J = 6.1 Hz, 3H) |
| 128 | 3-methyl-5-(1-((3-(1-methyl-1H-pyrazol-4-yl)-1H-indazol-5-yl)oxy) ethyl)qu inoline | 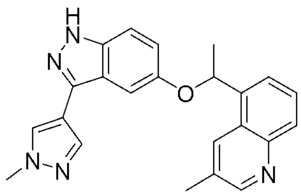 | 384.1 | 1H NMR (400 MHz, DMSO) δ 12.76 (s, 1H), 8.90 (s, 2H), 8.04 (s, 1H), 7.93 (d, J = 8.0 Hz, 1H), 7.82 (d, J = 8.0 Hz, 1H), 7.74 - 7.66 (m, 2H), 7.38 (d, J = 8.0 Hz, 1H), 7.28 (d, J = 2.0 Hz, 1H), 7.12 (dd, J = 9.0, 2.2 Hz, 1H), 6.44 (q, J = 6.0 Hz, 1H), 3.89 (s, 3H), 2.63 (s, 3H), 1.76 (d, J = 6.0 Hz, 3H). |
| 132 | 3-(1-methyl-1H-pyrazol-4-yl)-5-(1-(pyrazolo[1,5-a] pyridin-4-yl)ethoxy)-1H-indazole | 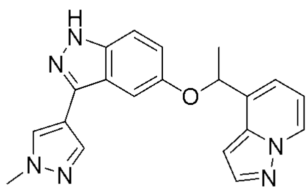 | 359.1 | 1H NMR (400 MHz, $CD_3OD$) δ 8.45 (d, *J* = 7.0 Hz, 1H), 8.05 (s, 1H), 7.79 (s, 1H), 7.75 (s, 1H), 7.34 (d, *J* = 13.3 Hz, 2H), 7.15 (d, *J* = 8.9 Hz, 1H), 7.11 (s, 1H), 6.96 (s, 1H), 6.86 (s, 1H), 5.78 (q, *J* = 6.4 Hz, 1H), 3.94 (s, 3H), 1.78 (d, *J* = 6.4 Hz, 3H). |
| 133 | 7-methyl-5-(1-((3-(1-methyl-1H-pyrazol-4-yl)-1H-indazol-5-yl)oxy) ethyl)qu inoline | 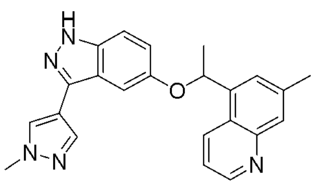 | 384.2 | 1H NMR (400 MHz, DMSO-$d_6$) δ 12.74 (s, 1H), 8.90 (d, *J* = 9.4 Hz, 2H), 8.00 (s, 1H), 7.73 - 7.66 (m, 3H), 7.56 (d, *J* = 5.6 Hz, 1H), 7.37 (d, *J* = 8.4 Hz, 1H), 7.25 (s, 1H), 7.10 (d, *J* = 10.3 Hz, 1H), 6.33 (d, *J* = 6.0 Hz, 1H), 3.87 (s, 3H), 2.48 (s, 3H), 1.74 (d, *J* = 6.3 Hz, 3H). |

(continued)

| Example | Name | Structure | LC-MS (M+H)+ | $^1$H NMR |
|---|---|---|---|---|
| 134 | 7-chloro-5-(1-((3-(1-methyl-1H-pyrazol-4-yl)-1H-indazol-5-yl)oxy)ethyl)qu inoline | 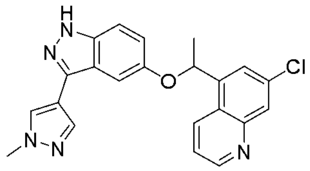 | 404.1 | $^1$H NMR (400 MHz, DMSO) δ 12.80 (s, 1H), 9.02 (dd, J = 6.2, 4.8 Hz, 2H), 8.08 (s, 1H), 8.02 (d, J = 2.0 Hz, 1H), 7.86 (d, J = 2.4 Hz, 1H), 7.77 (s, 1H), 7.71 (dd, J = 8.6, 4.4 Hz, 1H), 7.41 (d, J = 9.0 Hz, 1H), 7.32 (d, J = 2.0 Hz, 1H), 7.14 (dd, J = 9.0, 2.2 Hz, 1H), 6.44 (q, J = 6.2 Hz, 1H), 3.91 (s, 3H), 1.77 (d, J = 6.4 Hz, 3H). |
| 136 | 7-chloro-5-(1-((3-(1-methyl-1H-pyrazol-4-yl)-1H-indazol-5-yl)oxy)ethyl)qu inoxaline | 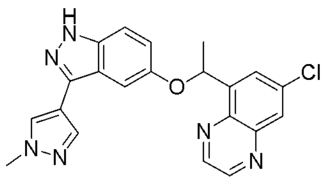 | 405.2 | $^1$H NMR (400 MHz, DMSO-d6) δ 9.18 - 9.12 (m, 2H), 8.15 (d, J = 2.4 Hz, 1H), 7.99 (s, 1H), 7.96 (d, J = 2.4 Hz, 1H), 7.61 (s, 1H), 7.41 (d, J = 9.0 Hz, 1H), 7.24 (d, J = 2.2 Hz, 1H), 7.13 (dd, J = 9.0, 2.3 Hz, 1H), 6.70 (q, J = 6.3 Hz, 1H), 3.90 (s, 3H), 1.75 (d, J = 6.3 Hz, 3H). |
| 137 | 5-(1-((3-(1-methyl-1H-pyrazol-4-yl)-1H-indazol-5-yl)oxy)ethyl)-7-(trifluoromethy l)quinoxaline | 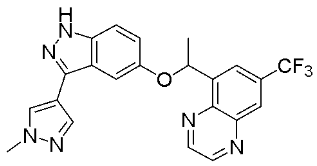 | 439.1 | $^1$H NMR (400 MHz, DMSO) δ 12.81 (s, 1H), 9.28 (s, 1H), 9.25 (s, 1H), 8.46 (s, 1H), 8.21 (s, 1H), 8.04 (s, 1H), 7.66 (s, 1H), 7.41 (d, J = 8.8 Hz, 1H), 7.31 (s, 1H), 7.15 (dd, J = 9.2, 2.0 Hz, 1H), 6.75 (q, J = 6.0 Hz, 1H), 3.91 (s, 3H), 1.78 (d, J = 6.4 Hz, 3H). |
| 139 | 7-cyclopropyl-5-(1-((3-(1-methyl-1H-pyrazol-4-yl)-1H-indazol-5-yl)oxy)ethyl)qu inoxaline | 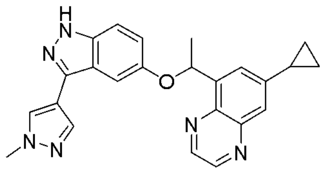 | 411.1 | $^1$H NMR (400 MHz, DMSO) δ 12.76 (s, 1H), 9.02 (s, 2H), 7.93 (s, 1H), 7.74 (s, 1H), 7.65 (d, J = 1.7 Hz, 1H), 7.57 (s, 1H), 7.39 (d, J = 9.0 Hz, 1H), 7.20 (s, 1H), 7.11 (dd, J = 8.9, 1.9 Hz, 1H), 6.69 (q, J = 6.4 Hz, 1H), 3.90 (s, 3H), 2.23 - 2.12 (m, 1H), 1.74 (d, J = 6.4 Hz, 3H), 1.08 (dd, J = 8.3, 2.5 Hz, 2H), 0.92 - 0.86 (m, 1H), 0.80 - 0.73 (m, 1H). |
| 140 | 7-methyl-5-(1-((3-(1-methyl-1H-pyrazol-4-yl)-1H-indazol-5-yl)oxy)ethyl)qu inoxaline | 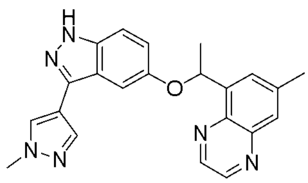 | 385.2 | $^1$H NMR (400 MHz, DMSO) δ 12.75 (s, 1H), 9.05 (d, J = 6.0 Hz, 2H), 7.91 (s, 1H), 7.84 (d, J = 13.2 Hz, 2H), 7.55 (s, 1H), 7.39 (d, J = 9.2 Hz, 1H), 7.18 (s, 1H), 7.11 (dd, J = 9.2, 2.0 Hz, 1H), 6.70 (q, J = 6.4 Hz, 1H), 3.89 (s, 3H), 1.74 (d, J = 6.4 Hz, 3H). |

(continued)

| Example | Name | Structure | LC-MS (M+H)+ | 1H NMR |
|---|---|---|---|---|
| 141 | 8-(1-((3-(1-methyl-1H-pyrazol-4-yl)-1H-indazol-5-yl) oxy)ethyl)qu inoxaline-6-carbonitrile | 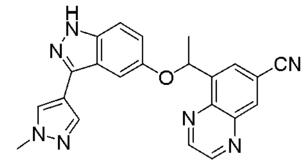 | 396.2 | $^1$H NMR (400 MHz, DMSO-d6) δ 12.82 (s, 1H), 9.32 - 9.20 (m, 2H), 8.69 (d, J = 1.8 Hz, 1H), 8.28 (d, J = 1.8 Hz, 1H), 8.03 (s, 1H), 7.64 (s, 1H), 7.41 (d, J = 9.0 Hz, 1H), 7.28 (d, J = 2.3 Hz, 1H), 7.15 (dd, J = 8.9, 2.2 Hz, 1H), 6.71 (q, J = 6.3 Hz, 1H), 3.91 (s, 3H), 1.81 - 1.67 (m, 3H). |
| 142 | 7-fluoro-5-(1-((3-(1-methyl-1H-pyrazol-4-yl)-1H-indazol-5-yl)oxy) ethyl)qu inoxaline | 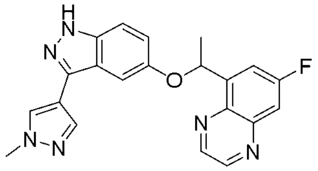 | 389.2 | $^1$H NMR (400 MHz, DMSO) δ 9.12 (s, 2H), 7.98 (s, 1H), 7.88-7.84 (m, 2H), 7.61 (s, 1H), 7.41 (d, J = 9.2 Hz, 1H), 7.24 (d, J = 2.0 Hz, 1H), 7.13 (dd, J = 9.2, 2.2 Hz, 1H), 6.77 - 6.65 (m, 1H), 1.75 (d, J = 6.4 Hz, 3H). |
| 145 | 7-(difluoromethyl )-5-(1-((3-(1-methyl-1H-pyrazol-4-yl)-1H-indazol-5-yl)oxy) ethyl)qu inoxaline | 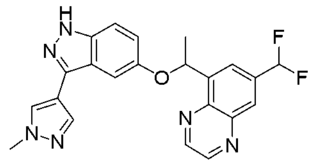 | 421.0 | $^1$H NMR(400 MHz, dmso) δ 12.75 (s, 1H), 9.23 - 9.09 (m, 2H), 8.22 (s, 1H), 8.10 (s, 1H), 7.94 (s, 1H), 7.56 (s, 1H), 7.41 - 7.34 (m, 1H), 7.23 (d,J = 22.5 Hz, 2H), 7.13 - 7.06 (m, 1H), 6.69 (dd,J = 12.6, 6.3 Hz, 1H), 3.86 (s, 3H), 1.72 (d,J = 6.3 Hz, 3H) |
| 147 | 5-(1-((3-(1-methyl-1H-pyrazol-4-yl)-1H-indazol-5-yl) oxy)ethyl)qu inoline-3-amine | 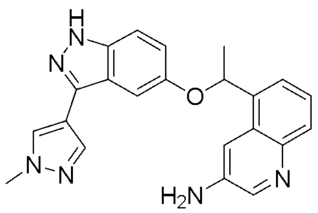 | 385.2 | $^1$H NMR (400 MHz, DMSO-$d_6$) δ 12.72 (s, 1H), 8.49 (s, 1H), 7.87 (s, 1H), 7.73 (s, 1H), 7.70 (s, 1H), 7.66 (s, 1H), 7.55 (d, *J* = 7.2 Hz, 1H), 7.36 (d, *J* = 8.9 Hz, 1H), 7.26 (d, *J* = 15.2 Hz, 1H), 7.15 (s, 1H), 7.07 (d, *J* = 9.2 Hz, 1H), 6.04 (d, *J* = 6.4 Hz, 1H), 5.80 (s, 2H), 3.88 (s, 3H), 1.72 (d, *J* = 6.3 Hz, 3H). |
| 148 | 7-methoxy-5-(1-((3-(1-methyl-1H-pyrazol-4-yl)-1H-indazol-5-yl)oxy) ethyl)qu inoline | 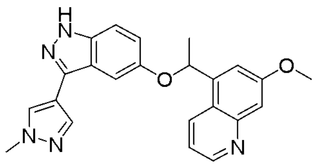 | 400.1 | $^1$H NMR (400 MHz, DMSO) δ 12.77 (s, 1H), 8.93 (dd, J = 12.8, 6.4 Hz, 2H), 8.02 (s, 1H), 7.73 (s, 1H), 7.56 (dd, J = 8.6, 4.4 Hz, 1H), 7.46 (d, J = 2.4 Hz, 1H), 7.39 (d, J = 9.0 Hz, 1H), 7.33 (d, J = 2.2 Hz, 1H), 7.27 (d, J = 1.8 Hz, 1H), 7.12 (dd, J = 9.0, 2.0 Hz, 1H), 6.39 (q, J = 6.4 Hz, 1H), 3.89 (s, 6H), 1.75 (d, J = 6.4 Hz, 3H). |

(continued)

| Example | Name | Structure | LC-MS (M+H)+ | 1H NMR |
|---|---|---|---|---|
| 150 | 5-(1-((3-(1-methyl-1H-pyrazol-4-yl)-1H-indazol-5-yl)oxy)ethyl)qu inoline-7-amine | 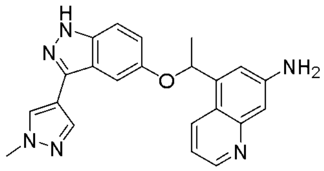 | 385.1 | 1H NMR (400 MHz, DMSO-d6) δ 12.78 (s, 1H), 8.79 (d, J = 8.4 Hz, 1H), 8.71 (d, J = 4.6 Hz, 1H), 8.16 (s, 0.3H), 7.96 (s, 1H), 7.72 (s, 1H), 7.41 (d, J = 9.0 Hz, 1H), 7.31 (q, J = 4.4 Hz, 2H), 7.21 (d, J = 2.2 Hz, 1H), 7.10 (dd, J = 8.8, 2.2 Hz, 1H), 6.88 (d, J = 2.1 Hz, 1H), 6.25 (q, J = 6.3 Hz, 1H), 6.18 (s, 1H), 3.88 (s, 3H), 1.71 (d, J = 6.2 Hz, 3H). |
| 155 | 3-(1-methyl-1H-pyrazol-4-yl)-5-(1-(5-methylpyrazolo[1,5-a]pyridin-4-yl)ethoxy)-1H-indazole | 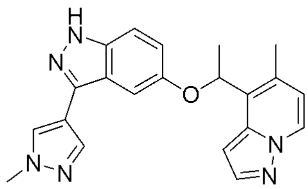 | 373.2 | 1H NMR (400 MHz, DMSO) δ 12.74 (s, 1H), 8.45 (d, J = 7.2 Hz, 1H), 7.97 (d, J = 2.4 Hz, 3H), 7.74 (s, 1H), 7.36 (d, J = 8.8 Hz, 1H), 7.13 (s, 1H), 7.07 (dd, J = 9.2, 2.4 Hz, 1H), 6.96 (d, J = 1.6 Hz, 1H), 6.69 (d, J = 7.2 Hz, 1H), 6.01 (t, J = 6.4 Hz, 1H), 3.93 (s, 3H), 1.72 (d, J = 6.4 Hz, 3H). |
| 157 | 5-(1-((3-(1-methyl-1H-pyrazol-4-yl)-1H-indazol-5-yl)oxy)ethyl)py ridino[3,4-b]pyrazine | 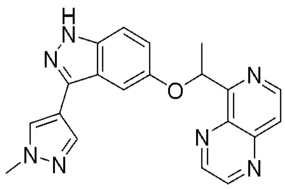 | 372.1 | 1H NMR (400 MHz, $CD_3OD$) δ 9.18 (s, 2H), 8.77 (s, 1H), 7.93 (s, 1H), 7.82 (s, 1H), 7.55 (s, 1H), 7.35 (s, 1H), 7.16 (s, 2H), 6.97 (s, 1H), 3.93 (s, 3H), 1.84 - 1.80 (m, 3H) |
| 159 | 5-(1-((3-(1-methyl-1H-pyrazol-4-yl)-1H-indazol-5-yl)oxy)ethyl)qu inoline-7-carbonitrile | 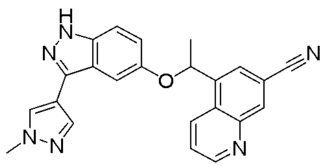 | 395.2 | 1H NMR (400 MHz, DMSO) δ 13.11 - 12.37 (m, 1H), 9.15 - 9.04 (m, 2H), 8.53 (s, 1H), 8.18 - 8.08 (m, 2H), 7.84 (dd, J = 8.8, 4.2 Hz, 1H), 7.79 (s, 1H), 7.41 (d, J = 9.0 Hz, 1H), 7.35 (d, J = 2.0 Hz, 1H), 7.16 (dd, J = 9.0, 2.2 Hz, 1H), 6.47 (q, J = 6.2 Hz, 1H), 3.92 (s, 3H), 1.78 (d, J = 6.4 Hz, 3H). |
| 161 | 7-fluoro-6-methyl-5-(1-((3-(1-methyl-1H-pyrazol-4-yl)-1H-indazol-5-yl)oxy)ethyl)quinoline | 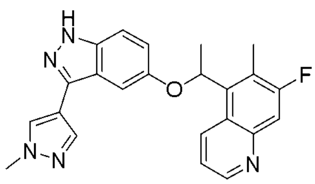 | 402.2 | 1H NMR (400 MHz, DMSO-*d*6) δ 12.74 (s, 1H), 9.22 (d, *J* = 12.4 Hz, 1H), 8.86 (s, 1H), 7.89 (s, 1H), 7.69 - 7.56 (m, 3H), 7.33 (d, *J* = 10.1 Hz, 1H), 7.05 (d, *J* = 23.7 Hz, 2H), 6.42 (s, 1H), 3.89 (s, 3H), 2.60 (s, 3H), 1.85 (d, *J* = 6.8 Hz, 3H). |

(continued)

| Example | Name | Structure | LC-MS (M+H)+ | 1H NMR |
|---|---|---|---|---|
| 167 | 6-chloro-5-(1-((3-(1-methyl-1H-pyrazol-4-yl)-1H-indazol-5-yl)oxy)ethyl)qu inoline | 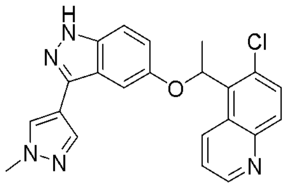 | 404.1 | $^1$H NMR (400 MHz, DMSO-d6) δ 12.76 (d, J = 11.0 Hz, 1H), 9.28 (d, J = 8.1 Hz, 1H), 8.90 (m, 1H), 8.16 (d, J = 9.3 Hz, 1H), 8.04 (s, 1H), 7.96 (d, J = 9.0 Hz, 1H), 7.82 (d, J = 9.8 Hz, 1H), 7.77 (s, 1H), 7.65 (dd, J = 8.9, 4.2 Hz, 1H), 7.35 (d, J = 9.0 Hz, 1H), 7.17 (d, J = 2.3 Hz, 1H), 7.06 (dd, J = 9.0, 2.3 Hz, 1H), 6.44 (q, J = 6.6 Hz, 1H), 3.96 (s, 3H), 1.91 (d, J = 6.7 Hz, 3H). |
| 171 | 3-(1-methyl-1H-pyrazol-4-yl)-5-(1-(6-methylpyrazolo [1,5-a]pyridin-4-yl)ethoxy)-1H-indazole | 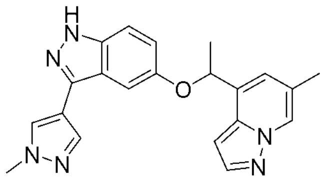 | 373.2 | $^1$H NMR (400 MHz, $CD_3OD$) δ 8.25 (s, 1H), 7.97 (s, 1H), 7.76 (d, J = 12.6 Hz, 2H), 7.37 (d, J = 9.1 Hz, 1H), 7.22 (s, 1H), 7.13 (d, J = 22.5 Hz, 2H), 6.89 (t, J = 1.5 Hz, 1H), 5.73 (s, 1H), 3.93 (s, 3H), 2.29 (s, 3H), 1.77 (s, 3H). |
| 172 | 5-(1-((3-(1-methyl-1H-pyrazol-4-yl)-1H-indazol-5-yl) oxy)ethyl)-6-(trifluoromethy l)quinoline | 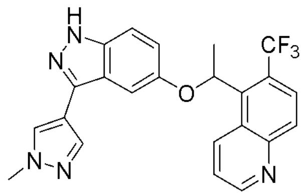 | 438.2 | |
| 174 | 5-(1-(6-chloropyrazolo [ 1,5-a]pyridin-4-yl) ethoxy)-3-(1-methyl-1H-pyrazol-4-yl)-1H-indazole | 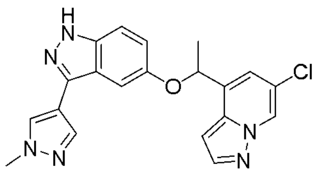 | 393.1 | $^1$H NMR (400 MHz, $CD_3OD$) δ 8.58 (s, 1H), 8.05 (d, J = 2.4 Hz, 1H), 7.84 (s, 1H), 7.80 (d, J = 0.8 Hz, 1H), 7.40 (d, J = 9.7 Hz, 1H), 7.35 (s, 1H), 7.15 (d, J = 8.7 Hz, 2H), 6.98 (d, J = 1.6 Hz, 1H), 5.75 (d, J = 6.4 Hz, 1H), 3.94 (s, 3H), 1.76 (d, J = 6.4 Hz, 3H). |
| 176 | 4-(1-((3-(1-methyl-1H-pyrazol-4-yl)-1H-indazol-5-yl) oxy)ethyl)py razolo[1,5-a] pyrazine | 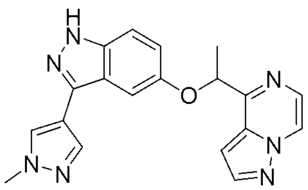 | 360.0 | $^1$H NMR (400 MHz, DMSO) δ 12.78 (s, 1H), 8.72 (d, J = 4.8 Hz, 1H), 8.19 (d, J = 2.0 Hz, 1H), 8.12 (s, 1H), 7.91 (d, J = 4.8 Hz, 1H), 7.81 (s, 1H), 7.39 (d, J = 9.2 Hz, 1H), 7.35 (s, 1H), 7.26 (s, 1H), 7.17 - 7.07 (m, 1H), 5.95 (q, J = 6.4 Hz, 1H), 3.95 (s, 3H), 1.78 (d, J = 6.4 Hz, 3H). |

(continued)

| Example | Name | Structure | LC-MS $(M+H)^+$ | $^1H$ NMR |
|---|---|---|---|---|
| 184 | 5-(1-(3-fluoropyrazolo [ 1,5-a]pyridin-4-yl) ethoxy)-3-(1-methyl-1H-pyrazol-4-yl)-1H-indazole | 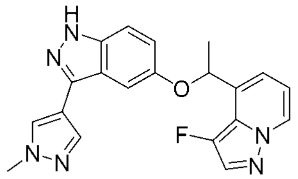 | 377.2 | $^1H$ NMR (400 MHz, DMSO) δ 12.78 (s, 1H), 8.47 (d, J = 6.8 Hz, 1H), 8.22 - 8.13 (m, 2H), 7.83 (s, 1H), 7.38 (dd, J = 11.6, 8.0 Hz, 3H), 7.07 (d, J = 8.8 Hz, 1H), 6.85 (t, J = 7.2 Hz, 1H), 5.92 (d, J = 6.4 Hz, 1H), 3.94 (s, 3H), 1.74 (d, J = 6.4 Hz, 3H). |
| 186 | 4-(1-((3-(1-methyl-1H-pyrazol-4-yl)-1H-indazol-5-yl) oxy)ethyl)py razolo[1,5-a] pyridine-3-carbonitrile | 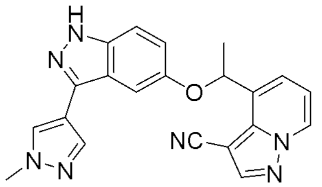 | 384.2 | $^1H$ NMR (400 MHz, DMSO)δ 12.83 (s, 1H), 8.91 (d, J = 6.8 Hz, 1H), 8.78 (s, 1H), 8.16 (s, 1H), 7.91 - 7.80 (m, 2H), 7.44 (d, J = 9.0 Hz, 1H), 7.36 (s, 1H), 7.26 (t, J = 7.0 Hz, 1H), 7.14 (dd, J = 9.0, 2.0 Hz, 1H), 6.15 (q, J = 6.3 Hz, 1H), 3.96 (s, 3H), 1.80 (d, J = 6.3 Hz, 3H). |
| 187 | 6-(methyl-d3) - 5-(1-((3-(1-methyl-1H-pyrazol-4-yl)-1H-indazol-5-yl)oxy) ethyl)qu inoline | 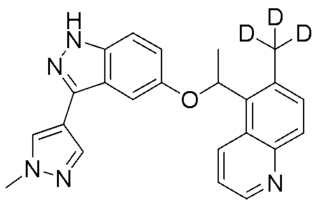 | 387.3 | $^1H$ NMR (400 MHz, $CD_3OD$) δ 9.27 (d, J = 8.9 Hz, 1H), 8.77 - 8.73 (m, 1H), 7.85 (d, J = 8.8 Hz, 1H), 7.68 - 7.51 (m, 4H), 7.31 (d, J = 9.1 Hz, 1H), 7.09 (m, 1H), 6.88 (s, 1H), 6.18 (d, J = 7.2 Hz, 1H), 3.92 (s, 3H), 1.89 (d, J = 6.7 Hz, 3H). |
| 191 | 6-methyl-4-(1-((3-(1-methyl-1H-pyrazol-4-yl)-1H-indazol-5-yl)oxy) ethyl)py razolo[1,5-a]pyrazine | 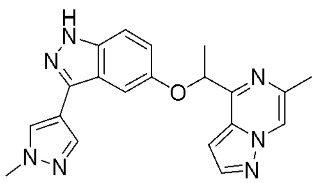 | 374.2 | $^1H$ NMR (400 MHz, DMSO) δ 12.78 (s, 1H), 8.58 (s, 1H), 8.15 - 8.05 (m, 2H), 7.80 (s, 1H), 7.39 (d, J = 8.8 Hz, 1H), 7.34 (s, 1H), 7.16 (d, J = 2.4 Hz, 1H), 7.14 - 7.08 (m, 1H), 5.86 (q, J = 6.4 Hz, 1H), 3.95 (s, 3H), 2.50 (s, 3H), 1.78 (d, J = 6.4 Hz, 3H). |
| 226 | 7-chloro-5-(1-(7-methoxy-3-(1-methyl-1H-pyrazol-4-yl)-1H-indazol-5-yl) oxy)ethyl)qu inoxaline | 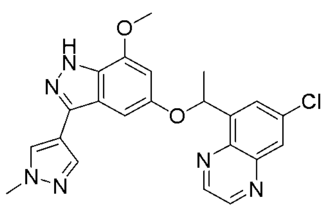 | 435.13 | $^1H$ NMR (400 MHz, DMSO) δ 13.02 (s, 1H), 9.17 (dd, J = 11.6, 1.8 Hz, 2H), 8.16 (d, J = 2.4 Hz, 1H), 7.98 (d, J = 2.4 Hz, 1H), 7.86 (s, 1H), 7.50 (s, 1H), 6.77 - 6.61 (m, 3H), 3.93 (s, 3H), 3.88 (s, 3H), 1.75 (d, J = 6.4 Hz, 3H). |
| 227 | 7-chloro-5-(1-((6-fluoro-3-(1-methyl-1H-pyrazol-4-yl)-1H-indazol-5-yl) oxy)ethyl)qu inoxaline | 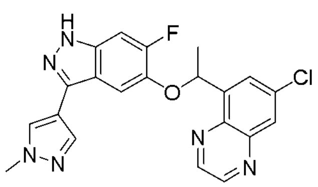 | 423.1 | $^1H$ NMR(400 MHz, DMSO) δ 12.90 (s, 1H), 9.12 (q,J = 1.8 Hz, 2H), 8.18 (d,J = 2.4 Hz, 1H), 8.03 (d,J = 2.3 Hz, 1H), 8.01 (s, 1H), 7.63 (s, 1H), 7.43 (dd,J = 15.7, 9.3 Hz, 2H), 6.76 (d,J = 6.4 Hz, 1H), 3.91 (s, 3H), 1.79 (d,J = 6.3 Hz, 3H) |

(continued)

| Example | Name | Structure | LC-MS (M+H)+ | 1H NMR |
|---|---|---|---|---|
| 228 | 7-chloro-5-(1-((7-fluoro-3-(1-methyl-1H-pyrazol-4-yl)-1H-indazol-5-yl) oxy)ethyl)qu inoxaline | 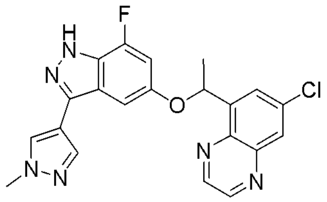 | 423.1 | $^1$H NMR(400 MHz, DMSO) δ 13.36 (s, 1H), 9.15 (dd,J = 8.1, 1.8 Hz, 2H), 8.17 (d,J = 2.4 Hz, 1H), 8.03 (s, 1H), 7.99 (d,J = 2.3 Hz, 1H), 7.62 (s, 1H), 7.10 (s, 2H), 6.72 (d,J = 6.4 Hz, 1H), 3.91 (s, 3H), 1.76 (d,J = 6.3 Hz, 3H) |

Example 108

5-(1-((3-(1-methyl-1H-pyrazol-4-yl)-1H-indazol-5-yl)oxy)ethyl)-1,7-naphthyridine

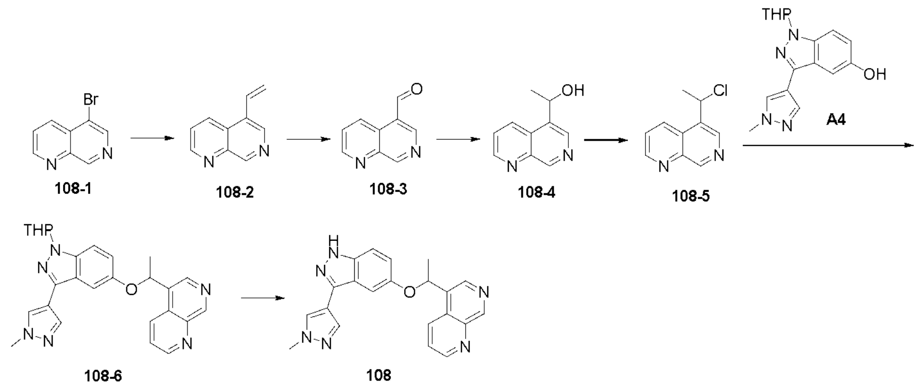


At room temperature, Compound 108-1 (0.26 g, 1.24 mmol), potassium vinyltrifluoroborate (0.25 g, 1.86 mmol), Pd(dppf)$Cl_2$ (0.091 g, 0.124 mmol) and $K_2CO_3$ (0.343 g, 2.48 mmol) were added to a mixture of 1,4-dioxane (6.2 mL) and water (3.1 mL). The resulting mixture was stirred at 90°C for 13 hours. LCMS showed the generation of a product. The reaction mixture was cooled to room temperature, and diluted with dichloromethane (10.0 mL) and water (5.0 mL). The organic phase was separated, and the aqueous phase was extracted with dichloromethane (5.0 mL x 3). The organic phases were combined, dried over anhydrous sodium sulfate, and filtered. The filtrate was concentrated to dryness under reduced pressure. The resulting residue was purified by silica gel column chromatography (eluent: petroleum ether:ethyl acetate=2:1) to obtain brown sticky liquid 108-2 (0.165 g, yield: 85%, purity: 97.25%). LCMS (ESI) m/z 157.2, $(M+H)^+$.

At 0°C, Compound 108-2 (0.165 g, 1.06 mmol) was added to a mixture of tetrahydrofuran (10.6 mL) and water (2.1 mL), and then $K_2OsO_4 \cdot 2H_2O$ (0.0391 g, 0.106 mmol) was added. The resulting mixture was stirred for 15 minutes, and then $NaIO_4$ (0.907 g, 4.24 mmol) was added. The reaction mixture was stirred at 0°C for 20 minutes, warmed up to room temperature and stirred for 13 hours. LCMS showed the generation of a product. The reaction mixture was diluted with dichloromethane (20.0 mL), dried over anhydrous sodium sulfate, and filtered. The filtrate was concentrated to dryness under reduced pressure. The resulting residue was purified by silica gel column chromatography (eluent: dichloromethane:ethyl acetate=5:1) to obtain an orange solid 108-3 (0.108 g, yield: 64%, purity: 99.06%): LCMS (ESI) m/z 159.1, $(M+H)^+$.

At 0°C, methyl magnesium bromide solution (3.0 M, 2-methyltetrahydrofuran solution) (0.34 mL, 1.02 mmol) was added dropwise to a solution of Compound 108-3 (0.108 g, 0.68 mmol) in tetrahydrofuran (3.0 mL). The reaction mixture was stirred at 0°C for 20 minutes, then warmed up to room temperature and stirred for 3 hours. LCMS showed the generation of a product. The reaction mixture was diluted with a solution of ammonium chloride in methanol (0.3 M, 4.0 mL), and concentrated to dryness under reduced pressure. The resulting residue was purified by silica gel column chromatography (eluent: dichloromethane:methanol=20:1) to obtain a light brown solid 108-4 (0.116 g, yield: 98%, purity: 93.27%). LCMS (ESI) m/z 175.1, $(M+H)^+$.

At 0°C, thionyl chloride (0.197 g, 1.66 mmol) was added dropwise to a solution of Compound 108-4 (0.116 g, 0.66 mmol) in dichloromethane (5.0 mL). The resulting mixture was stirred at 0°C for 10 minutes, warmed up to room

temperature and stirred for 2 hours. LCMS showed the generation of a product. The reaction mixture was diluted with dichloromethane (20.0 mL), and then saturated aqueous sodium bicarbonate solution was added to adjust the pH of the aqueous phase to about 7 to 8. The organic phase was separated, and the aqueous phase was extracted with dichloromethane (10.0 mL ×3). The organic phases were combined, dried over anhydrous sodium sulfate, and filtered. The filtrate was concentrated to dryness under reduced pressure. The resulting residue was dried under vacuum to obtain a crude product Compound 108-5 as a dark brown jelly (0.0272 g, crude yield: 21%, purity: 29.03%). LCMS (ESI) m/z 193.1, 195.1, (M+H)+.

At room temperature, the crude product Compound 108-5 (0.0272 g, 0.14 mmol), Compound A4 (0.0418 g, 0.14 mmol) and cesium carbonate (0.0684 g, 0.21 mmol) were added to N,N-dimethylformamide (1.4 mL). The resulting mixture was stirred at 65°C for 10 hours. LCMS showed the generation of a product. The reaction mixture was cooled to room temperature, diluted with ethyl acetate (10.0 mL), and washed with water (5.0 mL × 2). The organic phase was dried over anhydrous sodium sulfate, and filtered. The filtrate was concentrated to dryness under reduced pressure. The resulting residue was purified by silica gel thin-layer chromatography (eluent: dichloromethane:methanol=35:1) to obtain Compound 108-6 as a dark yellow jelly (0.0187 g, yield: 29%, purity: 86.72%). LCMS (ESI) m/z 455.3, (M+H)+.

At room temperature, trifluoroacetic acid (0.0938 g, 0.82 mmol) was added to a solution of Compound 108-6 (0.0187 g, 0.041 mmol) in dichloromethane (0.41 mL). The resulting mixture was stirred for 26 hours. LCMS showed the generation of a product. The reaction mixture was diluted with dichloromethane (10.0 mL), and then saturated aqueous sodium bicarbonate solution was added to adjust the pH of the aqueous phase to about 7 to 8. The organic phase was separated, and the aqueous phase was extracted with dichloromethane (10.0 mL × 3). The organic phases were combined, dried over anhydrous sodium sulfate, and filtered. The filtrate was concentrated to dryness under reduced pressure. The resulting residue was purified by silica gel thin-layer chromatography (eluent: dichloromethane:methanol=20:1) to obtain Compound 108 as a dark yellow solid (0.0077 g, yield: 51%, purity: 90.73%). LCMS (ESI) m/z 371.1, (M+H)+; $^1$H NMR (400 MHz, DMSO) δ 12.78 (s, 1H), 9.33 (s, 1H), 9.12 (s, 1H), 9.04 (d, J = 8.4 Hz, 1H), 8.85 (s, 1H), 8.16 (s, 1H), 7.942 - 7.888 (m, 1H), 7.82 (s, 1H), 7.435 - 7.364 (m, 2H), 7.15 (d, J = 8.8 Hz, 1H), 6.43 (q, J = 6.8 Hz, 1H), 3.93 (s, 3H), 1.83 (d, J = 6.4 Hz, 3H).

The following compound was synthesized according to the the route of Example 108.

| Example | Name | Structure | LC-MS $(M+H)^+$ | $^1$H NMR |
|---|---|---|---|---|
| 109 | 4-(1-((3-(1-methyl-1H-pyrazol-4-yl)-1H-indazol-5-yl)oxy)ethyl)ben zo [d]thiazole | 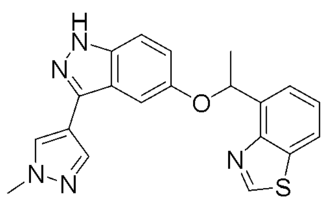 | 376 | $^1$H NMR (400 MHz, DMSO) δ 12.76 (s, 1H), 9.61 (s, 1H), 8.10 (d, J = 8.0 Hz, 1H), 7.92 (s, 1H), 7.67 (d, J = 7.2 Hz, 1H), 7.63 (s, 1H), 7.49 (t, J = 8.0 Hz, 1H), 7.39 (d, J = 8.8 Hz, 1H), 7.21 (s, 1H), 7.10 (dd, J = 9.2, 2.0 Hz, 1H), 6.46 (q, J = 6.4 Hz, 1H), 3.93 (s, 3H), 1.76 (d, J = 6.4 Hz, 3H). |

Example 110

6-methyl-5-(1-((3-(1-methyl-1H-pyrazol-4-yl)-1H-pyrazino [4,3-d]pyrimidin-5-yl)o xy)ethyl)quinoline

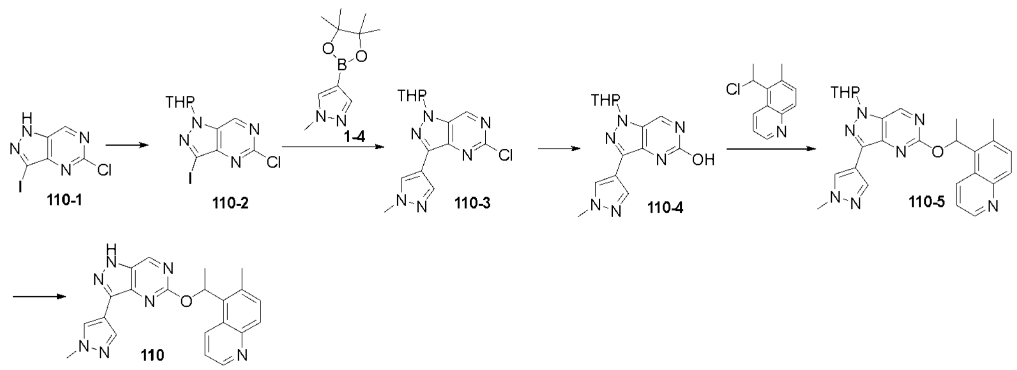


Compound 110-1 (400 mg, 1.43 mmol) was dissolved in DCM (5 mL) at room temperature, DHP (360 mg, 4.28

mmol) was added at 0°C, and then TsOH·$H_2O$ (27 mg, 0.14 mmol) was added. The reaction solution was stirred at room temperature for 60 min. LCMS showed the complete reaction of raw materials. The reaction solution was quenched with water, and extracted with DCM (20 mL × 3). The organic phases were combined and then subjected to rotary drying, and the crude was purified by silica gel column chromatography (eluent: petroleum ether:ethyl acetate=2:1) to obtain a product 110-2 as a yellow solid (233 mg, purity 85%): [M+H]+=365.0).

At room temperature, Compound 110-2 (70 mg, 0.19 mmol) was dissolved in dioxane (5 mL) and $H_2O$ (1 mL), Compound 1-4 (42 mg, 0.20 mmol) was added, and then Pd(dppf)$Cl_2$ (32 mg, 0.039 mmol) and sodium carbonate (57 mg, 0.54 mmol) were added under nitrogen protection. The reaction solution was stirred at 90°C for 2 h. LCMS showed no remaining raw materials. After the reaction solution was cooled to room temperature, the organic phases were combined and then subjected to rotary drying. The crude was purified by silica gel column chromatography (eluent: petroleum ether:ethyl acetate=1:5) to obtain a product 110-3 as a black solid (60 mg, purity 85%): [M+H]+=320.1).

Compound 110-3 (60 mg, 0.19 mmol) was dissolved in dioxane (5 mL) and $H_2O$ (1 mL), and KOH (42 mg, 0.75 mmol) was added. Then $Pd_2(dba)_3$ (86 mg, 0.094 mmol) and t-BuXPhos (80 mg, 0.19mmol) were added under nitrogen protection. The reaction solution was stirred at 100°C for 16 h. LCMS showed no remaining raw materials. After the reaction solution was cooled to room temperature, the organic phases were combined and then subjected to rotary drying. The crude was purified by silica gel column chromatography (eluent: dichloromethane:methanol=10:1) to obtain a product 110-4 as a black soild (40 mg, purity 80%): [M+H]+=301.2).

At room temperature, Compound 110-4 (40 mg, 0.13 mmol) and Compound 95-5 (30 mg, 0.15mmol) were dissolved in DMF (2 mL), and $Cs_2CO_3$ (43 mg, 0.13 mmol) was added. The reaction solution was stirred at 40°C for 16 h. LCMS showed the complete reaction of raw materials. The reaction solution was diluted with water and then extracted with EA (30 mL × 3). The organic phases were combined and then subjected to rotary drying. The crude was purified by silica gel column chromatography (eluent: dichloromethane:methanol=10:1) to obtain a product Compound 110-5 as a yellow oil (20 mg, purity 90%, LCMS:470.0[M+H]+).

Compound 110-5 (20 mg, 0.04 mmol) was dissolved in DCM (2 mL) at room temperature, and TFA (1 mL) was added thereto in an ice bath. The reaction solution was stirred at room temperature for 1 h. LCMS showed the complete reaction of raw materials. The reaction solution was concentrated, and the organic phase was collected, and then subjected to preparative high performance liquid chromatography to obtain Compound 110 as a white solid (1.7 mg, purity: 100%, LCMS :386.2[M+H]+).

Example 113

6-methyl-5-(1-((3-(1-methyl-1H-pyrazol-4-yl)-1H-indazol-5-yl)oxy)propyl)quinoli ne

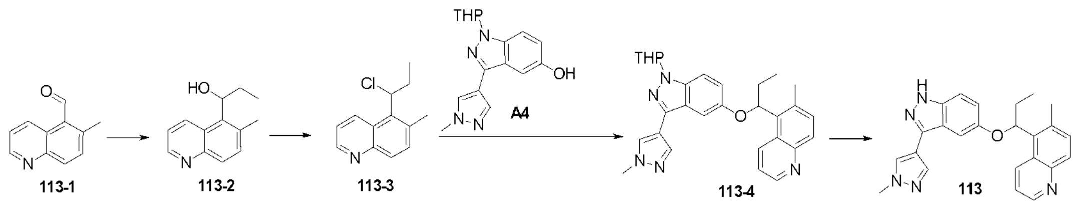


113-1 113-2 113-3 113-4 113

Compound 113-1 (200 mg, 1.17 mmol, 1.0 eq.) was placed in a three-necked flask, dissolved in THF (10 mL), purged with nitrogen, cooled to 0°C, and ethyl magnesium chloride (389.2 mg, 2.92 mmol, 2.5 eq.) was added dropwise. The mixture was stirred at room temperature for 1 hour. LC-MS and TLC monitoring showed the complete reaction of Compound 113-1. The reaction mixture was cooled in an ice water bath, quenched with water, and washed and extracted with ethyl acetate+water three times. The organic phase was dried over anhydrous $Na_2SO_4$, subjected to rotary drying, and purified by column chromatography (petroleum ether:ethyl acetate =2:1) to obtain Compound 113-2 as a yellow solid (150 mg, 63.8 %). $^1$H NMR (400 MHz, DMSO-d6) δ 9.07 (d, J = 9.7 Hz, 1H), 8.73 (s, 1H), 7.76 (d, J = 8.4 Hz, 1H), 7.49 (d, J = 8.6 Hz, 1H), 7.38 (d, J = 12.5 Hz, 1H), 5.53 (s, 1H), 5.18 (s, 1H), 1.95 (m, 1H), 1.73 (m, 7.0 Hz, 1H), 0.86 (t, J = 7.4 Hz, 4H).

Compound 113-2 (150 mg, 0.76 mmol, 1.0 eq.) was dissolved in DCM (8 mL), triethylamine (150.8 mg, 1.49 mmol, 2.0 eq.) was added, purged with nitrogen, cooled in an ice water bath, and MsCl (127.4 mg, 1.12 mmol, 1.5 eq.) was added. The mixture was stirred at room temperature overnight. LC-MS and TLC monitoring showed the complete reaction of Compound 113-2. The reaction mixture was washed and extracted with ethyl acetate+water three times. The organic phase was dried over anhydrous $Na_2SO_4$, subjected to rotary drying, and purified by thin-layer chromatography (developing solvent: DCM: MeOH=15:1) to obtain Compound 113-3 as a yellow oil (85 mg). $^1$H NMR (400 MHz, $CD_3OD$) δ 9.13 (d, J = 3.4 Hz, 1H), 8.75 (dd, J = 17.0, 5.6 Hz, 1H), 7.86 (dd, J = 18.9, 9.5 Hz, 1H), 7.59 (s, 1H), 7.48 - 7.41 (m, 1H), 4.97

(s, 1H), 2.56 (s, 3H), 2.13 (s, 1H), 1.85 (s, 1H), 0.96 (s, 3H).

Compound 113-3 (95 mg, 0.32 mmol, 1.0 eq.) was dissolved in acetonitrile (10 mL), and cesium carbonate (210 mg, 0.64 mmol, 2.0 eq.) and Compound A4 (85 mg, 0.39 mmol, 1.2 eq.) were added successively, and purged with nitrogen. The mixture was stirred at 80oC for 24 hours. LC-MS and TLC monitoring showed the complete reaction of Compound A4. The reaction mixture was washed and extracted with ethyl acetate+water three times. The organic phase was dried over anhydrous $Na_2SO_4$, subjected to rotary drying, and purified by thin-layer chromatography (developing solvent: DCM: MeOH=15:1) to obtain Compound 113-4 as a yellow oil (60 mg, 39.1 %). LCMS (ESI) m/z 482.20, (M+H) +.

Compound 113-4 (120 mg, 0.31 mmol, 1.0 eq.) was dissolved in MeOH (2.0 mL), added to dioxane (HCl) (5 mL), and the mixture was stirred at room temperature for 2.5 hours. The reaction mixture was directly subjected to rotary drying, followed by preparative high performance liquid chromatography to obtain Compound 113 as a light white solid (36.5 mg, 73.71 %, purity 99 %). LCMS (ESI) m/z 398.25, (M+H) +; $^1$H NMR (400 MHz, $CD_3OD$) δ 9.28 (s, 1H), 8.75 (s, 1H), 7.86 (d, J = 8.8 Hz, 1H), 7.61 (d, J = 10.1 Hz, 4H), 7.32 (d, J = 8.9 Hz, 1H), 7.11 (d, J = 8.9 Hz, 1H), 6.91 (s, 1H), 5.94 (s, 1H), 3.93 (s, 3H), 2.71 (s, 3H), 2.46 (dt, J = 13.6, 6.6 Hz, 1H), 2.11 (dt, J = 14.1, 6.9 Hz, 1H), 1.19 (t, J = 7.3 Hz, 3H).

Example 114

2-((3-(1-methyl-1H-pyrazol-4-yl)-1H-indazol-5-yl)oxy)-2-(6-methylquinolin-5-yl)ethan-1-ol

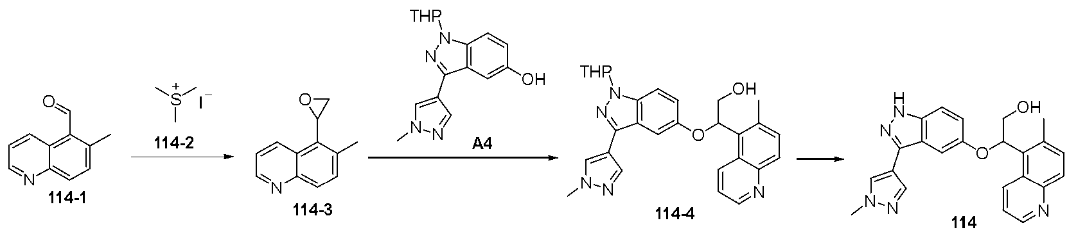


NaOH (187 mg, 4.68 mmol, 2.0 eq) was dissolved in toluene (10.0 ml) at room temperature, and Compound 114-2 (955 mg, 4.68 mmol, 2.0 eq) and the compound N-benzyl-N,N,N-triethylammonium chloride (533 mg, 2.34 mmol, 1.0 eq) were added at room temperature, purged with nitrogen, and then reacted at room temperature for 0.5 hour. Compound 114-1 (300 mg, 2.34 mmol, 1.0 eq) was dissolved in 2.0 mL of toluene, added to the reaction solution dropwise in an ice water bath, and reacted at room temperature overnight. TLC plate monitoring showed the disappearance of Compound 114-1 and the generation of a new spot. LCMS monitoring showed the generation of Compound 114-3. 30.0 ml of water was added to the reaction solution, and then the reaction solution was extracted with EA three times (10.0 ml×3). The organic phases were combined, washed and extracted with saturated brine twice, dried over anhydrous sodium sulfate, concentrated, and purified by silica gel column chromatography (eluent: ethyl acetate:petroleum ether=1:5) to obtain Compound 114-3 (160 mg, 49%). LCMS:(ESI) m/z 185.95, (M+H) +; $^1$H NMR (400 MHz, $CD_3OD$) δ 8.83 - 8.75 (m, 2H), 7.90 (d, J = 8.7 Hz, 1H), 7.61 (d, J = 8.7 Hz, 1H), 7.54 (dd, J = 8.7, 4.3 Hz, 1H), 4.32 (s, 1H), 3.39 - 3.35 (m, 1H), 2.84 (dd, J = 5.5, 2.7 Hz, 1H), 2.61 (s, 3H).

Compound 114-3 (93 mg, 0.5 mmol, 1.5 eq) was dissolved in acetonitrile (5.0 mL) at room temperature, and Compound A4 (100 mg, 0.34 mmol, 1.0 eq) and $Er(OTf)_3$ (420 mg, 0.68 mmol, 2.0 eq) were added, purged with nitrogen, and sealed to reflux at 80°C for reacting overnight. LCMS monitoring showed the generation of Compound 114-4. 15 mL of water was added, and then the reaction solution was extracted with ethyl acetate three times (10.0 ml×3). The organic phases were combined, dried over anhydrous sodium sulfate, concentrated, and purified by thin-layer chromatography (developing solvent: DCM/MeOH=20/1) to obtain Compound 114-4 (120 mg, crude) as a yellow oil. LCMS:(ESI) m/z 484.25, (M+H) +.

Compound 114-4 (120 mg, crude) was dissolved in 2.0 mL of methanol at room temperature, and 2.0 mL of 4N hydrochloric acid in 1,4-dioxane was added, and reacted at room temperature for 1 hour. The reaction solution was subjected to rotary drying, and purified by preparative high performance liquid chromatography to obtain Compound 114 (1.3 mg, white solid). LCMS (ESI) m/z 400.05, (M+H)+; $^1$H NMR (400 MHz, $CD_3OD$) δ 9.70 (d, J = 8.9 Hz, 1H), 9.01 (d, J = 5.1 Hz, 1H), 7.94 (d, J = 8.8 Hz, 1H), 7.82 (d, J = 14.1 Hz, 1H), 7.70 (d, J = 8.8 Hz, 1H), 7.45 (d, J = 15.2 Hz, 2H), 7.07 (s, 1H), 6.96 (d, J = 11.7 Hz, 1H), 6.53 (d, J = 2.2 Hz, 1H), 5.92 (t, J = 7.2 Hz, 1H), 5.14 (d, J = 22.4 Hz, 1H), 5.00 (s, 1H), 3.75 (s, 3H), 2.05 (s, 3H).

Example 115

6-methyl-5-(1-((3-(1-methyl-1H-pyrazol-4-yl)-1H-indazol-5-yl)thio)ethyl)quinolin e

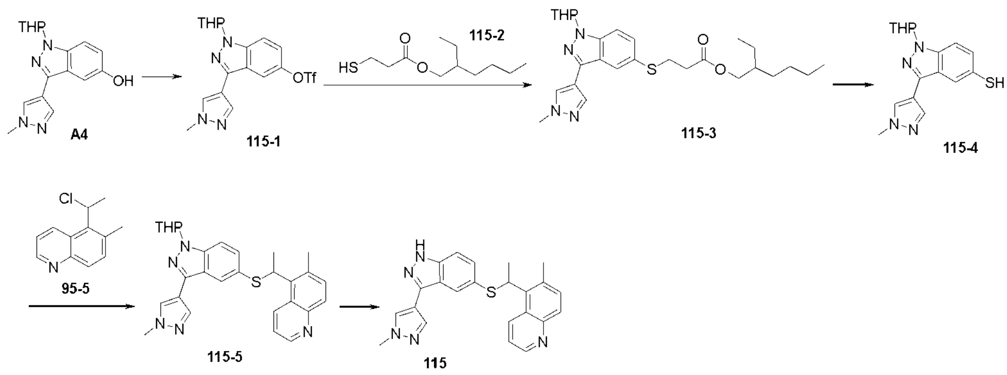


Compound A4 (1.0 g, 3.35 mmol, 1.0 eq.) was dissolved in DCM (10 mL), then the compound DIEA (1.7 g, 6.7 mmol, 4.0 eq.) was added to the reaction solution, and purged with nitrogen three times. $Tf_2O$ (1.9 g, 6.7 mmol, 2.0 eq) was slowly added dropwise to the reaction solution in an ice bath, and then reacted under stirring at room temperature overnight. TLC showed the conversion of most of raw materials into a product. The reaction solution was extracted with (10 ml) dichloromethane and water three times. The organic phases were combined, washed with saturated brine, dried over anhydrous sodium sulfate, filtered, concentrated, and purified by silica gel column chromatography (eluent: petroleum ether:ethyl acetate=100:1 ~50:1) to obtain Compound 115-1 as a white solid (596 mg, yield: 41%). $^1$H NMR (400 MHz, DMSO-d6) δ 8.48 (s, 1H), 8.23 (d, J = 2.3 Hz, 1H), 8.04 (s, 1H), 7.93 (d, J = 9.2 Hz, 1H), 7.57 - 7.51 (m, 1H), 5.91 (d, J = 9.3 Hz, 1H), 3.94 (s, 4H), 3.76 (t, J = 12.5 Hz, 1H), 2.46 - 2.36 (m, 1H), 2.00 (d, J = 15.5 Hz, 2H), 1.76 (s, 1H), 1.59 (s, 2H).

Compound 115-1 (500 mg, 1.16 mmol, 1.0 eq.) was dissolved in dioxane (10 mL), and then Compound 115-2 (506 mg, 2.32 mmol, 2.0 eq.), 4,5-bis(diphenylphosphino)-9,9-dimethyl-xanthene (69 mg, 0.12 mmol, 0.1 eq), Pd2(dba)3 (110 mg, 0.12 mmol, 0.1 eq) and DIPEA (449mg, 3.48 mmol, 3.0 eq) were added respectively, then purged with nitrogen three times, and reacted under stirring at 100°C overnight. LCMS showed the presence of a target product. The reaction solution was extracted with ethyl acetate (10 ml) three times. The organic phases were combined, washed with saturated brine, dried over anhydrous sodium sulfate, filtered, concentrated, and purified by thin-layer chromatography (DCM: MeOH=20:1) to obtain Compound 115-3 as a yellow oil (500 mg, yield: 86.44%). $^1$H NMR (400 MHz, DMSO-d6) δ 8.43 (s, 1H), 8.02 - 7.95 (m, 2H), 7.72 (d, J = 8.8 Hz, 1H), 7.47 (dd, J = 8.7, 1.6 Hz, 1H), 5.86 - 5.80 (m, 1H), 3.91 (d, J = 16.6 Hz, 6H), 3.78 - 3.69 (m, 1H), 3.18 (t, J = 6.8 Hz, 2H), 2.57 (t, J = 6.8 Hz, 2H), 2.00 (d, J = 18.2 Hz, 2H), 1.82 - 1.39 (m, 4H), 1.22 (d, J = 21.6 Hz, 8H), 0.80 (dd, J = 9.6, 5.5 Hz, 6H).

Compound 115-3 (500 mg, 1.0 mmol, 1.0 eq.) was dissolved in THF (10 mL), and then KOtBu (225 mg, 2.0 mmol, 2.0 eq.) was added to the reaction solution in an ice salt bath, then warmed up to room temperature and stirred for 1 hour. TLC showed the conversion of most of raw materials into a product. The reaction solution was filtered, and concentrated to obtain Compound 115-4 as a yellow solid (265 mg, yield: 84.39%). LCMS:(ESI) m/z 376, $(M+H)^+$.

Compound 115-4 (200 mg, 0.64 mmol, 1.0 eq.) was dissolved in MeCN (10 mL), and then Compound 95-5 (197 mg, 0.96 mmol, 1.5 eq.) and $Cs_2CO_3$ (624 mg, 1.92 mmol, 3.0 eq) were added respectively, then purged with nitrogen three times, and reacted under stirring at 80°C overnight. LCMS showed the presence of a target product. The reaction solution was extracted with ethyl acetate (10 ml) three times. The organic phases were combined, washed with saturated brine, dried over anhydrous sodium sulfate, filtered, concentrated, and purified by thin-layer chromatography (DCM: MeOH=20:1) to obtain Compound 115-5 as a white oil (81 mg, yield: 40.31 %). $^1$H NMR (400 MHz, $CD_3OD$) δ 9.36 (d, J = 8.8 Hz, 1H), 8.84 (s, 1H), 7.83 - 7.75 (m, 3H), 7.64 - 7.50 (m, 3H), 7.42 - 7.37 (m, 2H), 5.74 (d, J = 10.2 Hz, 1H), 5.18 (d, J = 7.6 Hz, 1H), 3.98 (s, 4H), 3.79 (t, J = 11.2 Hz, 1H), 2.49 (d, J = 12.3 Hz, 1H), 2.19 (d, J = 17.7 Hz, 4H), 2.07 - 1.95 (m, 1H), 1.90 (d, J = 7.4 Hz, 3H), 1.66 (d, J = 5.3 Hz, 2H).

Compound 115-5 (40 mg, 0.08 mmol, 1.0 eq.) was dissolved in MeCN (3 mL), and then 10 mL of hydrogen chloride-1,4 dioxane solution was added to the reaction system, and stirred at room temperature for 3 hours. LCMS showed the complete conversion of raw materials. The reaction solution was concentrated and purified by preparative high performance liquid chromatography to obtain Compound 115 as a white solid (16.8 mg, yield: 43.49 %, purity 98%). LCMS:(ESI) m/z 400, (M+H); $^1$H NMR (400 MHz, DMSO-d6) δ 13.05 (s, 1H), 9.12 (d, J = 8.8 Hz, 1H), 8.88 (s, 1H), 8.14 (s, 1H), 7.87 (s, 1H), 7.84 - 7.76 (m, 2H), 7.60 (dd, J = 8.9, 4.1 Hz, 1H), 7.43 (dd, J = 14.3, 8.7 Hz, 2H), 7.30 (d, J = 9.2 Hz, 1H), 5.10 (d, J = 7.8 Hz, 1H), 3.93 (s, 3H), 2.26 (s, 3H), 1.82 (d, J = 7.2 Hz, 3H).

Example 116

6-cyclopropyl-5-(1-((3-(1-methyl-1H-pyrazol-4-yl)-1H-indazol-5-yl)oxy)ethyl)qui noline

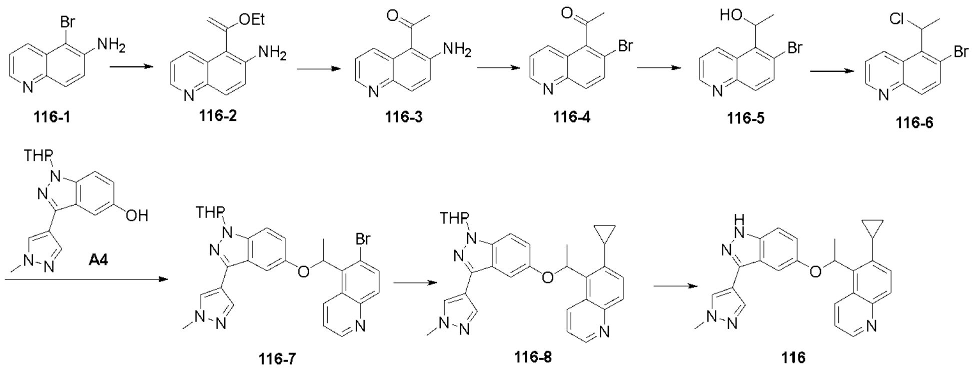

116-1 116-2 116-3 116-4 116-5 116-6

A4

116-7 116-8 116

At room temperature, Compound 116-1 (1.078 g, 4.83 mmol), tri-n-butyl (1-ethoxyvinyl)tin (2.617 g, 7.25 mmol) and $Pd(PPh_3)_4$ (0.558 g, 0.483 mmol) were added to 1,4-dioxane (24.0 mL). The resulting mixture was stirred at 120°C for 24 hours. LCMS showed the generation of a product. The reaction mixture was cooled to room temperature, and aqueous KF solution (1.0 M, 36.0 mL) was added. The resulting mixture was vigorously stirred for 10 minutes, and filtered through diatomite. The filter cake was washed successively with dichloromethane (30.0 mL ×2) and water (30.0 mL × 2). The filtrates were combined, the organic phase was separated, and the aqueous phase was extracted with dichloromethane (20.0 mL x 3). The organic phases were combined, washed successively with aqueous KF solution (1.0 M, 30.0 mL) and water (30.0 mL), dried over anhydrous sodium sulfate, and filtered. The filtrate was concentrated to dryness under reduced pressure. The resulting residue was dried under vacuum to obtain a crude product Compound 116-2 as a dark brown sticky liquid (3.92 g, crude yield: >100%, purity: 90.85%). LCMS (ESI) m/z 215.0, (M+H)+.

At room temperature, aqueous HCl solution (3.0 M, 16.1 mL, 48.3 mmol) was slowly added dropwise to a solution of the crude product Compound 116-2 (3.92 g, 4.83 mmol (a theoretical amount)) in tetrahydrofuran (24.2 mL). The resulting mixture was stirred for 20 minutes. LCMS showed the generation of a product. Saturated aqueous sodium bicarbonate solution was slowly added dropwise to the reaction mixture, and the pH of the aqueous phase was adjusted to about 7 to 8. The resulting mixture was extracted with dichloromethane (30.0 mL × 6). The organic phases were combined, dried over anhydrous sodium sulfate, and filtered. The filtrate was concentrated to dryness under reduced pressure. The resulting residue was purified by silica gel column chromatography (eluent: dichloromethane: ethyl acetate=10:1) to obtain Compound 116-3 as a dark yellow liquid (0.632 g, yield: 70%, purity: 98.38%): LCMS (ESI) m/z 187.0, (M+H)+.

At room temperature, Compound 116-3 (0.632 g, 3.4 mmol) and $CuBr_2$ (0.908 g, 4.07 mmol) were added to acetonitrile (17.0 mL). The resulting mixture was stirred for 10 minutes, and then amyl nitrite (0.516 g, 4.40 mmol) was added dropwise. The reaction mixture was stirred at 30°C. LCMS showed the generation of a product. The reaction mixture was cooled to room temperature, diluted with ethyl acetate (60.0 mL), and then washed with saturated aqueous sodium bicarbonate solution (20.0 mL x 3). The aqueous phases were combined, and extracted with ethyl acetate (30.0 mL × 2). The organic phases were combined, dried over anhydrous sodium sulfate, and filtered. The filtrate was concentrated to dryness under reduced pressure. The resulting residue was purified by silica gel column chromatography (eluent: dichloromethane:100%) to obtain Compound 116-4 as a yellow solid (0.507 g, yield: 60%, purity: 98.10%). LCMS (ESI) m/z 250.0, 252.0, (M+H)+.

At room temperature, $NaBH_4$ (0.115 g, 3.04 mmol) was added in three batches to a solution of Compound 116-4 (0.206 g, 0.82 mmol) in methanol (4.1 mL). The resulting mixture was stirred for 8 hours. LCMS showed the generation of a product. The reaction mixture was concentrated to dryness under reduced pressure, and saturated aqueous ammonium chloride solution (10.0 mL) and ethyl acetate (10.0 mL) were added to the resulting residue. The organic phase was separated, and the aqueous phase was extracted with ethyl acetate (10.0 mL × 4). The organic phases were combined, dried over anhydrous sodium sulfate, and filtered. The filtrate was concentrated to dryness under reduced pressure. The resulting residue was purified by silica gel column chromatography (eluent: petroleum ether: ethyl acetate=2:1) to obtain Compound 116-5 as a yellow sticky liquid (0.183 g, yield: 89%, purity: 96.86%): LCMS (ESI) m/z 251.9, 253.9, (M+H)+.

At 0°C, thionyl chloride (0.257 g, 2.16 mmol) was added dropwise to a solution of Compound 116-5 (0.183 g, 0.72 mmol) in dichloromethane (7.2 mL). The resulting mixture was stirred at 0°C for 10 minutes, warmed up to room temperature and stirred for 2 hours. LCMS showed the generation of a product. The reaction mixture was diluted with dichloromethane (10.0 mL), and concentrated to dryness under reduced pressure. Dichloromethane (10.0 mL) was added

to the resulting residue, and concentrated to dryness under reduced pressure again. This operation was repeated once again, and the resulting residue was dried under vacuum to obtain a crude product Compound 116-6 as a dark yellow solid (0.222 g, crude yield: 100%, purity: 47.06%). LCMS (ESI) m/z 269.9, 271.9, 273.9, (M+H)+.

At room temperature, the crude product Compound 116-6 (0.222 g, 0.72 mmol (a theoretical amount)) and cesium carbonate (1.173 g, 3.60 mmol) were added successively to N,N-dimethylformamide (7.2 mL). The resulting mixture was stirred for 2 minutes, and then Compound A4 (0.215 g, 0.72 mmol) was added. The reaction mixture was stirred at 60°C for 9 hours. LCMS showed the generation of a product. The reaction mixture was cooled to room temperature, diluted with ethyl acetate (30.0 mL), and then washed with water (10.0 mL × 3). The organic phase was dried over anhydrous sodium sulfate, and filtered. The filtrate was concentrated to dryness under reduced pressure. The resulting residue was purified by silica gel column chromatography (eluent: petroleum ether:dichloromethane:ethyl acetate=1:1:1) to obtain Compound 116-7 as a yellow solid (0.0872 g, yield: 23%, purity: 93.42%). LCMS (ESI) m/z 532.1, 534.1, (M+H)+.

At room temperature, Compound 116-7 (0.0426 g, 0.080 mmol), cyclopropylboronic acid (0.0103 g, 0.12 mmol), Pd(dppf)$Cl_2$ (0.0059 g, 0.0080 mmol) and $Na_2CO_3$ (0.017 g, 0.16 mmol) were added to a mixture of 1,4-dioxane (0.80 mL) and water (0.40 mL). The resulting mixture was stirred at 95°C for 12 hours. LCMS showed the generation of a product. The reaction mixture was cooled to room temperature, and diluted with ethyl acetate (15.0 mL) and water (5.0 mL). The organic phase was separated, and the aqueous phase was extracted with ethyl acetate (5.0 mL × 4). The organic phases were combined, dried over anhydrous sodium sulfate, and filtered. The filtrate was concentrated to dryness under reduced pressure. The resulting residue was purified by silica gel thin-layer chromatography (eluent: ethyl acetate : petroleum ether=4:1) to obtain Compound 116-8 as a yellow jelly (0.0251 g, yield: 63%, purity: 96.46%): LCMS (ESI) m/z 494.4, (M+H)+.

At room temperature, trifluoroacetic acid (0.290 g, 2.55 mmol) was added to a solution of Compound 116-8 (0.0251 g, 0.051 mmol) in dichloromethane (0.25 mL). The resulting mixture was stirred for 8 hours. LCMS showed the generation of a product. The reaction mixture was diluted with dichloromethane (5.0 mL), and saturated aqueous sodium bicarbonate solution was added to adjust the pH of the aqueous phase to about 7 to 8. The organic phase was separated, and the aqueous phase was extracted with dichloromethane (5.0 mL x 3). The organic phases were combined, dried over anhydrous sodium sulfate, and filtered. The filtrate was concentrated to dryness under reduced pressure. The resulting residue was purified by silica gel thin-layer chromatography (eluent: dichloromethane: methanol=20:1) to obtain a light brown solid 116 (0.0163 g, yield: 78%, purity: 100%): LCMS (ESI) m/z 410.2, $(M+H)^+$; $^1H$ NMR (400 MHz, DMSO) δ 12.74 (s, 1H), 9.27 (d, J = 8.8 Hz, 1H), 8.83 (dd, J = 4.0, 1.2 Hz, 1H), 7.97 (s, 1H), 7.84 (d, J = 8.8 Hz, 1H), 7.70 (s, 1H), 7.58 (dd, J = 8.8, 4.0 Hz, 1H), 7.38 (d, J = 8.8 Hz, 1H), 7.34 (d, J = 8.8 Hz, 1H), 7.20 (s, 1H), 7.07 (dd, J = 8.8, 2.0 Hz, 1H), 6.65 (q, J = 6.8 Hz, 1H), 3.94 (s, 3H), 2.63 (br s, 1H), 1.94 (d, J = 6.8 Hz, 3H), 1.28 - 1.24 (m, 2H), 1.97 - 1.21 (m, 2H).

Example 117

6-methyl-5-(1-((3-(1-methyl-1H-pyrazol-4-yl)-1H-pyrazino[3,4-b]pyridin-5-yl)oxy )ethyl)quinoline

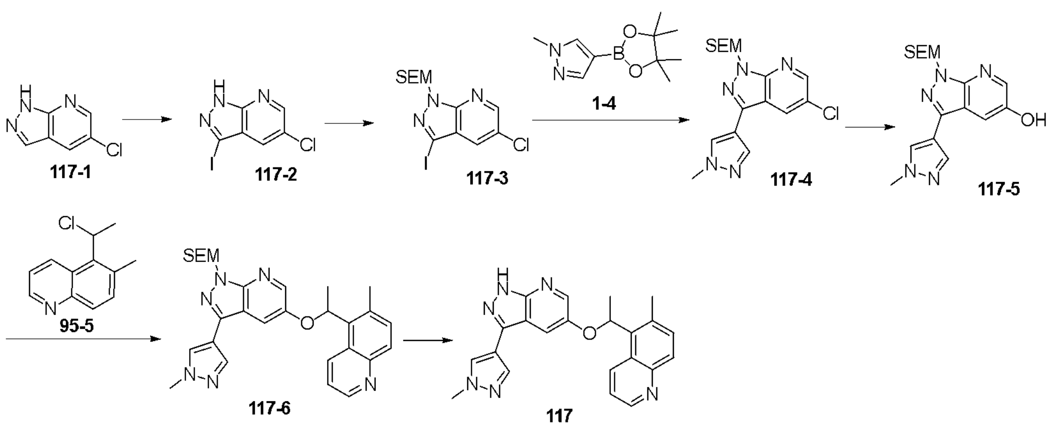


Compound 117-1 (500 mg, 3.26 mmol, 1.0 eq) was dissolved in DMF (5.0 mL), and NIS (1.5 g, 6.52 mmol, 2.0 eq) was added, and kept at room temperature overnight. LCMS monitoring showed the complete conversion of Compound 117-1 into Compound 117-2. 20.0 mL of water was added to the reaction solution, and a large amount of solid was precipitated, filtered, and subjected to rotary drying with an oil pump to obtain Compound 117-2 (800 mg, 87%). $^1H$ NMR

(400 MHz, DMSO-d6) δ 8.55 (d, J = 2.3 Hz, 1H), 8.07 - 8.03 (m, 1H).

Compound 117-2 (900 mg, 3.2 mmol, 1.0 eq) was dissolved in DMF (5.0 mL) at room temperature, NaH (155 mg, 6.4 mmol, 2.0 eq) was added in an ice water bath, stirred at room temperature for 0.5 hour, and then SEM-Cl (810 mg, 4.84 mmol, 1.5 eq) was added in an ice water bath, and reacted at room temperature overnight. LCMS monitoring showed the generation of Compound 117-3. 15 mL of water was added, and then the reaction solution was extracted with ethyl acetate three times (10.0 ml×3). The organic phases were combined, washed with aqueous 5% LiCl solution twice, dried over anhydrous sodium sulfate, concentrated, and purified by silica gel column chromatography (eluent: petroleum ether:ethyl acetate=5:1) to obtain a yellow oil 117-3 (760 mg, 57.5%). $^1$H NMR (400 MHz, $CD_3OD$) δ 8.56 (d, J = 2.2 Hz, 1H), 7.95 (d, J = 2.2 Hz, 1H), 5.77 (s, 2H), 3.64 (t, J = 8.0 Hz, 2H), 0.84 (t, J = 8.0 Hz, 2H), -0.11 (s, 9H) +.

Compound 117-3 (400 mg, 0.96 mmol, 1.0 eq) was dissolved in 10.0 mL of tetrahydrofuran at room temperature, and Compound 1-4 (600 mg, 2.88 mmol, 3.0 eq), $K_2CO_3$ (400 mg, 2.88 mmol, 3.0 eq), Pd[$PPh_3$]$_4$ (111 mg, 0.096 mmol, 0.1 eq) and 2.0 ml of water were added, purged with nitrogen, and reacted at 75°C overnight. LCMS monitoring showed the disappearance of Compound 117-3 and the generation of Compound 117-4. The reaction solution was subjected to suction filtration through diatomite, the filter cake was washed with ethyl acetate, an appropriate amount of water was added to the filtrate, and then the filtrate was extracted with EA 2-3 times. The obtained organic phases were combined, dried over anhydrous sodium sulfate, subjected to rotary drying, and purified by silica gel column chromatography (eluent: petroleum ether:ethyl acetate=5:1) to obtain Compound 117-4 as a yellow oil (300 mg, 84%). $^1$H NMR (400 MHz, $CD_3OD$) δ 8.53 (d, J = 2.2 Hz, 1H), 8.50 (d, J = 2.2 Hz, 1H), 8.29 (s, 1H), 8.05 (s, 1H), 5.79 (s, 2H), 3.98 (s, 3H), 3.70 - 3.62 (m, 2H), 0.86 (t, J = 8.0 Hz, 2H), -0.11 (s, 9H).

Compound 117-4 (200 mg, 0.55 mmol, 1.0 eq) was dissolved in dioxane (3.0 mL), and KOH (185 mg, 3.3 mmol, 6.0 eq), Pd2(dba)3 (45 mg, 0.055 mmol, 0.1 eq), methanesulfonato(2-di-t-butylphosphino-2',4',6'-triisopropyl-1,1'-biphenyl)(2'-amino-1,1'-biphen-2-yl)palladium(II) (26 mg, 0.027 mml, 0.05 eq) and water (3.0 mL) were added, purged with nitrogen, and kept at 100°C overnight. LCMS monitoring showed the generation of Compound 117-5. 5.0 mL of water was added to the reaction solution, and then the reaction solution was extracted with an appropriate amount of ethyl acetate. The obtained organic phases were combined, dried over anhydrous sodium sulfate, subjected to rotary drying, and purified by thin-layer chromatography (developing solvent: DCM/MeOH=20/1) to obtain a yellow solid 117-5 (25 mg, yield: 13%). $^1$H NMR (400 MHz, DMSO-d6) δ 9.77 (s, 1H), 8.33 (s, 1H), 8.22 (d, J = 2.6 Hz, 1H), 7.91 (d, J = 0.8 Hz, 1H), 7.68 (d, J = 2.6 Hz, 1H), 5.65 (s, 2H), 3.89 (s, 3H), 3.55 (t, J = 8.0 Hz, 2H), 0.80 - 0.75 (m, 2H), -0.15 (s, 9H).

Compound 117-5 (25 mg, 0.07 mmol, 1.0 eq) was dissolved in acetonitrile (5.0 mL), and Compound 95-5 (16 mg, 0.08 mmol, 1.2 eq) and $Cs_2CO_3$ (25 mg, 0.21 mmol, 3.0 eq) were added, purged with nitrogen, and kept at 80°C overnight. LCMS monitoring showed the generation of Compound 117-6. 8.0 mL of water was added to the reaction solution, and then the reaction solution was extracted with an appropriate amount of ethyl acetate. The obtained organic phases were combined, dried over anhydrous sodium sulfate, subjected to rotary drying, and purified by thin-layer chromatography (developing solvent: DCM/MeOH=20/1) to obtain a white solid 117-6 (23 mg, 61%). LCMS:(ESI) m/z 185.95, (M+H) +.

Compound 117-6 (23 mg) was dissolved in DCM (3.0 mL), and TFA (1.0 mL) was added, and reacted at room temperature for 1 hour. LCMS monitoring showed the complete reaction. The reaction solution was directly subjected to rotary drying, and purified by preparative high performance liquid chromatography to obtain a white solid 117 (7.2 mg, 41%). LCMS:(ESI) m/z 385.25, (M+H) +; $^1$H NMR (400 MHz, $CD_3OD$) δ 9.27 (d, J = 9.4 Hz, 1H), 8.81 - 8.76 (m, 1H), 8.27 (s, 1H), 7.90 - 7.81 (m, 2H), 7.68 (s, 1H), 7.64 - 7.57 (m, 2H), 7.49 (s, 1H), 6.36 - 6.27 (m, 1H), 3.95 (s, 3H), 2.72 (s, 3H), 1.99 - 1.94 (m, 3H).

The following compound was synthesized according to the the route of Example 117.

| Example | Name | Structure | LC-MS (M+H)$^+$ | $^1$H NMR |
|---|---|---|---|---|
| 131 | 6-methyl-5-(1-((3-(1-methyl-1H-pyrazol-4-yl)-1H-pyrazino[3,4-c]pyridin-5-yl)oxy)ethyl)quinoline | 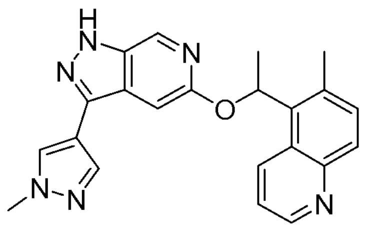 | 385.1 | $^1$H NMR (400 MHz, CDCl3) δ 9.35 (d, J = 12.8 Hz, 1H), 8.71 (s, 1H), 8.46 (s, 1H), 8.05 (s, 1H), 7.86 (s, 1H), 7.82 - 7.76 (m, 1H), 7.59 - 7.49 (m, 2H), 7.12 (s, 1H), 6.79 (d, J = 7.9 Hz, 1H), 3.95 (s, 3H), 2.76 (s, 3H), 1.88 (d, J = 7.3 Hz, 3H). |

Example 118

2-((3-(1-methyl-1H-pyrazol-4-yl)-1H-indazol-5-yl)oxy)-2-(6-methylquinolin-5-yl)acetonitrile

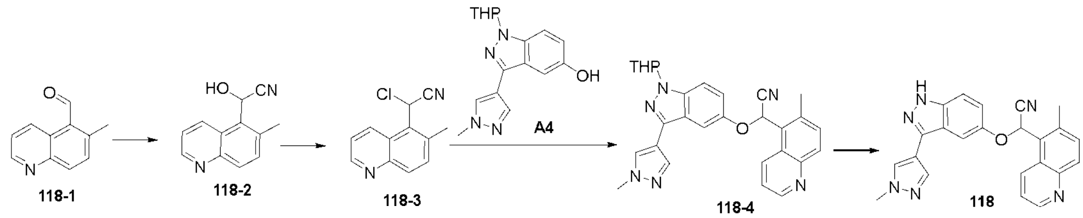

118-1 118-2 118-3 118-4 118

TMSCN (1.74 g, 17.5 mmol, 10.0 eq) was added to Compound 118-1 (300 mg, 1.75 mmol, 1.0 eq) and AcOH (5.0 mL) in an ice water bath, and reacted at room temperature for 3 days. TLC plate monitoring showed the disappearance of raw materials. Water was added, and then the reaction solution was extracted with EA. The organic phase was washed with saturated sodium bicarbonate, dried over anhydrous sodium sulfate, subjected to rotary drying, triturated with an appropriate amount of DCM, subjected to suction filtration, and the solid was collected, and subjected to rotary drying with an oil pump to obtain Compound 118-2 as a white solid (200 mg, 57%). $^1$H NMR (400 MHz, DMSO-d6) δ 8.86 (dd, J = 4.2, 1.5 Hz, 1H), 8.81 (d, J = 8.9 Hz, 1H), 7.95 (d, J = 8.7 Hz, 1H), 7.62 (d, J = 8.9 Hz, 1H), 7.14 (d, J = 4.6 Hz, 1H), 6.36 (d, J = 4.6 Hz, 1H), 2.59 (s, 3H).

Compound 118-2 (150 mg, 0.76 mmol, 1.0 eq) was dissolved in DCM (5.0 mL) at room temperature, TEA (231 mg, 2.2 mmol, 3.0 eq) was added, and then MsCl (131 mg, 1.14 mmol, 1.5 eq) was added in an ice water bath, and reacted at room temperature overnight. LCMS monitoring showed the generation of Compound 118-3. 15 mL of water was added, and then the reaction solution was extracted with DCM three times (10.0 mL×3). The organic phases were combined, washed with saturated aqueous sodium bicarbonate solution twice, dried over anhydrous sodium sulfate, concentrated, and purified by thin-layer chromatography (developing solvent: petroleum ether:ethyl acetate=1/1) to obtain Compound 118-3 as a yellow solid (80 mg, 48%). LCMS:(ESI) m/z 217.15, (M+H) +

Compound 118-3 (70 mg, 0.32 mmol, 1.5 eq) was dissolved in 10.0 mL of acetonitrile at room temperature, and Compound A4 (64 mg, 0.21 mmol, 1.0 eq) and $Cs_2CO_3$ (206 mg, 0.63 mmol, 3.0 eq) were added, purged with nitrogen, and reacted at 80°C for 10 minutes. LCMS monitoring showed the disappearance of Compound 118-3 and the generation of Compound 118-4. An appropriate amount of water was added to the reaction solution, and then the reaction solution was extracted with EA 2-3 times. The obtained organic phases were combined, dried over anhydrous sodium sulfate, subjected to rotary drying, and purified by thin-layer chromatography (developing solvent: DCM/MeOH=20/1) to obtain a yellow solid 118-4 (80 mg, 77%). LCMS:(ESI) m/z 185.95, (M+H) +; $^1$H NMR (400 MHz, DMSO-d6) δ 8.95 (s, 2H), 8.28 (s, 1H), 8.02 (s, 2H), 7.83 (s, 1H), 7.70 (d, J = 8.7 Hz, 3H), 7.27 (d, J = 2.5 Hz, 2H), 5.77 (d, J = 9.9 Hz, 1H), 3.94 (s, 3H), 3.85 (s, 1H), 3.69 (s, 1H), 2.74 (s, 3H), 2.30 (s, 1H), 2.01 (d, J = 13.0 Hz, 1H), 1.93 (d, J = 12.8 Hz, 1H), 1.71 (s, 1H), 1.55 (s, 2H).

Compound 118-4 (60 mg) was dissolved in 1.0 mL of methanol, and 1.0 mL of 4N HCl in dioxane was added, and reacted at room temperature for 0.5 hour. LCMS monitoring showed the complete reaction. The reaction solution was subjected to rotary drying and purified by preparative high performance liquid chromatography to obtain a white solid 118 (2.8 mg, 4%). LCMS:(ESI) m/z 384.10, (M+H) +; $^1$H NMR (400 MHz, DMSO-d6) δ 13.12 (s, 1H), 8.93 (s, 1H), 8.55 (d, J = 8.5 Hz, 1H), 8.39 (s, 1H), 8.12 (d, J = 8.7 Hz, 1H), 8.03 (s, 1H), 8.00 (s, 1H), 7.78 (s, 1H), 7.69 - 7.61 (m, 2H), 7.44 (d, J = 9.0 Hz, 1H), 3.91 (s, 3H), 2.71 (s, 3H).

Example 119

5-(1-((3-(1-methyl-1H-pyrazol-4-yl)-1H-indazol-5-yl)oxy)ethyl)cinnoline

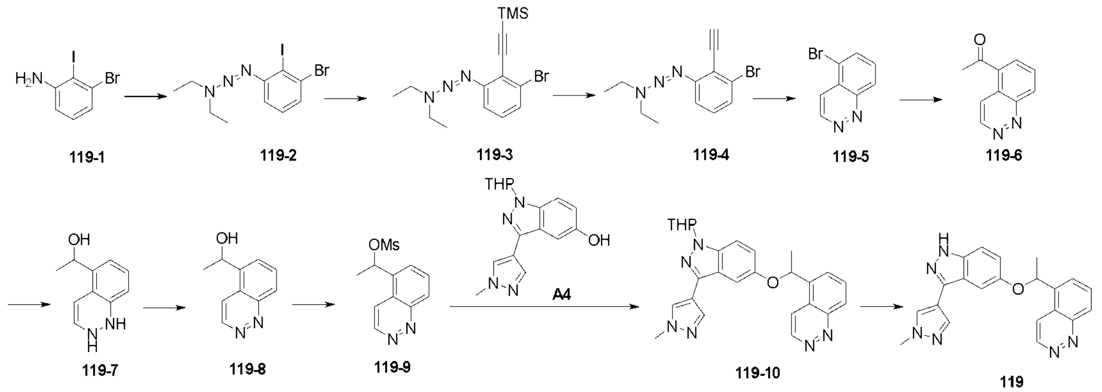


At 0°C, $NaNO_2$ (2663 mg, 38.6 mmol, 2.3 eq.) was slowly added to a mixed solution of Compound 119-1 (5.0 g, 16.8 mmol, 1.0 eq.) in acetonitrile (50.0 mL), water (25.0 mL) and 37% concentrated hydrochloric acid (12.0 mL). The reaction mixture was stirred at 0°C for 1 hour. At 0°C, the resulting reaction solution was was slowly added to a mixed solution of diethylamine (19.1 mL, 185 mmol, 11 eq.) and potassium carbonate (14.73 g, 107 mmol, 6.4 eq.) in acetonitrile (50.0 mL) and water (90.0 mL). The reaction mixture was stirred at room temperature for 16 hours. Water (100.0 mL) was added, and then the reaction mixture was extracted with ethyl acetate (100.0 mL x 2). The organic phases were combined, washed with saturated brine (100.0 mL), dried over anhydrous sodium sulfate, filtered, and concentrated under reduced pressure. The resulting residue was purified by silica gel column chromatography (eluent: ethyl acetate/petroleum ether=0-8.5%) to obtain Compound 119-2 (4.9 g, yield 76.4%).

At room temperature, Compound 119-2 (2.0 g, 5.25 mmol, 1.0 eq.), trimethylethynylsilane (619 mg, 6.3 mmol, 1.2 eq.), $Pd(PPh_3)_2Cl_2$ (1.1 g, 1.58 mmol, 0.3 eq.), CuI (301 mg, 1.58 mmol, 0.3 eq.) and triethylamine (137 mg, 0.42 mmol, 3.0 eq.) were added successively to DMF (20.0 mL). The reaction mixture was stirred at 40°C for 16 hours. The reaction solution was cooled to room temperature, water (80.0 mL) was added, and then the reaction solution was extracted with ethyl acetate (100.0 mL × 2). The organic phases were combined, washed with saturated brine (100.0 mL), dried over anhydrous sodium sulfate, filtered, and concentrated under reduced pressure. The resulting residue was purified by silica gel column chromatography (eluent: ethyl acetate/petroleum ether=0-7%) to obtain Compound 119-3 (1.2 g, yield 65%).

At room temperature, TBAF (1M, 6.8 mL, 6.8 mmol, 2.0 eq.) was slowly added to a solution of Compound 119-3 (1.2 g, 3.4 mmol, 1.0 eq.) in THF (20.0 mL). The reaction mixture was stirred at room temperature for 3 hours. The reaction solution was cooled to 0°C, slowly poured into aqueous $NH_4Cl$ solution (40.0 mL), and extracted with EA (20.0 mL × 2). The organic phases were combined, washed with saturated brine (20.0 mL), dried over anhydrous sodium sulfate, filtered, and concentrated under reduced pressure. The resulting residue was purified by silica gel column chromatography (eluent: ethyl acetate/petroleum ether=0-6.5%) to obtain Compound 119-4 (631 mg, yield 66%).

At room temperature, Compound 119-4 (631 mg, 2.25 mmol, 1.0 eq.) was added to 1,2-dichlorobenzene (12.0 mL). The reaction mixture was stirred at 220°C with microwave for 1 hour under nitrogen protection. The reaction mixture was concentrated under reduced pressure, and the resulting residue was purified by silica gel column chromatography (eluent: ethyl acetate/petroleum ether=0-25%) to obtain Compound 119-5 (303 mg, yield 64.4%).

At room temperature, Compound 119-5 (303 mg, 1.45 mmol, 1.0 eq.), tri-n-butyl(1-ethoxyvinyl)tin (785 mg, 2.17 mmol, 1.5 eq.) and $Pd(PPh_3)_4$ (335 mg, 0.29 mmol, 0.2 eq.) were added successively to dioxane (8.0 mL). The reaction mixture was stirred at 110°C for 16 hours under nitrogen protection. The reaction solution was cooled to room temperature, and saturated aqueous KF solution (10.0 mL) was added. The reaction solution was stirred at room temperature for 1 hour. The reaction solution was filtered, and the filtrate was extracted with ethyl acetate (10.0 mL × 2). The organic phases were combined, dried over anhydrous sodium sulfate, filtered, and concentrated under reduced pressure. The resulting residue was dissolved in THF (10.0 mL), and 3N HCl (5.0 mL) was slowly added. The reaction solution was stirred at room temperature for 40 minutes, saturated aqueous sodium bicarbonate solution was added to adjust the pH to >7, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/petroleum ether=0-64%) to obtain Compound 119-6 (388 mg, purity 55%, yield 85.5%).

At 0°C, $NaBH_4$ (120 mg, 3.16 mmol, 9.9 eq.) was slowly added to a mixed solution of Compound 119-6 (100 mg, purity 55%, 0.32 mmol, 1.0 eq.) in THF (5.0 mL) and methanol (5.0 mL). The reaction mixture was stirred at room temperature for 2 hours. The reaction solution was cooled to 0°C, slowly poured into aqueous $NH_4Cl$ solution (20.0 mL), and extracted with ethyl acetate (20.0 mL × 2). The organic phases were combined, washed with saturated brine (10.0 mL), dried over anhydrous sodium sulfate, filtered, and concentrated under reduced pressure. The resulting residue Compound 119-7 was directly used for the next reaction step without purification.

At room temperature, Compound 119-7 (57 mg, 0.32 mmol, 1.0 eq.) and $MnO_2$ (280 mg, 3.2 mmol, 10.0 eq.) were added successively to DCM (6.0 mL). The reaction mixture was stirred at 40°C for 6 hours. The reaction mixture was concentrated under reduced pressure, and the resulting residue was purified by silica gel column chromatography (eluent: ethyl acetate/petroleum ether=0-69%) to obtain Compound 119-8 (25 mg, total yield 45% for two steps).

At 0°C, methylsufonyl chloride (18 mg, 0.157 mmol, 1.1 eq.) was slowly added to a solution of Compound 119-8 (25 mg, 0.14 mmol, 1.0 eq.) and TEA (17 mg, 0.17 mmol, 1.2 eq.) in DCM (1.5 mL). The reaction solution was warmed up to room temperature, and stirred for 3 hours. Water (10.0 mL) was added, and then the reaction solution extracted with dichloromethane (10.0 mL x 2). The organic phases were combined, washed with saturated brine (10.0 mL), dried over anhydrous sodium sulfate, filtered, and concentrated under reduced pressure. The resulting residue 119-9 was directly used for the next reaction step without purification.

At room temperature, Compound 119-9 (36 mg, 0.14 mmol, 1.0 eq.), CompoundA4 (46 mg, 0.15 mmol, 1.1 eq.) and cesium carbonate (137 mg, 0.42 mmol, 3.0 eq.) were added successively to DMF (1.5 mL). The reaction mixture was stirred at 60°C for 3 hours. The reaction solution was cooled to room temperature, water (10.0 mL) was added, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-22%) to obtain Compound 119-10 (80 mg, purity 60%, total yield 73.8% for two steps).

At room temperature, TFA (1.0 mL) was slowly added to a solution of Compound 119-10 (80 mg, purity 60%, 0.106 mmol, 1.0 eq.) in DCM (3.0 mL). The reaction mixture was stirred at room temperature for 3 hours. The reaction mixture was concentrated under reduced pressure, and the resulting residue was dissolved in DCM (10.0 mL), aqueous sodium bicarbonate solution was added to adjust the pH to >7, and then the resulting residue was extracted with DCM (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-35%) to obtain Compound 119 (22 mg, yield 56.2%, purity 100%). LCMS (ESI) m/z=371.2 $(M+H)^+$; $^1H$ NMR (400 MHz, DMSO) δ 12.77 (s, 1H), 9.49 (d, J = 6.1 Hz, 1H), 8.76 (d, J = 6.2 Hz, 1H), 8.39 (t, J = 13.0 Hz, 1H), 8.13 - 8.04 (m, 2H), 7.95 (dt, J = 17.9, 9.0 Hz, 1H), 7.79 (s, 1H), 7.39 (d, J = 9.0 Hz, 1H), 7.34 (d, J = 1.5 Hz, 1H), 7.13 (dt, J = 14.7, 7.3 Hz, 1H), 6.45 (q, J = 6.2 Hz, 1H), 3.91 (s, 3H), 1.78 (d, J = 6.2 Hz, 3H).

Example 121

6-methyl-5-(2,2,2-trifluoro-1-((3-(1-methyl-1H-pyrazol-4-yl)-1H-indazol-5-yl)oxy )ethyl)quinoline

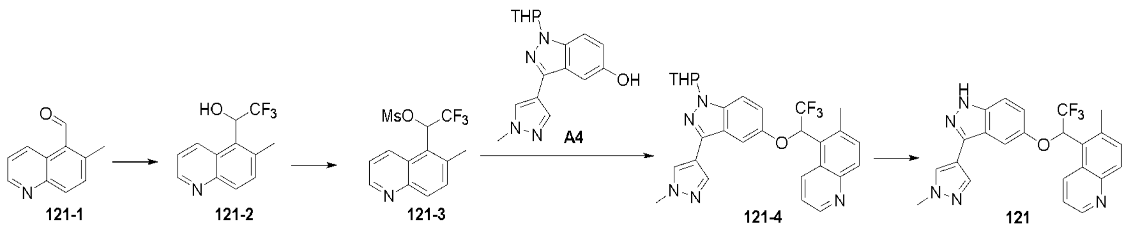

Compound 121-1 (500 mg, 2.9 mmol, 1.0 eq.) was dissolved in DMF (10 ml), purged with nitrogen, then cooled to 0°C, and $CF_3$-TMS (4.15 g, 29.2 mmol, 10.0 eq.) was added, and stirred at room temperature overnight. TLC showed no remaining raw materials. The reaction solution was extracted with ethyl acetate three times (10ml×3). The organic phases were combined, washed with saturated brine, dried over anhydrous sodium sulfate, filtered, concentrated, and purified by column chromatography (petroleum ether:EtOAc=0%-15%EA) to obtain Compound 121-2 as a yellow solid (300 mg, 42%). LCMS:(ESI) m/z 242, $(M+H)^+$

Compound 121-2 (300 mg, 1.2 mmol, 1.0 eq.) was dissolved in DCM (6 ml), TEA (376.9 mg, 3.7 mmol, 3.0 eq.) was added, cooled, and then MsCl (350.7 mg, 2.5 mmol, 2.0 eq.) was added, and stirred at room temperature for 2 hours. TLC showed no remaining raw materials, and the generation of a new spot. 10 mL of water was added, and then the reaction solution was extracted with DCM three times (10ml×3). The organic phases were combined, washed with saturated $NaHCO_3$, dried over anhydrous sodium sulfate, filtered, and concentrated to obtain Compound 121-3 as a yellow oil (700 mg, crude). LCMS:(ESI) m/z320, (M+H)+.

Compound A4 (150 mg, 0.5 mmol, 1.0 eq.) was dissolved in acetonitrile (10 ml), and then cesium carbonate (327.6 mg, 1.0 mmol, 3.0 eq.) and Compound 121-3 (402 mg, crude) were added. After the addition was completed, the reaction solution was reacted under stirring at 80°C for 3 hours. TLC showed a small amount of raw materials remaining.

The reaction solution was directly filtered, and the filtrate was dried under vacuum, and purified by thin-layer chromatography (developing solvent: DCM: MeOH=20:1) to obtain Compound 121-4 as a yellow oil (21 mg, crude). LCMS:(ESI) m/z 522, (M+H)+

Compound 121-4 (21 mg, 0.096 mmol, 1.0 eq) was dissolved in DCM/TFA (5 mL/1 mL), and stirred at room temperature for 1 hour. TLC monitoring showed the disappearance of 121-4 and the generation of a spot with increased polarity, and LCMS monitoring showed the generation of Compound 121. The reaction solution was directly subjected to rotary drying, and purified by preparative high performance liquid chromatography to obtain Compound 121 as a yellow solid (9.9 mg, yield: 56.3%, purity: 100%). LCMS:(ESI) m/z 438, (M+H)+; $^1H$ NMR (400 MHz, DMSO-d6) δ 13.00 (s, 1H), 8.93 (s, 1H), 8.28 (s, 1H), 8.18 - 8.10 (m, 2H), 7.95 (s, 1H), 7.75 (d, J = 8.7 Hz, 1H), 7.60 (s, 2H), 7.48 (s, 1H), 7.05 (s, 2H), 6.90 (s, 0H), 3.93 (s, 3H), 2.53 (s, 3H).

Example 122

6-methyl-5-(1-((3-(1-methyl-1H-pyrazol-4-yl)-1H-indazol-5-yl)oxy)ethyl-2,2,2-d3 )quinoline

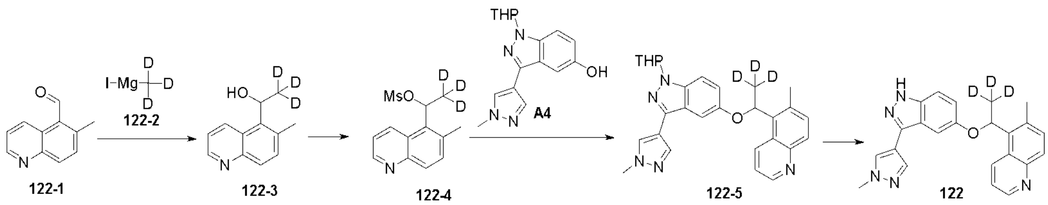

Compound 122-1 (100 mg, 0.58 mmol, 1.0 eq.) was dissolved in THF (5mL), and then Compound 122-2 (1.1 mL, 1.16 mmol, 2.0 eq.) was added. After the addition was completed, the reaction solution was purged with nitrogen three times, and reacted under stirring at -20°C for two hours. TLC showed no remaining raw materials, and the conversion of most of raw materials into a product. The reaction solution was extracted with (20 ml) ethyl acetate three times. The organic phases were combined, washed with saturated brine, dried over anhydrous sodium sulfate, filtered, concentrated, and purified by thin-layer chromatography (developing solvent: DCM: MeOH=20:1) to obtain Compound 122-3 as a yellow solid (80 mg, yield: 72%). LCMS:(ESI) m/z 191, (M+H) +.

Compound 122-3 (80 mg, 0.42 mmol, 1.0 eq.) was dissolved in DCM (5.0 mL), and then the compound MsCl (0.05 ml, 0.63 mmol, 1.5 eq.) and the compound TEA (127 mg, 1.26 mmol, 3.0 eq) were added respectively. After the addition was completed, the reaction solution was purged with nitrogen three times, and reacted under stirring at 25°C for two hours. TLC showed no remaining raw materials, and the conversion of most of raw materials into a product. The reaction solution was extracted with (20 ml) ethyl acetate three times. The organic phases were combined, washed with saturated brine, dried over anhydrous sodium sulfate, filtered, concentrated, and purified by pre-TLC (DCM: MeOH=25:1) to obtain Compound 122-4 as a yellow oil (100 mg, 88 %). LCMS:(ESI) m/z 209, (M+H) +.

Compound 122-4 (100 mg, 0.48 mmol, 1.5 eq.) was dissolved in acetonitrile (5 ml), and then Compound A4 (98 mg, 0.32 mmol, 1.0 eq.) and the compound $Cs_2CO_3$ (312 mg, 0.96 mmol, 3.0 eq) were added respectively. After the addition was completed, the reaction solution was purged with nitrogen three times, and reacted under stirring at 80°C overnight. TLC showed no remaining raw materials, and the conversion of most of raw materials into a product. Saturated ammonium chloride solution was added dropwise to quench the reaction. The reaction solution was extracted with (10 ml) ethyl acetate three times. The organic phases were combined, washed with saturated brine, dried over anhydrous sodium sulfate, filtered, concentrated, and purified by thin-layer chromatography (developing solvent: DCM: MeOH=20:1) to obtain Compound 122-5 as a yellow solid (80 mg, 53%). LCMS:(ESI) m/z 471, (M+H)+.

Compound 122-5 (80 mg, 0.17 mmol, 1.0 eq.) was dissolved in DCM (5 mL), and then the compound TFA (2.0 ml) was added. After the addition was completed, the reaction solution was purged with nitrogen three times, and reacted under stirring at 25°C for two hours. TLC showed no remaining raw materials, and the conversion of most of raw materials into a product. Saturated ammonium chloride solution was added dropwise to quench the reaction. The reaction solution was extracted with (10 mL) ethyl acetate three times. The organic phases were combined, washed with saturated brine, dried over anhydrous sodium sulfate, filtered, concentrated, and purified by preparative high performance liquid chromatography to obtain Compound 122 as a yellow solid (22 mg, purity :98%, with a yield: 35%). LCMS:(ESI) m/z 387, (M+H)+; $^1H$ NMR (400 MHz, $CD_3OD$) δ 9.29 (d, J = 9.1 Hz, 1H), 8.75 (s, 1H), 7.88 - 7.83 (m, 1H), 7.67 (s, 1H), 7.61 (s, 2H), 7.54 (d, J = 11.8 Hz, 1H), 7.32 (d, J = 9.2 Hz, 1H), 7.10 (d, J = 11.3 Hz, 1H), 6.90 (s, 1H), 6.19 (s, 1H), 3.93 (s, 3H), 2.71 (s, 3H).

Example 127

6-(difluoromethyl)-5-(1-((3-(1-methyl-1H-pyrazol-4-yl)-1H-indazol-5-yl)oxy)ethyl )quinoline

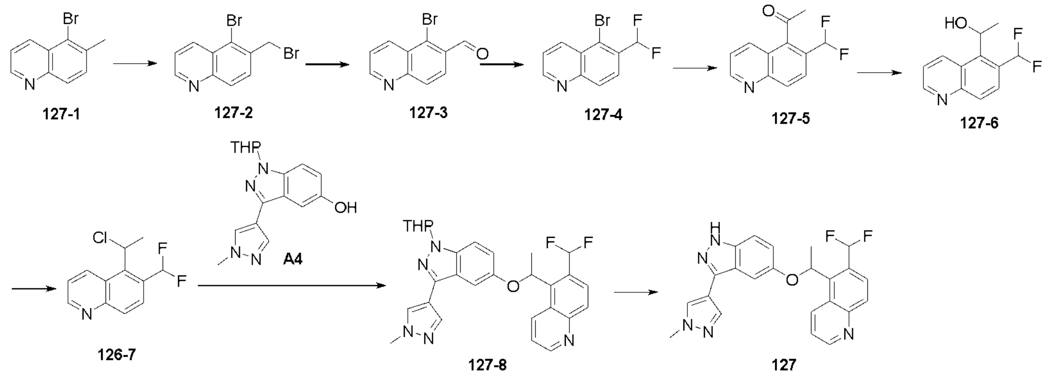

127-1
127-2
127-3
127-4
127-5
127-6
A4
126-7
127-8
127

At room temperature, Compound 127-1 (1 g, 4.5 mmol) was dissolved in $CCl_4$ (15 mL), and NBS (801 mg, 4.5 mmol) and AIBN (74 mg, 0.45 mmol) were added successively, then heated under nitrogen protection, and stirred at 85°C for 3 h. LCMS showed a ratio of the raw material, product, and dibromide by-product was 1:6:3. The reaction solution was cooled to room temperature, and then extracted with DCM (30 mL x 3). The organic phases were combined and then subjected to rotary drying. The crude was purified by silica gel column chromatography (eluent: petroleum ether:ethyl acetate=3:1) to obtain a product 127-2 as a yellow solid (710 mg, purity 93%). LCMS (ESI) m/z 300.02 & 302.02, (M+H)+.

The mixture 127-2 (710 mg, 2.369 mmol) was dissolved in MeCN (15 mL), and NMO (1.1 g, 9.436 mmol) was added thereto in an ice bath, and stirred at room temperature overnight. LCMS showed the generation of Product 3, and no remaining raw materials. The reaction solution was diluted with water, and then extracted with DCM (30 mL × 3). The organic phases were combined and subjected to rotary drying, and the crude was purified by silica gel column chromatography (eluent: petroleum ether:ethyl acetate=5:1) to obtain a product 127-3 as a white solid (546 mg, purity 90%). LCMS (ESI) m/z 236.07 & 308.07, (M+H)+.

Compound 127-3 (546 mg, 2.313 mmol) was dissolved in DCM (6 mL), cooled to 0°C, and DAST (620 mg, 2.775 mmol) was slowly added dropwise, and stirred at room temperature overnight. LCMS showed the generation of a product 127-4, and no remaining raw material 127-3. The reaction solution was quenched with saturated aqueous NaHCO3 solution in an ice bath, and extracted with DCM (30 mL × 3). The organic phases were combined and then subjected to rotary drying, and the crude was purified by silica gel column chromatography (eluent: petroleum ether:ethyl acetate=5:1) to obtain a product 127-4 as a white solid (220 mg, purity 92%). LCMS (ESI) m/z 258.05 & 260.05, (M+H)+.

At room temperature, Compound 127-4 (1.2 g, 4.65 mmol) was dissolved in dioxane (10 mL), and tri-n-butyl(1-ethoxyvinyl)tin (1.68 g, 4.65 mmol) was added. Then $Pd(PPh_3)_4$ (537 mg, 0.465 mmol) was added under nitrogen protection. The reaction solution was heated at 110°C overnight. LCMS showed no remaining raw materials. The reaction solution was cooled to room temperature, quenched with 30 mL of aqueous KF solution, and stirred for 0.5 hour. The generated solid was filtered through diatomite. The filtrate was extracted with ethyl acetate (40 mL × 3), and the organic phases were combined and then subjected to rotary drying. The crude was redissolved in EA (10 mL), and 2 mL of aqueous hydrochloric acid solution (3 M) was added, and stirred at room temperature for 2 hours. LCMS showed the generation of a product 127-5. The reaction solution was adjusted to neutral pH with aqueous $Na_2CO_3$ solution, and extracted with ethyl acetate. Then the organic phases were combined and subjected to rotary drying. The crude was purified by silica gel column chromatography (eluent: petroleum ether:ethyl acetate=1:1) to obtain a product 127-5 as a yellow oil (873 mg, purity 95%). LCMS (ESI) m/z 222.01, (M+H)+.

At room temperature, Compound 127-5 (873 mg, 3.94 mmol) was dissolved in DCM:MeOH=1:1 (10 mL), and $NaBH_4$ (298 mg, 7.89 mmol) was added at 0°C. The reaction solution was stirred at room temperature for 5 hours. LCMS showed the complete reaction of raw materials. The reaction solution was quenched with saturated aqueous $NH_4Cl$ solution, and extracted with DCM (50 mL × 3). The organic phases were combined and then subjected to rotary drying. The crude was purified by silica gel column chromatography (eluent: petroleum ether:ethyl acetate=5:1) to obtain a product Compound 127-6 as a brown oil (435 mg, purity 80% ). LCMS (ESI) m/z 224.08, (M+H)+.

Compound 127-6 (435 mg, 1.95 mmol) was dissolved in DCM (10 mL) at room temperature, and $SOCl_2$ (463 mg, 3.9 mmol) was added at 0°C. The reaction solution was stirred at room temperature for 4 hours. LCMS showed the complete reaction of raw materials. The reaction solution was directly subjected to rotary drying to obtain a crude product Compound 127-7 (513 mg).

At room temperature, Compound 127-7 (41 mg, 0.618 mmol) and Compound A4 (50 mg, 0.618 mmol) was dissolved in DMF (1 mL), and $Cs_2CO_3$ (220 mg, 0.67 mmol) was added, and stirred at room temperature overnight. The reaction solution was diluted with water and then extracted with EA (20 mL × 3). The organic phases were combined and then subjected to rotary drying. The crude was purified by silica gel column chromatography (eluent: DCM:MeOH=10:1) to obtain a product 127-8 as a brown solid (15 mg, purity 80%). LCMS (ESI) m/z 504.21, (M+H)+.

At room temperature, Compound 127-8 (15 mg) was dissolved in MeOH (1 mL), and HCl (1 mL, 4 M in dioxane) was added, and stirred at room temperature for 2 h. LCMS showed the complete reaction of raw materials. The reaction solution was subjected to rotary drying and then purified by preparative high performance liquid chromatography to obtain a product Compound 127 as a yellow solid (5.1 mg, purity 94.63%). LCMS (ESI) m/z 420.42, (M+H)+; $^1H$ NMR (400 MHz, DMSO) δ 12.76 (s, 1H), 9.37 (d, J = 8.6 Hz, 1H), 9.06 (d, J = 3.4 Hz, 1H), 8.13 - 7.94 (m, 3H), 7.91 (s, 1H), 7.77 (dd, J = 8.8, 4.0 Hz, 1H), 7.65 (s, 1H), 7.36 (d, J = 9.0 Hz, 1H), 7.18 (s, 1H), 7.09 (dd, J = 9.0, 2.0 Hz, 1H), 6.64 - 6.57 (m, 1H), 3.90 (s, 3H), 1.91 (d, J = 6.6 Hz, 3H).

The following compound was synthesized according to the the route of Example 127.

| Example | Name | Structure | LC-MS $(M+H)^+$ | $^1H$ NMR |
|---|---|---|---|---|
| 203 | 6-(difluoromethyl)-5-(1-((3-(1-methyl-1H-pyrazol-4-yl)-1H-pyrazino[4,3-b]pyridin-5-yl)oxy)ethyl)quinoline | 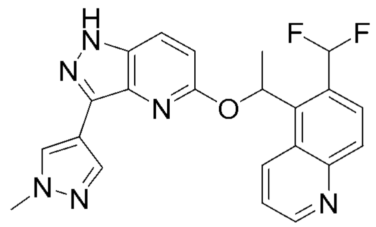 | 421.2 | $^1H$ NMR (400 MHz, DMSO) δ 12.98 (s, 1H), 9.29 (d, J = 8.0 Hz, 1H), 9.06 (d, J = 4.0 Hz, 1H), 8.11 - 7.90 (m, 4H), 7.83 (d, J = 7.8 Hz, 1H), 7.77 (dd, J = 8.7, 4.1 Hz, 1H), 7.66 (s, 1H), 7.24 (dd, J = 12.0, 6.0 Hz, 1H), 7.02 (d, J = 12.0 Hz, 1H), 3.85 (s, 3H), 1.91 (d, J = 8.0 Hz, 3H). |

Example 129

6-isopropyl-5-(1-((3-(1-methyl-1H-pyrazol-4-yl)-1H-indazol-5-yl)oxy)ethyl)quino xaline

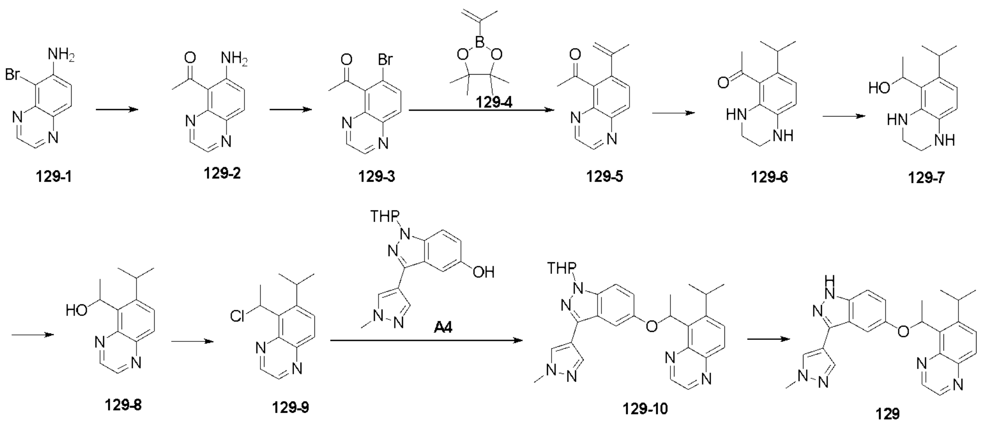


At room temperature, Compound 129-1 (3 g, 13.4 mmol, 1.0 eq.), tri-n-butyl(1-ethoxyvinyl)tin (7.25 g, 20.1 mmol, 1.5 eq.) and $Pd(PPh_3)_4$ (3.1 g, 2.68 mmol, 0.2 eq.) were added successively to dioxane (50.0 mL). The reaction mixture was stirred at 110°C for 48 hours under nitrogen protection. The reaction solution was cooled to room temperature, and saturated aqueous KF solution (30.0 mL) was added. The reaction solution was stirred at room temperature for 1 hour. The reaction solution was filtered, and the filtrate was extracted with ethyl acetate (50.0 mL x 2). The organic phases were combined, dried over anhydrous sodium sulfate, filtered, and concentrated under reduced pressure. The resulting residue was dissolved in THF (30.0 mL), and 3N HCl (20.0 mL) was slowly added. The reaction solution was stirred at room temperature for 40 minutes, saturated aqueous sodium bicarbonate solution was added to adjust the pH to >7, and then the reaction solution was extracted with ethyl acetate (50.0 mL × 2). The organic phases were combined, washed with saturated brine (20.0 mL), dried over anhydrous sodium sulfate, filtered, and concentrated under reduced pressure. The

resulting residue was purified by silica gel column chromatography (eluent: ethyl acetate/petroleum ether=0-43%) to obtain Compound 129-2 (983 mg, yield 39.2%).

At room temperature, t-BuONO (722 mg, purity 90%, 6.3 mmol, 1.2 eq.) was slowly added to a solution of Compound 129-2 (983 mg, 5.25 mmol, 1.0 eq.) and $CuBr_2$ (1.41 g, 6.3 mmol, 1.2 eq.) in $CH_3CN$ (45.0 mL). The reaction solution was stirred at room temperature for 16 hours. The reaction mixture was concentrated under reduced pressure, and the resulting residue was purified by silica gel column chromatography (eluent: ethyl acetate/petroleum ether=0-31 %) to obtain Compound 129-3 (970 mg, yield 73.6%).

At room temperature, Compound 129-3 (970 mg, 3.86 mmol, 1.0 eq.), Compound 129-4 (1.3 g, 7.72 mmol, 2.0 eq.), Pd(dppf)$Cl_2$ (560 mg, 0.77 mmol, 0.2 eq.) and potassium carbonate (1.6 g, 11.6 mmol, 3.0 eq.) were added successively to dioxane /$H_2O$ (30 mL/7.5 mL). The reaction mixture was stirred at 95°C for 16 hours. The reaction solution was cooled to room temperature, water (20.0 mL) was added, and then the reaction solution was extracted with ethyl acetate (30.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/petroleum ether=0~26%) to obtain Compound 129-5 (740 mg, yield 90.2%).

At room temperature, Compound 129-5 (150 mg, 0.707 mmol, 1.0 eq.) and palladium/carbon (palladium loading: 10 wt.%) (50 mg) were added successively to THF (10.0 mL). The reaction mixture was stirred at room temperature for 16 hours under a hydrogen atmosphere, filtered through diatomite, and the filtrate was concentrated under reduced pressure. The resulting residue 129-6 was directly used for the next reaction step without purification.

At 0°C, $NaBH_4$ (107 mg, 2.83 mmol, 4.0 eq.) was slowly added to a solution of Compound 129-6 (154 mg, 0.707 mmol, 1.0 eq.) in methanol (9.0 mL). The reaction mixture was stirred at room temperature for 2 hours. The reaction solution was cooled to 0°C, slowly poured into aqueous $NH_4Cl$ solution (20.0 mL), and extracted with EA (20.0 mL × 2). The organic phases were combined, washed with saturated brine (10.0 mL), dried over anhydrous sodium sulfate, filtered, and concentrated under reduced pressure. The resulting residue 129-7 was directly used for the next reaction step without purification.

At room temperature, Compound 129-7 (156 mg, 0.707 mmol, 1.0 eq.) and $MnO_2$ (615 mg, 7.07 mmol, 10.0 eq.) were added successively to DCM (6.0 mL). The reaction mixture was stirred at 40°C for 6 hours. The reaction mixture was concentrated under reduced pressure, and the resulting residue was purified by silica gel column chromatography (eluent: ethyl acetate/petroleum ether=0-23.5%) to obtain Compound 129-8 (71 mg, total yield 46.4% for three steps).

At 0°C, thionyl chloride (81mg, 0.68 mmol, 3.0 eq.) was slowly added to a solution of Compound 129-8 (49 mg, 0.227 mmol, 1.0 eq.) in DCM (3.0 mL). The reaction solution was warmed up to room temperature, and stirred for 1 hour. The reaction solution was concentrated under reduced pressure, and the resulting residue 129-9 was directly used for the next reaction step without purification.

At room temperature, Compound 129-9 (53 mg, 0.227 mmol, 1.0 eq.), Compound A4 (68 mg, 0.227 mmol, 1.0 eq.) and cesium carbonate (221mg, 0.68 mmol, 3.0 eq.) were added successively to DMF (2.0 mL). The reaction mixture was stirred at 60°C for 16 hours. The reaction solution was cooled to room temperature, water (10.0 mL) was added, and then the reaction solution was extracted with ethyl acetate (10.0 mL x 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-21%) to obtain Compound 129-10 (51 mg, purity 82%, total yield 37.1% for two steps).

At room temperature, TFA (1.0 mL) was slowly added to a solution of Compound 129-10 (51 mg, purity 82%, 0.106 mmol, 1.0 eq.) in DCM (3.0 mL). The reaction mixture was stirred at room temperature for 3 hours. The reaction mixture was concentrated under reduced pressure, and the resulting residue was dissolved in DCM (10.0 mL), aqueous sodium bicarbonate solution was added to adjust the pH to >7, and then the resulting residue was extracted with DCM (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-27%) to obtain Compound 129 (23 mg, yield 66.3%, purity 95.51%). LCMS (ESI) m/z =413.2 (M+H)+; $^1$H NMR (400 MHz, DMSO) δ 12.70 (s, 1H), 9.21 (d, J = 1.8 Hz, 1H), 9.09 (t, J = 9.1 Hz, 1H), 8.02 (d, J = 8.9 Hz, 1H), 7.94 - 7.83 (m, 1H), 7.64 (s, 1H), 7.37 (d, J = 9.0 Hz, 1H), 7.32 (t, J = 6.7 Hz, 1H), 7.28 (s, 1H), 7.05 (dd, J = 9.0, 2.2 Hz, 1H), 6.99 (s, 1H), 4.35 - 4.27 (m, 1H), 3.90 (s, 3H), 1.82 (d, J = 6.7 Hz, 3H), 1.34 (d, J = 6.9 Hz, 3H), 1.04 (d, J = 6.9 Hz, 3H).

The following compound was synthesized according to the the route of Example 129.

| Example | Name | Structure | LC-MS (M+H)+ | $^1H$ NMR |
|---|---|---|---|---|
| 112 | 6-methyl-5-(1-((3-(1-methyl-1H-pyrazol-4-yl)-1H-indazol-5-yl)oxy)ethyl)quinoxaline | 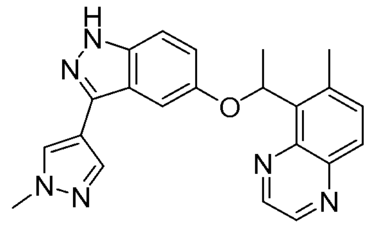 | 385.2 | $^1H$ NMR (400 MHz, DMSO) δ 12.74 (s, 1H), 9.20 (d, J = 1.8 Hz, 1H), 9.09 (d, J = 1.7 Hz, 1H), 7.95 (d, J = 8.6 Hz, 1H), 7.64 (t, J = 4.3 Hz, 2H), 7.38 (d, J = 9.0 Hz, 1H), 7.29 (s, 1H), 7.21 (q, J = 6.7 Hz, 1H), 7.08 (dd, J = 9.0, 2.2 Hz, 1H), 6.98 (s, 1H), 3.89 (s, 3H), 2.73 (s, 3H), 1.81 (d, J = 6.7 Hz, 3H). |

Example 130

6-(fluoromethyl)-5-(1-((3-(1-methyl-1H-pyrazol-4-yl)-1H-indazol-5-yl)oxy)ethyl)q uinoline

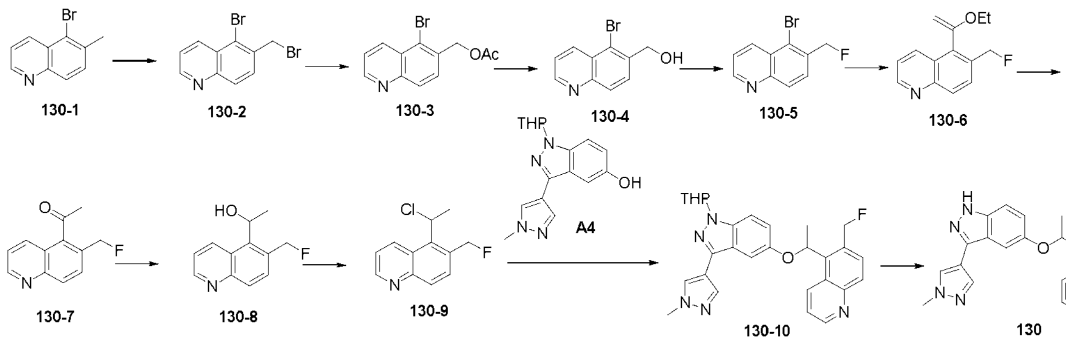


At room temperature, Compound 130-1 (1.004 g, 4.52 mmol), NBS (0.845 g, 4.75 mmol) and AIBN (0.111 g, 0.678 mmol) were added to carbon tetrachloride (23.0 mL). The resulting mixture was stirred at 80°C for 4 hours. LCMS showed the generation of a product. The reaction mixture was cooled to room temperature, and concentrated to dryness under reduced pressure. The resulting residue was purified by silica gel column chromatography (eluent: dichloromethane:petroleum ether=1.2:1) to obtain Compound 130-2 as a light yellow solid (1.126 g, yield: 83%, purity: 88%). LCMS (ESI) m/z 300.0, 301.9, 304.0, (M+H)+.

At room temperature, Compound 130-2 (1.005 g, 3.34 mmol) and anhydrous sodium acetate (0.685 g, 8.35 mmol) were added to N,N-dimethylformamide (8.3 mL). The resulting mixture was stirred at 80°C for 2 hours. LCMS showed the generation of a product. The reaction mixture was cooled to room temperature, and diluted with ethyl acetate (30.0 mL) and water (20.0 mL). The organic phase was separated, and the aqueous phase was extracted with ethyl acetate (20.0 mL × 3). The organic phases were combined, washed with water (20.0 mL x 2), dried over anhydrous sodium sulfate, and filtered. The filtrate was concentrated to dryness under reduced pressure. The resulting residue was purified by silica gel column chromatography (eluent: dichloromethane: petroleum ether=4:1) to obtain Compound 130-3 as a white solid (0.664 g, yield: 71%, purity: 95.52%). LCMS (ESI) m/z 280.0, 282.0 , (M+H)+.

At room temperature, Compound 130-3 (0.664 g, 2.37 mmol) was added to a mixture of tetrahydrofuran (8.0 mL) and water (8.0 mL), and then lithium hydroxide (0.114 g, 4.74 mmol) was added. The resulting mixture was stirred for 2 hours. LCMS showed the generation of a product. Aqueous HCl solution (3.0 M) was added to adjust the pH of the aqueous phase to about 7 to 8. The resulting mixture was extracted with a mixture of ethyl acetate and methanol (ethyl acetate :methanol=10:1, v/v) (20.0 mL × 5). The organic phases were combined, dried over anhydrous sodium sulfate, and filtered. The filtrate was concentrated to dryness under reduced pressure. The resulting residue was dried under vacuum to obtain a crude product Compound 130-4 as a white solid (0.603 g, crude yield: >100%, purity: 96.93%). LCMS (ESI) m/z 238.0, 240.0, (M+H)+.

At room temperature, the crude product Compound 130-4 (0.310 g, 1.30 (a theoretical amount)) was added to dichloromethane (11.0 mL). The resulting mixture was cooled in an ice water bath, and then DAST (0.454 g, 2.82 mmol) was slowly added dropwise. The resulting mixture was stirred at 0°C for 5 minutes, warmed up to room temperature and stirred for 13 hours. LCMS showed the generation of a product. The reaction mixture was diluted with dichloromethane

(30.0 mL), and saturated aqueous sodium bicarbonate solution was added to adjust the pH of the aqueous phase to about 7 to 8. The organic phase was separated, and the aqueous phase was extracted with dichloromethane (20.0 mL × 3). The organic phases were combined, dried over anhydrous sodium sulfate, and filtered. The filtrate was concentrated to dryness under reduced pressure. The resulting residue was purified by silica gel column chromatography (eluent: dichloromethane:petroleum ether=3:1) to obtain Compound 130-5 as a white solid (0.124 g, yield: 40%, purity: 97.84%). LCMS (ESI) m/z 240.0, 242.0, (M+H)+.

At room temperature, Compound 130-5 (0.124 g, 0.517 mmol), tri-n-butyl(1-ethoxyvinyl)tin (0.280 g, 0.775 mmol) and $Pd(PPh_3)_4$ (0.060 g, 0.0517 mmol) were added to 1,4-dioxane (2.6 mL). The resulting mixture was stirred at 120°C for 12 hours. LCMS showed the generation of a product. The reaction mixture was cooled to room temperature, and aqueous KF solution (1.0 M, 8.0 mL) was added. The resulting mixture was vigorously stirred for 30 minutes, and filtered through diatomite. The filter cake was washed successively with dichloromethane (5.0 mL x 3) and water (5.0 mL × 2). The filtrates were combined, the organic phase was separated, and the aqueous phase was extracted with dichloromethane (10.0 mL x 3). The organic phases were combined, washed successively with aqueous KF solution (1.0 M, 20.0 mL) and water (20.0 mL), dried over anhydrous sodium sulfate, and filtered. The filtrate was concentrated to dryness under reduced pressure. The resulting residue was dried under vacuum to obtain a crude product Compound 130-6 as a brown sticky liquid (0.294 g, purity: 51.3%). LCMS (ESI) m/z 232.0, (M+H)+.

At room temperature, aqueous HCl solution (3.0 M, 1.7 mL, 5.2 mmol) was slowly added dropwise to a solution of the crude product Compound 130-6 (0.294 g, 0.52 mmol (a theoretical amount)) in tetrahydrofuran (2.6 mL). The resulting mixture was stirred for 2 hours. LCMS showed the generation of a product. Saturated aqueous sodium bicarbonate solution was slowly added dropwise to the reaction mixture, and the pH of the aqueous phase was adjusted to about 7 to 8. The resulting mixture was extracted with dichloromethane (10.0 mL × 3). The organic phases were combined, dried over anhydrous sodium sulfate, and filtered. The filtrate was concentrated to dryness under reduced pressure. The resulting residue was purified by silica gel column chromatography (eluent: petroleum ether:ethyl acetate=4:1) to obtain Compound 130-7 as a yellow liquid (0.0766 g, total yield of the fifth and sixth steps: 38%, purity: 51.7%): LCMS (ESI) m/z 204.0, (M+H) +.

At room temperature, $NaBH_4$ (0.0285 g, 0.754 mmol) was added to a solution of Compound 130-7 (0.0766 g, 0.377 mmol) in methanol (1.9 mL). The resulting mixture was stirred for 3.5 hours. LCMS showed the generation of a product. The reaction mixture was diluted with saturated aqueous $NH_4Cl$ solution (5.0 mL), and the resulting mixture was extracted with ethyl acetate (5.0 mL × 5). The organic phases were combined, dried over anhydrous sodium sulfate, and filtered. The filtrate was concentrated to dryness under reduced pressure. The resulting residue was purified by silica gel thin-layer chromatography (eluent: petroleum ether: ethyl acetate=1.5:1) to obtain Compound 130-8 as a yellow liquid (0.0561 g, yield: 52%, purity: 71.48%): LCMS (ESI) m/z 206.1, (M+H)+.

At 0°C, thionyl chloride (0.0976 g, 0.82 mmol) was added dropwise to a solution of Compound 130-8 (0.0561 g, 0.273 mmol) in dichloromethane (2.7 mL). The resulting mixture was stirred at 0°C for 10 minutes, then warmed up to room temperature and stirred for 2 hours. LCMS showed the generation of a product. The reaction mixture was concentrated to dryness under reduced pressure. Dichloromethane (3.0 mL) was added to the resulting residue, and concentrated to dryness under reduced pressure again. This operation was repeated twice again, and the resulting residue was dried under vacuum to obtain a crude product Compound 130-9 as a yellow solid (0.0699 g, crude yield: 78%, purity: 79.24%). LCMS (ESI) m/z 223.8, 225.8, (M+H)+.

At room temperature, the crude product Compound 130-9 (0.0699 g, 0.273 mmol (a theoretical amount)) and cesium carbonate (0.445 g, 1.366 mmol) were added successively to N,N-dimethylformamide (2.7 mL). The resulting mixture was stirred at room temperature for 2 minutes, and then Compound A4 (0.0815 g, 0.273 mmol) was added. The reaction mixture was stirred at 50°C for 14 hours. LCMS showed the generation of a product. The reaction mixture was cooled to room temperature, diluted with ethyl acetate (20.0 mL), and then washed with water (10.0 mL × 2). The organic phase was dried over anhydrous sodium sulfate, and filtered. The filtrate was concentrated to dryness under reduced pressure. The resulting residue was purified by silica gel thin-layer chromatography (eluent: ethyl acetate :petroleum ether=6:1) to obtain a light brown jelly 130-10 (0.0485 g, yield: 31%, purity: 83.73%). LCMS (ESI) m/z 486.2, (M+H)+.

At room temperature, trifluoroacetic acid (0.745 g, 6.53 mmol) was added to a solution of Compound 130-10 (0.0485 g, 0.10 mmol) in dichloromethane (0.5 mL). The resulting mixture was stirred for 4 hours. LCMS showed the generation of a product. The reaction mixture was diluted with dichloromethane (10.0 mL), and saturated aqueous sodium bicarbonate solution was added to adjust the pH of the aqueous phase to about 7 to 8. The organic phase was separated, and the aqueous phase was extracted with dichloromethane (5.0 mL × 3). The organic phases were combined, dried over anhydrous sodium sulfate, and filtered. The filtrate was concentrated to dryness under reduced pressure. The resulting residue was purified by silica gel thin-layer chromatography (eluent: dichloromethane: methanol=20:1) to obtain a dark yellow solid. The solid was added to a mixture of tert-butyl methyl ether and ethyl acetate (tert-butyl methyl ether:ethyl acetate=10:1, v/v) (3.0 mL), warmed up to boiling, and then cooled to room temperature. The resulting mixture was filtered under reduced pressure. The filter cake was washed with a mixture of tert-butyl methyl ether and ethyl acetate (tert-butyl methyl ether:ethyl acetate=10:1, v/v) (0.5 × 2 mL), and dried under vacuum to obtain Compound 130 (0.0184 g, yield:

44%, purity: 96.76%). LCMS (ESI) m/z 402.0, (M+H)+; $^1$H NMR (400 MHz, DMSO) δ 12.75 (s, 1H), 9.31 (d, J = 8.6 Hz, 1H), 8.97 (d, J = 3.2 Hz, 1H), 8.00 (d, J = 8.4 Hz, 1H), 7.91 (s, 1H), 7.83 (d, J = 8.4 Hz, 1H), 7.72 - 7.63 (m, 2H), 7.35 (d, J = 8.8 Hz, 1H), 7.16 (s, 1H), 7.09 (d, J = 8.8 Hz, 1H), 6.47 (q, J = 6.4 Hz, 1H), 6.07 (dd, J = 47.2, 11.2 Hz, 1H), 5.84 (dd, J = 47.2, 11.2 Hz, 1H), 3.90 (s, 3H), 1.89 (d, J = 6.4 Hz, 3H).

Example 135

3-(1-methyl-1H-pyrazol-4-yl)-N-(1-(quinoxalin-5-yl) ethyl)-1H-indazole-5-amine

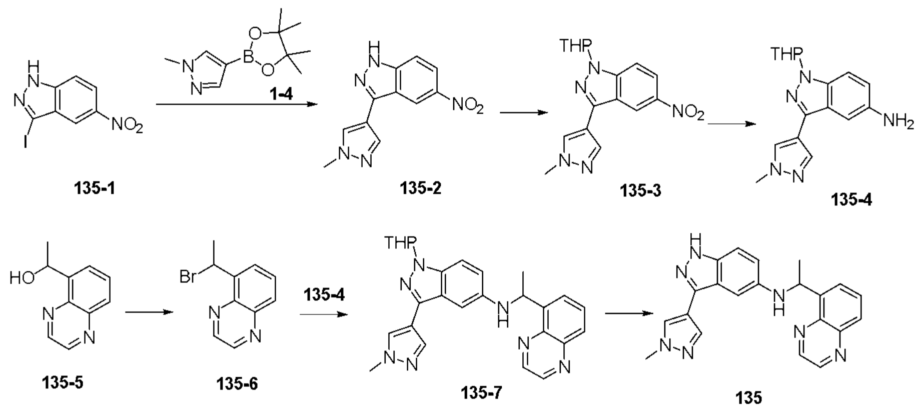


135-1 135-2 135-3 135-4

135-5 135-6 135-7 135

At room temperature, Compound 135-1 (100 mg, 1.0 mmol, 1.0 eq.), Compound 1-4 (323 mg, 1.6 mmol, 1.5 eq.), Pd(dppf)$Cl_2$ (151 mg, 0.21 mmol, 0.2 eq.) and potassium carbonate (430 mg, 3.1 mmol, 3.0 eq.) were added successively to dioxane /$H_2O$(4 mL/0.4 mL), purged with nitrogen three times, and stirred at 100°C for 12 hours. The reaction solution was poured into aqueous NaCl solution (10.0 mL), and extracted with EtOAc (10.0 mL × 2). The organic phases were combined, washed with saturated brine (10.0 mL), dried over anhydrous sodium sulfate, filtered, and concentrated under reduced pressure. The resulting residue was purified by thin-layer chromatography (developing solvent: EtOAc/PE = 4/1) to obtain Compound 135-2 (220 mg, yield 87.1%, a yellow solid). LCMS:(ESI) m/z 244.2, (M+H)+.

Compound 135-2 (220 mg, 0.90 mmol, 1.0 eq) and DHP (228 mg, 2.7 mmol, 3.0 eq) were dissolved in DCM (5.0 mL), and then p-toluenesulfonic acid monohydrate (17 mg, 0.091 mmol, 0.1 eq) was added, and stirred at room temperature for 16 hours. The reaction solution was poured into aqueous NaCl solution (10.0 mL), and extracted with EtOAc (20.0 mL x 2). The organic phases were combined, washed with saturated brine (20.0 mL), dried over anhydrous sodium sulfate, filtered, and concentrated under reduced pressure. The resulting residue was purified by thin-layer chromatography (developing solvent: EtOAc/PE = 1/1) to obtain Compound 135-3 (120 mg, yield 40.5%, a yellow solid). LCMS:(ESI) m/z 328.2, (M+H)+.

Compound 135-3 (120 mg, 0.37 mmol, 1.0 eq) was dissolved in EtOH (4.0 mL) and H2O (4.0 mL), and iron powder (102 mg, 1.8 mmol, 5.0 eq) and NH4Cl (192 mg, 3.7 mmol, 10.0 eq) were added, and stirred at 65°C for 2 hours. The reaction solution was filtered, and concentrated under reduced pressure to obtain Compound 135-4 (98 mg, yield 89.9%, a light yellow solid). LCMS:(ESI) m/z 298.2, (M+H)+.

At 0°C, a solution of $PBr_3$ (78 mg, 0.29 mmol, 1.0 eq.) in DCM(0.5 mL) was slowly added to a solution of Compound 135-5(50 mg, 0.29 mmol, 1.0 eq.) in DCM(3.0 mL), and stirred at room temperature for 0.5 hour. The reaction solution was concentrated to obtain Compound 135-6 (68 mg, 99.9%, a yellow oil). LCMS:(ESI) m/z 237.4, (M+H)+.

At room temperature, Compound 135-6 (41 mg, 0.17 mmol, 1.3 eq.), Compound 135-4(40 mg, 0.13 mmol, 1.0 eq.) and sodium bicarbonate (68 mg, 0.81 mmol, 6.0 eq.) were added successively to DMF (2.0 mL), and stirred at 60°C for 4 hours. The reaction solution was poured into aqueous NaCl solution (10.0 mL), and extracted with EtOAc (10.0 mL x 2). The organic phases were combined, washed with saturated brine (10.0 mL), dried over anhydrous sodium sulfate, filtered, and concentrated under reduced pressure. The resulting residue was purified by thin-layer chromatography (developing solvent: DCM/MeOH = 10/1) to obtain Compound 135-7 (42 mg, yield 68.8%, a light yellow solid). LCMS:(ESI) m/z 454.2, (M+H)+.

At room temperature, 4M hydrochloric acid in dioxane (2.0 mL) was slowly added to a mixed solution of Compound 135-7 (42 mg, 0.093 mmol) in DCM(2.0 mL) and MeOH (2.0 mL), and stirred at room temperature for 2 hours. The reaction solution was concentrated under reduced pressure, and the resulting residue was purified by preparative high performance liquid chromatography to obtain Compound 135 (2.6 mg, yield 7.6%, purity 100%, a light yellow solid). LCMS:(ESI) m/z 370.2, (M+H)+; $^1$H NMR (400 MHz, DMSO-d6) δ 12.45 (s, 1H), 9.16 (d, J = 1.8 Hz, 1H), 9.10 (d, J = 1.8 Hz,

1H), 8.03 - 7.88 (m, 2H), 7.81 (dd, J = 8.4, 7.2 Hz, 1H), 7.59 (s, 1H), 7.34 (s, 1H), 7.22 (d, J = 8.9 Hz, 1H), 6.91 (dd, J = 8.9, 2.0 Hz, 1H), 6.48 (d, J = 2.0 Hz, 1H), 6.35 (d, J = 7.4 Hz, 1H), 5.88 (p, J = 6.8 Hz, 1H), 3.83 (s, 3H), 1.59 (d, J = 6.7 Hz, 3H).

Example 138

7-ethyl-5-(1-((3-(1-methyl-1H-pyrazol-4-yl)-1H-indazol-5-yl)oxy)ethyl)quinoxalin e

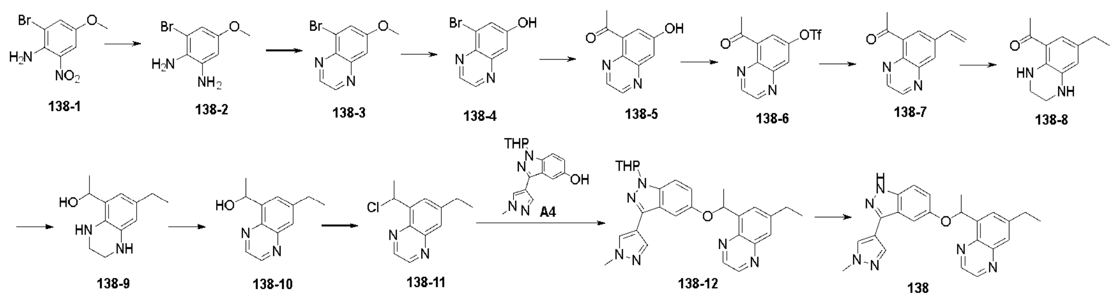

138-1 138-2 138-3 138-4 138-5 138-6 138-7 138-8
138-9 138-10 138-11 138-12 138

At room temperature, Compound 138-1 (1000 mg, 4.05 mmol, 1.0 eq.), iron powder (1133 mg, 20.23 mmol, 5 eq.) and ammonium chloride (2170 mg, 40.5 mmol, 10 eq.) were added successively to methanol/water (40 mL/10 mL). The reaction mixture was stirred at 65°C for 7 hours. The reaction solution was filtered through diatomite, the filtrate was concentrated under reduced pressure, water (20.0 mL) was added to the resulting residue, and then the resulting residue was extracted with dichloromethane (20.0 mL x 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/dichloromethane=0~13%) to obtain Compound 138-2 (770 mg, yield 87.6%).

At room temperature, Compound 138-2 (2975 mg, 13.7 mmol, 1.0 eq.) and glyoxal (40% content, 3977 mg, 27.4 mmol, 2.0 eq.) were added successively to ethanol (50 mL). The reaction mixture was stirred at room temperature for 3 hours. The reaction mixture was concentrated under reduced pressure, water (30.0 mL) was added to the resulting residue, and then the resulting residue was extracted with ethyl acetate (30.0 mL × 2). The organic phases were combined, washed with saturated brine (20.0 mL), dried over anhydrous sodium sulfate, filtered, and concentrated under reduced pressure. The resulting residue was purified by silica gel column chromatography (eluent: ethyl acetate/petroleum ether=0~30%) to obtain Compound 138-3 (1215 mg, yield 37.1%).

Compound 138-3 (570 mg, 2.38 mmol, 1.0 eq.) and pyridine hydrochloride (6.7 g) were stirred at 155°C for 4 hours. The reaction solution was cooled to room temperature, aqueous sodium bicarbonate solution was added to adjust the pH to >7, and then the reaction solution was extracted with dichloromethane (10.0 mL x 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/petroleum ether=0-44%) to obtain Compound 138-4 (100 mg, yield 18.6%).

At room temperature, Compound 138-4 (100 mg, 0.44 mmol, 1.0 eq.), tributyl(1-ethoxyvinyl)tin(241 mg, 0.66 mmol, 1.5 eq.) and $Pd(PPh_3)_4$ (103 mg, 0.088 mmol, 0.2 eq.) were added successively to dioxane (3.0 mL). The reaction mixture was stirred at 110°C for 16 hours under nitrogen protection. The reaction solution was cooled to room temperature, and aqueous KF solution (5.0 mL) was added. The reaction solution was stirred at room temperature for 1 hour. The reaction solution was filtered, and the filtrate was extracted with ethyl acetate (10.0 mL × 2). The organic phases were combined, dried over anhydrous sodium sulfate, filtered, and concentrated under reduced pressure. The resulting residue was dissolved in THF (5.0 mL), and 3N HCl (2.0 mL) was slowly added. The reaction solution was stirred at room temperature for 40 minutes, saturated aqueous sodium bicarbonate solution was added to adjust the pH to >7, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-18%) to obtain Compound 138-5 (30 mg, yield 35.9%).

At 0°C, trifluoromethanesulfonic anhydride (68 mg, 0.24 mmol, 1.5 eq.) was slowly added to a solution of Compound 138-5 (30 mg, 0.16 mmol, 1.0 eq.) and pyridine (19 mg, 0.24 mmol, 1.5 eq.) in DCM (1.6 mL). The reaction solution was stirred at room temperature for 2 hours. Aqueous sodium bicarbonate solution was added to the reaction solution to adjust the pH to >7, and then the reaction solution was extracted with dichloromethane (5.0 mL × 2). The organic phases were combined, washed with saturated brine (5.0 mL), dried over anhydrous sodium sulfate, filtered, and concentrated under reduced pressure. The resulting residue was purified by thin-layer chromatography (developping

agent: ethyl acetate/petroleum ether=2:3) to obtain Compound 138-6 (35 mg, yield 68.6%).

At room temperature, Compound 138-6 (35 mg, 0.11 mmol, 1.0 eq.), potassium vinyltrifluoroborate (23.5 mg, 0.17 mmol, 1.6 eq.), Pd(dppf)$C1_2$ (16 mg, 0.022 mmol, 0.2 eq.) and potassium acetate (33 mg, 0.33 mmol, 3.0 eq.) were added successively to dioxane /$H_2O$ (2 mL/0.5 mL). The reaction mixture was stirred at 90°C for 12 hours. The reaction solution was cooled to room temperature, and concentrated under reduced pressure. The resulting residue was purified by silica gel column chromatography (eluent: ethyl acetate/petroleum ether=0-20%) to obtain Compound 138-7 (20 mg, yield 92.3%).

At room temperature, Compound 138-7 (20 mg, 0.1 mmol, 1.0 eq.) and palladium/carbon (palladium loading: 10 wt.%) (20 mg) were added successively to THF (5.0 mL). The reaction mixture was stirred at room temperature for 5 hours under a hydrogen atmosphere, filtered through diatomite, and the filtrate was concentrated under reduced pressure. The resulting residue 138-8 was directly used for the next reaction step without purification.

At 0°C, $NaBH_4$ (19 mg, 0.5 mmol, 5.0 eq.) was slowly added to a solution of Compound 138-8 (21 mg, 0.1 mmol, 1.0 eq.) in methanol (3.0 mL). The reaction mixture was stirred at room temperature for 2 hours. The reaction solution was cooled to 0°C, slowly poured into $NH_4Cl$ aqueous solution (5.0 mL), and extracted with EA (10.0 mL × 2). The organic phases were combined, washed with saturated brine (10.0 mL), dried over anhydrous sodium sulfate, filtered, and concentrated under reduced pressure. The resulting residue 138-9 was directly used for the next reaction step without purification.

At room temperature, Compound 138-9 (21 mg, 0.1 mmol, 1.0 eq.) and MnO2 (88 mg, 1.0 mmol, 10.0 eq.) were added successively to DCM (2.0 mL). The reaction mixture was stirred at 40°C for 6 hours. The reaction mixture was concentrated under reduced pressure, and the resulting residue was purified by thin-layer chromatography (developping agent: ethyl acetate/petroleum ether=1:2) to obtain Compound 138-10 (6 mg, total yield 29.4% for three steps).

At 0°C, thionyl chloride (11 mg, 0.09 mmol, 3.0 eq.) was slowly added to a solution of Compound 138-10 (6 mg, 0.03 mmol, 1.0 eq.) in DCM (1.5 mL). The reaction solution was warmed up to room temperature, and stirred for 1 hour. The reaction solution was concentrated under reduced pressure, and the resulting residue 138-11 was directly used for the next reaction step without purification.

At room temperature, Compound 138-11 (6.7 mg, 0.03 mmol, 1.0 eq.), Compound A4 (8 mg, 0.027 mmol, 0.9 eq.) and cesium carbonate (29 mg, 0.09 mmol, 3.0 eq.) were added successively to DMF (1.0 mL). The reaction mixture was stirred at 60°C for 3 hours. The reaction solution was cooled to room temperature, water (5.0 mL) was added, and then the reaction solution was extracted with ethyl acetate (10.0 mL x 2). The organic phases were combined, washed with saturated brine (10.0 mL), dried over anhydrous sodium sulfate, filtered, and concentrated under reduced pressure. The resulting residue was purified by thin-layer chromatography (developping agent: dichloromethane/methanol=12:1) to obtain Compound 138-12 (7.5 mg, total yield 52.4% for two steps).

At room temperature, TFA (1.0 mL) was slowly added to a solution of Compound 138-12 (7.5 mg, 0.016 mmol, 1.0 eq) in DCM (3.0 mL). The reaction mixture was stirred at room temperature for 3 hours. The reaction mixture was concentrated under reduced pressure, and the resulting residue was dissolved in DCM (5.0 mL), aqueous sodium bicarbonate solution was added to adjust the pH to >7, and then the resulting residue was extracted with DCM (5.0 mL × 2). The organic phases were combined, washed with saturated brine (5.0 mL), dried over anhydrous sodium sulfate, filtered, and concentrated under reduced pressure. The resulting residue was purified by thin-layer chromatography (developping agent: dichloromethane/methanol=12:1) to obtain Compound 138 (5.5 mg, yield 89%, purity 97.04%). LCMS (ESI) m/z =399.2 (M+H)+; $^1$H NMR (400 MHz, DMSO) δ 12.70 (s, 1H), 9.09 - 9.01 (m, 2H), 7.96 - 7.88 (m, 2H), 7.83 (s, 1H), 7.58 (s, 1H), 7.39 (d, J = 9.0 Hz, 1H), 7.23 (s, 1H), 7.12 (dd, J = 9.0, 2.1 Hz, 1H), 6.75 - 6.63 (m, 1H), 3.90 (s, 3H), 2.84 (q, J = 7.5 Hz, 2H), 1.75 (d, J = 6.3 Hz, 3H), 1.23 (t, J = 7.5 Hz, 3H).

Example 143

7-bromo-5-(1-((3-(1-methyl-1H-pyrazol-4-yl)-1H-indazol-5-yl)oxy)ethyl)quinoxali ne

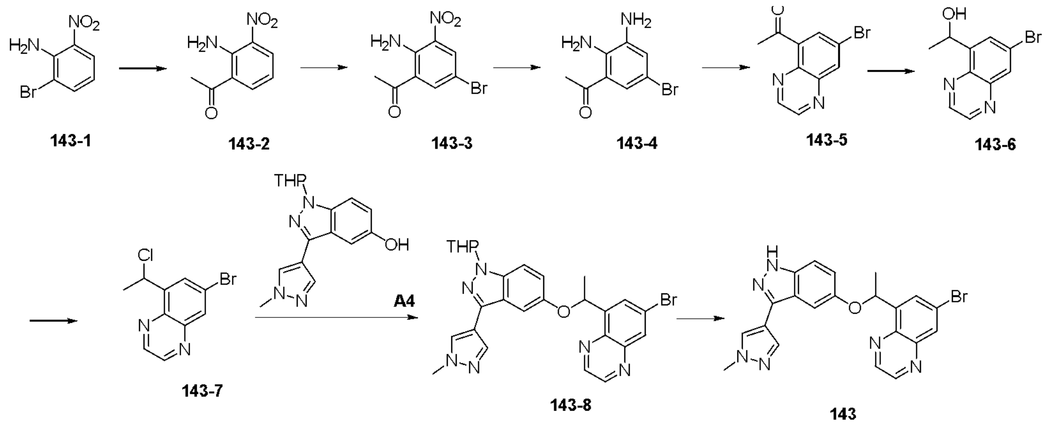


143-1 143-2 143-3 143-4 143-5 143-6

143-7 143-8 143

The compound tri-n-butyl(1-ethoxyvinyl)tin (3.3 g, 9.2 mmol, 1.0 eq) and $Pd(PPh_3)_4$ (286 mg, 1.8 mmol, 0.2 eq) were added successively to a solution of Compound 143-1 (2.0 g, 9.2 mmol, 1.0 eq) in dioxane (25.0 ml), purged with nitrogen three times, and stirred at 130°C for 16 hours. Diluted hydrochloric acid (3M, 60.0 mL) was added to the reaction solution, stirred for 1 hour, and then saturated aqueous potassium fluoride solution (60.0 mL) was added, and stirred for 1 hour. The mixed solution was filtered through diatomite, and the filtrate was poured into aqueous NaCl solution (15.0 mL), and extracted with EtOAc (60.0 mL x 2). The organic phases were combined, washed with saturated brine (60.0 mL), dried over anhydrous sodium sulfate, filtered, and concentrated under reduced pressure. The resulting residue was purified by silica gel column chromatography (EtOAc/PE: 10% ~100%) to obtain Compound 143-2 (1.4 g, yield 84.3%, a yellow solid). LCMS:(ESI) m/z 181.2, (M+H)+.

Compound 143-2 (1.0 g, 5.8 mmol, 1.0 eq) and NBS (1.2 g, 6.9 mmol, 1.2 eq) were added successively to HAOc (15.0 ml), and stirred at room temperature for 1 hour. The reaction solution was concentrated, then poured into aqueous NaHO3 solution (20.0 mL), and extracted with EtOAc (20.0 mL × 2). The organic phases were combined, washed with saturated brine (20.0 mL), dried over anhydrous sodium sulfate, filtered, and concentrated under reduced pressure to obtain a crude Compound 143-3 (2.1 g, a yellow solid). LCMS:(ESI) m/z 261.2, (M+H)+.

Compound 143-3 (2.1 g, 7.9 mmol, 1.0 eq) was dissolved in EtOH (30.0 mL) and $H_2O$ (15.0 mL), and iron powder (1.8 g, 31.7 mmol, 4.0 eq) and $NH_4Cl$ (2.1 g, 39.6 mmol, 5.0 eq) were added, and stirred at 80°C for 4 hours. The reaction solution was filtered, and concentrated under reduced pressure to obtain Compound 143-4 (1.4 g, yield 77.2%, a yellow solid). LCMS:(ESI) m/z 231.2, (M+H)+.

Compound 143-4 (1.4 g, 8.3 mmol, 1.0 eq) and an aqueous solution of 40% glyoxal (1.2 g, 8.3 mmol, 1.0 eq) were added to EtOH (25.0 mL), and stirred at 80°C for 1 hour. The reaction solution was concentrated, then poured into aqueous NaCl solution (20.0 mL), and extracted with EtOAc (20.0 mL x 2). The organic phases were combined, washed with saturated brine (20.0 mL), dried over anhydrous sodium sulfate, filtered, and concentrated under reduced pressure. The resulting residue was was purified by silica gel column chromatography (EtOAc/PE = 1/5) to obtain Compound 143-5(565 mg, total yield 27.1% for three steps, a light yellow solid). LCMS:(ESI) m/z 253.0, (M+H)+.

Compound 143-5 (565 mg, 2.3 mmol, 1.0 eq) was dissolved in MeOH (5.0 mL) at room temperature, and $NaBH_4$ (43 mg, 1.1 mmol, 0.5 eq) was added at 0°C, and stirred at room temperature for 1 hour. The reaction solution was concentrated under reduced pressure to obtain Compound 143-6 (580 mg, yield >100%, a light yellow solid). LCMS:(ESI) m/z 255.0, (M+H)+.

At 0°C, a solution of $SOCl_2$ (122 mg, 1.0 mmol, 2.0 eq.) in DCM(0.5 mL) was slowly added to a solution of Compound 143-6 (130 mg, 0.51 mmol, 1.0 eq.) in DCM(2.0 mL), and stirred at room temperature for 2 hours. The reaction solution was concentrated to obtain Compound 143-7 (140 mg, 100%, a brown solid). LCMS:(ESI) m/z 273.0, (M+H)+.

At room temperature, Compound 143-7(136 mg, 0.50 mmol, 1.5 eq.), Compound A4(100 mg, 0.34 mmol, 1.0 eq.) and cesium carbonate (437 mg, 1.3 mmol, 4.0 eq.) were added successively to DMF (2.0 mL), and stirred at 60°C for 2 hours. The reaction solution was poured into aqueous NaCl solution (20.0 mL), and extracted with EtOAc (20.0 mL x 2). The organic phases were combined, washed with saturated brine (20.0 mL), dried over anhydrous sodium sulfate, filtered, and concentrated under reduced pressure. The resulting residue was purified by thin-layer chromatography (developing solvent:DCM/MeOH = 10/1) to obtain Compound 143-8 (170 mg, yield 95.1%, a light yellow solid). LCMS:(ESI) m/z 535.2, (M+H)+.

At room temperature, 4M hydrochloric acid in dioxane (2.0 mL) was slowly added to a mixed solution of Compound 143-8 (30 mg, 0.056 mmol) in DCM(1.0 mL) and MeOH (1.0 mL), and stirred at room temperature for 1 hour. The reaction solution was concentrated and then purified by silica gel column chromatography (DCM/MeOH = 10/1) to obtain Compound 143 (10 mg, yield 39.6%, purity 100%, a light yellow solid).

LCMS:(ESI) m/z 499.1, (M+H)+; $^1H$ NMR (400 MHz, DMSO-d6) δ 12.79 (s, 1H), 9.17 (d, J = 1.8 Hz, 1H), 9.13 (d, J

= 1.8 Hz, 1H), 8.31 (d, J = 2.2 Hz, 1H), 8.08 (d, J = 2.2 Hz, 1H), 8.00 (s, 1H), 7.61 (s, 1H), 7.42 (d, J = 9.0 Hz, 1H), 7.24 (d, J = 2.3 Hz, 1H), 7.13 (dd, J = 8.9, 2.3 Hz, 1H), 6.69 (q, J = 6.3 Hz, 1H), 3.91 (s, 3H), 1.75 (d, J = 6.3 Hz, 3H).

Example 144

7 -isopropyl-5-(1-((3-(1-methyl-1H-pyrazol-4-y1)-1H-indazol-5-yl)oxy)ethyl)quino xaline

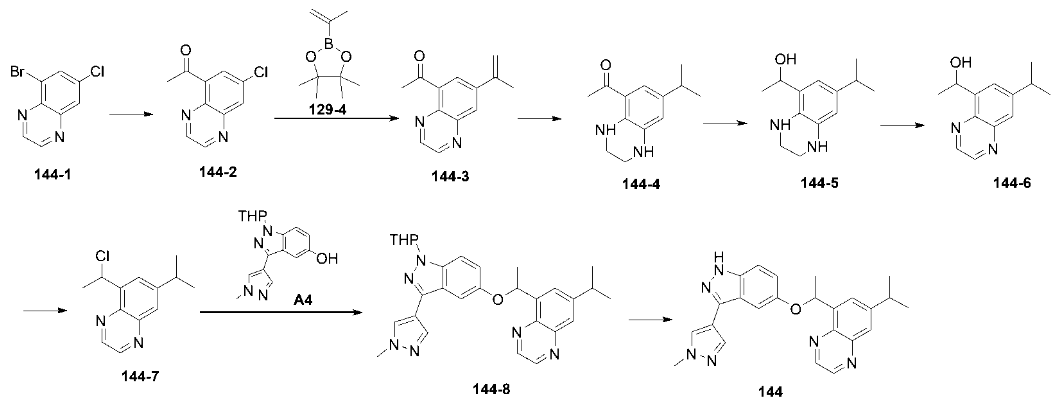


At room temperature, Compound 144-1 (1040 mg, 4.27 mmol, 1.0 eq.), tributyl(1-ethoxyvinyl)tin(1851 mg, 5.13 mmol, 1.2 eq.) and $Pd(PPh_3)_4$ (986 mg, 0.85 mmol, 0.2 eq.) were added successively to dioxane (15.0 mL). The reaction mixture was stirred at 100°C for 48 hours under nitrogen protection. The reaction solution was cooled to room temperature, and aqueous KF solution (15.0 mL) was added. The reaction solution was stirred at room temperature for 1 hour. The reaction solution was filtered, and the filtrate was extracted with ethyl acetate (20.0 mL × 2). The organic phases were combined, dried over anhydrous sodium sulfate, filtered, and concentrated under reduced pressure. The resulting residue was dissolved in THF (10.0 mL), and 3N HCl (5.0 mL) was slowly added. The reaction solution was stirred at room temperature for 40 minutes, saturated aqueous sodium bicarbonate solution was added to adjust the pH to >7, and then the reaction solution was extracted with ethyl acetate (20.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: ethyl acetate/ (dichloromethane/petroleum ether=1:8) =0-16%) to obtain Compound 144-2 (683 mg, yield 77.4%).

At room temperature, Compound 144-2 (90 mg, 0.435 mmol, 1.0 eq.), 129-4 (147 mg, 0.87 mmol, 2.0 eq.), $Pd(dppf)Cl_2$ (63 mg, 0.087 mmol, 0.2 eq.) and potassium carbonate (180 mg, 1.3 mmol, 3.0 eq.) were added successively to1,4-dioxane /$H_2O$ (4 mL/1 mL). The reaction mixture was stirred at 105°C for 16 hours. The reaction solution was cooled to room temperature, and concentrated under reduced pressure. The resulting residue was purified by silica gel column chromatography (eluent: ethyl acetate/petroleum ether=0-20%) to obtain Compound 144-3 (85 mg, yield 91.9%).

At room temperature, Compound 144-3 (85 mg, 0.4 mmol, 1.0 eq.) and palladium/carbon (palladium loading: 10 wt.%) (30 mg) were added successively to THF (6.0 mL). The reaction mixture was stirred at room temperature for 16 hours under a hydrogen atmosphere, filtered through diatomite, and the filtrate was concentrated under reduced pressure. The resulting residue Compound 144-4 was directly used for the next reaction step without purification.

At 0°C, $NaBH_4$ (53 mg, 1.4 mmol, 3.5 eq.) was slowly added to a solution of Compound 144-4 (90 mg, 0.4 mmol, 1.0 eq.) in methanol (5.0 mL). The reaction mixture was stirred at room temperature for 2 hours. The reaction solution was cooled to 0°C, slowly poured into $NH_4Cl$ aqueous solution (10.0 mL), and extracted with EA (10.0 mL × 2). The organic phases were combined, washed with saturated brine (10.0 mL), dried over anhydrous sodium sulfate, filtered, and concentrated under reduced pressure. The resulting residue 144-5 was directly used for the next reaction step without purification.

At room temperature, Compound 144-5 (88 mg, 0.4 mmol, 1.0 eq.) and MnO2 (350 mg, 4.0 mmol, 10.0 eq.) were added successively to DCM (5.0 mL). The reaction mixture was stirred at 40°C for 6 hours. The reaction mixture was concentrated under reduced pressure, and the resulting residue was purified by silica gel column chromatography (eluent: ethyl acetate/petroleum ether=0-27%) to obtain Compound 144-6 (50 mg, total yield 57.7% for three steps).

At 0°C, thionyl chloride (84 mg, 0.7 mmol, 3.0 eq.) was slowly added to a solution of Compound 144-6 (50 mg, 0.236 mmol, 1.0 eq.) in DCM (3 mL). The reaction solution was warmed up to room temperature, and stirred for 1 hour. The reaction solution was concentrated under reduced pressure, and the resulting residue 144-7 was directly used for the next reaction step without purification.

At room temperature, Compound 144-7 (55 mg, 0.236 mmol, 1.0 eq.), Compound A4 (49 mg, 0.164 mmol, 0.7 eq.) and cesium carbonate (230 mg, 0.7 mmol, 3.0 eq.) were added successively to DMF (3.0 mL). The reaction mixture was stirred at 60°C for 8 hours. The reaction solution was cooled to room temperature, water (10.0 mL) was added, and then the reaction solution was extracted with ethyl acetate (10.0 mL x 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-16%) to obtain Compound 144-8 (83 mg, total yield 72.3% for two steps).

At room temperature, TFA (2.0 mL) was slowly added to a solution of Compound 144-8 (83 mg, 0.167 mmol, 1.0 eq) in DCM (6.0 mL). The reaction mixture was stirred at room temperature for 3 hours. The reaction mixture was concentrated under reduced pressure, and the resulting residue was dissolved in DCM (10.0 mL), aqueous sodium bicarbonate solution was added to adjust the pH to >7, and extracted with DCM (10.0 mL x 2). The organic phases were combined, washed with saturated brine (10.0 mL), dried over anhydrous sodium sulfate, filtered, and concentrated under reduced pressure. The resulting residue was purified by thin-layer chromatography (developping agent: dichloromethane/methanol=12:1) to obtain Compound 144 (39 mg, yield 56.6%, purity 100%). LCMS (ESI) m/z =413.3 (M+H)+; $^1$H NMR (400 MHz, DMSO)δ 12.74 (s, 1H), 9.04 (d, J = 4.4 Hz, 2H), 8.03 - 7.92 (m, 2H), 7.83 (d, J = 1.4 Hz, 1H), 7.62 (s, 1H), 7.38 (d, J = 9.0 Hz, 1H), 7.27 (s, 1H), 7.13 (dd, J = 9.0, 1.9 Hz, 1H), 6.70 (q, J = 6.3 Hz, 1H), 3.91 (s, 3H), 3.13 (dq, J = 13.4, 6.9 Hz, 1H), 1.76 (d, J = 6.3 Hz, 3H), 1.26 (d, J = 6.9 Hz, 6H).

The following compound was synthesized according to the the route of Example 144.

| Example | Name | Structure | LC-MS (M+H)+ | $^1$H NMR |
|---|---|---|---|---|
| 237 | 7-isopropyl-5-(1-((3-(1-methyl-1H-pyrazol-4-yl)-1H-indazol-5-yl)oxy)ethyl)pyridi no[3,4-b]pyrazine | 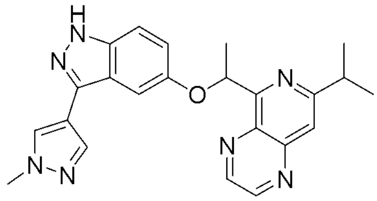 | 414.2 | $^1$HNMR(400 MHz, DMSO) δ 12.70 (s, 1H), 9.18 (d,J = 1.7 Hz, 1H), 9.10 (d,J = 1.8 Hz, 1H), 8.03 (s, 1H), 7.74 (s, 1H), 7.63 (s, 1H), 7.32 (dd,J = 14.9, 5.5 Hz, 2H), 7.09 (dd,J = 9.0, 2.2 Hz, 1H), 6.83 (q,J = 6.4 Hz, 1H), 3.92 (d,J = 10.3 Hz, 3H), 3.21 (dd,J = 13.7, 6.9 Hz, 1H), 1.80 (d,J = 6.4 Hz, 3H), 1.28 (dd,J = 6.9, 2.4 Hz, 6H). |

Example 146

7-ethynyl-5-(1-((3-(1-methyl-1H-pyrazol-4-yl)-1H-indazol-5-yl)oxy)ethyl)quinoxa line

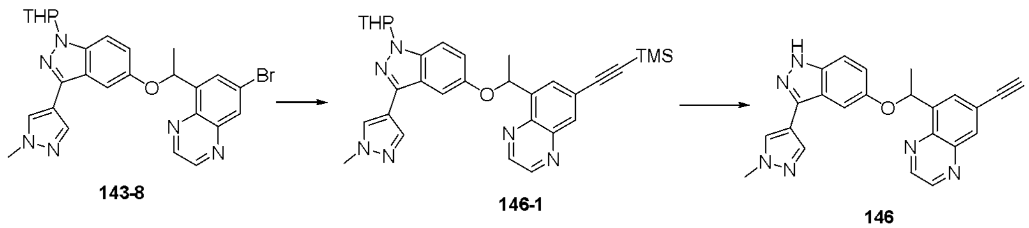

143-8 146-1 146

At room temperature, Compound 143-8(70 mg, 0.13 mmol, 1.0 eq.), trimethylsilylacetylene (49 mg, 0.50 mmol, 3.8 eq.), Pd(dppf)$Cl_2$ (18 mg, 0.026 mmol, 0.2 eq.), CuI (10 mg, 0.053 mmol, 0.4 eq.) and $Et_3N$ (85 mg, 0.84 mmol, 6.4 eq.) were added successively to THF (2.0 mL), purged with nitrogen three times, and stirred at 90°C for 4 hours. The reaction solution was poured into aqueous NaCl solution (10.0 mL), and extracted with EtOAc (20.0 mL × 2). The organic phases were combined, washed with saturated brine (20.0 mL), dried over anhydrous sodium sulfate, filtered, and concentrated under reduced pressure. The resulting residue was purified by thin-layer chromatography (developing solvent:DCM/-MeOH = 10/1) to obtain Compound 146-1 (44 mg, yield 60.9%, a yellow solid). LCMS:(ESI) m/z 511.3, (M+H)+.

At room temperature, TFA (1.5 mL) was added to Compound 146-1 (44 mg, 0.071 mmol) in DCM (1.5 mL), and stirred at 40°C for 4 hours. Aqueous $NaHCO_3$ solution (10.0 mL) was poured into the reaction solution to adjust the pH to 8,

and then the reaction solution was extracted with EtOAc (10.0 mL × 2). The organic phases were combined, washed with saturated brine (10.0 mL), dried over anhydrous sodium sulfate, filtered, and concentrated under reduced pressure. The resulting residue was purified by thin-layer chromatography (developing solvent:DCM/MeOH = 10/1) to obtain Compound 146 (9.6 mg, yield 34.4%, purity 100%, a light yellow solid). LCMS:(ESI) m/z 395.2, (M+H)+; $^1$HNMR (400 MHz, DMSO-d6) δ 12.77 (s, 1H), 9.14 (q, J = 1.9 Hz, 2H), 8.15 (d, J = 1.8 Hz, 1H), 7.99 - 7.94 (m, 3H), 7.58 (s, 1H), 7.41 (d, J = 9.0 Hz, 1H), 7.21 (d, J = 2.3 Hz, 1H), 7.12 (dd, J = 9.0, 2.3 Hz, 1H), 6.68 (q, J = 6.3 Hz, 1H), 4.53 (s, 1H), 3.90 (s, 3H), 1.75 (d, J = 6.3 Hz, 3H).

Example 149

4-(5-(5-(1-(quinoxalin-5-yl)ethoxy)-1H-indazol-3-yl)pyridin-2-yl)thiomorpholine1,1-dioxide

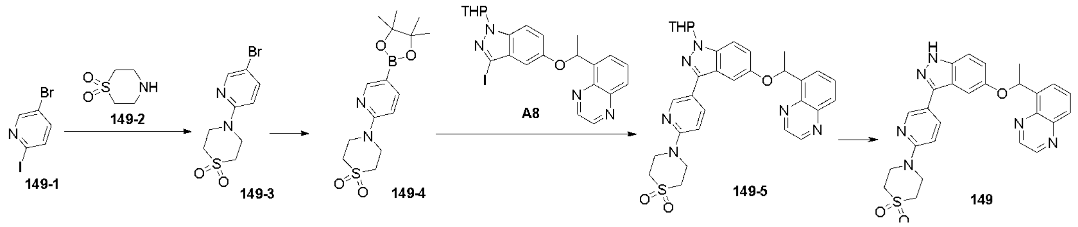


Compound 149-1 (270 mg, 0.96 mmol, 1.3 eq.) was dissolved in toluene (10 ml), and then Compound 149-2 (100 mg, 0.74 mmol, 1.0 eq), t-BuoNa (106 mg, 1.1 mmol, 1.5 eq), 4,5-bis(diphenylphosphino)-9,9-dimethyl-xanthene (25.5 mg, 0.04 mmol, 0.06 eq) and $Pd_2(dba)_3$ (13.9 mg, 0.15 mmol, 0.02 eq) were added respectively. After the addition was completed, the reaction solution was purged with nitrogen three times, and reacted under stirring at 98°C overnight. TLC showed no remaining raw materials, and the conversion of most of raw materials into a product. Saturated ammonium chloride solution was added dropwise to quench the reaction. The reaction solution was extracted with (20 ml) ethyl acetate three times. The organic phases were combined, washed with saturated brine, dried over anhydrous sodium sulfate, filtered, concentrated, and purified by thin-layer chromatography (developing solvent: DCM: MeOH= 20:1) to obtain Compound 149-3 as a yellow solid (110 mg, 37%). LCMS:(ESI) m/z 292, (M+H)+; $^1$H NMR (400 MHz, $CD_3OD$) δ 8.19 (s, 1H), 7.67 (d, J = 9.1 Hz, 1H), 6.91 (d, J = 9.1 Hz, 1H), 4.11 (d, J = 10.0 Hz, 4H), 3.09 - 3.02 (m, 4H).

Compound 149-3 (100 mg, 0.34 mmol, 1.0 eq.) was dissolved in dioxane (5 ml), and then bis(pinacolato)diboron (132 mg, 0.52 mmol, 1.5 eq.), KOAc (100.9 mg, 1.0 mmol, 3.0 eq) and $Pd(dppf)Cl_2$ (25 mg, 0.034 mmol, 0.1 eq) were added respectively. After the addition was completed, the reaction solution was purged with nitrogen three times, and reacted under stirring at 110°C overnight. TLC showed no remaining raw materials, and the conversion of most of raw materials into a product. Saturated ammonium chloride solution was added dropwise to quench the reaction. The reaction solution was extracted with (10 ml) ethyl acetate three times. The organic phases were combined, washed with saturated brine, dried over anhydrous sodium sulfate, filtered, concentrated, and purified by thin-layer chromatography (developing solvent: DCM: MeOH=20:1) to obtain Compound 149-4 as a yellow solid (60 mg, 51%). LCMS:(ESI) m/z 339, (M+H)+; $^1$H NMR (400 MHz, $CD_3OD$) δ 8.44 (s, 1H), 7.87 (s, 1H), 6.96 - 6.90 (m, 1H), 4.17 (s, 4H), 3.06 (s, 4H), 1.31 (s, 12H).

Compound 149-4 (60 mg, 0.2 mmol, 1.3 eq) was dissolved in THF/$H_2O$ (5/1 ml), and Compound A8 (75 mg, 0.15 mmol, 1.0 eq.), the compound $K_2CO_3$ (61 mg, 0.44 mmol, 3.0 eq) and $Pd[PPh_3]_4$ (17.3 mg, 0.02 mmol, 0.1 eq) were added respectively. After the addition was completed, the reaction solution was purged with nitrogen three times, and reacted under stirring at 80°C overnight. TLC showed no remaining raw materials, and the conversion of most of raw materials into a product. Saturated ammonium chloride solution was added dropwise to quench the reaction. The reaction solution was extracted with (10 ml) ethyl acetate three times. The organic phases were combined, washed with saturated brine, dried over anhydrous sodium sulfate, filtered, concentrated, and purified by thin-layer chromatography (developing solvent: DCM: MeOH= 20:1) to obtain Compound 149-5 as a yellow solid (60 mg, 86%). LCMS:(ESI) m/z 585, (M+H)+.

Compound 149-5 (80 mg, 0.17 mmol, 1.0 eq.) was dissolved in MeOH (5 ml), and then a solution of HCl in dioxane (2.0 ml) was added. After the addition was completed, the reaction solution was purged with nitrogen three times, and reacted under stirring at 25°C for two hours. TLC showed no remaining raw materials, and the conversion of most of raw materials into a product. Saturated ammonium chloride solution was added dropwise to quench the reaction. The reaction solution was extracted with (10 ml) ethyl acetate three times. The organic phases were combined, washed with saturated brine, dried over anhydrous sodium sulfate, filtered, concentrated, and purified by thin-layer chromatography (developing solvent:DCM:MeOH=20:1) to obtain Compound 149 as a yellow solid (19.3 mg, purity :98%, yield: 38%). LCMS:(ESI) m/z 501, (M+H)+; $^1$H NMR (399 MHz, $CD_3OD$) δ 9.06 (s, 1H), 8.96 (s, 1H), 8.12 (s, 1H), 7.98 (s, 2H), 7.78 (d, J = 13.4 Hz, 2H), 7.39 (s, 1H), 7.17 (d, J = 2.3 Hz, 1H), 7.08 (s, 1H), 6.93 (s, 1H), 6.74 (s, 1H), 4.22 (s, 4H), 3.15 (s, 4H), 1.76 (d, J = 6.4 Hz,

3H).

Example 151

N,N-dimethyl-5-(1-(3-(1-methyl-1H-pyrazol-4-yl)-1H-indazol-5-yl)oxy)ethyl)quin oline-7-amine

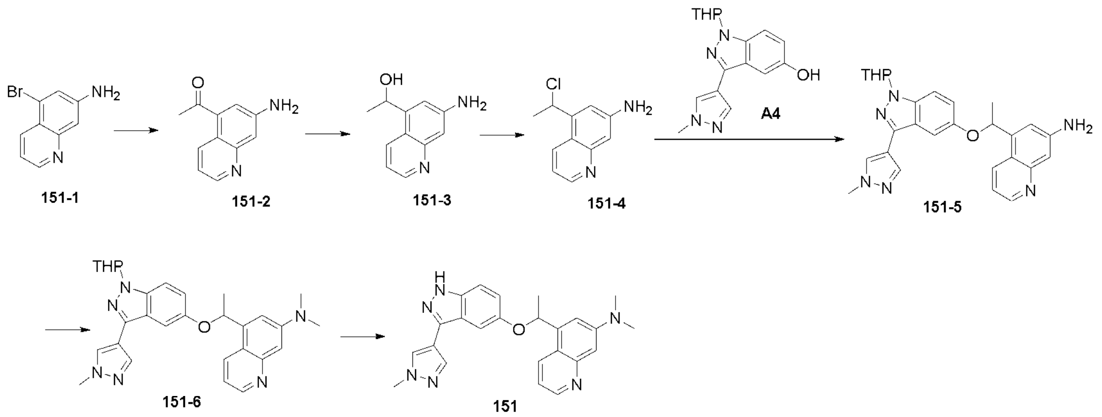


151-1 151-2 151-3 151-4 151-5

151-6 151

Compound tributyl(1-ethoxyvinyl)tin (178 mg, 0.49 mmol, 1.1 eq) and $Pd(PPh_3)_4$ (103 mg, 0.090 mmol, 0.2 eq) were added successively to a solution of Compound 151-1 (100 mg, 0.45 mmol, 1.0 eq) in dioxane (3.0 ml), purged with nitrogen three times, and stirred at 100°C for 16 hours. Diluted hydrochloric acid (3M, 3.0 mL) was added to the reaction solution, stirred for 1 hour, and then saturated aqueous potassium fluoride solution (3.0 mL) was added, and stirred for 1 hour. The mixed solution was filtered through diatomite, and the filtrate was poured into aqueous NaCl solution (15.0 mL), and extracted with EtOAc (15.0 mL x 2). The organic phases were combined, washed with saturated brine (10.0 mL), dried over anhydrous sodium sulfate, filtered, and concentrated under reduced pressure. The resulting residue was purified by thin-layer chromatography (developing solvent:DCM/MeOH = 10/1) to obtain Compound 151-2 (70 mg, yield 83.9%, a light yellow solid). LCMS:(ESI) m/z 187.2, (M+H)+.

Compound 151-2 (70 mg, 0.38 mmol, 1.0 eq) was dissolved in MeOH (2.0 mL) at room temperature, and $NaBH_4$ (14 mg, 0.38 mmol, 1.0 eq) was added at 0°C, and stirred at room temperature for 1 hour. The reaction solution was poured into ice water (10.0 mL), and extracted with DCM (10.0 mL x 2). The organic phases were combined, washed with saturated brine (10.0 mL), dried over anhydrous sodium sulfate, filtered, and concentrated under reduced pressure to obtain Compound 151-3 (75 mg, yield 95.4%, a light yellow solid). LCMS:(ESI) m/z 189.2, (M+H)+.

At 0°C, a solution of $SOCl_2$ (569 mg, 4.8 mmol, 12.0 eq.) in DCM (0.5 mL) was slowly added to a solution of Compound 151-3 (75 mg, 0.40 mmol, 1.0 eq.) in DCM(3.0 mL), and stirred at room temperature for 1 hour. The reaction solution was concentrated to obtain Compound 151-4 (80 mg, 97.1%, a yellow oil). LCMS:(ESI) m/z 207.2, (M+H)+.

At room temperature, Compound 151-4 (62 mg, 0.30 mmol, 1.3 eq.), Compound A4 (60 mg, 0.20 mmol, 1.0 eq.) and cesium carbonate (327 mg, 1.0 mmol, 5.0 eq.) were added successively to DMF (3.0 mL), and stirred at 60°C for 2 hours. The reaction solution was poured into aqueous NaCl solution (10.0 mL), and extracted with EtOAc (10.0 mL x 2). The organic phases were combined, washed with saturated brine (10.0 mL), dried over anhydrous sodium sulfate, filtered, and concentrated under reduced pressure. The resulting residue was purified by thin-layer chromatography (developing solvent:DCM/MeOH = 10/1) to obtain Compound 151-5 (60 mg, yield 63.7%, a light yellow solid). LCMS:(ESI) m/z 469.2, (M+H)+.

Compound 151-5 (30 mg, 0.064 mmol, 1.0 eq) was dissolved in MeOH (3.0 ml), and an aqueous solution of 40% formaldehyde (95 mg, 1.3 mmol, 20.0 eq), AcOH (0.77 mg, 0.013 mmol, 0.2 eq) and NaCNBH3 (20 mg, 0.32 mmol, 5.0 eq) were added successively, and reacted at room temperature for 24 hours. The reaction solution was poured into aqueous NaCl solution (10.0 mL), and extracted with EtOAc (10.0 mL × 2). The organic phases were combined, washed with saturated brine (10.0 mL), dried over anhydrous sodium sulfate, filtered, and concentrated under reduced pressure. The resulting residue was purified by thin-layer chromatography (developing solvent:DCM/MeOH = 10/1) to obtain Compound 151-6 (34 mg, yield > 100%, a yellow solid). LCMS:(ESI) m/z 497.0, (M+H)+.

At room temperature, 4M hydrochloric acid in dioxane (2.0 mL) was slowly added to a mixed solution of Compound 151-6 (34 mg, 0.068 mmol) in DCM (2.0 mL) and MeOH (2.0 mL), and stirred at room temperature for 2 hours. The reaction solution was concentrated under reduced pressure, and the resulting residue was purified by preparative high performance liquid chromatography to obtain Compound 151 (7.7 mg, yield 26.4%, purity 96.7%, a yellow solid). LCMS:(ESI) m/z 413.2, (M+H)+; $^1H$ NMR (400 MHz, DMSO-d6) δ 12.75 (s, 1H), 8.88 (d, J = 8.4 Hz, 1H), 8.74 (d, J = 4.5 Hz,

1H), 8.08 (s, 1H), 7.78 (s, 1H), 7.61 (d, J = 2.6 Hz, 1H), 7.41 - 7.31 (m, 3H), 7.13 (dd, J = 8.9, 2.2 Hz, 1H), 6.90 (d, J = 2.5 Hz, 1H), 6.33 (q, J = 6.4 Hz, 1H), 3.90 (s, 3H), 3.07 (s, 6H), 1.80 - 1.77 (m, 3H).

Example 152

imino(methyl)(5-(5-(1-(quinoxalin-5-yl)ethoxy)-1H-indazol-3-yl)pyridin-2-yl)-sulfanone

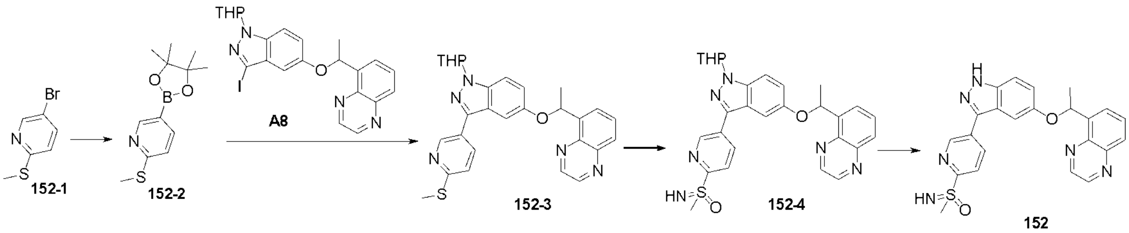

152-1 152-2 A8 152-3 152-4 152

Compound 152-1 (200 mg, 0.98 mmol, 1.0 eq) was dissolved in dioxane (10 ml), and then bis(pinacolato)diboron (373.3 mg, 1.5 mmol, 1.5 eq.), KOAc (288.5 mg, 3.0 mmol, 3.0 qe) and Pd (dppf) $Cl_2$ (71.7 mg, 0.1 mmol, 0.1 eq.) were added. After the addition was completed, the reaction solution was purged with nitrogen, and reacted under stirring at 105°C overnight. TLC showed no remaining raw materials. The reaction solution was directly filtered through diatomite, and the filtrate was dried under vacuum, triturated with ethyl acetate and then with petroleum ether to obtain Compound 152-2 as a green oil (330 mg, crude). LCMS:(ESI) m/z 252, (M+H)+.

Compound A8 (100 mg, 0.2 mmol, 1.0 eq.) was dissolved in dioxane (8 ml) and $H_2O$ (2 mL), and Compound 152-2 (176 mg, crude), $K_2CO_3$ (82.9 mg, 0.6 mmol, 3.0 eq.) and Pd[PPh3]4 (23.1 mg, 0.02 mmol, 0.1 eq.) were added, and stirred at 105°C overnight. TLC showed no remaining raw materials, and the generation of a new spot. The reaction solution was washed and extracted with ethyl acetate, subjected to rotary drying, and purified by thin-layer chromatography (developing solvent: petroleum ether:ethyl acetate=3:1) to obtain Compound 152-3 as a yellow oil (70 mg, crude): LCMS:(ESI) m/z514, (M+H)+.

Compound 152-3 (70 mg, crude) was dissolved in MeOH (5 mL), and $CH_3COONH_4$ (16.3 mg, 0.21 mmol, 1.5 eq.) and iodobenzene diacetate (95.2 mg, 0.3 mmol, 2.0 eq.) were added, and stirred at room temperature for 1 hour. TLC showed a small amount of raw materials remaining, and the generation of a new spot. Water was added, and then the reaction solution was extracted with ethyl acetate three times. The organic phases were combined, dried over anhydrous sodium sulfate, filtered, concentrated, and purified by thin-layer chromatography (developing solvent: petroleum ether:ethyl acetate=3:1) to obtain Compound 152-4 as a yellow oil (25 mg, crude). LCMS:(ESI) m/z529, (M+H)+.

Compound 152-4 (25 mg, crude) was dissolved in DCM/TFA (6 mL/1 mL), and stirred at room temperature for 2 hours. TLC monitoring showed the disappearance of Compound 152-4 and the generation of a spot with increased polarity, and LCMS monitoring showed the generation of Compound 152. The reaction solution was directly subjected to rotary drying, and purified by preparative high pressure liquid chromatography to obtain Compound 152 as a yellow solid (5.4 mg, yield 25.7%, purity 98.8%). LCMS:(ESI) m/z 445, (M+H)+; $^1$HNMR (400 MHz, CDCl3) $\delta$ 9.11 (s, 1H), 8.99 (s, 1H), 8.92 (d, J = 16.6 Hz, 1H), 8.33 (d, J = 12.0 Hz, 1H), 8.18 (d, J = 8.2 Hz, 1H), 8.01 (d, J = 10.8 Hz, 2H), 7.82 (s, 1H), 7.48 (d, J = 9.3 Hz, 1H), 7.28 (s, 1H), 7.22 (d, J = 11.3 Hz, 1H), 6.82 (s, 1H), 3.61 (d, J = 6.6 Hz, 3H), 1.81 - 1.76 (m, 3H).

The following compounds were synthesized according to the the route of Example 152.

| Example | Name | Structure | LC-MS $(M+H)^+$ | $^1H$ NMR |
|---|---|---|---|---|
| 153 | 1-imino-4-(5-(1-(quinoxalin-5-yl)ethoxy)-1H-indazol-3-yl)pyridin-2-yl)-thiomorpholine 1-oxide | 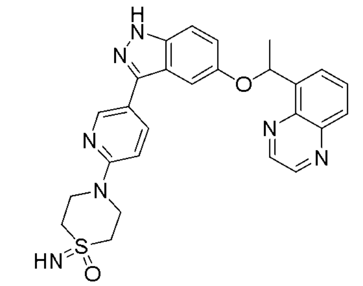 | 500.1 | $^1H$ NMR (400 MHz, $CD_3OD$) δ 9.08 (s, 1H), 8.99 (s, 1H), 8.19 (d, J = 2.3 Hz, 1H), 8.02 (d, J = 7.8 Hz, 2H), 7.82 (s, 2H), 7.40 (s, 1H), 7.20 (s, 1H), 7.11 (s, 1H), 7.01 (d, J = 8.9 Hz, 1H), 6.77 (d, J = 6.4 Hz, 1H), 4.67 (d, J = 15.2 Hz, 2H), 4.00 - 3.91 (m, 2H), 3.65 (s, 4H), 1.78 (d, J = 6.3 Hz, 3H). |
| 154 | 1-imino-4-(4-(5-(1-(quinoxalin-5-yl)ethoxy)-1H-indazol-3-yl)-1H-pyrazol-1-yl)hexahydro-thiopyran 1-oxide | 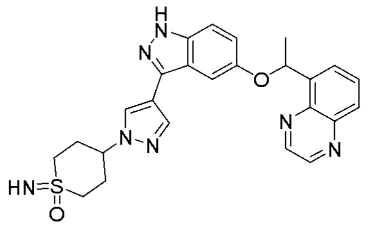 | 488.2 | $^1H$ NMR (400 MHz, DMSO-d6) δ 12.76 (s, 1H), 9.12 (d, J = 28.7 Hz, 2H), 7.98 (s, 3H), 7.81 (s, 1H), 7.62 (s, 1H), 7.37 (s, 1H), 7.17 (s, 1H), 7.08 (s, 1H), 6.74 (s, 1H), 3.72 (s, 1H), 3.18 (s, 6H), 2.20 (s, 2H), 1.72 (s, 3H). |

Example 156

(5-(1-((3-(1-methyl-1H-pyrazol-4-yl)-1H-indazol-5-yl)oxy)ethyl)quinolin-6-yl)m ethano

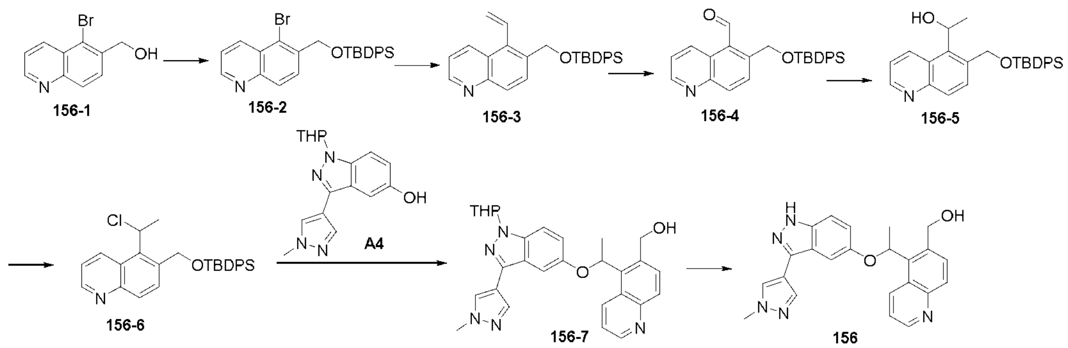


At room temperature, Compound 156-1 (0.293 g, 1.23 mmol) and imidazole (0.210 g, 3.08 mmol) were added to N,N-dimethylformamide (6.2 mL). The resulting reaction solution was cooled in an ice water bath, and then tert-butyldiphenylchlorosilane (0.508 g, 1.85 mmol) was slowly added dropwise. The resulting mixture was stirred at 0°C for 5 minutes, then warmed up to room temperature and stirred for 18 hours. LCMS showed the generation of a product. The reaction mixture was diluted with ethyl acetate (25.0 mL) and saturated aqueous sodium bicarbonate solution (10.0 mL). The organic phase was separated, washed with water (10.0 mL x 2), dried over anhydrous sodium sulfate, and filtered. The filtrate was concentrated to dryness under reduced pressure. The resulting residue was purified by silica gel column chromatography (eluent: dichloromethane:petroleum ether=1.5:1) to obtain Compound 156-2 as a colorless clear liquid (0.497 g, yield: 91%, purity: 92.95%). LCMS (ESI) m/z 476.0, 478.0, (M+H)+.

At room temperature, Compound 156-2 (0.497 g, 1.04 mmol), potassium vinyltrifluoroborate (0.210 g, 1.57 mmol), Pd(dppf)$Cl_2$ (0.0764 g, 0.104 mmol) and $Na_2CO_3$ (0.221 g, 2.08 mmol) were added to a mixture of 1,4-dioxane (5.2 mL) and water (2.6 mL). The resulting mixture was stirred at 90°C for 24 hours. LCMS showed the generation of a product. The reaction mixture was cooled to room temperature, and diluted with dichloromethane (20.0 mL) and water (10.0 mL). The organic phase was separated, and the aqueous phase was extracted with dichloromethane (10.0 mL × 3). The

organic phases were combined, dried over anhydrous sodium sulfate, and filtered. The filtrate was concentrated to dryness under reduced pressure. The resulting residue was purified by silica gel column chromatography (eluent: petroleum ether:ethyl acetate=10:1) to obtain Compound 156-3 as a light yellow liquid (0.355 g, yield: 74%, purity: 91.99%). LCMS (ESI) m/z 424.0, (M+H)+.

At 0°C, Compound 156-3 (0.355 g, 0.84 mmol) was added to a mixture of tetrahydrofuran (8.4 mL) and water (1.7 mL), and then K2OsO4·$2H_2O$ (0.031 g, 0.084 mmol) was added. The resulting mixture was stirred for 20 minutes, and then $NaIO_4$ (0.718 g, 3.36 mmol) was added. The reaction mixture was stirred at 0°C for 15 minutes, warmed up to room temperature and stirred for 48 hours. LCMS showed the generation of a product. The reaction mixture was diluted with dichloromethane (30.0 mL) and water (15.0 mL). The organic phase was separated, and the aqueous phase was extracted with dichloromethane (15.0 mL × 3). The organic phases were combined, washed with saturated aqueous sodium sulfite solution (20.0 mL × 2), dried over anhydrous sodium sulfate, and filtered. The filtrate was concentrated to dryness under reduced pressure. The resulting residue was purified by silica gel column chromatography (eluent: petroleum ether:ethyl acetate=6:1) to obtain 156-4 as a light yellow sticky liquid (0.252 g, yield: 67%, purity: 95.58%). LCMS (ESI) m/z 426.0, (M+H)+.

At 0°C, methyl magnesium bromide solution (3.0 M, 2-methyltetrahydrofuran solution) (0.30 mL, 0.90 mmol) was added dropwise to a solution of Compound 156-4 (0.252 g, 0.59 mmol) in tetrahydrofuran (3.0 mL). The reaction mixture was stirred at 0°C for 5 minutes, then warmed up to room temperature and stirred for 28 hours. LCMS showed the generation of a product. The reaction mixture was diluted with saturated aqueous ammonium chloride solution (5.0 mL), and the resulting mixture was extracted with ethyl acetate (10.0 mL × 3). The organic phases were combined, dried over anhydrous sodium sulfate, and filtered. The filtrate was concentrated to dryness under reduced pressure. The resulting residue was purified by silica gel column chromatography (eluent: petroleum ether:ethyl acetate=3:1) to obtain Compound 156-5 as a white solid (0.238 g, yield: 86%, purity: 94.31%). LCMS (ESI) m/z 442.0, (M+H)+.

At 0°C, thionyl chloride (0.0999 g, 0.84 mmol) was added dropwise to a solution of Compound 156-5 (0.124 g, 0.28 mmol) in dichloromethane (2.8 mL). The resulting mixture was stirred at 0°C for 5 minutes, then warmed up to room temperature and stirred for 8 hours. LCMS showed the generation of a product. The reaction mixture was concentrated to dryness under reduced pressure. Dichloromethane (5.0 mL) was added to the resulting residue, and concentrated to dryness under reduced pressure again. This operation was repeated three times again, and the resulting residue was dried under vacuum to obtain a crude product 156-6 as a light yellow solid (0.139 g, crude yield: 63%, purity: 63.44%). LCMS (ESI) m/z 460.2 (M+H)+.

At room temperature, the crude product Compound 156-6 (0.139 g, 0.28 mmol (a theoretical amount)) and cesium carbonate (0.458 g, 1.40 mmol) were added successively to N,N-dimethylformamide (1.4 mL). The resulting mixture was stirred at room temperature for 2 minutes, and then Compound A4 (0.0838 g, 0.28 mmol) was added. The reaction mixture was stirred at 45°C for 26 hours. LCMS showed the generation of a product. The reaction mixture was cooled to room temperature, and diluted with ethyl acetate (20.0 mL) and saturated aqueous ammonium chloride solution (10.0 mL). The organic phase was separated, and the aqueous phase was extracted with ethyl acetate (10.0 mL × 3). The organic phases were combined, dried over anhydrous sodium sulfate, and filtered. The filtrate was concentrated to dryness under reduced pressure. The resulting residue was purified by silica gel thin-layer chromatography (eluent: dichloromethane:methanol=25:1) to obtain Compound 156-7 as a light brown solid (0.0468 g, yield: 28%, purity: 81.88%). LCMS (ESI) m/z 484.0, (M+H)+.

At room temperature, trifluoroacetic acid (0.552 g, 4.84 mmol) was added to a solution of Compound 156-7 (0.0468 g, 0.0968 mmol) in dichloromethane (0.48 mL). The resulting mixture was stirred for 8 hours. LCMS showed the generation of a product. The reaction mixture was diluted with dichloromethane (10.0 mL), and saturated aqueous sodium bicarbonate solution was added to adjust the pH of the aqueous phase to about 7 to 8. The organic phase was separated, and the aqueous phase was extracted with dichloromethane (5.0 mL × 3). The organic phases were combined, dried over anhydrous sodium sulfate, and filtered. The filtrate was concentrated to dryness under reduced pressure. The resulting residue was purified by silica gel thin-layer chromatography (eluent: dichloromethane: methanol=12:1) to obtain Compound 156 as a beige solid (0.0238 g, yield: 60%, purity: 97.88%). LCMS (ESI) m/z 400.2, (M+H)+; $_1$H NMR (400 MHz, DMSO) δ 12.71 (s, 1H), 9.27 (d, J = 8.8 Hz, 1H), 8.87 (d, J = 2.8 Hz, 1H), 7.98 (s, 1H), 7.93 (d, J = 8.8 Hz, 1H), 7.88 (d, J = 8.8 Hz, 1H), 7.71 (s, 1H), 7.58 (dd, J = 8.8, 4.0 Hz, 1H), 7.33 (d, J = 8.8 Hz, 1H), 7.23 (s, 1H), 7.08 (d, J = 8.8 Hz, 1H), 6.38 (d, J = 6.4 Hz, 1H), 5.69 (s, 1H), 5.09 (d, J = 12.8 Hz, 1H), 4.83 (d, J = 12.8 Hz, 1H), 3.92 (s, 3H), 1.89 (d, J = 6.4 Hz, 3H).

Example 158

5-(1-((3-(1-(1-methylazetidin-3-yl)-1H-pyrazol-4-yl)-1H-indazol-5-yl)oxy)ethyl)q uinoxaline

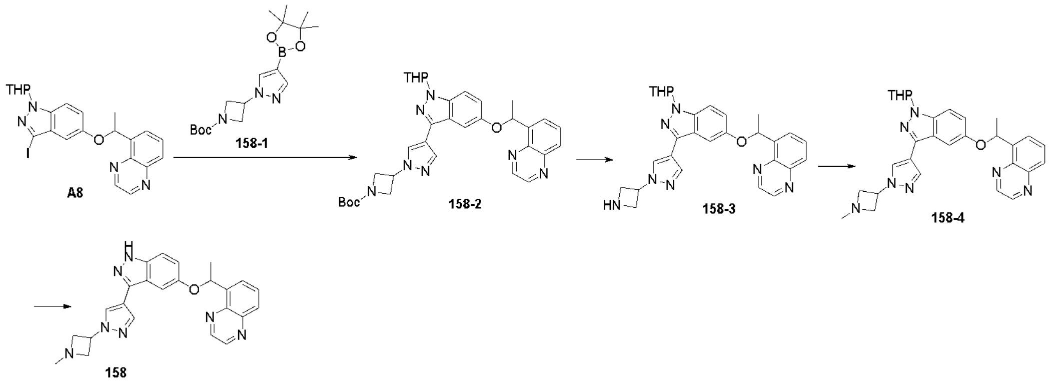


Compound A8 (200 mg, 0.4 mmol, 1.0 eq.) was dissolved in THF/$H_2O$ (5/1 ml), and then Compound 158-1 (168 mg, 0.48 mmol, 1.2 eq.), the compound $K_2CO_3$ (165.6 mg, 1.2 mmol, 3.0 eq.) and the compound Pd($pph_3$)$_4$ (46 mg, 0.04 mmol, 0.1 eq.) were added respectively. After the addition was completed, the reaction solution was purged with nitrogen three times, and reacted under stirring at 75°C overnight. TLC showed no remaining raw materials, and the conversion of most of raw materials into a product. Then the reaction solution was extracted with (20 mL) ethyl acetate three times. The organic phases were combined, washed with saturated brine, dried over anhydrous sodium sulfate, filtered, concentrated, and purified by thin-layer chromatography (developping agent: petroleum ether:ethyl acetate=2:1) to obtain Compound 158-2 as a yellow solid (60 mg, 25%). LCMS:(ESI) m/z 596, (M+H) +.

Compound 158-2 (60 mg, 0.1 mmol, 1.0 eq.) was dissolved in DCM/TFA (5 mL/0.5 mL). After the addition was completed, the reaction solution was reacted under stirring at 25°C for 0.2 hour. TLC showed no remaining raw materials, and the conversion of most of raw materials into a product. The reaction solution was extracted with (10 ml) ethyl acetate three times. The organic phases were combined, washed with saturated brine, dried over anhydrous sodium sulfate, filtered, concentrated, and purified by thin-layer chromatography (developping agent: DCM:MeOH=20:1) to obtain Compound 158-3 as a yellow solid (40 mg, 80 %). LCMS:(ESI) m/z 496, (M+H) +.

Compound 158-3 (50 mg, 0.1 mmol, 1.0 eq.) was dissolved in methanol (4 mL), then formaldehyde (9.8 mg, 0.12 mmol, 1.2 eq.) and acetic acid (1 drop) were added, stirred at room temperature for 1 hour, then cooled and NaBH($OAc)_3$ (64.1 mg, 0.3 mmol, 3.0 eq.) was added, and then stirred at room temperature for 1 hour. TLC showed a small amount of raw materials remaining. 10 mL of water was added, and then the reaction solution was extracted with ethyl acetate three times (10mL×3). The organic phases were combined, dried over anhydrous sodium sulfate, filtered, concentrated, and purified by thin-layer chromatography (developping agent: DCM: MeOH = 20:1) to obtain Compound 158-4 as a yellow oil (26 mg, crude). LCMS:(ESI) m/z 510, (M+H)+.

Compound 158-4 (26 mg, crude) was dissolved in DCM/TFA (5 mL/1 mL), and stirred at room temperature for 2 hours. TLC showed the complete reaction. The reaction solution was directly subjected to rotary drying, and purified by preparative high performance liquid chromatography to obtain Compound 158 as a yellow solid (2.7 mg, 12%). LCMS:(ESI) m/z 426, (M+H)+; $^1$H NMR (400 MHz, $CD_3OD$) δ 9.05 (s, 1H), 8.97 (s, 1H), 7.99 (s, 2H), 7.93 (s, 1H), 7.84 - 7.79 (m, 1H), 7.72 (s, 1H), 7.37 (d, J = 9.7 Hz, 1H), 7.17 (d, J = 7.3 Hz, 1H), 7.12 (s, 1H), 6.75 (d, J = 6.5 Hz, 1H), 5.23 (d, J = 8.7 Hz, 1H), 4.40 (t, J = 9.0 Hz, 2H), 4.20 (t, J = 8.2 Hz, 2H), 2.89 (s, 3H), 1.77 (d, J = 6.3 Hz, 3H).

Example 160

N,N-dimethyl-2-(4-(5-(1-(quinoxalin-5-yl)ethoxy)-1H-indazol-3-yl)-1H-pyrazol-1-yl)ethane-1-sulfonamide

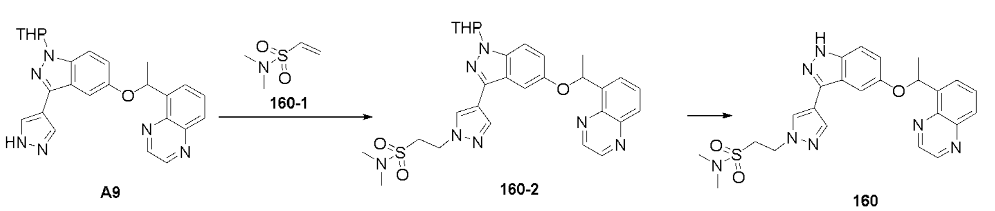


DBU (41.47 mg, 0.27 mmol, 3 eq) was added to a solution of Compound A9 (40 mg, 0.091 mmol, 1.0 eq) and Compound 160-1 (14.45 mg, 0.14 mmol, 1.5eq) in dioxane (10 mL), purged with N2 three times, and stirred at 90°C for 6 hours. LCMS showed the complete reaction of Compound A9. The reaction mixture was cooled, 20 mL of water was

added, and then the reaction mixture was extracted with ethyl acetate, separated and dried by rotary drying to obtain a liquid as a yellow oil. The crude was subjected to thin-layer chromatography (developing solvent: petroleum ether:ethyl acetate=1:8) to obtain Compound 160-2 (40 mg, yield: 76%). LCMS (ESI) m/z 576.2, (M+H)+.

Compound 160-2 (40 mg, 0.07 mmol, 1.0 eq.) was dissolved in DCM (2 mL), and TFA (1 mL) was added, and reacted at 25°C for 3 hours. LCMS showed the complete reaction of Compound 160-2 and the generation of a product. The reaction solution was subjected to rotary drying to obtain a crude which was purified by thin-layer chromatography (developing solvent: DCM:MeOH=10:1) to obtain Compound 160 as a white solid (20.5 mg, yield 60%). LCMS (ESI) m/z 492.2, (M+H)+); $^1$H NMR (400 MHz, DMSO) $\delta$ 9.10 (dd, J = 15.6, 1.6 Hz, 2H), 8.19 (s, 1H), 8.09 - 7.96 (m, 2H), 7.92 - 7.82 (m, 1H), 7.70 - 7.55 (m, 2H), 7.39 (d, J = 9.2 Hz, 1H), 7.27 (d, J = 2.0 Hz, 1H), 7.10 (dd, J = 9.2, 2.4 Hz, 1H), 6.74 (q, J = 6.4 Hz, 1H), 4.56 (t, J = 7.2 Hz, 2H), 3.68 (d, J = 7.6 Hz, 4H), 2.77 (s, 7H), 1.75 (d, J = 6.4 Hz, 3H).

Example 162

4-(4-(5-(1-(quinoxalin-5-yl)ethoxy)-1H-indazol-3-yl)-1H-pyrazol-1-yl)piperidine-1-carbonitrile

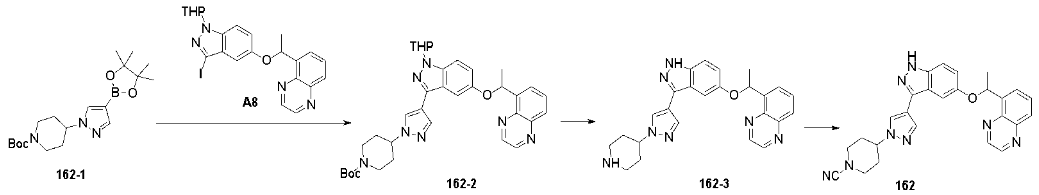


Compound 162-1 (113 mg, 0.30 mmol, 1.5 eq.), CompoundA8 (100mg, 0.20 mmol, 1.0 eq.), potassium carbonate (83 mg, 0.60 mmol, 3.0 eq.) and Pd(dppf)$Cl_2$ (15 mg, 0.02 mmol, 0.1 eq.) were dissolved in a mixed solution of dioxane (2 mL) and water (0.4 mL), and stirred at 90°C for 12 hours. LCMS showed the generation of a product. The reaction mixture was cooled, extracted with dichloromethane, washed with saturated brine, dried over anhydrous sodium sulfate, concentrated, subjected to rotary drying, and purified by silica gel column chromatography (eluent: petroleum ether:ethyl acetate= 1:1) to obtain an off-white solid 162-2 (100 mg, yield 80.20%): LCMS (ESI) m/z 624.32, (M+H)+.

1 mL of trifluoroacetic acid was added to a solution of Compound 162-2 (100 mg, 0.16 mmol, 1.0 eq.) in dichloromethane (2 mL), and stirred at room temperature for 1 hour. LCMS showed the generation of a product. The reaction solution was directly concentrated by rotary drying to obtain a brownish oil 162-3 (70 mg, yield 99.36%): LCMS (ESI) m/z 440.21, (M+H)+.

Compound 162-3 (40 mg, 0.06 mmol, 1.0 eq.), cyanogen bromide (10mg, 0.10 mmol, 1.5 eq.) and N,N-diisopropylethylamine (25 mg, 0.20 mmol, 3.0 eq.) were dissolved in dichloromethane solution (2 mL), and stirred at room temperature for 2 hours. LCMS showed the generation of a product. The reaction mixture was extracted with dichloromethane, washed with saturated brine, dried over anhydrous sodium sulfate, concentrated, subjected to rotary drying, and purified by preparative high performance liquid chromatography to obtain a product Compound 162 (1.5 mg, purity 100.00%): LCMS (ESI) m/z 465.2, (M+H)+).

Example 163

5-(1-((3-(1-(1-(methylsulfonyl)piperidin-4-yl)-1H-pyrazol-4-yl)-1-indazol-5-yl)ox y)ethyl)quinoxaline

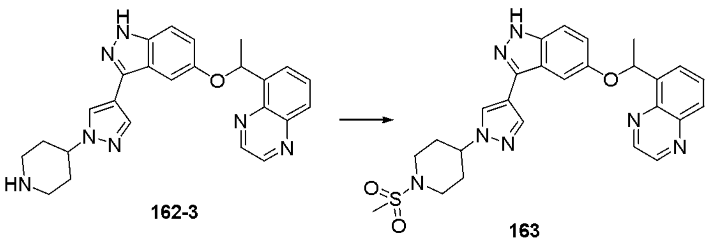

Triethylamine (28 mg, 0.27 mmol, 3.0 eq) was added to a solution of Compound 162-3 (40 mg, 0.09 mmol, 1.0 eq) in dichloromethane (1 mL), and methylsufonyl chloride (16 mg, 0.14 mmol, 1.5 eq) was added to the mixed solution at 0°C,

and stirred at room temperature for 2 hours. LCMS showed the generation of a product. The reaction solution was extracted with dichloromethane, washed with saturated brine, dried over anhydrous sodium sulfate, concentrated, subjected to rotary drying, and purified by preparative high performance liquid chromatography to obtain a product Compound 163 (4.0 mg, purity 100.00%). LCMS (ESI) m/z 518.2, (M+H)+); $^1$H NMR (400 MHz, DMSO) $\delta$ 13.47 - 13.34 (m, 1H), 12.77 (s, 1H), 9.12 (d, J = 2.0 Hz, 1H), 9.09 (d, J = 1.6 Hz, 1H), 8.04 (d, J = 6.4 Hz, 2H), 7.99 (d, J = 7.2 Hz, 1H), 7.86 (dd, J = 15.2, 7.2 Hz, 1H), 7.63 (s, 1H), 7.39 (d, J = 8.8 Hz, 1H), 7.20 (s, 1H), 7.11 (dt, J = 11.2, 5.6 Hz, 1H), 6.76 - 6.68 (m, 1H), 4.40 - 4.30 (m, 1H), 3.74 (d, J = 10.4 Hz, 2H), 2.99 (d, J = 5.6 Hz, 5H), 2.08 (dd, J = 36.0, 11.2 Hz, 3H), 2.06 - 1.90 (m, 2H), 1.75 (d, J = 6.4 Hz, 3H).

Example 164

7-cyclopropyl-5-(1-((3-(1-methyl-1H-pyrazol-4-yl)-1H-indazol-5-yl)oxy)ethyl)qui noline

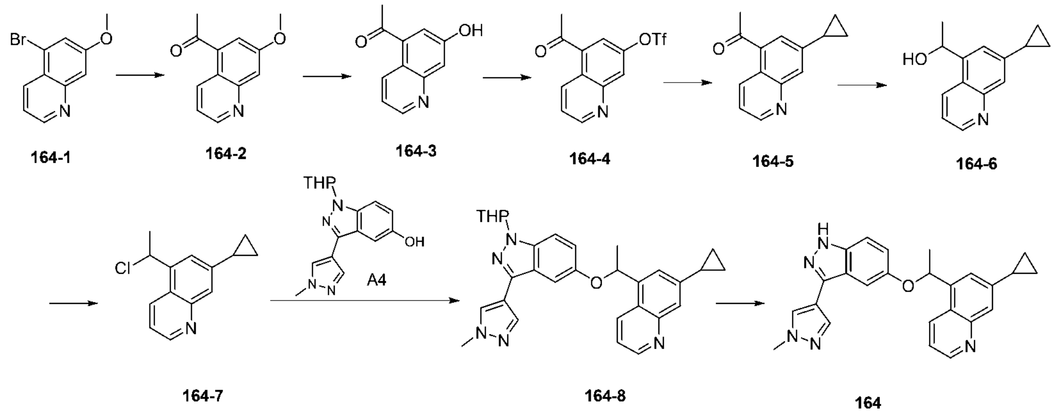


164-1 164-2 164-3 164-4 164-5 164-6 164-7 164-8 164

Tetrakis(triphenylphosphine)palladium (970 mg, 0.84 mmol, 0.2 eq) was added to a solution of Compound 164-1 (1 g, 4.2 mmol 1.0 eq) and tributyl(1-ethoxyvinyl)tin (2.28 g, 6.3 mmol, 1.5eq) in dioxane (6 mL), purged with $N_2$ three times, and stirred at 110°C for 12 hours. LCMS showed the complete reaction of Compound 164-1. The reaction mixture was cooled, and 20 mL of KF solution was added, stirred at room temperature for 30 min, filtered, then extracted with EA, and washed with saturated brine. The organic phase was concentrated to obtain a liquid as a yellow oil, and dioxane (10 mL) and 4M HCl (6 mL) were added, and stirred at room temperature for 30 min. LCMS showed the complete reaction. The pH was adjusted to 7 with saturated $NaHCO_3$ (20 mL), and then the reaction solution was extracted with ethyl acetate, separated and dried by rotary drying to obtain a liquid as a yellow oil. The crude was purified by silica gel column chromatography (eluent: petroleum ether:ethyl acetate=3:1) to obtain Compound 164-2 (750 mg, yield: 88%). LCMS (ESI) m/z 202.2, (M+H)+.

Compound 164-2 (750 mg, 3.37 mmol, 1.0 eq.) and pyridine hydrochloride (10 g) were mixed, heated to 160°C and stirred for 4 hours. LCMS showed the generation of a product. The reaction solution was cooled, saturated Na2CO3 solution was added to the reaction solution to adjust the pH to 8, and then the reaction solution was extracted with EA, separated and dried by rotary drying to obtain a yellow solid which was purified by silica gel column chromatography (eluent: petroleum ether:ethyl acetate=2:1) to obtain Compound 164-3 as a yellow solid (450 mg, yield 65%). LCMS (ESI) m/z 188.2, (M+H)+.

Compound 164-3 (450 mg, 2.4 mmol, 1.0 eq.) and pyridine (380 mg, 4.81 mmol, 2 eq.) were dissolved in DCM (10 mL), and $Tf_2O$ (1.36 g, 4.81 mmol, 2 eq.) was added dropwise at 0°C, and reacted at 25°C for 2 h. LCMS showed the complete reaction of Compound 164-3 and the generation of a product. Saturated aqueous $NaHCO_3$ solution (50mL) was added to the reaction mixture, and then the reaction mixture was extracted with DCM. The organic phase was separated, dried by rotary drying, and purified by silica gel column chromatography (eluent: petroleum ether:ethyl acetate=4:1) to obtain Compound 164-4 as a yellow oil liquid (300 mg, yield 40 %).

LCMS (ESI) m/z 320.2, (M+H)+).

Compound 164-4 (120 mg, 0.376 mmol, 1.0 eq.) and cyclopropylboronic acid (32.3 mg, 0.376 mmol, 1 eq.) were dissolved in toluene (2 mL) and water (0.2 mL), and $Cs_2CO_3$ (167 mg, 0.51 mmol, 2 eq.) and Pd(dppf)$Cl_2$ (61 mg, 0.07 mmol, 0.2 eq) were added, and reacted at 90°C for 6 hours. LCMS showed the complete reaction of Compound 5 and the generation of a product. The reaction solution was filtered, dried by rotary drying, and purified by thin-layer chromatography (developing solvent: petroleum ether:ethyl acetate=3:1) to obtain Compound 164-5 as a colorless oil liquid (70 mg, yield 88%). LCMS (ESI) m/z 212.2, (M+H)+.

Compound 164-5 (70 mg, 0.33 mmol, 1.0 eq.) was dissolved in EtOAc (10 mL), and $NaBH_4$ (9.5 mg, 0.25 mol, 1 eq.) was added, and stirred at room temperature for 30 min. LCMS showed the generation of a product. The reaction solution was quenched with saturated $NH_4Cl$ solution, extracted with EtOAc, separated and dried by rotary drying to obtain a yellow solid which was subjected to thin-layer chromatography (developing solvent: petroleum ether:ethyl acetate=1:1) to obtain Compound 164-6 as an off-white solid (70 mg, yield 99%). LCMS (ESI) m/z 214.2 (M+H)+.

Compound 164-6 (70 mg, 0.33 mmol, 1.0 eq.) was dissolved in DCM (3 mL), and $SOCl_2$ (78mg, 0.66 mmol, 2 eq.) was added dropwise at 0°C, and reacted at room temperature for 1 h. LCMS showed the complete reaction of Compound 164-6 and the generation of a product. The reaction mixture was extracted with aqueous $NaHCO_3$ solution and DCM. The organic phase was separated and dried by rotary drying to obtain Compound 164-7 as a yellow oil liquid (70 mg, yield 92 %). LCMS (ESI) m/z 232.2, (M+H)+).

Compound 164-7 (70 mg, 0.3 mmol, 1.0 eq.) and Compound A4 (90 mg, 0.3 mmol, 1 eq.) were dissolved in DMF (2 mL), and $Cs_2CO_3$ (196 mg, 0.6 mmol, 2 eq.) was added, and reacted at 60°C for 6 hours. LCMS showed the complete reaction of Compound 164-7 and the generation of a product. The reaction mixture was extracted with water and EtOAc. The organic phase was separated, dried by rotary drying, and purified by thin-layer chromatography (developing solvent:DCM:MeOH=10:1) to obtain Compound 164-8 as a yellow solid (110 mg, yield 74%). LCMS (ESI) m/z 494.2, (M+H)+).

Compound 164-8 (110 mg, 0.16 mmol, 1.0 eq.) was dissolved in DCM (2 mL), and TFA (1 mL) was added, and reacted at 25°C for 3 hours. LCMS showed the complete reaction of Compound 164-8 and the generation of a product. The reaction solution was subjected to rotary drying to obtain a crude which was purified by thin-layer chromatography (developing solvent:DCM:MeOH=10:1) to obtain Compound 164 as a white solid (85 mg, yield93 %). LCMS (ESI) m/z 410.2, (M+H)+); $^1H$ NMR (400 MHz, DMSO) δ 9.04 (d, J = 8.4 Hz, 1H), 8.94 (d, J = 4.4 Hz, 1H), 8.08 (s, 1H), 7.77 (s, 1H), 7.63 (t, J = 5.2 Hz, 4H), 7.38 (d, J = 9.2 Hz, 1H), 7.30 (d, J = 2.0 Hz, 1H), 7.12 (dd, J = 9.2, 2.4 Hz, 1H), 6.36 (q, J = 6.4 Hz, 1H), 3.91 (s, 3H), 2.20 - 2.12 (m, 1H), 1.78 (d, J = 6.4 Hz, 3H), 1.09 (dd, J = 8.4, 2.3 Hz, 2H), 0.87 (dd, J = 18.4, 13.2 Hz, 1H), 0.78 (t, J = 16.4 Hz, 1H).

Example 165

6-methyl-5-(1-((3-(1-methyl-1H-1,2,3-triazol-4-yl)-1H-indazol-5-yl)oxy)ethyl)qui noline

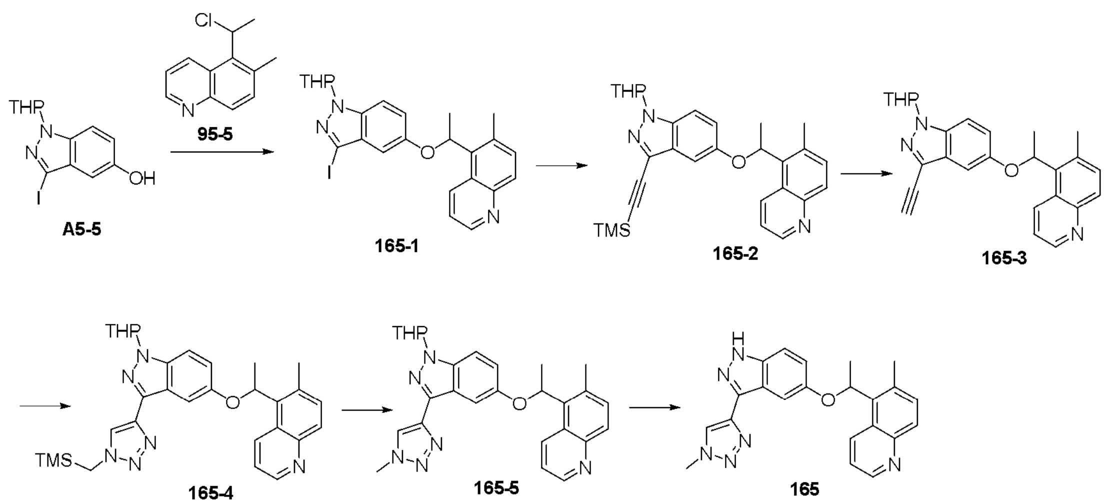

At room temperature, Compound A5-5 (6.4 g, 18.587 mmol) and Compound 95-5 (3.8 g, 18.597 mmol) were dissolved in 60 mL of DMF, and $Cs_2CO_3$ (12.12 g, 37.19 mmol) was added. The reaction solution was reacted at 60°C for 12 h. LCMS showed the complete reaction and no remaining raw materials. The reaction solution was quenched with water, and then extracted with EA (100 mL x 3). The organic phases were combined and then subjected to rotary drying. The crude was purified by silica gel column chromatography (eluent: petroleum ether:ethyl acetate=3:1) to obtain a product as a white solid (5.93 g, yield 62.11%, purity 98%). LCMS (ESI) m/z 514.01, (M+H)+.

At room temperature, Compound 165-1 (50 mg, 0.12 mmol) was dissolved in DMF (2 mL), and trimethylsilylacetylene (17 mg, 0.17mmol) was added. Then, [1,1'-bis(diphenylphosphino)ferrocene]palladium dichloride dichloromethane complex (9 mg, 0.011mmol), DIEA (30 mg, 0.23mmol) and CuI (2.2 mg, 0.011mmol) were added under nitrogen protection. The reaction solution was stirred at 100°C for 16 h. LCMS showed no remaining raw materials. The reaction

solution was cooled to room temperature, then quenched with 100 mL of aqueous solution, and the solid generated was filtered through diatomite. The filtrate was extracted with ethyl acetate (100 mL × 3), and the organic phases were combined and then subjected to rotary drying. The crude was purified by silica gel column chromatography (eluent: petroleum ether:ethyl acetate=1:1) to obtain a product 165-2 as a yellow solid (40 mg, purity 100%, yield 71%, [M+H]+=484.0).

At room temperature, Compound 165-2 (110 mg, 0.23 mmol) was dissolved in MeOH (5 mL), and $K_2CO_3$ (31 mg, 0.23 mmol) was added at room temperature, and stirred at room temperature for 2h. LCMS showed the complete reaction of raw materials. The organic phases were combined and then subjected to rotary drying. The crude was purified by silica gel column chromatography (eluent: petroleum ether:ethyl acetate=1:3) to obtain a product 165-3 as a yellow oil (88 mg, purity 100%, yield 94%,[M+H]+=412.0).

At room temperature, Compound 165-3 (50 mg, 0.12 mmol) was dissolved in DMF (2 mL), and trimethylsilylazidomethane (23 mg, 0.18 mmol), DIEA (31 mg, 0.24mmol) and CuI (2.3 mg, 0.01 mmol) were added at room temperature. The reaction solution was stirred at room temperature for 2 hours. LCMS showed the complete reaction of raw materials. The reaction solution was directly subjected to rotary drying to obtain a crude which was purified by silica gel column chromatography (eluent: petroleum ether:ethyl acetate=5:1) to obtain a product 165-4 as a yellow oil (19 mg, purity 100%, yield >29%, [M+H]+=540.8).

At room temperature, Compound 165-4 (30 mg, 0.05 mmol) and the compound TBAF (22 mg, 0.08mmol) were dissolved in THF (2 mL), and $C_{S2}CO_3$ (136 mg, 0.42 mmol) was added. The reaction solution was stirred at 60°C for 2 h. LCMS showed the complete reaction of raw materials. The reaction solution was diluted with water and then extracted with EA (30 mL x 3). The organic phases were combined and then subjected to rotary drying. The crude was purified by silica gel column chromatography (eluent: DCM:MeOH=10:1) to obtain a product 165-5 as a yellow oil (20 mg, purity 90%, LCMS:469.2[M+H]+).

Compound 165-5 (20 mg, 0.04 mmol) was dissolved in DCM (2 mL) at room temperature, and TFA (1 mL) was added thereto in an ice bath. The reaction solution was stirred at room temperature for 1 h. LCMS showed the complete reaction of raw materials. The reaction solution was concentrated, and the organic phase was collected, and then purified by preparative high performance liquid chromatography to obtain Compound 165 as a white solid (7.1 mg, purity: 100%, LCMS: 385.0[M+H]+). $^1$H NMR(400 MHz, DMSO) δ 12.95 (s, 1H), 9.21 (d,J = 8.6 Hz, 1H), 8.83 - 8.74 (m, 1H), 8.36 (s, 1H), 7.80 (d,J = 8.6 Hz, 1H), 7.65 (s, 1H), 7.58 (d,J = 8.7 Hz, 1H), 7.53 (dd,J = 8.8, 4.1 Hz, 1H), 7.38 (d,J = 9.0 Hz, 1H), 7.09 (dd,J = 9.0, 2.4 Hz, 1H), 6.23 (d,J = 6.7 Hz, 1H), 4.14 (s, 3H), 2.81 (s, 3H), 1.87 (d,J = 6.7 Hz, 3H).

Example 166

6-ethynyl-5-(1-((3-(1-methyl-lH-pyrazol-4-yl)-1H-indazol-5-yl)oxy)ethyl)quinolin e

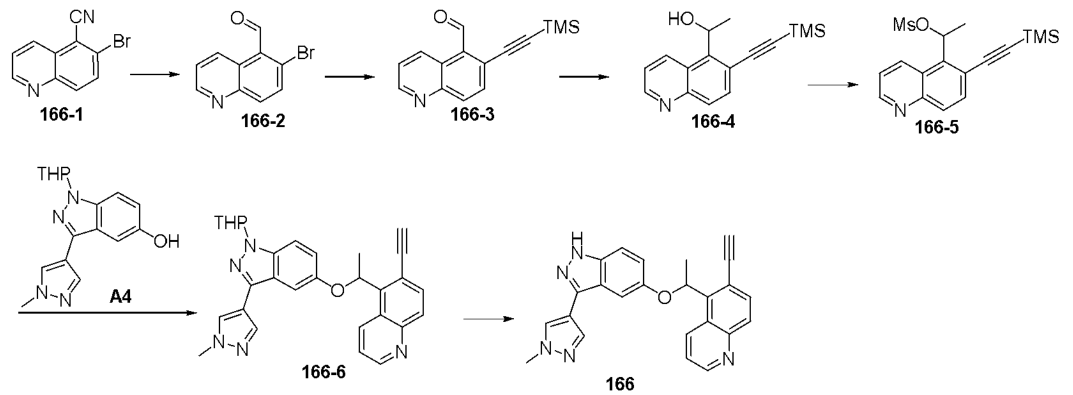


Compound 166-1 (800 mg, 3.4 mmol, 1.0 eq.) was dissolved in toluene (20.0 ml), and then the compound DIBAL-H (965 mg, 6.8 mmol, 2.0 eq.) was added. After the addition was completed, the reaction solution was purged with nitrogen three times, and reacted under stirring at -10°C for 1 hour. TLC showed no remaining raw materials, and the conversion of most of raw materials into a product. Then, the reaction solution was extracted with (20 mL) ethyl acetate three times. The organic phases were combined, washed with saturated brine, dried over anhydrous sodium sulfate, filtered, concentrated, and purified by silica gel column chromatography (eluent: DCM:MeOH=100:1-30:1) to obtain Compound 166-2 as a yellow solid (460 mg, 56%). LCMS:(ESI) m/z 237, (M+H) +.

Compound 166-2 (70 mg, 0.3 mmol, 1.0 eq.) was dissolved in dioxane (10 mL), and then the compound

trimethylsilylacetylene (63.0 mg, 0.6 mmol, 2.0 eq.), the compound CuI (12.2 mg, 0.065 mmol, 0.2 eq.), the compound TEA (97 mg, 0.96 mmol, 3.0 eq.) and the compound $Pd(pph_2)Cl_2$ (22.4 mg, 0.03 mmol, 0.1 eq.) were added respectively. After the addition was completed, the reaction solution was reacted under stirring at 25°C for 12 hours. TLC showed no remaining raw materials, and the conversion of most of raw materials into a product. The reaction solution was extracted with (10 ml) ethyl acetate three times. The organic phases were combined, washed with saturated brine, dried over anhydrous sodium sulfate, filtered, concentrated, and purified by thin-layer chromatography (developping agent: DCM:MeOH=20:1) to obtain Compound 166-3 as a yellow solid (40 mg, 50 %). LCMS:(ESI) m/z 254, (M+H) +.

Compound 166-3 (40 mg, 0.15 mmol, 1.0 eq.) was dissolved in THF (5 mL), purged with nitrogen three times, and then the compound $CH_3MgBr$ (0.6 mL, 0.3 mmol, 2.0 eq.) was added. After the addition was completed, the reaction solution was reacted under stirring at 0-25°C for 2 hours. TLC showed no remaining raw materials, and the conversion of most of raw materials into a product. The reaction solution was extracted with (10 ml) ethyl acetate three times. The organic phases were combined, washed with saturated brine, dried over anhydrous sodium sulfate, filtered, concentrated, and purified by thin-layer chromatography (developping agent: DCM:MeOH=20:1) to obtain Compound 166-4 as a yellow solid (25 mg, 62%). LCMS:(ESI) m/z 270, (M+H) +.

Compound 166-4 (25 mg, 0.09 mmol, 1.0 eq.) was dissolved in DCM (5 mL), and then the compound TEA (28 mg, 0.28 mmol, 3.0 eq.) and the compound MsCl (31.0 mg, 0.186 mmol, 1.5 eq) were added respectively. After the addition was completed, the reaction solution was purged with nitrogen three times, and reacted under stirring at 25°C for 2 hours. TLC showed no remaining raw materials, and the conversion of most of raw materials into a product. The reaction solution was extracted with (10 ml) ethyl acetate three times. The organic phases were combined, washed with saturated brine, dried over anhydrous sodium sulfate, filtered, concentrated, and purified by thin-layer chromatography (developping agent: DCM:MeOH=30:1) to obtain Compound 166-5 as a yellow solid (20 mg, 46%). LCMS:(ESI) m/z 348 (M+H) +.

Compound 166-5 (15 mg, 0.05 mmol, 1.0 eq.) was dissolved in MeCN (5 mL), and then the compound $C_{S2}CO_3$ (23.0 mg, 0.15 mmol, 3.0 eq.) and Compound A4 (20 mg, 0.1 mmol, 1.5 eq) were added respectively. After the addition was completed, the reaction solution was purged with nitrogen three times, and reacted under stirring at 80°C for 3 hours. TLC showed no remaining raw materials, and the conversion of most of raw materials into a product. The reaction solution was extracted with (10 ml) ethyl acetate three times. The organic phases were combined, washed with saturated brine, dried over anhydrous sodium sulfate, filtered, concentrated, and purified by thin-layer chromatography (developing solvent: DCM:MeOH=30:1) to obtain Compound 166-6 as a yellow solid (10 mg, 57%).

LCMS:(ESI) m/z 478 (M+H) +.

Compound 166-6 (10 mg, 0.02 mmol, 1.0 eq.) was dissolved in DCM (3.0 ml), and then the compound TFA (1.0 mL) was added. After the addition was completed, the reaction solution was purged with nitrogen three times, and reacted under stirring at 25°C for 2.0 hours. TLC showed no remaining raw materials, and the conversion of most of raw materials into a product. The reaction solution was extracted with (10 ml) ethyl acetate three times. The organic phases were combined, washed with saturated brine, dried over anhydrous sodium sulfate, filtered, concentrated, and purified by preparative high performance liquid chromatography to obtain Compound 166 as a white solid (2.0 mg, purity 99%, yield 28%). LCMS:(ESI) m/z 394, (M+H) +; $^1$H NMR (400 MHz, $CD_3OD$) δ 9.38 (d, J = 11.6 Hz, 1H), 8.80 (s, 1H), 7.98 - 7.85 (m, 4H), 7.56 (d, J = 14.3 Hz, 1H), 7.31 (d, J = 9.7 Hz, 1H), 7.21 - 7.11 (m, 2H), 6.58 (d, J = 6.2 Hz, 1H), 4.46 (s, 1H), 4.01 (s, 3H), 1.94 (d, J = 6.9 Hz, 3H).

Example 168

6-methyl-5-(1-((3-(1-methyl-1H-pyrazol-4-yl)-1H-indazol-5-yl)oxy)ethyl)cinnolin e

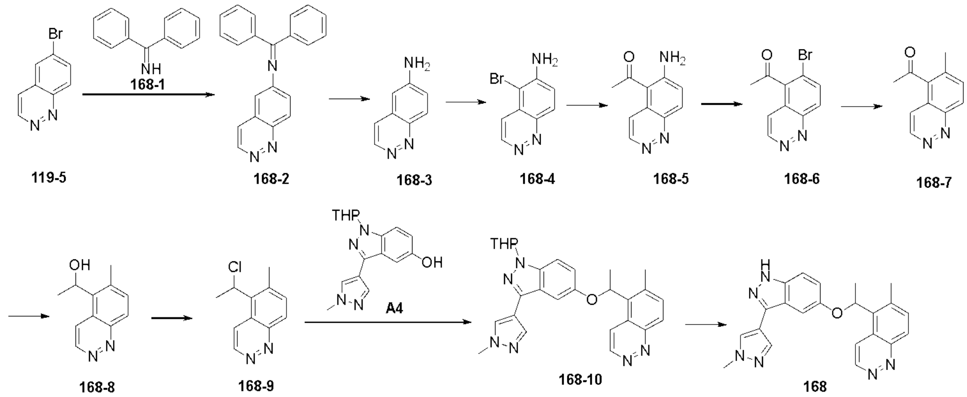


119-5 168-2 168-3 168-4 168-5 168-6 168-7

168-8 168-9 168-10 168

At room temperature, Compound 119-5 (905 mg, 4.33 mmol, 1.0 eq.), Compound 168-1 (942 mg, 5.2 mmol, 1.2 eq.), methanesulfonato(2-di-t-butylphosphino-2',4',6'-triisopropyl-1,1'-biphenyl)(2'-amino-1,1'-biphen-2-yl)palladium(II) (625 mg, 0.65 mmol, 0.15 eq.), 4,5-bis(diphenylphosphino)-9,9-dimethyl-xanthene (376 mg, 0.65 mmol, 0.15 eq.) and cesium carbonate (2.8 g, 8.66 mmol, 2.0 eq.) were added successively to dioxane (16.0 mL). The reaction mixture was stirred at 100°C for 3 hours. The reaction solution was cooled to room temperature, and concentrated under reduced pressure. The resulting residue was purified by silica gel column chromatography (eluent: (dichloromethane:methanol=4:1)/dichloromethane=0-20%) to obtain Compound 168-2 (1.107 g, yield 82.7%).

At room temperature, concentrated hydrochloric acid (12M, 12.0 mL, 144 mmol, 40.2 eq.) was slowly added to a solution of Compound 168-2 (1107 mg, 3.58 mmol, 1.0 eq.) in THF (12.0 mL). The reaction mixture was stirred at room temperature for 1 hour. Water (30.0 mL) was added, and then the reaction mixture was extracted with ethyl acetate (20.0 mL × 2). The organic phase was discarded. Aqueous sodium bicarbonate solution was added to the aqueous phase to adjust the pH to >7, and then the aqueous phase was extracted with DCM/i-PrOH=85:15 (30.0 mL × 3). The organic phases were combined, washed with saturated brine (20.0 mL), dried over anhydrous sodium sulfate, filtered, and concentrated under reduced pressure. The resulting residue was purified by silica gel column chromatography (eluent: (dichloromethane:methanol=4:1)/dichloromethane=0-30%) to obtain Compound 168-3 (502 mg, yield 96.6%).

At room temperature, NBS (647 mg, 3.63 mmol, 1.05 eq.) was slowly added to a mixed solution of Compound 168-3 (502 mg, 3.46 mmol, 1.0 eq.) in acetonitrile (8.0 mL) and DMF (8.0 mL). The reaction mixture was stirred at room temperature for 3 hours. Water (30.0 mL) was added to the reaction solution, and then the reaction solution was extracted with EA (20.0 mL × 2). The organic phases were combined, washed with saturated brine (20.0 mL), dried over anhydrous sodium sulfate, filtered, and concentrated under reduced pressure. The resulting residue was purified by silica gel column chromatography (eluent: (dichloromethane:methanol=4:1)/dichloromethane=0-21.6%) to obtain Compound 168-4 (680 mg, yield 87.8%).

At room temperature, Compound 168-4 (680 mg, 3.03 mmol, 1.0 eq.), tributyl(1-ethoxyvinyl)tin (1644 mg, 4.55 mmol, 1.5 eq.) and $Pd(PPh_3)_4$ (700 mg, 0.606 mmol, 0.2 eq.) were added successively to dioxane (20.0 mL). The reaction mixture was stirred at 110°C for 16 hours under nitrogen protection. The reaction solution was cooled to room temperature, and aqueous KF solution (20.0 mL) was added. The reaction solution was stirred at room temperature for 1 hour. The reaction solution was filtered, and the filtrate was extracted with ethyl acetate (20.0 mL × 2). The organic phases were combined, dried over anhydrous sodium sulfate, filtered, and concentrated under reduced pressure. The resulting residue was dissolved in THF (10.0 mL), and 3N HCl (5.0 mL) was slowly added. The reaction solution was stirred at room temperature for 40 minutes, saturated aqueous sodium bicarbonate solution was added to adjust the pH to >7, and then the reaction solution was extracted with ethyl acetate (20.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-19%) to obtain Compound 168-5 (403 mg, yield 71%).

At room temperature, t-BuONO (296 mg, purity 90%, 2.58 mmol, 1.2 eq.) was slowly added to a solution of Compound 168-5 (403 mg, 2.15 mmol, 1.0 eq.) and $CuBr_2$ (577 mg, 2.58 mmol, 1.2 eq.) in $CH_3CN$ (25.0 mL). The reaction solution was stirred at room temperature for 16 hours. The reaction solution was concentrated under reduced pressure, and the resulting residue was purified by silica gel column chromatography (eluent: ethyl acetate/dichloromethane=0-10%) to obtain Compound 168-6 (437 mg, yield 80.9%).

At room temperature, Compound 168-6 (145 mg, 0.58 mmol, 1.0 eq.), the compound methylboronic acid (290 mg, purity 50%, 1.16 mmol, 2.0 eq.), $Pd(dppf)C1_2$ (83 mg, 0.116 mmol, 0.2 eq.) and potassium carbonate (239 mg, 1.74 mmol, 3.0 eq.) were added successively to dioxane /$H_2O$ (6 mL/1.5 mL). The reaction mixture was stirred at 95°C for 16 hours. The reaction solution was cooled to room temperature, water (10.0 mL) was added, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-18%) to obtain Compound 168-7 (35 mg, yield 32.5%).

At 0°C, $NaBH_4$ (8 mg, 0.207 mmol, 1.1 eq.) was slowly added to a solution of Compound 168-7 (35 mg, 0.188 mmol, 1.0 eq.) in methanol (3.0 mL). The reaction mixture was stirred at room temperature for 1 hour. The reaction solution was cooled to 0°C, slowly poured into aqueous $NH_4Cl$ solution (10.0 mL), and extracted with EA (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane: methanol=4:1)/dichloromethane=0~ 25%) to obtain Compound 168-8 (21 mg, yield 59%).

At 0°C, thionyl chloride (34 mg, 0.29 mmol, 3.0 eq.) was slowly added to a solution of Compound 168-8 (18 mg, 0.096 mmol, 1.0 eq.) in DCM (2.0 mL). The reaction solution was warmed up to room temperature, and stirred for 1 hour. The reaction was concentrated under reduced pressure, and the resulting residue was Compound 168-9 was directly used for the next reaction step without purification.

At room temperature, Compound 168-9 (19.8 mg, 0.096 mmol, 1.0 eq.), Compound A4 (28.5 mg, 0.096 mmol, 1.0

eq.) and cesium carbonate (93 mg, 0.29 mmol, 3.0 eq.) were added successively to DMF (1.5 mL). The reaction mixture was stirred at 60°C for 16 hours. The reaction solution was cooled to room temperature, water (10.0 mL) was added, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (eluent: (dichloromethane:methanol=4:1)/dichloromethane=0-19%) to obtain Compound 168-10 (18 mg, purity 57.5%, total yield 23.1% for two steps).

At room temperature, TFA (1.0 mL) was slowly added to a solution of Compound 168-10 (18 mg, purity 57.5%,0.022 mmol, 1.0 eq.) in DCM (3.0 mL). The reaction mixture was stirred at room temperature for 3 hours. The reaction mixture was concentrated under reduced pressure, and the resulting residue was dissolved in DCM (10.0 mL), aqueous sodium bicarbonate solution was added to adjust the pH to >7, and extracted with DCM (10.0 mL x 2). The organic phases were combined, washed with saturated brine (10.0 mL), dried over anhydrous sodium sulfate, filtered, and concentrated under reduced pressure. The resulting residue was purified by thin-layer chromatography (developping agent: petroleum ether: ethyl acetate (developping agent: dichloromethane:methanol=12:1) to obtain 168 (6 mg, yield 70.7%, purity 89.18%). LCMS (ESI) m/z=385.2 (M+H)+; $^1$H NMR (400 MHz, DMSO) δ 12.73 (s, 1H), 9.37 (d, J = 6.3 Hz, 1H), 8.92 (t, J = 7.9 Hz, 1H), 8.26 (t, J = 8.0 Hz, 1H), 7.97 (d, J = 5.5 Hz, 1H), 7.82 - 7.75 (m, 1H), 7.71 (s, 1H), 7.33 (t, J = 10.0 Hz, 1H), 7.13 (d, J = 8.7 Hz, 1H), 7.05 (dt, J = 11.7, 5.8 Hz, 1H), 6.41 - 6.31 (m, 1H), 3.92 (s, 3H), 2.76 (s, 3H), 1.85 (d, J = 6.6 Hz, 3H).

Example 169

3-(4-(5-(1-(7-chloroquinoxalin-5-yl)ethoxy)-1H-indazol-3-yl)-1H-pyrazol-1-yl)aze tidine-1-carboxamide

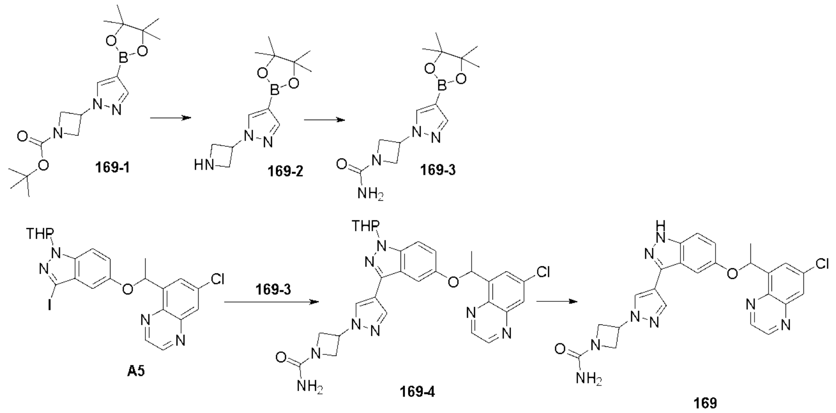

169-1 169-2 169-3
A5 169-4 169

At room temperature, trifluoroacetic acid (7.01 g, 61.52 mmol) was added dropwise to a solution of Compound 169-1 (1.07 g, 3.076 mmol) in dichloromethane (15.4 mL). The resulting reaction solution was stirred for 12 hours. LCMS showed the generation of a product. The reaction mixture was concentrated to dryness under reduced pressure, and anhydrous ethanol (10.0 mL) was added to the resulting residue, and concentrated to dryness under reduced pressure again. This operation was repeated twice again, and the resulting residue was dried under vacuum to obtain a crude product Compound 169-2 as a light brown solid (1.27 g, purity: 83.06%). LCMS (ESI) m/z 250.2, $(M+H)^+$.

At room temperature, the crude product Compound 169-2 (0.660 g, 1.60 mmol (a theoretical amount)) and triethylamine (0.648 g, 6.40 mmol) were added to tetrahydrofuran (8.0 mL), and then trimethylsilyl isocyanate (0.346 g, 2.40 mmol) was slowly added dropwise. The resulting mixture was stirred for 3 hours. LCMS showed the generation of a product. The reaction mixture was concentrated to dryness under reduced pressure, and dried under vacuum to obtain a crude product Compound 169-3 as a light brown waxy solid (1.024 g, purity: 70.36%). LCMS (ESI) m/z 293.2, $(M+H)^+$.

Compound A5 (35 mg, 0.06 mmol, 1.0 eq.) and Compound 169-3 (28 mg, 0.09 mmol, 1 eq.) were dissolved in dioxane (2 mL) and water (0.2 mL), and $Cs_2CO_3$ (63 mg, 0.19 mmol, 2 eq.) and Pd(dppf)$Cl_2$ (10 mg, 0.01 mmol, 0.2 eq) were added, and reacted at 90°C for 3 hours. LCMS showed the complete reaction of Compound 7 and the generation of a product. The reaction mixture was filtered, dried by rotary drying, and purified by thin-layer chromatography (developping agent: petroleum ether:ethyl acetate=1:8) to obtain Compound 169-4 as a colorless oil liquid (30 mg, yield 80%). LCMS (ESI) m/z 573.2, (M+H)+.

Compound 169-4 (30 mg, 0.05 mmol, 1.0 eq.) was dissolved in DCM (2 mL), and TFA (1 mL) was added, and reacted at 25°C for 3 hours. LCMS showed the complete reaction and the generation of a product. The reaction solution was subjected to rotary drying to obtain a crude which was purified by preparative high performance liquid chromatography

to obtain Compound 169 as an off-white solid (10.7 mg, yield41 %). LCMS (ESI) m/z 489.2, (M+H)+); $^1$H NMR (400 MHz, DMSO) δ 9.13 - 9.09 (m, 2H), 8.18 (d, J = 8.4 Hz, 1H), 8.12 (t, J = 12.0 Hz, 1H), 7.94 (t, J = 13.2 Hz, 1H), 7.79 (d, J = 14.0 Hz, 1H), 7.41 (t, J = 7.2 Hz, 1H), 7.29 (t, J = 8.8 Hz, 1H), 7.12 (dt, J = 7.6, 4.0 Hz, 1H), 6.73 - 6.66 (m, 1H), 5.31 - 5.12 (m, 1H), 4.40 - 4.24 (m, 2H), 4.14 (dd, J = 22.8, 14.6 Hz, 2H), 1.72 (t, J = 20.4 Hz, 3H).

The following compound was synthesized according to the the route of Example 169.

| Example | Name | Structure | LC-MS $(M+H)^+$ |
|---|---|---|---|
| 239 | 7-chloro-5-(1-((3-(1-(oxetan-3-yl)-1H-pyrazol-4-yl)-1H-indazol-5-yl)oxy)ethyl)quinoxaline | 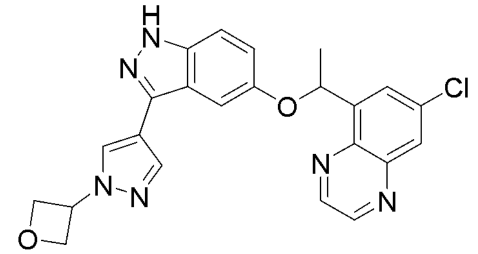 | 447.1 |

Example 170

N,N-dimethyl-3-(4-(5-(1-(quinoxalin-5-yl)ethoxy)-1H-indazol-3-yl)-1H-pyrazol-1-yl)cyclobutan-1-amine

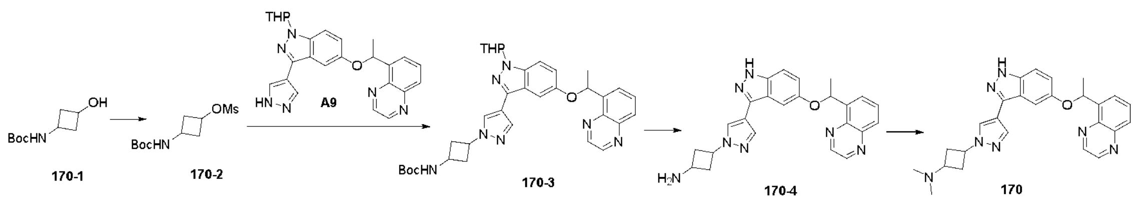

Compound 170-1 (100 mg, 0.53 mmol, 1.0 eq) and $Et_3N$ (162 mg, 1.6 mmol, 3.0 eq) were added to DCM(2.0 ml), and MsCl (67 mg, 0.59 mmol, 1.1 eq) was added at 0°C, and reacted under stirring at room temperature for 3 hours. The reaction solution was poured into NaCl (10.0 mL), and extracted with DCM (10.0 mL × 2). The organic phases were combined, washed with saturated brine (10.0 mL), dried over anhydrous sodium sulfate, filtered, and concentrated under reduced pressure to obtain a crude Compound 170-2 (150 mg, as a white solid ). LCMS:(ESI) m/z 166.2, (M-100+H)+.

At room temperature, Compound 170-2 (60 mg, 0.13 mmol, 1.0 eq.), CompoundA9 (150 mg, 0.52 mmol, 4.0 eq.) and cesium carbonate (266 mg, 0.82 mmol, 6.0 eq.) were added successively to DMF (2.0 mL), and stirred at 80°C for 2 hours. The reaction solution was poured into aqueous NaCl solution (10.0 mL), and extracted with EtOAc (10.0 mL × 2). The organic phases were combined, washed with saturated brine (10.0 mL), dried over anhydrous sodium sulfate, filtered, and concentrated under reduced pressure. The resulting residue was purified by thin-layer chromatography (developing solvent: petroleum ether:ethyl acetate = 1/1) to obtain Compound 170-3 (82 mg, yield 98.7%, a light yellow solid). LCMS:(ESI) m/z 610.2, (M+H)+.

At room temperature, 4M hydrochloric acid in dioxane (2.0 mL) was slowly added to a mixed solution of Compound 170-3 (82 mg, 0.13 mmol) in DCM (2.0 mL) and MeOH (2.0 mL), and stirred at room temperature for 1 hour. The reaction solution was concentrated under reduced pressure and then extracted with EtOAc (15.0 mL × 2) and aqueous $Na_2CO_3$ solution (15.0 mL). The organic phases were combined, washed with saturated brine (10.0 mL), dried over anhydrous sodium sulfate, filtered, and concentrated under reduced pressure to obtain Compound 170-4 (45 mg, yield 78.6%, a light yellow solid).

LCMS:(ESI) m/z 426.2, (M+H)+.

Compound 170-4 (40 mg, 0.094 mmol, 1.0 eq) was dissolved in MeOH (2.0 ml), and an aqueous solution of 40% formaldehyde (35 mg, 0.47 mmol, 5.0 eq), AcOH (0.56 mg, 0.0094 mmol, 0.1 eq) and $NaCNBH_3$ (13 mg, 0.21 mmol, 2.2 eq) were added successively, and reacted at room temperature for 4 hours. The reaction solution was poured into aqueous NaCl solution (10.0 mL), and extracted with EtOAc (10.0 mL x 2). The organic phases were combined, washed with saturated brine (10.0 mL), dried over anhydrous sodium sulfate, filtered, and concentrated under reduced pressure. The resulting residue was purified by preparative high performance liquid chromatography to obtain 170 (10.1 mg, yield 23.5%, purity 99.2%, a light yellow solid). LCMS:(ESI) m/z 454.3, (M+H)+; $^1$HNMR (400 MHz, DMSO-d6) δ 12.75 (s, 0.3H), 9.15 -

9.06 (m, 2H), 8.19 (s, 1H), 8.07 - 7.97 (m, 3H), 7.91 - 7.83 (m, 1H), 7.66 (d, J = 9.2 Hz, 1H), 7.39 (d, J = 9.0 Hz, 1H), 7.22 (d, J = 2.3 Hz, 1H), 7.11 (dd, J = 9.0, 2.2 Hz, 1H), 6.72 (q, J = 6.3, 5.8 Hz, 1H), 4.87 (p, J = 7.0 Hz, 1H), 2.99 (q, J = 6.3 Hz, 1H), 2.64 - 2.55 (m, 1H), 2.47 (d, J = 6.8 Hz, 2H), 2.19 (d, J = 6.4 Hz, 6H), 1.82 - 1.70 (m, 3H).

Example 173

(3-(4-(5-(1-(7-chloroquinoxalin-5-yl)ethoxy)-1H-indazol-3-yl)-1H-pyrazol-1-yl)propyl)(imino)(methyl)-sulfanone

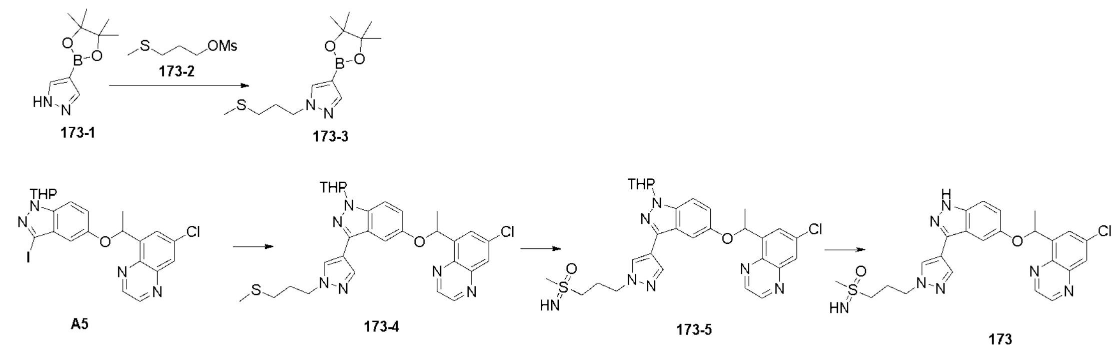


173-1 173-3

A5 173-4 173-5 173

Compound 173-1 (70 mg, 0.13 mmol, 1.0 eq.) and Compound 173-2 (70 mg, 0.386, mmol, 1.5 eq.) were dissolved in DMF, and $Cs_2CO_3$ (170 mg, 0.52 mmol, 4 eq.) was added, and reacted at 50°C for 1.2 hours. LCMS showed the complete reaction of 173-1 and the generation of a product. The reaction mixture was extracted with water and EtOAc. The organic phase was separated and dried by rotary drying to obtain Compound 173-3 as a yellow oil liquid (70 mg, purity 60%). LCMS (ESI) m/z 283.2, (M+H)+).

Compound A5 (70 mg, 0.06 mmol, 1.0 eq) and Compound 173-3 (19 mg, 0.09 mmol, 1.5 eq) were dissolved in dioxane (2mL) and $H_2O$ (2mL), and then $Cs_2CO_3$ (64 mg, 0.19 mmol, 3 eq) and Pd(dppf)$Cl_2$ (19.1 mg, 0.026 mmol, 0.2 eq) were added, purged with $N_2$ three times, and stirred at 90°C for 3 hours. LCMS showed the complete reaction of Compound A5. The reaction solution was filtered and subjected to rotary drying, and the crude was purified by thin-layer chromatography (developping agent: petroleum ether:ethyl acetate=1:9) to obtain Compound 173-4 (60 mg, yield: 81%). LCMS (ESI) m/z 563.2, (M+H)+.

Compound 173-4 (50 mg, 0.08 mmol, 1.0 eq) and the compound ammonium carbamate (20.79 mg, 0.26 mmol, 3 eq) were added to a mixed solution of t-BuOH (1.5 mL) and DCM (0.5mL), and then iodobenzene diacetate (85.8 mg, 0.27 mmol, 3 eq) was added to the solution, purged with $N_2$ three times, and stirred at 25°C for 3 hours. LCMS showed the complete reaction of Compound 173-4. 20 mL of water was added, and then the reaction solution was extracted with ethyl acetate, separated and dried by rotary drying to obtain a liquid as a yellow oil. The crude was subjected to thin-layer chromatography (developing solvent: DCM:MeOH=10:1) to obtain Compound 173-5 (40 mg, yield: 68%). LCMS (ESI) m/z 594.2, (M+H)+.

Compound 173-5 (40 mg, 0.07 mmol, 1.0 eq.) was dissolved in DCM (2 mL), and TFA (1 mL) was added, and reacted at 25°C for 3 hours. LCMS showed the complete reaction of Compound 173-5 and the generation of a product. The reaction solution was subjected to rotary drying to obtain a crude which was purified by preparative high performance liquid chromatography to obtain Compound 173 (6.7 mg, yield 15%) as a white solid. LCMS (ESI) m/z 510.2, (M+H)+); $^1$H NMR (400 MHz, DMSO) δ 12.79 (d, J = 19.2 Hz, 1H), 9.13 (t, J = 8.0 Hz, 3H), 8.24 - 8.05 (m, 2H), 7.95 (t, J = 9.6 Hz, 1H), 7.67 (d, J = 13.3 Hz, 1H), 7.41 (t, J = 9.2 Hz, 1H), 7.26 (d, J = 12.2 Hz, 1H), 7.11 (dt, J = 22.4, 11.2 Hz, 1H), 6.75 - 6.65 (m, 1H), 4.40 - 4.17 (m, 2H), 3.11 (s, 2H), 2.99 (s, 6H), 2.40 - 2.17 (m, 2H), 1.72 (t, J = 19.2 Hz, 6H).

The following compound was synthesized according to the the route of Example 173.

| Example | Name | Structure | LC-MS (M+H)+ | 1H NMR |
|---|---|---|---|---|
| 185 | (3-(4-(5-(1-(7-fluoroquinoxalin-5-yl)ethoxy)-1H-indazol-3-yl)-1H-pyrazol-1-yl)propyl)(imin o)(methyl)-sulfanone | 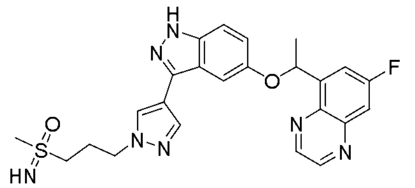 | 494.2 | $^1$H NMR (400 MHz, DMSO) δ 12.81 (s, 1H), 9.12 (s, 2H), 8.09 (s, 1H), 7.86 (ddd, J = 21.4, 9.2, 2.8 Hz, 2H), 7.68 (s, 1H), 7.41 (d, J = 9.2 Hz, 1H), 7.28 (s, 1H), 7.13 (dd, J = 8.8, 2.0 Hz, 1H), 6.71 (t, J = 6.0 Hz, 1H), 4.31 (t, J = 6.8 Hz, 2H), 3.77 (s, 1H), 3.11 - 3.03 (m, 2H), 2.94 (s, 3H), 2.33 - 2.23 (m, 2H), 1.76 (d, J = 6.4 Hz, 3H). |

Example 175

2-amino-1-(3-(4-(5-(1-(quinoxalin-5-yl)ethoxy)-1H-indazol-3-yl)-1-pyrazol-1-yl)a zetidin-1-yl)ethan-1-one

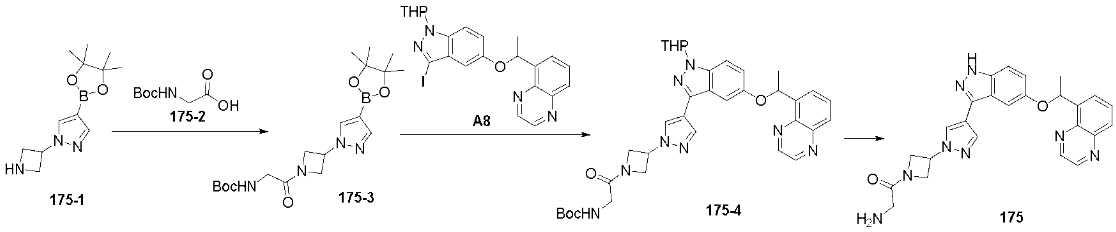


Compound 175-1 (80 mg, 0.22 mmol, 1.0 eq) and Compound 175-2 (59 mg, 0.34 mmol, 1.5 eq.) were dissolved in DMF (3 mL), and TEA (140 mg, 1 mmol, 5 eq.) and BOP (248.6 mg, 0.56 mmol, 2.5 eq.) were added at 25°C, and reacted under stirring at 25°C for 1 hour. LCMS showed the complete reaction of Compound 175-1. 30 ml of water was added, and then the reaction solution was extracted with EA, and washed with saturated brine. The organic phase was concentrated to obtain Compound 175-3 as a yellow oil liquid (80 mg, crude purity: 60%). LCMS (ESI) m/z 407.2, (M+H)+.

Compound 175-3 (80 mg, crude) and Compound A8 (80 mg, 0.09 mmol, 1 eq) were dissolved in dioxane (2mL) and $H_2O$ (2mL), and then $Cs_2CO_3$ (93 mg, 0.29 mmol, 3 eq) and Pd(dppf)$Cl_2$ (14 mg, 0.019 mmol, 0.2 eq) were added, purged with $N_2$ three times, and stirred at 90°C for 2 hours. LCMS showed the complete reaction of Compound 175-3. The reaction solution was filtered and subjected to rotary drying, and the crude was purified by thin-layer chromatography (developping agent: petroleum ether:ethyl acetate=10:1) to obtain Compound 175-4 (50 mg, yield: 79.8%). LCMS (ESI) m/z 653.2, (M+H)+.

Compound 175-4 (25 mg, 0.08 mmol, 1.0 eq.) was dissolved in DCM (2 mL), and TFA (1 mL) was added, and reacted at 25°C for 3 hours. LCMS showed the complete reaction of Compound 175-4 and the generation of a product. The reaction solution was subjected to rotary drying to obtain a crude which was purified by preparative high performance liquid chromatography to obtain Compound 175 (27.1 mg, yield 76%) as a white solid. LCMS (ESI) m/z 469.2, (M+H)+; $^1$H NMR (400 MHz, DMSO) δ 9.11 (d, J = 12.4 Hz, 2H), 8.30 (s, 1H), 8.22 (d, J = 7.2 Hz, 1H), 8.07 - 7.98 (m, 2H), 7.87 (t, J = 7.6 Hz, 1H), 7.77 (d, J = 7.2 Hz, 1H), 7.39 (d, J = 9.2 Hz, 1H), 7.27 (s, 1H), 7.10 (dd, J = 9.2, 2.0 Hz, 1H), 6.73 (t, J = 6.4 Hz, 1H), 5.36 (s, 1H), 4.65 (t, J = 8.4 Hz, 1H), 4.44 (dd, J = 19.6, 11.3 Hz, 2H), 4.28 - 4.21 (m, 1H), 3.34 (s, 2H), 1.75 (d, J = 6.4 Hz, 3H).

The following compounds were synthesized according to the the route of Example 175.

| Example | Name | Structure | LC-MS (M+H)+ | $^1$H NMR |
|---|---|---|---|---|
| 177 | 3-(4-(5-(1-(quinoxalin-5-yl)ethoxy)-1H-indazol-3-yl)-1H-pyrazol-1-yl)azetidine-1-carboximidamide | 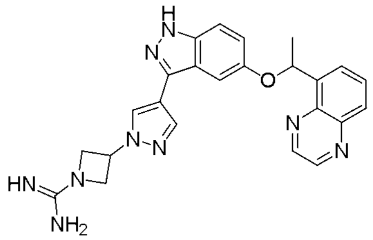 | 454 | |
| 178 | N,N-dimethyl-3-(4-(5-(1-(quinoxalin-5-yl)ethoxy)-1H-indazol-3-yl)-1H-pyrazol-1-yl) azetidine-1-carboxamide | 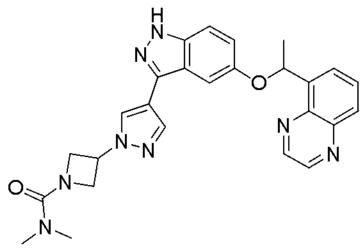 | 483.2 | $^1$HNMR(400 MHz, DMSO) δ 12.80 (s, 1H), 9.11 (dd,J = 16.6, 1.7 Hz, 2H), 8.17 (s, 1H), 8.08 - 7.98 (m, 2H), 7.87 (t,J = 7.8 Hz, 1H), 7.76 (s, 1H), 7.39 (d,J = 9.0 Hz, 1H), 7.27 (s, 1H), 7.10 (dd,J = 9.0, 2.1 Hz, 1H), 6.74 (d,J = 6.4 Hz, 1H), 5.25 (s, 1H), 4.37 (t,J = 9.3 Hz, 2H), 4.30 - 4.16 (m, 2H), 2.85 (s, 6H), 1.75 (d,J = 6.3 Hz, 3H). |

Example 180

3-oxo-3-(3-(4-(5-(1-(quinoxalin-5-yl)ethoxy)-1H-indazol-3-yl)-1H-pyrazol-1-yl)az etidin-1-yl)propionitrile

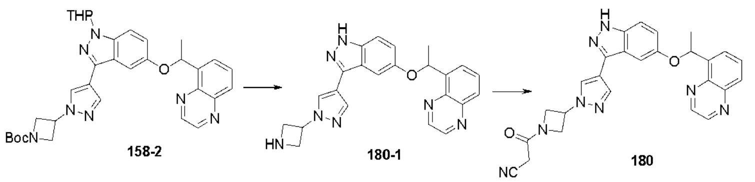

158-2 180-1 180

Compound 158-2 (80 mg, 0.13 mmol, 1.0 eq.) was dissolved in DCM (2 mL), and TFA (1 mL) was added, and reacted at 25°C for 3 hours. LCMS showed the complete reaction of Compound 158-2 and the generation of a product. The reaction solution was subjected to rotary drying to obtain Compound 180-1 (50 mg, yield81%) as an off-white solid. LCMS (ESI) m/z 412.2, (M+H)+.

The compound cyanoacetic acid (50 mg, 0.59 mmol, 1.0 eq) and Compound 180-1 (25 mg, crude.) were dissolved in DMF (2mL), and TEA (356 mg, 3.52 mmol, 6 eq.) and BOP (260 mg, 0.28 mmol, 1.2 eq.) were added at 25°C, and reacted under stirring at 25°C for 1 hour. LCMS showed the complete reaction. 30 ml of water was added, and then the reaction solution was extracted with EA, and washed with saturated brine. The organic phase was concentrated to obtain a liquid as a yellow oil, which was purified by thin-layer chromatography (developping agent: DCM:MeOH=10:1) to obtain Compound 180 (4 mg, purity: 95.53%). LCMS (ESI) m/z 479.2 (M+H)+; $^1$H NMR (400 MHz, DMSO) δ 12.74 (s, 1H), 9.09 (dd, J = 17.2, 11.2 Hz, 2H), 8.22 (dd, J = 10.8, 6.8 Hz, 1H), 8.08 - 7.98 (m, 2H), 7.91 - 7.82 (m, 1H), 7.76 (dd, J = 11.2, 8.0 Hz, 1H), 7.38 (dd, J = 9.6, 5.6 Hz, 1H), 7.29 - 7.24 (m, 1H), 7.10 (dd, J = 11.2, 6.8 Hz, 1H), 6.78 - 6.69 (m, 1H), 5.33 (d, J = 18.4 Hz, 1H), 4.73 - 4.63 (m, 1H), 4.55 - 4.38 (m, 2H), 4.24 (d, J = 18.4 Hz, 2H), 3.86 (t, J = 7.8 Hz, 2H), 1.73 (t, J = 12.8 Hz, 3H).

The following compounds were synthesized according to the the route of Example 180.

| Example | Name | Structure | LC-MS (M+H)+ | 1H NMR |
|---|---|---|---|---|
| 188 | 2-methoxy-1-(3-(4-(5-(1-(quinoxalin-5-yl)ethoxy)-1H-indazol-3-yl)-1H-pyrazol-1-yl)azetidin-1-yl)ethan-1-one | 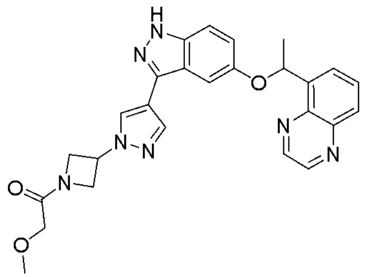 | 484.2 | $^1$H NMR(400 MHz, DMSO) δ 12.80 (s, 1H), 9.11 (d,J = 13.9 Hz, 2H), 8.22 (d,J = 6.6 Hz, 1H), 8.03 (t,J = 8.5 Hz, 2H), 7.87 (t,J = 7.8 Hz, 1H), 7.78 (d,J = 6.2 Hz, 1H), 7.39 (d,J = 9.0 Hz, 1H), 7.28 (s, 1H), 7.10 (dd,J = 9.0, 2.0 Hz, 1H), 6.74 (q,J = 6.3 Hz, 1H), 5.44 - 5.26 (m, 1H), 4.69 (t,J = 8.8 Hz, 1H), 4.55 - 4.40 (m, 2H), 4.24 (dd,J = 9.9, 5.3 Hz, 1H), 4.02 (s, 2H), 1.75 (d,J = 6.3 Hz, 3H). |
| 189 | 1-(3-(4-(5-(1-(7-chloroquinolin-5-yl)ethoxy)-1H-indazol-3-yl)-1H-pyrazol-1-yl)azetidin-1-yl)-2-hydroxyethan-1-one | 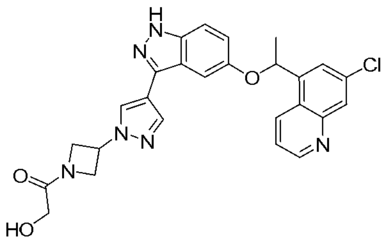 | 503.2 | $^1$H NMR(400 MHz, DMSO) δ 12.84 (s, 1H), 9.05 - 8.92 (m, 2H), 8.34 (d,J = 4.8 Hz, 1H), 8.01 (d,J = 1.9 Hz, 1H), 7.95 (d,J = 3.1 Hz, 1H), 7.84 (d,J = 2.0 Hz, 1H), 7.71 (d,J = 3.2 Hz, 1H), 7.47 - 7.35 (m, 2H), 7.15 (dd,J = 9.0, 2.0 Hz, 1H), 6.45 (q,J = 6.2 Hz, 1H), 5.34 (d,J = 5.4 Hz, 1H), 5.08 (s, 1H), 4.77 - 4.68 (m, 1H), 4.54 (dd,J = 15.3, 10.0 Hz, 1H), 4.48 - 4.39 (m, 1H), 4.27 (d,J = 6.2 Hz, 1H), 4.03 (s, 2H), 1.77 (d,J = 6.3 Hz, 3H). |
| 190 | 2-hydroxy-2-methyl-1-(3-(4-(5-(1-(quinoxalin-5-yl)ethoxy)-1H-indazol-3-yl)-1H-pyrazol-1-yl)azetidin-1-yl)propan-1-one | 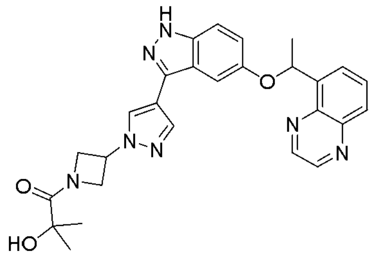 | 498.2 | $^1$H NMR (400 MHz, DMSO) δ 12.82 (s, 1H), 9.11 (d, J = 18.0 Hz, 2H), 8.17 (d, J = 7.8 Hz, 1H), 8.03 (t, J = 8.4 Hz, 2H), 7.87 (t, J = 8.0 Hz, 1H), 7.77 (d, J = 4.4 Hz, 1H), 7.39 (d, J = 8.8 Hz, 1H), 7.27 (s, 1H), 7.11 (dd, J = 9.0, 2.0 Hz, 1H), 6.74 (q, J = 6.0 Hz, 1H), 5.29 (dd, J = 17.0, 11.2 Hz, 2H), 4.90 (t, J = 8.8 Hz, 1H), 4.69 (dt, J = 10.8, 6.2 Hz, 1H), 4.39 (dt, J = 13.2, 5.4 Hz, 1H), 4.19 (ddd, J = 5.8, 5.4, 2.8 Hz, 1H), 1.75 (d, J = 6.4 Hz, 3H), 1.34 (d, J = 2.4 Hz, 6H). |
| 192 | 2-hydroxy-1-(3-(4-(5-(1-(7-methylquinolin-5-yl)ethoxy)-1H-indazol-3-yl)-1-pyrazol-1-yl)azetidin-1-yl)ethan-1-one | 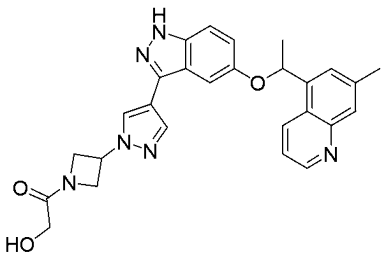 | 483.2 | $^1$HNMR (400 MHz, DMSO) δ 12.85 (s, 1H), 8.90 (d, J = 5.2 Hz, 3H), 8.28 (t, J = 8.8 Hz, 1H), 7.91 (d, J = 4.4 Hz, 1H), 7.71 (d, J = 17.2 Hz, 3H), 7.58 (s, 1H), 7.40 (d, J = 8.8 Hz, 1H), 7.34 (s, 1H), 7.12 (d, J = 10.8 Hz, 1H), 6.37 (d, J = 6.4 Hz, 1H), 5.28 (d, J = 42.8 Hz, 1H), 4.70 (d, J = 6.8 Hz, 1H), 4.61 - 4.37 (m, 1H), 4.30 - 4.11 (m, 1H), 4.03 (s, 2H), 2.49 (s, 3H), 1.76 (d, J = 6.4 Hz, 3H). |

(continued)

| Example | Name | Structure | LC-MS $(M+H)^+$ | $^1H$ NMR |
|---|---|---|---|---|
| 193 | 2-(methylamino)-1-(3-(4-(5-(1-(quinoxalin-5-yl)ethoxy)-1H-indazol-3-yl)-1H-pyrazol-1-yl)azetidin-1-yl) ethan-1-one | 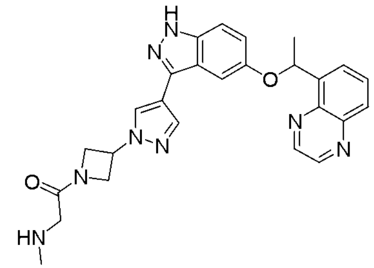 | 483.2 | $^1H$ NMR (400 MHz, $CD_3OD$) δ 9.05 (s, 1H), 8.96 (s, 1H), 7.99 (d, J = 19.0 Hz, 3H), 7.83 (s, 1H), 7.70 (d, J = 19.8 Hz, 1H), 7.39 (s, 1H), 7.18 (s, 1H), 7.11 (s, 1H), 6.78 (s, 1H), 5.34 (s, 1H), 4.73 (s, 1H), 4.59 (s, 2H), 4.42 (s, 1H), 3.82 (s, 2H), 2.72 (s, 3H), 1.77 (d, J = 6.4 Hz, 3H) |
| 205 | (1-aminocyclopropy 1)(3-(4-(5-(1-(quinoxalin-5-yl)ethoxy)-1H-indazol-3-yl)-1H-pyrazol-1-yl)azetidin-1-yl) methanone | 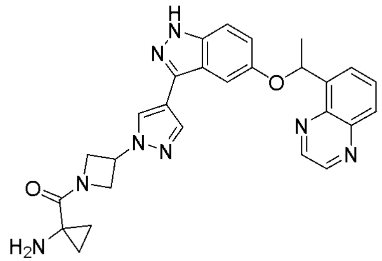 | 495.2 | $^1H$ NMR (400 MHz, DMSO) δ 12.81 (s, 1H), 9.12 (dd, J = 17.2, 1.6 Hz, 2H), 8.19 (s, 1H), 8.05 - 7.99 (m, 2H), 7.90 - 7.83 (m, 1H), 7.77 (s, 1H), 7.39 (d, J = 9.2 Hz, 1H), 7.27 (s, 1H), 7.11 (dd, J = 9.2, 2.4 Hz, 1H), 6.75 (q, J = 6.0 Hz, 1H), 5.34 - 5.23 (m, 1H), 4.95 (d, J = 83.2 Hz, 1H), 4.29 (d, J = 60.8 Hz, 1H), 2.47 - 2.29 (m, 1H), 1.75 (d, J = 6.4 Hz, 3H), 1.12 (d, J = 3.6 Hz, 2H), 0.74 (d, J = 3.6 Hz, 2H). |
| 206 | 1-(3-(4-(5-(1-(7-chloroquinolin-5-yl) ethoxy)-1H-indazol-3-yl)-1H-pyrazol-1-yl)azetidin-1-yl)-2-hydroxy-2-methylpropan-1-one | 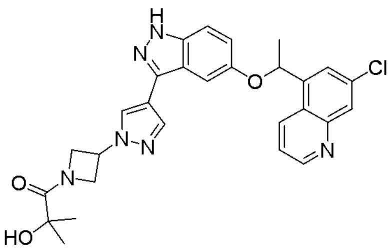 | 531.2 | $^1H$ NMR(400 MHz, dmso) δ 12.79 (s, 1H), 8.95 (d,J = 5.6 Hz, 2H), 8.25 (d,J = 6.3 Hz, 1H), 7.99 - 7.86 (m, 2H), 7.80 (s, 1H), 7.70 - 7.61 (m, 1H), 7.41 - 7.32 (m, 2H), 7.10 (dd,J = 9.0, 2.2 Hz, 1H), 6.40 (d,J = 6.5 Hz, 1H), 5.21 (d,J = 24.3 Hz, 2H), 4.90 - 4.81 (m, 1H), 4.67 (d,J = 4.3 Hz, 1H), 4.38 - 4.28 (m, 1H), 4.17 (d,J = 4.6 Hz, 1H), 1.73 (d,J = 6.3 Hz, 3H), 1.25 (d,J = 31.8 Hz, 6H). |
| 207 | 1-(3-(4-(5-(1-(7-chloroquinoxalin -5-yl) ethoxy)-1H-indazol-3-yl)-1H-pyrazol-1-yl)azetidin-1-yl)-2-hydroxyethan-1-one | 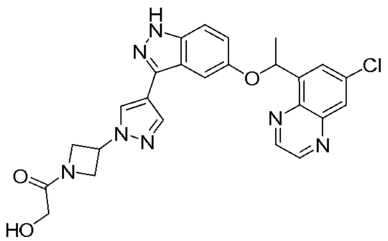 | 504.2 | $^1H$ NMR (400 MHz, dmso) δ 12.81 (s, 1H), 9.13 - 9.04 (m, 2H), 8.21 (d, J = 5.6 Hz, 1H), 8.10 (d, J = 2.4 Hz, 1H), 7.92 (d, J = 2.4 Hz, 1H), 7.78 (d, J = 4.4 Hz, 1H), 7.37 (d, J = 9.0 Hz, 1H), 7.28 (s, 1H), 7.08 (dd, J = 9.0, 2.0 Hz, 1H), 6.65 (dd, J = 12.6, 6.2 Hz, 1H), 5.36 - 5.26 (m, 1H), 5.03 (s, 1H), 4.66 (t, J = 8.8 Hz, 1H), 4.52 - 4.44 (m, 1H), 4.38 (t, J = 9.0 Hz, 1H), 4.19 (dd, J = 10.2, 5.0 Hz, 1H), 3.98 (s, 2H), 1.71 (d, J = 6.4 Hz, 3H). |

(continued)

| Example | Name | Structure | LC-MS (M+H)+ | $^1$H NMR |
|---|---|---|---|---|
| 208 | 2-amino-1-(3-(4-(5-(1-(6-(difluoromethyl) quinoline-5-yl)ethoxy)-1H-indazol-3-yl)-1H-pyrazol-1-yl)azetidin-1-yl)-2-methylpropan-1-one | 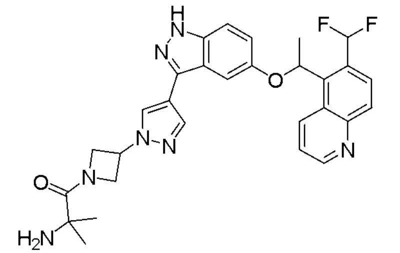 | 546.2 | $^1$H NMR (400 MHz, dmso) δ 12.80 (s, 1H), 9.32 (d, J = 8.6 Hz, 1H), 9.00 (d, J = 3.0 Hz, 1H), 8.21 (s, 1H), 8.14 (s, 1H), 8.07 - 7.90 (m, 3H), 7.82 (s, 1H), 7.71 (dd, J = 8.8, 4.0 Hz, 1H), 7.33 (d, J = 9.2 Hz, 1H), 7.22 (s, 1H), 7.05 (dd, J = 9.0, 2.2 Hz, 1H), 6.59 - 6.54 (m, 1H), 5.27 - 5.21 (m, 1H), 4.94 (s, 1H), 4.72 (s, 1H), 4.37 (s, 1H), 4.19 (s, 1H), 1.87 (d, J = 6.6 Hz, 3H), 1.30 (s, 6H). |
| 209 | 1-(3-(4-(5-(1-(7-chloroquinoxalin -5-yl) ethoxy)-1H-indazol-3-yl)-1H-pyrazol-1-yl)azetidin-1-yl)-2-hydroxy-2-methylpropan-1-one | 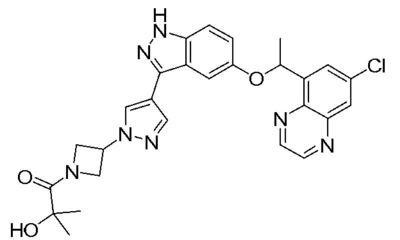 | 532.2 | $^1$H NMR (400 MHz, dmso) δ 12.81 (s, 1H), 9.13 - 9.05 (m, 2H), 8.18 (d, J = 6.6 Hz, 1H), 8.10 (d, J = 2.4 Hz, 1H), 7.92 (d, J = 2.2 Hz, 1H), 7.79 (d, J = 3.8 Hz, 1H), 7.38 (d, J = 9.0 Hz, 1H), 7.27 (s, 1H), 7.13 - 7.03 (m, 2H), 6.65 (dd, J = 12.8, 6.2 Hz, 1H), 5.21 (ddd, J = 16.4, 12.0, 6.8 Hz, 2H), 4.88 - 4.82 (m, 1H), 4.69 - 4.63 (m, 1H), 4.36 - 4.30 (m, 1H), 4.18 - 4.12 (m, 1H), 1.71 (d, J = 6.4 Hz, 3H), 1.29 (d, J = 1.6 Hz, 6H). |
| 210 | 2-amino-1-(3-(4-(5-(1-(7-chloroquinoxalin -5-yl)ethoxy)-1H-indazol-3-yl)-1-pyrazol-1-yl)azetidin-1-yl)-2-methylpropan-1-one | 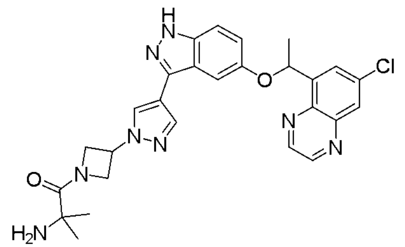 | 531.2 | $^1$H NMR (400 MHz, dmso) δ 12.81 (s, 1H), 9.08 (dd, J = 8.8, 1.8 Hz, 2H), 8.23 (d, J = 17.8 Hz, 2H), 8.10 (d, J = 2.4 Hz, 1H), 7.92 (d, J = 2.4 Hz, 1H), 7.79 (s, 1H), 7.38 (d, J = 9.0 Hz, 1H), 7.28 (d, J = 1.8 Hz, 1H), 7.08 (dd, J = 9.0, 2.2 Hz, 1H), 6.65 (q, J = 6.2 Hz, 1H), 5.25 (td, J = 7.8, 4.0 Hz, 1H), 4.92 (s, 1H), 4.71 (s, 1H), 4.34 (s, 1H), 4.17 (s, 1H), 1.71 (d, J = 6.4 Hz, 3H), 1.28 (s, 6H). |
| 211 | 2-amino-1-(3-(4-(5-(1-(7-chloroquinolin-5-yl)ethoxy)-1H-indazol-3-yl)-1-pyrazol-1-yl)azetidin-1-yl)-2-methylpropan-1-one | 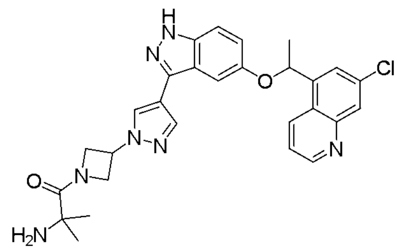 | 530.2 | $^1$H NMR(400 MHz, dmso) δ 12.80 (s, 1H), 8.97 - 8.92 (m, 2H), 8.26 (d,J = 14.7 Hz, 2H), 7.96 (d,J = 1.9 Hz, 1H), 7.92 (s, 1H), 7.80 (d,J = 2.0 Hz, 1H), 7.65 (dd,J = 8.6, 4.2 Hz, 1H), 7.35 (dd,J = 18.1, 10.4 Hz, 2H), 7.10 (dd,J = 9.0, 2.2 Hz, 1H), 6.40 (d,J = 6.4 Hz, 1H), 5.23 (d,J = 5.0 Hz, 2H), 4.93 (s, 1H), 4.72 (s, 1H), 4.33 (s, 1H), 4.17 (s, 1H), 3.93 (d,J = 57.1 Hz, 1H), 1.72 (d,J = 6.3 Hz, 3H), 1.26 (s, 6H). |

(continued)

| Example | Name | Structure | LC-MS (M+H)+ | 1H NMR |
|---|---|---|---|---|
| 212 | (3-(4-(5-(1-(7-chloroquinoxalin -5-yl) ethoxy)-1H-indazol-3-yl)-1H-pyrazol-1-yl)azetidin-1-yl)(1-(methylamino)cy clopropyl)metha none | 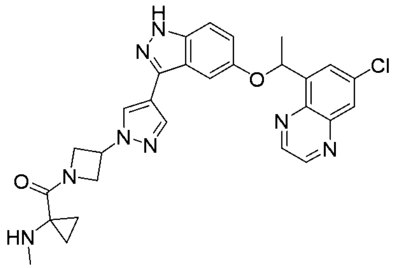 | 543.05 | 1H NMR (400 MHz, $CD_3OD$) δ 9.05 - 9.02 (m, 1H), 8.98 (d, J = 1.8 Hz, 1H), 8.02 (d, J = 4.0 Hz, 2H), 7.96 (d, J = 2.4 Hz, 1H), 7.78 (s, 1H), 7.41 (d, J = 9.0 Hz, 1H), 7.23 - 7.14 (m, 2H), 6.71 (d, J = 6.4 Hz, 1H), 5.28 (s, 1H), 4.61 (d, J = 53.2 Hz, 4H), 2.82 (s, 3H), 1.77 (d, J = 6.3 Hz, 3H), 1.73 (s, 2H), 1.48 (s, 2H). |
| 213 | 2-hydroxy-2-methyl-1-(3-(4-(5-(1-(7-methylquinolin-5-yl)ethoxy)-1H-indazol-3-yl)-1H-pyrazol-1-yl)azetidin-1-yl) propan-1-one | 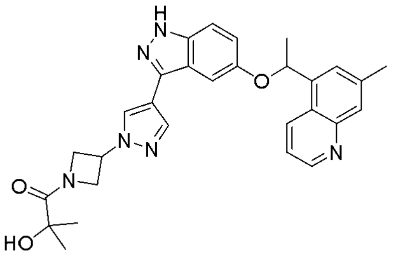 | 511.2 | 1H NMR (400 MHz, DMSO-d6) δ 12.82 (s, 1H), 8.96 - 8.87 (m, 2H), 8.27 (d, J = 8.3 Hz, 1H), 7.93 (d, J = 2.2 Hz, 1H), 7.79 - 7.68 (m, 2H), 7.59 (dd, J = 8.4, 4.4 Hz, 1H), 7.45 - 7.33 (m, 2H), 7.13 (dd, J = 9.0, 2.2 Hz, 1H), 6.38 (q, J = 6.3 Hz, 1H), 5.33 - 5.18 (m, 2H), 4.90 (t, J = 9.3 Hz, 1H), 4.78 - 4.68 (m, 1H), 4.38 (t, J = 9.2 Hz, 1H), 4.21 (m, 1H), 2.50 (s, 3H), 1.76 (d, J = 6.3 Hz, 3H), 1.40 - 1.28 (m, 6H). |
| 215 | 2-amino-2-methyl-1-(3-(4-(5-(1-(7-methylquinolin-5-yl)ethoxy)-1H-indazol-3-yl)-1H-pyrazol-1-yl)azetidin-1-yl) propan-1-one | 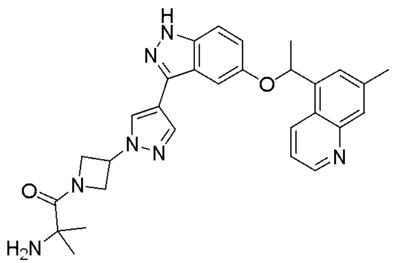 | 510.3 | 1H NMR (400 MHz, DMSO-d6) δ 12.84 (s, 0.2H), 8.90 (d, J = 7.9 Hz, 2H), 8.30 (d, J = 9.0 Hz, 2H), 7.94 (s, 1H), 7.78 - 7.66 (m, 2H), 7.64 - 7.52 (m, 1H), 7.46 - 7.31 (m, 2H), 7.13 (dd, J=9.0, 2.2 Hz, 1H), 6.37 (q, J = 6.3 Hz, 1H), 5.32 (d, J = 6.9 Hz, 1H), 4.96 (s, 1H), 4.75 (s, 1H), 4.42 (s, 1H), 4.24 (s, 1H), 2.50 (s, 3H), 1.76 (d, J = 6.3 Hz, 3H), 1.39 (s, 6H). |
| 216 | 2-methyl-2-(methylamino)-1-(3-(4-(5-(1-(quinoxalin-5-yl) ethoxy)-1H-indazol-3-yl)-1H-pyrazol-1-yl)azetidin-1-yl)propan-1-one | 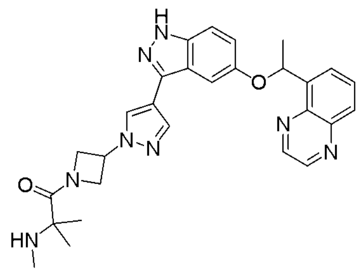 | 511.3 | 1H NMR (400 MHz, DMSO) δ 12.76 (d, J = 59.6 Hz, 1H), 9.09 (dt, J = 20.0, 10.0 Hz, 2H), 8.33 (s, 2H), 8.15 (d, J = 13.2 Hz, 1H), 8.07 - 7.98 (m, 2H), 7.86 (dd, J = 14.4, 6.4 Hz, 1H), 7.77 (d, J = 7.6 Hz, 1H), 7.39 (d, J = 9.2 Hz, 1H), 7.25 (d, J = 13.6 Hz, 1H), 7.10 (dt, J = 9.2, 4.4 Hz, 1H), 6.77 - 6.72 (m, 1H), 5.25 (d, J = 19.6 Hz, 1H), 4.71 (dd, J = 80.0, 42.8 Hz, 1H), 4.37 (s, 1H), 4.21 (s, 1H), 2.24 (s, 3H), 1.75 (d, J = 6.4 Hz, 3H), 1.24 (s, 6H). |

(continued)

| Example | Name | Structure | LC-MS (M+H)+ | 1H NMR |
|---|---|---|---|---|
| 217 | 2-hydroxy-2-methyl-1-(3-(4-(5-(1-(6-methylquinolin-5-yl)ethoxy)-1H-indazol-3-yl)-1H-pyrazol-1-yl)azetidin-1-yl) propan-1-one | 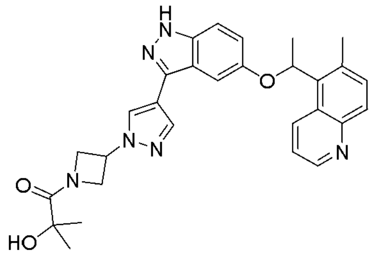 | 511.2 | 1H NMR (400 MHz, DMSO) δ 12.81 (s, 1H), 9.21 (t, J = 8.4 Hz, 1H), 8.83 (d, J = 2.8 Hz, 1H), 8.14 (d, J = 7.2 Hz, 1H), 7.83 (d, J = 8.8 Hz, 2H), 7.59 (dd, J = 8.8, 4.8 Hz, 2H), 7.34 (d, J = 9.2 Hz, 1H), 7.12 (s, 1H), 7.05 (dt, J = 9.2, 4.8 Hz, 1H), 6.31 (t, J = 11.6 Hz, 1H), 5.29 (d, J = 5.6 Hz, 2H), 4.91 (q, J = 9.6 Hz, 1H), 4.77 - 4.66 (m, 1H), 4.46 - 4.33 (m, 1H), 4.20 (d, J = 21.6 Hz, 1H), 2.71 (s, 3H), 1.86 (d, J = 6.4 Hz, 3H), 1.34 (d, J = 3.2 Hz, 6H). |
| 218 | 1-(3-(4-(5-(1-(quinoxalin-5-yl)ethoxy)-1H-indazol-3-yl)-1H-pyrazol-1-yl) azetidin-1-yl)ethan-1-one | 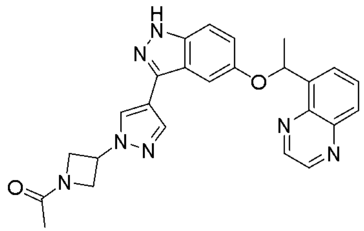 | 454.1 | 1H NMR (400 MHz, DMSO) δ 12.83 (s, 1H), 9.13 (dd, J = 9.6, 6.7 Hz, 2H), 8.23 (d, J = 6.4 Hz, 1H), 8.03 (dd, J = 11.2, 7.6 Hz, 2H), 7.88 (t, J = 7.6 Hz, 1H), 7.78 (d, J = 8.0 Hz, 1H), 7.40 (d, J = 9.2 Hz, 1H), 7.29 (s, 1H), 7.11 (dd, J = 9.2, 2.0 Hz, 1H), 6.75 (q, J = 6.0 Hz, 1H), 5.36 - 5.28 (m, 1H), 4.64 (t, J = 8.4 Hz, 1H), 4.47 (dd, J = 13.6, 8.0 Hz, 1H), 4.37 (t, J = 8.8 Hz, 1H), 4.18 (dd, J = 10.0, 5.2 Hz, 1H), 1.89 (s, 3H), 1.76 (d, J = 6.4 Hz, 3H). |
| 219 | cyclopropyl(3-(4-(5-(1-(quinoxalin-5-yl)ethoxy)-1H-indazol-3-yl)-1H-pyrazol-1-yl)azetidin-1-yl)methanone | 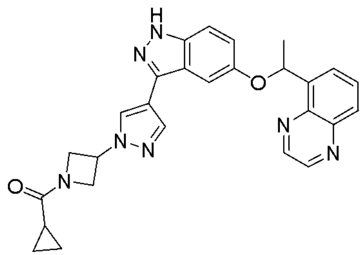 | 480.2 | 1H NMR (400 MHz, DMSO) δ 12.82 (s, 1H), 9.12 (d, J = 14.2 Hz, 2H), 8.23 (d, J = 5.2 Hz, 1H), 8.06 - 7.99 (m, 2H), 7.87 (t, J = 8.0 Hz, 1H), 7.79 (d, J = 6.4 Hz, 1H), 7.39 (d, J = 9.2 Hz, 1H), 7.28 (s, 1H), 7.10 (dd, J = 9.2, 2.0 Hz, 1H), 6.75 (q, J = 6.0 Hz, 1H), 5.36 (dt, J = 13.2, 4.0 Hz, 1H), 4.78 (t, J = 6.8 Hz, 1H), 4.58 (dd, J = 14.2, 6.0 Hz, 1H), 4.39 (t, J = 8.0 Hz, 1H), 4.19 (dd, J = 10.0, 5.2 Hz, 1H), 1.75 (d, J = 6.4 Hz, 3H), 1.69 - 1.62 (m, 1H), 0.80 (d, J = 8.0 Hz, 4H). |
| 221 | (1-hydroxycyclobut yl)(3-(4-(5-(1-(quinoxalin-5-yl)ethoxy)-1H-indazol-3-yl)-1H-pyrazol-1-yl)azetidin-1-yl) methanone | 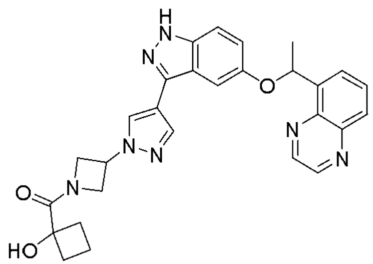 | 510.2 | 1H NMR (400 MHz, DMSO)δ 12.82 (s, 1H), 9.11 (dd, J = 19.8, 1.6 Hz, 2H), 8.17 (d, J = 7.6 Hz, 1H), 8.07 - 7.97 (m, 2H), 7.87 (t, J = 7.8 Hz, 1H), 7.77 (d, J = 2.5 Hz, 1H), 7.39 (d, J = 9.0 Hz, 1H), 7.27 (s, 1H), 7.11 (dd, J = 9.0, 2.0 Hz, 1H), 6.75 (q, J = 6.0 Hz, 1H), 5.88 (s, 1H), 5.30 (d, J = 5.2 Hz, 1H), 4.74 (t, J = 9.1 Hz, 1H), 4.60 - 4.51 (m, 1H), 4.41 (t, J = 7.8 Hz, 1H), 4.22 (dd, J = 10.1, 5.2 Hz, 1H), 2.53 (s, 2H), 2.12 - 2.00 (m, 2H), 1.84 - 1.77 (m, 1H), 1.75 (d, J = 6.0 Hz, 3H), 1.71 - 1.59 (m, 1H). |

(continued)

| Example | Name | Structure | LC-MS $(M+H)^+$ | $^1H$ NMR |
|---|---|---|---|---|
| 222 | (3-(4-(5-(1-(7-chloroquinoxalin -5-yl) ethoxy)-1H-indazol-3-yl)-1H-pyrazol-1-yl)azetidin-1-yl)(1-hydroxycyclopro pyl)methanone | 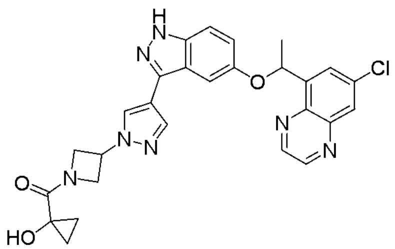 | 530.2 | $^1H$ NMR (400 MHz, $CD_3OD$) δ 9.06 (s, 1H), 8.99 (s, 1H), 8.02 (s, 2H), 7.95 (s, 1H), 7.73 (s, 1H), 7.41 (d, J = 9.0 Hz, 1H), 7.17 (d, J = 6.8 Hz, 2H), 6.73 (s, 1H), 5.29 (s, 1H), 5.07 (s, 1H), 4.91 (s, 1H), 4.51 (s, 1H), 4.36 (s, 1H), 1.77 (d, J = 6.3 Hz, 3H), 1.28 (d, J = 18.5 Hz, 2H), 0.98 (d, J = 3.7 Hz, 2H). |
| 223 | (3-(4-(5-(1-(7-chloroquinoxalin -5-yl) ethoxy)-1H-indazol-3-yl)-1H-pyrazol-1-yl)azetidin-1-yl)(1-methoxycyclopro pyl)methanone | 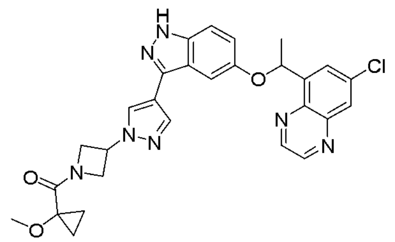 | 544.2 | $^1H$NMR (400MHz, $CD_3OD$) δ 9.05 (s, 1H), 8.97 (s, 1H), 8.01 (s, 2H), 7.95 (s, 1H), 7.76 (d, J = 12.0 Hz, 1H), 7.41 (d, J = 9.0 Hz, 1H), 7.23 - 7.15 (m, 2H), 6.73 (d, J = 6.5 Hz, 1H), 5.32 (s, 1H), 4.95 (s, 1H), 4.79 (s, 1H), 4.54 (s, 1H), 4.40 (s, 1H), 3.39 (s, 3H), 1.76 (d, J = 6.3 Hz, 3H), 1.24 (d, J= 18.3 Hz, 2H), 1.09 (d, J = 4.0 Hz, 2H). |
| 224 | 1-(3-(4-(5-(1-(7-chloroquinoxalin -5-yl) ethoxy)-1H-indazol-3-yl)-1-pyrazol-1-yl)azetidin-1-yl)-2-methoxy-2-methyl-propan-1-one | 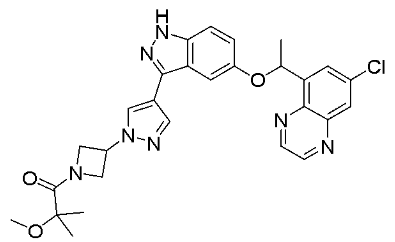 | 546.3 | $^1H$NMR (400 MHz, $CD_3OD$) δ 9.05 (s, 1H), 8.98 (s, 1H), 8.01 (d, J = 7.5 Hz, 2H), 7.95 (s, 1H), 7.74 (d, J = 13.2 Hz, 1H), 7.41 (d, J = 9.0 Hz, 1H), 7.18 (d, J = 15.7 Hz, 2H), 6.73 (d, J = 6.5 Hz, 1H), 5.26 (s, 1H), 4.91 (s, 1H), 4.74 (d, J = 4.4 Hz, 1H), 4.50 (s, 1H), 4.37 (s, 1H), 3.32 (s, 3H), 1.77 (d, J = 6.4 Hz, 3H), 1.42 (d, J = 7.9 Hz, 6H). |
| 238 | (3-(4-(5-(1-(7-chloroquinoxalin -5-yl) ethoxy)-1H-indazol-3-yl)-1H-pyrazol-1-yl)azetidin-1-yl)(cyclopropyl) methanone | 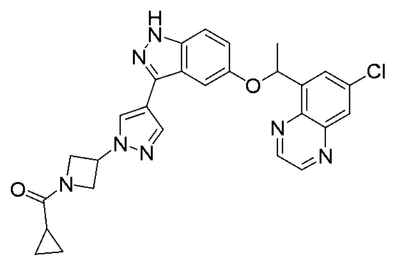 | 514.2 | $^1H$ NMR (400 MHz, DMSO) δ 12.86 (s, 1H), 9.14 (d, J = 5.6 Hz, 2H), 8.28 (d, J = 4.4 Hz, 1H), 8.15 (d, J = 2.4 Hz, 1H), 7.97 (d, J = 2.4 Hz, 1H), 7.85 (d, J = 5.2 Hz, 1H), 7.42 (m, 1H), 7.33 (s, 1H), 7.13 (dd, J = 9.2, 2.0 Hz, 1H), 6.70 (q, J = 6.4 Hz, 1H), 5.38-5.36 (m, 1H), 4.78 (t, J = 8.4 Hz, 1H), 4.65 - 4.53 (m, 1H), 4.39 (t, J = 9.2 Hz, 1H), 4.20 (dd, J = 9.6, 5.2 Hz, 1H), 1.76 (d, J = 6.4 Hz, 3H), 1.65 (dd, J = 12.4, 5.6 Hz, 1H), 0.80 (d, J = 7.6 Hz, 4H). |

(continued)

| Example | Name | Structure | LC-MS (M+H)+ | $^1$H NMR |
|---|---|---|---|---|
| 240 | 3-(4-(5-(1-(7-chloroquinoxalin -5-yl) ethoxy)-1H-indazol-3-yl)-1H-pyrazol-1-yl)azetidine-1-carbaldehyde | 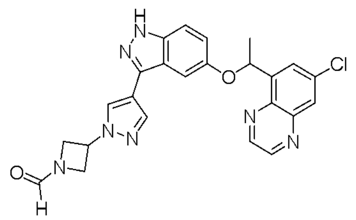 | 474.2 | $^1$H NMR (400 MHz, DMSO-d6) δ 12.86 (s, 1H), 9.13 (dd, J = 5.5, 1.9 Hz, 2H), 8.28 (d, J = 6.2 Hz, 1H), 8.20 - 8.09 (m, 2H), 7.96 (d, J = 2.4 Hz, 1H), 7.84 (d, J = 3.7 Hz, 1H), 7.42 (d, J = 9.0 Hz, 1H), 7.38 - 7.30 (m, 1H), 7.13 (dd, J = 9.0, 2.2 Hz, 1H), 6.69 (q, J = 6.3 Hz, 1H), 5.42 (m, 1H), 4.68 (t, J = 8.6 Hz, 1H), 4.52 - 4.39 (m, 2H), 4.22 (dd, J = 10.4, 5.5 Hz, 1H), 1.75 (d, J = 6.3 Hz, 3H). |

Example 181

5-(1-((3-(1-(3-(methylsulfonyl)propyl)-1H-pyrazol-4-yl)-1H-indazol-5-yl)oxy)ethy l)quinoxaline

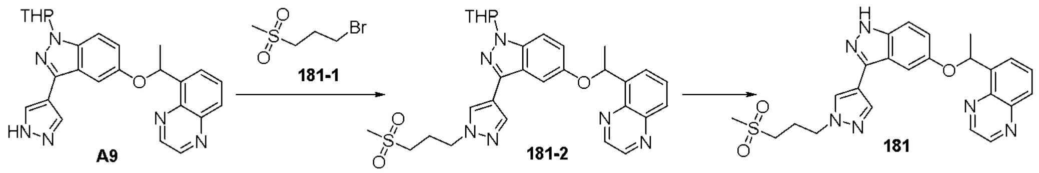


A9 181-2 181

At room temperature, Compound A9 (50 mg, 0. 11 mmol) was dissolved in DMF (2 mL), and 181-1 (45 mg, 0.23 mmol) and $CS_2CO_3$ (111 mg, 0.34 mmol) were added at RT, and reacted at 60°C for 6 hours. LCMS showed the complete reaction of raw materials. The reaction solution was directly subjected to rotary drying to obtain a crude which was purified by silica gel column chromatography (eluent: DCM:MeOH=10:1) to obtain a product Compound 181-2 as a white oil (53 mg, purity 93%, yield >77%, [M+H]+=561.2).

Compound 181-2 (53 mg, 0.09 mmol) was dissolved in DCM (2 mL) at room temperature, and TFA (1 mL) was added thereto in an ice bath. The reaction solution was stirred at room temperature for 1 h. LCMS showed the complete reaction of raw materials. The reaction solution was concentrated, and the organic phase was collected, and then purified by high pressure liquid chromatography to obtain Compound 181 as a light yellow solid (32.9 mg, purity: 100%, LCMS: 477.0[M+H]+). $^1$H NMR (400 MHz, DMSO) δ 12.79 (s, 1H), 9.11 (dd, J = 16.5, 1.7 Hz, 2H), 8.46 (s, 1H), 8.07 - 8.00 (m, 3H), 7.93 - 7.83 (m, 1H), 7.64 (s, 1H), 7.39 (d, J = 8.9 Hz, 1H), 7.24 (s, 1H), 7.10 (dd, J = 9.0, 2.1 Hz, 1H), 6.75 (q, J = 6.4 Hz, 1H), 4.30 (t, J = 6.8 Hz, 2H), 3.19 - 3.11 (m, 2H), 3.04 (s, 3H), 2.36 - 2.21 (m, 2H), 1.76 (d, J = 6.3 Hz, 3H).

The following compounds were synthesized according to the the route of Example 181.

| Example | Name | Structure | LC-MS $(M+H)^+$ | $^1H$ NMR |
|---|---|---|---|---|
| 179 | 3-(4-(5-(1-(quinoxalin-5-yl)ethoxy)-1H-indazol-3-yl)-1H-pyrazol-1-yl)propane-1-sulfonamide | 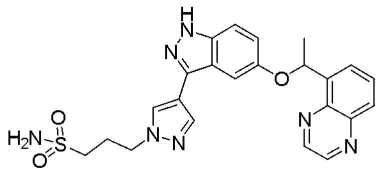 | 478.2 | $^1H$ NMR (400 MHz, DMSO) δ 12.77 (s, 1H), 9.10 (dt, J = 15.9, 8.0 Hz, 2H), 8.03 (dd, J = 11.0, 6.2 Hz, 3H), 7.87 (t, J = 7.8 Hz, 1H), 7.63 (s, 1H), 7.38 (d, J = 9.0 Hz, 1H), 7.23 (s, 1H), 7.10 (dt, J = 9.5, 4.7 Hz, 1H), 6.89 (s, 2H), 6.74 (q, J = 6.3 Hz, 1H), 4.30 (t, J = 6.7 Hz, 2H), 3.06 - 2.96 (m, 2H), 2.30 - 2.20 (m, 2H), 1.75 (d, J = 6.3 Hz, 3H). |
| 200 | 1-methyl-4-((4-(5-(1-(quinoxalin-5-yl)ethoxy)-1H-indazol-3-yl)-1H-pyrazol-1-yl)methyl)pyrrolidin-2-one | 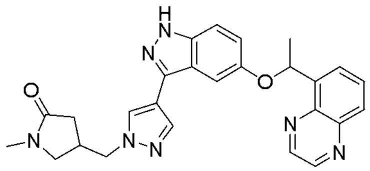 | 468.2 | $^1H$ NMR (400 MHz, DMSO)δ 12.78 (s, 1H), 9.10 (d, J = 16.1 Hz, 2H), 8.13 - 7.98 (m, 3H), 7.87 (t, J = 7.4 Hz, 1H), 7.63 (s, 1H), 7.39 (d, J = 9.0 Hz, 1H), 7.24 (s, 1H), 7.11 (dd, J = 9.0, 2.0 Hz, 1H), 6.74 (dd, J = 6.3, 2.4 Hz, 1H), 4.21 (d, J = 7.2 Hz, 2H), 3.43 - 3.37 (m, 1H), 3.18 (dd, J = 9.9, 5.0 Hz, 1H), 2.87 (d, J = 5.6 Hz, 1H), 2.73 (d, J = 8.5 Hz, 3H), 2.38 (dt, J = 17.7, 8.9 Hz, 1H), 2.19 - 2.07 (m, 1H), 1.76 (d, J = 6.3 Hz, 3H). |
| 220 | 7-chloro-5-(1-((3-(1-(3-(methylsulfonyl)pro pyl)-1H-pyrazol-4-yl)-1H-indazol-5-yl)oxy)ethyl)quinox aline | 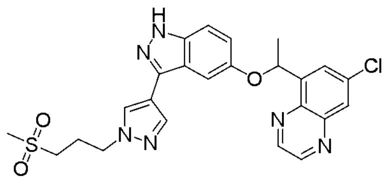 | 511.1 | $^1H$ NMR (400 MHz, $CD_3OD$) δ 9.06 (s, 1H), 8.99 (s, 1H), 8.02 (s, 1H), 7.94 (s, 1H), 7.90 (s, 1H), 7.65 (s, 1H), 7.40 (d, J = 9.1 Hz, 1H), 7.22 - 7.17 (m, 1H), 7.13 (s, 1H), 6.74 (d, J = 6.7 Hz, 1H), 4.38 - 4.31 (m, 2H), 3.12 (d, J = 15.4 Hz, 2H), 2.99 (s, 3H), 2.44 - 2.34 (m, 2H), 1.77 (d, J = 6.4 Hz, 3H). |

Example 182

N-methyl-3-(4-(5-(1-(quinoxalin-5-yl)ethoxy)-1H-indazol-3-yl)-1H-pyrazol-1-yl)c yclobutan-1-amine

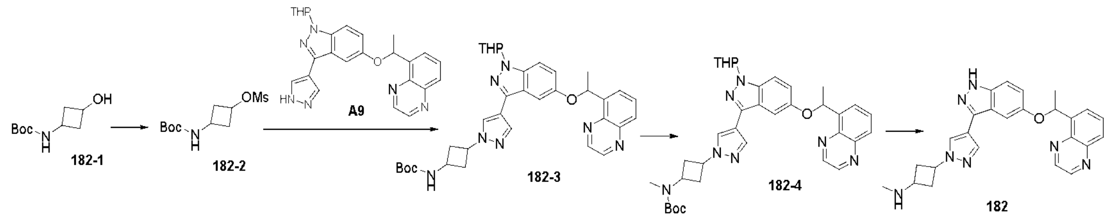

At 0°C, methylsufonyl chloride (67 mg, 0.59 mmol, 1.1 eq.) was slowly added to a solution of Compound 182-1 (100 mg, 0.53 mmol, 1.0 eq.) and TEA (70 mg, 0.69 mmol, 1.3 eq.) in DCM (5.0 mL). The reaction solution was warmed up to room temperature, and stirred for 3 hour. Water (10.0 mL) was added, and then the reaction solution extracted with dichloromethane (10.0 mL× 2). The organic phases were combined, washed with saturated brine (10.0 mL), dried over anhydrous sodium sulfate, filtered, and concentrated under reduced pressure. The resulting residue 182-2 was directly used for the next reaction step without purification.

At room temperature, Compound 182-2 (141 mg, 0.53 mmol, 4.0 eq.), Compound A9 (60 mg, 0.13 mmol, 1.0 eq.) and cesium carbonate (266 mg, 0.8 mmol, 6.0 eq.) were added successively to DMF (2.0 mL). The reaction mixture was stirred at 70°C for 16 hours. The reaction solution was cooled to room temperature, water (10.0 mL) was added, and then the reaction solution was extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by thin-layer chromatography (developping agent: ethyl acetate/petroleum ether=1:1) to obtain Compound 182-3 (18 mg, yield 21.7%).

At 0°C, a solution of Compound 182-3 (18 mg, 0.03 mmol, 1.0 eq.) in THF (0.8 mL) was slowly added to a suspension of NaH (6 mg, 0.15 mmol, 5.0 eq.) in THF (0.5 mL). The reaction solution was warmed up to room temperature, and stirred for 0.5 hour. Then, the reaction solution was cooled to 0°C, and $CH_3I$ (13 mg, 0.09 mmol, 3.0 eq.) was added dropwise, then warmed up to room temperature, and stirred for 16 hours. The reaction solution was cooled to 0°C, slowly poured into aqueous $NH_4Cl$ solution (10.0 mL), and extracted with ethyl acetate (10.0 mL × 2). The organic phases were combined, washed with saturated brine (10.0 mL), dried over anhydrous sodium sulfate, filtered, and concentrated under reduced pressure. The resulting residue 182-4 was directly used for the next reaction step without purification.

At room temperature, a solution of hydrochloric acid in methanol (4M, 3.0 mL) was slowly added to Compound 182-4 (20 mg, 0.03 mmol, 1.0 eq.). The reaction mixture was stirred at room temperature for 3 hours. The reaction mixture was concentrated under reduced pressure, and the resulting residue was dissolved in DCM (5.0 mL), aqueous sodium bicarbonate solution was added to adjust the pH to >7, and then the resulting residue was extracted with DCM (5.0 mL × 2). The organic phases were combined, washed with saturated brine (5.0 mL), dried over anhydrous sodium sulfate, filtered, and concentrated under reduced pressure. The resulting residue was purified by thin-layer chromatography (developping agent: dichloromethane:methanol=6:1) to obtain Compound 182 (5.6 mg, total yield 43% for two steps, purity 100%). LCMS (ESI) m/z=440.2 (M+H)+; $^1$H NMR (400 MHz, DMSO)δ 12.77 (s, 1H), 9.10 (d, J = 13.2 Hz, 2H), 8.08 - 7.98 (m, 3H), 7.86 (dd, J = 15.3, 7.6 Hz, 1H), 7.66 - 7.57 (m, 1H), 7.38 (d, J = 9.0 Hz, 1H), 7.20 (s, 1H), 7.10 (t, J = 8.6 Hz, 1H), 6.73 (q, J = 6.3 Hz, 1H), 4.59 - 4.48 (m, 1H), 2.98 (t, J = 7.7 Hz, 1H), 2.78 - 2.57 (m, 2H), 2.29 (d, J = 5.2 Hz, 3H), 2.20 (dd, J = 24.2, 13.8 Hz, 2H), 1.75 (d, J = 6.3 Hz, 3H).

Example 183

7-methyl-5-(1-((3-(1-methyl-1H-pyrazol-4-yl)-1H-indazol-5-yl)oxy)ethyl)pyridino [3,4-b]pyrazine

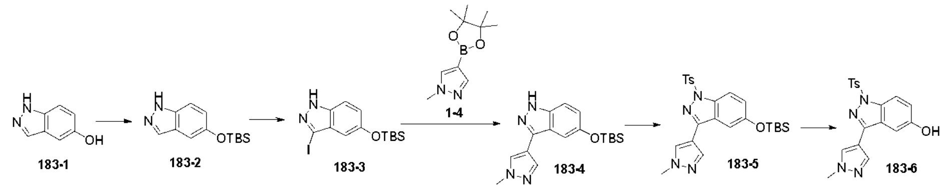

183-1 183-2 183-3 1-4 183-4 183-5 183-6

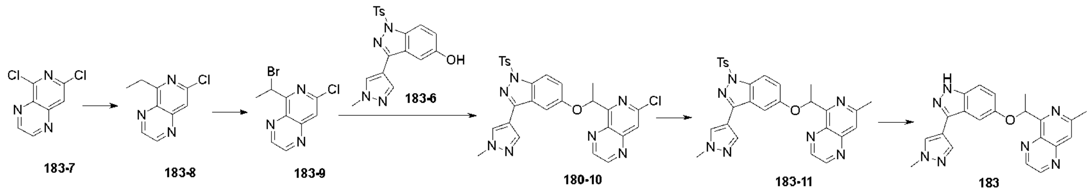

183-7 183-8 183-9 183-6 180-10 183-11 183

At room temperature, Compound 183-1 (10 g, 74.55 mmol) was dissolved in DMF (100 mL), and imidazole (7.6 g, 111.82 mmol) and TBSCl (13.4 g, 89.46 mmol) were added successively thereto in an ice bath. After the addition was completed, the reaction solution was stirred at room temperature for 5 h. LCMS showed the complete reaction of raw materials. The reaction solution was quenched with water and then extracted with ethyl acetate (200 mL × 3). The organic phases were combined and then subjected to rotary drying. The crude was purified by silica gel column chromatography (eluent: petroleum ether:ethyl acetate=1:1) to obtain a product Compound 183-2 as a yellow solid (16.74 g, purity 95%, yield 85.87% ). LCMS (ESI) m/z 249.13, (M+H)+.

At room temperature, Compound 183-2 (16.74 g, 67.39 mmol) was dissolved in DMF (150 mL), and then NIS (18.2 g, 80.87 mmol) was added. After the addition was completed, the reaction solution was stirred at room temperature for 7 h. LCMS showed the complete reaction of raw materials. The reaction solution was quenched with water and then extracted with ethyl acetate (300 mL × 3). The organic phases were combined and then subjected to rotary drying. The crude was purified by silica gel column chromatography (eluent: petroleum ether:ethyl acetate=3:1) to obtain a product Compound 183-3 as a brown solid (23.8 g, purity 95%, yield 89.63%). LCMS (ESI) m/z 375.01, (M+H)+.

At room temperature, Compound 183-3 (23.8 g, 63.58 mmol) and Compound 1-4 (29.1 g, 139.89 mmol) were dissolved in dioxane/$H_2O$ (250 mL, v/v=3/1), and then $K_2CO_3$ (35.1 g, 254.34 mmol) was added. Pd(dppf)$Cl_2$·DCM (2.6 g, 3.18 mmol) was added under nitrogen protection. The reaction solution was stirred at 85°C for 36 h. LCMS showed the complete reaction of raw materials. The reaction solution was quenched with water and then filtered through diatomite. The filtrate was extracted with ethyl acetate (300 mL × 3), and the organic phases were combined and then subjected to rotary drying. The crude was purified by silica gel column chromatography (eluent: dichloromethane:ethyl acetate=1:7) to obtain a product Compound 183-4 as a brown oil (14 g, purity 95%, yield 63.67% ). LCMS (ESI) m/z 329.17, (M+H)+.

Compound 183-4 (14 g, 42.62 mmol) was dissolved in THF (150 mL), and then TsCl (12.2 g, 63.93 mmol), TEA (12.9 g, 127.86 mmol) and DMAP (520 mg, 4.26 mmol) were added successively. The reaction solution was reacted at 50°C for 24 h. LCMS showed the complete reaction of raw materials. The reaction solution was diluted with water and then extracted with ethyl acetate (200 mL × 3). The organic phases were combined and then subjected to rotary drying. The crude was purified by silica gel column chromatography (eluent: petroleum ether:ethyl acetate=5:1) to obtain a product Compound 183-5 as a brown solid (14.5 g, purity 95% , yield 66.96% ). LCMS (ESI) m/z 483.18, (M+H)+.

At room temperature, Compound 183-5 (14.5 g, 30.08 mmol) was dissolved in DMF (150 mL), and then CsF (9.14 g, 60.16 mmol) was added. The reaction solution was reacted at room temperature for 2 h. LCMS showed the complete reaction of raw materials. The reaction solution was diluted with water and then extracted with ethyl acetate (200 mL × 3). The organic phases were combined and then subjected to rotary drying. The crude was purified by silica gel column chromatography (eluent: DCM:MeOH=10:1) to obtain a product Compound 183-6 as a white solid (9.2 g, purity 95%, 78.97% yield). LCMS (ESI) m/z 369.01, (M+H)+.

At room temperature, Compound 183-7 (10 g, 49.99 mmol) was dissolved in dioxane (100 mL), and Pd(dppf)$Cl_2$·DCM (2 g, 2.5 mmol) was added under nitrogen protection. The reaction solution was cooled to 0°C, and then $Zn(Et)_2$ (25 mL, 49.99 mmol) was slowly added. After the addition was completed, the reaction solution was stirred at 40°C for 20~30 min. LCMS showed the complete reaction of raw materials and the generation of 48% by-product. The reaction solution was quenched with water and then extracted with ethyl acetate (200 mL × 3). The organic phases were combined and then subjected to rotary drying. The crude was purified by silica gel column chromatography (eluent: petroleum ether:ethyl acetate=10:1) to obtain a product Compound 183-8 as a white solid (4.87 g, purity 95% , yield 50.3%). LCMS (ESI) m/z 194.01, (M+H)+.

At room temperature, Compound 183-8 (4.87 g, 25.15 mmol) was dissolved in CCl4 (50 mL), and NBS (4.92 g, 27.66 mmol) and AIBN (412 mg, 2.51 mmol) were added successively. The reaction solution was stirred at 80°C for 5 h. LCMS showed the complete reaction of raw materials. The reaction solution was quenched with water, and then extracted with DCM (100 mL× 3). The organic phases were combined and then subjected to rotary drying. The crude was purified by silica gel column chromatography (eluent: petroleum ether:ethyl acetate=10:1) to obtain a product 183-9 as a white oil (6.5

g, purity 95%, yield 90.08%). LCMS (ESI) m/z 272.01 & 274.01, (M+H)+.

At room temperature, Compound 183-9 (680 mg, 2.49 mmol) and Compound 183-6 (920 mg, 2.49 mmol) were dissolved in DMF (10 mL), and then the compound $K_2CO_3$ (690 mg, 4.99 mmol) was added. The reaction solution was stirred at room temperature for 2h. LCMS showed the complete reaction of raw materials. The reaction solution was quenched with water and then extracted with ethyl acetate (30 mL × 3). The organic phases were combined and then subjected to rotary drying. The crude was purified by silica gel column chromatography (eluent: dichloromethane:ethyl acetate=1:8) to obtain a product 183-10 as a brown solid (1.2 g, purity 90%, 81.51%). LCMS (ESI) m/z 560.01, (M+H)+.

At room temperature, Compound 183-10 (1.1 g, 1.96 mmol) and methylboronic acid (2.46 g, 9.82 mmol) were dissolved in THF/$H_2O$ (12 mL, v/v=4/1), and then $Na_2CO_3$ (832 mg, 7.85 mmol) was added. $Pd(PPh_3)_4$ (227 mg, 0.196 mmol) was added under nitrogen protection. The reaction solution was stirred at 80°C for 48 h. LCMS showed the complete reaction of raw materials. The reaction solution was diluted with water and then extracted with ethyl acetate (100 mL × 3). The organic phases were combined and then subjected to rotary drying. The crude was purified by silica gel column chromatography (eluent: DCM:MeOH=10:1) to obtain a product Compound 183-11 as a brown oil (1.15 g, purity 83%). LCMS (ESI) m/z 540.17, (M+H)+.

At room temperature, Compound 183-11 (1.15 g, 1.767 mmol) was dissolved in MeOH (20 mL), and then $K_2CO_3$ (488 mg, 3.535 mmol) was added. The reaction solution was stirred at 70°C for 1 h. LCMS showed the complete reaction of raw materials. The reaction solution was diluted with water and extracted with ethyl acetate (20 mL × 3). The organic phases were combined and then subjected to rotary drying, and the crude was purified by silica gel column chromatography (eluent: DCM:MeOH=10:1) to obtain a product Compound 183 as a yellow solid (470 mg, purity 95%). LCMS (ESI) m/z 386.17, (M+H)+. $^1$H NMR (400 MHz, DMSO) δ 12.73 (s, 1H), 9.24 - 9.04 (m, 2H), 7.99 (s, 1H), 7.81 (s, 1H), 7.61 (s, 1H), 7.36 (d, J = 9.0 Hz, 1H), 7.27 (s, 1H), 7.08 (dd, J = 9.0, 2.0 Hz, 1H), 6.81 (q, J = 6.4 Hz, 1H), 3.91 (s, 3H), 2.69 (s, 3H), 1.78 (d, J = 6.4 Hz, 3H).

Example 194

N,N-dimethyl-3-(4-(5-(1-(quinoxalin-5-yl)ethoxy)-1H-indazol-3-yl)-1H-pyrazol-1-yl)azetidine-1-sulfonamide

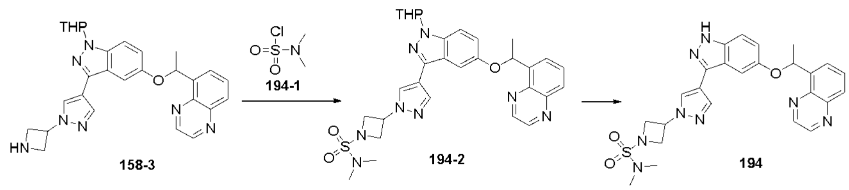


158-3 194-2 194

Compound 158-3 (110 mg, 0.22 mmol, 1.0 eq) was dissolved in DCM (5.0 mL), TEA (70 mg, 0.66 mmol, 3.0 eq) was added, purged with nitrogen, and Compound 194-1 (48 mg, 0.34 mmol, 1.5 eq) was added thereto in an ice water bath, and reacted at room temperature overnight. LCMS monitoring showed the generation of a product. Water was added to the reaction solution, and then the reaction solution was extracted with DCM (10.0 mL) three times. The organic phases were combined, concentrated, and purified by thin-layer chromatography (developping agent: DCM/MeOH=15/1) to obtain Compound 194-2 (60 mg, 44%). LCMS:(ESI) m/z 603.20, [M+1] +.

Compound 194-2 (60 mg) was dissolved in DCM (3.0 mL), and TFA (1.0 mL) was added, and reacted at room temperature for 2 hours. LCMS monitoring showed the complete reaction. The reaction solution was subjected to rotary drying, and purified by preparative high performance liquid chromatography to obtain Compound 194 (6.2 mg, 12%). LCMS:(ESI) m/z 519, (M+H) +; $^1$H NMR (400 MHz, $CD_3OD$) δ 9.08 (s, 1H), 8.98 (s, 1H), 7.99 (d, J = 14.5 Hz, 3H), 7.81 (s, 1H), 7.65 (s, 1H), 7.37 (d, J = 9.4 Hz, 1H), 7.17 (d, J = 9.1 Hz, 1H), 7.12 (s, 1H), 6.78 (d, J = 6.2 Hz, 1H), 5.22 (s, 1H), 4.35 (s, 2H), 4.30 (s, 2H), 2.89 (s, 6H), 1.78 (d, J = 6.3 Hz, 3H).

The following compound was synthesized according to the the route of Example 194.

| Example | Name | Structure | LC-MS (M+H)+ | 1H NMR |
|---|---|---|---|---|
| 195 | 5-(1-((3-(1-(1-(methylsulfonyl)azetidin-3-yl)-1H-pyrazol-4-yl)-1H-indazol-5-yl)oxy)ethyl)quino xaline | 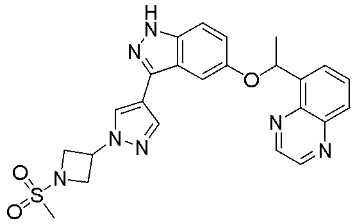 | 490.2 | 1H NMR (400 MHz, $CD_3OD$) δ 9.07 (s, 1H), 8.97 (s, 1H), 8.01 (d, J = 11.7 Hz, 2H), 7.94 (s, 1H), 7.81 (d, J = 15.6 Hz, 1H), 7.67 (s, 1H), 7.37 (d, J = 9.1 Hz, 1H), 7.17 (d, J = 6.8 Hz, 1H), 7.11 (s, 1H), 6.77 (d, J = 6.3 Hz, 1H), 5.25 (d, J = 14.1 Hz, 1H), 4.39 (t, J = 7.7 Hz, 4H), 3.12 (s, 3H), 1.77 (d, J = 6.4 Hz, 3H). |

Example 196

2-amino-N,2-dimethyl-N-(3-(4-(5-(1-(quinoxalin-5-yl)ethoxy)-1H-indazol-3-yl)-1H-pyrazol-1-yl)cyclobutyl)propanamide

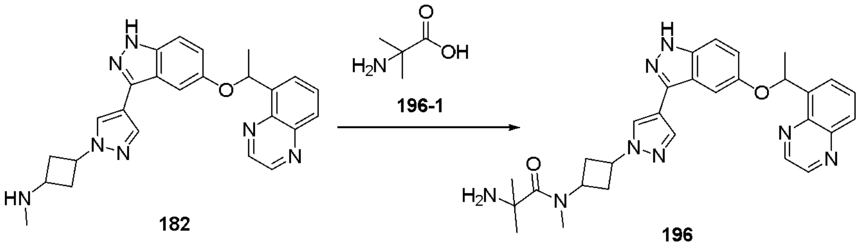


Compound 182 (40 mg, 0.09 mmol, 1.0 eq) and Compound 196-1 (9.4 mg, 0.09 mmol, 1 eq) were dissolved in DMF (2mL), and TEA (47 mg, 0.36 mmol, 6 eq.) and BOP (80.5 mg, 0.18 mmol, 2 eq.) were added at 25°C, and reacted under stirring at 25°C for 1 hour. LCMS showed the complete reaction of Compound 1. 30 ml of water was added, and then the reaction solution was extracted with EA, and washed with saturated brine. The organic phase was concentrated to obtain a crude which was then purified by thin-layer chromatography (developping agent: DCM:MeOH=10:1) to obtain Compound 196 (15.5 mg, yield 31.7%) as a white solid . LCMS (ESI) m/z 525.2, (M+H)+; 1H NMR (400 MHz, DMSO) δ 12.77 (s, 1H), 9.16 - 9.05 (m, 2H), 8.08 (d, J = 12.4 Hz, 1H), 8.05 - 7.95 (m, 2H), 7.87 (t, J = 7.6 Hz, 1H), 7.69 (d, J = 8.8 Hz, 1H), 7.38 (d, J = 8.8 Hz, 1H), 7.22 (d, J = 9.6 Hz, 1H), 7.10 (d, J = 8.8 Hz, 1H), 6.72 (d, J = 6.8 Hz, 1H), 3.12 (s, 3H), 2.71 (d, J = 21.2 Hz, 2H), 1.75 (d, J = 6.4 Hz, 3H), 1.52 (s, 6H), 1.41 - 1.18 (m, 4H).

The following compounds were synthesized according to the the route of Example 196.

| Example | Name | Structure | LC-MS $(M+H)^+$ | $^1H$ NMR |
|---|---|---|---|---|
| 197 | 2-hydroxy-N,2-dimethyl-N-(3-(4-(5-(1-(quinoxalin-5-yl)ethoxy)-1H-indazol-3-yl)-1H-pyrazol-1-yl)cyclobutyl)propanamide | 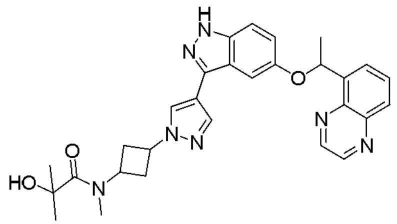 | 526.2 | $^1H$ NMR (400 MHz, DMSO) δ 12.76 (d, J = 20.4 Hz, 1H), 9.10 (dt, J = 26.0, 10.0 Hz, 2H), 8.13 - 7.99 (m, 4H), 7.86 (dd, J = 18.0, 10.4 Hz, 1H), 7.68 (t, J = 9.2 Hz, 1H), 7.37 (t, J = 11.6 Hz, 1H), 7.22 (d, J = 12.4 Hz, 1H), 7.09 (t, J = 10.0 Hz, 1H), 6.72 (t, J = 10.0 Hz, 1H), 4.98 - 4.50 (m, 1H), 2.95 (d, J = 35.4 Hz, 2H), 2.69 (s, 2H), 2.55 (s, 3H), 1.71 (t, J = 27.2 Hz, 3H), 1.37 (d, J = 4.8 Hz, 6H). |
| 199 | N-methyl-N-(3-(4-(5-(1-(quinoxalin-5-yl)ethoxy)-1H-indazol-3-yl)-1H-pyrazol-1-yl)cyclobutyl)acet amide | 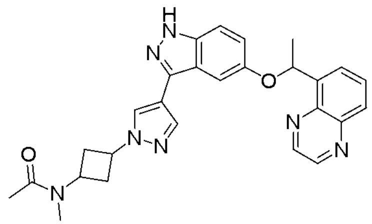 | 482.2 | $^1H$ NMR(400 MHz, DMSO) δ 12.78 (s, 1H), 9.19 - 8.98 (m, 2H), 8.12 - 7.98 (m, 3H), 7.87 (t,J = 7.8 Hz, 1H), 7.70 (d,J = 4.2 Hz, 1H), 7.38 (d,J = 8.9 Hz, 1H), 7.23 (s, 1H), 7.10 (d,J = 9.0 Hz, 1H), 6.79 - 6.69 (m, 1H), 4.85 (dd,J = 24.9, 15.4 Hz, 1H), 4.63 (dd,J = 16.4, 8.3 Hz, 1H), 3.01 (dd,J = 45.2, 7.2 Hz, 3H), 2.77 (d,J = 8.6 Hz, 2H), 2.72 - 2.60 (m, 2H), 2.09 (d,J = 22.0 Hz, 3H), 1.75 (d,J = 6.3 Hz, 3H) |

Example 198

N-methyl-N-(3-(4-(5-(1-(quinoxalin-5-yl)ethoxy)-1H-indazol-3-yl)-1H-pyrazol-1-yl)cyclobutyl)methanesulfonamide

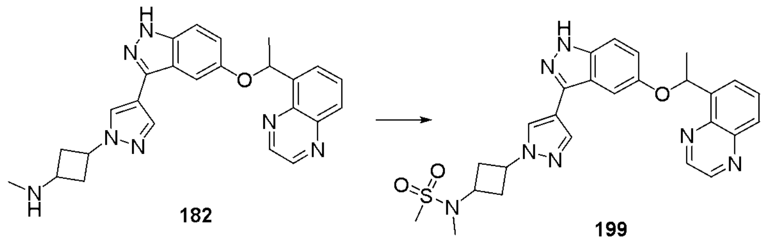

182 199

At room temperature, Compound 182 (40 mg, 0.09 mmol) and methylsulfonyl chloride (20 mg, 0.18 mmol) were dissolved in DCM (2 mL), and TEA (18 mg, 0.18 mmol) was added. The reaction solution was stirred at room temperature for 1 h. LCMS showed the complete reaction of raw materials. The reaction solution was diluted with water and then extracted with EA (20 mL × 3). The organic phases were combined and then subjected to rotary drying. The crude was purified by preparative high performance liquid chromatography to obtain a white solid 199 (15.1 mg, purity 100%, yield >29%, [M+H]+=518.0). $^1$H NMR(400 MHz, DMSO) δ 12.78 (s, 1H), 9.17 - 9.07 (m, 2H), 8.11 - 7.99 (m, 3H), 7.87 (t,J = 7.8 Hz, 1H), 7.70 (d,J = 6.1 Hz, 1H), 7.39 (d,J = 9.0 Hz, 1H), 7.24 (s, 1H), 7.11 (d,J = 9.0 Hz, 1H), 6.74 (q,J = 6.3 Hz, 1H), 4.77 - 4.58 (m, 1H), 4.20 - 4.01 (m, 1H), 2.93 (s, 3H), 2.90 (s, 2H), 2.88 (s, 2H), 2.80 - 2.67 (m, 3H), 1.76 (d,J = 6.3 Hz, 3H).

Example 201

N-cyclopropyl-3-(4-(5-(1-(quinoxalin-5-yl)ethoxy)-1H-indazol-3-yl)-1H-pyrazol-1-yl)azetidine-1-carboxamide

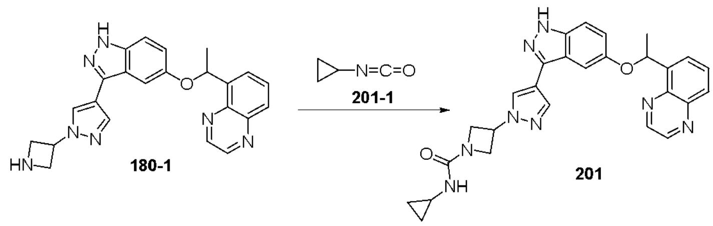


180-1 201

Compound 180-1 (120 mg, 0.29 mmol, 1.0 eq) was dissolved in DCM (5 mL), and then Compound 201-1 (28.8 mg, 0.35 mmol, 1.2 eq) and TEA (87.8 mg, 0.87 mmol, 3.0 eq) were added respectively. After the addition was completed, the reaction solution was purged with nitrogen three times, and reacted under stirring at 25°C for 16 hours. TLC showed no remaining raw materials, and the conversion of most of raw materials into a product. Then, the reaction solution was extracted with (10 mL) ethyl acetate three times. The organic phases were combined, washed with saturated brine, dried over anhydrous sodium sulfate, filtered, concentrated, and purified by thin-layer chromatography (developing solvent: DCM:MeOH=25:1) to obtain Compound 201 as a yellow solid (42.0 mg, purity: 97.8, yield: 29%). LCMS:(ESI) m/z 495, (M+H) +; $^1$H NMR (400 MHz, $CD_3OD$) δ 9.06 (s, 1H), 8.96 (s, 1H), 8.01 (s, 2H), 7.92 (s, 1H), 7.81 (d, J = 8.4 Hz, 1H), 7.65 (s, 1H), 7.36 (d, J = 8.9 Hz, 1H), 7.15 (s, 1H), 7.09 (s, 1H), 6.77 (s, 1H), 5.21 (s, 1H), 4.39 (s, 2H), 4.24 (s, 2H), 2.57 (s, 1H), 1.76 (d, J = 6.4 Hz, 3H), 0.70 (s, 2H), 0.52 (s, 2H).

Example 202

imino(methyl)(3-(4-(5-(1-(quinoxalin-5-yl)ethoxy)-1H-indazol-3-yl)-1H-pyrazol-1-yl)cyclobutyl)-sulfanone

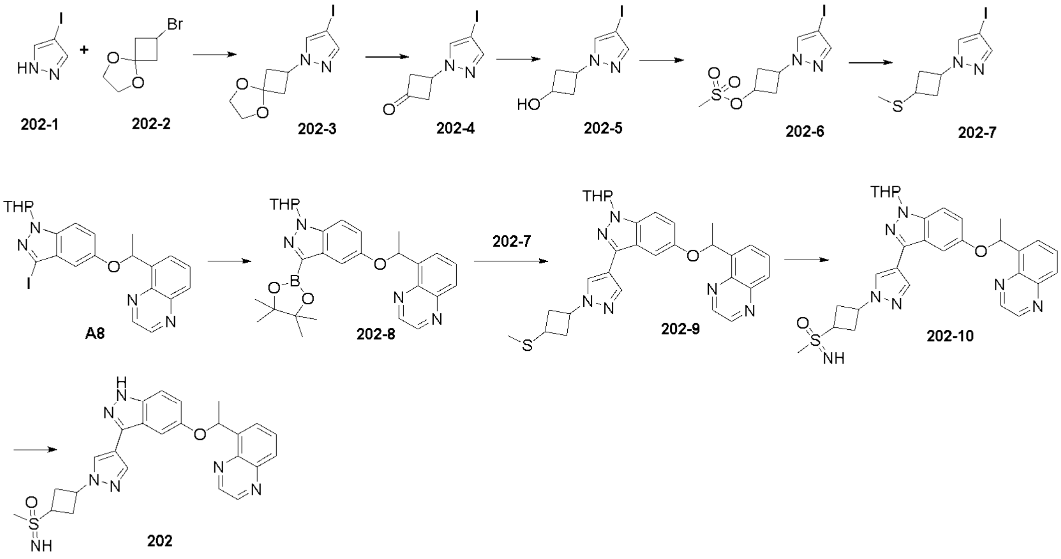

202-1 202-2 202-3 202-4 202-5 202-6 202-7

A8 202-8 202-9 202-10

202

At room temperature, Compound 202-1 (0.870 g, 4.48 mmol), Compound 202-2 (1.039 g, 5.38 mmol) and cesium carbonate (2.922 g, 8.96 mmol) were added to N,N-dimethylformamide (4.5 mL). The resulting mixture was stirred at 60°C for 24 hours. LCMS showed the generation of a product. The reaction mixture was cooled to room temperature, and diluted with water (15.0 mL). The resulting mixture was extracted with ethyl acetate (10.0 mL × 4). The organic phases were combined, washed with water (10.0 mL × 2), dried over anhydrous sodium sulfate, and filtered. The filtrate was concentrated to dryness under reduced pressure. The resulting residue was purified by silica gel column chromatography (eluent: petroleum ether:ethyl acetate=7:1) to obtain a colorless clear liquid 202-3 (0.952 g, yield: 65%, purity: 93.03%). LCMS (ESI) m/z 306.8, (M+H)+.

At room temperature, Compound 202-3 (0.476 g, 1.56 mmol) and pyridinium p-toluenesulfonate (1.954 g, 7.78 mmol) were added to a mixture of 1,4-dioxane (3.9 mL) and water (3.9 mL). The resulting mixture was stirred at 80°C for 46 hours. LCMS showed the generation of a product. The reaction mixture was cooled to room temperature, diluted with ethyl acetate (15.0 mL), washed with water (10.0 mL × 2), dried over anhydrous sodium sulfate, and filtered. The filtrate was concentrated to dryness under reduced pressure. The resulting residue was dried under vacuum to obtain a crude product 202-4 as a yellow liquid (0.395 g, crude yield: 75%, purity: 77.03%). LCMS (ESI) m/z 262.8, (M+H)+.

At 0°C, NaBH4 (0.0570 g, 1.51 mmol) was added in two batches to a solution of the crude product Compound 202-4 (0.395 g, 1.51 mmol (a theoretical amount)) in methanol (3.8 mL). The resulting mixture was stirred at 0°C for 2.5 hours. LCMS showed the generation of a product. The reaction mixture was concentrated to dryness under reduced pressure, and saturated aqueous ammonium chloride solution (5.0 mL) and ethyl acetate (5.0 mL) were added to the resulting residue. The organic phase was separated, and the aqueous phase was extracted with ethyl acetate (5.0 mL × 3). The organic phases were combined, dried over anhydrous sodium sulfate, and filtered. The filtrate was concentrated to dryness under reduced pressure. The resulting residue was purified by silica gel column chromatography (eluent: petroleum ether:ethyl acetate=2.5:1) to obtain a colorless clear liquid 202-5 (0.30 g, yield: 75%, purity: 99.37%). LCMS (ESI) m/z 264.8, (M+H)+.

At room temperature, Compound 202-5 (0.30 g, 1.14 mmol) and triethylamine (0.230 g, 2.28 mmol) were added to dichloromethane (5.7 mL). The resulting reaction solution was cooled in an ice water bath, and then methylsufonyl chloride (0.156 g, 1.37 mmol) was slowly added dropwise. The resulting mixture was stirred at 0°C for 1 hour. LCMS showed the generation of a product. The reaction mixture was diluted with dichloromethane (10.0 mL), washed with saturated aqueous sodium bicarbonate solution (5.0 mL × 2), dried over anhydrous sodium sulfate, and filtered. The filtrate was concentrated to dryness under reduced pressure. The resulting residue was dried under vacuum to obtain a crude product 202-6 as a light yellow solid (0.396 g, crude yield: 95%, purity: 93.53%). LCMS (ESI) m/z 343.0, (M+H)+.

At room temperature, sodium thiomethoxide (0.239 g, 3.41 mmol) was added to a solution of the crude product Compound 202-6 (0.396 g, 1.14 mmol (a theoretical amount)) in N,N-dimethylformamide (2.8 mL). The resulting mixture was stirred for 2 hours. LCMS showed the generation of a product. The reaction mixture was diluted with ethyl acetate (10.0 mL) and water (10.0 mL). The organic phase was separated, and the aqueous phase was extracted with ethyl acetate (10.0 mL × 3). The organic phases were combined, dried over anhydrous sodium sulfate, and filtered. The filtrate was concentrated to dryness under reduced pressure. The resulting residue was purified by silica gel column chromatography (eluent: petroleum ether:ethyl acetate=30:1) to obtain Compound 202-7 as a white solid (0.268 g, yield: 76%, purity: 95.30%). LCMS (ESI) m/z 295.0, (M+H)+.

At room temperature, Compound A8 (0.30 g, 0.60 mmol), bis(pinacolato)diboron (0.305 g, 1.2 mmol), Pd(dppf)

$Cl_2 \cdot CH_2Cl_2$ (0.049 g, 0.060 mmol) and potassium acetate (0.177 g, 1.80 mmol) were added to 1,4-dioxane (3.0 mL). The resulting mixture was stirred at 70°C for 6 hour. LCMS showed the generation of a product. The reaction mixture was cooled to room temperature, diluted with ethyl acetate (10.0 mL), and filtered under reduced pressure. The filter cake was washed with ethyl acetate (5.0 mL × 3). The filtrates were combined, and concentrated to dryness under reduced pressure. The resulting residue was dried under vacuum to obtain a crude product 202-8 as a brown jelly (0.646 g, crude yield: 96%, purity: 44.85%). LCMS (ESI) m/z 501.2, (M+H)+.

At room temperature, Compound 202-7 (0.115 g, 0.39 mmol), the crude product Compound 202-8 (0.646 g, 0.60 mmol (a theoretical amount)), Pd(dppf)$Cl_2 \cdot CH_2Cl_2$ (0.0212 g, 0.026 mmol) and $Na_2CO_3$ (0.124 g, 1.17 mmol) were added to a mixture of 1,4-dioxane (4.0 mL) and water (2.0 mL). The resulting mixture was stirred at 80°C for 22 hours. LCMS showed the generation of a product. The reaction mixture was cooled to room temperature, and diluted with dichloromethane (15.0 mL) and water (10.0 mL). The organic phase was separated, and the aqueous phase was extracted with dichloromethane (10.0 mL × 3). The organic phases were combined, dried over anhydrous sodium sulfate, and filtered. The filtrate was concentrated to dryness under reduced pressure. The resulting residue was purified by silica gel thin-layer chromatography (eluent: petroleum ether:ethyl acetate=3:1) to obtain Compound 202-9 as a yellow jelly (0.0623 g, yield: 8%, purity: 27.56%). LCMS (ESI) m/z 541.3, (M+H)+.

At room temperature, Compound 202-9 (0.0623 g, 0.115 mmol) and ammonium carbamate (0.0180 g, 0.230 mmol) were added to a mixture of tertiary butano (1.2 mL) and dichloromethane (0.40 mL), and then iodobenzene diacetate (0.111 g, 0.345 mmol) was added. The resulting mixture was stirred for 4 hours. LCMS showed the generation of a product. The reaction mixture was diluted with dichloromethane (10.0 mL), and then saturated aqueous sodium bicarbonate solution was added to adjust the pH of the aqueous phase to about 7 to 8. The organic phase was separated, and the aqueous phase was extracted with dichloromethane (10.0 mL × 3). The organic phases were combined, washed successively with saturated aqueous sodium sulfite solution (10.0 mL) and water (10.0 mL), dried over anhydrous sodium sulfate, and filtered. The filtrate was concentrated to dryness under reduced pressure. The resulting residue was purified by silica gel thin-layer chromatography (eluent: dichloromethane:methanol=20:1) to obtain Compound 202-10 as a light yellow solid (0.0207 g, yield: 25%, purity: 78.09%). LCMS (ESI) m/z 572.3, (M+H)+.

At room temperature, a solution of HCl in methanol (4.0 M) (0.36 mL, 1.44 mmol) was added to Compound 202-10 (0.0207 g, 0.036 mmol). The resulting mixture was stirred for 4 hours. LCMS showed the generation of a product. The reaction mixture was concentrated to dryness under reduced pressure, and saturated aqueous sodium bicarbonate solution was added to the residue to adjust the pH of the aqueous phase to about 7 to 8. The resulting mixture was extracted with dichloromethane (5.0 mL × 4). The organic phases were combined, dried over anhydrous sodium sulfate, and filtered. The filtrate was concentrated to dryness under reduced pressure. The resulting residue was purified by silica gel thin-layer chromatography (eluent: dichloromethane: methanol=12:1) to obtain Compound 202 as a beige solid (0.0154 g, yield: 82%, purity: 94.07%). LCMS (ESI) m/z 488.3, (M+H)+; $^1$H NMR (400 MHz, DMSO) δ 12.80 (s, 1H), 9.16 - 9.11 (m, 2H), 8.10 (s, 1H), 8.07 - 8.04 (m, 1H), 8.04 - 7.99 (m, 2H), 7.90 - 7.84 (m, 1H), 7.71 (d, J = 2.8 Hz, 1H), 7.39 (d, J = 9.2 Hz, 1H), 7.22 (d, J = 7.6 Hz, 1H), 7.11 (d, J = 9.2 Hz, 1H), 6.74 (q, J = 6.4 Hz, 1H), 5.13 - 5.02 (m, 1H), 3.99 - 3.95 (m, 1H), 2.97 (s, 3H), 2.94 - 2.87 (m, 4H), 1.75 (d, J = 6.4 Hz, 3H).

Example 214

2-(3-(4-(5-(1-(quinoxalin-5-yl)ethoxy)-1H-indazol-3-yl)-1H-pyrazol-1-yl)azetidin-1-yl)acetamide

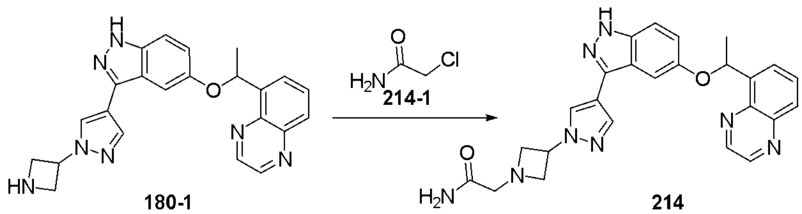


180-1

214

At room temperature, Compound 180-1 (45 mg, 0.11 mmol) and Compound 214-1 (10 mg, 0.11 mmol) were dissolved in DMF (1 mL), and then DIEA (141 mg, 1.1 mmol) was added. The reaction solution was stirred at 50°C overnight. LCMS showed the complete reaction of raw materials. The reaction solution was diluted with water and extracted with ethyl acetate (20 mL × 3). The organic phases were combined, subjected to rotary drying, and then purified by preparative high performance liquid chromatography to obtain a final product Compound 214 (26.6 mg, purity 100% ). LCMS (ESI) m/z 468.21, (M+H)+. $^1$H NMR (400 MHz,DMSO) δ 12.75 (s, 1H), 9.06 (dd, J = 12.8, 1.8 Hz, 2H), 8.17 (s, 1H), 8.02 - 7.93 (m, 2H), 7.86 - 7.79 (m, 1H), 7.63 (s, 1H), 7.34 (d, J = 8.8 Hz, 1H), 7.20 (d, J = 1.8 Hz, 2H), 7.06 (dd, J = 9.0, 2.2 Hz,

2H), 6.69 (q, J = 6.4 Hz, 1H), 5.05 - 4.96 (m, 1H), 3.81 (t, J = 7.6 Hz, 2H), 3.53 - 3.49 (m, 2H), 3.13 (s, 2H), 1.71 (d, J = 6.4 Hz, 3H).

Example 229

5-(1-((3-(1-(3-(methylsulfonyl)cyclobutyl)-1H-pyrazol-4-yl)-1H-indazol-5-yl)oxy) ethyl)quinoxaline

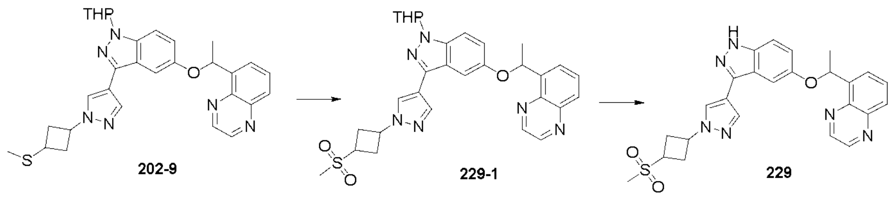

202-9 229-1 229

At 0°C, a solution of potassium peroxomonosulfate (0.0332 g, 0.0958 mmol) in water (0.87 mL) was added dropwise to a solution of Compound 202-9 (0.0471 g, 0.087 mmol) in methanol (0.87 mL). The resulting mixture was stirred at 0°C for 5 minutes, then warmed up to room temperature and stirred for 1 hours. LCMS showed the generation of a product. The reaction mixture was diluted with dichloromethane (10.0 mL) and saturated aqueous sodium sulfite solution (5.0 mL). The organic phase was separated, and the aqueous phase was extracted with dichloromethane (5.0 mL x 3). The organic phases were combined, dried over anhydrous sodium sulfate, and filtered. The filtrate was concentrated to dryness under reduced pressure. The resulting residue was dried under vacuum to obtain a crude product 229-1 as a yellow jelly (0.051 g, crude yield: 83%, purity: 81.24%). LCMS (ESI) m/z 573.2, (M+H)+.

At room temperature, a solution of HCl in methanol (4.0 M) (0.89 mL, 3.56 mmol) was added to the crude product Compound 229-1 (0.051 g, 0.089 mmol). The resulting mixture was stirred for 4 hours. LCMS showed the generation of a product. The reaction mixture was concentrated to dryness under reduced pressure, and saturated aqueous sodium bicarbonate solution was added to the residue to adjust the pH of the aqueous phase to about 7 to 8. The resulting mixture was extracted with dichloromethane (5.0 mL × 4). The organic phases were combined, dried over anhydrous sodium sulfate, and filtered. The filtrate was concentrated to dryness under reduced pressure. The resulting residue was purified by high performance liquid chromatography to obtain a white solid 229 (0.025 g, yield: 58%, purity: 100%). LCMS (ESI) m/z 489.2, (M+H)+; $^1$H NMR (400 MHz, DMSO) δ 12.81 (s, 1H), 9.12 (d, J = 1.8 Hz, 1H), 9.10 (d, J = 1.8 Hz, 1H), 8.14 (s, 1H), 8.04 (dd, J = 8.4, 1.6 Hz, 1H), 8.03 - 8.00 (m, 1H), 7.87 (t, J = 8.0 Hz, 1H), 7.73 (s, 1H), 7.39 (d, J = 9.0 Hz, 1H), 7.25 (s, 1H), 7.11 (dd, J = 9.0, 2.2 Hz, 1H), 6.74 (q, J = 6.4 Hz, 1H), 5.15 - 5.04 (m, 1H), 4.05 (tt, J = 9.2, 4.8 Hz, 1H), 3.09 (s, 3H), 2.98 - 2.91 (m, 4H), 1.75 (d, J = 6.4 Hz, 3H).

Example 230

7-cyclopropyl-5-(1-((3-(1-methyl-1H-pyrazol-4-yl)-1H-indazol-5-yl)oxy)ethyl)pyri dino[3,4-b]pyrazine

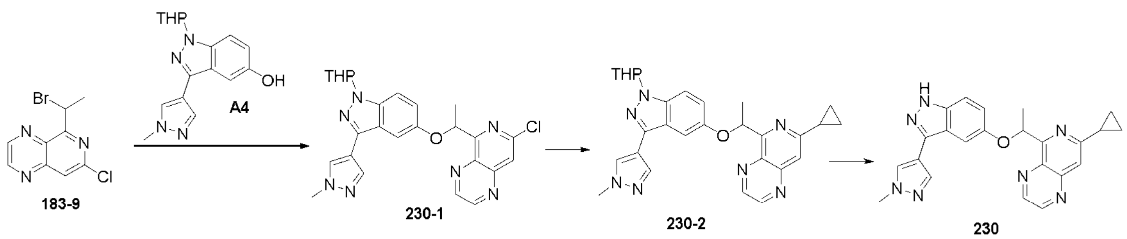

183-9 A4 230-1 230-2 230

At room temperature, Compound 183-9 (0.0551 g, 0.202 mmol), Compound A4 (0.0602 g, 0.202 mmol) and potassium carbonate (0.0558 g, 0.404 mmol) were added to anhydrous acetonitrile (2.0 mL). The resulting mixture was vigorously stirred at room temperature for 8.0 hours. LCMS showed the generation of a product. The reaction mixture was diluted with ethyl acetate (15.0 mL), filtered under reduced pressure, and the filter cake was washed with ethyl acetate (3.0 mL × 3). The filtrates were combined, and concentrated to dryness under reduced pressure. The resulting residue was purified by silica gel column chromatography (eluent: petroleum ether:ethyl acetate=1:6) to obtain a yellow solid 230-1 (0.0962 g, yield: 90%, purity: 92.96%). LCMS (ESI) m/z 490.2, 492.2, (M+H)+.

At room temperature, Compound 230-1 (0.0962 g, 0.196 mmol), cyclopropylboronic acid (0.0337 g, 0.393 mmol), $Pd(dppf)Cl_2 \cdot CH_2Cl_2$ (0.0160 g, 0.0196 mmol) and $K_2CO_3$ (0.0814 g, 0.589 mmol) were added to a mixture of 1,4-dioxane (1.0 mL) and water (0.50 mL). The resulting mixture was stirred at 100°C for 19 hour. LCMS showed the generation of a product. The reaction mixture was cooled to room temperature, and diluted with dichloromethane (10.0 mL) and water (5.0 mL). The organic phase was separated, and the aqueous phase was extracted with dichloromethane (5.0 mL × 3). The organic phases were combined, dried over anhydrous sodium sulfate, and filtered. The filtrate was concentrated to dryness under reduced pressure. The resulting residue was purified by silica gel column chromatography (eluent: ethyl acetate:petroleum ether=1.5:1-2.5:1) to obtain a brown jelly 230-2 (0.0611 g, yield: 54%, purity: 85.62%): LCMS (ESI) m/z 496.2, (M+H)+.

At room temperature, Compound 230-2 (0.0611 g, 0.123 mmol) and pyridinium p-toluenesulfonate (0.155 g, 0.615 mmol) were added to a mixture of 1,4-dioxane (1.2 mL) and water (1.2 mL). The resulting mixture was stirred at 80°C for 15 hours, then warmed up to 100°C and stirred for 2 hours. LCMS showed the generation of a product. The reaction mixture was cooled to room temperature, diluted with ethyl acetate (15.0 mL), and washed with water (10.0 mL × 3). The organic phase was dried over anhydrous sodium sulfate, and filtered. The filtrate was concentrated to dryness under reduced pressure. The resulting residue was purified by high performance liquid chromatography to obtain an off-white solid 230 (0.0205 g, yield: 40%, purity: 100%). LCMS (ESI) m/z 412.2, (M+H)+; $^1H$ NMR (400 MHz, DMSO) $\delta$ 12.75 (s, 1H), 9.17 (d, J = 1.8 Hz, 1H), 9.07 (d, J = 1.8 Hz, 1H), 8.01 (s, 1H), 7.82 (s, 1H), 7.62 (s, 1H), 7.36 (d, J = 9.0 Hz, 1H), 7.24 (d, J = 1.6 Hz, 1H), 7.08 (dd, J = 9.0, 2.2 Hz, 1H), 6.82 (q, J = 6.4 Hz, 1H), 3.93 (s, 3H), 2.37 - 2.29 (m, 1H), 1.76 (d, J = 6.4 Hz, 3H), 1.14 - 0.89 (m, 4H).

Example 233

7-methyl-5-(1-((7-methyl-3-(1-methyl-1H-pyrazol-4-yl)-1H-indazol-5-yl)oxy)ethyl )pyridino[3,4-b]pyrazine

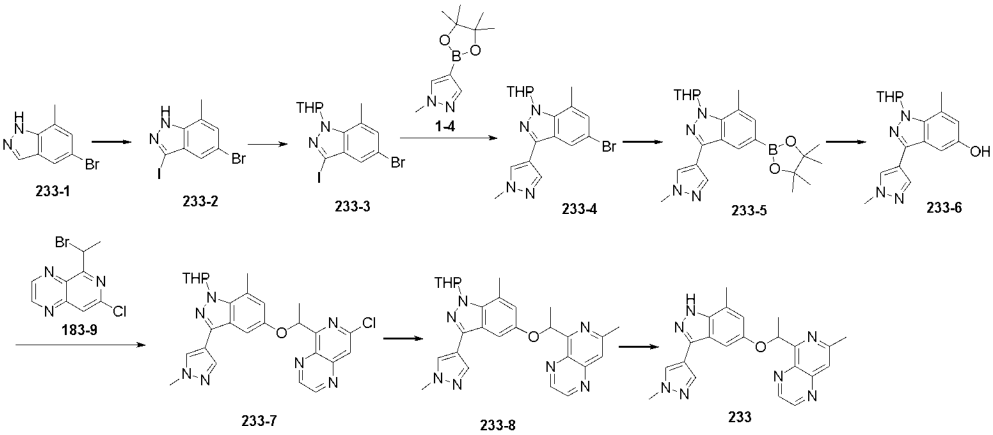

233-1 233-2 233-3 1-4 233-4 233-5 233-6 183-9 233-7 233-8 233

At room temperature, Compound 233-1 (5 g, 23.69 mmol) was dissolved in DMF (50 mL), and $I_2$ (12 g, 47.38 mmol) and KOH (3.9 g, 71.07 mmol) were added successively thereto in an ice bath. The reaction solution was reacted at room temperature for 4 h. LCMS showed the complete reaction of raw materials. The reaction solution was quenched with water, and extracted with ethyl acetate (300 mL x 3). The organic phases were combined and then subjected to rotary drying. The crude was purified by silica gel column chromatography (eluent: petroleum ether:ethyl acetate=5:1) to obtain a product Compound 233-2 as a white solid (7.5 g, purity 95% , 89.3%). LCMS (ESI) m/z 336.01 & 338.01, (M+H)+.

At room temperature, Compound 233-2 (7.5 g, 22.25 mmol) was dissolved in THF (80 mL), and DHP (9.35 g, 111.27 mmol) and p-TsOH (192 mg, 1.11 mmol) were added successively. The reaction solution was stirred at 50°C overnight. LCMS showed the complete reaction of raw materials. The reaction solution was diluted with water and extracted with ethyl acetate (300 mL × 3). The organic phases were combined and subjected to rotary drying. The crude was purified by silica gel column chromatography (eluent: petroleum ether:ethyl acetate=10:1) to obtain a product Compound 233-3 as a white solid (7.2 g, purity 90%): LCMS (ESI) m/z 421.01 & 423.01, (M+H)+.

At room temperature, Compound 233-3 (1.5 g, 3.56 mmol) and Compound 1-4 (741 mg, 3.56 mmol) were dissolved in dioxane /$H_2O$ (20 mL, v/v=4/1), and then $K_2CO_3$ (983 mg, 7.12 mmol) was added. $Pd(dppf)Cl_2 \cdot DCM$ (288 mg, 0.356 mmol) was added under nitrogen protection. The reaction solution was stirred at 80°C overnight. LCMS showed the complete reaction of raw materials. The reaction solution was diluted with water and extracted with ethyl acetate (50 mL × 3). The organic phases were combined and subjected to rotary drying. The crude was purified by silica gel column

chromatography (eluent: petroleum ether:ethyl acetate=1:2) to obtain a product Compound 233-4 as a yellow solid (656 mg, purity 95%, 49.1%): LCMS (ESI) m/z 375.07 & 377.07, (M+H)+.

At room temperature, Compound 233-4 (656 mg, 1.748 mmol) and bis(pinacolato)diboron (532 mg, 2.097 mmol) were dissolved in dioxane (8 mL), and then AcOK (514 mg, 5.244 mmol) was added. Pd(dppf)$Cl_2$·DCM (141 mg, 0.174 mmol) was added under nitrogen protection. The reaction solution was stirred at 80°C overnight. LCMS showed the complete reaction of raw materials. The reaction solution was diluted with water and extracted with ethyl acetate (50 mL × 3). The organic phases were combined and subjected to rotary drying. The crude was purified by silica gel column chromatography (eluent: petroleum ether:ethyl acetate=1:1) to obtain a product 233-5 as a brown oil (685 mg, purity 95% , 88.1%): LCMS (ESI) m/z 423.26, (M+H)+.

At room temperature, Compound 233-5 (685 mg, 1.622 mmol) was dissolved in MeOH (10 mL), and $NaBO_3 \cdot 4H_2O$ (500 mg, 3.244 mmol) was slowly added thereto in an ice bath. The reaction solution was stirred at room temperature for 2 h. LCMS showed the complete reaction of raw materials. The reaction solution was quenched with aqueous NaHSO3 solution, then diluted with water, and extracted with DCM (50 mL x 3). The organic phases were combined and then subjected to rotary drying. The crude was purified by silica gel column chromatography (eluent: petroleum ether:ethyl acetate=1:1) to obtain a product Compound 233-6 as a white solid (421 mg, purity 95%, yield 78.9%): LCMS (ESI) m/z 313.16, (M+H)+.

At room temperature, Compound 233-6 (57 mg, 0.182 mmol) and Compound 183-9 (50 mg, 0.182 mmol) were dissolved in DMF (1 mL), and $K_2CO_3$ (50 mg, 0.365 mmol) was added. The reaction solution was stirred at room temperature for 2 h. LCMS showed the complete reaction of raw materials. The reaction solution was cooled to room temperature, then diluted with water, and extracted with ethyl acetate (20 mL × 3). The organic phases were combined and then subjected to rotary drying. The crude was purified by silica gel column chromatography (eluent: DCM:MeOH=10:1) to obtain a product Compound 233-7 as a brown solid (76 mg, purity 95%, yield 78.49%). LCMS (ESI) m/z 504.18, (M+H)+.

A mixture of Compound 233-7 (76 mg, 0.15 mmol) and methylboronic acid (190 mg, 0.754 mmol, 50% in THF) was dissolved in THF/$H_2O$ (2 mL, v/v=4/1), and then $K_2CO_3$ (42 mg, 0.3 mmol) was added. Pd(dppf)$Cl_2$·DCM (12 mg, 0.015 mmol) was added under nitrogen protection. The reaction solution was stirred at 80°C for 6 h. LCMS showed no remaining raw materials. The reaction solution was diluted with water and then extracted with ethyl acetate (20 mL × 3). The organic phases were combined and subjected to rotary drying, and the crude was purified by silica gel column chromatography (eluent: DCM:MeOH=10:1) to obtain a product Compound 233-8 as a brown oil (65 mg, purity 90%, yield 80.2%). LCMS (ESI) m/z 484.24, (M+H)+.

At room temperature, Compound 233-8 (65 mg, 0.134 mmol) was dissolved in dioxane/H2O (2 mL, v/v=1/1), and pyridine hydrochloride (155 mg, 1.34 mmol) was added. The reaction solution was stirred at 80°C for 24 h. LCMS showed about 23% of raw material was remained, and the extension of reaction time and the supplementation of hydrochloride were both ineffective. The reaction solution was diluted with water and then extracted with DCM (20 mL × 3). The organic phases were combined and then subjected to rotary drying. The crude was purified by preparative high performance liquid chromatography to obtain a product Compound 233 as a yellow solid (12.9 mg, purity 98%, yield 23.5%). LCMS (ESI) m/z 400.18, (M+H)+. $^1$H NMR (400 MHz, DMSO) δ 12.83 (s, 1H), 9.18 (dd, J = 31.6, 1.8 Hz, 2H), 7.96 (s, 1H), 7.83 (s, 1H), 7.58 (s, 1H), 7.07 (s, 1H), 6.92 (s, 1H), 6.81 (q, J = 6.4 Hz, 1H), 3.92 (s, 3H), 2.71 (s, 3H), 2.43 (s, 3H), 1.78 (d, J = 6.4 Hz, 3H).

The following compounds were synthesized according to the the route of Example 233.

| Example | Name | Structure | LC-MS $(M+H)^+$ | $^1$H NMR |
|---|---|---|---|---|
| 204 | 7-methyl-5-(1-((3-(1-methyl-1H-pyrazol-4-yl)-1H-pyrazino[4,3-b]pyridin-5-yl)oxy) ethyl)pyridi no[3,4-b] pyrazine | 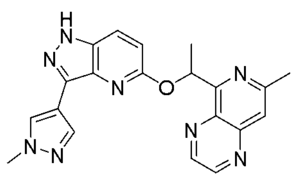 | 397.2 | $^1$H NMR(400 MHz, DMSO) δ 12.91 (s, 1H), 9.25 (d,J = 1.8 Hz, 1H), 9.19 (d,J = 1.7 Hz, 1H), 7.91 (d,J = 9.0 Hz, 1H), 7.74 (s, 1H), 7.71 (s, 1H), 7.46 (s, 1H), 7.40 (d,J = 6.7 Hz, 1H), 7.02 (d,J = 9.0 Hz, 1H), 3.75 (s, 3H), 2.59 (s, 3H), 1.79 (d,J = 6.6 Hz, 3H). |
| 225 | 5-(1-((7-fluoro-3-(1-methyl-1H-pyrazol-4-yl)-1H-indazol-5-yl) oxy)ethyl)-7-methyl-pyridino[3,4 -b]pyra-zine | 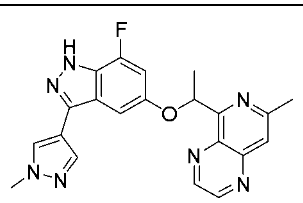 | 404.0 | $^1$H NMR(400 MHz, DMSO) δ 13.32 (s, 1H), 9.22 (d,J = 1.7 Hz, 1H), 9.13 (d,J = 1.7 Hz, 1H), 8.06 (s, 1H), 7.85 (s, 1H), 7.64 (s, 1H), 7.16 (s, 1H), 7.04 (d,J = 12.3 Hz, 1H), 6.85 (d,J = 6.4 Hz, 1H), 3.93 (s, 3H), 2.71 (s, 3H), 1.80 (d,J = 6.4 Hz, 3H) |

(continued)

| Example | Name | Structure | LC-MS $(M+H)^+$ | $^1H$ NMR |
|---|---|---|---|---|
| 231 | 5-(1-((7-methoxy-3-(1-methyl-1H-pyrazol-4-yl)-1H-indazol-5-yl)oxy)ethyl)-7-methylpyrido[3,4-b]pyrazine | 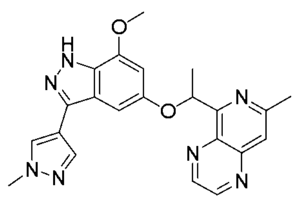 | 416.2 | $^1H$ NMR (400 MHz, DMSO) δ 12.95 (s, 1H), 9.22 (d, J = 4.4 Hz, 1H), 9.15 (d, J = 6.8 Hz, 1H), 7.90 (s, 1H), 7.82 (s, 1H), 7.53 (s, 1H), 6.87 - 6.81 (m, 1H), 6.79 (s, 1H), 6.62 (s, 1H), 3.91 (s, 6H), 2.09 (s, 3H), 1.78 (d, J = 6.4 Hz, 3H). |
| 234 | 5-(1-((6-fluoro-3-(1-methyl-1H-pyrazol-4-yl)-1H-indazol-5-yl)oxy)ethyl)-7-methylpyrido[3,4-b]pyrazine | 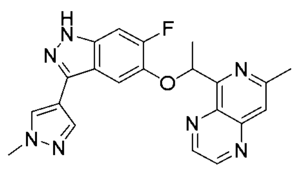 | 404.16 | $^1H$ NMR (400 MHz, DMSO) δ 12.85 (s, 1H), 9.20 (d, J = 1.7 Hz, 1H), 9.09 (d, J = 1.6 Hz, 1H), 8.00 (s, 1H), 7.86 (s, 1H), 7.61 (s, 1H), 7.43 (d, J = 8.0 Hz, 1H), 7.35 (d, J = 11.0 Hz, 1H), 6.86 (d, J = 6.4 Hz, 1H), 3.93 (s, 3H), 2.73 (s, 3H), 1.83 (d, J = 6.4 Hz, 3H). |

Example 235

5-(1-((3-(1-methyl-1H-pyrazol-4-yl)-1H-indazol-5-yl)oxy)ethyl)pyridino[3,4-b]pyr azine-7-carbonitrile

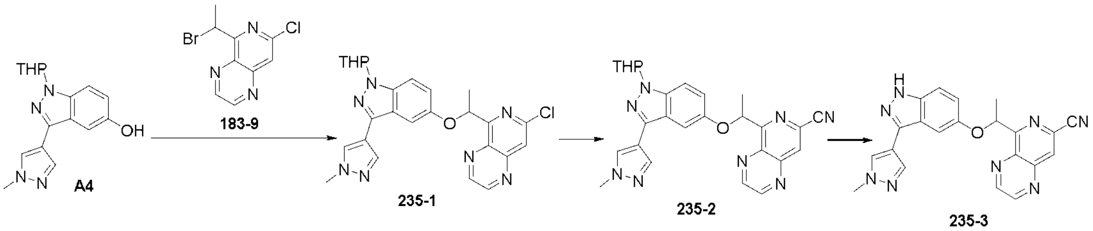


Compound A4 (50 mg, 0.17 mmol, 1.0 eq.) and Compound 183-9 (45.7 mg, 0.17 mmol, 1.0 eq.) were dissolved in DMF (2 mL), and $Cs_2CO_3$ (46.25 mg, 0.33 mmol, 2 eq.) was added, and reacted at 25°C for 4 hours. LCMS showed the complete reaction of Compound A4 and the generation of a product. The reaction mixture was extracted with water and EtOAc. The organic phase was separated, dried by rotary drying, and purified by thin-layer chromatography (developping agent: petroleum ether:ethyl acetate=1:2) to obtain Compound 235-1 as a colorless oil liquid (70 mg, yield 85%). LCMS (ESI) m/z 490.2, (M+H)+).

Compound 235-1 (60 mg, 0.12 mmol, 1.0 eq) and zinc cyanide (43 mg, 0.37 mmol, 3.0 eq) were dissolved in DMF (2 mL), and then $Pd(PPh_3)_4$ (27.7 mg, 0.024 mmol, 0.2 eq) was added, purged with N2 three times, and stirred at 80°C for 16 hours. LCMS showed the complete reaction of most Compound 235-1. The reaction solution was filtered, then was extracted with water and ethyl acetate, separated and dried by rotary drying. The crude was purified by thin-layer chromatography (developping agent: DCM:MeOH=10:1) to obtain Compound 235-2 (50 mg, purity :60%). LCMS (ESI) m/z 481.2, (M+H)+.

Compound 235-2 (50 mg, 0.06 mmol, 1.0 eq.) was dissolved in THF:$H_2O$=1:1 (2 mL), and pyridine hydrochloride (72.1 mg, 0.6 mmol, 10 eq.) was added, and reacted at 80°C for 3 hours. LCMS showed the complete reaction of Compound 235-2 and the generation of a product. The reaction solution was cooled, then extracted with water and ethyl acetate, separated and dried by rotary drying to obtain a crude which was purified by preparative high performance liquid chromatography to obtain Compound 235-3 (7 mg, yield 28.30 %) as a yellow solid. LCMS (ESI) m/z 397.2, (M+H)+); $^1H$ NMR (400 MHz, DMSO) δ 12.78 (s, 1H), 9.42 (d, J = 1.6 Hz, 1H), 9.34 (t, J = 9.2 Hz, 1H), 8.83 (d, J = 6.8 Hz, 1H), 8.08 (s, 1H), 7.68 (d, J = 8.8 Hz, 1H), 7.39 (d, J = 9.2 Hz, 1H), 7.33 (s, 1H), 7.11 (dd, J = 9.2, 2.0 Hz, 1H), 6.86 (d, J = 6.4 Hz, 1H), 3.93 (s, 3H), 1.80 (d, J = 6.4 Hz, 3H).

Example 236

7-(difluoromethyl)-5-(1-((3-(1-methyl-1H-pyrazol-4-yl)-1H-indazol-5-yl)oxy)ethyl )pyridino[3,4-b]pyrazine

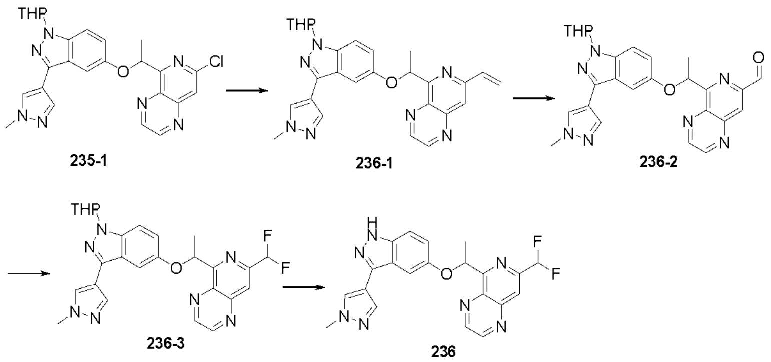

235-1 236-1 236-2 236-3 236

At room temperature, Compound 235-1 (232 mg, 0.473 mmol) and potassium vinyltrifluoroborate (76 mg, 0.568 mmol) were dissolved in THF/$H_2O$ (3 mL, v/v=3/1), and $Na_2CO_3$ (200 mg, 1.894 mmol) was added. Then, $Pd(PPh_3)_4$ (55 mg, 0.047 mmol) was added under nitrogen protection, and purged with nitrogen three times . The reaction solution was stirred at 80°C overnight. LCMS showed the complete reaction of raw materials. The reaction solution was cooled to room temperature, then diluted with water, and extracted with ethyl acetate (20 mL × 3). The organic phases were combined and then subjected to rotary drying. The crude was purified by silica gel column chromatography (eluent: petroleum ether:ethyl acetate=1:10) to obtain a product Compound 236-1 as a yellow oil (178 mg, purity 100%, yield 78.1%). LCMS (ESI) m/z 482.22, (M+H)+.

Compound 236-1 (178 mg, 0.37 mmol) was dissolved in THF/$H_2O$ (4 mL, v/v=1/1), and $NaIO_4$ (158 mg, 0.74 mmol) and $K_2OsO_4 \cdot 2H_2O$ (13 mg, 0.037 mmol) were added successively thereto in an ice bath, and stirred at room temperature for 1 h. LCMS showed the generation of a product 236-2, and no remaining raw materials, and 17% of the byproduct excessively oxidized. The reaction solution was diluted with water and then extracted with ethyl acetate (30 mL × 3). The organic phases were combined and subjected to rotary drying, and the crude was purified by silica gel column chromatography (eluent: DCM:MeOH=10:1) to obtain a product Compound 236-2 as a yellow solid (84 mg, purity 90%, yield 42.3%). LCMS (ESI) m/z 484.21, (M+H)+.

Compound 236-2 (84 mg, 0.17 mmol) was dissolved in DCM (2 mL), cooled to 0°C, and BAST (77 mg, 0.34 mmol) was slowly added dropwise, and stirred at room temperature for 4 h. LCMS showed no remaining raw materials. The reaction solution was quenched with saturated aqueous $NaHCO_3$ solution in an ice bath, and extracted with DCM (20 mL × 3). The organic phases were combined and then subjected to rotary drying, and the crude was purified by silica gel column chromatography (eluent: DCM:MeOH=10:1) to obtain a product Compound 236-3 as a brown oil (60 mg, purity 95%, yield 64.91%). LCMS (ESI) m/z 506.21, (M+H)+.

At room temperature, Compound 236-3 (60 mg, 0.118 mmol) was dissolved in dioxane/$H_2O$ (2 mL, v/v=1/1), and pyridine hydrochloride (137 mg, 1.18 mmol) was added. The reaction solution was stirred at 80°C for 24 h. The reaction solution was diluted with water and then extracted with DCM (20 mL × 3). The organic phases were combined and then subjected to rotary drying. The crude was purified by preparative high performance liquid chromatography to obtain a product Compound 236 as a yellow solid (18.9 mg, purity 100%, yield 37.8%). LCMS (ESI) m/z 422.15, (M+H)+. $^1H$ NMR (400 MHz, DMSO) δ 12.78 (s, 1H), 9.33 (dd, J = 24.2, 1.8 Hz, 2H), 8.28 (s, 1H), 8.07 (s, 1H), 7.68 (s, 1H), 7.41 - 7.09 (m, 4H), 6.89 (q, J = 6.4 Hz, 1H), 3.93 (s, 3H), 1.82 (d, J = 6.4 Hz, 3H).

The following compound was synthesized according to the the route of Example 236.

| Example | Name | Structure | LC-MS $(M+H)^+$ | $^1H$ NMR |
|---|---|---|---|---|
| 232 | 6-(difluoromethyl)-5-(1-((3-(1-methyl-1H-pyrazol-4-yl)-1H-indazol-5-yl)oxy)ethyl) quino xaline | 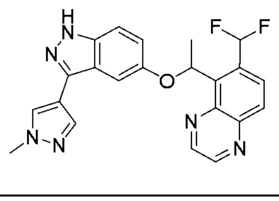 | 421.2 | $_1H$ NMR (400 MHz, DMSO-d6) δ 12.77 (s, 1H), 9.26 (dd, J = 17.9, 1.8 Hz, 2H), 8.22 (d, J = 8.9 Hz, 1H), 8.08 (d, J = 8.9 Hz, 1H), 7.77 (s, 1H), 7.38 (d, J = 8.7 Hz, 2H), 7.23 (q, J = 6.7 Hz, 1H), 7.12 - 7.04 (m, 2H), 3.89 (s, 3H), 1.84 (d, J = 6.7 Hz, 3H). |

Example 241

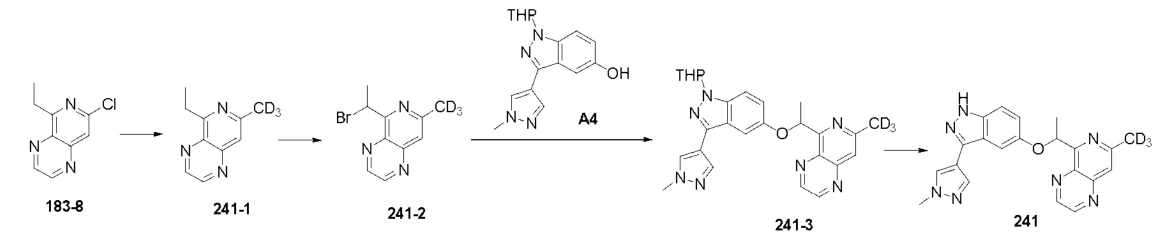


183-8 241-1 241-2 241-3 241

Compound 183-8 (150 mg, 0.77 mmol, 1.0 eq) was dissolved in THF (4.0 mL), [1,3-bis(diphenylphosphino) propane]nickel(II) chloride (630 mg, 1.16 mmol, 1.5 eq) was added, purged with nitrogen, and the compound $CD_3MgI$ (3.9 mL, 3.9 mmol, 5.0 eq) was added, heated to 50°C and reacted for 1 hour. The reaction solution was subjected to suction filtration through diatomite, and the filter cake was washed with EA until there was no product. 20 mL of water was added to the filtrate, and then the filtrate was extracted. The organic phases were combined, dried over anhydrous sodium sulfate, filtered, and concentrated under reduced pressure. The resulting residue was purified by thin-layer chromatography (developping agent: PE/EtOAc = 2/1) to obtain Compound 241-1 (29 mg, yield 21.24%, a light yellow solid). LCMS:(ESI) m/z 177.2, (M+H)+.

Compound 241-1 (29 mg, 0.16 mmol, 1.0 eq), NBS (32 mg, 0.18 mmol, 1.1 eq) and AIBN (3 mg, 0.016 mmol, 0.1 eq) were added to $CCl_4$ (1.0 mL), and stirred at 90°C for 2 hours. The reaction solution was concentrated and then filtered to obtain Compound 241-2 (40 mg, yield 95.3%, as a yellow oil). LCMS:(ESI) m/z 257.2, (M+H)+.

At room temperature, Compound 241-2 (35 mg, 0.11 mmol, 1.0 eq.), Compound A4(35 mg, 0.14 mmol, 1.2 eq.) and cesium carbonate (115 mg, 0.35 mmol, 3.0 eq.) were added successively to DMF (1.0 mL), and stirred at 50°C for 2 hours. The reaction solution was poured into aqueous NaCl solution (20.0 mL), and extracted with EtOAc (20.0 mL × 2). The organic phases were combined, washed with saturated brine (20.0 mL), dried over anhydrous sodium sulfate, filtered, and concentrated under reduced pressure to obtain Compound 241-3 (50 mg, yield 90.2%, a light yellow solid). LCMS:(ESI) m/z 473.2, (M+H)+.

At room temperature, pyridine hydrochloride (61 mg, 0.53 mmol, 5.0 eq.) was added to Compound 241-3 (50 mg, 0.11 mmol, 1.0 eq.) in a mixed solvent of EtOH (1.0 mL) and $H_2O$ (1.0 mL), and stirred at 80°C for 8 hours. Aqueous $NaHCO_3$ solution (10.0 mL) was poured into the reaction solution to adjust the pH to 8, and then the reaction solution was extracted with EtOAc (10.0 mL × 2). The organic phases were combined, washed with saturated brine (10.0 mL), dried over anhydrous sodium sulfate, filtered, and concentrated under reduced pressure. The resulting residue was purified by thin-layer chromatography (developing solvent:DCM/MeOH = 10/1) to obtain Compound 241 (10.0 mg, yield 23.5%, purity 96.78%, a light yellow solid). LCMS:(ESI) m/z 389.2, (M+H)+; $^1H$ NMR (400 MHz, DMSO-d6) δ 12.72 (s, 1H), 9.20 (d, J = 1.8 Hz, 1H), 9.11 (d, J = 1.8 Hz, 1H), 7.99 (s, 1H), 7.81 (s, 1H), 7.61 (d, J = 0.8 Hz, 1H), 7.35 (d, J = 9.0 Hz, 1H), 7.28 (d, J = 2.3 Hz, 1H), 7.08 (dd, J = 9.0, 2.3 Hz, 1H), 6.81 (q, J = 6.4 Hz, 1H), 3.91 (s, 3H), 1.78 (d, J = 6.4 Hz, 3H).

Test Example 1

*In vitro* enzymatic activity inhibition assays

(I) FGFR1 kinase activity inhibition assay

FGFR1 kinase activity was measured using Invitrogen LanthaScreen™ assay technology, which utilized fluorescently-labeled peptides and terbium-labeled antibodies to directly measure an amount of the phosphorylated substrate through time-resolved fluorescence energy transfer (TR-FRET).

① The test compounds were diluted: starting from a concentration of 1 μM, with a buffer solution in a three-fold serial dilution, the buffer solution being composed of 50 mM HEPES, 10 mM $MgCl_2$, 0.01% TritonX-100, 0.01% BSA, 4 mM DTT (pH 7.5), and 1% DMSO;

② 0.01 nM recombinant human GST fusion protein FGFR1's cytoplasmic domain [398-822 (terminal) amino acids] (Carna: 08-133) was formulated into a 2 × solution, mixed well with the test compounds, and incubated at 23°C for 10 minutes;

③ 28 nM Fluorescein-Poly GT peptide substrate (Life Technologies: PV3611) and 28 μM ATP were formulated into a 2 × solution, mixed well with the test compounds and enzyme in ②, and incubated at 23°C for 30 minutes;

④ 10 μL of 2nM Tb-PY20 antibody (Life Technologies: PV3552) and 10 mM EDTA were added to quench the kinase reaction, and incubated at 22°C for 60 minutes.
⑤ The reaction was measured using a PerkinElmer EnVision multimode plate reader through TR-FRET dual wavelength detection.

1. The RFU values were copied from the Envision program.
2. The ratio of RFU 520nm/RFU 495nm was calculated.
3. The value of the ratio was converted to a percentage inhibition value.

$$\% \text{ Inhibition} = (\text{max-data})/(\text{max-min})\times 100$$

"Min" represents the ratio of the control without enzyme, "max" represents the ratio of the DMSO control, and "data" represents the ratio of the compounds in wells with different concentrations.
The results were finally displayed in Excel, and the curves were fitted by XLFit excel plugin version 5.5.0.5, with the $IC_{50}$ calculation formula: Y=Bottom + (Top-Bottom)/(1+($IC_{50}$/X)^HillSlope.
"Top" refers to the maximum response, "Bottom" refers to the minimum response, and "HillSlope" refers to the slope of the curve.

(II) FGFR2, FGFR3 and FGFR4 kinase activity inhibition assays

According to the same method as that in the FGFR1 kinase activity inhibition assay, the inhibitory activities against FGFR2, FGFR3 and FGFR4 were tested using FGFR2's cytoplasmic domain [399-821 (terminal) amino acids] (Carna: 08-134), FGFR3's cytoplasmic domain [436-806 (terminal) amino acids] (Carna: 08-135) and FGFR4's cytoplasmic domain [460-802 (terminal) amino acids] (Carna: 08-136), respectively. Table 1 reports the kinase activity inhibition results and selectivity towards FGFR3 of each compound.

Table 1

| Exa mpl e | $IC_{50}$ (nM) | | | | Selectivity (X) | | |
|---|---|---|---|---|---|---|---|
| | FGFR1 | FGFR2 | FGFR3 | FGFR4 | FGFR1/FGFR3 | FGFR2/FGFR3 | FGFR4/FGFR3 |
| 1 | + | + | + | | A | A | |
| 2 | + | ++ | +++ | + | B | A | B |
| 3 | + | ++ | +++ | + | B | A | A |
| 4 | ++ | +++ | ++++ | +++ | C | A | A |
| 5 | +++ | ++++ | ++++ | +++ | A | A | B |
| 6 | | | + | | | | |
| 7 | + | ++ | +++ | + | B | A | B |
| 8 | + | | + | | A | | |
| 9 | | | + | | | | |
| 10 | ++ | | +++ | | A | | |
| 11 | ++ | +++ | +++ | ++ | A | A | A |
| 12 | ++ | +++ | +++ | ++ | B | A | C |
| 13 | ++ | | +++ | | A | | |
| 14 | ++ | +++ | +++ | ++ | A | A | A |
| 15 | ++ | +++ | +++ | + | A | A | C |
| 16 | ++ | | +++ | | A | | |
| 17 | +++ | ++++ | ++++ | + | A | A | D |
| 18 | +++ | | +++ | | A | | |
| 19 | ++ | | ++ | | A | | |

(continued)

| Exa mpl e | $IC_{50}$ (nM) | | | | Selectivity (X) | | |
|---|---|---|---|---|---|---|---|
| | FGFR1 | FGFR2 | FGFR3 | FGFR4 | FGFR1/FGFR3 | FGFR2/FGFR3 | FGFR4/FGFR3 |
| 20 | +++ | | ++++ | | A | | |
| 21 | + | | ++ | | A | | |
| 22 | ++ | | +++ | | A | | |
| 23 | ++ | +++ | +++ | + | B | A | D |
| 24 | ++ | +++ | ++++ | ++ | A | A | C |
| 25 | + | | + | | B | | |
| 26 | + | | ++ | | B | | |
| 27 | + | ++ | +++ | + | B | A | C |
| 28 | +++ | ++++ | ++++ | +++ | A | A | A |
| 29 | +++ | ++++ | ++++ | +++ | A | A | A |
| 30 | ++ | | ++ | | A | | |
| 31 | +++ | ++++ | ++++ | ++ | B | A | B |
| 32 | +++ | | ++++ | | A | | |
| 33 | | | ++ | | | | |
| 34 | +++ | | +++ | | A | | |
| 35 | +++ | | ++++ | | A | | |
| 36 | ++ | ++ | +++ | | A | A | |
| 37 | +++ | +++ | +++ | | A | A | |
| 38 | | | ++ | | | | |
| 39 | ++ | +++ | +++ | | A | A | |
| 40 | ++ | | +++ | | A | | |
| 41 | ++ | ++ | ++ | | A | A | |
| 42 | ++ | | +++ | | B | | |
| 43 | | | + | | | | |
| 44 | | | + | | | | |
| 45 | + | | ++ | | A | | |
| 46 | ++ | | ++++ | | B | | |
| 47 | | | ++ | | | | |
| 48 | ++ | ++ | +++ | | A | A | |
| 49 | ++ | | +++ | | B | | |
| 50 | ++ | | ++++ | | A | | |
| 51 | | | + | | | | |
| 52 | + | | ++ | | A | | |
| 53 | +++ | | ++++ | | A | | |
| 54 | +++ | | ++++ | | A | | |
| 55 | ++ | | ++++ | | A | | |
| 56 | +++ | | ++++ | | A | | |
| 57 | +++ | | ++++ | | A | | |

(continued)

| Exa mpl e | IC$_{50}$ (nM) | | | | Selectivity (X) | | |
|---|---|---|---|---|---|---|---|
| | FGFR1 | FGFR2 | FGFR3 | FGFR4 | FGFR1/FGFR3 | FGFR2/FGFR3 | FGFR4/FGFR3 |
| 58 | | | | | | | |
| 59 | +++ | ++++ | ++++ | ++ | A | A | B |
| 60 | ++ | +++ | +++ | ++ | A | A | B |
| 61 | ++ | +++ | +++ | ++ | A | A | B |
| 62 | +++ | +++ | ++++ | ++ | A | A | D |
| 63 | ++ | | +++ | | A | | |
| 64 | ++ | | +++ | | A | | |
| 65 | + | + | ++ | | B | C | |
| 66 | | | + | | | | |
| 67 | | | + | | | | |
| 68 | | | + | | | | |
| 69 | | | + | | | | |
| 70 | | | + | | | | |
| 71 | | | + | | | | |
| 72 | | | + | | | | |
| 73 | | | + | | | | |
| 74 | + | + | ++ | + | C | B | D |
| 75 | | | + | | | | |
| 76 | + | | + | | A | | |
| 77 | | | + | | | | |
| 78 | | | + | | | | |
| 79 | | | + | | | | |
| 80 | + | + | ++ | + | D | D | D |
| 81 | | | + | | | | |
| 82 | | | + | | | | |
| 83 | | | + | | | | |
| 84 | | | + | | | | |
| 85 | ++ | +++ | +++ | ++ | A | A | A |
| 86 | | | + | | | | |
| 87 | + | | ++ | | B | | |
| 88 | ++ | +++ | +++ | ++ | A | A | C |
| 89 | | | + | | | | |
| 90 | | | + | | | | |
| 91 | | | ++ | | | | |
| 92 | +++ | +++ | ++++ | +++ | A | A | A |
| 93 | | | ++ | | | | |
| 94 | + | ++ | ++ | + | C | A | B |
| 95 | ++ | +++ | +++ | ++ | C | A | C |

(continued)

| Exa mpl e | IC$_{50}$ (nM) | | | | Selectivity (X) | | |
|---|---|---|---|---|---|---|---|
| | FGFR1 | FGFR2 | FGFR3 | FGFR4 | FGFR1/FGFR3 | FGFR2/FGFR3 | FGFR4/FGFR3 |
| 96 | ++ | | +++ | | A | | |
| 97 | | | + | | | | |
| 98 | + | | ++ | | A | | |
| 99 | + | | ++ | | A | | |
| 100 | ++ | | +++ | | A | | |
| 101 | ++ | | +++ | | A | | |
| 102 | | | +++ | | | | |
| 103 | | | +++ | | | | |
| 104 | | | +++ | | | | |
| 105 | | | ++ | | | | |
| 106 | | | ++ | | | | |
| 107 | + | ++ | +++ | + | B | A | B |
| 108 | | | + | | | | |
| 109 | | | ++ | | | | |
| 110 | ++ | | +++ | | A | | |
| 111 | ++ | | +++ | | A | | |
| 112 | ++ | | ++++ | | A | | |
| 113 | ++ | | +++ | | A | | |
| 114 | | | + | | | | |
| 115 | | | + | | | | |
| 116 | ++ | ++ | +++ | + | B | A | B |
| 117 | | | ++ | | | | |
| 118 | | | ++ | | | | |
| 119 | ++ | +++ | +++ | ++ | B | A | B |
| 120 | + | | +++ | | B | | |
| 121 | | | + | | | | |
| 122 | ++ | +++ | ++++ | ++ | B | A | B |
| 123 | | | + | | | | |
| 124 | ++ | +++ | ++++ | ++ | B | A | B |
| 125 | | | ++ | | | | |
| 126 | ++ | +++ | +++ | + | B | A | C |
| 127 | + | +++ | ++++ | + | C | A | C |
| 128 | + | | +++ | | B | | |
| 129 | +++ | | +++ | | A | | |
| 130 | ++ | +++ | ++++ | ++ | C | A | D |
| 131 | | | +++ | | | | |
| 132 | ++ | +++ | ++++ | ++ | B | A | B |
| 133 | ++ | +++ | ++++ | + | B | A | D |

(continued)

| Exa mpl e | IC$_{50}$ (nM) | | | | Selectivity (X) | | |
|---|---|---|---|---|---|---|---|
| | FGFR1 | FGFR2 | FGFR3 | FGFR4 | FGFR1/FGFR3 | FGFR2/FGFR3 | FGFR4/FGFR3 |
| 134 | + | ++ | +++ | + | C | A | D |
| 135 | ++ | +++ | +++ | ++ | B | A | B |
| 136 | ++ | +++ | ++++ | ++ | B | A | C |
| 137 | | | ++ | | | | |
| 138 | ++ | +++ | ++++ | ++ | C | A | C |
| 139 | + | | +++ | | C | | |
| 140 | +++ | +++ | ++++ | ++ | A | A | B |
| 141 | ++ | | ++++ | | B | | |
| 142 | +++ | +++ | ++++ | ++ | A | A | B |
| 143 | ++ | | +++ | | B | | |
| 144 | + | | ++++ | | C | | |
| 145 | +++ | | ++++ | | A | | |
| 146 | | | +++ | | | | |
| 147 | ++ | | +++ | | A | | |
| 148 | + | | +++ | | C | | |
| 149 | ++ | | ++++ | | B | | |
| 150 | +++ | | ++++ | | A | | |
| 151 | | | ++ | | | | |
| 152 | | | +++ | | | | |
| 153 | | | ++++ | | | | |
| 154 | | | +++ | | | | |
| 155 | ++ | +++ | ++++ | ++ | B | A | B |
| 156 | ++ | +++ | +++ | ++ | B | A | A |
| 157 | ++ | +++ | +++ | ++ | A | A | A |
| 158 | ++ | +++ | +++ | ++ | B | A | C |
| 159 | + | ++ | +++ | | B | A | |
| 160 | + | | +++ | | B | | |
| 161 | ++ | ++ | +++ | ++ | B | A | B |
| 162 | ++ | ++ | +++ | | A | A | |
| 163 | ++ | ++ | +++ | | A | A | |
| 164 | | | +++ | | | | |
| 165 | | | ++ | | | | |
| 166 | | | ++ | | | | |
| 167 | | | ++ | | | | |
| 168 | ++ | | +++ | | A | | |
| 169 | ++ | | +++ | | A | | |
| 170 | +++ | +++ | ++++ | ++ | A | A | C |
| 171 | ++ | +++ | ++++ | ++ | B | A | B |

(continued)

| Exa mpl e | IC$_{50}$ (nM) | | | | Selectivity (X) | | |
|---|---|---|---|---|---|---|---|
| | FGFR1 | FGFR2 | FGFR3 | FGFR4 | FGFR1/FGFR3 | FGFR2/FGFR3 | FGFR4/FGFR3 |
| 172 | | | ++ | | | | |
| 173 | +++ | +++ | ++++ | ++ | A | A | B |
| 174 | ++ | +++ | ++++ | ++ | B | B | D |
| 175 | +++ | | ++++ | | A | | |
| 176 | ++ | | +++ | | A | | |
| 177 | ++ | | +++ | | B | | |
| 178 | +++ | | ++++ | | B | | |
| 179 | ++ | | ++++ | | B | | |
| 180 | +++ | | +++ | | A | | |
| 181 | ++ | | ++++ | ++ | A | | A |
| 182 | ++ | | +++ | ++ | A | | A |
| 183 | ++ | +++ | ++++ | ++ | B | A | B |
| 184 | ++ | | ++++ | +++ | B | | A |
| 185 | ++ | | ++++ | ++ | B | | B |
| 186 | +++ | | ++++ | | A | | A |
| 187 | ++ | ++ | +++ | ++ | B | A | B |
| 188 | ++ | +++ | ++++ | ++ | B | A | B |
| 189 | + | ++ | ++++ | ++ | D | B | C |
| 190 | ++ | +++ | ++++ | ++ | B | A | B |
| 191 | ++ | +++ | ++++ | ++ | C | A | C |
| 192 | ++ | | ++++ | | B | | |
| 193 | +++ | | ++++ | | A | | |
| 194 | ++ | +++ | ++++ | ++ | B | A | C |
| 195 | ++ | +++ | ++++ | ++ | B | A | B |
| 196 | ++ | | ++++ | | B | | |
| 197 | ++ | | ++++ | | B | | |
| 198 | ++ | | ++++ | | B | | |
| 199 | ++ | | ++++ | | A | | |
| 200 | ++ | | +++ | | B | | |
| 201 | +++ | | ++++ | | A | | |
| 202 | +++ | | ++++ | | A | | |
| 203 | ++ | ++ | ++++ | ++ | B | B | C |
| 204 | +++ | | ++++ | | A | | |
| 205 | +++ | | ++++ | | B | | |
| 206 | ++ | | +++ | | C | | |
| 207 | ++ | | ++++ | | B | | |
| 208 | ++ | | ++++ | | B | | |
| 209 | ++ | | ++++ | | B | | |

(continued)

| Exa mpl e | $IC_{50}$ (nM) | | | | Selectivity (X) | | |
|---|---|---|---|---|---|---|---|
| | FGFR1 | FGFR2 | FGFR3 | FGFR4 | FGFR1/FGFR3 | FGFR2/FGFR3 | FGFR4/FGFR3 |
| 210 | +++ | | ++++ | | A | | |
| 211 | ++ | | ++++ | | B | | |
| 212 | ++ | +++ | ++++ | ++ | B | B | D |
| 213 | ++ | +++ | ++++ | ++ | C | A | C |
| 214 | ++ | | ++++ | | B | | |
| 215 | ++ | | ++++ | | B | | |
| 216 | +++ | | ++++ | | A | | |
| 217 | ++ | | ++++ | | C | | |
| 218 | ++ | +++ | ++++ | ++ | B | A | B |
| 219 | ++ | | ++++ | | A | | |
| 220 | ++ | | ++++ | | C | | |
| 221 | +++ | | ++++ | | B | | |
| 222 | ++ | | ++++ | | B | | |
| 223 | ++ | ++ | ++++ | + | B | B | D |
| 224 | ++ | +++ | ++++ | ++ | B | C | D |
| 225 | +++ | | ++++ | | A | | |
| 226 | + | | +++ | | B | | |
| 227 | +++ | | ++++ | | A | | |
| 228 | ++ | | +++ | | B | | |
| 229 | ++ | | ++++ | | B | | |
| 230 | +++ | | ++++ | | A | | |
| 231 | | | +++ | | | | |
| 232 | +++ | | ++++ | | A | | |
| 233 | ++ | +++ | +++ | + | B | A | C |
| 234 | +++ | ++++ | ++++ | +++ | A | A | B |
| 235 | | | ++ | | | | |
| 236 | +++ | +++ | ++++ | ++ | A | A | C |
| 237 | ++ | | +++ | | B | | |
| 238 | ++ | | ++++ | | B | | |
| 239 | ++ | | +++ | | C | | |
| 240 | ++ | | ++++ | | B | | |
| 241 | +++ | ++++ | ++++ | +++ | B | A | A |

"++++" represents $IC_{50}$<1 nM, "+++" represents 1 nM ≤ $IC_{50}$<10 nM, "++" represents 10 nM ≤ $IC_{50}$<100 nM, and "+" represents 100 nM ≤ $IC_{50}$;
A represents 1 ≤ multiple X<20, B represents 20 ≤ multiple X<50, C represents 50 ≤ multiple X<100, and D represents 100 ≤ multiple X.
Conclusion: in the kinase activity inhibition assays, the compounds of the present invention have exhibited strong inhibitory activity, particularly against FGFR3 kinase.

Test Example 2

Experiment of inhibition of compounds on proliferation activity of Ba/F3-BCR-FGFR1 and Ba/F3-FGFR3-BAIAP2L1 cells

The present experiment used CellTiter-Glo® Luminescent Cell Viability Assay (Promega: G7573) kit, which was a homogeneous cell viability detection method and determined the cell viability of cultured cells by quantifying ATP.

① Ba/F3-BCR-FGFR1 or Ba/F3-FGFR3-BAIAP2L1 cells were cultured in 1640 complete medium (Gibco: 11879020) (10% FBS (Gibco: 10091-148)+1% Pen Strep (Gibco: 15140122)) under the condition of 37°C and 5% $CO_2$.
(2) The cells in logarithmic growth phase were harvested and counted using a platelet counter. The trypan blue exclusion method was used to detect cell viability to ensure the cell viability was above 90%.
③ The cell density was adjusted using 1640 complete medium, and then, the cells were seeded into a 96-well cell culture plate at 90 μL/well with a total of 2000 cells.
④ A 10-fold drug solution was prepared starting from a maximum drug concentration of 10 μM and 4-fold serially diluted into10 concentrations. Then, 10 μL of each of the serially diluted compounds was transferred to the corresponding experimental wells of a 96-well cell plate, with two replicates for each drug concentration.
⑤ The 96-well plate with the cells that had been treated with the drugs was placed under the condition of 37°C and 5% $CO_2$ to be further cultured for 72 hours, and then analyzed by CTG.
⑥ The CTG reagent was melted and the cell plate was equilibrated to room temperature for 30 minutes.
⑦ 50 μL of the CTG solution was added to each well.
⑧ The cell plate was shaken on an orbital shaker for 5 minutes to lyse the cells.
⑨ The cell plate was placed at room temperature for 20 minutes to stabilize the luminescence signal. The luminescence values were read to collect the data.
⑩ The data were analyzed using XLFit excel plugin version 5.5.0.5, and fitted with non-linear S-curve regression to obtain the dose-response curves and thus calculate the $IC_{50}$ values.

$$Y=Bottom + (Top-Bottom)/(1+(IC_{50}/X)^\wedge HillSlope$$

"Top" refers to the maximum response, "Bottom" refers to the minimum response, and "HillSlope" refers to the slope of the curve.

$$\text{Cell inhibition rate (\%)}=(Max- Lum_{test\ drug})/\ (Max-Min) \times 100\%.$$

"Max" represents the average value of the cell control+DMSO, and "Min" represents the average value of the medium control+DMSO.

Table 2 reports the experiment results of inhibition of the compounds on proliferation activity of Ba/F3-BCR-FGFR1 and Ba/F3-FGFR3-BAIAP2L1 cells.

Table 2

| Example | $IC_{50}$ (nM) | | Selectivity (X) |
|---|---|---|---|
| | Ba/F3-BCR-FGFR1 | Ba/F3-FGFR3-BAIAP2L1 | FGFR1/FGFR3 |
| **12** | 592.5 | 4.6 | 129 |
| **29** | 38.0 | 0.3 | 127 |
| **54** | 176.7 | 1.2 | 147 |
| **55** | 88.5 | 0.7 | 126 |
| **85** | 614.6 | 5.4 | 114 |
| **88** | 3417.8 | 19 | 180 |
| **92** | 98.6 | 2.8 | 35 |
| **95** | 406.3 | 8 | 51 |
| **96** | 430.5 | 11.8 | 37 |
| **98** | 2140.4 | 47.6 | 45 |
| **99** | 1762.2 | 41.8 | 42 |

(continued)

| Example | $IC_{50}$ (nM) | | Selectivity (X) |
|---|---|---|---|
| | Ba/F3-BCR-FGFR1 | Ba/F3-FGFR3-BAIAP2L1 | FGFR1/FGFR3 |
| **100** | 1152.2 | 26.8 | 43 |
| **101** | 246.4 | 10.6 | 23 |
| **127** | 941.1 | 4.8 | 195 |
| **130** | 1194.4 | 12.9 | 92 |
| **136** | 376.8 | 3.4 | 111 |
| **183** | 55.3 | 0.4 | 139 |
| **188** | 276.6 | 3.1 | 91 |
| **190** | 424.7 | 3.8 | 113 |
| **218** | 440.9 | 8.4 | 53 |
| **227** | 390.2 | 3.1 | 126 |
| **236** | 161.3 | 0.9 | 182 |
| **241** | 62.3 | 0.8 | 76 |
| Conclusion: the compounds of the present invention have exhibited good inhibitory activity against proliferation of Ba/F3-FGFR3-BAIAP2L1 cells and good selectivity towards Ba/F3-BCR-FGFR1 cells. | | | |

Test Example 3

Experiment of inhibition of compounds on proliferation activity of RT112 cells (bladder cancer cells)

The present experiment used CellTiter-Glo® Luminescent Cell Viability Assay (Promega: G7573) kit, which was a homogeneous cell viability detection method and determined the cell viability of cultured cells by quantifying ATP.

① RT112 cells were cultured in MEM complete medium (Gibco: 51200038) (10% FBS (Gibco: 10091-148)+1% Pen Strep (Gibco: 15140122)+1% NEAA (Gibco: 11140050)) under the condition of 37°C and 5% $CO_2$.
(2) The cells in logarithmic growth phase were harvested and counted using a platelet counter. The trypan blue exclusion method was used to detect cell viability to ensure the cell viability was above 90%.
③ The cell density was adjusted using MEM complete medium, and then, the cells were seeded into a 96-well cell culture plate at 90 μL/well with a total of 3000 cells.
④ The 96-well plate with the cells was placed under the condition of 37°C and 5% $CO_2$ to be cultured for 24 hours.
⑤ 24 hours later, a 10-fold drug solution was prepared starting from a maximum drug concentration of 10 μM and 4-fold serially diluted into10 concentrations. Then, 10 μL of each of the serially diluted compounds was transferred to the corresponding experimental wells of a 96-well cell plate, with two replicates for each drug concentration.
⑥ The 96-well plate with the cells that had been treated with the drugs was placed under the condition of 37°C and 5% $CO_2$ to be further cultured for 72 hours, and then analyzed by CTG.
⑦ The CTG reagent was melted and the cell plate was equilibrated to room temperature for 30 minutes.
⑧ 50 μL of the CTG solution was added to each well.
⑨ The cell plate was shaken on an orbital shaker for 5 minutes to lyse the cells.
⑩ The cell plate was placed at room temperature for 20 minutes to stabilize the luminescence signal. The luminescence values were read to collect the data.
⑪ The data were analyzed using XLFit excel plugin version 5.5.0.5, and fitted with non-linear S-curve regression to obtain the dose-response curves and thus calculate the $IC_{50}$ values.

$$Y=Bottom + (Top-Bottom)/(1+(IC_{50}/X)^\wedge HillSlope$$

"Top" refers to the maximum response, "Bottom" refers to the minimum response, and "HillSlope" refers to the slope of the curve.

$$\text{Cell inhibition rate (\%)}=(\text{Max- Lum}_{\text{test drug}})/\ (\text{Max-Min})\times 100\%$$

"Max" represents the average value of the cell+DMSO control, and "Min" represents the average value of the medium control+DMSO.

Table 3 reports the experiment results of inhibition of the compounds on proliferation activity of RT112 cells (bladder cancer cells).

Table 3

| Example | $IC_{50}$ (nM) RT112 |
|---|---|
| **12** | 43.1 |
| **29** | 3.6 |
| **54** | 14 |
| **55** | 15.9 |
| **85** | 58.8 |
| **88** | 166.6 |
| **92** | 24.3 |
| **95** | 23.4 |
| **101** | 85 |
| **127** | 25.5 |
| **130** | 37.9 |
| **136** | 47.5 |
| **183** | 9.7 |
| **188** | 10.7 |
| **190** | 18.6 |
| **218** | 16.7 |
| **227** | 34.5 |
| **236** | 15.5 |
| **241** | 13.5 |
| Conclusion: the compounds of the invention have exhibited good inhibitory activity against proliferation of RT112 cells (bladder cancer cells), and have showed significant inhibitory effect on tumor growth in a RT112 mouse tumor model. | |

Test Example 4

Experiment of inhibition of compounds on proliferation activity of RT112-FGFR3-V555M cells

The present experiment used CellTiter-Glo® Luminescent Cell Viability Assay (Promega: G7573) kit, which was a homogeneous cell viability detection method and determined the cell viability of cultured cells by quantifying ATP.

① RT112-FGFR3-V555M cells were cultured in MEM complete medium (Gibco: 51200038) (10% FBS (Gibco: 10091-148)+1% Pen Strep (Gibco: 15140122)+1% NEAA (Gibco: 11140050)) under the condition of 37°C and 5% $CO_2$.
(2) The cells in logarithmic growth phase were harvested and counted using a platelet counter. The trypan blue exclusion method was used to detect cell viability to ensure the cell viability was above 90%.
③ The cell density was adjusted using MEM complete medium, and then, the cells were seeded into a 96-well cell culture plate at 90 μL/well with a total of 5000 cells.
④ The 96-well plate with the cells was placed under the condition of 37°C and 5% $CO_2$ to be cultured for 24 hours.
⑤ 24 hours later, a 10-fold drug solution was prepared starting from a maximum drug concentration of 10 μM and 4-fold serially diluted into10 concentrations. Then, 10 μL of each of the serially diluted compounds was transferred to the

corresponding experimental wells of a 96-well cell plate, with two replicates for each drug concentration.

⑥ The 96-well plate with the cells that had been treated with the drugs was placed under the condition of 37°C and 5% $CO_2$ to be further cultured for 72 hours, and then analyzed by CTG.

⑦ The CTG reagent was melted and the cell plate was equilibrated to room temperature for 30 minutes.

⑧ 50 μL of the CTG solution was added to each well.

⑨ The cell plate was shaken on an orbital shaker for 5 minutes to lyse the cells.

⑩ The cell plate was placed at room temperature for 20 minutes to stabilize the luminescence signal. The luminescence values were read to collect the data.

⑪ The data were analyzed using XLFit excel plugin version 5.5.0.5, and fitted with non-linear S-curve regression to obtain the dose-response curves and thus calculate the $IC_{50}$ values.

$$Y=Bottom + (Top-Bottom)/(1+(IC50/X)^HillSlope$$

"Top" refers to the maximum response, "Bottom" refers to the minimum response, and "HillSlope" refers to the slope of the curve.

$$\text{Cell inhibition rate (\%)}=(\text{Max- Lum}_{\text{test drug}})/\ (\text{Max-Min})\times 100\%$$

"Max" represents the average value of the cell+DMSO control, and "Min" represents the average value of the medium control+DMSO.

Table 4 reports the experiment results of inhibition of the compounds on proliferation activity of RT112-FGFR3-V555M cells.

Table 4

| Example | $IC_{50}$ (nM) RT112-FGFR3-V555M |
|---|---|
| **85** | 334 |
| **95** | 110 |
| **127** | 117.9 |
| **130** | 152.2 |
| **136** | 118.8 |
| **183** | 10.4 |
| **188** | 70.1 |
| **190** | 81.6 |
| **218** | 64.7 |
| **227** | 127.7 |
| **236** | 38.9 |
| **241** | 18.2 |
| Conclusion: the compounds of the invention have exhibited good inhibitory activity against proliferation of RT112-FGFR3-V555M mutated cells, and have showed significant inhibitory effect on tumor growth in a RT112-FGFR3-V555M mouse tumor model, thereby having significant advantages over the first generation FGFR inhibitors in overcoming drug-resistant mutations. | |

## Claims

**1.** A compound of formula (I),

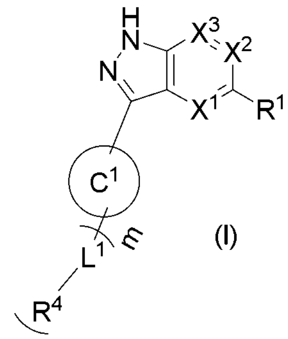

(I)

or a pharmaceutically acceptable salt, hydrate, solvate, isotope substitute or stereoisomer thereof, wherein,

$X^1$ is N atom or $CR^5$;
$X^2$ is N atom or $CR^2$;
$X^3$ is N atom or $CR^3$;
$R^1$ is selected from the group consisting of

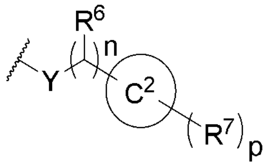

,

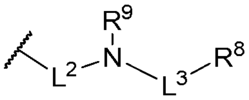

and

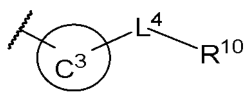

;

Y is selected from the group consisting of O atom, S atom, -S(O)-, $-S(O)_2-$ and $NR^{11}$; $R^2$ and $R^5$ are each independently selected from the group consisting of H atom, - OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of $C_{1-6}$ alkoxy, -OH, -COOH, $-NR^{11}R^{12}$, -CN, halogen, $C_{1-6}$ haloalkyl, $C_{1-6}$ haloalkoxy and $C_{1-6}$ hydroxyalkyl;
$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$;
provided that $R^3$ is not H atom when X is $CR^5$, $R^1$ is

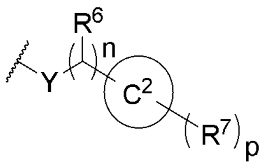

and $C^2$ is a 6 membered nitrogen-containing heteroaryl ring;
ring $C^1$ is selected from the group consisting of $C_{6-10}$ aryl, 5 to 10 membered heteroaryl, $C_{3-8}$ cycloalkyl, 3 to 8 membered heterocyclyl, 5 to 10 membered heteroaryl fused with 3 to 8 membered heterocyclyl, 5 to 10 membered heteroaryl fused with $C_{6-10}$ aryl, 3 to 8 membered heterocyclyl fused with 5 to 10 membered heteroaryl, and $C_{6-10}$ aryl fused with 5 to 10 membered heteroaryl;
each $L^1$ is independently selected from the group consisting of chemical bond, $-C_{1-6}$ alkylene-, -O-, $-C_{1-6}$ alkylene-O-, $-O-C_{1-6}$ alkylene-, -C(O)-, -O-C(O)-, -C(O)-O-, -S-, - S(O)-, $-S(O)_2-$, $-S(O)(=NR^{11})-$, $-C_{1-6}$ alkylene-C(O)-, $-C(O)-C_{1-6}$ alkylene-, $-C_{1-6}$ alkylene-$S(O)_2$-, $-S(O)_2-C_{1-6}$ alkylene-, $-C_{1-6}$ alkylene-$S(O)(=NR^{11})$-, -S(O)(=NR)-$C_{1-6}$ alkylene-, 3 to 8 membered heterocyclylene, $C_{3-8}$ cycloalkylene, $C_{6-10}$ arylene, 5 to 10 membered

heteroarylene, -$C_{1-6}$ alkylene-O-$C_{1-6}$ alkylene-O-, -O-$C_{1-6}$ alkylene-O-$C_{1-6}$ alkylene-, -$NR^{11}$-, -$NR^{11}$-$C_{1-6}$ alkylene-, -$C_{1-6}$ alkylene-$NR^{11}$-, -$C_{1-6}$ alkylene-$NR^{11}$-$C_{1-6}$ alkylene-O-, -O-$C_{1-6}$ alkylene-$NR^{11}$-$C_{1-6}$ alkylene-, -NH-C(O)-, -C(O)-$NR^{11}$-, -C(O)-$NR^{11}$-$C_{1-6}$ alkylene-, -$C_{1-6}$ alkylene-C(O)-$NR^{11}$-, -$C_{1-6}$ alkylene-$NR^{11}$-C(O)- and -$NR^{11}$-C(O)-$C_{1-6}$ alkylene-, wherein the $C_{1-6}$ alkylene is each independently and optionally substituted by one or more substituents selected from the group consisting of $C_{1-6}$ alkyl, halogen, -OH, -COOH, -$NR^{11}R^{12}$, -CN, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;

each $R^4$ is independently selected from the group consisting of H atom, -OH, - COOH, -CN, halogen, $C_{1-6}$ alkyl, $C_{1-6}$ deuterated alkyl, $C_{2-6}$ alkenyl, $C_{2-6}$ alkynyl, $C_{1-6}$ alkoxy, $C_{1-6}$ haloalkyl, $C_{1-6}$ haloalkoxy, -C(O)-$NR^{11}R^{12}$, -$NR^{11}R^{12}$, $C_{1-6}$ hydroxyalkyl, $C_{6-10}$ aryl, 5 to 10 membered heteroaryl, $C_{3-8}$ cycloalkyl and 3 to 10 membered heterocyclyl, wherein the $C_{6-10}$ aryl, 5 to 10 membered heteroaryl, $C_{3-8}$ cycloalkyl and 3 to 10 membered heterocyclyl are each independently and optionally substituted by one or more substituents selected from the group consisting of -CN, -OH, $C_{1-6}$ alkyl, $C_{1-6}$ hydroxyalkyl, -C(O)-$R^{14}$, -C(O)-$NR^{11}R^{12}$, oxo, $C_{1-6}$ alkoxy, -$S(O)_2$-$C_{1-6}$ alkyl, -$NR^{11}R^{12}$, imino, halogen, -$C_{1-6}$ alkylene-$NR^{11}R^{12}$, -$NR^{11}$-C(O)-$R^{14}$, -$NR^{11}$-$S(O)_2$-$R^{14}$, -S(O)(=$NR^{11}$)-$R^{14}$, -$S(O)_2$-$R^{14}$, -C(=$NR^{11}$)-$NR^{11}R^{12}$, -$S(O)_2$-$NR^{11}R^{12}$ and -$C_{1-6}$ alkylene-C(O)-$NR^{11}R^{12}$;

ring $C^2$ is selected from the group consisting of $C_{6-10}$ aryl, 5 to 10 membered heteroaryl, $C_{3-8}$ cycloalkyl, 3 to 8 membered heterocyclyl, $C_{3-8}$ cycloalkyl fused with 5 to 10 membered heteroaryl, 5 to 10 membered heteroaryl fused with $C_{6-10}$ aryl, $C_{6-10}$ aryl fused with 3 to 8 membered heterocyclyl, 5 to 10 membered heteroaryl fused with $C_{3-8}$ cycloalkyl, $C_{6-10}$ aryl fused with 5 to 10 membered hereroaryl, and 3 to 8 membered heterocyclyl fused with $C_{6-10}$ aryl;

ring $C^3$ is selected from the group consisting of $C_{6-10}$ aryl, 5 to 10 membered heteroaryl, $C_{3-8}$ cycloalkyl, 3 to 8 membered heterocyclyl, $C_{3-8}$ cycloalkyl fused with 5 to 10 membered heteroaryl, 5 to 10 membered heteroaryl fused with $C_{6-10}$ aryl, 5 to 10 membered heteroaryl fused with $C_{3-8}$ cycloalkyl, and $C_{6-10}$ aryl fused with 5 to 10 membered heteroaryl;

$R^6$ is selected from the group consisting of H atom, -OH, -COOH, -$NR^{11}R^{12}$, -CN, halogen, $C_{1-6}$ alkyl, $C_{1-6}$ deuterated alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, -$C_{1-6}$ alkylene-$NR^{11}R^{12}$, $C_{6-10}$ aryl, 5 to 10 membered heteroaryl, $C_{3-8}$ cycloalkyl and 3 to 8 membered heterocyclyl;

$R^7$ is selected from the group consisting of H atom, -OH, -COOH, -C(O)-$R^{14}$, -C(O)-$NR^{11}R^{12}$, -$NR^{11}R^{12}$, -CN, halogen, oxo, $C_{1-6}$ alkyl, $C_{1-6}$ deuterated alkyl, $C_{2-6}$ alkenyl, $C_{2-6}$ alkynyl, $C_{1-6}$ alkoxy, $C_{1-6}$ deuterated alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;

$L^2$ is selected from the group consisting of -C(O)-, -$S(O)_2$- and -S(O)-;

$L^3$ and $L^4$ are each independently selected from the group consisting of chemical bond, -$C_{1-6}$ alkylene-, -O-, -$C_{1-6}$ alkylene-O-, -O-$C_{1-6}$ alkylene-, -C(O)-, -O-C(O)-, -C(O)-O-, -S-, -S(O)-, -$S(O)_2$-, -$C_{1-6}$ alkylene-C(O)-, -C(O)-$C_{1-6}$ alkylene-, -$C_{1-6}$ alkylene-$S(O)_2$-, -$S(O)_2$-$C_{1-6}$ alkylene-, -$C_{1-6}$ alkylene-O-$C_{1-6}$ alkylene-O-, -O-$C_{1-6}$ alkylene-O-$C_{1-6}$ alkylene-, -$NR^{11}$-, -$NR^{11}$-$C_{1-6}$ alkylene-, -$C_{1-6}$ alkylene-$NR^{11}$-, -$C_{1-6}$ alkylene-$NR^{11}$-$C_{1-6}$ alkylene-O-, -O-$C_{1-6}$ alkylene-$NR^{11}$-$C_{1-6}$ alkylene-, -$NR^{11}$-C(O)-, -C(O)-$NR^{11}$-, -C(O)-$NR^{11}$-$C_{1-6}$ alkylene-, -$C_{1-6}$ alkylene-C(O)-$NR^{11}$-, -$C_{1-6}$ alkylene-$NR^{11}$-C(O)- and -$NR^{11}$-C(O)-$C_{1-6}$ alkylene-, wherein the $C_{1-6}$ alkylene is each independently and optionally substituted by one or more substituents selected from the group consisting of $C_{1-6}$ alkyl, halogen, -OH, -COOH, -$NR^{11}R^{12}$, -CN, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;

$R^8$ and $R^{10}$ are each independently selected from the group consisting of H atom, - OH, -COOH, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of -CN, -OH, $C_{1-6}$ alkyl, -C(O)-$C_{1-6}$ alkyl, -C(O)-$C_{1-6}$ hydroxyalkyl, - C(O)-$NR^{11}R^{12}$, oxo, $C_{1-6}$ alkoxy, -$S(O)_2$-$C_{1-6}$ alkyl, -$NR^{11}R^{12}$ and halogen;

$R^9$ is selected from the group consisting of H atom, $C_{1-6}$ alkyl, $C_{1-6}$ alkoxy, $C_{1-6}$ haloalkyl, $C_{1-6}$ haloalkoxy and $C_{1-6}$ hydroxyalkyl;

each $R^{11}$ is independently selected from the group consisting of H atom, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $C_{3-8}$ cycloalkyl, wherein the $C_{3-8}$ cycloalkyl is optionally substituted by one or more substituents selected from the group consisting of $C_{1-6}$ alkyl, $C_{1-6}$ alkoxy, -OH, -COOH, - $NH_2$, -CN, halogen, $C_{1-6}$ haloalkyl, $C_{1-6}$ haloalkoxy and $C_{1-6}$ hydroxyalkyl;

each $R^{12}$ is independently selected from the group consisting of H atom, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $C_{3-8}$ cycloalkyl, wherein the $C_{3-8}$ cycloalkyl is optionally substituted by one or more substituents selected from the group consisting of $C_{1-6}$ alkyl, $C_{1-6}$ alkoxy, -OH, -COOH, - $NH_2$, -CN, halogen, $C_{1-6}$ haloalkyl, $C_{1-6}$ haloalkoxy and $C_{1-6}$ hydroxyalkyl;

or, $R^{11}$ and $R^{12}$ together with the N atom to which they are attached form 3 to 8 membered heterocyclyl, wherein

the 3 to 8 membered heterocyclyl is optionally substituted by one or more substituents selected from the group consisting of $C_{1-6}$ alkyl, $C_{1-6}$ alkoxy, -OH, -COOH, $-NH_2$, -CN, halogen, $C_{1-6}$ haloalkyl, $C_{1-6}$ haloalkoxy and $C_{1-6}$ hydroxyalkyl;

$R^{13}$ is selected from the group consisting of 3 to 8 membered heterocyclyl, $C_{3-8}$ cycloalkyl, $C_{6-10}$ aryl and 5 to 10 membered heteroaryl, wherein the 3 to 8 membered heterocyclyl, $C_{3-8}$ cycloalkyl, $C_{6-10}$ aryl and 5 to 10 membered heteroaryl are each independently and optionally substituted by one or more substituents selected from the group consisting of $C_{1-6}$ alkoxy, -OH, -COOH, $-NR^{11}R^{12}$, -CN, halogen, $C_{1-6}$ haloalkyl, $C_{1-6}$ haloalkoxy and $C_{1-6}$ hydroxyalkyl;

each $R^{14}$ is independently selected from the group consisting of H atom, $C_{1-6}$ alkyl, $C_{1-6}$ alkoxy, $C_{1-6}$ haloalkoxy and $C_{3-8}$ cycloalkyl, wherein the $C_{1-6}$ alkyl and $C_{3-8}$ cycloalkyl are each independently and optionally substituted by one or more substituents selected from the group consisting of -CN, -OH, -COOH, $-NR^{11}R^{12}$, halogen and $C_{1-6}$ alkoxy;

m is 0, 1 or 2;

n is 0 or 1; and

p is 0, 1 or 2.

**2.** The compound according to claim 1, being a compound of formula (II),

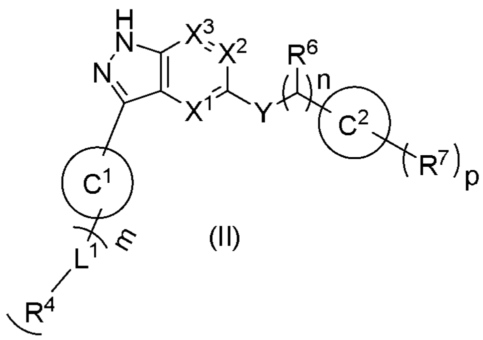

(II)

wherein, $X^1$, $X^2$, $X^3$, Y, $C^1$, $C^2$, $R^4$, $R^6$, $R^7$, $L^1$, m, n and p are as defined in claim 1.

**3.** The compound according to claim 1 or 2, being a compound of formula (II-1),

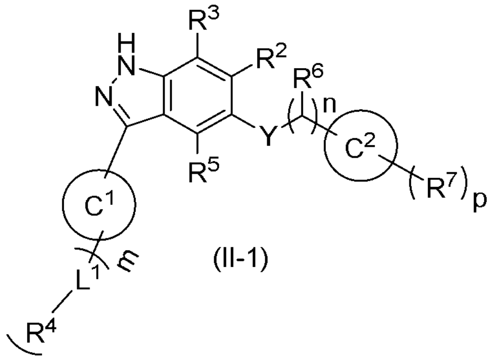

(II-1)

wherein,

Y is selected from the group consisting of O atom, S atom, -S(O)-, $-S(O)_2-$, NH and N-$C_{1-6}$ alkyl;

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$;

$C^1$, $C^2$, $R^2$-$R^7$, $R^{11}$-$R^{13}$, $L^1$, m, n and p are as defined in claim 1;

provided that $R^3$ is not H atom when $C^2$ is 6 membered nitrogen-containing heteroaryl ring, preferably pyridyl, pyridazinyl or pyrimidinyl;

particularly,

$R^3$ is selected from the group consisting of H atom, $-N(C_{1-6}\ alkyl)_2$, $-NH_2$, $-NH(C_{1-6}\ alkyl)$, -CN, halogen, $C_{1-6}$ alkyl, $C_{1-6}$ alkoxy, $C_{1-6}$ haloalkyl, $C_{3-8}$ cycloalkyl, $-O-C_{3-8}$ cycloalkyl, -O-3 to 8 membered heterocyclyl and $-O-C_{6-10}$ aryl; provided that $R^3$ is not H atom when $C^2$ is 6 membered nitrogen-containing heteroaryl ring, preferably pyridyl, pyridazinyl or pyrimidinyl;
more particularly,
$R^3$ is selected from the group consisting of H atom, F atom, $-CF_3$, methyl, Cl atom, methoxy, cyclopropyl, isopropyl, -CN, isopropoxy, furanyl-O-, ethoxy, cyclopropoxy, phenyl-O- and $-N(CH_3)_2$;
provided that $R^3$ is not H atom when $C^2$ is 6 membered nitrogen-containing heteroaryl ring, preferably pyridyl, pyridazinyl or pyrimidinyl.

**4.** The compound according to claim 1 or 2, being a compound of formula (II-2),

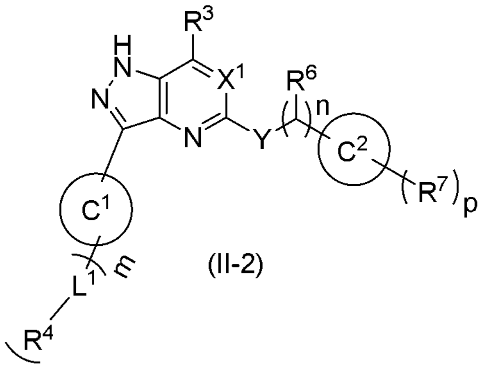

(II-2)

wherein,

Y is selected from the group consisting of O atom, S atom, -S(O)-, $-S(O)_2-$, NH and $N-C_{1-6}$ alkyl;
$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$;
$X^1$, $C^1$, $C^2$, $R^3$, $R^4$, $R^6$, $R^7$, $R^{11}$-$R^{13}$, $L^1$, m, n and p are as defined in claim 1;
particularly,
$R^3$ is selected from the group consisting of H atom, $C_{1-6}$ alkyl, $C_{1-6}$ alkoxy and $C_{3-8}$ cycloalkyl;
more particularly,
$R^3$ is selected from the group consisting of H atom, methyl, methoxy and cyclopropyl.

**5.** The compound according to claim 1, being a compound of formula (III),

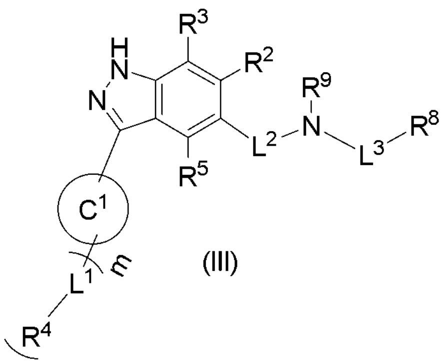

(III)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom or F atom;

$C^1$, $R^2$, $R^4$, $R^5$, $R^8$, $R^9$, $R^{11}$-$R^{13}$, $L^1$, $L^2$, $L^3$ and m are as defined in claim 1.

**6.** The compound according to claim 1 or 5, being a compound of formula (III-1),

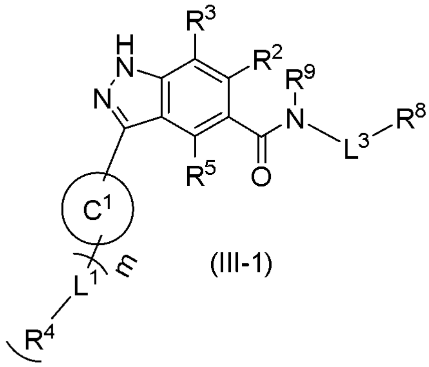

(III-1)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom or F atom;

$C^1$, $R^2$, $R^4$, $R^5$, $R^8$, $R^9$, $R^{11}$-$R^{13}$, $L^1$, $L^3$ and m are as defined in claim 1.

**7.** The compound according to claim 1 or 5, being a compound of formula (III-2),

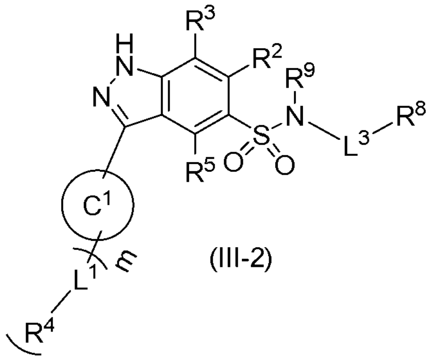

(III-2)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom or F atom;

$C^1$, $R^2$, $R^4$, $R^5$, $R^8$, $R^9$, $R^{11}$-$R^{13}$, $L^1$, $L^3$ and m are as defined in claim 1.

**8.** The compound according to claim 1, being a compound of formula (IV),

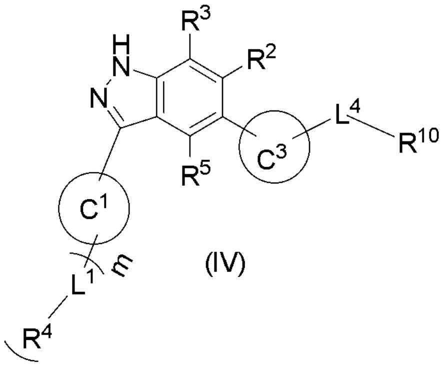

(IV)

wherein,

$R^3$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, 3 to 8 membered heterocyclyl and $-O-R^{13}$; preferably, $R^3$ is H atom or halogen; more preferably, $R^3$ is H atom;
$C^1$, $C^3$, $R^2$, $R^4$, $R^5$, $R^{10}$, $R^{11}$-$R^{13}$, $L^1$, $L^4$ and m are as defined in claim 1;
particularly,
$C^3$ is selected from the group consisting of $C_{6-10}$ aryl, 5 to 10 membered heteroaryl, $C_{3-8}$ cycloalkyl, and 3 to 8 membered heterocyclyl; preferably triazolyl, tetrazolyl, pyridyl, pyrimidinyl, pyrazolyl, imidazolyl, thiazolyl, thienyl, furyl, pyranyl, pyrrolyl, pyrazinyl, pyridazinyl, piperidyl, piperazinyl, pyrrolidinyl, morpholinyl, tetrahydropyranyl, phenyl, naphthyl, cyclohexyl, cyclopentyl, cyclobutyl or cyclopropyl; more preferably 1,2,3-triazolyl; even more preferably

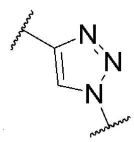
;

and/or
$L^4$ is selected from the group consisting of chemical bond, $-C_{1-6}$ alkylene-, $-C_{1-6}$ alkylene-NH-, $-C_{1-6}$ alkylene-N($C_{1-6}$ alkyl)-, -NH-$C_{1-6}$ alkylene- and -N($C_{1-6}$ alkyl)-$C_{1-6}$ alkylene-; preferably chemical bond, $-CH_2-$, $-CH_2-CH_2-$, $-CH_2-CH_2-NH-$, $-CH_2-CH_2-N(CH_3)-$, $-NH-CH_2-CH_2-$ and $-CH_2-CH_2-N(CH_3)-$; and/or
$R^{10}$ is selected from the group consisting of H atom, -OH, -COOH, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy and $C_{1-6}$ hydroxyalkyl; preferably H atom and methyl.

**9.** The compound according to any one of claims 1 to 8, wherein,

ring $C^1$ is selected from the group consisting of 5 to 10 membered heteroaryl, $C_{3-8}$ cycloalkyl, 3 to 8 membered heterocyclyl, 5 to 10 membered heteroaryl fused with 3 to 8 membered heterocyclyl, 5 to 10 membered heteroaryl fused with $C_{6-10}$ aryl, 3 to 8 membered heterocyclyl fused with 5 to 10 membered heteroaryl, and $C_{6-10}$ aryl fused with 5 to 10 membered heteroaryl;
preferably, ring $C^1$ is selected from the group consisting of triazolyl, tetrazolyl, pyridyl, pyrimidinyl, pyrazolyl, imidazolyl, thiazolyl, thienyl, furyl, pyranyl, pyrrolyl, pyrazinyl, pyridazinyl, pyrazolo[1,5-a]pyridyl, tetrahydropyrazolo[1,5-a]pyridyl, tetrahydropyrrolo[1,2-b]pyrazolyl, tetrahydropyrrolo[3,4-d]imidazolyl, benzo[d]imidazolyl, pyrazolo[5,1-b][1,3]oxazinanyl, cyclopropyl and pyrrolidinyl;
more preferably, ring $C^1$ is selected from the group consisting of

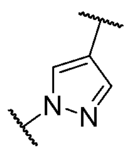
,

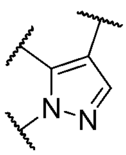, 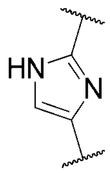, 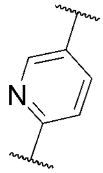, 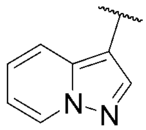, 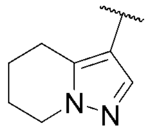, 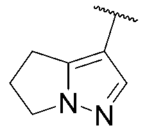, 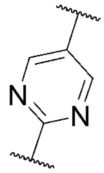,

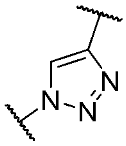, 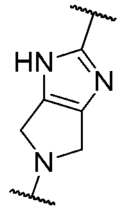, 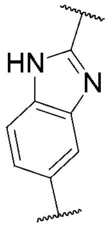, 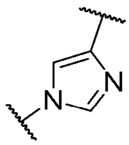, 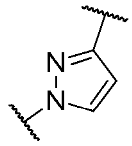, 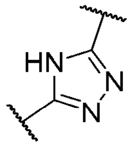, 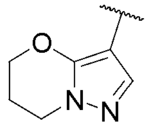,

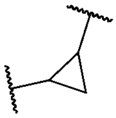 and 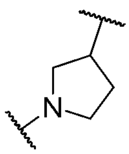 .

**10.** The compound according to any one of claims 1 to 9, wherein,

each $L^1$ is independently selected from the group consisting of chemical bond, -$C_{1-6}$ alkylene-, -O-, -$C_{1-6}$ alkylene-O-, -O-$C_{1-6}$ alkylene-, -S-, -S(O)-, -$S(O)_2$-, -S(O)(=$NR^{11}$)-, -$C_{1-6}$ alkylene-C(O)-, -C(O)-$C_{1-6}$ alkylene-, -$C_{1-6}$ alkylene-$S(O)_2$-, -$S(O)_2$-$C_{1-6}$ alkylene-, 3 to 8 membered heterocyclylene, -$C_{1-6}$ alkylene-S(O)(=$NR^{11}$)-, -S(O)(=$NR^{11}$)-$C_{1-6}$ alkylene-, $C_{3-8}$ cycloalkylene, $C_{6-10}$ arylene, 5 to 10 membered heteroarylene, -$C_{1-6}$ alkylene-O-$C_{1-6}$ alkylene-O-, -O-$C_{1-6}$ alkylene-O-$C_{1-6}$ alkylene-, -$NR^{11}$-, -$C_{1-6}$ alkylene-$NR^{11}$-, -$NR^{11}$-$C_{1-6}$ alkylene-, -$C_{1-6}$ alkylene-$NR^{11}$-$C_{1-6}$ alkylene-O-, -O-$C_{1-6}$ alkylene-$NR^{11}$-$C_{1-6}$ alkylene-, -$NR^{11}$-C(O)-, -C(O)-$NR^{11}$-, -$C_{1-6}$ alkylene-C(O)-$NR^{11}$- and -$NR^{11}$-C(O)-$C_{1-6}$ alkylene-, wherein the $C_{1-6}$ alkylene is each independently and optionally substituted by one or more substituents selected from the group consisting of $C_{1-6}$ alkyl, halogen, -OH, -COOH, -$NH_2$, -CN, $C_{1-6}$ alkoxy, $C_{1-6}$ haloalkyl, $C_{1-6}$ haloalkoxy and $C_{1-6}$ hydroxyalkyl; each $R^{11}$ is independently H atom or $C_{1-6}$ alkyl;

preferably, each $L^1$ is independently selected from the group consisting of chemical bond, -$C_{1-6}$ alkylene-, -$C_{1-6}$ alkylene-O-, -O-$C_{1-6}$ alkylene-, -$S(O)_2$-, -S(O)(=$NR^{11}$)-, -$C_{1-6}$ alkylene-C(O)-, -C(O)-$C_{1-6}$ alkylene-, -$C_{1-6}$ alkylene-$S(O)_2$-, -$S(O)_2$-$C_{1-6}$ alkylene-, -$C_{1-6}$ alkylene-S(O)(=$NR^{11}$)-, -S(O)(=$NR^{11}$)-$C_{1-6}$ alkylene-, 3 to 8 membered heterocyclylene, $C_{3-8}$ cycloalkylene, $C_{6-10}$ arylene, 5 to 10 membered heteroarylene, -$C_{1-6}$ alkylene-O-$C_{1-6}$ alkylene-O-, -O-$C_{1-6}$ alkylene-O-$C_{1-6}$ alkylene-, -$C_{1-6}$ alkylene-$NR^{11}$-, -$NR^{11}$-$C_{1-6}$ alkylene-, -$C_{1-6}$ alkylene-$NR^{11}$-$C_{1-6}$ alkylene-O-, -O-$C_{1-6}$ alkylene-$NR^{11}$-$C_{1-6}$ alkylene-, - $C_{1-6}$ alkylene-C(O)-$NR^{11}$- and -$NR^{11}$-C(O)-$C_{1-6}$ alkylene-, wherein the $C_{1-6}$ alkylene is each independently and optionally substituted by one or more substituents selected from the group consisting of $C_{1-6}$ alkyl, halogen and -OH; each $R^{11}$ is independently H atom or $C_{1-6}$ alkyl;

more preferably, each $L^1$ is independently selected from the group consisting of chemical bond, -$CH_2$-$CH_2$-, -$CH_2$-, -$CH_2$-$CH_2$-$CH_2$-, -$S(O)_2$-, -S(O)(=NH)-, -$S(O)_2$-$CH_2$-$CH_2$-, -$NCH_3$-$CH_2$-$CH_2$-, -NH-C(O)-$CH_2$-, -$NCH_3$-$CH_2$-$CH_2$-$CH_2$-, -C(O)-$CH_2$-, -O-$CH_2$-$CH_2$-$NCH_3$-$CH_2$-$CH_2$-, -O-$CH_2$-$CH_2$-O-$CH_2$-$CH_2$-, -$C(CH_3)_2$-$CH_2$-, -$C(CH_3)(OH)$-$CH_2$-, -O-$CH_2$-$CH_2$-, -CH(F)-$CH_2$-, -$CH(CH_3)$-$CH_2$-, azetidinyl, -$CH_2$-$CH_2$-$S(O)_2$-, -$CH_2$-$CH_2$-$NCH_3$-, -$CH_2$-C(O)-NH-, -$CH_2$-$CH_2$-$CH_2$-$NCH_3$-, -$CH_2$-C(O)-, -$CH_2$-$CH_2$-$NCH_3$-$CH_2$-$CH_2$-O-, -$CH_2$-$CH_2$-O-$CH_2$-$CH_2$-O-, -$CH_2$-$C(CH_3)_2$-, -$CH_2$-$C(CH_3)(OH)$-, -$CH_2$-$CH_2$-O-, -$CH_2$-CH(F)-, -$CH_2$-$CH(CH_3)$-, -$CH_2$-$CH_2$-$CH_2$-S(O)(=NH)- and -S(O)(=NH) - $CH_2$-$CH_2$-$CH_2$-.

**11.** The compound according to any one of claims 1 to 10, wherein,

each $R^4$ is independently selected from the group consisting of H atom, -OH, - COOH, -CN, halogen, $C_{1-6}$ alkyl, $C_{1-6}$ alkoxy, $C_{1-6}$ haloalkyl, $C_{1-6}$ haloalkoxy, $C_{1-6}$ hydroxyalkyl, -C(O)-$NR^{11}R^{12}$, -$NR^{11}R^{12}$, $C_{6-10}$ aryl, 5 to 10 membered heteroaryl, $C_{3-8}$ cycloalkyl, 3 to 8 membered monocyclic heterocyclyl, 7 to 10 membered spiro heterocyclyl and 7 to 10 membered bridged heterocyclyl, wherein the $C_{6-10}$ aryl, 5 to 10 membered heteroaryl, $C_{3-8}$ cycloalkyl and 3 to 8 membered monocyclic heterocyclyl are each independently and optionally substituted

by one or more substituents selected from the group consisting of -CN, -OH, $C_{1-6}$ alkyl, $C_{1-6}$ hydroxyalkyl, -C(O)-$R^{14}$, -C(O)-$NR^{11}R^{12}$, oxo, $C_{1-6}$ alkoxy, -$S(O)_2$-$C_{l-6}$ alkyl, -$NR^{11}R^{12}$, imino, halogen, -$C_{1-6}$ alkylene-$NR^{11}R^{12}$, -$NR^{11}$-C(O)-$R^{14}$, -$NR^{11}$-$S(O)_2$-$R^{14}$, -S(O)(=$NR^{11}$)-$R^{14}$, -$S(O)_2$-$R^{14}$, -C(=$NR^{11}$)-$NR^{11}R^{12}$, -$S(O)_2$-$NR^{11}R^{12}$ and -$C_{1-6}$ alkylene-C(O)-$NR^{11}R^{12}$; each $R^{11}$ is independently H atom, $C_{1-6}$ alkyl or $C_{3-8}$ cycloalkyl; each $R^{12}$ is independently H atom, $C_{1-6}$ alkyl or $C_{3-8}$ cycloalkyl; each $R^{14}$ is independently selected from the group consisting of H atom, $C_{1-6}$ alkyl and $C_{3-8}$ cycloalkyl, wherein the $C_{1-6}$ alkyl and $C_{3-8}$ cycloalkyl are each independently and optionally substituted by one or more substituents selected from the group consisting of -CN, -OH, -$NR^{11}R^{12}$ and $C_{1-6}$ alkoxy;

preferably, each $R^4$ is independently selected from the group consisting of H atom, - OH, -COOH, -CN, halogen, $C_{1-6}$ alkyl, $C_{1-6}$ hydroxyalkyl, -C(O)-$NR^{11}R^{12}$, -$NR^{11}R^{12}$, phenyl, pyridyl, imidazolyl, pyrazolyl, cyclopropyl, oxetanyl, azetidinyl, azolidinyl, morpholinyl, piperidyl, tetrahydropyranyl, piperazinyl, 1,1-dioxo-thiomorpholinyl, 1-oxo-1-imino-thiomorpholinyl, thiomorpholinyl, pyrrolidinyl, diazaspiro[4.5]decanyl, diazabicyclo[2.2.1]heptanyl and diazabicyclo[3.2.1]octyl, wherein the phenyl, pyridyl, imidazolyl, pyrazolyl, cyclopropyl, oxetanyl, azetidinyl, azolidinyl, morpholinyl, piperidyl, tetrahydropyranyl, piperazinyl, 1,1-dioxo-thiomorpholinyl, 1-oxo-1-imino-thiomorpholinyl, thiomorpholinyl, pyrrolidinyl, diazaspiro[4.5]decanyl, diazabicyclo[2.2.1]heptanyl and diazabicyclo[3.2.1]octyl are each independently and optionally substituted by one or more substituents selected from the group consisting of - CN, -OH, $C_{1-6}$ alkyl, $C_{1-6}$ hydroxyalkyl, -C(O)-$R^{14}$, -C(O)-$NR^{11}R^{12}$, oxo, $C_{1-6}$ alkoxy, - $S(O)_2$-$C_{1-6}$ alkyl, -$NR^{11}R^{12}$, imino, -$C_{1-6}$ alkylene-$NR^{11}R^{12}$, -$N^{11}$-C(O)-R $^{14}$, -$NR^{11}$-$S(O)_2$-$R^{14}$, -S(O)(=$N^{11}$)-R $^{14}$, -$S(O)_2$-$R^{14}$, -C(=$NR^{11}$)-$NR^{11}R^{12}$, -$S(O)_2$-$N^{11}$R $^{12}$ and -$C_{1-6}$ alkylene-C(O)-$NR^{11}R^{12}$; $R^{11}$ is H atom, $C_{1-6}$ alkyl or $C_{3-6}$ cycloalkyl; $R^{12}$ is H atom, $C_{1-6}$ alkyl or $C_{3-6}$ cycloalkyl; each $R^{14}$ is independently selected from the group consisting of H atom, $C_{1-6}$ alkyl and $C_{3-6}$ cycloalkyl, wherein the $C_{1-6}$ alkyl and $C_{3-6}$ cycloalkyl are each independently and optionally substituted by one or more substituents selected from the group consisting of -CN, -OH, -$NR^{11}R^{12}$ and $C_{1-6}$ alkoxy;

more preferably, each $R^4$ is independently selected from the group consisting of H atom, methyl,

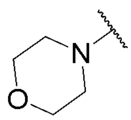,

propyl,

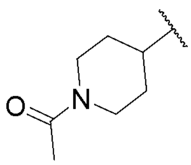,

cyclopropyl,

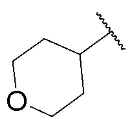, 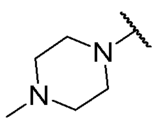

, 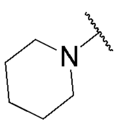, 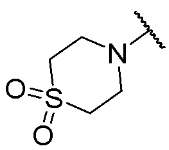, 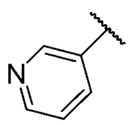, 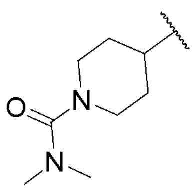, 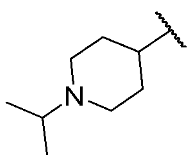,

-OH, F atom, CN,

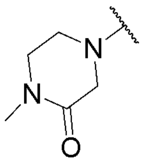, 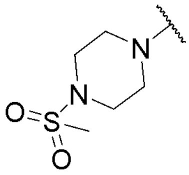, 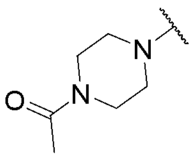, 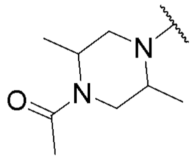, 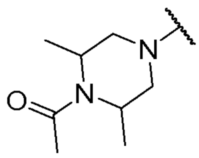,

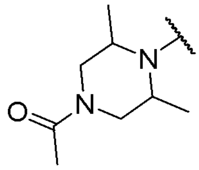, 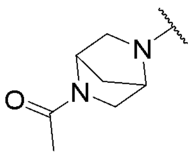, 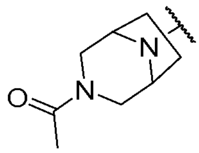, 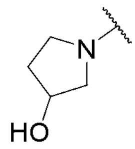, 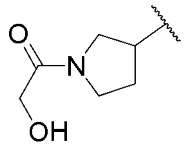,

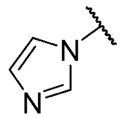, 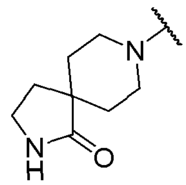, 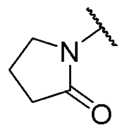,

phenyl,

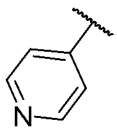, 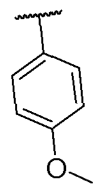, 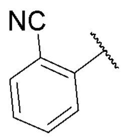,

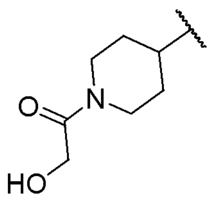, 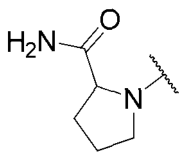, 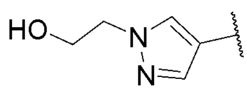, 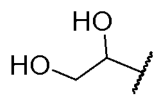, 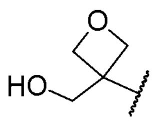,

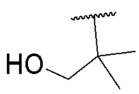, 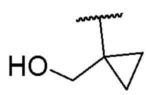, 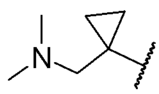,

-COOH, -C(O)-$NH_2$, -$N(CH_3)_2$,

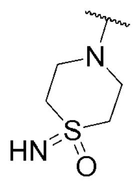,

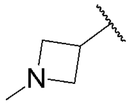, 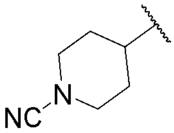, 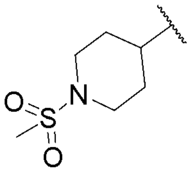, 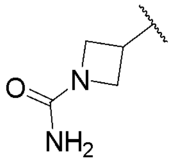, 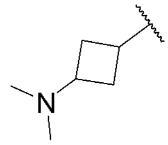,

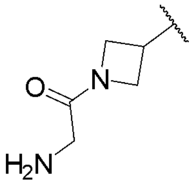, 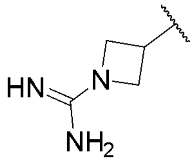, 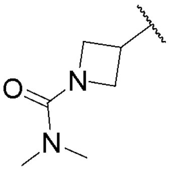, 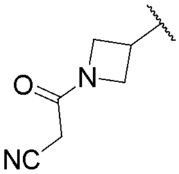, 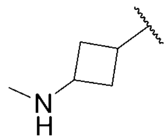,

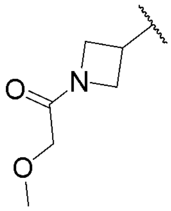, 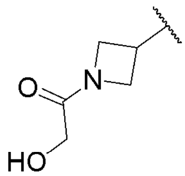, 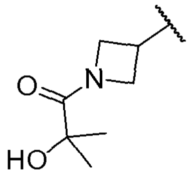, 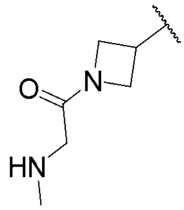, 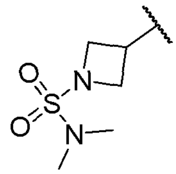,

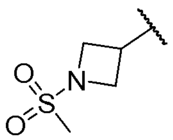, 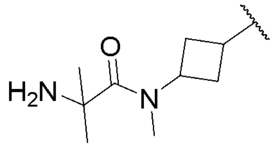, 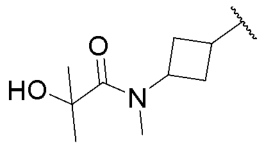, 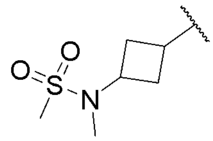,

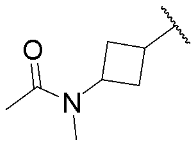, 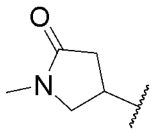, 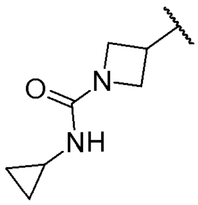, 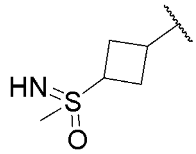, 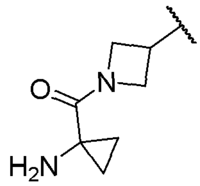,

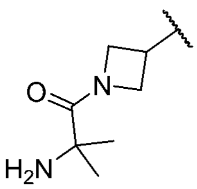, 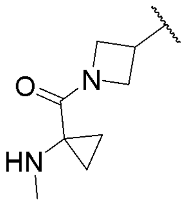, 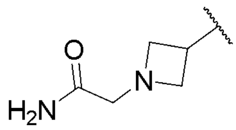, 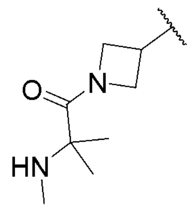, 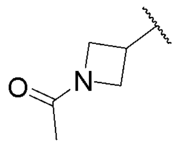,

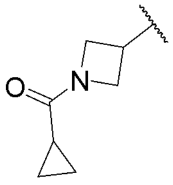, 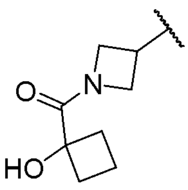, 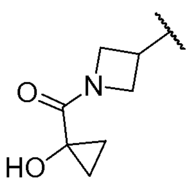, 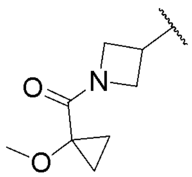, 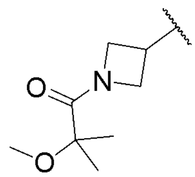,

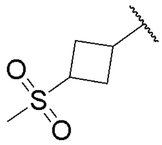, 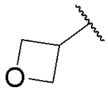 and 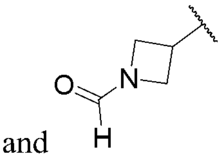.

**12.** The compound according to any one of claims 1, 3 and 5 to 11, wherein,

$R^2$ is selected from the group consisting of H atom, -OH, -COOH, $-NR^{11}R^{12}$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, $C_{1-6}$ alkoxy-$C_{6-10}$ aryl-, 5 to 10 membered heteroaryl, $C_{3-8}$ cycloalkyl and 3 to 8 membered heterocyclyl;
preferably, $R^2$ is selected from the group consisting of H atom, halogen, $C_{1-6}$ alkoxy, $C_{6-10}$ aryl and $C_{1-6}$ alkoxy-$C_{6-10}$ aryl-;
more preferably, $R^2$ is selected from the group consisting of H atom,

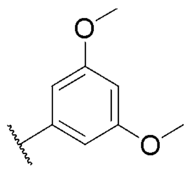

, methoxy and F atom.

**13.** The compound according to any one of claims 1, 3 and 5 to 12, wherein,

$R^5$ is selected from the group consisting of H atom, -OH, -COOH, $-NH_2$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;
preferably, $R^5$ is selected from the group consisting of H atom, -OH, -COOH, $-NH_2$, -CN, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl;
more preferably, $R^5$ is H atom.

**14.** The compound according to any one of claims 1 to 4 and 9 to 13, wherein,

ring $C^2$ is selected from the group consisting of 5 to 10 membered heteroaryl, 3 to 8 membered heterocyclyl, $C_{3-8}$ cycloalkyl fused with 5 to 10 membered heteroaryl, 5 to 10 membered heteroaryl fused with $C_{6-10}$ aryl, $C_{6-10}$ aryl fused with 3 to 8 membered heterocyclyl, 5 to 10 membered heteroaryl fused with $C_{3-8}$ cycloalkyl, $C_{6-10}$ aryl fused with 5 to 10 membered hereroaryl, and 3 to 8 membered heterocyclyl fused with $C_{6-10}$ aryl;
preferably, ring $C^2$ is selected from the group consisting of triazolyl, tetrazolyl, pyridyl, pyrimidinyl, pyrazolyl, imidazolyl, thiazolyl, thienyl, furyl, pyranyl, pyrrolyl, pyrazinyl, pyridazinyl, piperidyl, piperazinyl, pyrrolidinyl, morpholinyl, tetrahydropyranyl, tetrahydroisoquinolyl, tetrahydroquinolyl, imidazo[1,2-a]pyridyl, quinolyl, isoquinolyl, naphthyridinyl, quinoxalinyl, quinazolinyl, triazolopyridyl, benzodioxanyl, benzoimidazolyl, cinnolinyl, benzooxazolyl, benzothiazolyl, indolinyl, benzofuryl, thienopyridyl, pyrazolopyridyl and pyridinopyrazinyl;
more preferably, ring $C^2$ is selected from the group consisting of

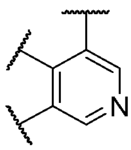,

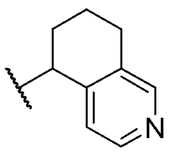, 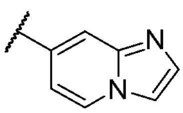, 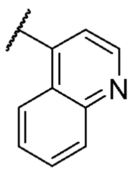, 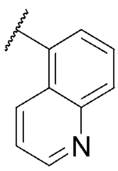, 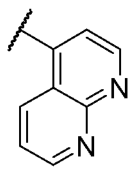, 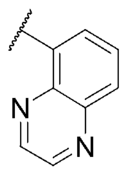,

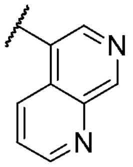, 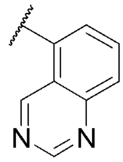, 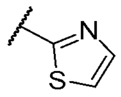, 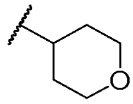, 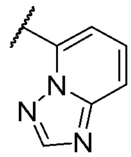, 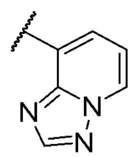, 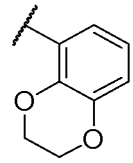,

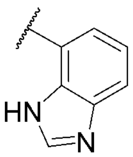, 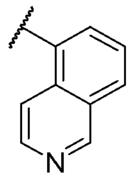, 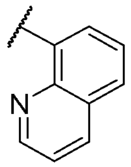, 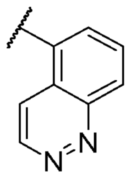, 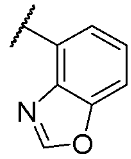, 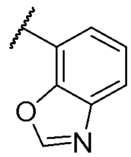, 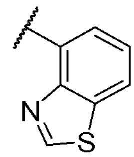,

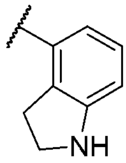, 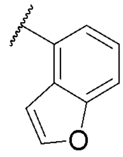, 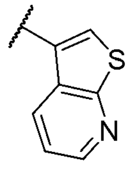, 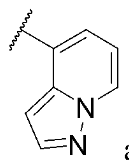 and 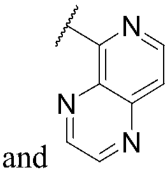 ;

and/or
$R^6$ is selected from the group consisting of H atom, -OH, -COOH, $-NR^{11}R^{12}$, -CN, halogen, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $-C_{1-6}$ alkylene-$NR^{11}R^{12}$; each $R^{11}$ is independently H atom or $C_{1-6}$ alkyl; each $R^{12}$ is independently H atom or $C_{1-6}$ alkyl;
preferably, $R^6$ is selected from the group consisting of $C_{1-6}$ alkyl, $C_{1-6}$ deuterated alkyl, $C_{1-6}$ hydroxyalkyl, $-C_{1-6}$ alkylene-NH($C_{1-6}$ alkyl), -CN and $C_{1-6}$ haloalkyl;
more preferably, $R^6$ is selected from the group consisting of methyl, ethyl, hydroxymethyl,

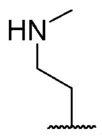,

-CN, trifluoromethyl and trideuteriomethyl; and/or
$R^7$ is selected from the group consisting of H atom, -OH, -COOH, $-NH_2$, -NH($C_{1-6}$ alkyl), -N($C_{1-6}$ alkyl$)_2$, -CN, halogen, oxo, $C_{1-6}$ alkyl, $C_{1-6}$ deuterated alkyl, $C_{1-6}$ alkoxy, $C_{1-6}$ haloalkyl, $C_{1-6}$ haloalkoxy, $C_{1-6}$ hydroxyalkyl and $C_{3-8}$ cycloalkyl;
preferably, $R^7$ is selected from the group consisting of H atom, $-NH_2$, -N($C_{1-6}$ alkyl$)_2$, -CN, halogen, oxo, $C_{1-6}$ alkyl, $C_{1-6}$ deuterated alkyl, $C_{1-6}$ alkoxy, $C_{1-6}$ hydroxyalkyl, $C_{3-6}$ cycloalkyl and $C_{1-6}$ haloalkyl;
more preferably, $R^7$ is selected from the group consisting of H atom, $-NH_2$, $-N(CH_3)_2$, -CN, Cl atom, F atom, Br atom, oxo, methyl, ethyl, isopropyl, methoxy, cyclopropyl, difluoromethyl, monofluoromethyl, trifluoromethyl, hydroxymethyl and trideuteriomethyl.

**15.** The compound according to any one of claims 1, 5 to 7 and 9 to 14, wherein,

$L^3$ is selected from the group consisting of chemical bond, $-C_{1-6}$ alkylene-, -O-, $-C_{1-}6$ alkylene-O-, $-O-C_{1-6}$ alkylene-, -S-, -S(O)-, $-S(O)_2-$, $-C_{1-6}$ alkylene-C(O)-, $-C(O)-C_{1-6}$ alkylene-, $-C_{1-6}$ alkylene-$S(O)_2$-, $-S(O)_2-C_{1-6}$ alkylene-, $-C_{1-6}$ alkylene-O-$C_{1-6}$ alkylene-O-, -O-$C_{1-6}$ alkylene-O-$C_{1-6}$ alkylene-, $-NR^{11}-$, $-NR^{11}-C_{1-6}$ alkylene-, $-C_{1-6}$ alkylene-$NR^{11}$-, - $C_{1-6}$ alkylene-$NR^{11}$-$C_{1-6}$ alkylene-O-, -O-$C_{1-6}$ alkylene-$NR^{11}$-$C_{1-6}$ alkylene-, $-NR^{11}$-C(O)-, -C(O)-$NR^{11}$-, $-C_{1-6}$ alkylene-C(O)-$NR^{11}$- and $-NR^{11}$-C(O)-$C_{1-6}$ alkylene-, wherein the $C_{1-}6$ alkylene is each independently and optionally substituted by one or more substituents selected from the group consisting of $C_{1-6}$ alkyl, halogen, -OH, -COOH, $-NH_2$, -CN, $C_{1-6}$ alkoxy, $C_{1-6}$ haloalkyl, $C_{1-6}$ haloalkoxy, and $C_{1-6}$ hydroxyalkyl; each $R^{11}$ is independently H atom or $C_{1-6}$ alkyl;
preferably, $L^3$ is selected from the group consisting of chemical bond, $-C_{1-6}$ alkylene-, $-C_{1-6}$ alkylene-NH-, $-C_{1-6}$ alkylene-N($C_{1-6}$ alkyl)-, -NH-$C_{1-6}$ alkylene- and -N($C_{1-6}$ alkyl)-$C_{1-6}$ alkylene-; wherein the $C_{1-6}$ alkylene is each independently and optionally substituted by one or more $C_{1-6}$ alkyl or -OH groups;

preferably, $L^3$ is selected from the group consisting of chemical bond, $-CH_2-CH_2-NCH_3-$, $-CH_2-CH_2-NH-$, $-CH_2-C(CH_3)(OH)-$, $-CH_2-C(CH_3)_2-$, $-NCH_3-CH_2-CH_2-$, $-NH-CH_2-CH_2-$, $-C(CH_3)(OH)-CH_2-$ and $-C(CH_3)_2-CH_2-$; and/or

$R^8$ is selected from the group consisting of H atom, -OH, -COOH, -CN, halogen, $C_1$-6 alkyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{6}$-10 aryl, 5 to 10 membered heteroaryl, $C_{3-8}$ cycloalkyl and 3 to 8 membered heterocyclyl are each independently and optionally substituted by one or more substituents selected from the group consisting of -CN, -OH, $C_{1-6}$ alkyl, -C(O)-$C_{1-6}$ alkyl, -C(O)-$C_{1-6}$ hydroxyalkyl, $C_{1-6}$ alkoxy, $-NH_2$ and halogen;

preferably, $R^8$ is selected from the group consisting of H atom, -OH, $C_{6-10}$ aryl, 5 to 10 membered heteroaryl and 3 to 8 membered heterocyclyl, wherein the $C_{6-10}$ aryl and 5 to 10 membered heteroaryl are each independently and optionally substituted by one or more $C_{1-6}$ alkyl or halogen groups;

more preferably, $R^8$ is selected from the group consisting of H, methyl, -OH,

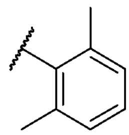, 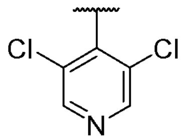, 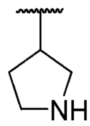 and 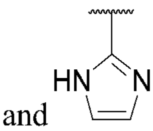;

and/or

$R^9$ is selected from the group consisting of H atom, $C_{1-6}$ alkyl, $C_{1-6}$ alkoxy, $C_{1-6}$ haloalkyl and $C_{1-6}$ hydroxyalkyl;

preferably, $R^9$ is H atom or $C_{1-6}$ alkyl;

more preferably, $R^9$ is H atom or methyl.

**16.** The compound according to any one of claims 1 to 15, being selected from the group consisting of

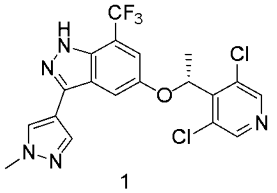

1,

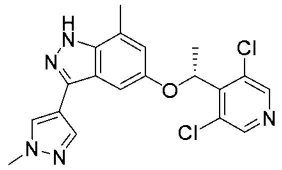

2,

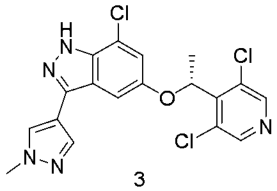

3,

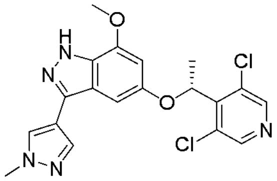

4,

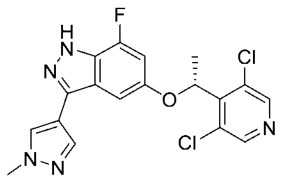

5,

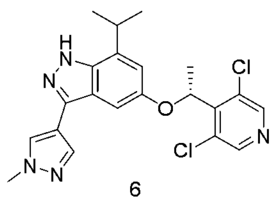

6,

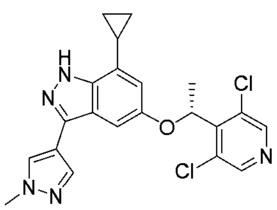

7,

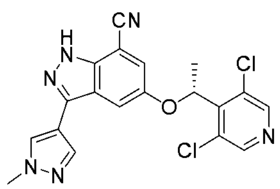

8,

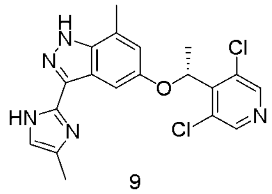

9,

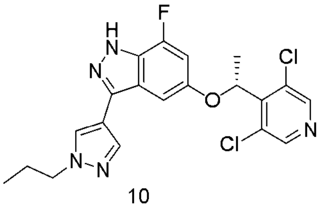
10

,

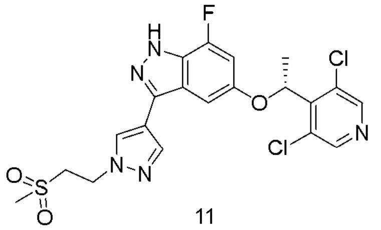
11

,

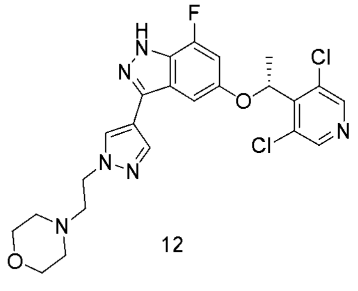
12

,

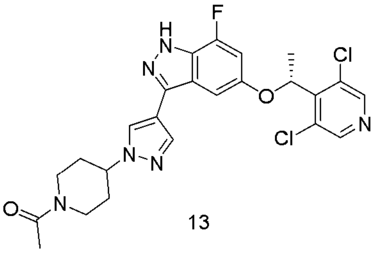
13

,

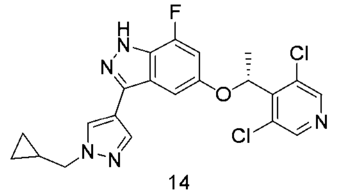
14

,

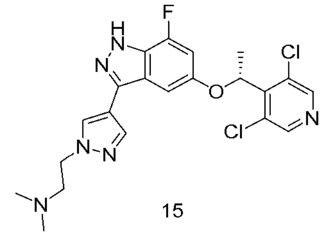
15

,

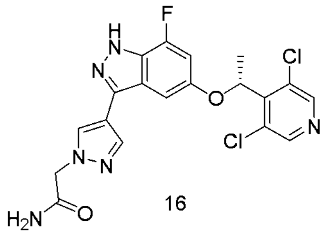
16

,

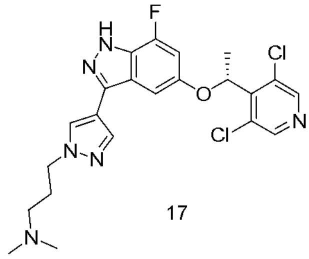
17

,

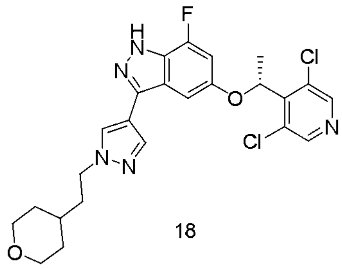
18

,

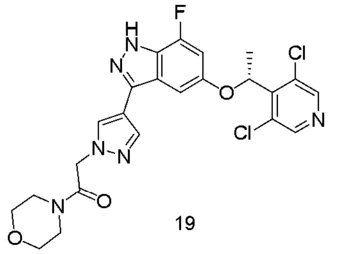
19

,

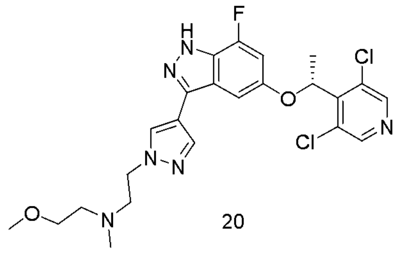
20

,

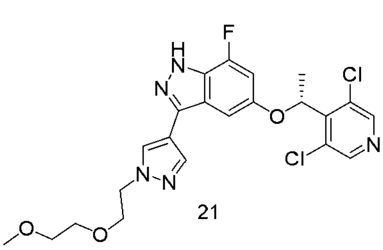
21

,

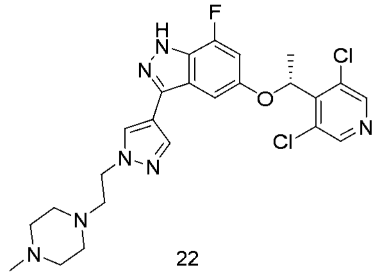
22

,

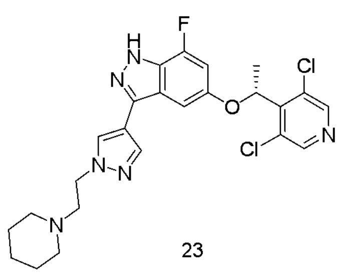
23 ,
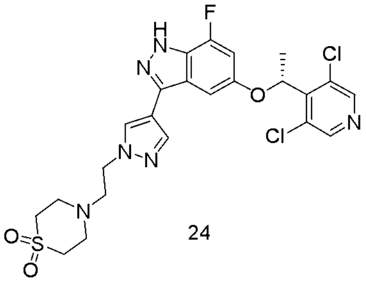
24 ,
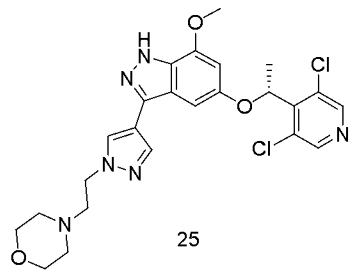
25 ,

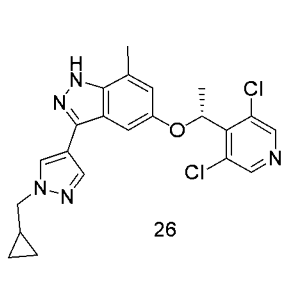
26 ,
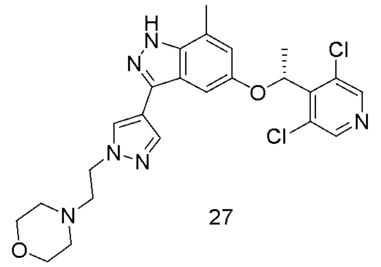
27 ,
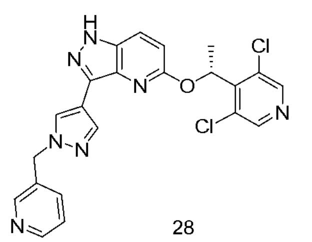
28 ,

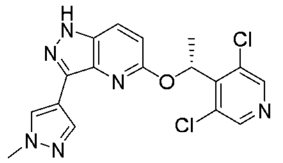
29 ,
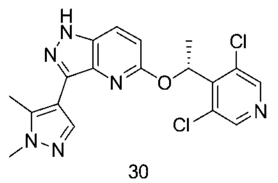
30 ,
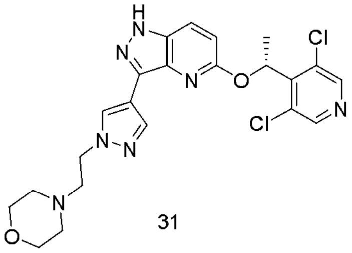
31 ,

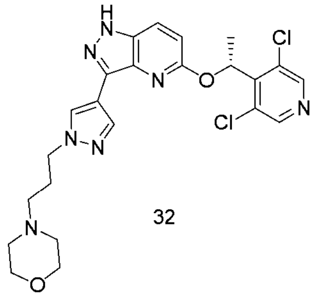
32 ,
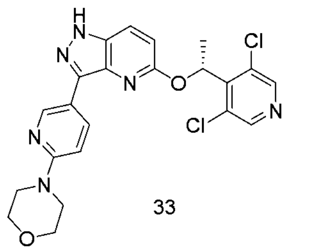
33 ,
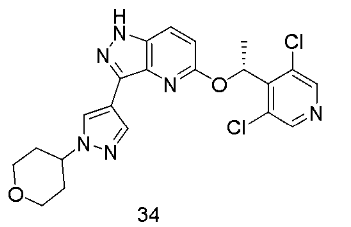
34 ,

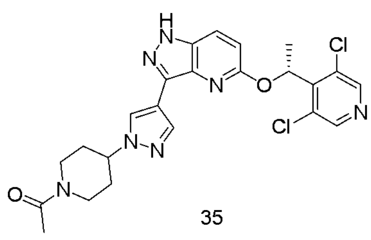
35 ,
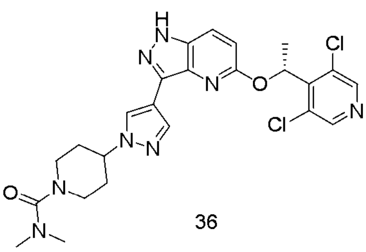
36 ,
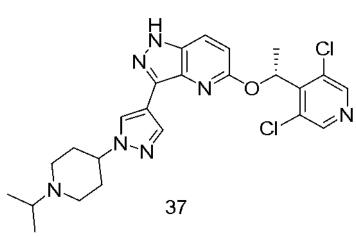
37

, 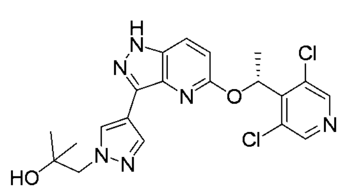
38 ,
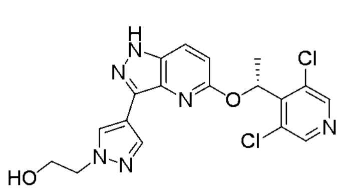
39 ,
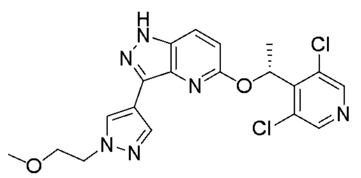
40 ,

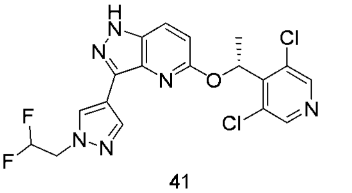
41
,
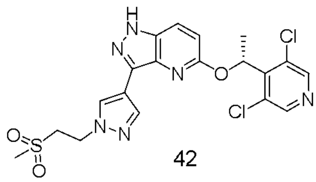
42
,
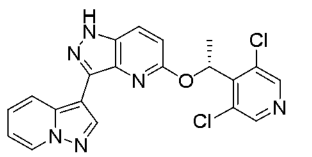
43
,

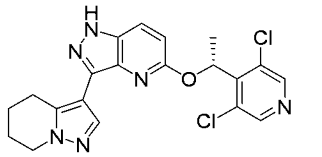
44
,
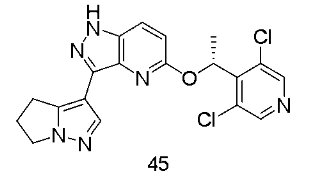
45
,
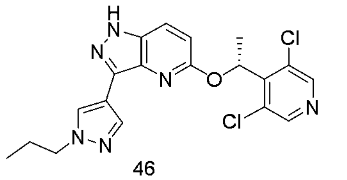
46
,

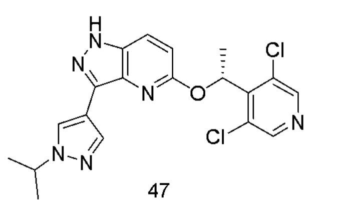
47
,
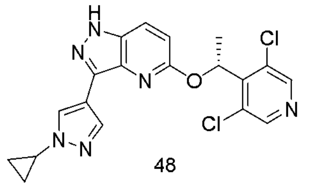
48
,
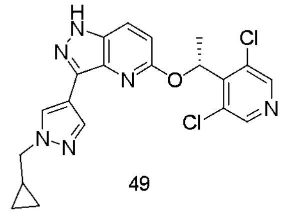
49
,

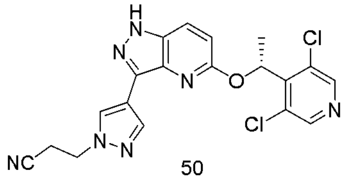
50
,
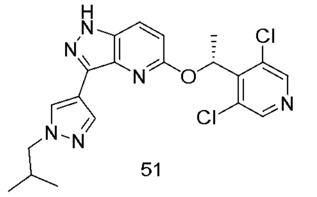
51
,
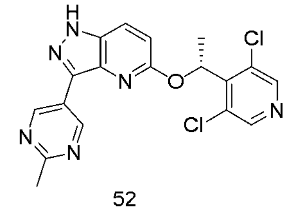
52
,

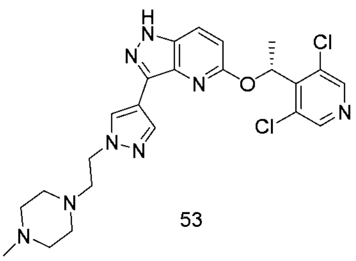
53
,
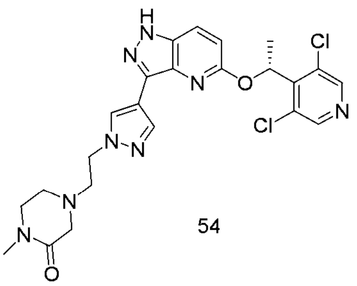
54
,
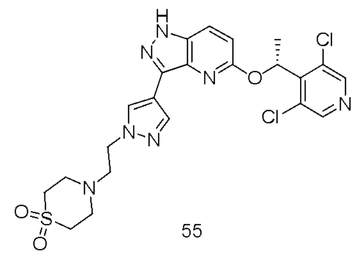
55
,

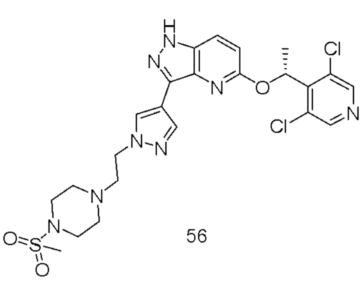
56
,
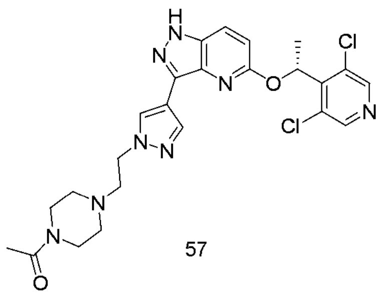
57
,
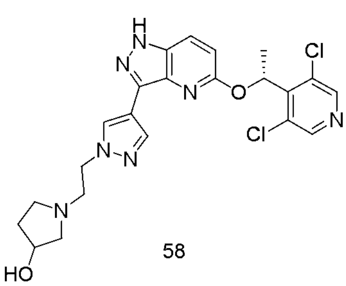
58

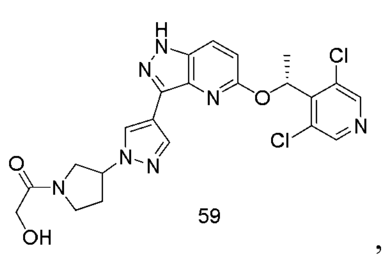
59 ,

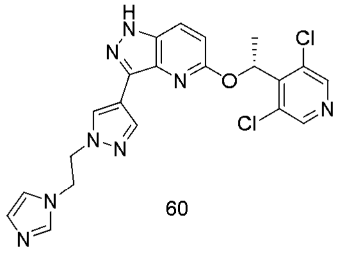
60 ,

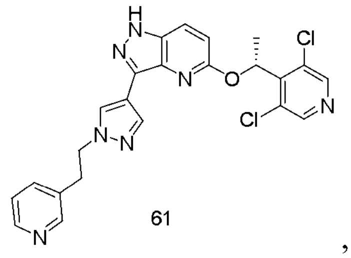
61 ,

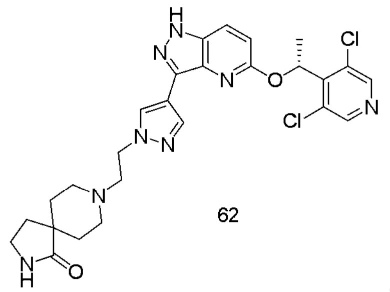
62 ,

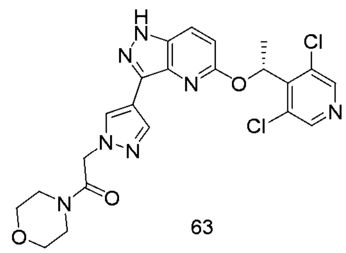
63 ,

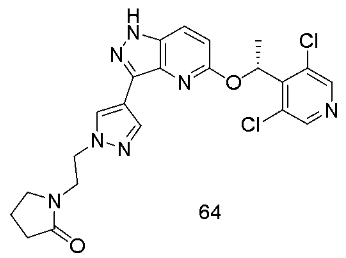
64 ,

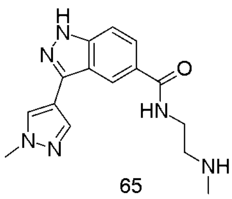
65 ,

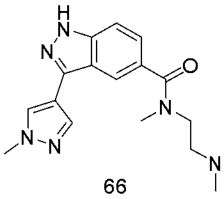
66 ,

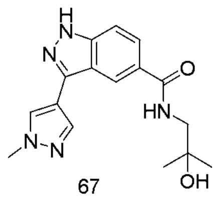
67 ,

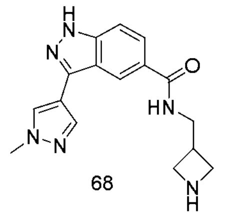
68 ,

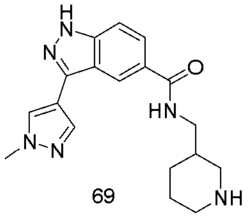
69 ,

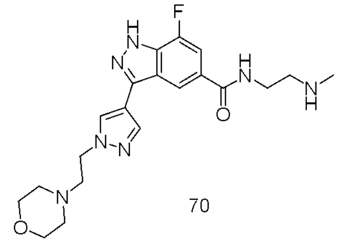
70 ,

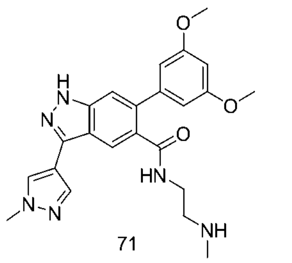
71 ,

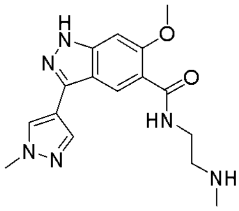
72 ,

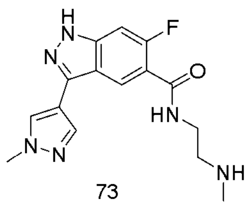
73 ,

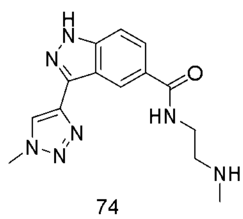
74 ,

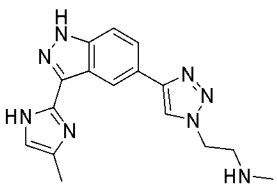
75 ,

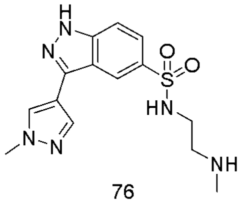
76 ,

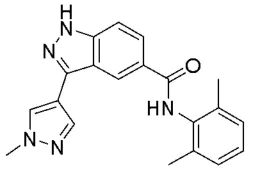
77 ,

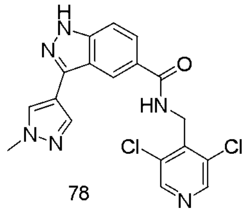
78 ,

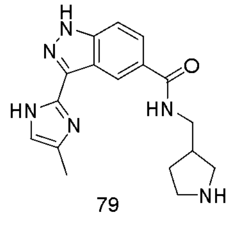
79 ,

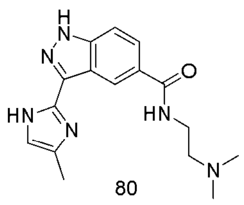
80,

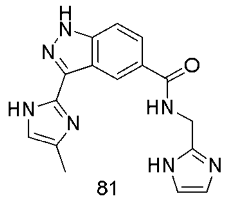
81,

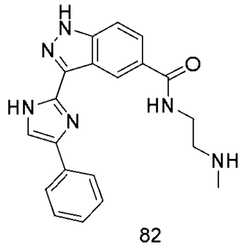
82,

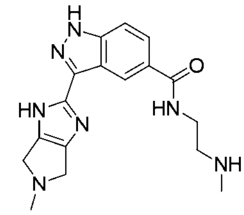
83,

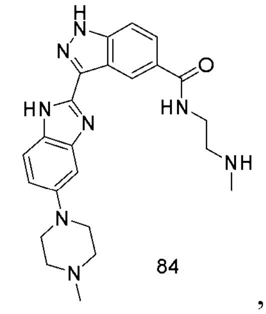
84,

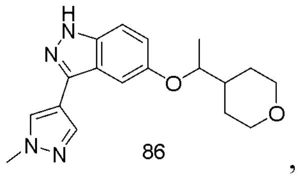
86,

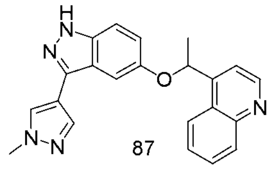
87,

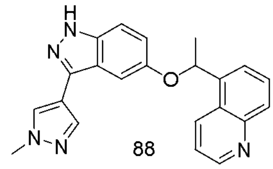
88,

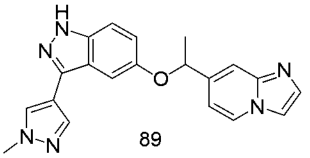
89,

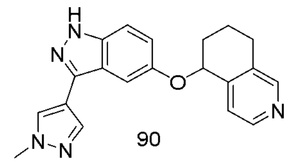
90,

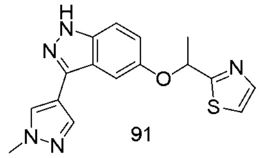
91,

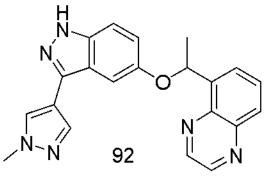
92,

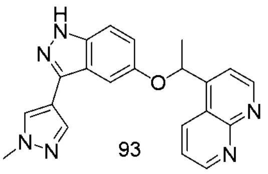
93,

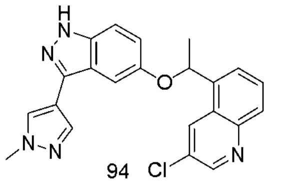
94,

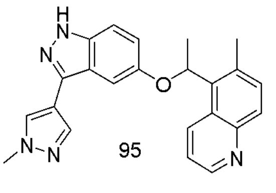
95,

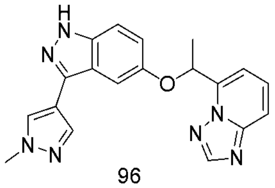
96,

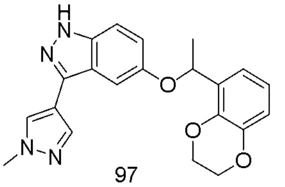
97,

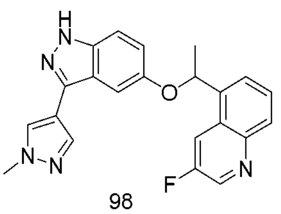
98,

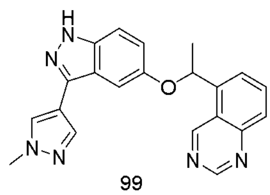
99,

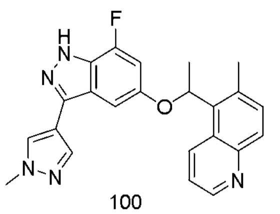
100,

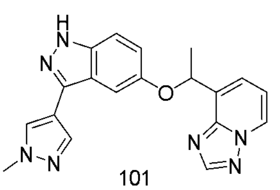
101,

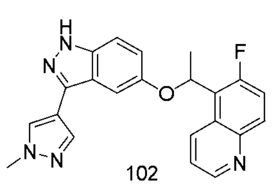
102,

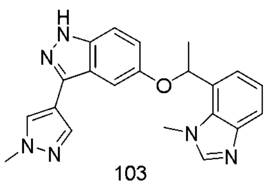
103,

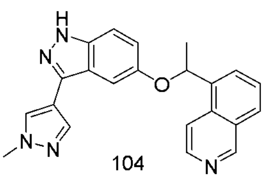
104 ,

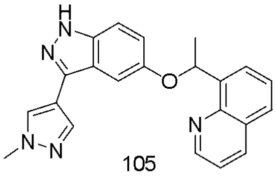
105 ,

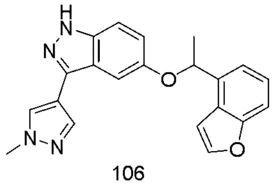
106 ,

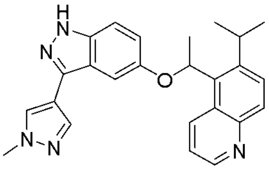
107 ,

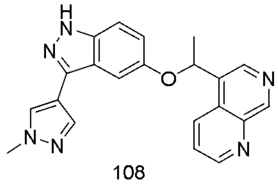
108 ,

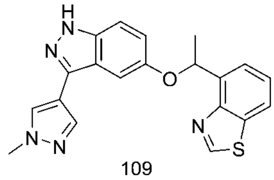
109 ,

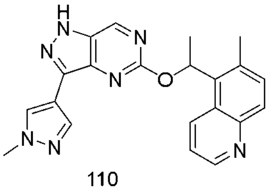
110 ,

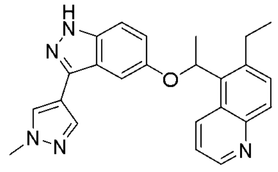
111 ,

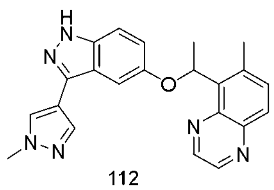
112 ,

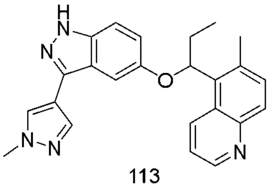
113 ,

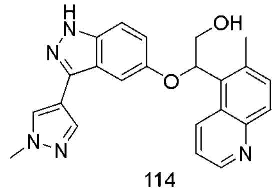
114 ,

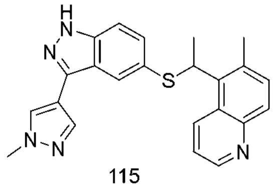
115 ,

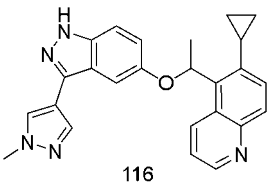
116 ,

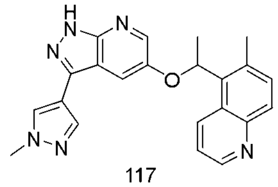
117 ,

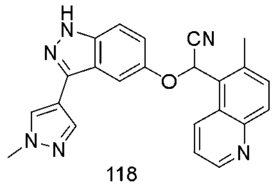
118 ,

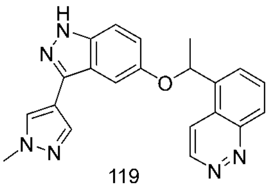
119 ,

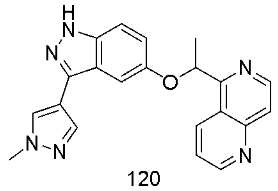
120 ,

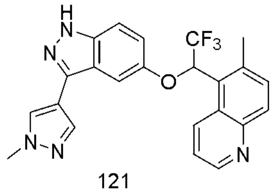
121 ,

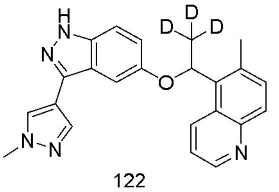
122 ,

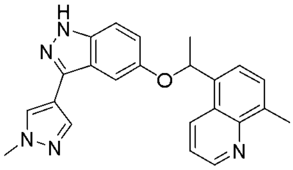
123 ,

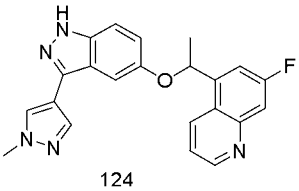
124 ,

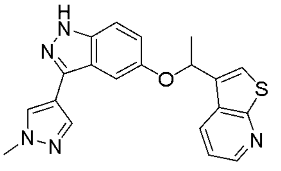
125
,
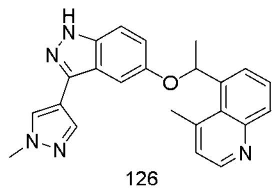
126
,
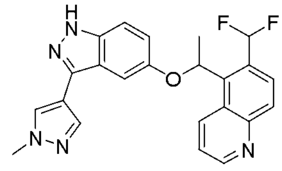
127
,

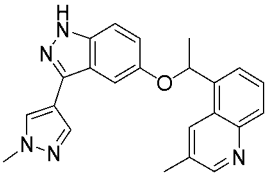
128
,
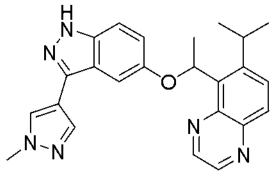
129
,
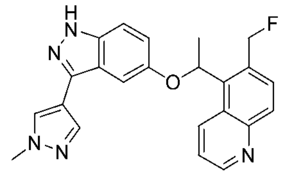
130
,

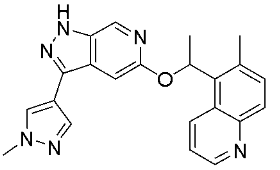
131
,
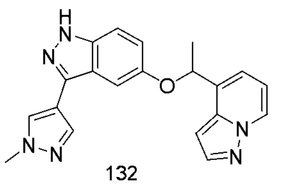
132
,
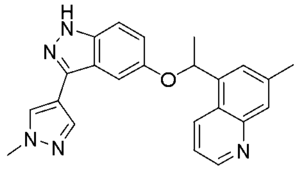
133
,

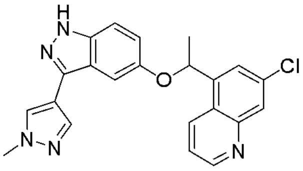
134
,
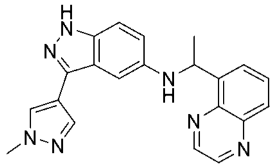
135
,
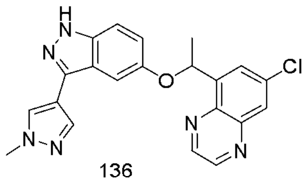
136
,

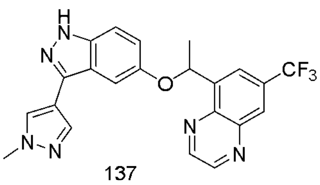
137
,
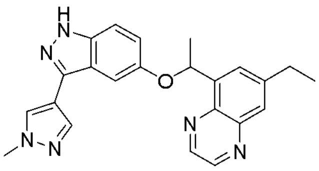
138
,
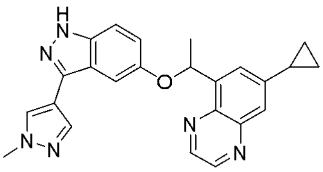
139
,

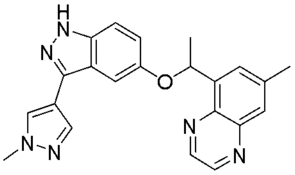
140
,
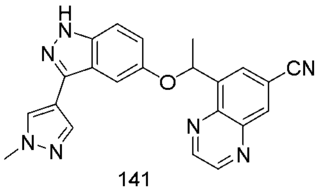
141
,
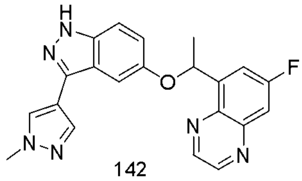
142
,

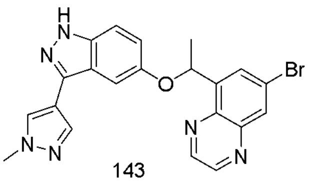
143
,
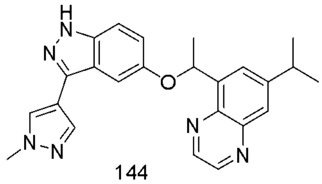
144
,
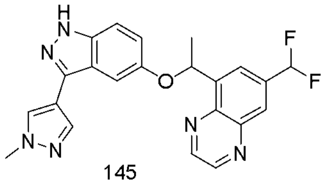
145
,

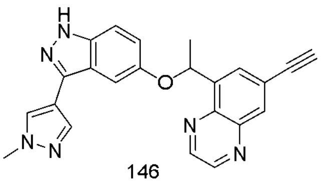
146 ,

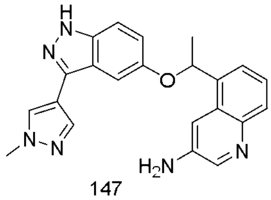
147 ,

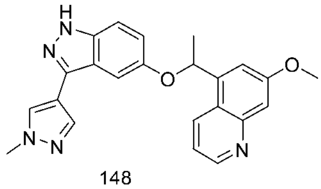
148 ,

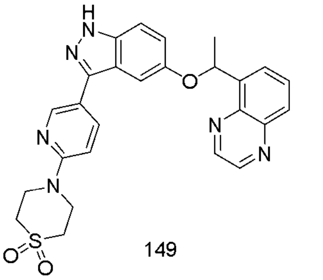
149 ,

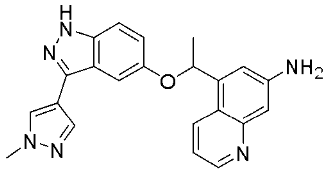
150 ,

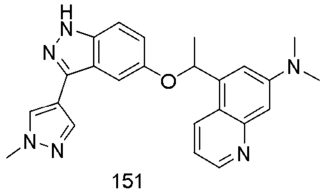
151 ,

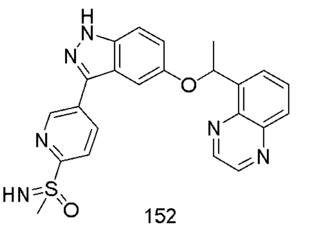
152 ,

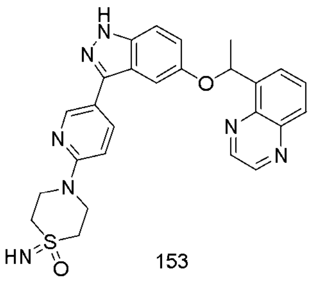
153 ,

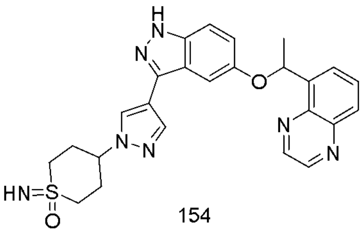
154 ,

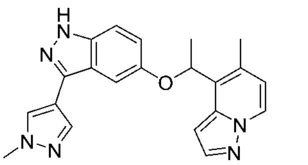
155 ,

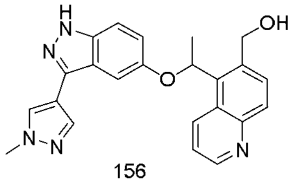
156 ,

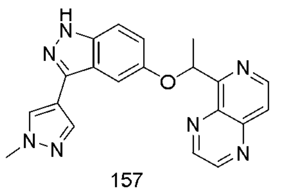
157 ,

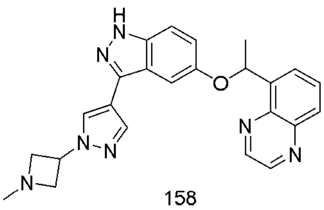
158 ,

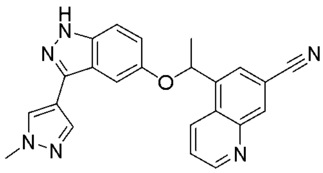
159 ,

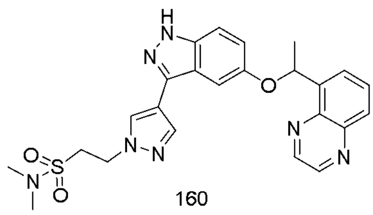
160 ,

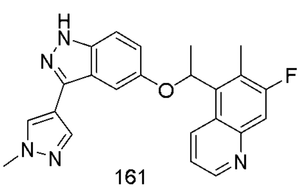
161 ,

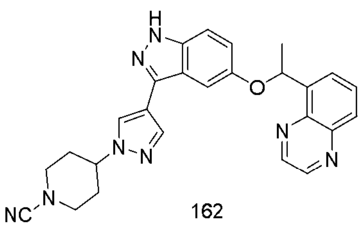
162 ,

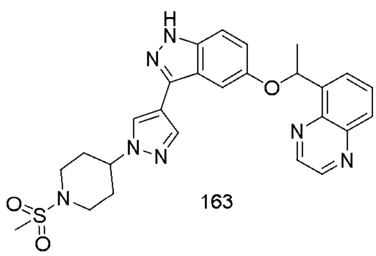
163 ,

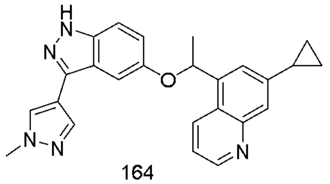
164
,
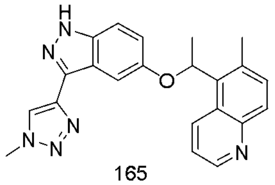
165
,
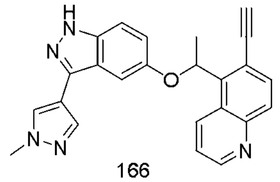
166
,

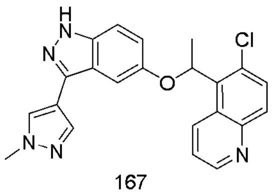
167
,
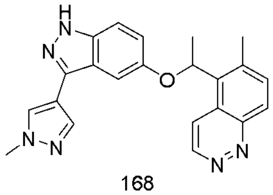
168
,
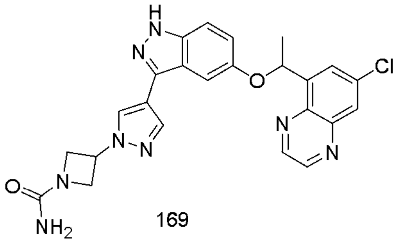
169
,

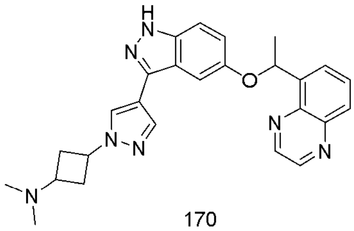
170
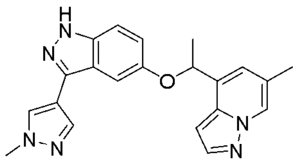
171
,
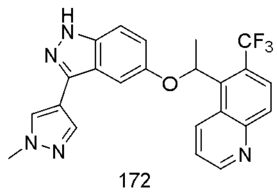
172
,

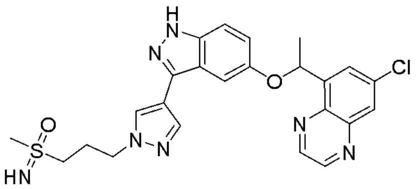
173
,
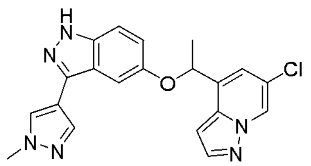
174
,

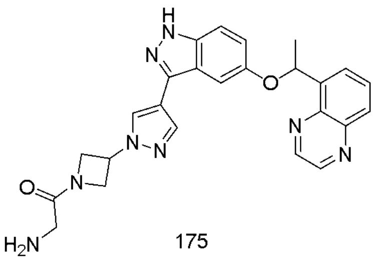
175
,
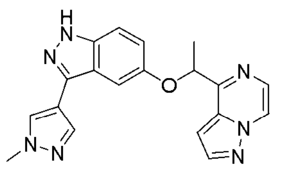
176
,
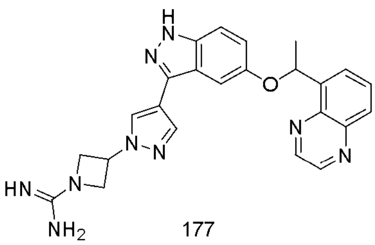
177
,

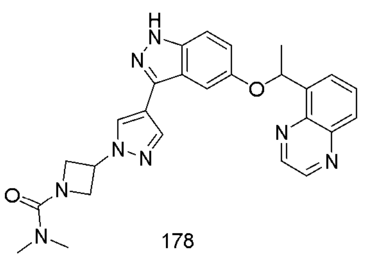
178
,
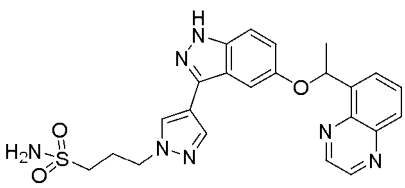
179
,

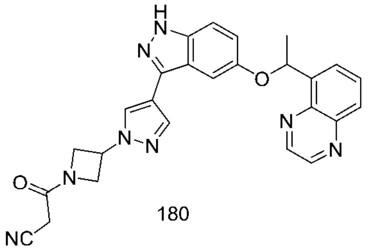
180
,
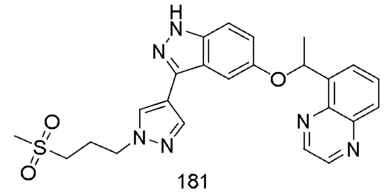
181
,

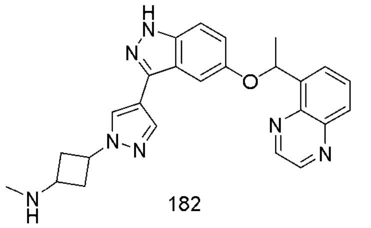
182
,
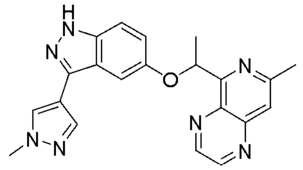
183
,
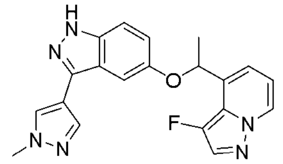
184
,

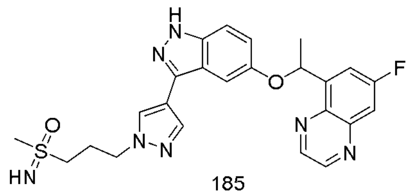
185
,
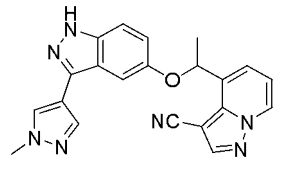
186
,
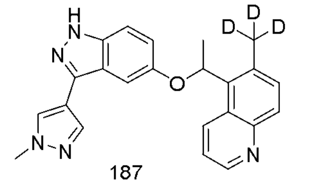
187
,

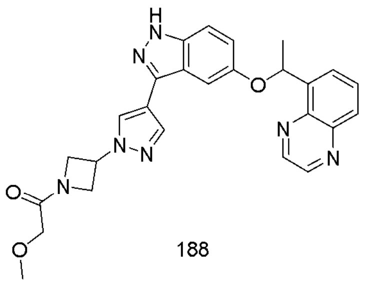
188
,
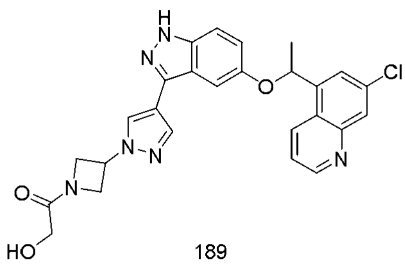
189
,

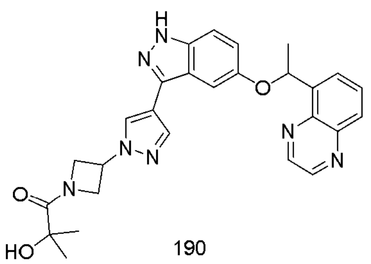
190
,
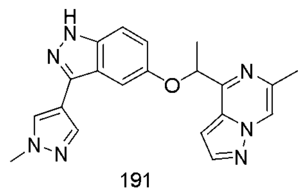
191
,
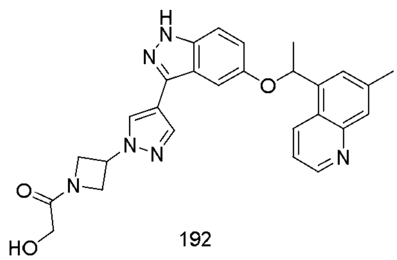
192
,

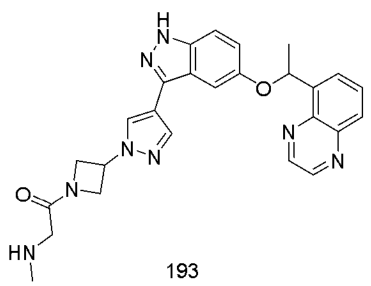
193
,
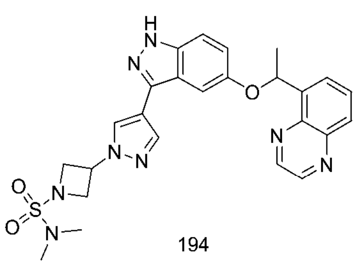
194
,
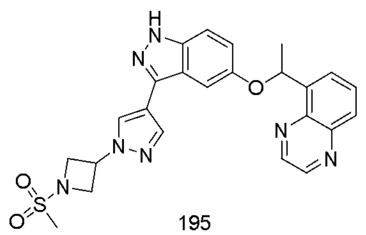
195

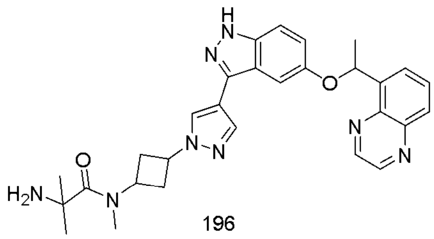
196
,
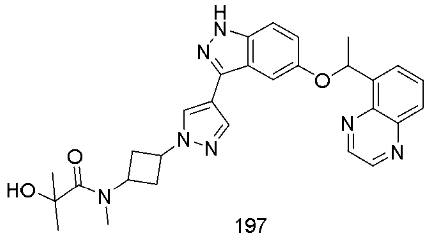
197
,

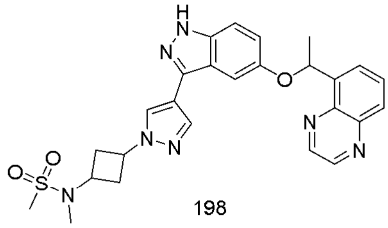
198
,
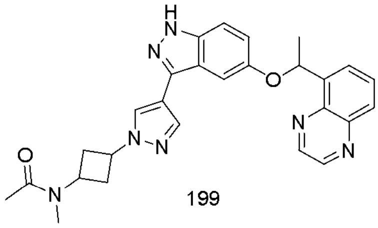
199
,

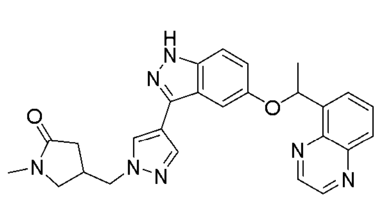
200
,
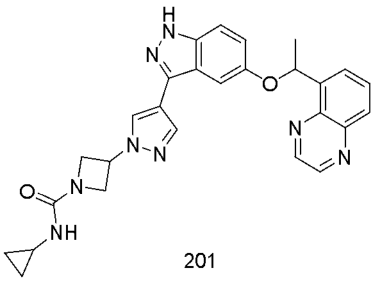
201
,

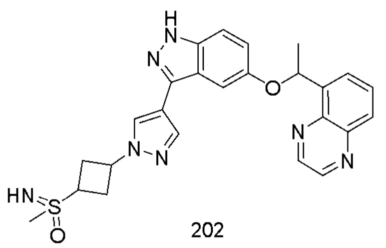
202
,
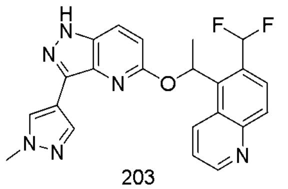
203
,
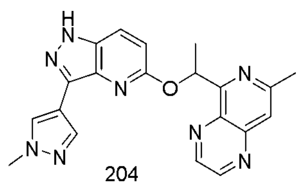
204
,

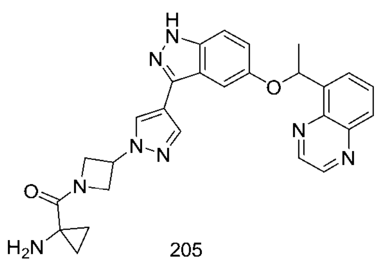
205
,
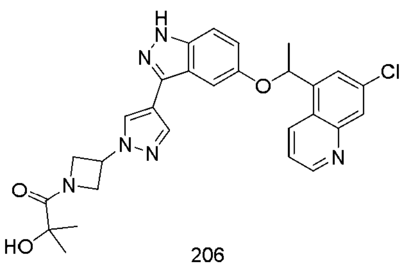
206
,

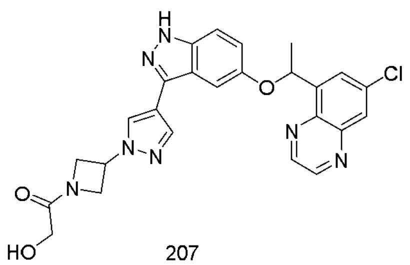
207
,
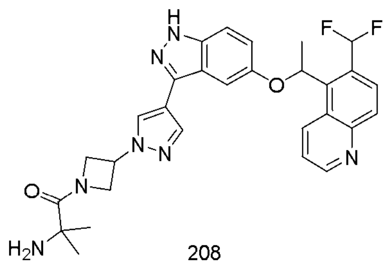
208
,

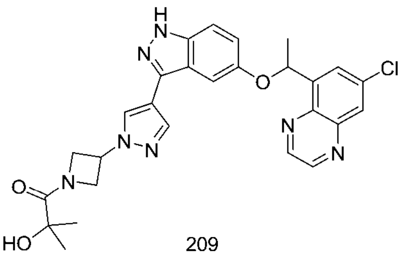

209 ,

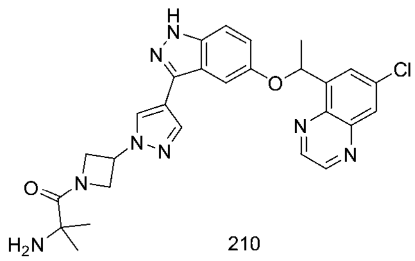

210 ,

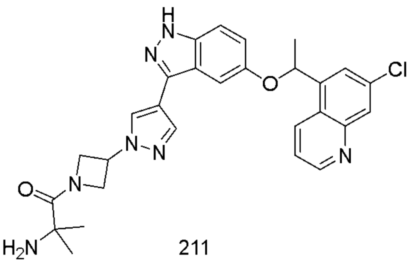

211 ,

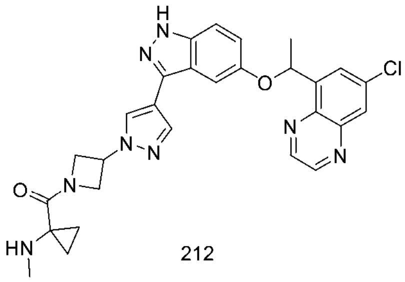

212 ,

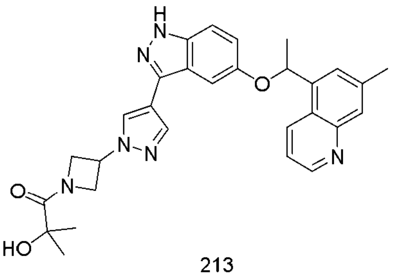

213 ,

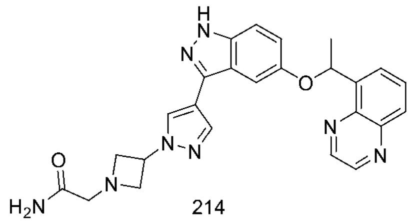

214 ,

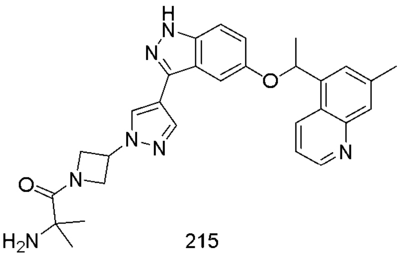

215 ,

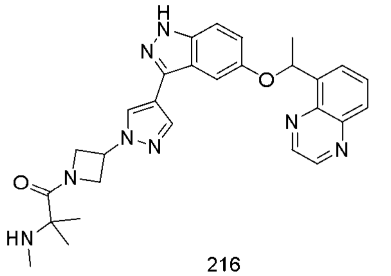

216 ,

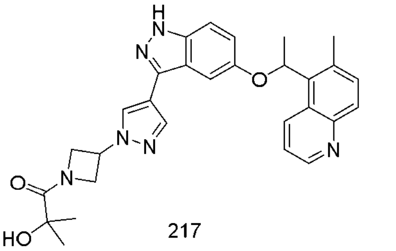

217 ,

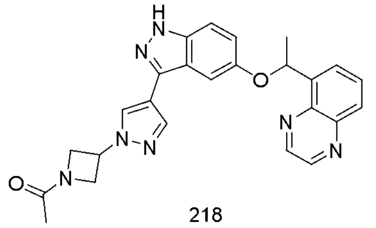

218 ,

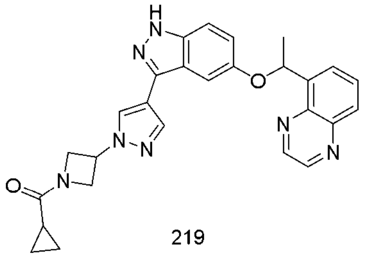
219 ,

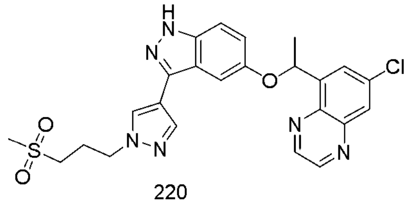
220 ,

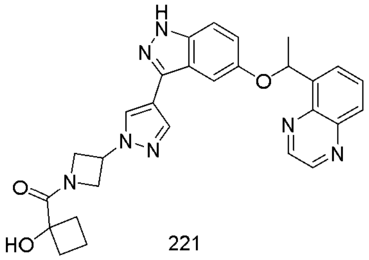
221 ,

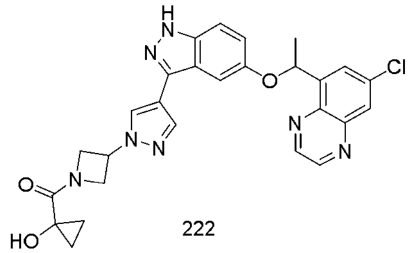
222 ,

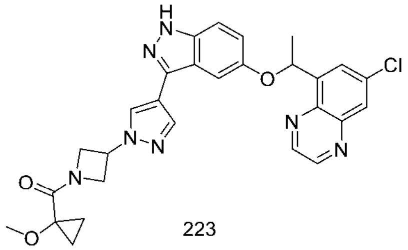
223 ,

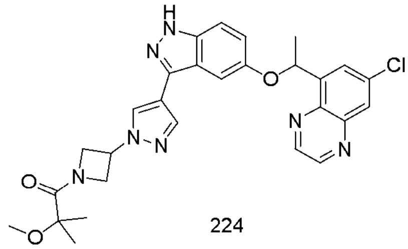
224 ,

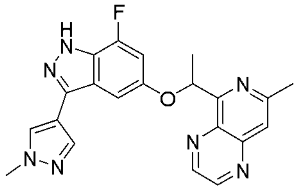
225 ,

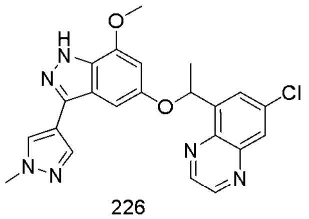
226 ,

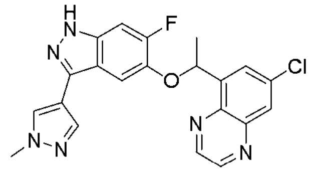
227 ,

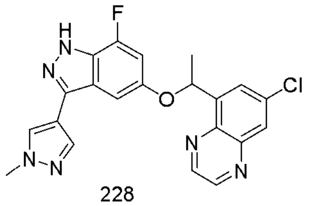
228 ,

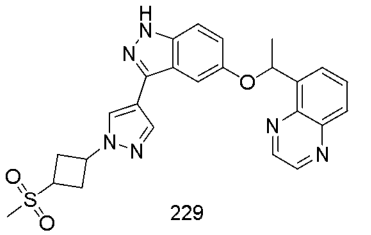
229 ,

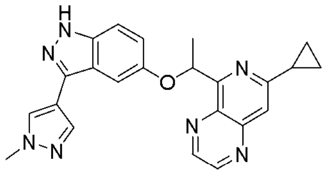
230 ,

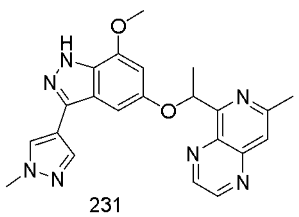
231 ,

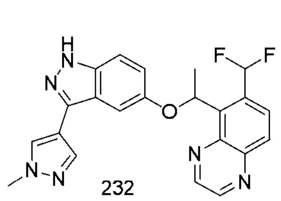
232 ,

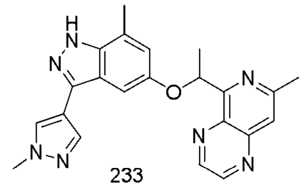
233 ,

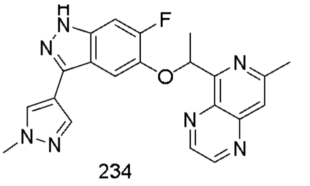
234,
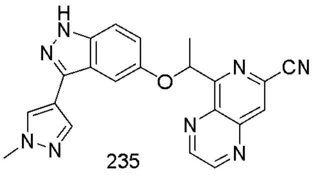
235,
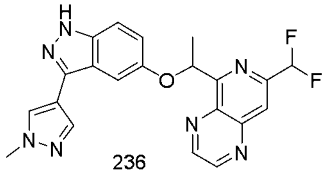
236,

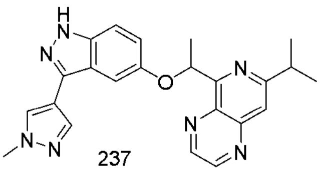
237,
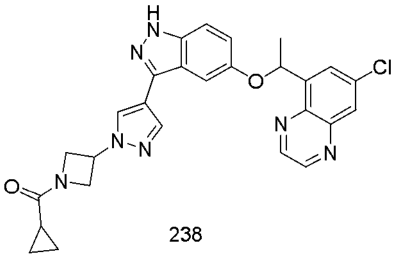
238,
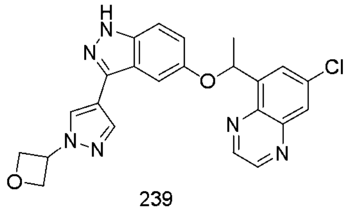
239,

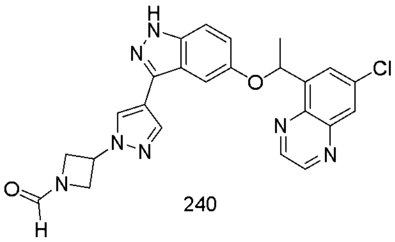
240
and
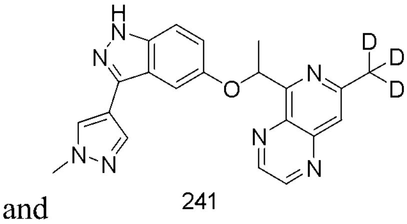
241
.

**17.** A method for preparing the compound of formula (II) according to claim 2, comprising:

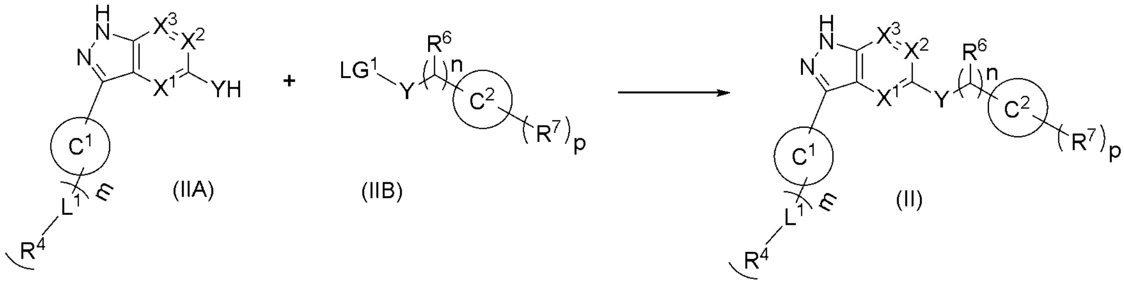
(IIA) (IIB) (II)

reacting a compound of formula (IIA) with a compound of formula (IIB) to obtain the compound of formula (II); wherein, $LG^1$ is a leaving group, preferably halogen, methylsulfonyloxy or p-tolylsulfonyloxy; and $X^1$, $X^2$, $X^3$, Y, $C^1$, $C^2$, $R^4$, $R^6$, $R^7$, $L^1$, m, n and p are as defined in claim 2;
or

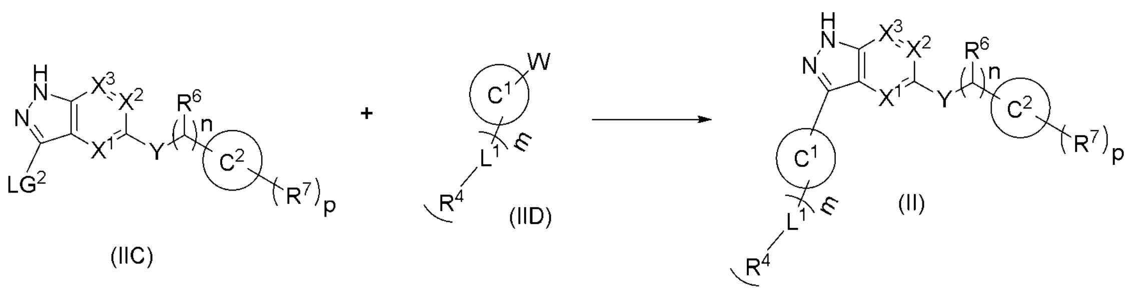
(IIC) (IID) (II)

reacting a compound of formula (IIC) with a compound of formula (IID) to obtain the compound of formula (II); wherein, $LG^2$ is a leaving group, preferably halogen, methylsulfonyloxy or p-tolylsulfonyloxy;
W is

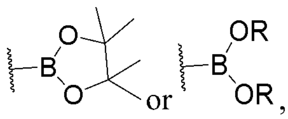

or ,

R is hydrogen atom or $C_{1-6}$ alkyl; and
$X^1$, $X^2$, $X^3$, Y, $C^1$, $C^2$, $R^4$, $R^6$, $R^7$, $L^1$, m, n and p are as defined in claim 2;
or

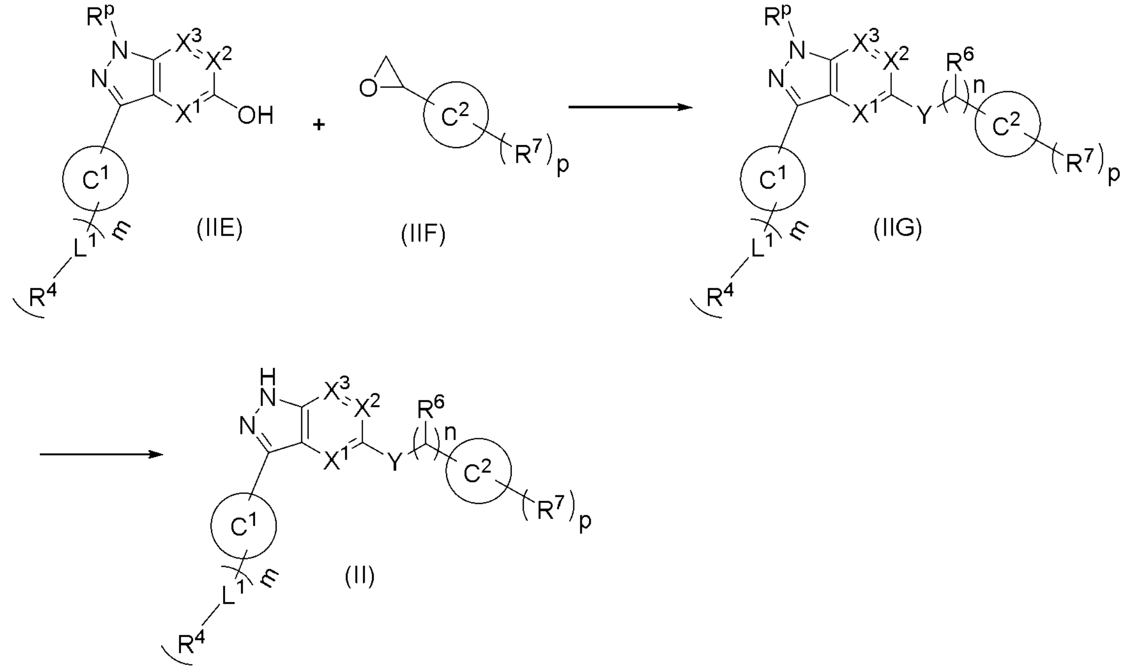

(IIE) (IIF) (IIG) (II)

reacting a compound of formula (IIE) with a compound of formula (IIF) to obtain a compound of formula (IIG), and removing the protecting group $R^p$ from the compound of formula (IIG) to obtain the compound of formula (II);
wherein, $R^p$ is tetrahydropyranyl, (trimethylsilyl)ethoxymethyl, p-tosyl, t-butyloxycarbonyl, benzyl or p-methoxybenzyl;
Y is O atom, $R^6$ is $-CH_2OH$, n is 1, and
$X^1$, $X^2$, $X^3$, $C^1$, $C^2$, $R^4$, $R^7$, $L^1$, m and p are as defined in claim 2;
or

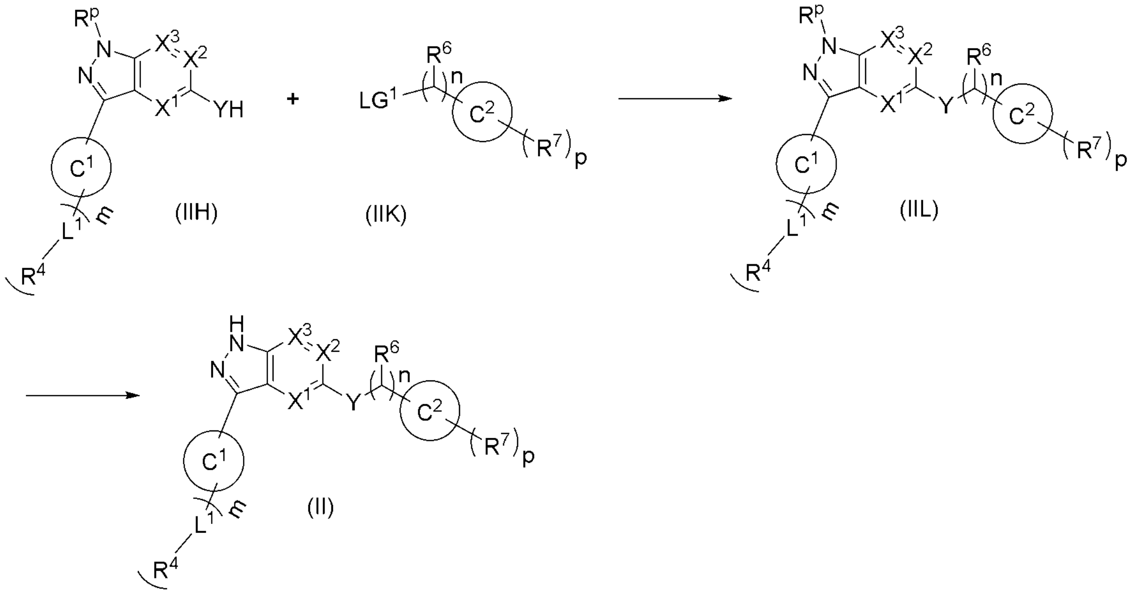

(IIH) (IIK) (IIL) (II)

reacting a compound of formula (IIH) with a compound of formula (IIK) to obtain a compound of formula (IIL), and removing the protecting group $R^p$ from the compound of formula (IIL) to obtain the compound of formula (II);
wherein, $R^p$ is tetrahydropyranyl, (trimethylsilyl)ethoxymethyl, p-tosyl, t-butyloxycarbonyl, benzyl or p-methoxybenzyl;
$LG^1$ is a leaving group, preferably halogen, methylsulfonyloxy or p-tolylsulfonyloxy; and
$X^1$, $X^2$, $X^3$, Y, $C^1$, $C^2$, $R^4$, $R^6$, $R^7$, $L^1$, m, n and p are as defined in claim 2;
or

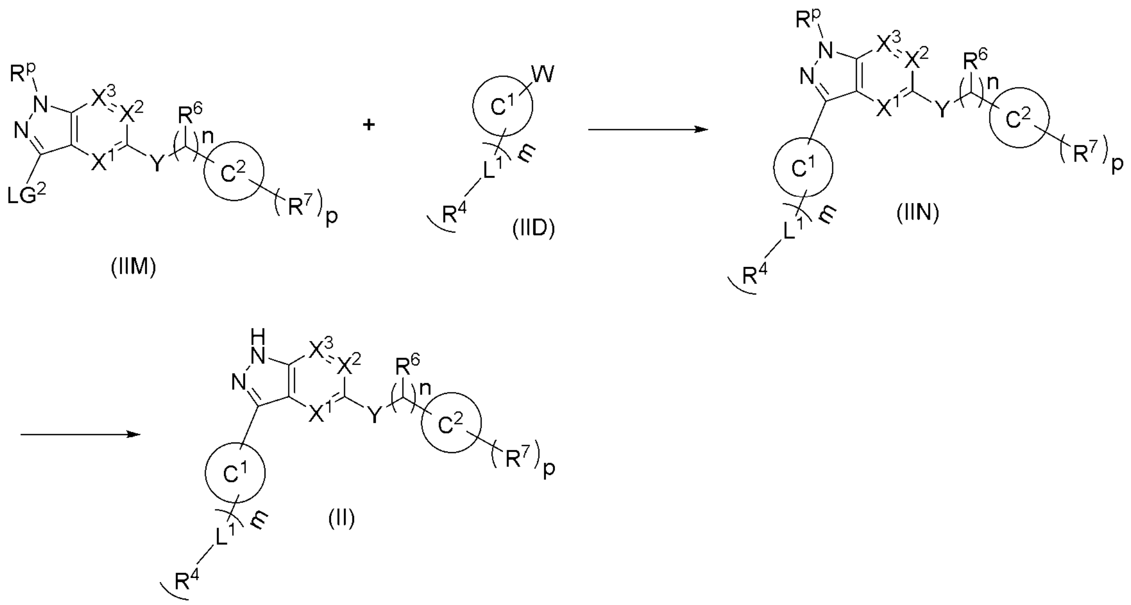

(IIM) (IID) (IIN) (II)

reacting a compound of formula (IIM) with a compound of formula (IID) to obtain a compound of formula (IIN), and removing the protecting group $R^p$ from the compound of formula (IIN) to obtain the compound of formula (II);
wherein, $R^p$ is tetrahydropyranyl, (trimethylsilyl)ethoxymethyl, p-tosyl, t-butyloxycarbonyl, benzyl or p-methoxybenzyl;
$LG^2$ is a leaving group, preferably halogen, methylsulfonyloxy or p-tolylsulfonyloxy;
W is

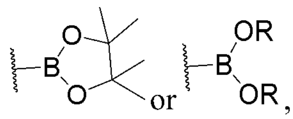

R is hydrogen atom or $C_{1-6}$ alkyl; and
$X^1$, $X^2$, $X^3$, Y, $C^1$, $C^2$, $R^4$, $R^6$, $R^7$, $L^1$, m, n and p are as defined in claim 2;
or

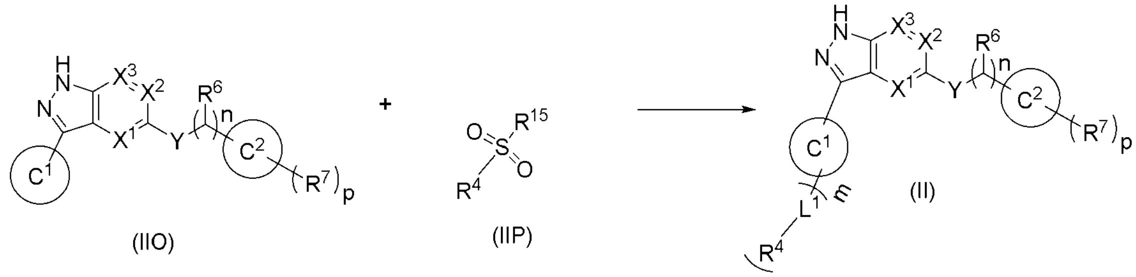

reacting a compound of formula (IIO) with a compound of formula (IIP) to obtain the compound of formula (II); wherein, m is 1, $L^1$ is $-S(O)_2-C_{1-6}$ alkyl-, $R^{15}$ is $C_{1-6}$ alkenyl or $-C_{1-6}$ alkylene-halogen; and
$X^1$, $X^2$, $X^3$, Y, $C^1$, $C^2$, $R^4$, $R^6$, $R^7$, n and p are as defined in claim 2.

**18.** A method for preparing the compound of formula (III-1) according to claim 6, comprising:

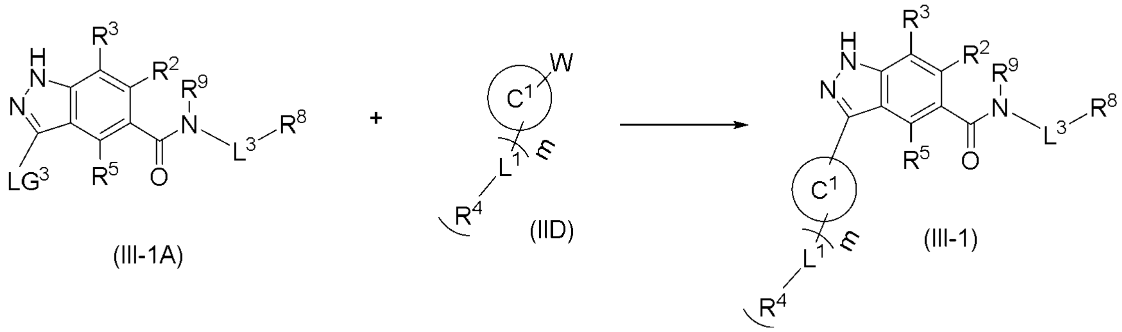

reacting a compound of formula (III-1A) with a compound of formula (IID) to obtain the compound of formula (III-1); wherein, $LG^3$ is a leaving group, preferably halogen, methylsulfonyloxy or p-tolylsulfonyloxy;
W is

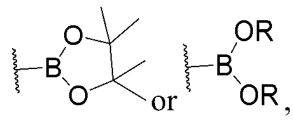

R is hydrogen atom or $C_{1-6}$ alkyl; and
$C^1$, $R^2$, $R^4$, $R^5$, $R^8$, $R^9$, $L^1$, $L^3$ and m are as defined in claim 6.

**19.** A method for preparing the compound of formula (III-2) according to claim 7, comprising:

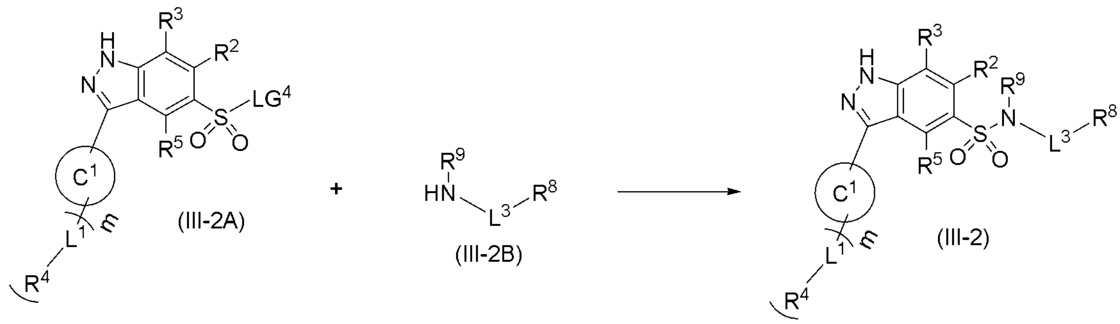

(III-2A) (III-2B) (III-2)

reacting a compound of formula (III-2A) with a compound of formula (III-2B) to obtain the compound of formula (III-2);

wherein, $LG^4$ is halogen, preferably C1 atom;

$C^1$, $R^2$, $R^4$, $R^5$, $R^8$, $R^9$, $L^1$, $L^3$ and m are as defined in claim 7.

**20.** A pharmaceutical composition comprising the compound according to any one of claims 1 to 16, and one or more pharmaceutically acceptable excipients.

**21.** A use of the compound according to any one of claims 1 to 16 or the pharmaceutical composition according to claim 20 in the preparation of a FGFR inhibitor.

**22.** A use of the compound according to any one of claims 1 to 16 or the pharmaceutical composition according to claim 20 in the preparation of a medicament for treating and/or preventing a tumor; particularly, the tumor is a cancer; the cancer is preferably selected from the group consisting of cholangiocarcinoma, liver cancer, breast cancer, prostatic cancer, lung cancer, thyroid cancer, gastric cancer, ovarian cancer, colorectal cancer, endometrial cancer, urothelial cell carcinoma, testicular cancer, cervical cancer, leukemia, skin cancer, squamous-cell carcinoma, basal cell carcinoma, bladder cancer, esophagus cancer, head and neck cancer, kidney cancer, pancreatic cancer, bone cancer, lymphoma, melanoma, sarcoma, peripheral neuroepithelioma, glioma, ependymoma, neuroblastoma, gangliocytoma, medulloblastoma, pinealocytoma, meningeoma, neurofibroma, schwannoma and Wilm's tumor; and more preferably, is selected from the group consisting of cholangiocarcinoma, liver cancer, breast cancer, prostatic cancer, lung cancer, thyroid cancer, gastric cancer, ovarian cancer, colorectal cancer, endometrial cancer and urothelial cell carcinoma.

## INTERNATIONAL SEARCH REPORT

International application No.
**PCT/CN2023/143092**

**A. CLASSIFICATION OF SUBJECT MATTER**

C07D 401/14(2006.01)i; A61K 31/4545(2006.01)i; A61K 31/4439(2006.01)i; A61K 31/496(2006.01)i; A61K 31/5377(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC:C07D; A61K; A61P

Documentation searched other than minimum documentation to the extent that such documents are included in the fields searched

Electronic data base consulted during the international search (name of data base and, where practicable, search terms used)

REGISTRY(STN), CAPLUS(STN), MARPAT(STN), DWPI, CNKI, 百度, Baidu: 江苏亚虹医药科技股份有限公司, 上海亚虹医药科技有限公司, 吡唑, 苯并吡唑, 吲唑, FGFR, 抑制剂, 癌症, 肿瘤, pyrazole, benzopyrazole, pyrazole, inhibitor, 结构检索, structure search.

**C. DOCUMENTS CONSIDERED TO BE RELEVANT**

| Category* | Citation of document, with indication, where appropriate, of the relevant passages | Relevant to claim No. |
|---|---|---|
| X | US 2004092546 A1 (SUGEN, INC.) 13 May 2004 (2004-05-13)<br>claims 1 and 12-20, and description, pages 23-28, and table 1 | 1, 5-6, 8-15, 20-22 |
| X | WO 2021121420 A1 (JIANGSU KDN PHARMACEUTICAL TECHNOLOGY CO., LTD.) 24 June 2021 (2021-06-24)<br>description, pages 12-14, and page 40, embodiment 12 | 1, 5-6, 8-15, 18, 20, 22 |
| PX | WO 2023098699 A1 (NANJING IMMUNOPHAGE BIOTECH CO., LTD.) 08 June 2023 (2023-06-08)<br>claims 1 and 20-23, and description, page 11, paragraph [0030] | 1, 5-6, 8-15, 20, 22 |
| X | CN 106317023 A (SHANGHAI INSTITUTE OF MATERIA MEDICA, CHINESE ACADEMY OF SCIENCES) 11 January 2017 (2017-01-11)<br>claims 1 and 6-9, and description, page 25, paragraphs [0126]-[0127] | 2-4, 16-18 |
| X | CN 114524818 A (SHENZHEN KANGSU PHARMACEUTICAL TECHNOLOGY CO., LTD.) 24 May 2022 (2022-05-24)<br>claims 1-13, and description, paragraphs [0232]-[0546] | 2-4, 16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 March 2024** | **26 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/143092**

**C. DOCUMENTS CONSIDERED TO BE RELEVANT**

| Category* | Citation of document, with indication, where appropriate, of the relevant passages | Relevant to claim No. |
|---|---|---|
| PX | WO 2023279041 A1 (TYRA BIOSCIENCES, INC.) 05 January 2023 (2023-01-05) claims 1 and 77-78, and description, paragraphs [0345]-[0346] and [00454]-[00456] | 2-4, 16-18 |
| X | WO 2022147246 A1 (TYRA BIOSCIENCES, INC.) 07 July 2022 (2022-07-07) claim 1, and description, paragraphs [00419]-[00421] and [00544]-[00607] | 2-4, 16 |
| PX | WO 2023240271 A1 (FOUNTAIN THERAPEUTICS INC.) 14 December 2023 (2023-12-14) claims 1 and 43, and description, paragraphs [017] and [060] | 2-4, 16 |

Form PCT/ISA/210 (second sheet) (July 2022)

**INTERNATIONAL SEARCH REPORT**

International application No.
**PCT/CN2023/143092**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-15, 17-22**
because they relate to parts of the international application that do not comply with the prescribed requirements to such an extent that no meaningful international search can be carried out, specifically:

Claims 1-15 summarize a relatively broad scope of protection on the basis of general formula (I), which results in a search spillover. However, the compounds of the embodiments of the description merely relate to a small part of groups in said claims; therefore, claims 1-15 are not supported by the description, and do not comply with PCT Article 6. By the same reasoning, claims 17-22 containing the above-mentioned compounds also do not comply with PCT Article 6. The search and written opinion with regard to claims 1-22 are provided starting from claim 3 and defining that C1 is a 5-10 membered heteroaryl group and Y is O.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/143092**

| Patent document cited in search report | Publication date (day/month/year) | Patent family member(s) | Publication date (day/month/year) |
|---|---|---|---|
| US 2004092546 A1 | 13 May 2004 | AU 2003259749 A1 | 25 February 2004 |
| | | US 7186716 B2 | 06 March 2007 |
| | | MXPA 05001688 A | 19 April 2005 |
| | | JP 2006501217 A | 12 January 2006 |
| | | CA 2495216 A1 | 19 February 2004 |
| | | EP 1545515 A1 | 29 June 2005 |
| | | BR 0313396 A | 28 June 2005 |
| | | WO 2004014368 A1 | 19 February 2004 |
| WO 2021121420 A1 | 24 June 2021 | None | |
| WO 2023098699 A1 | 08 June 2023 | None | |
| CN 106317023 A | 11 January 2017 | WO 2017008708 A1 | 19 January 2017 |
| CN 114524818 A | 24 May 2022 | EP 4261213 A1 | 18 October 2023 |
| | | WO 2022152274 A1 | 21 July 2022 |
| | | JP 2023554506 A | 27 December 2023 |
| | | US 2023374025 A1 | 23 November 2023 |
| WO 2023279041 A1 | 05 January 2023 | None | |
| WO 2022147246 A1 | 07 July 2022 | None | |
| WO 2023240271 A1 | 14 December 2023 | None | |

Form PCT/ISA/210 (patent family annex) (July 2022)